# EUROPEAN PATENT APPLICATION

(11) **EP 4 786 604 A2**
(43) Date of publication of application: **05.08.2026**
(21) Application number: 26175155.6
(22) Date of filing: 11.09.2020
(51) Int. Cl.: C12P 21/02

(54) **DCR3 VARIANT**

(30) Priority: 13.09.2019 JP 2019167728
(62) Divisional of application: 20864177.9
(71) Applicant: Kyowa Kirin Co., Ltd., Tokyo 100-0004 (JP)
(72) Inventor: KANAI, Akiko, Tokyo, 100-0004 (JP); HOSOMI, Hitomi, Tokyo, 100-0004 (JP); YONEZAWA, Sakiko, Tokyo, 100-0004 (JP)
(74) Representative: Hoffmann Eitle

(57) **Abstract**

An object of the present invention is to provide a DcR3 variant that has binding activity (preferably neutralizing activity) to a ligand of DcR3, and that results in a decreased amount of aggregates as compared to wild-type DcR3 when produced using a cell derived from a mammal as a host, and/or that exhibits improved *in vivo* kinetics; a DNA encoding the DcR3 variant; a vector including the DNA; a transformant obtained by introducing the vector; a method for producing a variant using the transformant; and a prophylactic or therapeutic agent for an autoimmune disease, an inflammatory disease or an allergic disease including the variant as an active ingredient, and, in order to achieve the object, the present invention provides a DcR3 variant including a part of DcR3 and a part of a TNF superfamily molecule.

SEQUENCE LISTING

<110>  KYOWA KIRIN CO., LTD.

<120>  DcR3 variant

<130>  233864PX

<160>  355

<170>  PatentIn version 3.5

<210>  1
<211>  900
<212>  DNA
<213>  Homo sapiens

<220>
<221>  CDS
<222>  (1)..(900)

<220>
<223>  Human DcR3 cDNA (Accession number: NM_003823.3)

<400>  1
atg agg gcg ctg gag ggg cca ggc ctg tcg ctg ctg tgc ctg gtg ttg
48
Met Arg Ala Leu Glu Gly Pro Gly Leu Ser Leu Leu Cys Leu Val Leu
1               5                   10                  15

gcg ctg cct gcc ctg ctg ccg gtg ccg gct gta cgc gga gtg gca gaa
96
Ala Leu Pro Ala Leu Leu Pro Val Pro Ala Val Arg Gly Val Ala Glu
            20                  25                  30

aca ccc acc tac ccc tgg cgg gac gca gag aca ggg gag cgg ctg gtg
144
Thr Pro Thr Tyr Pro Trp Arg Asp Ala Glu Thr Gly Glu Arg Leu Val
        35                  40                  45

tgc gcc cag tgc ccc cca ggc acc ttt gtg cag cgg ccg tgc cgc cga
192
Cys Ala Gln Cys Pro Pro Gly Thr Phe Val Gln Arg Pro Cys Arg Arg
    50                  55                  60

gac agc ccc acg acg tgt ggc ccg tgt cca ccg cgc cac tac acg cag
240
Asp Ser Pro Thr Thr Cys Gly Pro Cys Pro Pro Arg His Tyr Thr Gln
65                  70                  75                  80

ttc tgg aac tac cta gag cgc tgc cgc tac tgc aac gtc ctc tgc ggg
288
Phe Trp Asn Tyr Leu Glu Arg Cys Arg Tyr Cys Asn Val Leu Cys Gly
                85                  90                  95

gag cgt gag gag gag gca cgg gct tgc cac gcc acc cac aac cgt gcc
336
Glu Arg Glu Glu Glu Ala Arg Ala Cys His Ala Thr His Asn Arg Ala
            100                 105                 110

tgc cgc tgc cgc acc ggc ttc ttc gcg cac gct ggt ttc tgc ttg gag
384
Cys Arg Cys Arg Thr Gly Phe Phe Ala His Ala Gly Phe Cys Leu Glu
        115                 120                 125

cac gca tcg tgt cca cct ggt gcc ggc gtg att gcc ccg ggc acc ccc
432
His Ala Ser Cys Pro Pro Gly Ala Gly Val Ile Ala Pro Gly Thr Pro
    130                 135                 140

agc cag aac acg cag tgc cag ccg tgc ccc cca ggc acc ttc tca gcc
480
Ser Gln Asn Thr Gln Cys Gln Pro Cys Pro Pro Gly Thr Phe Ser Ala
145                 150                 155                 160

agc agc tcc agc tca gag cag tgc cag ccc cac cgc aac tgc acg gcc
528
Ser Ser Ser Ser Ser Glu Gln Cys Gln Pro His Arg Asn Cys Thr Ala
                165                 170                 175

ctg ggc ctg gcc ctc aat gtg cca ggc tct tcc tcc cat gac acc ctg
576
Leu Gly Leu Ala Leu Asn Val Pro Gly Ser Ser Ser His Asp Thr Leu
            180                 185                 190

tgc acc agc tgc act ggc ttc ccc ctc agc acc agg gta cca gga gct
624
Cys Thr Ser Cys Thr Gly Phe Pro Leu Ser Thr Arg Val Pro Gly Ala
        195                 200                 205

gag gag tgt gag cgt gcc gtc atc gac ttt gtg gct ttc cag gac atc
672
Glu Glu Cys Glu Arg Ala Val Ile Asp Phe Val Ala Phe Gln Asp Ile
    210                 215                 220

tcc atc aag agg ctg cag cgg ctg ctg cag gcc ctc gag gcc ccg gag
720
Ser Ile Lys Arg Leu Gln Arg Leu Leu Gln Ala Leu Glu Ala Pro Glu
225                 230                 235                 240

ggc tgg ggt ccg aca cca agg gcg ggc cgc gcg gcc ttg cag ctg aag
768
Gly Trp Gly Pro Thr Pro Arg Ala Gly Arg Ala Ala Leu Gln Leu Lys
                245                 250                 255

ctg cgt cgg cgg ctc acg gag ctc ctg ggg gcg cag gac ggg gcg ctg
816
Leu Arg Arg Arg Leu Thr Glu Leu Leu Gly Ala Gln Asp Gly Ala Leu
            260                 265                 270

ctg gtg cgg ctg ctg cag gcg ctg cgc gtg gcc agg atg ccc ggg ctg
864
Leu Val Arg Leu Leu Gln Ala Leu Arg Val Ala Arg Met Pro Gly Leu
        275                 280                 285

gag cgg agc gtc cgt gag cgc ttc ctc cct gtg cac
900
Glu Arg Ser Val Arg Glu Arg Phe Leu Pro Val His
    290                 295                 300

<210>  2
<211>  300
<212>  PRT
<213>  Homo sapiens

<220>
<223>  Amino acid sequence of Human DcR3 (Accession number: NP_003814.1)

<400>  2

Met Arg Ala Leu Glu Gly Pro Gly Leu Ser Leu Leu Cys Leu Val Leu
1               5                   10                  15

Ala Leu Pro Ala Leu Leu Pro Val Pro Ala Val Arg Gly Val Ala Glu
            20                  25                  30

Thr Pro Thr Tyr Pro Trp Arg Asp Ala Glu Thr Gly Glu Arg Leu Val
        35                  40                  45

Cys Ala Gln Cys Pro Pro Gly Thr Phe Val Gln Arg Pro Cys Arg Arg
    50                  55                  60

Asp Ser Pro Thr Thr Cys Gly Pro Cys Pro Pro Arg His Tyr Thr Gln
65                  70                  75                  80

Phe Trp Asn Tyr Leu Glu Arg Cys Arg Tyr Cys Asn Val Leu Cys Gly
                85                  90                  95

Glu Arg Glu Glu Glu Ala Arg Ala Cys His Ala Thr His Asn Arg Ala
            100                 105                 110

Cys Arg Cys Arg Thr Gly Phe Phe Ala His Ala Gly Phe Cys Leu Glu
        115                 120                 125

His Ala Ser Cys Pro Pro Gly Ala Gly Val Ile Ala Pro Gly Thr Pro
    130                 135                 140

Ser Gln Asn Thr Gln Cys Gln Pro Cys Pro Pro Gly Thr Phe Ser Ala
145                 150                 155                 160

Ser Ser Ser Ser Ser Glu Gln Cys Gln Pro His Arg Asn Cys Thr Ala
                165                 170                 175

Leu Gly Leu Ala Leu Asn Val Pro Gly Ser Ser Ser His Asp Thr Leu
            180                 185                 190

Cys Thr Ser Cys Thr Gly Phe Pro Leu Ser Thr Arg Val Pro Gly Ala

        195                 200                 205

Glu Glu Cys Glu Arg Ala Val Ile Asp Phe Val Ala Phe Gln Asp Ile
    210                 215                 220

Ser Ile Lys Arg Leu Gln Arg Leu Leu Gln Ala Leu Glu Ala Pro Glu
225                 230                 235                 240

Gly Trp Gly Pro Thr Pro Arg Ala Gly Arg Ala Ala Leu Gln Leu Lys
                245                 250                 255

Leu Arg Arg Arg Leu Thr Glu Leu Leu Gly Ala Gln Asp Gly Ala Leu
            260                 265                 270

Leu Val Arg Leu Leu Gln Ala Leu Arg Val Ala Arg Met Pro Gly Leu
        275                 280                 285

Glu Arg Ser Val Arg Glu Arg Phe Leu Pro Val His
    290                 295                 300

<210>  3
<211>  813
<212>  DNA
<213>  Homo sapiens

<220>
<221>  CDS
<222>  (1)..(813)

<220>
<223>  Nucleotide sequence of Human DcR3 (without a signal peptide)

<400>  3
gtg gca gaa aca ccc acc tac ccc tgg cgg gac gca gag aca ggg gag
48
Val Ala Glu Thr Pro Thr Tyr Pro Trp Arg Asp Ala Glu Thr Gly Glu
1               5                   10                  15

cgg ctg gtg tgc gcc cag tgc ccc cca ggc acc ttt gtg cag cgg ccg
96
Arg Leu Val Cys Ala Gln Cys Pro Pro Gly Thr Phe Val Gln Arg Pro
            20                  25                  30

tgc cgc cga gac agc ccc acg acg tgt ggc ccg tgt cca ccg cgc cac
144
Cys Arg Arg Asp Ser Pro Thr Thr Cys Gly Pro Cys Pro Pro Arg His
        35                  40                  45

tac acg cag ttc tgg aac tac cta gag cgc tgc cgc tac tgc aac gtc
192
Tyr Thr Gln Phe Trp Asn Tyr Leu Glu Arg Cys Arg Tyr Cys Asn Val
    50                  55                  60

ctc tgc ggg gag cgt gag gag gag gca cgg gct tgc cac gcc acc cac
240
Leu Cys Gly Glu Arg Glu Glu Glu Ala Arg Ala Cys His Ala Thr His
65                  70                  75                  80

aac cgt gcc tgc cgc tgc cgc acc ggc ttc ttc gcg cac gct ggt ttc
288
Asn Arg Ala Cys Arg Cys Arg Thr Gly Phe Phe Ala His Ala Gly Phe
                85                  90                  95

tgc ttg gag cac gca tcg tgt cca cct ggt gcc ggc gtg att gcc ccg
336
Cys Leu Glu His Ala Ser Cys Pro Pro Gly Ala Gly Val Ile Ala Pro
            100                 105                 110

ggc acc ccc agc cag aac acg cag tgc cag ccg tgc ccc cca ggc acc
384
Gly Thr Pro Ser Gln Asn Thr Gln Cys Gln Pro Cys Pro Pro Gly Thr
        115                 120                 125

ttc tca gcc agc agc tcc agc tca gag cag tgc cag ccc cac cgc aac
432
Phe Ser Ala Ser Ser Ser Ser Ser Glu Gln Cys Gln Pro His Arg Asn
    130                 135                 140

tgc acg gcc ctg ggc ctg gcc ctc aat gtg cca ggc tct tcc tcc cat
480
Cys Thr Ala Leu Gly Leu Ala Leu Asn Val Pro Gly Ser Ser Ser His
145                 150                 155                 160

gac acc ctg tgc acc agc tgc act ggc ttc ccc ctc agc acc agg gta
528
Asp Thr Leu Cys Thr Ser Cys Thr Gly Phe Pro Leu Ser Thr Arg Val
                165                 170                 175

cca gga gct gag gag tgt gag cgt gcc gtc atc gac ttt gtg gct ttc
576
Pro Gly Ala Glu Glu Cys Glu Arg Ala Val Ile Asp Phe Val Ala Phe
            180                 185                 190

cag gac atc tcc atc aag agg ctg cag cgg ctg ctg cag gcc ctc gag
624
Gln Asp Ile Ser Ile Lys Arg Leu Gln Arg Leu Leu Gln Ala Leu Glu
        195                 200                 205

gcc ccg gag ggc tgg ggt ccg aca cca agg gcg ggc cgc gcg gcc ttg
672
Ala Pro Glu Gly Trp Gly Pro Thr Pro Arg Ala Gly Arg Ala Ala Leu
    210                 215                 220

cag ctg aag ctg cgt cgg cgg ctc acg gag ctc ctg ggg gcg cag gac
720
Gln Leu Lys Leu Arg Arg Arg Leu Thr Glu Leu Leu Gly Ala Gln Asp
225                 230                 235                 240

ggg gcg ctg ctg gtg cgg ctg ctg cag gcg ctg cgc gtg gcc agg atg
768
Gly Ala Leu Leu Val Arg Leu Leu Gln Ala Leu Arg Val Ala Arg Met
                245                 250                 255

ccc ggg ctg gag cgg agc gtc cgt gag cgc ttc ctc cct gtg cac
813
Pro Gly Leu Glu Arg Ser Val Arg Glu Arg Phe Leu Pro Val His
            260                 265                 270

<210>  4
<211>  271
<212>  PRT
<213>  Homo sapiens

<220>
<223>  Amino acid sequence of Human DcR3 (without a signal peptide)

<400>  4

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser Cys Thr Gly Phe Pro Leu Ser Thr Arg Val
                165                 170                 175

Pro Gly Ala Glu Glu Cys Glu Arg Ala Val Ile Asp Phe Val Ala Phe
                180                 185                 190

Gln Asp Ile Ser Ile Lys Arg Leu Gln Arg Leu Leu Gln Ala Leu Glu
        195                 200                 205

Ala Pro Glu Gly Trp Gly Pro Thr Pro Arg Ala Gly Arg Ala Ala Leu
    210                 215                 220

Gln Leu Lys Leu Arg Arg Arg Leu Thr Glu Leu Leu Gly Ala Gln Asp
225                 230                 235                 240

Gly Ala Leu Leu Val Arg Leu Leu Gln Ala Leu Arg Val Ala Arg Met
                245                 250                 255

Pro Gly Leu Glu Arg Ser Val Arg Glu Arg Phe Leu Pro Val His
                260                 265                 270

<210>  5
<211>  123
<212>  DNA
<213>  Homo sapiens

<220>
<221>  CDS
<222>  (1)..(123)

<220>
<223>  Nucleotide sequence of CRD1 of Human DcR3

<400>  5
gtg gca gaa aca ccc acc tac ccc tgg cgg gac gca gag aca ggg gag
48
Val Ala Glu Thr Pro Thr Tyr Pro Trp Arg Asp Ala Glu Thr Gly Glu
1               5                   10                  15

cgg ctg gtg tgc gcc cag tgc ccc cca ggc acc ttt gtg cag cgg ccg
96
Arg Leu Val Cys Ala Gln Cys Pro Pro Gly Thr Phe Val Gln Arg Pro
                20                  25                  30

tgc cgc cga gac agc ccc acg acg tgt
123
Cys Arg Arg Asp Ser Pro Thr Thr Cys
        35                  40

<210>  6
<211>  41
<212>  PRT
<213>  Homo sapiens

<220>
<223>  Amino acid sequence of CRD1 of Human DcR3

<400>  6

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys
        35                  40

<210>  7
<211>  123
<212>  DNA
<213>  Homo sapiens

<220>
<221>  CDS
<222>  (1)..(123)

<220>
<223>  Nucleotide sequence of CRD2 of Human DcR3

<400>  7
tgt cca ccg cgc cac tac acg cag ttc tgg aac tac cta gag cgc tgc
48
Cys Pro Pro Arg His Tyr Thr Gln Phe Trp Asn Tyr Leu Glu Arg Cys
1               5                   10                  15

cgc tac tgc aac gtc ctc tgc ggg gag cgt gag gag gag gca cgg gct
96
Arg Tyr Cys Asn Val Leu Cys Gly Glu Arg Glu Glu Glu Ala Arg Ala
            20                  25                  30

tgc cac gcc acc cac aac cgt gcc tgc
123
Cys His Ala Thr His Asn Arg Ala Cys
        35                  40

<210>  8
<211>  41
<212>  PRT
<213>  Homo sapiens

<220>
<223>  Amino acid sequence of CRD2 of Human DcR3

<400>  8

Cys Pro Pro Arg His Tyr Thr Gln Phe Trp Asn Tyr Leu Glu Arg Cys
1               5                   10                  15

Arg Tyr Cys Asn Val Leu Cys Gly Glu Arg Glu Glu Glu Ala Arg Ala
            20                  25                  30

Cys His Ala Thr His Asn Arg Ala Cys
        35                  40



<210>  9
<211>  108
<212>  DNA
<213>  Homo sapiens

<220>
<221>  CDS
<222>  (1)..(108)

<220>
<223>  Nucleotide sequence of CRD3 of Human DcR3

<400>  9
tgc cgc acc ggc ttc ttc gcg cac gct ggt ttc tgc ttg gag cac gca
48
Cys Arg Thr Gly Phe Phe Ala His Ala Gly Phe Cys Leu Glu His Ala
1               5                   10                  15

tcg tgt cca cct ggt gcc ggc gtg att gcc ccg ggc acc ccc agc cag
96
Ser Cys Pro Pro Gly Ala Gly Val Ile Ala Pro Gly Thr Pro Ser Gln
            20                  25                  30

aac acg cag tgc
108
Asn Thr Gln Cys
        35



<210>  10
<211>  36
<212>  PRT
<213>  Homo sapiens

<220>
<223>  Amino acid sequence of CRD3 of Human DcR3

<400>  10

Cys Arg Thr Gly Phe Phe Ala His Ala Gly Phe Cys Leu Glu His Ala
1               5                   10                  15


Ser Cys Pro Pro Gly Ala Gly Val Ile Ala Pro Gly Thr Pro Ser Gln
            20                  25                  30


Asn Thr Gln Cys
        35



<210>  11
<211>  123
<212>  DNA

<213>  Homo sapiens

<220>
<221>  CDS
<222>  (1)..(123)

<220>
<223>  Nucleotide sequence of CRD4 of Human DcR3

<400>  11
tgc ccc cca ggc acc ttc tca gcc agc agc tcc agc tca gag cag tgc
48
Cys Pro Pro Gly Thr Phe Ser Ala Ser Ser Ser Ser Ser Glu Gln Cys
1               5                   10                  15

cag ccc cac cgc aac tgc acg gcc ctg ggc ctg gcc ctc aat gtg cca
96
Gln Pro His Arg Asn Cys Thr Ala Leu Gly Leu Ala Leu Asn Val Pro
            20                  25                  30

ggc tct tcc tcc cat gac acc ctg tgc
123
Gly Ser Ser Ser His Asp Thr Leu Cys
        35                  40


<210>  12
<211>  41
<212>  PRT
<213>  Homo sapiens

<220>
<223>  Amino acid sequence of CRD4 of Human DcR3

<400>  12

Cys Pro Pro Gly Thr Phe Ser Ala Ser Ser Ser Ser Ser Glu Gln Cys
1               5                   10                  15


Gln Pro His Arg Asn Cys Thr Ala Leu Gly Leu Ala Leu Asn Val Pro
            20                  25                  30


Gly Ser Ser Ser His Asp Thr Leu Cys
        35                  40


<210>  13
<211>  1203
<212>  DNA
<213>  Homo sapiens

<220>
<221>  CDS
<222>  (1)..(1203)

<220>
<223>  Nucleotide sequence of Human OPG (Accession number: NM_002546.3)

<400> 13
atg aac aac ttg ctg tgc tgc gcg ctc gtg ttt ctg gac atc tcc att
48
Met Asn Asn Leu Leu Cys Cys Ala Leu Val Phe Leu Asp Ile Ser Ile
1               5                   10                  15

aag tgg acc acc cag gaa acg ttt cct cca aag tac ctt cat tat gac
96
Lys Trp Thr Thr Gln Glu Thr Phe Pro Pro Lys Tyr Leu His Tyr Asp
            20                  25                  30

gaa gaa acc tct cat cag ctg ttg tgt gac aaa tgt cct cct ggt acc
144
Glu Glu Thr Ser His Gln Leu Leu Cys Asp Lys Cys Pro Pro Gly Thr
        35                  40                  45

tac cta aaa caa cac tgt aca gca aag tgg aag acc gtg tgc gcc cct
192
Tyr Leu Lys Gln His Cys Thr Ala Lys Trp Lys Thr Val Cys Ala Pro
    50                  55                  60

tgc cct gac cac tac tac aca gac agc tgg cac acc agt gac gag tgt
240
Cys Pro Asp His Tyr Tyr Thr Asp Ser Trp His Thr Ser Asp Glu Cys
65                  70                  75                  80

cta tac tgc agc ccc gtg tgc aag gag ctg cag tac gtc aag cag gag
288
Leu Tyr Cys Ser Pro Val Cys Lys Glu Leu Gln Tyr Val Lys Gln Glu
                85                  90                  95

tgc aat cgc acc cac aac cgc gtg tgc gaa tgc aag gaa ggg cgc tac
336
Cys Asn Arg Thr His Asn Arg Val Cys Glu Cys Lys Glu Gly Arg Tyr
            100                 105                 110

ctt gag ata gag ttc tgc ttg aaa cat agg agc tgc cct cct gga ttt
384
Leu Glu Ile Glu Phe Cys Leu Lys His Arg Ser Cys Pro Pro Gly Phe
        115                 120                 125

gga gtg gtg caa gct gga acc cca gag cga aat aca gtt tgc aaa aga
432
Gly Val Val Gln Ala Gly Thr Pro Glu Arg Asn Thr Val Cys Lys Arg
    130                 135                 140

tgt cca gat ggg ttc ttc tca aat gag acg tca tct aaa gca ccc tgt
480
Cys Pro Asp Gly Phe Phe Ser Asn Glu Thr Ser Ser Lys Ala Pro Cys
145                 150                 155                 160

aga aaa cac aca aat tgc agt gtc ttt ggt ctc ctg cta act cag aaa
528
Arg Lys His Thr Asn Cys Ser Val Phe Gly Leu Leu Leu Thr Gln Lys
                165                 170                 175

gga aat gca aca cac gac aac ata tgt tcc gga aac agt gaa tca act
576
Gly Asn Ala Thr His Asp Asn Ile Cys Ser Gly Asn Ser Glu Ser Thr
            180                 185                 190

caa aaa tgt gga ata gat gtt acc ctg tgt gag gag gca ttc ttc agg
624
Gln Lys Cys Gly Ile Asp Val Thr Leu Cys Glu Glu Ala Phe Phe Arg
        195                 200                 205

ttt gct gtt cct aca aag ttt acg cct aac tgg ctt agt gtc ttg gta
672
Phe Ala Val Pro Thr Lys Phe Thr Pro Asn Trp Leu Ser Val Leu Val
    210                 215                 220

gac aat ttg cct ggc acc aaa gta aac gca gag agt gta gag agg ata
720
Asp Asn Leu Pro Gly Thr Lys Val Asn Ala Glu Ser Val Glu Arg Ile
225                 230                 235                 240

aaa cgg caa cac agc tca caa gaa cag act ttc cag ctg ctg aag tta
768
Lys Arg Gln His Ser Ser Gln Glu Gln Thr Phe Gln Leu Leu Lys Leu
                245                 250                 255

tgg aaa cat caa aac aaa gac caa gat ata gtc aag aag atc atc caa
816
Trp Lys His Gln Asn Lys Asp Gln Asp Ile Val Lys Lys Ile Ile Gln
            260                 265                 270

gat att gac ctc tgt gaa aac agc gtg cag cgg cac att gga cat gct
864
Asp Ile Asp Leu Cys Glu Asn Ser Val Gln Arg His Ile Gly His Ala
        275                 280                 285

aac ctc acc ttc gag cag ctt cgt agc ttg atg gaa agc tta ccg gga
912
Asn Leu Thr Phe Glu Gln Leu Arg Ser Leu Met Glu Ser Leu Pro Gly
    290                 295                 300

aag aaa gtg gga gca gaa gac att gaa aaa aca ata aag gca tgc aaa
960
Lys Lys Val Gly Ala Glu Asp Ile Glu Lys Thr Ile Lys Ala Cys Lys
305                 310                 315                 320

ccc agt gac cag atc ctg aag ctg ctc agt ttg tgg cga ata aaa aat
1008
Pro Ser Asp Gln Ile Leu Lys Leu Leu Ser Leu Trp Arg Ile Lys Asn
                325                 330                 335

ggc gac caa gac acc ttg aag ggc cta atg cac gca cta aag cac tca
1056
Gly Asp Gln Asp Thr Leu Lys Gly Leu Met His Ala Leu Lys His Ser
            340                 345                 350

aag acg tac cac ttt ccc aaa act gtc act cag agt cta aag aag acc
1104
Lys Thr Tyr His Phe Pro Lys Thr Val Thr Gln Ser Leu Lys Lys Thr
        355                 360                 365

atc agg ttc ctt cac agc ttc aca atg tac aaa ttg tat cag aag tta
1152
Ile Arg Phe Leu His Ser Phe Thr Met Tyr Lys Leu Tyr Gln Lys Leu
    370                 375                 380

ttt tta gaa atg ata ggt aac cag gtc caa tca gta aaa ata agc tgc
1200
Phe Leu Glu Met Ile Gly Asn Gln Val Gln Ser Val Lys Ile Ser Cys
385                 390                 395                 400

tta
1203
Leu

<210>  14
<211>  401
<212>  PRT
<213>  Homo sapiens

<220>
<223>  Amino acid sequence of Human OPG (Accession number: NP_002537.3)

<400>  14

Met Asn Asn Leu Leu Cys Cys Ala Leu Val Phe Leu Asp Ile Ser Ile
1               5                   10                  15

Lys Trp Thr Thr Gln Glu Thr Phe Pro Pro Lys Tyr Leu His Tyr Asp
            20                  25                  30

Glu Glu Thr Ser His Gln Leu Leu Cys Asp Lys Cys Pro Pro Gly Thr
        35                  40                  45

Tyr Leu Lys Gln His Cys Thr Ala Lys Trp Lys Thr Val Cys Ala Pro
    50                  55                  60

Cys Pro Asp His Tyr Tyr Thr Asp Ser Trp His Thr Ser Asp Glu Cys
65                  70                  75                  80

Leu Tyr Cys Ser Pro Val Cys Lys Glu Leu Gln Tyr Val Lys Gln Glu
                85                  90                  95

Cys Asn Arg Thr His Asn Arg Val Cys Glu Cys Lys Glu Gly Arg Tyr
            100                 105                 110

Leu Glu Ile Glu Phe Cys Leu Lys His Arg Ser Cys Pro Pro Gly Phe
        115                 120                 125

Gly Val Val Gln Ala Gly Thr Pro Glu Arg Asn Thr Val Cys Lys Arg
    130                 135                 140

Cys Pro Asp Gly Phe Phe Ser Asn Glu Thr Ser Ser Lys Ala Pro Cys
145                 150                 155                 160

Arg Lys His Thr Asn Cys Ser Val Phe Gly Leu Leu Leu Thr Gln Lys
                165                 170                 175

Gly Asn Ala Thr His Asp Asn Ile Cys Ser Gly Asn Ser Glu Ser Thr
            180                 185                 190

Gln Lys Cys Gly Ile Asp Val Thr Leu Cys Glu Glu Ala Phe Phe Arg
        195                 200                 205

Phe Ala Val Pro Thr Lys Phe Thr Pro Asn Trp Leu Ser Val Leu Val
    210                 215                 220

Asp Asn Leu Pro Gly Thr Lys Val Asn Ala Glu Ser Val Glu Arg Ile
225                 230                 235                 240

Lys Arg Gln His Ser Ser Gln Glu Gln Thr Phe Gln Leu Leu Lys Leu
                245                 250                 255

Trp Lys His Gln Asn Lys Asp Gln Asp Ile Val Lys Lys Ile Ile Gln
            260                 265                 270

Asp Ile Asp Leu Cys Glu Asn Ser Val Gln Arg His Ile Gly His Ala
        275                 280                 285

Asn Leu Thr Phe Glu Gln Leu Arg Ser Leu Met Glu Ser Leu Pro Gly
    290                 295                 300

Lys Lys Val Gly Ala Glu Asp Ile Glu Lys Thr Ile Lys Ala Cys Lys
305                 310                 315                 320

Pro Ser Asp Gln Ile Leu Lys Leu Leu Ser Leu Trp Arg Ile Lys Asn
                325                 330                 335

Gly Asp Gln Asp Thr Leu Lys Gly Leu Met His Ala Leu Lys His Ser
            340                 345                 350

Lys Thr Tyr His Phe Pro Lys Thr Val Thr Gln Ser Leu Lys Lys Thr
        355                 360                 365

Ile Arg Phe Leu His Ser Phe Thr Met Tyr Lys Leu Tyr Gln Lys Leu
    370                 375                 380

Phe Leu Glu Met Ile Gly Asn Gln Val Gln Ser Val Lys Ile Ser Cys
385                 390                 395                 400

Leu

<210> 15
<211> 1140
<212> DNA
<213> Homo sapiens

<220>
<221> CDS
<222> (1)..(1140)

<220>
<223> Nucleotide sequence of Human OPG (without a signal peptide)

<400> 15
gaa acg ttt cct cca aag tac ctt cat tat gac gaa gaa acc tct cat
48
Glu Thr Phe Pro Pro Lys Tyr Leu His Tyr Asp Glu Glu Thr Ser His
1               5                   10                  15

cag ctg ttg tgt gac aaa tgt cct cct ggt acc tac cta aaa caa cac
96
Gln Leu Leu Cys Asp Lys Cys Pro Pro Gly Thr Tyr Leu Lys Gln His
            20                  25                  30

tgt aca gca aag tgg aag acc gtg tgc gcc cct tgc cct gac cac tac
144
Cys Thr Ala Lys Trp Lys Thr Val Cys Ala Pro Cys Pro Asp His Tyr
        35                  40                  45

tac aca gac agc tgg cac acc agt gac gag tgt cta tac tgc agc ccc
192
Tyr Thr Asp Ser Trp His Thr Ser Asp Glu Cys Leu Tyr Cys Ser Pro
    50                  55                  60

gtg tgc aag gag ctg cag tac gtc aag cag gag tgc aat cgc acc cac
240
Val Cys Lys Glu Leu Gln Tyr Val Lys Gln Glu Cys Asn Arg Thr His
65                  70                  75                  80

aac cgc gtg tgc gaa tgc aag gaa ggg cgc tac ctt gag ata gag ttc
288
Asn Arg Val Cys Glu Cys Lys Glu Gly Arg Tyr Leu Glu Ile Glu Phe
                85                  90                  95

tgc ttg aaa cat agg agc tgc cct cct gga ttt gga gtg gtg caa gct
336
Cys Leu Lys His Arg Ser Cys Pro Pro Gly Phe Gly Val Val Gln Ala
            100                 105                 110

gga acc cca gag cga aat aca gtt tgc aaa aga tgt cca gat ggg ttc
384
Gly Thr Pro Glu Arg Asn Thr Val Cys Lys Arg Cys Pro Asp Gly Phe
        115                 120                 125

ttc tca aat gag acg tca tct aaa gca ccc tgt aga aaa cac aca aat
432
Phe Ser Asn Glu Thr Ser Ser Lys Ala Pro Cys Arg Lys His Thr Asn
    130                 135                 140

tgc agt gtc ttt ggt ctc ctg cta act cag aaa gga aat gca aca cac
480
Cys Ser Val Phe Gly Leu Leu Leu Thr Gln Lys Gly Asn Ala Thr His
145                 150                 155                 160

gac aac ata tgt tcc gga aac agt gaa tca act caa aaa tgt gga ata
528
Asp Asn Ile Cys Ser Gly Asn Ser Glu Ser Thr Gln Lys Cys Gly Ile
                165                 170                 175

gat gtt acc ctg tgt gag gag gca ttc ttc agg ttt gct gtt cct aca
576
Asp Val Thr Leu Cys Glu Glu Ala Phe Phe Arg Phe Ala Val Pro Thr
            180                 185                 190

aag ttt acg cct aac tgg ctt agt gtc ttg gta gac aat ttg cct ggc
624
Lys Phe Thr Pro Asn Trp Leu Ser Val Leu Val Asp Asn Leu Pro Gly
        195                 200                 205

acc aaa gta aac gca gag agt gta gag agg ata aaa cgg caa cac agc
672
Thr Lys Val Asn Ala Glu Ser Val Glu Arg Ile Lys Arg Gln His Ser
    210                 215                 220

tca caa gaa cag act ttc cag ctg ctg aag tta tgg aaa cat caa aac
720
Ser Gln Glu Gln Thr Phe Gln Leu Leu Lys Leu Trp Lys His Gln Asn
225                 230                 235                 240

aaa gac caa gat ata gtc aag aag atc atc caa gat att gac ctc tgt
768
Lys Asp Gln Asp Ile Val Lys Lys Ile Ile Gln Asp Ile Asp Leu Cys
                245                 250                 255

gaa aac agc gtg cag cgg cac att gga cat gct aac ctc acc ttc gag
816
Glu Asn Ser Val Gln Arg His Ile Gly His Ala Asn Leu Thr Phe Glu
            260                 265                 270

cag ctt cgt agc ttg atg gaa agc tta ccg gga aag aaa gtg gga gca
864
Gln Leu Arg Ser Leu Met Glu Ser Leu Pro Gly Lys Lys Val Gly Ala
        275                 280                 285

gaa gac att gaa aaa aca ata aag gca tgc aaa ccc agt gac cag atc
912
Glu Asp Ile Glu Lys Thr Ile Lys Ala Cys Lys Pro Ser Asp Gln Ile
    290                 295                 300

ctg aag ctg ctc agt ttg tgg cga ata aaa aat ggc gac caa gac acc
960
Leu Lys Leu Leu Ser Leu Trp Arg Ile Lys Asn Gly Asp Gln Asp Thr
305                 310                 315                 320

ttg aag ggc cta atg cac gca cta aag cac tca aag acg tac cac ttt
1008
Leu Lys Gly Leu Met His Ala Leu Lys His Ser Lys Thr Tyr His Phe
                325                 330                 335

ccc aaa act gtc act cag agt cta aag aag acc atc agg ttc ctt cac
1056
Pro Lys Thr Val Thr Gln Ser Leu Lys Lys Thr Ile Arg Phe Leu His
            340                 345                 350

agc ttc aca atg tac aaa ttg tat cag aag tta ttt tta gaa atg ata
1104
Ser Phe Thr Met Tyr Lys Leu Tyr Gln Lys Leu Phe Leu Glu Met Ile
        355                 360                 365

ggt aac cag gtc caa tca gta aaa ata agc tgc tta
1140
Gly Asn Gln Val Gln Ser Val Lys Ile Ser Cys Leu
    370                 375                 380


<210>  16
<211>  380
<212>  PRT
<213>  Homo sapiens

<220>
<223>  Amino acid sequence of Human OPG (without a signal peptide)

<400>  16

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Asp His Tyr
        35                  40                  45


Tyr Thr Asp Ser Trp His Thr Ser Asp Glu Cys Leu Tyr Cys Ser Pro
    50                  55                  60


Val Cys Lys Glu Leu Gln Tyr Val Lys Gln Glu Cys Asn Arg Thr His
65                  70                  75                  80


Asn Arg Val Cys Glu Cys Lys Glu Gly Arg Tyr Leu Glu Ile Glu Phe
                85                  90                  95


Cys Leu Lys His Arg Ser Cys Pro Pro Gly Phe Gly Val Val Gln Ala
            100                 105                 110


Gly Thr Pro Glu Arg Asn Thr Val Cys Lys Arg Cys Pro Asp Gly Phe

115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Cys Gly Ile
                165                 170                 175

Asp Val Thr Leu Cys Glu Glu Ala Phe Phe Arg Phe Ala Val Pro Thr
            180                 185                 190

Lys Phe Thr Pro Asn Trp Leu Ser Val Leu Val Asp Asn Leu Pro Gly
        195                 200                 205

Thr Lys Val Asn Ala Glu Ser Val Glu Arg Ile Lys Arg Gln His Ser
    210                 215                 220

Ser Gln Glu Gln Thr Phe Gln Leu Leu Lys Leu Trp Lys His Gln Asn
225                 230                 235                 240

Lys Asp Gln Asp Ile Val Lys Lys Ile Ile Gln Asp Ile Asp Leu Cys
                245                 250                 255

Glu Asn Ser Val Gln Arg His Ile Gly His Ala Asn Leu Thr Phe Glu
            260                 265                 270

Gln Leu Arg Ser Leu Met Glu Ser Leu Pro Gly Lys Lys Val Gly Ala
        275                 280                 285

Glu Asp Ile Glu Lys Thr Ile Lys Ala Cys Lys Pro Ser Asp Gln Ile
    290                 295                 300

Leu Lys Leu Leu Ser Leu Trp Arg Ile Lys Asn Gly Asp Gln Asp Thr
305                 310                 315                 320

Leu Lys Gly Leu Met His Ala Leu Lys His Ser Lys Thr Tyr His Phe
                325                 330                 335

Pro Lys Thr Val Thr Gln Ser Leu Lys Lys Thr Ile Arg Phe Leu His
            340                 345                 350

Ser Phe Thr Met Tyr Lys Leu Tyr Gln Lys Leu Phe Leu Glu Met Ile

355                 360                 365

Gly Asn Gln Val Gln Ser Val Lys Ile Ser Cys Leu
    370                 375                 380

<210>  17
<211>  123
<212>  DNA
<213>  Homo sapiens

<220>
<221>  CDS
<222>  (1)..(123)

<220>
<223>  Nucleotide sequence of CRD1 of Human OPG

<400>  17
gaa acg ttt cct cca aag tac ctt cat tat gac gaa gaa acc tct cat
48
Glu Thr Phe Pro Pro Lys Tyr Leu His Tyr Asp Glu Glu Thr Ser His
1               5                   10                  15

cag ctg ttg tgt gac aaa tgt cct cct ggt acc tac cta aaa caa cac
96
Gln Leu Leu Cys Asp Lys Cys Pro Pro Gly Thr Tyr Leu Lys Gln His
            20                  25                  30

tgt aca gca aag tgg aag acc gtg tgc
123
Cys Thr Ala Lys Trp Lys Thr Val Cys
        35                  40

<210>  18
<211>  41
<212>  PRT
<213>  Homo sapiens

<220>
<223>  Amino acid sequence of CRD1 of Human OPG

<400>  18

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys
        35                  40

<210>  19
<211>  123

<212>  DNA
<213>  Homo sapiens

<220>
<221>  CDS
<222>  (1)..(123)

<220>
<223>  Nucleotide sequence of CRD2 of Human OPG

<400>  19
tgc cct gac cac tac tac aca gac agc tgg cac acc agt gac gag tgt
48
Cys Pro Asp His Tyr Tyr Thr Asp Ser Trp His Thr Ser Asp Glu Cys
1               5                   10                  15

cta tac tgc agc ccc gtg tgc aag gag ctg cag tac gtc aag cag gag
96
Leu Tyr Cys Ser Pro Val Cys Lys Glu Leu Gln Tyr Val Lys Gln Glu
            20                  25                  30

tgc aat cgc acc cac aac cgc gtg tgc
123
Cys Asn Arg Thr His Asn Arg Val Cys
        35                  40


<210>  20
<211>  41
<212>  PRT
<213>  Homo sapiens

<220>
<223>  Amino acid sequence of CRD2 of Human OPG

<400>  20

Cys Pro Asp His Tyr Tyr Thr Asp Ser Trp His Thr Ser Asp Glu Cys
1               5                   10                  15


Leu Tyr Cys Ser Pro Val Cys Lys Glu Leu Gln Tyr Val Lys Gln Glu
            20                  25                  30


Cys Asn Arg Thr His Asn Arg Val Cys
        35                  40


<210>  21
<211>  108
<212>  DNA
<213>  Homo sapiens

<220>
<221>  CDS
<222>  (1)..(108)

<220>
<223>  Nucleotide sequence of CRD3 of Human OPG

<400>  21
tgc aag gaa ggg cgc tac ctt gag ata gag ttc tgc ttg aaa cat agg
48
Cys Lys Glu Gly Arg Tyr Leu Glu Ile Glu Phe Cys Leu Lys His Arg
1               5                   10                  15

agc tgc cct cct gga ttt gga gtg gtg caa gct gga acc cca gag cga
96
Ser Cys Pro Pro Gly Phe Gly Val Val Gln Ala Gly Thr Pro Glu Arg
            20                  25                  30

aat aca gtt tgc
108
Asn Thr Val Cys
        35



<210>  22
<211>  36
<212>  PRT
<213>  Homo sapiens

<220>
<223>  Amino acid sequence of CRD3 of Human OPG

<400>  22

Cys Lys Glu Gly Arg Tyr Leu Glu Ile Glu Phe Cys Leu Lys His Arg
1               5                   10                  15


Ser Cys Pro Pro Gly Phe Gly Val Val Gln Ala Gly Thr Pro Glu Arg
            20                  25                  30


Asn Thr Val Cys
        35



<210>  23
<211>  123
<212>  DNA
<213>  Homo sapiens

<220>
<221>  CDS
<222>  (1)..(123)

<220>
<223>  Nucleotide sequence of CRD4 of Human OPG

<400>  23
tgt cca gat ggg ttc ttc tca aat gag acg tca tct aaa gca ccc tgt
48
Cys Pro Asp Gly Phe Phe Ser Asn Glu Thr Ser Ser Lys Ala Pro Cys
1               5                   10                  15

aga aaa cac aca aat tgc agt gtc ttt ggt ctc ctg cta act cag aaa
96

Arg Lys His Thr Asn Cys Ser Val Phe Gly Leu Leu Leu Thr Gln Lys
            20                  25                  30

gga aat gca aca cac gac aac ata tgt
123
Gly Asn Ala Thr His Asp Asn Ile Cys
        35                  40

<210>  24
<211>  41
<212>  PRT
<213>  Homo sapiens

<220>
<223>  Amino acid sequence of CRD4 of Human OPG

<400>  24

Cys Pro Asp Gly Phe Phe Ser Asn Glu Thr Ser Ser Lys Ala Pro Cys
1               5                   10                  15

Arg Lys His Thr Asn Cys Ser Val Phe Gly Leu Leu Leu Thr Gln Lys
            20                  25                  30

Gly Asn Ala Thr His Asp Asn Ile Cys
        35                  40

<210>  25
<211>  813
<212>  DNA
<213>  Artificial Sequence

<220>
<221>  CDS
<222>  (1)..(813)

<220>
<223>  Nucleotide sequence of Chimera B-HBD

<400>  25
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct ccc ggc act
384
Gly Thr Pro Ser Gln Asn Thr Gln Cys Gln Pro Cys Pro Pro Gly Thr
        115                 120                 125

ttt tct gcc tct tcc agt tca tca gag caa tgc cag cct cac aga aac
432
Phe Ser Ala Ser Ser Ser Ser Ser Glu Gln Cys Gln Pro His Arg Asn
    130                 135                 140

tgt acc gct ctg ggg ctc gcc ctg aat gtc ccc ggc tct tca agt cac
480
Cys Thr Ala Leu Gly Leu Ala Leu Asn Val Pro Gly Ser Ser Ser His
145                 150                 155                 160

gat acc ctg tgc acc agc tgc act ggc ttc ccc ctc agc acc agg gta
528
Asp Thr Leu Cys Thr Ser Cys Thr Gly Phe Pro Leu Ser Thr Arg Val
                165                 170                 175

cca gga gct gag gag tgt gag cgt gcc gtc atc gac ttt gtg gct ttc
576
Pro Gly Ala Glu Glu Cys Glu Arg Ala Val Ile Asp Phe Val Ala Phe
            180                 185                 190

cag gac atc tcc atc aag agg ctg cag cgg ctg ctg cag gcc ctc gag
624
Gln Asp Ile Ser Ile Lys Arg Leu Gln Arg Leu Leu Gln Ala Leu Glu
        195                 200                 205

gcc ccg gag ggc tgg ggt ccg aca cca agg gcg ggc cgc gcg gcc ttg
672
Ala Pro Glu Gly Trp Gly Pro Thr Pro Arg Ala Gly Arg Ala Ala Leu
    210                 215                 220

cag ctg aag ctg cgt cgg cgg ctc acg gag ctc ctg ggg gcg cag gac
720
Gln Leu Lys Leu Arg Arg Arg Leu Thr Glu Leu Leu Gly Ala Gln Asp
225                 230                 235                 240

ggg gcg ctg ctg gtg cgg ctg ctg cag gcg ctg cgc gtg gcc agg atg
768
Gly Ala Leu Leu Val Arg Leu Leu Gln Ala Leu Arg Val Ala Arg Met
                245                 250                 255

ccc ggg ctg gag cgg agc gtc cgt gag cgc ttc ctc cct gtg cac
813
Pro Gly Leu Glu Arg Ser Val Arg Glu Arg Phe Leu Pro Val His
            260                 265                 270

<210>  26
<211>  271
<212>  PRT
<213>  Artificial Sequence

<220>
<223>  Amino acid sequence of Chimera B-HBD

<400>  26

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser Cys Thr Gly Phe Pro Leu Ser Thr Arg Val
                165                 170                 175

Pro Gly Ala Glu Glu Cys Glu Arg Ala Val Ile Asp Phe Val Ala Phe
            180                 185                 190

Gln Asp Ile Ser Ile Lys Arg Leu Gln Arg Leu Leu Gln Ala Leu Glu
        195                 200                 205

Ala Pro Glu Gly Trp Gly Pro Thr Pro Arg Ala Gly Arg Ala Ala Leu
    210                 215                 220

Gln Leu Lys Leu Arg Arg Arg Leu Thr Glu Leu Leu Gly Ala Gln Asp
225                 230                 235                 240

Gly Ala Leu Leu Val Arg Leu Leu Gln Ala Leu Arg Val Ala Arg Met
                245                 250                 255

Pro Gly Leu Glu Arg Ser Val Arg Glu Arg Phe Leu Pro Val His
            260                 265                 270

<210>  27
<211>  834
<212>  DNA
<213>  Artificial Sequence

<220>
<221>  CDS
<222>  (1)..(834)

<220>
<223>  Nucleotide sequence of Chimera C-HBD

<400>  27
gta gca gag aca ccc acc tat cca tgg agg gac gct gag acc ggt gag
48
Val Ala Glu Thr Pro Thr Tyr Pro Trp Arg Asp Ala Glu Thr Gly Glu
1               5                   10                  15

cgg ctc gtt tgc gcc caa tgt cca cca ggg acc ttc gtt cag cgg ccc
96
Arg Leu Val Cys Ala Gln Cys Pro Pro Gly Thr Phe Val Gln Arg Pro
            20                  25                  30

tgc cga cgg gac agt cca aca aca tgt ggc cca tgc cca cca aga cac
144
Cys Arg Arg Asp Ser Pro Thr Thr Cys Gly Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672
Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr
225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275

<210> 28
<211> 278
<212> PRT
<213> Artificial Sequence

<220>
<223> Amino acid sequence of Chimera C-HBD

<400> 28

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln
        195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  29
<211>  834
<212>  DNA
<213>  Artificial Sequence

<220>
<221>  CDS
<222>  (1)..(834)

<220>
<223>  Nucleotide sequence of Chimera A-HBD

<400>  29
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His

        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672
Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr

225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275

<210>  30
<211>  278
<212>  PRT
<213>  Artificial Sequence

<220>
<223>  Amino acid sequence of Chimera A-HBD

<400>  30

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln
        195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  31
<211>  834
<212>  DNA
<213>  Artificial Sequence

<220>
<221>  CDS
<222>  (1)..(834)

<220>
<223>  Nucleotide sequence of 103-123OPG-HBD

<400>  31
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96

Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt cct cca gga ttt ggg gtg gtc caa gca
336
Cys Leu Glu His Ala Ser Cys Pro Pro Gly Phe Gly Val Val Gln Ala
            100                 105                 110

ggg aca ccc gag cgg aac acc gta tgc aaa cga tgt cct gac ggc ttt
384
Gly Thr Pro Glu Arg Asn Thr Val Cys Lys Arg Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672

Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr
225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275


<210>  32
<211>  278
<212>  PRT
<213>  Artificial Sequence

<220>
<223>  Amino acid sequence of 103-123OPG-HBD

<400>  32

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Glu Arg Cys Arg Tyr Cys Asn Val
    50                  55                  60


Leu Cys Gly Glu Arg Glu Glu Glu Ala Arg Ala Cys His Ala Thr His
65                  70                  75                  80


Asn Arg Ala Cys Arg Cys Arg Thr Gly Phe Phe Ala His Ala Gly Phe
                85                  90                  95


Cys Leu Glu His Ala Ser Cys Pro Pro Gly Phe Gly Val Val Gln Ala
            100                 105                 110

Gly Thr Pro Glu Arg Asn Thr Val Cys Lys Arg Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln
        195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  33
<211>  834
<212>  DNA
<213>  Artificial Sequence

<220>
<221>  CDS
<222>  (1)..(834)

<220>
<223>  Nucleotide sequence of N131S/N144S-HBD

<400>  33

gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct agt gag act tca agt aaa gct ccc tgc cgc aag cac acc agc
432
Phe Ser Ser Glu Thr Ser Ser Lys Ala Pro Cys Arg Lys His Thr Ser
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672
Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr
225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275

<210>  34
<211>  278
<212>  PRT
<213>  Artificial Sequence

<220>
<223>  Amino acid sequence of N131S/N144S-HBD

<400>  34

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Ser Glu Thr Ser Ser Lys Ala Pro Cys Arg Lys His Thr Ser
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln
        195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  35
<211>  834
<212>  DNA
<213>  Artificial Sequence

<220>
<221>  CDS

<222>  (1)..(834)

<220>
<223>  Nucleotide sequence of T133A/S146A-HBD

<400>  35
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag gct tca agt aaa gct ccc tgc cgc aag cac acc aac
432
Phe Ser Asn Glu Ala Ser Ser Lys Ala Pro Cys Arg Lys His Thr Asn
    130                 135                 140

tgt gct gtg ttt ggg ttg ttg ctg acc cag aag ggg aac gca act cac
480
Cys Ala Val Phe Gly Leu Leu Leu Thr Gln Lys Gly Asn Ala Thr His
145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672
Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr
225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275


<210>  36
<211>  278
<212>  PRT
<213>  Artificial Sequence

<220>
<223>  Amino acid sequence of T133A/S146A-HBD

<400>  36

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Glu Arg Cys Arg Tyr Cys Asn Val

50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Ala Ser Ser Lys Ala Pro Cys Arg Lys His Thr Asn
    130                 135                 140

Cys Ala Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln
        195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  37

<211>  834
<212>  DNA
<213>  Artificial Sequence

<220>
<221>  CDS
<222>  (1)..(834)

<220>
<223>  Nucleotide sequence of N131S/N144S/N157S-HBD

<400>  37
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct agt gag act tca agt aaa gct ccc tgc cgc aag cac acc agc
432
Phe Ser Ser Glu Thr Ser Ser Lys Ala Pro Cys Arg Lys His Thr Ser
    130                 135                 140

tgt agt gtg ttt ggg ttg ttg ctg acc cag aag ggg agc gca act cac
480
Cys Ser Val Phe Gly Leu Leu Leu Thr Gln Lys Gly Ser Ala Thr His

145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672
Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr
225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275

<210>  38
<211>  278
<212>  PRT
<213>  Artificial Sequence

<220>
<223>  Amino acid sequence of N131S/N144S/N157S-HBD

<400>  38

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Ser Glu Thr Ser Ser Lys Ala Pro Cys Arg Lys His Thr Ser
    130                 135                 140

Cys Ser Val Phe Gly Leu Leu Leu Thr Gln Lys Gly Ser Ala Thr His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln
        195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  39
<211>  834
<212>  DNA
<213>  Artificial Sequence

<220>
<221>  CDS
<222>  (1)..(834)

<220>
<223>  Nucleotide sequence of T133A/S146A/T159A-HBD

<400>  39
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag gct tca agt aaa gct ccc tgc cgc aag cac acc aac
432
Phe Ser Asn Glu Ala Ser Ser Lys Ala Pro Cys Arg Lys His Thr Asn

130                 135                 140

tgt gct gtg ttt ggg ttg ttg ctg acc cag aag ggg aac gca gct cac
480
Cys Ala Val Phe Gly Leu Leu Leu Thr Gln Lys Gly Asn Ala Ala His
145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672
Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr
225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275

<210>  40
<211>  278
<212>  PRT
<213>  Artificial Sequence

<220>
<223>  Amino acid sequence of T133A/S146A/T159A-HBD

<400>  40

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Ala Ser Ser Lys Ala Pro Cys Arg Lys His Thr Asn
    130                 135                 140

Cys Ala Val Phe Gly Leu Leu Leu Thr Gln Lys Gly Asn Ala Ala His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln
        195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  41
<211>  834
<212>  DNA
<213>  Artificial Sequence

<220>
<221>  CDS
<222>  (1)..(834)

<220>
<223>  Nucleotide sequence of Chimera A-E57K-HBD

<400>  41
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aga tgc cgc tat tgt aac gtt
192
Tyr Thr Gln Phe Trp Asn Tyr Leu Lys Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384

Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672
Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr
225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275


<210>  42
<211>  278
<212>  PRT
<213>  Artificial Sequence

<220>

<223>  Amino acid sequence of Chimera A-E57K-HBD

<400>  42

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln
        195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  43
<211>  834
<212>  DNA
<213>  Artificial Sequence

<220>
<221>  CDS
<222>  (1)..(834)

<220>
<223>  Nucleotide sequence of Chimera A-E57L-HBD

<400>  43
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg aga tgc cgc tat tgt aac gtt
192
Tyr Thr Gln Phe Trp Asn Tyr Leu Leu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672
Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr
225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275

<210>  44
<211>  278
<212>  PRT
<213>  Artificial Sequence

<220>
<223>  Amino acid sequence of Chimera A-E57L-HBD

<400>  44

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln

195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  45
<211>  834
<212>  DNA
<213>  Artificial Sequence

<220>
<221>  CDS
<222>  (1)..(834)

<220>
<223>  Nucleotide sequence of Chimera A-R60K-HBD

<400>  45
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc aag tat tgt aac gtt
192
Tyr Thr Gln Phe Trp Asn Tyr Leu Glu Arg Cys Lys Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag tgc act ggc
528
Asp Asn Ile Cys Ser Gly Asn Ser Glu Ser Thr Gln Lys Cys Thr Gly
                165                 170                 175

ttc ccc ctc agc acc agg gta cca gga gct gag gag tgt gag cgt gcc
576
Phe Pro Leu Ser Thr Arg Val Pro Gly Ala Glu Glu Cys Glu Arg Ala
            180                 185                 190

gtc atc gac ttt gtg gct ttc cag gac atc tcc atc aag agg ctg cag
624
Val Ile Asp Phe Val Ala Phe Gln Asp Ile Ser Ile Lys Arg Leu Gln
        195                 200                 205

cgg ctg ctg cag gcc ctc gag gcc ccg gag ggc tgg ggt ccg aca cca
672
Arg Leu Leu Gln Ala Leu Glu Ala Pro Glu Gly Trp Gly Pro Thr Pro
    210                 215                 220

agg gcg ggc cgc gcg gcc ttg cag ctg aag ctg cgt cgg cgg ctc acg
720
Arg Ala Gly Arg Ala Ala Leu Gln Leu Lys Leu Arg Arg Arg Leu Thr
225                 230                 235                 240

gag ctc ctg ggg gcg cag gac ggg gcg ctg ctg gtg cgg ctg ctg cag
768
Glu Leu Leu Gly Ala Gln Asp Gly Ala Leu Leu Val Arg Leu Leu Gln
                245                 250                 255

gcg ctg cgc gtg gcc agg atg ccc ggg ctg gag cgg agc gtc cgt gag
816
Ala Leu Arg Val Ala Arg Met Pro Gly Leu Glu Arg Ser Val Arg Glu
            260                 265                 270

cgc ttc ctc cct gtg cac
834
Arg Phe Leu Pro Val His
        275

<210>  46
<211>  278
<212>  PRT
<213>  Artificial Sequence

<220>
<223>  Amino acid sequence of Chimera A-R60K-HBD

<400>  46

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Lys Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Cys Thr Gly
                165                 170                 175

Phe Pro Leu Ser Thr Arg Val Pro Gly Ala Glu Glu Cys Glu Arg Ala
            180                 185                 190

Val Ile Asp Phe Val Ala Phe Gln Asp Ile Ser Ile Lys Arg Leu Gln
        195                 200                 205

Arg Leu Leu Gln Ala Leu Glu Ala Pro Glu Gly Trp Gly Pro Thr Pro
    210                 215                 220

Arg Ala Gly Arg Ala Ala Leu Gln Leu Lys Leu Arg Arg Arg Leu Thr
225                 230                 235                 240

Glu Leu Leu Gly Ala Gln Asp Gly Ala Leu Leu Val Arg Leu Leu Gln
                245                 250                 255

Ala Leu Arg Val Ala Arg Met Pro Gly Leu Glu Arg Ser Val Arg Glu
            260                 265                 270

Arg Phe Leu Pro Val His
        275

<210>  47
<211>  315
<212>  DNA
<213>  Homo sapiens

<220>
<221>  CDS
<222>  (1)..(315)

<220>
<223>  Nucleotide sequence of HBD of Wild type DcR3

<400>  47
tgc act ggc ttc ccc ctc agc acc agg gta cca gga gct gag gag tgt
48
Cys Thr Gly Phe Pro Leu Ser Thr Arg Val Pro Gly Ala Glu Glu Cys
1               5                   10                  15

gag cgt gcc gtc atc gac ttt gtg gct ttc cag gac atc tcc atc aag
96
Glu Arg Ala Val Ile Asp Phe Val Ala Phe Gln Asp Ile Ser Ile Lys
            20                  25                  30

agg ctg cag cgg ctg ctg cag gcc ctc gag gcc ccg gag ggc tgg ggt
144
Arg Leu Gln Arg Leu Leu Gln Ala Leu Glu Ala Pro Glu Gly Trp Gly
        35                  40                  45

ccg aca cca agg gcg ggc cgc gcg gcc ttg cag ctg aag ctg cgt cgg
192
Pro Thr Pro Arg Ala Gly Arg Ala Ala Leu Gln Leu Lys Leu Arg Arg

50                  55                  60

cgg ctc acg gag ctc ctg ggg gcg cag gac ggg gcg ctg ctg gtg cgg
240
Arg Leu Thr Glu Leu Leu Gly Ala Gln Asp Gly Ala Leu Leu Val Arg
65                  70                  75                  80

ctg ctg cag gcg ctg cgc gtg gcc agg atg ccc ggg ctg gag cgg agc
288
Leu Leu Gln Ala Leu Arg Val Ala Arg Met Pro Gly Leu Glu Arg Ser
                85                  90                  95

gtc cgt gag cgc ttc ctc cct gtg cac
315
Val Arg Glu Arg Phe Leu Pro Val His
            100                 105

<210>  48
<211>  105
<212>  PRT
<213>  Homo sapiens

<220>
<223>  Amino acid sequence of HBD of Wild type DcR3

<400>  48

Cys Thr Gly Phe Pro Leu Ser Thr Arg Val Pro Gly Ala Glu Glu Cys
1               5                   10                  15

Glu Arg Ala Val Ile Asp Phe Val Ala Phe Gln Asp Ile Ser Ile Lys
            20                  25                  30

Arg Leu Gln Arg Leu Leu Gln Ala Leu Glu Ala Pro Glu Gly Trp Gly
        35                  40                  45

Pro Thr Pro Arg Ala Gly Arg Ala Ala Leu Gln Leu Lys Leu Arg Arg
    50                  55                  60

Arg Leu Thr Glu Leu Leu Gly Ala Gln Asp Gly Ala Leu Leu Val Arg
65                  70                  75                  80

Leu Leu Gln Ala Leu Arg Val Ala Arg Met Pro Gly Leu Glu Arg Ser
                85                  90                  95

Val Arg Glu Arg Phe Leu Pro Val His
            100                 105

<210>  49
<211>  498
<212>  DNA
<213>  Artificial Sequence

<220>
<221>  CDS
<222>  (1)..(498)

<220>
<223>  Nucleotide sequence of Chimera B

<400>  49
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct ccc ggc act
384
Gly Thr Pro Ser Gln Asn Thr Gln Cys Gln Pro Cys Pro Pro Gly Thr
        115                 120                 125

ttt tct gcc tct tcc agt tca tca gag caa tgc cag cct cac aga aac
432
Phe Ser Ala Ser Ser Ser Ser Ser Glu Gln Cys Gln Pro His Arg Asn
    130                 135                 140

tgt acc gct ctg ggg ctc gcc ctg aat gtc ccc ggc tct tca agt cac
480
Cys Thr Ala Leu Gly Leu Ala Leu Asn Val Pro Gly Ser Ser Ser His
145                 150                 155                 160

gat acc ctg tgc acc agc
498
Asp Thr Leu Cys Thr Ser
                165

<210>  50
<211>  166
<212>  PRT
<213>  Artificial Sequence

<220>
<223>  Amino acid sequence of Chimera B

<400>  50

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser
                165

<210> 51
<211> 519
<212> DNA
<213> Artificial Sequence

<220>
<221> CDS
<222> (1)..(519)

<220>
<223> Nucleotide sequence of Chimera C

<400> 51
gta gca gag aca ccc acc tat cca tgg agg gac gct gag acc ggt gag
48
Val Ala Glu Thr Pro Thr Tyr Pro Trp Arg Asp Ala Glu Thr Gly Glu
1               5                   10                  15

cgg ctc gtt tgc gcc caa tgt cca cca ggg acc ttc gtt cag cgg ccc
96
Arg Leu Val Cys Ala Gln Cys Pro Pro Gly Thr Phe Val Gln Arg Pro
            20                  25                  30

tgc cga cgg gac agt cca aca aca tgt ggc cca tgc cca cca aga cac
144
Cys Arg Arg Asp Ser Pro Thr Thr Cys Gly Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480

Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210>  52
<211>  173
<212>  PRT
<213>  Artificial Sequence

<220>
<223>  Amino acid sequence of Chimera C

<400>  52

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys

165                 170

<210>  53
<211>  519
<212>  DNA
<213>  Artificial Sequence

<220>
<221>  CDS
<222>  (1)..(519)

<220>
<223>  Nucleotide sequence of Chimera A

<400>  53
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210>  54
<211>  173
<212>  PRT
<213>  Artificial Sequence

<220>
<223>  Amino acid sequence of Chimera A

<400>  54

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  55
<211>  519
<212>  DNA
<213>  Artificial Sequence

<220>
<221>  CDS
<222>  (1)..(519)

<220>
<223>  Nucleotide sequence of 103-123OPG

<400>  55
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt cct cca gga ttt ggg gtg gtc caa gca
336
Cys Leu Glu His Ala Ser Cys Pro Pro Gly Phe Gly Val Val Gln Ala
            100                 105                 110

ggg aca ccc gag cgg aac acc gta tgc aaa cga tgt cct gac ggc ttt
384
Gly Thr Pro Glu Arg Asn Thr Val Cys Lys Arg Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  56
<211>  173
<212>  PRT
<213>  Artificial Sequence

<220>
<223>  Amino acid sequence of 103-123OPG

<400>  56

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Glu Arg Cys Arg Tyr Cys Asn Val
    50                  55                  60


Leu Cys Gly Glu Arg Glu Glu Glu Ala Arg Ala Cys His Ala Thr His
65                  70                  75                  80


Asn Arg Ala Cys Arg Cys Arg Thr Gly Phe Phe Ala His Ala Gly Phe
                85                  90                  95


Cys Leu Glu His Ala Ser Cys Pro Pro Gly Phe Gly Val Val Gln Ala
            100                 105                 110


Gly Thr Pro Glu Arg Asn Thr Val Cys Lys Arg Cys Pro Asp Gly Phe
        115                 120                 125


Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  57
<211>  519
<212>  DNA
<213>  Artificial Sequence

<220>
<221>  CDS
<222>  (1)..(519)

<220>
<223>  Nucleotide sequence of N131S/N144S

<400>  57
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct agt gag act tca agt aaa gct ccc tgc cgc aag cac acc agc
432
Phe Ser Ser Glu Thr Ser Ser Lys Ala Pro Cys Arg Lys His Thr Ser
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  58
<211>  173
<212>  PRT
<213>  Artificial Sequence

<220>
<223>  Amino acid sequence of N131S/N144S

<400>  58

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Glu Arg Cys Arg Tyr Cys Asn Val
    50                  55                  60


Leu Cys Gly Glu Arg Glu Glu Glu Ala Arg Ala Cys His Ala Thr His
65                  70                  75                  80


Asn Arg Ala Cys Arg Cys Arg Thr Gly Phe Phe Ala His Ala Gly Phe
                85                  90                  95


Cys Leu Glu His Ala Ser Cys Pro Pro Gly Ala Gly Val Ile Ala Pro
            100                 105                 110


Gly Thr Pro Ser Gln Asn Thr Gln Cys Gln Pro Cys Pro Asp Gly Phe

115                 120                 125

Phe Ser Ser Glu Thr Ser Ser Lys Ala Pro Cys Arg Lys His Thr Ser
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  59
<211>  519
<212>  DNA
<213>  Artificial Sequence

<220>
<221>  CDS
<222>  (1)..(519)

<220>
<223>  Nucleotide sequence of T133A/S146A

<400>  59
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336

Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag gct tca agt aaa gct ccc tgc cgc aag cac acc aac
432
Phe Ser Asn Glu Ala Ser Ser Lys Ala Pro Cys Arg Lys His Thr Asn
    130                 135                 140

tgt gct gtg ttt ggg ttg ttg ctg acc cag aag ggg aac gca act cac
480
Cys Ala Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210> 60
<211> 173
<212> PRT
<213> Artificial Sequence

<220>
<223> Amino acid sequence of T133A/S146A

<400> 60

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Ala Ser Ser Lys Ala Pro Cys Arg Lys His Thr Asn
    130                 135                 140

Cys Ala Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  61
<211>  519
<212>  DNA
<213>  Artificial Sequence

<220>
<221>  CDS
<222>  (1)..(519)

<220>
<223>  Nucleotide sequence of N131S/N144S/N157S

<400>  61
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct agt gag act tca agt aaa gct ccc tgc cgc aag cac acc agc
432
Phe Ser Ser Glu Thr Ser Ser Lys Ala Pro Cys Arg Lys His Thr Ser
    130                 135                 140

tgt agt gtg ttt ggg ttg ttg ctg acc cag aag ggg agc gca act cac
480
Cys Ser Val Phe Gly Leu Leu Leu Thr Gln Lys Gly Ser Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210> 62
<211> 173
<212> PRT
<213> Artificial Sequence

<220>
<223> Amino acid sequence of N131S/N144S/N157S

<400> 62

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Ser Glu Thr Ser Ser Lys Ala Pro Cys Arg Lys His Thr Ser
    130                 135                 140

Cys Ser Val Phe Gly Leu Leu Leu Thr Gln Lys Gly Ser Ala Thr His
145                 150                 155                 160

Asp Asn Ile Cys Ser Gly Asn Ser Glu Ser Thr Gln Lys
                165                 170

<210>  63
<211>  519
<212>  DNA
<213>  Artificial Sequence

<220>
<221>  CDS
<222>  (1)..(519)

<220>
<223>  Nucleotide sequence of T133A/S146A/T159A

<400>  63
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag gct tca agt aaa gct ccc tgc cgc aag cac acc aac
432
Phe Ser Asn Glu Ala Ser Ser Lys Ala Pro Cys Arg Lys His Thr Asn
    130                 135                 140

tgt gct gtg ttt ggg ttg ttg ctg acc cag aag ggg aac gca gct cac
480
Cys Ala Val Phe Gly Leu Leu Leu Thr Gln Lys Gly Asn Ala Ala His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  64
<211>  173
<212>  PRT
<213>  Artificial Sequence

<220>
<223>  Amino acid sequence of T133A/S146A/T159A

<400>  64

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Glu Arg Cys Arg Tyr Cys Asn Val
    50                  55                  60


Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Ala Ser Ser Lys Ala Pro Cys Arg Lys His Thr Asn
    130                 135                 140

Cys Ala Val Phe Gly Leu Leu Leu Thr Gln Lys Gly Asn Ala Ala His
145                 150                 155                 160

Asp Asn Ile Cys Ser Gly Asn Ser Glu Ser Thr Gln Lys
                165                 170

<210>  65
<211>  519
<212>  DNA
<213>  Artificial Sequence

<220>
<221>  CDS
<222>  (1)..(519)

<220>
<223>  Nucleotide sequence of Chimera A-E57K

<400>  65
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aga tgc cgc tat tgt aac gtt
192
Tyr Thr Gln Phe Trp Asn Tyr Leu Lys Arg Cys Arg Tyr Cys Asn Val
    50                  55                  60

ctg tgt ggt gag cgt gag gaa gaa gct cga gca tgc cat gct act cac
240

Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  66
<211>  173
<212>  PRT
<213>  Artificial Sequence

<220>
<223>  Amino acid sequence of Chimera A-E57K

<400>  66

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  67
<211>  519
<212>  DNA
<213>  Artificial Sequence

<220>
<221>  CDS
<222>  (1)..(519)

<220>
<223>  Nucleotide sequence of Chimera A-E57L

<400>  67
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg aga tgc cgc tat tgt aac gtt
192
Tyr Thr Gln Phe Trp Asn Tyr Leu Leu Arg Cys Arg Tyr Cys Asn Val

50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  68
<211>  173
<212>  PRT
<213>  Artificial Sequence

<220>
<223>  Amino acid sequence of Chimera A-E57L

<400>  68

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  69
<211>  519
<212>  DNA
<213>  Artificial Sequence

<220>
<221>  CDS
<222>  (1)..(519)

<220>
<223>  Nucleotide sequence of Chimera A-R60K

<400>  69
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc aag tat tgt aac gtt
192
Tyr Thr Gln Phe Trp Asn Tyr Leu Glu Arg Cys Lys Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  70
<211>  173
<212>  PRT
<213>  Artificial Sequence

<220>
<223>  Amino acid sequence of Chimera A-R60K

<400>  70

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His

35                  40                  45

Tyr Thr Gln Phe Trp Asn Tyr Leu Glu Arg Cys Lys Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  71
<211>  693
<212>  DNA
<213>  Artificial Sequence

<220>
<221>  CDS
<222>  (1)..(693)

<220>
<223>  Nucleotide sequence of Fc(g1S)

<400>  71
ccc aaa tct tcc gac aaa act cac aca tgc cca ccg tgc cca gca cct
48
Pro Lys Ser Ser Asp Lys Thr His Thr Cys Pro Pro Cys Pro Ala Pro
1               5                   10                  15

gaa ctc ctg ggg gga ccg tca gtc ttc ctc ttc ccc cca aaa ccc aag
96
Glu Leu Leu Gly Gly Pro Ser Val Phe Leu Phe Pro Pro Lys Pro Lys
            20                  25                  30

gac acc ctc atg atc tcc cgg acc cct gag gtc aca tgc gtg gtg gtg
144

Asp Thr Leu Met Ile Ser Arg Thr Pro Glu Val Thr Cys Val Val Val
        35                  40                  45

gac gtg agc cac gaa gac cct gag gtc aag ttc aac tgg tac gtg gac
192
Asp Val Ser His Glu Asp Pro Glu Val Lys Phe Asn Trp Tyr Val Asp
    50                  55                  60

ggc gtg gag gtg cat aat gcc aag aca aag ccg cgg gag gag cag tac
240
Gly Val Glu Val His Asn Ala Lys Thr Lys Pro Arg Glu Glu Gln Tyr
65                  70                  75                  80

aac agc acg tac cgt gtg gtc agc gtc ctc acc gtc ctg cac cag gac
288
Asn Ser Thr Tyr Arg Val Val Ser Val Leu Thr Val Leu His Gln Asp
                85                  90                  95

tgg ctg aat ggc aag gag tac aag tgc aag gtc tcc aac aaa gcc ctc
336
Trp Leu Asn Gly Lys Glu Tyr Lys Cys Lys Val Ser Asn Lys Ala Leu
            100                 105                 110

cca gcc ccc atc gag aaa acc atc tcc aaa gcc aaa ggg cag ccc cga
384
Pro Ala Pro Ile Glu Lys Thr Ile Ser Lys Ala Lys Gly Gln Pro Arg
        115                 120                 125

gaa cca cag gtg tac acc ctg ccc cca tcc cgg gat gag ctg acc aag
432
Glu Pro Gln Val Tyr Thr Leu Pro Pro Ser Arg Asp Glu Leu Thr Lys
    130                 135                 140

aac cag gtc agc ctg acc tgc ctg gtc aaa ggc ttc tat ccc agc gac
480
Asn Gln Val Ser Leu Thr Cys Leu Val Lys Gly Phe Tyr Pro Ser Asp
145                 150                 155                 160

atc gcc gtg gag tgg gag agc aat ggg cag ccg gag aac aac tac aag
528
Ile Ala Val Glu Trp Glu Ser Asn Gly Gln Pro Glu Asn Asn Tyr Lys
                165                 170                 175

acc acg cct ccc gtg ctg gac tcc gac ggc tcc ttc ttc ctc tac agc
576
Thr Thr Pro Pro Val Leu Asp Ser Asp Gly Ser Phe Phe Leu Tyr Ser
            180                 185                 190

aag ctc acc gtg gac aag agc agg tgg cag cag ggg aac gtc ttc tca
624
Lys Leu Thr Val Asp Lys Ser Arg Trp Gln Gln Gly Asn Val Phe Ser
        195                 200                 205

tgc tcc gtg atg cat gag gct ctg cac aac cac tac acg cag aag agc
672
Cys Ser Val Met His Glu Ala Leu His Asn His Tyr Thr Gln Lys Ser
    210                 215                 220

ctc tcc ctg tct ccg ggt aaa
693

Leu Ser Leu Ser Pro Gly Lys
225                 230

<210>  72
<211>  231
<212>  PRT
<213>  Artificial Sequence

<220>
<223>  Amino acid sequence of Fc(g1S)

<400>  72

Pro Lys Ser Ser Asp Lys Thr His Thr Cys Pro Pro Cys Pro Ala Pro
1               5                   10                  15

Glu Leu Leu Gly Gly Pro Ser Val Phe Leu Phe Pro Pro Lys Pro Lys
            20                  25                  30

Asp Thr Leu Met Ile Ser Arg Thr Pro Glu Val Thr Cys Val Val Val
        35                  40                  45

Asp Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Asn Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Ala Leu
            100                 105                 110

Pro Ala Pro Ile Glu Lys Thr Ile Ser Lys Ala Lys Gly Gln Pro Arg
        115                 120                 125

Glu Pro Gln Val Tyr Thr Leu Pro Pro Ser Arg Asp Glu Leu Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Thr Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Asn His Tyr Thr Gln Lys Ser
    210                 215                 220

Leu Ser Leu Ser Pro Gly Lys
225                 230

<210>  73
<211>  687
<212>  DNA
<213>  Artificial Sequence

<220>
<221>  CDS
<222>  (1)..(687)

<220>
<223>  Nucleotide sequence of Fc(g4PEK)

<400>  73
gag tcc aaa tat ggt ccc cca tgc cca cca tgc cca gca cct gag ttc
48
Glu Ser Lys Tyr Gly Pro Pro Cys Pro Pro Cys Pro Ala Pro Glu Phe
1               5                   10                  15

gag ggg gga cca tca gtc ttc ctg ttc ccc cca aaa ccc aag gac act
96
Glu Gly Gly Pro Ser Val Phe Leu Phe Pro Pro Lys Pro Lys Asp Thr
            20                  25                  30

ctc atg atc tcc cgg acc cct gag gtc acg tgc gtg gtg gtg gac gtg
144
Leu Met Ile Ser Arg Thr Pro Glu Val Thr Cys Val Val Val Asp Val
        35                  40                  45

agc cag gaa gac ccc gag gtc cag ttc aac tgg tac gtg gat ggc gtg
192
Ser Gln Glu Asp Pro Glu Val Gln Phe Asn Trp Tyr Val Asp Gly Val
    50                  55                  60

gag gtg cat aat gcc aag aca aag ccg cgg gag gag cag ttc aac agc
240
Glu Val His Asn Ala Lys Thr Lys Pro Arg Glu Glu Gln Phe Asn Ser
65                  70                  75                  80

acg tac cgt gtg gtc agc gtc ctc acc gtc ctg cac cag gac tgg ctg
288
Thr Tyr Arg Val Val Ser Val Leu Thr Val Leu His Gln Asp Trp Leu
                85                  90                  95

aac ggc aag gag tac aag tgc aag gtc tcc aac aaa ggc ctc ccg tcc
336
Asn Gly Lys Glu Tyr Lys Cys Lys Val Ser Asn Lys Gly Leu Pro Ser

100                 105                 110

tcc atc gag aaa acc atc tcc aaa gcc aaa ggg cag ccc cga gag cca
384
Ser Ile Glu Lys Thr Ile Ser Lys Ala Lys Gly Gln Pro Arg Glu Pro
        115                 120                 125

cag gtg tac acc ctg ccc cca tcc cag gag gag atg acc aag aac cag
432
Gln Val Tyr Thr Leu Pro Pro Ser Gln Glu Glu Met Thr Lys Asn Gln
    130                 135                 140

gtc agc ctg acc tgc ctg gtc aaa ggc ttc tac ccc agc gac atc gcc
480
Val Ser Leu Thr Cys Leu Val Lys Gly Phe Tyr Pro Ser Asp Ile Ala
145                 150                 155                 160

gtg gag tgg gag agc aat ggg cag ccg gag aac aac tac aag acc acg
528
Val Glu Trp Glu Ser Asn Gly Gln Pro Glu Asn Asn Tyr Lys Thr Thr
                165                 170                 175

cct ccc gtg ctg gac tcc gac ggc tcc ttc ttc ctc tac agc aag cta
576
Pro Pro Val Leu Asp Ser Asp Gly Ser Phe Phe Leu Tyr Ser Lys Leu
            180                 185                 190

acc gtg gac aag agc agg tgg cag gag ggg aat gtc ttc tca tgc tcc
624
Thr Val Asp Lys Ser Arg Trp Gln Glu Gly Asn Val Phe Ser Cys Ser
        195                 200                 205

gtg atg cat gag gct ctg cac aac cac tac aca cag aag agc ctc tcc
672
Val Met His Glu Ala Leu His Asn His Tyr Thr Gln Lys Ser Leu Ser
    210                 215                 220

ctg tct ctg ggt aaa
687
Leu Ser Leu Gly Lys
225


<210>  74
<211>  229
<212>  PRT
<213>  Artificial Sequence

<220>
<223>  Amino acid sequence of Fc(g4PEK)

<400>  74

Glu Ser Lys Tyr Gly Pro Pro Cys Pro Pro Cys Pro Ala Pro Glu Phe
1               5                   10                  15


Glu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Lys Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210>  75
<211>  1209
<212>  DNA
<213>  Artificial Sequence

<220>
<221>  CDS

<222>  (1)..(1209)

<220>
<223>  Nucleotide sequence of Chimera B-Fc(IEGRMD-g1S)

<400>  75
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct ccc ggc act
384
Gly Thr Pro Ser Gln Asn Thr Gln Cys Gln Pro Cys Pro Pro Gly Thr
        115                 120                 125

ttt tct gcc tct tcc agt tca tca gag caa tgc cag cct cac aga aac
432
Phe Ser Ala Ser Ser Ser Ser Ser Glu Gln Cys Gln Pro His Arg Asn
    130                 135                 140

tgt acc gct ctg ggg ctc gcc ctg aat gtc ccc ggc tct tca agt cac
480
Cys Thr Ala Leu Gly Leu Ala Leu Asn Val Pro Gly Ser Ser Ser His
145                 150                 155                 160

gat acc ctg tgc acc agc atc gag ggg cgc atg gat ccc aaa tct tcc
528
Asp Thr Leu Cys Thr Ser Ile Glu Gly Arg Met Asp Pro Lys Ser Ser
                165                 170                 175

gac aaa act cac aca tgc cca ccg tgc cca gca cct gaa ctc ctg ggg
576
Asp Lys Thr His Thr Cys Pro Pro Cys Pro Ala Pro Glu Leu Leu Gly
            180                 185                 190

gga ccg tca gtc ttc ctc ttc ccc cca aaa ccc aag gac acc ctc atg
624
Gly Pro Ser Val Phe Leu Phe Pro Pro Lys Pro Lys Asp Thr Leu Met
        195                 200                 205

atc tcc cgg acc cct gag gtc aca tgc gtg gtg gtg gac gtg agc cac
672
Ile Ser Arg Thr Pro Glu Val Thr Cys Val Val Val Asp Val Ser His
    210                 215                 220

gaa gac cct gag gtc aag ttc aac tgg tac gtg gac ggc gtg gag gtg
720
Glu Asp Pro Glu Val Lys Phe Asn Trp Tyr Val Asp Gly Val Glu Val
225                 230                 235                 240

cat aat gcc aag aca aag ccg cgg gag gag cag tac aac agc acg tac
768
His Asn Ala Lys Thr Lys Pro Arg Glu Glu Gln Tyr Asn Ser Thr Tyr
            245                 250                 255

cgt gtg gtc agc gtc ctc acc gtc ctg cac cag gac tgg ctg aat ggc
816
Arg Val Val Ser Val Leu Thr Val Leu His Gln Asp Trp Leu Asn Gly
            260                 265                 270

aag gag tac aag tgc aag gtc tcc aac aaa gcc ctc cca gcc ccc atc
864
Lys Glu Tyr Lys Cys Lys Val Ser Asn Lys Ala Leu Pro Ala Pro Ile
        275                 280                 285

gag aaa acc atc tcc aaa gcc aaa ggg cag ccc cga gaa cca cag gtg
912
Glu Lys Thr Ile Ser Lys Ala Lys Gly Gln Pro Arg Glu Pro Gln Val
    290                 295                 300

tac acc ctg ccc cca tcc cgg gat gag ctg acc aag aac cag gtc agc
960
Tyr Thr Leu Pro Pro Ser Arg Asp Glu Leu Thr Lys Asn Gln Val Ser
305                 310                 315                 320

ctg acc tgc ctg gtc aaa ggc ttc tat ccc agc gac atc gcc gtg gag
1008
Leu Thr Cys Leu Val Lys Gly Phe Tyr Pro Ser Asp Ile Ala Val Glu
            325                 330                 335

tgg gag agc aat ggg cag ccg gag aac aac tac aag acc acg cct ccc
1056
Trp Glu Ser Asn Gly Gln Pro Glu Asn Asn Tyr Lys Thr Thr Pro Pro
            340                 345                 350

gtg ctg gac tcc gac ggc tcc ttc ttc ctc tac agc aag ctc acc gtg
1104
Val Leu Asp Ser Asp Gly Ser Phe Phe Leu Tyr Ser Lys Leu Thr Val
        355                 360                 365

gac aag agc agg tgg cag cag ggg aac gtc ttc tca tgc tcc gtg atg
1152
Asp Lys Ser Arg Trp Gln Gln Gly Asn Val Phe Ser Cys Ser Val Met
    370                 375                 380

cat gag gct ctg cac aac cac tac acg cag aag agc ctc tcc ctg tct
1200
His Glu Ala Leu His Asn His Tyr Thr Gln Lys Ser Leu Ser Leu Ser
385                 390                 395                 400

ccg ggt aaa
1209
Pro Gly Lys

<210>  76
<211>  403
<212>  PRT
<213>  Artificial Sequence

<220>
<223>  Amino acid sequence of Chimera B-Fc(IEGRMD-g1S)

<400>  76

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn

130                 135                 140

Cys Thr Ala Leu Gly Leu Ala Leu Asn Val Pro Gly Ser Ser Ser His
145                 150                 155                 160

Asp Thr Leu Cys Thr Ser Ile Glu Gly Arg Met Asp Pro Lys Ser Ser
                165                 170                 175

Asp Lys Thr His Thr Cys Pro Pro Cys Pro Ala Pro Glu Leu Leu Gly
            180                 185                 190

Gly Pro Ser Val Phe Leu Phe Pro Pro Lys Pro Lys Asp Thr Leu Met
        195                 200                 205

Ile Ser Arg Thr Pro Glu Val Thr Cys Val Val Val Asp Val Ser His
    210                 215                 220

Glu Asp Pro Glu Val Lys Phe Asn Trp Tyr Val Asp Gly Val Glu Val
225                 230                 235                 240

His Asn Ala Lys Thr Lys Pro Arg Glu Glu Gln Tyr Asn Ser Thr Tyr
                245                 250                 255

Arg Val Val Ser Val Leu Thr Val Leu His Gln Asp Trp Leu Asn Gly
            260                 265                 270

Lys Glu Tyr Lys Cys Lys Val Ser Asn Lys Ala Leu Pro Ala Pro Ile
        275                 280                 285

Glu Lys Thr Ile Ser Lys Ala Lys Gly Gln Pro Arg Glu Pro Gln Val
    290                 295                 300

Tyr Thr Leu Pro Pro Ser Arg Asp Glu Leu Thr Lys Asn Gln Val Ser
305                 310                 315                 320

Leu Thr Cys Leu Val Lys Gly Phe Tyr Pro Ser Asp Ile Ala Val Glu
                325                 330                 335

Trp Glu Ser Asn Gly Gln Pro Glu Asn Asn Tyr Lys Thr Thr Pro Pro
            340                 345                 350

Val Leu Asp Ser Asp Gly Ser Phe Phe Leu Tyr Ser Lys Leu Thr Val
        355                 360                 365

Asp Lys Ser Arg Trp Gln Gln Gly Asn Val Phe Ser Cys Ser Val Met

370                 375                 380

His Glu Ala Leu His Asn His Tyr Thr Gln Lys Ser Leu Ser Leu Ser
385                 390                 395                 400

Pro Gly Lys

<210>  77
<211>  1230
<212>  DNA
<213>  Artificial Sequence

<220>
<221>  CDS
<222>  (1)..(1230)

<220>
<223>  Nucleotide sequence of Chimera C-Fc(IEGRMD-g1S)

<400>  77
gta gca gag aca ccc acc tat cca tgg agg gac gct gag acc ggt gag
48
Val Ala Glu Thr Pro Thr Tyr Pro Trp Arg Asp Ala Glu Thr Gly Glu
1               5                   10                  15

cgg ctc gtt tgc gcc caa tgt cca cca ggg acc ttc gtt cag cgg ccc
96
Arg Leu Val Cys Ala Gln Cys Pro Pro Gly Thr Phe Val Gln Arg Pro
            20                  25                  30

tgc cga cgg gac agt cca aca aca tgt ggc cca tgc cca cca aga cac
144
Cys Arg Arg Asp Ser Pro Thr Thr Cys Gly Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag atc gag ggg
528
Asp Asn Ile Cys Ser Gly Asn Ser Glu Ser Thr Gln Lys Ile Glu Gly
                165                 170                 175

cgc atg gat ccc aaa tct tcc gac aaa act cac aca tgc cca ccg tgc
576
Arg Met Asp Pro Lys Ser Ser Asp Lys Thr His Thr Cys Pro Pro Cys
            180                 185                 190

cca gca cct gaa ctc ctg ggg gga ccg tca gtc ttc ctc ttc ccc cca
624
Pro Ala Pro Glu Leu Leu Gly Gly Pro Ser Val Phe Leu Phe Pro Pro
        195                 200                 205

aaa ccc aag gac acc ctc atg atc tcc cgg acc cct gag gtc aca tgc
672
Lys Pro Lys Asp Thr Leu Met Ile Ser Arg Thr Pro Glu Val Thr Cys
    210                 215                 220

gtg gtg gtg gac gtg agc cac gaa gac cct gag gtc aag ttc aac tgg
720
Val Val Val Asp Val Ser His Glu Asp Pro Glu Val Lys Phe Asn Trp
225                 230                 235                 240

tac gtg gac ggc gtg gag gtg cat aat gcc aag aca aag ccg cgg gag
768
Tyr Val Asp Gly Val Glu Val His Asn Ala Lys Thr Lys Pro Arg Glu
                245                 250                 255

gag cag tac aac agc acg tac cgt gtg gtc agc gtc ctc acc gtc ctg
816
Glu Gln Tyr Asn Ser Thr Tyr Arg Val Val Ser Val Leu Thr Val Leu
            260                 265                 270

cac cag gac tgg ctg aat ggc aag gag tac aag tgc aag gtc tcc aac
864
His Gln Asp Trp Leu Asn Gly Lys Glu Tyr Lys Cys Lys Val Ser Asn
        275                 280                 285

aaa gcc ctc cca gcc ccc atc gag aaa acc atc tcc aaa gcc aaa ggg
912
Lys Ala Leu Pro Ala Pro Ile Glu Lys Thr Ile Ser Lys Ala Lys Gly
    290                 295                 300

cag ccc cga gaa cca cag gtg tac acc ctg ccc cca tcc cgg gat gag
960
Gln Pro Arg Glu Pro Gln Val Tyr Thr Leu Pro Pro Ser Arg Asp Glu
305                 310                 315                 320

ctg acc aag aac cag gtc agc ctg acc tgc ctg gtc aaa ggc ttc tat
1008
Leu Thr Lys Asn Gln Val Ser Leu Thr Cys Leu Val Lys Gly Phe Tyr
                325                 330                 335

ccc agc gac atc gcc gtg gag tgg gag agc aat ggg cag ccg gag aac
1056
Pro Ser Asp Ile Ala Val Glu Trp Glu Ser Asn Gly Gln Pro Glu Asn
            340                 345                 350

aac tac aag acc acg cct ccc gtg ctg gac tcc gac ggc tcc ttc ttc
1104
Asn Tyr Lys Thr Thr Pro Pro Val Leu Asp Ser Asp Gly Ser Phe Phe
        355                 360                 365

ctc tac agc aag ctc acc gtg gac aag agc agg tgg cag cag ggg aac
1152
Leu Tyr Ser Lys Leu Thr Val Asp Lys Ser Arg Trp Gln Gln Gly Asn
    370                 375                 380

gtc ttc tca tgc tcc gtg atg cat gag gct ctg cac aac cac tac acg
1200
Val Phe Ser Cys Ser Val Met His Glu Ala Leu His Asn His Tyr Thr
385                 390                 395                 400

cag aag agc ctc tcc ctg tct ccg ggt aaa
1230
Gln Lys Ser Leu Ser Leu Ser Pro Gly Lys
                405                 410


<210>  78
<211>  410
<212>  PRT
<213>  Artificial Sequence

<220>
<223>  Amino acid sequence of Chimera C-Fc(IEGRMD-g1S)

<400>  78

Val Ala Glu Thr Pro Thr Tyr Pro Trp Arg Asp Ala Glu Thr Gly Glu
1               5                   10                  15


Arg Leu Val Cys Ala Gln Cys Pro Pro Gly Thr Phe Val Gln Arg Pro
            20                  25                  30


Cys Arg Arg Asp Ser Pro Thr Thr Cys Gly Pro Cys Pro Pro Arg His
        35                  40                  45


Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Ile Glu Gly
                165                 170                 175

Arg Met Asp Pro Lys Ser Ser Asp Lys Thr His Thr Cys Pro Pro Cys
            180                 185                 190

Pro Ala Pro Glu Leu Leu Gly Gly Pro Ser Val Phe Leu Phe Pro Pro
        195                 200                 205

Lys Pro Lys Asp Thr Leu Met Ile Ser Arg Thr Pro Glu Val Thr Cys
    210                 215                 220

Val Val Val Asp Val Ser His Glu Asp Pro Glu Val Lys Phe Asn Trp
225                 230                 235                 240

Tyr Val Asp Gly Val Glu Val His Asn Ala Lys Thr Lys Pro Arg Glu
                245                 250                 255

Glu Gln Tyr Asn Ser Thr Tyr Arg Val Val Ser Val Leu Thr Val Leu
            260                 265                 270

His Gln Asp Trp Leu Asn Gly Lys Glu Tyr Lys Cys Lys Val Ser Asn
        275                 280                 285

Lys Ala Leu Pro Ala Pro Ile Glu Lys Thr Ile Ser Lys Ala Lys Gly
    290                 295                 300

Gln Pro Arg Glu Pro Gln Val Tyr Thr Leu Pro Pro Ser Arg Asp Glu
305                 310                 315                 320

Leu Thr Lys Asn Gln Val Ser Leu Thr Cys Leu Val Lys Gly Phe Tyr
                325                 330                 335

Pro Ser Asp Ile Ala Val Glu Trp Glu Ser Asn Gly Gln Pro Glu Asn
            340                 345                 350

Asn Tyr Lys Thr Thr Pro Pro Val Leu Asp Ser Asp Gly Ser Phe Phe
        355                 360                 365

Leu Tyr Ser Lys Leu Thr Val Asp Lys Ser Arg Trp Gln Gln Gly Asn
    370                 375                 380

Val Phe Ser Cys Ser Val Met His Glu Ala Leu His Asn His Tyr Thr
385                 390                 395                 400

Gln Lys Ser Leu Ser Leu Ser Pro Gly Lys
                405                 410

<210>  79
<211>  1230
<212>  DNA
<213>  Artificial Sequence

<220>
<221>  CDS
<222>  (1)..(1230)

<220>
<223>  Nucleotide sequence of Chimera A-Fc(IEGRMD-g1S)

<400>  79
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192

Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag atc gag ggg
528
Asp Asn Ile Cys Ser Gly Asn Ser Glu Ser Thr Gln Lys Ile Glu Gly
                165                 170                 175

cgc atg gat ccc aaa tct tcc gac aaa act cac aca tgc cca ccg tgc
576
Arg Met Asp Pro Lys Ser Ser Asp Lys Thr His Thr Cys Pro Pro Cys
            180                 185                 190

cca gca cct gaa ctc ctg ggg gga ccg tca gtc ttc ctc ttc ccc cca
624
Pro Ala Pro Glu Leu Leu Gly Gly Pro Ser Val Phe Leu Phe Pro Pro
        195                 200                 205

aaa ccc aag gac acc ctc atg atc tcc cgg acc cct gag gtc aca tgc
672
Lys Pro Lys Asp Thr Leu Met Ile Ser Arg Thr Pro Glu Val Thr Cys
    210                 215                 220

gtg gtg gtg gac gtg agc cac gaa gac cct gag gtc aag ttc aac tgg
720
Val Val Val Asp Val Ser His Glu Asp Pro Glu Val Lys Phe Asn Trp
225                 230                 235                 240

tac gtg gac ggc gtg gag gtg cat aat gcc aag aca aag ccg cgg gag
768

Tyr Val Asp Gly Val Glu Val His Asn Ala Lys Thr Lys Pro Arg Glu
                245                 250                 255

gag cag tac aac agc acg tac cgt gtg gtc agc gtc ctc acc gtc ctg
816
Glu Gln Tyr Asn Ser Thr Tyr Arg Val Val Ser Val Leu Thr Val Leu
            260                 265                 270

cac cag gac tgg ctg aat ggc aag gag tac aag tgc aag gtc tcc aac
864
His Gln Asp Trp Leu Asn Gly Lys Glu Tyr Lys Cys Lys Val Ser Asn
        275                 280                 285

aaa gcc ctc cca gcc ccc atc gag aaa acc atc tcc aaa gcc aaa ggg
912
Lys Ala Leu Pro Ala Pro Ile Glu Lys Thr Ile Ser Lys Ala Lys Gly
    290                 295                 300

cag ccc cga gaa cca cag gtg tac acc ctg ccc cca tcc cgg gat gag
960
Gln Pro Arg Glu Pro Gln Val Tyr Thr Leu Pro Pro Ser Arg Asp Glu
305                 310                 315                 320

ctg acc aag aac cag gtc agc ctg acc tgc ctg gtc aaa ggc ttc tat
1008
Leu Thr Lys Asn Gln Val Ser Leu Thr Cys Leu Val Lys Gly Phe Tyr
                325                 330                 335

ccc agc gac atc gcc gtg gag tgg gag agc aat ggg cag ccg gag aac
1056
Pro Ser Asp Ile Ala Val Glu Trp Glu Ser Asn Gly Gln Pro Glu Asn
            340                 345                 350

aac tac aag acc acg cct ccc gtg ctg gac tcc gac ggc tcc ttc ttc
1104
Asn Tyr Lys Thr Thr Pro Pro Val Leu Asp Ser Asp Gly Ser Phe Phe
        355                 360                 365

ctc tac agc aag ctc acc gtg gac aag agc agg tgg cag cag ggg aac
1152
Leu Tyr Ser Lys Leu Thr Val Asp Lys Ser Arg Trp Gln Gln Gly Asn
    370                 375                 380

gtc ttc tca tgc tcc gtg atg cat gag gct ctg cac aac cac tac acg
1200
Val Phe Ser Cys Ser Val Met His Glu Ala Leu His Asn His Tyr Thr
385                 390                 395                 400

cag aag agc ctc tcc ctg tct ccg ggt aaa
1230
Gln Lys Ser Leu Ser Leu Ser Pro Gly Lys
                405                 410


<210>  80
<211>  410
<212>  PRT
<213>  Artificial Sequence

<220>

<223>  Amino acid sequence of Chimera A-Fc(IEGRMD-g1S)

<400>  80

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Ile Glu Gly
                165                 170                 175

Arg Met Asp Pro Lys Ser Ser Asp Lys Thr His Thr Cys Pro Pro Cys
            180                 185                 190

Pro Ala Pro Glu Leu Leu Gly Gly Pro Ser Val Phe Leu Phe Pro Pro
        195                 200                 205

Lys Pro Lys Asp Thr Leu Met Ile Ser Arg Thr Pro Glu Val Thr Cys
    210                 215                 220

Val Val Val Asp Val Ser His Glu Asp Pro Glu Val Lys Phe Asn Trp
225                 230                 235                 240

Tyr Val Asp Gly Val Glu Val His Asn Ala Lys Thr Lys Pro Arg Glu
                245                 250                 255

Glu Gln Tyr Asn Ser Thr Tyr Arg Val Val Ser Val Leu Thr Val Leu
            260                 265                 270

His Gln Asp Trp Leu Asn Gly Lys Glu Tyr Lys Cys Lys Val Ser Asn
        275                 280                 285

Lys Ala Leu Pro Ala Pro Ile Glu Lys Thr Ile Ser Lys Ala Lys Gly
    290                 295                 300

Gln Pro Arg Glu Pro Gln Val Tyr Thr Leu Pro Pro Ser Arg Asp Glu
305                 310                 315                 320

Leu Thr Lys Asn Gln Val Ser Leu Thr Cys Leu Val Lys Gly Phe Tyr
                325                 330                 335

Pro Ser Asp Ile Ala Val Glu Trp Glu Ser Asn Gly Gln Pro Glu Asn
            340                 345                 350

Asn Tyr Lys Thr Thr Pro Pro Val Leu Asp Ser Asp Gly Ser Phe Phe
        355                 360                 365

Leu Tyr Ser Lys Leu Thr Val Asp Lys Ser Arg Trp Gln Gln Gly Asn
    370                 375                 380

Val Phe Ser Cys Ser Val Met His Glu Ala Leu His Asn His Tyr Thr
385                 390                 395                 400

Gln Lys Ser Leu Ser Leu Ser Pro Gly Lys
                405                 410

<210>  81
<211>  1206
<212>  DNA
<213>  Artificial Sequence

<220>
<221>  CDS
<222>  (1)..(1206)

<220>
<223>  Nucleotide sequence of Chimera A-Fc(g4PEK)

<400> 81
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  82
<211>  402
<212>  PRT
<213>  Artificial Sequence

<220>
<223>  Amino acid sequence of Chimera A-Fc(g4PEK)

<400>  82

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210> 83
<211> 1206
<212> DNA
<213> Artificial Sequence

<220>
<221> CDS
<222> (1)..(1206)

<220>
<223> Nucleotide sequence of 103-123OPG-Fc(g4PEK)

<400> 83
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt cct cca gga ttt ggg gtg gtc caa gca
336
Cys Leu Glu His Ala Ser Cys Pro Pro Gly Phe Gly Val Val Gln Ala
            100                 105                 110

ggg aca ccc gag cgg aac acc gta tgc aaa cga tgt cct gac ggc ttt
384
Gly Thr Pro Glu Arg Asn Thr Val Cys Lys Arg Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  84
<211>  402
<212>  PRT
<213>  Artificial Sequence

<220>
<223>  Amino acid sequence of 103-123OPG-Fc(g4PEK)

<400>  84

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Phe Gly Val Val Gln Ala
            100                 105                 110

Gly Thr Pro Glu Arg Asn Thr Val Cys Lys Arg Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  85
<211>  1206
<212>  DNA
<213>  Artificial Sequence

<220>
<221>  CDS
<222>  (1)..(1206)

<220>
<223>  Nucleotide sequence of N131S/N144S-Fc(g4PEK)

<400>  85
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240

Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct agt gag act tca agt aaa gct ccc tgc cgc aag cac acc agc
432
Phe Ser Ser Glu Thr Ser Ser Lys Ala Pro Cys Arg Lys His Thr Ser
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816

Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  86
<211>  402
<212>  PRT
<213>  Artificial Sequence

<220>
<223>  Amino acid sequence of N131S/N144S-Fc(g4PEK)

<400>  86

Glu Thr Phe Pro Pro Lys Tyr Leu His Tyr Asp Glu Glu Thr Ser His

1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Ser Glu Thr Ser Ser Lys Ala Pro Cys Arg Lys His Thr Ser
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg

                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210> 87
<211> 1206
<212> DNA
<213> Artificial Sequence

<220>
<221> CDS
<222> (1)..(1206)

<220>
<223> Nucleotide sequence of T133A/S146A-Fc(g4PEK)

<400> 87
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag gct tca agt aaa gct ccc tgc cgc aag cac acc aac
432
Phe Ser Asn Glu Ala Ser Ser Lys Ala Pro Cys Arg Lys His Thr Asn
    130                 135                 140

tgt gct gtg ttt ggg ttg ttg ctg acc cag aag ggg aac gca act cac
480
Cys Ala Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210> 88
<211> 402
<212> PRT
<213> Artificial Sequence

<220>
<223> Amino acid sequence of T133A/S146A-Fc(g4PEK)

<400> 88

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Ala Ser Ser Lys Ala Pro Cys Arg Lys His Thr Asn
    130                 135                 140

Cys Ala Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  89
<211>  1206
<212>  DNA
<213>  Artificial Sequence

<220>
<221>  CDS
<222>  (1)..(1206)

<220>
<223>  Nucleotide sequence of N131S/N144S/N157S-Fc(g4PEK)

<400>  89
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct agt gag act tca agt aaa gct ccc tgc cgc aag cac acc agc
432
Phe Ser Ser Glu Thr Ser Ser Lys Ala Pro Cys Arg Lys His Thr Ser
    130                 135                 140

tgt agt gtg ttt ggg ttg ttg ctg acc cag aag ggg agc gca act cac
480
Cys Ser Val Phe Gly Leu Leu Leu Thr Gln Lys Gly Ser Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  90
<211>  402
<212>  PRT
<213>  Artificial Sequence

<220>
<223>  Amino acid sequence of N131S/N144S/N157S-Fc(g4PEK)

<400>  90

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
                100                 105                 110

Gly Thr Pro Ser Gln Asn Thr Gln Cys Gln Pro Cys Pro Asp Gly Phe
        115                 120                 125

Phe Ser Ser Glu Thr Ser Ser Lys Ala Pro Cys Arg Lys His Thr Ser
    130                 135                 140

Cys Ser Val Phe Gly Leu Leu Leu Thr Gln Lys Gly Ser Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
                180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
                260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  91
<211>  1206
<212>  DNA
<213>  Artificial Sequence

<220>
<221>  CDS
<222>  (1)..(1206)

<220>
<223>  Nucleotide sequence of T133A/S146A/T159A-Fc(g4PEK)

<400>  91
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288

Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag gct tca agt aaa gct ccc tgc cgc aag cac acc aac
432
Phe Ser Asn Glu Ala Ser Ser Lys Ala Pro Cys Arg Lys His Thr Asn
    130                 135                 140

tgt gct gtg ttt ggg ttg ttg ctg acc cag aag ggg aac gca gct cac
480
Cys Ala Val Phe Gly Leu Leu Leu Thr Gln Lys Gly Asn Ala Ala His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864

Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  92
<211>  402
<212>  PRT
<213>  Artificial Sequence

<220>
<223>  Amino acid sequence of T133A/S146A/T159A-Fc(g4PEK)

<400>  92

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Ala Ser Ser Lys Ala Pro Cys Arg Lys His Thr Asn
    130                 135                 140

Cys Ala Val Phe Gly Leu Leu Leu Thr Gln Lys Gly Asn Ala Ala His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  93
<211>  1206
<212>  DNA
<213>  Artificial Sequence

<220>
<221>  CDS
<222>  (1)..(1206)

<220>
<223>  Nucleotide sequence of Chimera A-E57K-Fc(g4PEK)

<400>  93
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aga tgc cgc tat tgt aac gtt
192
Tyr Thr Gln Phe Trp Asn Tyr Leu Lys Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  94
<211>  402
<212>  PRT
<213>  Artificial Sequence

<220>
<223>  Amino acid sequence of Chimera A-E57K-Fc(g4PEK)

<400>  94

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  95
<211>  1206
<212>  DNA
<213>  Artificial Sequence

<220>
<221>  CDS
<222>  (1)..(1206)

<220>
<223>  Nucleotide sequence of Chimera A-E57L-Fc(g4PEK)

<400>  95
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg aga tgc cgc tat tgt aac gtt
192
Tyr Thr Gln Phe Trp Asn Tyr Leu Leu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  96
<211>  402
<212>  PRT
<213>  Artificial Sequence

<220>
<223>  Amino acid sequence of Chimera A-E57L-Fc(g4PEK)

<400>  96

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe

115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp

355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  97
<211>  1206
<212>  DNA
<213>  Artificial Sequence

<220>
<221>  CDS
<222>  (1)..(1206)

<220>
<223>  Nucleotide sequence of Chimera A-R60K-Fc(g4PEK)

<400>  97
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc aag tat tgt aac gtt
192
Tyr Thr Gln Phe Trp Asn Tyr Leu Glu Arg Cys Lys Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336

Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912

Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys



<210>  98
<211>  402
<212>  PRT
<213>  Artificial Sequence

<220>
<223>  Amino acid sequence of Chimera A-R60K-Fc(g4PEK)

<400>  98

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Lys Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  99
<211>  1524
<212>  DNA
<213>  Artificial Sequence

<220>
<221>  CDS
<222>  (1)..(1524)

<220>
<223>  Nucleotide sequence of DcR3 FL-Fc

<400>  99
gtg gca gaa aca ccc acc tac ccc tgg cgg gac gca gag aca ggg gag
48
Val Ala Glu Thr Pro Thr Tyr Pro Trp Arg Asp Ala Glu Thr Gly Glu
1               5                   10                  15

cgg ctg gtg tgc gcc cag tgc ccc cca ggc acc ttt gtg cag cgg ccg
96
Arg Leu Val Cys Ala Gln Cys Pro Pro Gly Thr Phe Val Gln Arg Pro
            20                  25                  30

tgc cgc cga gac agc ccc acg acg tgt ggc ccg tgt cca ccg cgc cac
144
Cys Arg Arg Asp Ser Pro Thr Thr Cys Gly Pro Cys Pro Pro Arg His
        35                  40                  45

tac acg cag ttc tgg aac tac cta gag cgc tgc cgc tac tgc aac gtc
192
Tyr Thr Gln Phe Trp Asn Tyr Leu Glu Arg Cys Arg Tyr Cys Asn Val
    50                  55                  60

ctc tgc ggg gag cgt gag gag gag gca cgg gct tgc cac gcc acc cac
240
Leu Cys Gly Glu Arg Glu Glu Glu Ala Arg Ala Cys His Ala Thr His
65                  70                  75                  80

aac cgt gcc tgc cgc tgc cgc acc ggc ttc ttc gcg cac gct ggt ttc
288
Asn Arg Ala Cys Arg Cys Arg Thr Gly Phe Phe Ala His Ala Gly Phe
                85                  90                  95

tgc ttg gag cac gca tcg tgt cca cct ggt gcc ggc gtg att gcc ccg
336
Cys Leu Glu His Ala Ser Cys Pro Pro Gly Ala Gly Val Ile Ala Pro
            100                 105                 110

ggc acc ccc agc cag aac acg cag tgc cag ccg tgc ccc cca ggc acc
384
Gly Thr Pro Ser Gln Asn Thr Gln Cys Gln Pro Cys Pro Pro Gly Thr
        115                 120                 125

ttc tca gcc agc agc tcc agc tca gag cag tgc cag ccc cac cgc aac
432
Phe Ser Ala Ser Ser Ser Ser Ser Glu Gln Cys Gln Pro His Arg Asn
    130                 135                 140

tgc acg gcc ctg ggc ctg gcc ctc aat gtg cca ggc tct tcc tcc cat
480
Cys Thr Ala Leu Gly Leu Ala Leu Asn Val Pro Gly Ser Ser Ser His
145                 150                 155                 160

gac acc ctg tgc acc agc tgc act ggc ttc ccc ctc agc acc agg gta
528
Asp Thr Leu Cys Thr Ser Cys Thr Gly Phe Pro Leu Ser Thr Arg Val
                165                 170                 175

cca gga gct gag gag tgt gag cgt gcc gtc atc gac ttt gtg gct ttc
576
Pro Gly Ala Glu Glu Cys Glu Arg Ala Val Ile Asp Phe Val Ala Phe
            180                 185                 190

cag gac atc tcc atc aag agg ctg cag cgg ctg ctg cag gcc ctc gag
624
Gln Asp Ile Ser Ile Lys Arg Leu Gln Arg Leu Leu Gln Ala Leu Glu
        195                 200                 205

gcc ccg gag ggc tgg ggt ccg aca cca agg gcg ggc cgc gcg gcc ttg
672
Ala Pro Glu Gly Trp Gly Pro Thr Pro Arg Ala Gly Arg Ala Ala Leu
    210                 215                 220

cag ctg aag ctg cgt cgg cgg ctc acg gag ctc ctg ggg gcg cag gac
720
Gln Leu Lys Leu Arg Arg Arg Leu Thr Glu Leu Leu Gly Ala Gln Asp
225                 230                 235                 240

ggg gcg ctg ctg gtg cgg ctg ctg cag gcg ctg cgc gtg gcc agg atg
768
Gly Ala Leu Leu Val Arg Leu Leu Gln Ala Leu Arg Val Ala Arg Met
                245                 250                 255

ccc ggg ctg gag cgg agc gtc cgt gag cgc ttc ctc cct gtg cac atc
816
Pro Gly Leu Glu Arg Ser Val Arg Glu Arg Phe Leu Pro Val His Ile
            260                 265                 270

gag ggg cgc atg gat ccc aaa tct tcc gac aaa act cac aca tgc cca
864
Glu Gly Arg Met Asp Pro Lys Ser Ser Asp Lys Thr His Thr Cys Pro
        275                 280                 285

ccg tgc cca gca cct gaa ctc ctg ggg gga ccg tca gtc ttc ctc ttc
912
Pro Cys Pro Ala Pro Glu Leu Leu Gly Gly Pro Ser Val Phe Leu Phe
    290                 295                 300

ccc cca aaa ccc aag gac acc ctc atg atc tcc cgg acc cct gag gtc
960
Pro Pro Lys Pro Lys Asp Thr Leu Met Ile Ser Arg Thr Pro Glu Val
305                 310                 315                 320

aca tgc gtg gtg gtg gac gtg agc cac gaa gac cct gag gtc aag ttc
1008
Thr Cys Val Val Val Asp Val Ser His Glu Asp Pro Glu Val Lys Phe
                325                 330                 335

aac tgg tac gtg gac ggc gtg gag gtg cat aat gcc aag aca aag ccg
1056
Asn Trp Tyr Val Asp Gly Val Glu Val His Asn Ala Lys Thr Lys Pro
            340                 345                 350

cgg gag gag cag tac aac agc acg tac cgt gtg gtc agc gtc ctc acc
1104
Arg Glu Glu Gln Tyr Asn Ser Thr Tyr Arg Val Val Ser Val Leu Thr
        355                 360                 365

gtc ctg cac cag gac tgg ctg aat ggc aag gag tac aag tgc aag gtc
1152
Val Leu His Gln Asp Trp Leu Asn Gly Lys Glu Tyr Lys Cys Lys Val
    370                 375                 380

tcc aac aaa gcc ctc cca gcc ccc atc gag aaa acc atc tcc aaa gcc
1200
Ser Asn Lys Ala Leu Pro Ala Pro Ile Glu Lys Thr Ile Ser Lys Ala
385                 390                 395                 400

aaa ggg cag ccc cga gaa cca cag gtg tac acc ctg ccc cca tcc cgg
1248
Lys Gly Gln Pro Arg Glu Pro Gln Val Tyr Thr Leu Pro Pro Ser Arg
                405                 410                 415

gat gag ctg acc aag aac cag gtc agc ctg acc tgc ctg gtc aaa ggc
1296
Asp Glu Leu Thr Lys Asn Gln Val Ser Leu Thr Cys Leu Val Lys Gly
            420                 425                 430

ttc tat ccc agc gac atc gcc gtg gag tgg gag agc aat ggg cag ccg
1344
Phe Tyr Pro Ser Asp Ile Ala Val Glu Trp Glu Ser Asn Gly Gln Pro
        435                 440                 445

gag aac aac tac aag acc acg cct ccc gtg ctg gac tcc gac ggc tcc
1392
Glu Asn Asn Tyr Lys Thr Thr Pro Pro Val Leu Asp Ser Asp Gly Ser
    450                 455                 460

ttc ttc ctc tac agc aag ctc acc gtg gac aag agc agg tgg cag cag
1440
Phe Phe Leu Tyr Ser Lys Leu Thr Val Asp Lys Ser Arg Trp Gln Gln
465                 470                 475                 480

ggg aac gtc ttc tca tgc tcc gtg atg cat gag gct ctg cac aac cac
1488
Gly Asn Val Phe Ser Cys Ser Val Met His Glu Ala Leu His Asn His
                485                 490                 495

tac acg cag aag agc ctc tcc ctg tct ccg ggt aaa
1524
Tyr Thr Gln Lys Ser Leu Ser Leu Ser Pro Gly Lys
            500                 505

<210> 100
<211> 508
<212> PRT
<213> Artificial Sequence

<220>
<223> Amino acid sequence of DcR3 FL-Fc

<400> 100

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser Cys Thr Gly Phe Pro Leu Ser Thr Arg Val
                165                 170                 175

Pro Gly Ala Glu Glu Cys Glu Arg Ala Val Ile Asp Phe Val Ala Phe
            180                 185                 190

Gln Asp Ile Ser Ile Lys Arg Leu Gln Arg Leu Leu Gln Ala Leu Glu
        195                 200                 205

Ala Pro Glu Gly Trp Gly Pro Thr Pro Arg Ala Gly Arg Ala Ala Leu
    210                 215                 220

Gln Leu Lys Leu Arg Arg Arg Leu Thr Glu Leu Leu Gly Ala Gln Asp
225                 230                 235                 240

Gly Ala Leu Leu Val Arg Leu Leu Gln Ala Leu Arg Val Ala Arg Met
                245                 250                 255

Pro Gly Leu Glu Arg Ser Val Arg Glu Arg Phe Leu Pro Val His Ile
            260                 265                 270

Glu Gly Arg Met Asp Pro Lys Ser Ser Asp Lys Thr His Thr Cys Pro
        275                 280                 285

Pro Cys Pro Ala Pro Glu Leu Leu Gly Gly Pro Ser Val Phe Leu Phe
    290                 295                 300

Pro Pro Lys Pro Lys Asp Thr Leu Met Ile Ser Arg Thr Pro Glu Val
305                 310                 315                 320

Thr Cys Val Val Val Asp Val Ser His Glu Asp Pro Glu Val Lys Phe
                325                 330                 335

Asn Trp Tyr Val Asp Gly Val Glu Val His Asn Ala Lys Thr Lys Pro
            340                 345                 350

Arg Glu Glu Gln Tyr Asn Ser Thr Tyr Arg Val Val Ser Val Leu Thr
        355                 360                 365

Val Leu His Gln Asp Trp Leu Asn Gly Lys Glu Tyr Lys Cys Lys Val
    370                 375                 380

Ser Asn Lys Ala Leu Pro Ala Pro Ile Glu Lys Thr Ile Ser Lys Ala
385                 390                 395                 400

Lys Gly Gln Pro Arg Glu Pro Gln Val Tyr Thr Leu Pro Pro Ser Arg
                405                 410                 415

Asp Glu Leu Thr Lys Asn Gln Val Ser Leu Thr Cys Leu Val Lys Gly
            420                 425                 430

Phe Tyr Pro Ser Asp Ile Ala Val Glu Trp Glu Ser Asn Gly Gln Pro
        435                 440                 445

Glu Asn Asn Tyr Lys Thr Thr Pro Pro Val Leu Asp Ser Asp Gly Ser
    450                 455                 460

Phe Phe Leu Tyr Ser Lys Leu Thr Val Asp Lys Ser Arg Trp Gln Gln
465                 470                 475                 480

Gly Asn Val Phe Ser Cys Ser Val Met His Glu Ala Leu His Asn His
                485                 490                 495

Tyr Thr Gln Lys Ser Leu Ser Leu Ser Pro Gly Lys
            500                 505

<210>  101
<211>  1209
<212>  DNA
<213>  Artificial Sequence

<220>
<221>  CDS
<222>  (1)..(1209)

<220>
<223>  Nucleotide sequence of S195-Fc

<400>  101
gtg gca gaa aca ccc acc tac ccc tgg cgg gac gca gag aca ggg gag
48

Val Ala Glu Thr Pro Thr Tyr Pro Trp Arg Asp Ala Glu Thr Gly Glu
1               5                   10                  15

cgg ctg gtg tgc gcc cag tgc ccc cca ggc acc ttt gtg cag cgg ccg
96
Arg Leu Val Cys Ala Gln Cys Pro Pro Gly Thr Phe Val Gln Arg Pro
            20                  25                  30

tgc cgc cga gac agc ccc acg acg tgt ggc ccg tgt cca ccg cgc cac
144
Cys Arg Arg Asp Ser Pro Thr Thr Cys Gly Pro Cys Pro Pro Arg His
        35                  40                  45

tac acg cag ttc tgg aac tac cta gag cgc tgc cgc tac tgc aac gtc
192
Tyr Thr Gln Phe Trp Asn Tyr Leu Glu Arg Cys Arg Tyr Cys Asn Val
    50                  55                  60

ctc tgc ggg gag cgt gag gag gag gca cgg gct tgc cac gcc acc cac
240
Leu Cys Gly Glu Arg Glu Glu Glu Ala Arg Ala Cys His Ala Thr His
65                  70                  75                  80

aac cgt gcc tgc cgc tgc cgc acc ggc ttc ttc gcg cac gct ggt ttc
288
Asn Arg Ala Cys Arg Cys Arg Thr Gly Phe Phe Ala His Ala Gly Phe
                85                  90                  95

tgc ttg gag cac gca tcg tgt cca cct ggt gcc ggc gtg att gcc ccg
336
Cys Leu Glu His Ala Ser Cys Pro Pro Gly Ala Gly Val Ile Ala Pro
            100                 105                 110

ggc acc ccc agc cag aac acg cag tgc cag ccg tgc ccc cca ggc acc
384
Gly Thr Pro Ser Gln Asn Thr Gln Cys Gln Pro Cys Pro Pro Gly Thr
        115                 120                 125

ttc tca gcc agc agc tcc agc tca gag cag tgc cag ccc cac cgc aac
432
Phe Ser Ala Ser Ser Ser Ser Ser Glu Gln Cys Gln Pro His Arg Asn
    130                 135                 140

tgc acg gcc ctg ggc ctg gcc ctc aat gtg cca ggc tct tcc tcc cat
480
Cys Thr Ala Leu Gly Leu Ala Leu Asn Val Pro Gly Ser Ser Ser His
145                 150                 155                 160

gac acc ctg tgc acc agc atc gag ggg cgc atg gat ccc aaa tct tcc
528
Asp Thr Leu Cys Thr Ser Ile Glu Gly Arg Met Asp Pro Lys Ser Ser
                165                 170                 175

gac aaa act cac aca tgc cca ccg tgc cca gca cct gaa ctc ctg ggg
576
Asp Lys Thr His Thr Cys Pro Pro Cys Pro Ala Pro Glu Leu Leu Gly
            180                 185                 190

gga ccg tca gtc ttc ctc ttc ccc cca aaa ccc aag gac acc ctc atg
624

Gly Pro Ser Val Phe Leu Phe Pro Pro Lys Pro Lys Asp Thr Leu Met
        195                 200                 205

atc tcc cgg acc cct gag gtc aca tgc gtg gtg gtg gac gtg agc cac
672
Ile Ser Arg Thr Pro Glu Val Thr Cys Val Val Val Asp Val Ser His
    210                 215                 220

gaa gac cct gag gtc aag ttc aac tgg tac gtg gac ggc gtg gag gtg
720
Glu Asp Pro Glu Val Lys Phe Asn Trp Tyr Val Asp Gly Val Glu Val
225                 230                 235                 240

cat aat gcc aag aca aag ccg cgg gag gag cag tac aac agc acg tac
768
His Asn Ala Lys Thr Lys Pro Arg Glu Glu Gln Tyr Asn Ser Thr Tyr
                245                 250                 255

cgt gtg gtc agc gtc ctc acc gtc ctg cac cag gac tgg ctg aat ggc
816
Arg Val Val Ser Val Leu Thr Val Leu His Gln Asp Trp Leu Asn Gly
            260                 265                 270

aag gag tac aag tgc aag gtc tcc aac aaa gcc ctc cca gcc ccc atc
864
Lys Glu Tyr Lys Cys Lys Val Ser Asn Lys Ala Leu Pro Ala Pro Ile
        275                 280                 285

gag aaa acc atc tcc aaa gcc aaa ggg cag ccc cga gaa cca cag gtg
912
Glu Lys Thr Ile Ser Lys Ala Lys Gly Gln Pro Arg Glu Pro Gln Val
    290                 295                 300

tac acc ctg ccc cca tcc cgg gat gag ctg acc aag aac cag gtc agc
960
Tyr Thr Leu Pro Pro Ser Arg Asp Glu Leu Thr Lys Asn Gln Val Ser
305                 310                 315                 320

ctg acc tgc ctg gtc aaa ggc ttc tat ccc agc gac atc gcc gtg gag
1008
Leu Thr Cys Leu Val Lys Gly Phe Tyr Pro Ser Asp Ile Ala Val Glu
                325                 330                 335

tgg gag agc aat ggg cag ccg gag aac aac tac aag acc acg cct ccc
1056
Trp Glu Ser Asn Gly Gln Pro Glu Asn Asn Tyr Lys Thr Thr Pro Pro
            340                 345                 350

gtg ctg gac tcc gac ggc tcc ttc ttc ctc tac agc aag ctc acc gtg
1104
Val Leu Asp Ser Asp Gly Ser Phe Phe Leu Tyr Ser Lys Leu Thr Val
        355                 360                 365

gac aag agc agg tgg cag cag ggg aac gtc ttc tca tgc tcc gtg atg
1152
Asp Lys Ser Arg Trp Gln Gln Gly Asn Val Phe Ser Cys Ser Val Met
    370                 375                 380

cat gag gct ctg cac aac cac tac acg cag aag agc ctc tcc ctg tct
1200

His Glu Ala Leu His Asn His Tyr Thr Gln Lys Ser Leu Ser Leu Ser
385                 390                 395                 400

ccg ggt aaa
1209
Pro Gly Lys

<210>  102
<211>  403
<212>  PRT
<213>  Artificial Sequence

<220>
<223>  Amino acid sequence of S195-Fc

<400>  102

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser Ile Glu Gly Arg Met Asp Pro Lys Ser Ser

                165                 170                 175

Asp Lys Thr His Thr Cys Pro Pro Cys Pro Ala Pro Glu Leu Leu Gly
            180                 185                 190

Gly Pro Ser Val Phe Leu Phe Pro Pro Lys Pro Lys Asp Thr Leu Met
        195                 200                 205

Ile Ser Arg Thr Pro Glu Val Thr Cys Val Val Val Asp Val Ser His
    210                 215                 220

Glu Asp Pro Glu Val Lys Phe Asn Trp Tyr Val Asp Gly Val Glu Val
225                 230                 235                 240

His Asn Ala Lys Thr Lys Pro Arg Glu Glu Gln Tyr Asn Ser Thr Tyr
                245                 250                 255

Arg Val Val Ser Val Leu Thr Val Leu His Gln Asp Trp Leu Asn Gly
            260                 265                 270

Lys Glu Tyr Lys Cys Lys Val Ser Asn Lys Ala Leu Pro Ala Pro Ile
        275                 280                 285

Glu Lys Thr Ile Ser Lys Ala Lys Gly Gln Pro Arg Glu Pro Gln Val
    290                 295                 300

Tyr Thr Leu Pro Pro Ser Arg Asp Glu Leu Thr Lys Asn Gln Val Ser
305                 310                 315                 320

Leu Thr Cys Leu Val Lys Gly Phe Tyr Pro Ser Asp Ile Ala Val Glu
                325                 330                 335

Trp Glu Ser Asn Gly Gln Pro Glu Asn Asn Tyr Lys Thr Thr Pro Pro
            340                 345                 350

Val Leu Asp Ser Asp Gly Ser Phe Phe Leu Tyr Ser Lys Leu Thr Val
        355                 360                 365

Asp Lys Ser Arg Trp Gln Gln Gly Asn Val Phe Ser Cys Ser Val Met
    370                 375                 380

His Glu Ala Leu His Asn His Tyr Thr Gln Lys Ser Leu Ser Leu Ser
385                 390                 395                 400

Pro Gly Lys

<210> 103
<211> 846
<212> DNA
<213> Artificial Sequence

<220>
<221> CDS
<222> (1)..(846)

<220>
<223> Nucleotide sequence of DcR3 FL-FLAG

<400> 103
gtg gca gaa aca ccc acc tac ccc tgg cgg gac gca gag aca ggg gag
48
Val Ala Glu Thr Pro Thr Tyr Pro Trp Arg Asp Ala Glu Thr Gly Glu
1               5                   10                  15

cgg ctg gtg tgc gcc cag tgc ccc cca ggc acc ttt gtg cag cgg ccg
96
Arg Leu Val Cys Ala Gln Cys Pro Pro Gly Thr Phe Val Gln Arg Pro
            20                  25                  30

tgc cgc cga gac agc ccc acg acg tgt ggc ccg tgt cca ccg cgc cac
144
Cys Arg Arg Asp Ser Pro Thr Thr Cys Gly Pro Cys Pro Pro Arg His
        35                  40                  45

tac acg cag ttc tgg aac tac cta gag cgc tgc cgc tac tgc aac gtc
192
Tyr Thr Gln Phe Trp Asn Tyr Leu Glu Arg Cys Arg Tyr Cys Asn Val
    50                  55                  60

ctc tgc ggg gag cgt gag gag gag gca cgg gct tgc cac gcc acc cac
240
Leu Cys Gly Glu Arg Glu Glu Glu Ala Arg Ala Cys His Ala Thr His
65                  70                  75                  80

aac cgt gcc tgc cgc tgc cgc acc ggc ttc ttc gcg cac gct ggt ttc
288
Asn Arg Ala Cys Arg Cys Arg Thr Gly Phe Phe Ala His Ala Gly Phe
                85                  90                  95

tgc ttg gag cac gca tcg tgt cca cct ggt gcc ggc gtg att gcc ccg
336
Cys Leu Glu His Ala Ser Cys Pro Pro Gly Ala Gly Val Ile Ala Pro
            100                 105                 110

ggc acc ccc agc cag aac acg cag tgc cag ccg tgc ccc cca ggc acc
384
Gly Thr Pro Ser Gln Asn Thr Gln Cys Gln Pro Cys Pro Pro Gly Thr
        115                 120                 125

ttc tca gcc agc agc tcc agc tca gag cag tgc cag ccc cac cgc aac
432
Phe Ser Ala Ser Ser Ser Ser Ser Glu Gln Cys Gln Pro His Arg Asn
    130                 135                 140

tgc acg gcc ctg ggc ctg gcc ctc aat gtg cca ggc tct tcc tcc cat
480
Cys Thr Ala Leu Gly Leu Ala Leu Asn Val Pro Gly Ser Ser Ser His
145                 150                 155                 160

gac acc ctg tgc acc agc tgc act ggc ttc ccc ctc agc acc agg gta
528
Asp Thr Leu Cys Thr Ser Cys Thr Gly Phe Pro Leu Ser Thr Arg Val
                165                 170                 175

cca gga gct gag gag tgt gag cgt gcc gtc atc gac ttt gtg gct ttc
576
Pro Gly Ala Glu Glu Cys Glu Arg Ala Val Ile Asp Phe Val Ala Phe
            180                 185                 190

cag gac atc tcc atc aag agg ctg cag cgg ctg ctg cag gcc ctc gag
624
Gln Asp Ile Ser Ile Lys Arg Leu Gln Arg Leu Leu Gln Ala Leu Glu
        195                 200                 205

gcc ccg gag ggc tgg ggt ccg aca cca agg gcg ggc cgc gcg gcc ttg
672
Ala Pro Glu Gly Trp Gly Pro Thr Pro Arg Ala Gly Arg Ala Ala Leu
    210                 215                 220

cag ctg aag ctg cgt cgg cgg ctc acg gag ctc ctg ggg gcg cag gac
720
Gln Leu Lys Leu Arg Arg Arg Leu Thr Glu Leu Leu Gly Ala Gln Asp
225                 230                 235                 240

ggg gcg ctg ctg gtg cgg ctg ctg cag gcg ctg cgc gtg gcc agg atg
768
Gly Ala Leu Leu Val Arg Leu Leu Gln Ala Leu Arg Val Ala Arg Met
                245                 250                 255

ccc ggg ctg gag cgg agc gtc cgt gag cgc ttc ctc cct gtg cac ggt
816
Pro Gly Leu Glu Arg Ser Val Arg Glu Arg Phe Leu Pro Val His Gly
            260                 265                 270

acc gca gac tac aag gac gac gat gac aag
846
Thr Ala Asp Tyr Lys Asp Asp Asp Asp Lys
        275                 280


<210>  104
<211>  282
<212>  PRT
<213>  Artificial Sequence

<220>
<223>  Amino acid sequence of DcR3 FL-FLAG

<400>  104

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser Cys Thr Gly Phe Pro Leu Ser Thr Arg Val
                165                 170                 175

Pro Gly Ala Glu Glu Cys Glu Arg Ala Val Ile Asp Phe Val Ala Phe
            180                 185                 190

Gln Asp Ile Ser Ile Lys Arg Leu Gln Arg Leu Leu Gln Ala Leu Glu
        195                 200                 205

Ala Pro Glu Gly Trp Gly Pro Thr Pro Arg Ala Gly Arg Ala Ala Leu
    210                 215                 220

Gln Leu Lys Leu Arg Arg Arg Leu Thr Glu Leu Leu Gly Ala Gln Asp
225                 230                 235                 240

Gly Ala Leu Leu Val Arg Leu Leu Gln Ala Leu Arg Val Ala Arg Met
                245                 250                 255

Pro Gly Leu Glu Arg Ser Val Arg Glu Arg Phe Leu Pro Val His Gly
            260                 265                 270

Thr Ala Asp Tyr Lys Asp Asp Asp Asp Lys
        275                 280

<210>  105
<211>  18
<212>  DNA
<213>  Artificial Sequence

<220>
<221>  CDS
<222>  (1)..(18)

<220>
<223>  Nucleotide sequence of Linker IEGRMD

<400>  105
atc gag ggg cgc atg gat
18
Ile Glu Gly Arg Met Asp
1               5

<210>  106
<211>  6
<212>  PRT
<213>  Artificial Sequence

<220>
<223>  Amino acid sequence of Linker IEGRMD

<400>  106

Ile Glu Gly Arg Met Asp
1               5

<210>  107
<211>  498
<212>  DNA
<213>  Artificial Sequence

<220>
<221>  CDS
<222>  (1)..(498)

<220>
<223>  Nucleotide sequence of DcR3 from which HBD has been deleted
(without a signal peptide)

<400>  107
gtg gca gaa aca ccc acc tac ccc tgg cgg gac gca gag aca ggg gag
48
Val Ala Glu Thr Pro Thr Tyr Pro Trp Arg Asp Ala Glu Thr Gly Glu
1               5                   10                  15

cgg ctg gtg tgc gcc cag tgc ccc cca ggc acc ttt gtg cag cgg ccg
96
Arg Leu Val Cys Ala Gln Cys Pro Pro Gly Thr Phe Val Gln Arg Pro
            20                  25                  30

tgc cgc cga gac agc ccc acg acg tgt ggc ccg tgt cca ccg cgc cac
144
Cys Arg Arg Asp Ser Pro Thr Thr Cys Gly Pro Cys Pro Pro Arg His
        35                  40                  45

tac acg cag ttc tgg aac tac cta gag cgc tgc cgc tac tgc aac gtc
192
Tyr Thr Gln Phe Trp Asn Tyr Leu Glu Arg Cys Arg Tyr Cys Asn Val
    50                  55                  60

ctc tgc ggg gag cgt gag gag gag gca cgg gct tgc cac gcc acc cac
240
Leu Cys Gly Glu Arg Glu Glu Glu Ala Arg Ala Cys His Ala Thr His
65                  70                  75                  80

aac cgt gcc tgc cgc tgc cgc acc ggc ttc ttc gcg cac gct ggt ttc
288
Asn Arg Ala Cys Arg Cys Arg Thr Gly Phe Phe Ala His Ala Gly Phe
                85                  90                  95

tgc ttg gag cac gca tcg tgt cca cct ggt gcc ggc gtg att gcc ccg
336
Cys Leu Glu His Ala Ser Cys Pro Pro Gly Ala Gly Val Ile Ala Pro
            100                 105                 110

ggc acc ccc agc cag aac acg cag tgc cag ccg tgc ccc cca ggc acc
384
Gly Thr Pro Ser Gln Asn Thr Gln Cys Gln Pro Cys Pro Pro Gly Thr
        115                 120                 125

ttc tca gcc agc agc tcc agc tca gag cag tgc cag ccc cac cgc aac
432
Phe Ser Ala Ser Ser Ser Ser Ser Glu Gln Cys Gln Pro His Arg Asn
    130                 135                 140

tgc acg gcc ctg ggc ctg gcc ctc aat gtg cca ggc tct tcc tcc cat
480
Cys Thr Ala Leu Gly Leu Ala Leu Asn Val Pro Gly Ser Ser Ser His
145                 150                 155                 160

gac acc ctg tgc acc agc
498
Asp Thr Leu Cys Thr Ser
                165


<210>  108
<211>  166
<212>  PRT
<213>  Artificial Sequence

<220>
<223>  Amino acid sequence of DcR3 from which HBD has been deleted
(without a signal peptide)

<400>  108

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser
                165

<210>  109
<211>  24
<212>  DNA
<213>  Artificial Sequence

<220>
<221>  CDS
<222>  (1)..(24)

<220>
<223>  Nucleotide sequence of FLAG tag

<400>  109

gac tac aag gac gac gat gac aag
24
Asp Tyr Lys Asp Asp Asp Asp Lys
1               5

<210>  110
<211>  8
<212>  PRT
<213>  Artificial Sequence

<220>
<223>  Amino acid sequence of FLAG tag

<400>  110

Asp Tyr Lys Asp Asp Asp Asp Lys
1               5

<210>  111
<211>  813
<212>  DNA
<213>  Artificial Sequence

<220>
<221>  CDS
<222>  (1)..(813)

<220>
<223>  Nucleotide sequence of R218Q DcR3

<400>  111
gtg gca gaa aca ccc acc tac ccc tgg cgg gac gca gag aca ggg gag
48
Val Ala Glu Thr Pro Thr Tyr Pro Trp Arg Asp Ala Glu Thr Gly Glu
1               5                   10                  15

cgg ctg gtg tgc gcc cag tgc ccc cca ggc acc ttt gtg cag cgg ccg
96
Arg Leu Val Cys Ala Gln Cys Pro Pro Gly Thr Phe Val Gln Arg Pro
            20                  25                  30

tgc cgc cga gac agc ccc acg acg tgt ggc ccg tgt cca ccg cgc cac
144
Cys Arg Arg Asp Ser Pro Thr Thr Cys Gly Pro Cys Pro Pro Arg His
        35                  40                  45

tac acg cag ttc tgg aac tac cta gag cgc tgc cgc tac tgc aac gtc
192
Tyr Thr Gln Phe Trp Asn Tyr Leu Glu Arg Cys Arg Tyr Cys Asn Val
    50                  55                  60

ctc tgc ggg gag cgt gag gag gag gca cgg gct tgc cac gcc acc cac
240
Leu Cys Gly Glu Arg Glu Glu Glu Ala Arg Ala Cys His Ala Thr His
65                  70                  75                  80

aac cgt gcc tgc cgc tgc cgc acc ggc ttc ttc gcg cac gct ggt ttc
288

Asn Arg Ala Cys Arg Cys Arg Thr Gly Phe Phe Ala His Ala Gly Phe
                85                  90                  95

tgc ttg gag cac gca tcg tgt cca cct ggt gcc ggc gtg att gcc ccg
336
Cys Leu Glu His Ala Ser Cys Pro Pro Gly Ala Gly Val Ile Ala Pro
            100                 105                 110

ggc acc ccc agc cag aac acg cag tgc cag ccg tgc ccc cca ggc acc
384
Gly Thr Pro Ser Gln Asn Thr Gln Cys Gln Pro Cys Pro Pro Gly Thr
        115                 120                 125

ttc tca gcc agc agc tcc agc tca gag cag tgc cag ccc cac cgc aac
432
Phe Ser Ala Ser Ser Ser Ser Ser Glu Gln Cys Gln Pro His Arg Asn
    130                 135                 140

tgc acg gcc ctg ggc ctg gcc ctc aat gtg cca ggc tct tcc tcc cat
480
Cys Thr Ala Leu Gly Leu Ala Leu Asn Val Pro Gly Ser Ser Ser His
145                 150                 155                 160

gac acc ctg tgc acc agc tgc act ggc ttc ccc ctc agc acc agg gta
528
Asp Thr Leu Cys Thr Ser Cys Thr Gly Phe Pro Leu Ser Thr Arg Val
                165                 170                 175

cca gga gct gag gag tgt gag cgt gcc gtc atc gac ttt gtg gct ttc
576
Pro Gly Ala Glu Glu Cys Glu Arg Ala Val Ile Asp Phe Val Ala Phe
            180                 185                 190

cag gac atc tcc atc aag agg ctg cag cgg ctg ctg cag gcc ctc gag
624
Gln Asp Ile Ser Ile Lys Arg Leu Gln Arg Leu Leu Gln Ala Leu Glu
        195                 200                 205

gcc ccg gag ggc tgg ggt ccg aca cca cag gcg ggc cgc gcg gcc ttg
672
Ala Pro Glu Gly Trp Gly Pro Thr Pro Gln Ala Gly Arg Ala Ala Leu
    210                 215                 220

cag ctg aag ctg cgt cgg cgg ctc acg gag ctc ctg ggg gcg cag gac
720
Gln Leu Lys Leu Arg Arg Arg Leu Thr Glu Leu Leu Gly Ala Gln Asp
225                 230                 235                 240

ggg gcg ctg ctg gtg cgg ctg ctg cag gcg ctg cgc gtg gcc agg atg
768
Gly Ala Leu Leu Val Arg Leu Leu Gln Ala Leu Arg Val Ala Arg Met
                245                 250                 255

ccc ggg ctg gag cgg agc gtc cgt gag cgc ttc ctc cct gtg cac
813
Pro Gly Leu Glu Arg Ser Val Arg Glu Arg Phe Leu Pro Val His
            260                 265                 270

<210>  112

<211>  271
<212>  PRT
<213>  Artificial Sequence

<220>
<223>  Amino acid sequence of R218Q DcR3

<400>  112

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser Cys Thr Gly Phe Pro Leu Ser Thr Arg Val
                165                 170                 175

Pro Gly Ala Glu Glu Cys Glu Arg Ala Val Ile Asp Phe Val Ala Phe
            180                 185                 190

Gln Asp Ile Ser Ile Lys Arg Leu Gln Arg Leu Leu Gln Ala Leu Glu
        195                 200                 205

Ala Pro Glu Gly Trp Gly Pro Thr Pro Gln Ala Gly Arg Ala Ala Leu
    210                 215                 220

Gln Leu Lys Leu Arg Arg Arg Leu Thr Glu Leu Leu Gly Ala Gln Asp
225                 230                 235                 240

Gly Ala Leu Leu Val Arg Leu Leu Gln Ala Leu Arg Val Ala Arg Met
                245                 250                 255

Pro Gly Leu Glu Arg Ser Val Arg Glu Arg Phe Leu Pro Val His
            260                 265                 270

<210>  113
<211>  579
<212>  DNA
<213>  Homo sapiens

<220>
<221>  CDS
<222>  (1)..(579)

<220>
<223>  Nucleotide sequence of soluble recombinant of human LIGHT

<400>  113
cat cat cat cat cat cat cat cat cat cat ggt ggt ggt agt ggt ggt
48
His His His His His His His His His His Gly Gly Gly Ser Gly Gly
1               5                   10                  15

ggt agt ggt ggt ggt agt atc gag gga cgg gac gga cct gca ggc tcc
96
Gly Ser Gly Gly Gly Ser Ile Glu Gly Arg Asp Gly Pro Ala Gly Ser
            20                  25                  30

tgg gag cag ctg ata caa gag cga agg tct cac gag gtc aac cca gca
144
Trp Glu Gln Leu Ile Gln Glu Arg Arg Ser His Glu Val Asn Pro Ala
        35                  40                  45

gcg cat ctc aca ggg gcc aac tcc agc ttg acc ggc agc ggg ggg ccg
192
Ala His Leu Thr Gly Ala Asn Ser Ser Leu Thr Gly Ser Gly Gly Pro
    50                  55                  60

ctg tta tgg gag act cag ctg ggc ctg gcc ttc ctg agg ggc ctc agc
240
Leu Leu Trp Glu Thr Gln Leu Gly Leu Ala Phe Leu Arg Gly Leu Ser
65                  70                  75                  80

tac cac gat ggg gcc ctt gtg gtc acc aaa gct ggc tac tac tac atc
288
Tyr His Asp Gly Ala Leu Val Val Thr Lys Ala Gly Tyr Tyr Tyr Ile
                85                  90                  95

tac tcc aag gtg cag ctg ggc ggt gtg ggc tgc ccg ctg ggc ctg gcc
336
Tyr Ser Lys Val Gln Leu Gly Gly Val Gly Cys Pro Leu Gly Leu Ala
            100                 105                 110

agc acc atc acc cac ggc ctc tac aag cgc aca ccc cgc tac ccc gag
384
Ser Thr Ile Thr His Gly Leu Tyr Lys Arg Thr Pro Arg Tyr Pro Glu
        115                 120                 125

gag ctg gag ctg ttg gtc agc cag cag tca ccc tgc gga cgg gcc acc
432
Glu Leu Glu Leu Leu Val Ser Gln Gln Ser Pro Cys Gly Arg Ala Thr
    130                 135                 140

agc agc tcc cgg gtc tgg tgg gac agc agc ttc ctg ggt ggt gtg gta
480
Ser Ser Ser Arg Val Trp Trp Asp Ser Ser Phe Leu Gly Gly Val Val
145                 150                 155                 160

cac ctg gag gct ggg gag aag gtg gtc gtc cgt gtg ctg gat gaa cgc
528
His Leu Glu Ala Gly Glu Lys Val Val Val Arg Val Leu Asp Glu Arg
                165                 170                 175

ctg gtt cga ctg cgt gat ggt acc cgg tct tac ttc ggg gct ttc atg
576
Leu Val Arg Leu Arg Asp Gly Thr Arg Ser Tyr Phe Gly Ala Phe Met
            180                 185                 190

gtg
579
Val

<210>  114
<211>  193
<212>  PRT
<213>  Homo sapiens

<220>
<223>  Amino acid sequence of soluble recombinant of human LIGHT

<400>  114

His His His His His His His His His His Gly Gly Gly Ser Gly Gly
1               5                   10                  15

Gly Ser Gly Gly Gly Ser Ile Glu Gly Arg Asp Gly Pro Ala Gly Ser
            20                  25                  30

Trp Glu Gln Leu Ile Gln Glu Arg Arg Ser His Glu Val Asn Pro Ala
        35                  40                  45

Ala His Leu Thr Gly Ala Asn Ser Ser Leu Thr Gly Ser Gly Gly Pro
    50                  55                  60

Leu Leu Trp Glu Thr Gln Leu Gly Leu Ala Phe Leu Arg Gly Leu Ser
65                  70                  75                  80

Tyr His Asp Gly Ala Leu Val Val Thr Lys Ala Gly Tyr Tyr Tyr Ile
                85                  90                  95

Tyr Ser Lys Val Gln Leu Gly Gly Val Gly Cys Pro Leu Gly Leu Ala
            100                 105                 110

Ser Thr Ile Thr His Gly Leu Tyr Lys Arg Thr Pro Arg Tyr Pro Glu
        115                 120                 125

Glu Leu Glu Leu Leu Val Ser Gln Gln Ser Pro Cys Gly Arg Ala Thr
    130                 135                 140

Ser Ser Ser Arg Val Trp Trp Asp Ser Ser Phe Leu Gly Gly Val Val
145                 150                 155                 160

His Leu Glu Ala Gly Glu Lys Val Val Val Arg Val Leu Asp Glu Arg
                165                 170                 175

Leu Val Arg Leu Arg Asp Gly Thr Arg Ser Tyr Phe Gly Ala Phe Met
            180                 185                 190

Val

<210>  115
<211>  594
<212>  DNA
<213>  Homo sapiens

<220>
<221>  CDS
<222>  (1)..(594)

<220>
<223>  Nucleotide sequence of soluble recombinant of human TL1A

<400>  115
cat cat cat cat cat cat ggt ggt ggt agt ggt ggt ggt agt ggt ggt
48
His His His His His His Gly Gly Gly Ser Gly Gly Gly Ser Gly Gly
1               5                   10                  15

ggt agt cta aaa gga cag gag ttt gca cct tca cat cag caa gtt tat
96
Gly Ser Leu Lys Gly Gln Glu Phe Ala Pro Ser His Gln Gln Val Tyr

20                  25                  30

gca cct ctt aga gca gac gga gat aag cca agg gca cac ctg aca gtt
144
Ala Pro Leu Arg Ala Asp Gly Asp Lys Pro Arg Ala His Leu Thr Val
        35                  40                  45

gtg aga caa act ccc aca cag cac ttt aaa aat cag ttc cca gct ctg
192
Val Arg Gln Thr Pro Thr Gln His Phe Lys Asn Gln Phe Pro Ala Leu
    50                  55                  60

cac tgg gaa cat gaa cta ggc ctg gcc ttc acc aag aac cga atg aac
240
His Trp Glu His Glu Leu Gly Leu Ala Phe Thr Lys Asn Arg Met Asn
65                  70                  75                  80

tat acc aac aaa ttc ctg ctg atc cca gag tcg gga gac tac ttc att
288
Tyr Thr Asn Lys Phe Leu Leu Ile Pro Glu Ser Gly Asp Tyr Phe Ile
                85                  90                  95

tac tcc cag gtc aca ttc cgt ggg atg acc tct gag tgc agt gaa atc
336
Tyr Ser Gln Val Thr Phe Arg Gly Met Thr Ser Glu Cys Ser Glu Ile
            100                 105                 110

aga caa gca ggc cga cca aac aag cca gac tcc atc act gtg gtc atc
384
Arg Gln Ala Gly Arg Pro Asn Lys Pro Asp Ser Ile Thr Val Val Ile
        115                 120                 125

acc aag gta aca gac agc tac cct gag cca acc cag ctc ctc atg ggg
432
Thr Lys Val Thr Asp Ser Tyr Pro Glu Pro Thr Gln Leu Leu Met Gly
    130                 135                 140

acc aag tct gta tgc gaa gta ggt agc aac tgg ttc cag ccc atc tac
480
Thr Lys Ser Val Cys Glu Val Gly Ser Asn Trp Phe Gln Pro Ile Tyr
145                 150                 155                 160

ctc gga gcc atg ttc tcc ttg caa gaa ggg gac aag cta atg gtg aac
528
Leu Gly Ala Met Phe Ser Leu Gln Glu Gly Asp Lys Leu Met Val Asn
                165                 170                 175

gtc agt gac atc tct ttg gtg gat tac aca aaa gaa gat aaa acc ttc
576
Val Ser Asp Ile Ser Leu Val Asp Tyr Thr Lys Glu Asp Lys Thr Phe
            180                 185                 190

ttt gga gcc ttc tta cta
594
Phe Gly Ala Phe Leu Leu
        195


<210>  116
<211>  198

<212>  PRT
<213>  Homo sapiens

<220>
<223>  Amino acid sequence of soluble recombinant of human TL1A

<400>  116

His His His His His His Gly Gly Gly Ser Gly Gly Gly Ser Gly Gly
1               5                   10                  15

Gly Ser Leu Lys Gly Gln Glu Phe Ala Pro Ser His Gln Gln Val Tyr
            20                  25                  30

Ala Pro Leu Arg Ala Asp Gly Asp Lys Pro Arg Ala His Leu Thr Val
        35                  40                  45

Val Arg Gln Thr Pro Thr Gln His Phe Lys Asn Gln Phe Pro Ala Leu
    50                  55                  60

His Trp Glu His Glu Leu Gly Leu Ala Phe Thr Lys Asn Arg Met Asn
65                  70                  75                  80

Tyr Thr Asn Lys Phe Leu Leu Ile Pro Glu Ser Gly Asp Tyr Phe Ile
                85                  90                  95

Tyr Ser Gln Val Thr Phe Arg Gly Met Thr Ser Glu Cys Ser Glu Ile
            100                 105                 110

Arg Gln Ala Gly Arg Pro Asn Lys Pro Asp Ser Ile Thr Val Val Ile
        115                 120                 125

Thr Lys Val Thr Asp Ser Tyr Pro Glu Pro Thr Gln Leu Leu Met Gly
    130                 135                 140

Thr Lys Ser Val Cys Glu Val Gly Ser Asn Trp Phe Gln Pro Ile Tyr
145                 150                 155                 160

Leu Gly Ala Met Phe Ser Leu Gln Glu Gly Asp Lys Leu Met Val Asn
                165                 170                 175

Val Ser Asp Ile Ser Leu Val Asp Tyr Thr Lys Glu Asp Lys Thr Phe
            180                 185                 190

Phe Gly Ala Phe Leu Leu
        195

<210>  117
<211>  462
<212>  DNA
<213>  Homo sapiens

<220>
<221>  CDS
<222>  (1)..(462)

<220>
<223>  Nucleotide sequence of soluble recombinant of human FasL

<400>  117
cat cat cat cat cat cat ccc agt cca ccc cct gaa aaa aag gag ctg
48
His His His His His His Pro Ser Pro Pro Pro Glu Lys Lys Glu Leu
1               5                   10                  15

agg aaa gtg gcc cat tta aca ggc aag tcc aac tca agg tcc atg cct
96
Arg Lys Val Ala His Leu Thr Gly Lys Ser Asn Ser Arg Ser Met Pro
            20                  25                  30

ctg gaa tgg gaa gac acc tat gga att gtc ctg ctt tct gga gtg aag
144
Leu Glu Trp Glu Asp Thr Tyr Gly Ile Val Leu Leu Ser Gly Val Lys
        35                  40                  45

tat aag aag ggt ggc ctt gtg atc aat gaa act ggg ctg tac ttt gta
192
Tyr Lys Lys Gly Gly Leu Val Ile Asn Glu Thr Gly Leu Tyr Phe Val
    50                  55                  60

tat tcc aaa gta tac ttc cgg ggt caa tct tgc aac aac ctg ccc ctg
240
Tyr Ser Lys Val Tyr Phe Arg Gly Gln Ser Cys Asn Asn Leu Pro Leu
65                  70                  75                  80

agc cac aag gtc tac atg agg aac tct aag tat ccc cag gat ctg gtg
288
Ser His Lys Val Tyr Met Arg Asn Ser Lys Tyr Pro Gln Asp Leu Val
                85                  90                  95

atg atg gag ggg aag atg atg agc tac tgc act act ggg cag atg tgg
336
Met Met Glu Gly Lys Met Met Ser Tyr Cys Thr Thr Gly Gln Met Trp
            100                 105                 110

gcc cgc agc agc tac ctg ggg gca gtg ttc aat ctt acc agt gct gat
384
Ala Arg Ser Ser Tyr Leu Gly Ala Val Phe Asn Leu Thr Ser Ala Asp
        115                 120                 125

cat tta tat gtc aac gta tct gag ctc tct ctg gtc aat ttt gag gaa
432
His Leu Tyr Val Asn Val Ser Glu Leu Ser Leu Val Asn Phe Glu Glu
    130                 135                 140

tct cag acg ttt ttc ggc tta tat aag ctc
462

Ser Gln Thr Phe Phe Gly Leu Tyr Lys Leu
145                 150

<210>  118
<211>  154
<212>  PRT
<213>  Homo sapiens

<220>
<223>  Amino acid sequence of soluble recombinant of human FasL

<400>  118

His His His His His His Pro Ser Pro Pro Pro Glu Lys Lys Glu Leu
1               5                   10                  15

Arg Lys Val Ala His Leu Thr Gly Lys Ser Asn Ser Arg Ser Met Pro
            20                  25                  30

Leu Glu Trp Glu Asp Thr Tyr Gly Ile Val Leu Leu Ser Gly Val Lys
        35                  40                  45

Tyr Lys Lys Gly Gly Leu Val Ile Asn Glu Thr Gly Leu Tyr Phe Val
    50                  55                  60

Tyr Ser Lys Val Tyr Phe Arg Gly Gln Ser Cys Asn Asn Leu Pro Leu
65                  70                  75                  80

Ser His Lys Val Tyr Met Arg Asn Ser Lys Tyr Pro Gln Asp Leu Val
                85                  90                  95

Met Met Glu Gly Lys Met Met Ser Tyr Cys Thr Thr Gly Gln Met Trp
            100                 105                 110

Ala Arg Ser Ser Tyr Leu Gly Ala Val Phe Asn Leu Thr Ser Ala Asp
        115                 120                 125

His Leu Tyr Val Asn Val Ser Glu Leu Ser Leu Val Asn Phe Glu Glu
    130                 135                 140

Ser Gln Thr Phe Phe Gly Leu Tyr Lys Leu
145                 150

<210>  119
<211>  579
<212>  DNA
<213>  Macaca fascicularis

<220>

<221>  CDS
<222>  (1)..(579)

<220>
<223>  Nucleotide sequence of soluble recombinant of Macaca fascicularis
LIGHT

<400>  119
cat cat cat cat cat cat cat cat cat cat ggt ggt ggt agt ggt ggt
48
His His His His His His His His His His Gly Gly Gly Ser Gly Gly
1               5                   10                  15

ggt agt ggt ggt ggt agt atc gag gga cgg gac gga cac gga ggc tcc
96
Gly Ser Gly Gly Gly Ser Ile Glu Gly Arg Asp Gly His Gly Gly Ser
            20                  25                  30

tgg gag cag ctg ata caa gag cga agg tct cac cag aat aac cca gca
144
Trp Glu Gln Leu Ile Gln Glu Arg Arg Ser His Gln Asn Asn Pro Ala
        35                  40                  45

gca cat ctc aca ggg gcc aac tcc agc ttg acg ggc agc ggg gga ccg
192
Ala His Leu Thr Gly Ala Asn Ser Ser Leu Thr Gly Ser Gly Gly Pro
    50                  55                  60

ctg cta tgg gag acc cag ctg ggc ctg gcc ttc ctg agg ggc ctc gac
240
Leu Leu Trp Glu Thr Gln Leu Gly Leu Ala Phe Leu Arg Gly Leu Asp
65                  70                  75                  80

tac cac gat ggg gcc ctt gtg gcc aac aag gct ggc tac tac tac atc
288
Tyr His Asp Gly Ala Leu Val Ala Asn Lys Ala Gly Tyr Tyr Tyr Ile
                85                  90                  95

tac tcc aag gtg cag ctg ggc ggt gtg ggc tgc ccg ctg ggc ctg gcc
336
Tyr Ser Lys Val Gln Leu Gly Gly Val Gly Cys Pro Leu Gly Leu Ala
            100                 105                 110

agc acc atc acc cac ggc ctc tac aag cgc acg ccc cgc tac ccc gag
384
Ser Thr Ile Thr His Gly Leu Tyr Lys Arg Thr Pro Arg Tyr Pro Glu
        115                 120                 125

gag ctg gag ctg ttg gtc agc cag cag tca ccc tgc gga cgg gcc acc
432
Glu Leu Glu Leu Leu Val Ser Gln Gln Ser Pro Cys Gly Arg Ala Thr
    130                 135                 140

agc aac tcc cgc gtc tgg tgg gac agc agc ttc ctg ggt ggt gtg gtg
480
Ser Asn Ser Arg Val Trp Trp Asp Ser Ser Phe Leu Gly Gly Val Val
145                 150                 155                 160

cac ctg gag gtt ggg gag gag gtg gtc gtc cgt gtg ctg gat gag cgc
528

His Leu Glu Val Gly Glu Glu Val Val Val Arg Val Leu Asp Glu Arg
                165                 170                 175

ctg gtc cga ctg cgt gat ggc acc cgg tct tac ttc ggg gct ttc atg
576
Leu Val Arg Leu Arg Asp Gly Thr Arg Ser Tyr Phe Gly Ala Phe Met
            180                 185                 190

gtg
579
Val

<210>  120
<211>  193
<212>  PRT
<213>  Macaca fascicularis

<220>
<223>  Amino acid sequence of soluble recombinant of Macaca fascicularis
LIGHT

<400>  120

His His His His His His His His His His Gly Gly Gly Ser Gly Gly
1               5                   10                  15

Gly Ser Gly Gly Gly Ser Ile Glu Gly Arg Asp Gly His Gly Gly Ser
            20                  25                  30

Trp Glu Gln Leu Ile Gln Glu Arg Arg Ser His Gln Asn Asn Pro Ala
        35                  40                  45

Ala His Leu Thr Gly Ala Asn Ser Ser Leu Thr Gly Ser Gly Gly Pro
    50                  55                  60

Leu Leu Trp Glu Thr Gln Leu Gly Leu Ala Phe Leu Arg Gly Leu Asp
65                  70                  75                  80

Tyr His Asp Gly Ala Leu Val Ala Asn Lys Ala Gly Tyr Tyr Tyr Ile
                85                  90                  95

Tyr Ser Lys Val Gln Leu Gly Gly Val Gly Cys Pro Leu Gly Leu Ala
            100                 105                 110

Ser Thr Ile Thr His Gly Leu Tyr Lys Arg Thr Pro Arg Tyr Pro Glu
        115                 120                 125

Glu Leu Glu Leu Leu Val Ser Gln Gln Ser Pro Cys Gly Arg Ala Thr
    130                 135                 140

Ser Asn Ser Arg Val Trp Trp Asp Ser Ser Phe Leu Gly Gly Val Val
145                 150                 155                 160

His Leu Glu Val Gly Glu Glu Val Val Val Arg Val Leu Asp Glu Arg
                165                 170                 175

Leu Val Arg Leu Arg Asp Gly Thr Arg Ser Tyr Phe Gly Ala Phe Met
            180                 185                 190

Val

<210>  121
<211>  594
<212>  DNA
<213>  Macaca fascicularis

<220>
<221>  CDS
<222>  (1)..(594)

<220>
<223>  Nucleotide sequence of soluble recombinant of Macaca fascicularis
TL1A

<400>  121
cat cat cat cat cat cat ggt ggt ggt agt ggt ggt ggt agt ggt ggt
48
His His His His His His Gly Gly Gly Ser Gly Gly Gly Ser Gly Gly
1               5                   10                  15

ggt agt cta aaa gga cag gag ttt gca cct tca cat cag caa gtt tat
96
Gly Ser Leu Lys Gly Gln Glu Phe Ala Pro Ser His Gln Gln Val Tyr
            20                  25                  30

gca cct ctt aga gca gat gga gat aag cca agg gca cac ctg aca gtt
144
Ala Pro Leu Arg Ala Asp Gly Asp Lys Pro Arg Ala His Leu Thr Val
        35                  40                  45

gtg aga caa act ccc aca cag cac tta aaa aat cag ttc cca gct ctg
192
Val Arg Gln Thr Pro Thr Gln His Leu Lys Asn Gln Phe Pro Ala Leu
    50                  55                  60

cac tgg gaa cat gaa cta ggc ctg gcc ttc acc aag aac cga atg aac
240
His Trp Glu His Glu Leu Gly Leu Ala Phe Thr Lys Asn Arg Met Asn
65                  70                  75                  80

tat acc aac aaa ttc ctg ctg atc cca gag tcg gga gac tac ttc gtt
288
Tyr Thr Asn Lys Phe Leu Leu Ile Pro Glu Ser Gly Asp Tyr Phe Val
                85                  90                  95

tac tcc cag gtc aca ttc cgt ggg atg acc tct gag tgc agt gaa atc
336
Tyr Ser Gln Val Thr Phe Arg Gly Met Thr Ser Glu Cys Ser Glu Ile
            100                 105                 110

aga caa gca ggc cga cca aac aag cca gac tcc atc act gtg gtc atc
384
Arg Gln Ala Gly Arg Pro Asn Lys Pro Asp Ser Ile Thr Val Val Ile
        115                 120                 125

acc aag gta aca gac agc tac cct gag cca acc cag ctc ctc atg ggg
432
Thr Lys Val Thr Asp Ser Tyr Pro Glu Pro Thr Gln Leu Leu Met Gly
    130                 135                 140

acc aag tct gtg tgt gaa gta ggc agt aac tgg ttc cag ccc atc tac
480
Thr Lys Ser Val Cys Glu Val Gly Ser Asn Trp Phe Gln Pro Ile Tyr
145                 150                 155                 160

ctc gga gcc atg ttc tcc ttg caa gaa ggg gac aag ctc atg gtg aac
528
Leu Gly Ala Met Phe Ser Leu Gln Glu Gly Asp Lys Leu Met Val Asn
                165                 170                 175

gtc agt gac atc tct ttg gtg gat tac aca aaa gaa gat aaa acc ttc
576
Val Ser Asp Ile Ser Leu Val Asp Tyr Thr Lys Glu Asp Lys Thr Phe
            180                 185                 190

ttt gga gcc ttc tta cta
594
Phe Gly Ala Phe Leu Leu
        195



<210>  122
<211>  198
<212>  PRT
<213>  Macaca fascicularis

<220>
<223>  Amino acid sequence of soluble recombinant of Macaca fascicularis
TL1A

<400>  122

His His His His His His Gly Gly Gly Ser Gly Gly Gly Ser Gly Gly
1               5                   10                  15


Gly Ser Leu Lys Gly Gln Glu Phe Ala Pro Ser His Gln Gln Val Tyr
            20                  25                  30


Ala Pro Leu Arg Ala Asp Gly Asp Lys Pro Arg Ala His Leu Thr Val
        35                  40                  45

Val Arg Gln Thr Pro Thr Gln His Leu Lys Asn Gln Phe Pro Ala Leu
    50                  55                  60

His Trp Glu His Glu Leu Gly Leu Ala Phe Thr Lys Asn Arg Met Asn
65                  70                  75                  80

Tyr Thr Asn Lys Phe Leu Leu Ile Pro Glu Ser Gly Asp Tyr Phe Val
                85                  90                  95

Tyr Ser Gln Val Thr Phe Arg Gly Met Thr Ser Glu Cys Ser Glu Ile
            100                 105                 110

Arg Gln Ala Gly Arg Pro Asn Lys Pro Asp Ser Ile Thr Val Val Ile
        115                 120                 125

Thr Lys Val Thr Asp Ser Tyr Pro Glu Pro Thr Gln Leu Leu Met Gly
    130                 135                 140

Thr Lys Ser Val Cys Glu Val Gly Ser Asn Trp Phe Gln Pro Ile Tyr
145                 150                 155                 160

Leu Gly Ala Met Phe Ser Leu Gln Glu Gly Asp Lys Leu Met Val Asn
                165                 170                 175

Val Ser Asp Ile Ser Leu Val Asp Tyr Thr Lys Glu Asp Lys Thr Phe
            180                 185                 190

Phe Gly Ala Phe Leu Leu
        195

<210>  123
<211>  462
<212>  DNA
<213>  Macaca fascicularis

<220>
<221>  CDS
<222>  (1)..(462)

<220>
<223>  Nucleotide sequence of soluble recombinant of Macaca fascicularis
FasL

<400>  123
cat cat cat cat cat cat ccc agt cca ccc cct gag aaa aag gag cag
48
His His His His His His Pro Ser Pro Pro Pro Glu Lys Lys Glu Gln
1               5                   10                  15

agg aaa gtg gcc cat tta aca ggc aag ccc aac tca agg tcc atg cct
96
Arg Lys Val Ala His Leu Thr Gly Lys Pro Asn Ser Arg Ser Met Pro
            20                  25                  30

ctg gaa tgg gaa gac acc tat gga att gtc ctg ctt tct gga gtg aag
144
Leu Glu Trp Glu Asp Thr Tyr Gly Ile Val Leu Leu Ser Gly Val Lys
        35                  40                  45

tat aag aag ggt ggc ctt gtg atc aat gaa act ggg ctg tac ttt gta
192
Tyr Lys Lys Gly Gly Leu Val Ile Asn Glu Thr Gly Leu Tyr Phe Val
    50                  55                  60

tat tcc aaa gta tac ttc cgg ggt caa tct tgc acc aac ctg ccc ctg
240
Tyr Ser Lys Val Tyr Phe Arg Gly Gln Ser Cys Thr Asn Leu Pro Leu
65                  70                  75                  80

agc cac aag gtc tac atg agg aac tct aag tat ccc cag gat ctg gtg
288
Ser His Lys Val Tyr Met Arg Asn Ser Lys Tyr Pro Gln Asp Leu Val
                85                  90                  95

atg atg gag ggg aag atg atg agc tac tgc act act ggg cag atg tgg
336
Met Met Glu Gly Lys Met Met Ser Tyr Cys Thr Thr Gly Gln Met Trp
            100                 105                 110

gcc cac agc agc tac ctg ggg gca gtg ttc aat ctt acc agt gct gat
384
Ala His Ser Ser Tyr Leu Gly Ala Val Phe Asn Leu Thr Ser Ala Asp
        115                 120                 125

cat tta tat gtc aat gta tct gag ctc tct ctg gtc aat ttt gag gaa
432
His Leu Tyr Val Asn Val Ser Glu Leu Ser Leu Val Asn Phe Glu Glu
    130                 135                 140

tct cag aca ttt ttt ggc cta tat aag ctc
462
Ser Gln Thr Phe Phe Gly Leu Tyr Lys Leu
145                 150


<210>  124
<211>  154
<212>  PRT
<213>  Macaca fascicularis

<220>
<223>  Amino acid sequence of soluble recombinant of Macaca fascicularis
FasL

<400>  124

His His His His His His Pro Ser Pro Pro Pro Glu Lys Lys Glu Gln
1               5                   10                  15

Arg Lys Val Ala His Leu Thr Gly Lys Pro Asn Ser Arg Ser Met Pro
            20                  25                  30

Leu Glu Trp Glu Asp Thr Tyr Gly Ile Val Leu Leu Ser Gly Val Lys
        35                  40                  45

Tyr Lys Lys Gly Gly Leu Val Ile Asn Glu Thr Gly Leu Tyr Phe Val
    50                  55                  60

Tyr Ser Lys Val Tyr Phe Arg Gly Gln Ser Cys Thr Asn Leu Pro Leu
65                  70                  75                  80

Ser His Lys Val Tyr Met Arg Asn Ser Lys Tyr Pro Gln Asp Leu Val
                85                  90                  95

Met Met Glu Gly Lys Met Met Ser Tyr Cys Thr Thr Gly Gln Met Trp
            100                 105                 110

Ala His Ser Ser Tyr Leu Gly Ala Val Phe Asn Leu Thr Ser Ala Asp
        115                 120                 125

His Leu Tyr Val Asn Val Ser Glu Leu Ser Leu Val Asn Phe Glu Glu
    130                 135                 140

Ser Gln Thr Phe Phe Gly Leu Tyr Lys Leu
145                 150

<210> 125
<211> 582
<212> DNA
<213> Mus musculus

<220>
<221> CDS
<222> (1)..(582)

<220>
<223> Nucleotide sequence of soluble recombinant of mouse LIGHT

<400> 125
cat cat cat cat cat cat cat cat cat cat ggt ggt ggt agt ggt ggt
48
His His His His His His His His His His Gly Gly Gly Ser Gly Gly
1               5                   10                  15

ggt agt ggt ggt ggt agt atc gag gga cgg gat gga ggc aaa ggc tcc
96
Gly Ser Gly Gly Gly Ser Ile Glu Gly Arg Asp Gly Gly Lys Gly Ser
            20                  25                  30

tgg gag aag ctg ata caa gat caa cga tct cac cag gcc aac cca gca
144
Trp Glu Lys Leu Ile Gln Asp Gln Arg Ser His Gln Ala Asn Pro Ala
        35                  40                  45

gca cat ctt aca gga gcc aac gcc agc ttg ata ggt att ggt gga cct
192
Ala His Leu Thr Gly Ala Asn Ala Ser Leu Ile Gly Ile Gly Gly Pro
    50                  55                  60

ctg tta tgg gag aca cga ctt ggc ctg gcc ttc ttg agg ggc ttg acg
240
Leu Leu Trp Glu Thr Arg Leu Gly Leu Ala Phe Leu Arg Gly Leu Thr
65                  70                  75                  80

tat cat gat ggg gcc ctg gtg acc atg gag ccc ggt tac tac tat gtg
288
Tyr His Asp Gly Ala Leu Val Thr Met Glu Pro Gly Tyr Tyr Tyr Val
                85                  90                  95

tac tcc aaa gtg cag ctg agc ggc gtg ggc tgc ccc cag ggg ctg gcc
336
Tyr Ser Lys Val Gln Leu Ser Gly Val Gly Cys Pro Gln Gly Leu Ala
            100                 105                 110

aat ggc ctc ccc atc acc cat gga cta tac aag cgc aca tcc cgc tac
384
Asn Gly Leu Pro Ile Thr His Gly Leu Tyr Lys Arg Thr Ser Arg Tyr
        115                 120                 125

ccg aag gag tta gaa ctg ctg gtc agt cgg cgg tca ccc tgt ggc cgg
432
Pro Lys Glu Leu Glu Leu Leu Val Ser Arg Arg Ser Pro Cys Gly Arg
    130                 135                 140

gcc aac agc tcc cga gtc tgg tgg gac agc agc ttc ctg ggc ggc gtg
480
Ala Asn Ser Ser Arg Val Trp Trp Asp Ser Ser Phe Leu Gly Gly Val
145                 150                 155                 160

gta cat ctg gag gct ggg gaa gag gtg gtg gtc cgc gtg cct gga aac
528
Val His Leu Glu Ala Gly Glu Glu Val Val Val Arg Val Pro Gly Asn
                165                 170                 175

cgc ctg gtc aga cca cgt gac ggc acc agg tcc tat ttc gga gct ttc
576
Arg Leu Val Arg Pro Arg Asp Gly Thr Arg Ser Tyr Phe Gly Ala Phe
            180                 185                 190

atg gtc
582
Met Val

<210>  126
<211>  194
<212>  PRT

<213>  Mus musculus

<220>
<223>  Amino acid sequence of soluble recombinant of mouse LIGHT

<400>  126

His His His His His His His His His His Gly Gly Gly Ser Gly Gly
1               5                   10                  15

Gly Ser Gly Gly Gly Ser Ile Glu Gly Arg Asp Gly Gly Lys Gly Ser
            20                  25                  30

Trp Glu Lys Leu Ile Gln Asp Gln Arg Ser His Gln Ala Asn Pro Ala
        35                  40                  45

Ala His Leu Thr Gly Ala Asn Ala Ser Leu Ile Gly Ile Gly Gly Pro
    50                  55                  60

Leu Leu Trp Glu Thr Arg Leu Gly Leu Ala Phe Leu Arg Gly Leu Thr
65                  70                  75                  80

Tyr His Asp Gly Ala Leu Val Thr Met Glu Pro Gly Tyr Tyr Tyr Val
                85                  90                  95

Tyr Ser Lys Val Gln Leu Ser Gly Val Gly Cys Pro Gln Gly Leu Ala
            100                 105                 110

Asn Gly Leu Pro Ile Thr His Gly Leu Tyr Lys Arg Thr Ser Arg Tyr
        115                 120                 125

Pro Lys Glu Leu Glu Leu Leu Val Ser Arg Arg Ser Pro Cys Gly Arg
    130                 135                 140

Ala Asn Ser Ser Arg Val Trp Trp Asp Ser Ser Phe Leu Gly Gly Val
145                 150                 155                 160

Val His Leu Glu Ala Gly Glu Glu Val Val Val Arg Val Pro Gly Asn
                165                 170                 175

Arg Leu Val Arg Pro Arg Asp Gly Thr Arg Ser Tyr Phe Gly Ala Phe
            180                 185                 190

Met Val

<210>  127

<211>  585
<212>  DNA
<213>  Mus musculus

<220>
<221>  CDS
<222>  (1)..(585)

<220>
<223>  Nucleotide sequence of soluble recombinant of mouse TL1A


<400>  127
cat cat cat cat cat cat ggt ggt ggt agt ggt ggt ggt agt ggt ggt
48
His His His His His His Gly Gly Gly Ser Gly Gly Gly Ser Gly Gly
1               5                   10                  15

ggt agt ata aca gaa gag agg tct gag cct tca cca cag caa gtt tac
96
Gly Ser Ile Thr Glu Glu Arg Ser Glu Pro Ser Pro Gln Gln Val Tyr
            20                  25                  30

tca cct ccc aga ggc aag ccg aga gca cac ctg aca att aag aaa caa
144
Ser Pro Pro Arg Gly Lys Pro Arg Ala His Leu Thr Ile Lys Lys Gln
        35                  40                  45

acc cca gca cca cat ctg aaa aat cag ctc tct gct cta cac tgg gaa
192
Thr Pro Ala Pro His Leu Lys Asn Gln Leu Ser Ala Leu His Trp Glu
    50                  55                  60

cat gac cta ggg atg gcc ttc acc aag aac ggg atg aag tac atc aac
240
His Asp Leu Gly Met Ala Phe Thr Lys Asn Gly Met Lys Tyr Ile Asn
65                  70                  75                  80

aaa tcc ctg gtg atc cca gag tca gga gac tat ttc atc tac tcc cag
288
Lys Ser Leu Val Ile Pro Glu Ser Gly Asp Tyr Phe Ile Tyr Ser Gln
                85                  90                  95

atc aca ttc cga ggg acc aca tct gtg tgt ggt gac atc agt cgg ggg
336
Ile Thr Phe Arg Gly Thr Thr Ser Val Cys Gly Asp Ile Ser Arg Gly
            100                 105                 110

aga cga cca aac aag cca gac tcc atc acc atg gtt atc acc aag gta
384
Arg Arg Pro Asn Lys Pro Asp Ser Ile Thr Met Val Ile Thr Lys Val
        115                 120                 125

gca gac agc tac cct gag cct gcc cgc cta cta aca ggg tcc aag tct
432
Ala Asp Ser Tyr Pro Glu Pro Ala Arg Leu Leu Thr Gly Ser Lys Ser
    130                 135                 140

gtg tgt gaa ata agc aac aac tgg ttc cag tcc ctc tac ctt ggg gcc
480

Val Cys Glu Ile Ser Asn Asn Trp Phe Gln Ser Leu Tyr Leu Gly Ala
145                 150                 155                 160

acg ttc tcc ttg gaa gaa gga gac aga cta atg gta aac gtc agt gac
528
Thr Phe Ser Leu Glu Glu Gly Asp Arg Leu Met Val Asn Val Ser Asp
                165                 170                 175

atc tcc ttg gtg gat tac aca aaa gaa gat aaa act ttc ttt gga gct
576
Ile Ser Leu Val Asp Tyr Thr Lys Glu Asp Lys Thr Phe Phe Gly Ala
            180                 185                 190

ttc ttg cta
585
Phe Leu Leu
        195

<210>  128
<211>  195
<212>  PRT
<213>  Mus musculus

<220>
<223>  Amino acid sequence of soluble recombinant of mouse TL1A

<400>  128

His His His His His His Gly Gly Gly Ser Gly Gly Gly Ser Gly Gly
1               5                   10                  15

Gly Ser Ile Thr Glu Glu Arg Ser Glu Pro Ser Pro Gln Gln Val Tyr
            20                  25                  30

Ser Pro Pro Arg Gly Lys Pro Arg Ala His Leu Thr Ile Lys Lys Gln
        35                  40                  45

Thr Pro Ala Pro His Leu Lys Asn Gln Leu Ser Ala Leu His Trp Glu
    50                  55                  60

His Asp Leu Gly Met Ala Phe Thr Lys Asn Gly Met Lys Tyr Ile Asn
65                  70                  75                  80

Lys Ser Leu Val Ile Pro Glu Ser Gly Asp Tyr Phe Ile Tyr Ser Gln
                85                  90                  95

Ile Thr Phe Arg Gly Thr Thr Ser Val Cys Gly Asp Ile Ser Arg Gly
            100                 105                 110

Arg Arg Pro Asn Lys Pro Asp Ser Ile Thr Met Val Ile Thr Lys Val
        115                 120                 125

Ala Asp Ser Tyr Pro Glu Pro Ala Arg Leu Leu Thr Gly Ser Lys Ser
    130                 135                 140

Val Cys Glu Ile Ser Asn Asn Trp Phe Gln Ser Leu Tyr Leu Gly Ala
145                 150                 155                 160

Thr Phe Ser Leu Glu Glu Gly Asp Arg Leu Met Val Asn Val Ser Asp
                165                 170                 175

Ile Ser Leu Val Asp Tyr Thr Lys Glu Asp Lys Thr Phe Phe Gly Ala
            180                 185                 190

Phe Leu Leu
        195

<210>  129
<211>  462
<212>  DNA
<213>  Mus musculus

<220>
<221>  CDS
<222>  (1)..(462)

<220>
<223>  Nucleotide sequence of soluble recombinant of mouse FasL

<400>  129
cat cat cat cat cat cat ccc agt aca ccc tct gaa aaa aaa gag ccg
48
His His His His His His Pro Ser Thr Pro Ser Glu Lys Lys Glu Pro
1               5                   10                  15

agg agt gtg gcc cat tta aca ggg aac ccc cac tca agg tcc atc cct
96
Arg Ser Val Ala His Leu Thr Gly Asn Pro His Ser Arg Ser Ile Pro
            20                  25                  30

ctg gaa tgg gaa gac aca tat gga acc gct ctg atc tct gga gtg aag
144
Leu Glu Trp Glu Asp Thr Tyr Gly Thr Ala Leu Ile Ser Gly Val Lys
        35                  40                  45

tat aag aaa ggt ggc ctt gtg atc aac gaa act ggg ttg tac ttc gtg
192
Tyr Lys Lys Gly Gly Leu Val Ile Asn Glu Thr Gly Leu Tyr Phe Val
    50                  55                  60

tat tcc aaa gta tac ttc cgg ggt cag tct tgc aac aac cag ccc cta
240
Tyr Ser Lys Val Tyr Phe Arg Gly Gln Ser Cys Asn Asn Gln Pro Leu
65                  70                  75                  80

aac cac aag gtc tat atg agg aac tct aag tat cct gag gat ctg gtg
288
Asn His Lys Val Tyr Met Arg Asn Ser Lys Tyr Pro Glu Asp Leu Val
                85                  90                  95

cta atg gag gag aag agg ttg aac tac tgc act act gga cag ata tgg
336
Leu Met Glu Glu Lys Arg Leu Asn Tyr Cys Thr Thr Gly Gln Ile Trp
            100                 105                 110

gcc cac agc agc tac ctg ggg gca gta ttc aat ctt acc agt gct gac
384
Ala His Ser Ser Tyr Leu Gly Ala Val Phe Asn Leu Thr Ser Ala Asp
        115                 120                 125

cat tta tat gtc aac ata tct caa ctc tct ctg atc aat ttt gag gaa
432
His Leu Tyr Val Asn Ile Ser Gln Leu Ser Leu Ile Asn Phe Glu Glu
    130                 135                 140

tct aag acc ttt ttc ggc tta tat aag ctc
462
Ser Lys Thr Phe Phe Gly Leu Tyr Lys Leu
145                 150


<210>  130
<211>  154
<212>  PRT
<213>  Mus musculus

<220>
<223>  Amino acid sequence of soluble recombinant of mouse FasL

<400>  130

His His His His His His Pro Ser Thr Pro Ser Glu Lys Lys Glu Pro
1               5                   10                  15


Arg Ser Val Ala His Leu Thr Gly Asn Pro His Ser Arg Ser Ile Pro
            20                  25                  30


Leu Glu Trp Glu Asp Thr Tyr Gly Thr Ala Leu Ile Ser Gly Val Lys
        35                  40                  45


Tyr Lys Lys Gly Gly Leu Val Ile Asn Glu Thr Gly Leu Tyr Phe Val
    50                  55                  60


Tyr Ser Lys Val Tyr Phe Arg Gly Gln Ser Cys Asn Asn Gln Pro Leu
65                  70                  75                  80


Asn His Lys Val Tyr Met Arg Asn Ser Lys Tyr Pro Glu Asp Leu Val
                85                  90                  95

Leu Met Glu Glu Lys Arg Leu Asn Tyr Cys Thr Thr Gly Gln Ile Trp
            100                 105                 110

Ala His Ser Ser Tyr Leu Gly Ala Val Phe Asn Leu Thr Ser Ala Asp
        115                 120                 125

His Leu Tyr Val Asn Ile Ser Gln Leu Ser Leu Ile Asn Phe Glu Glu
    130                 135                 140

Ser Lys Thr Phe Phe Gly Leu Tyr Lys Leu
145                 150

<210>  131
<211>  501
<212>  DNA
<213>  Homo sapiens

<220>
<221>  CDS
<222>  (1)..(501)

<220>
<223>  Nucleotide sequence of extracellular region of human LIGHT (Asp74-
Val240)

<400>  131
gac gga cct gca ggc tcc tgg gag cag ctg ata caa gag cga agg tct
48
Asp Gly Pro Ala Gly Ser Trp Glu Gln Leu Ile Gln Glu Arg Arg Ser
1               5                   10                  15

cac gag gtc aac cca gca gcg cat ctc aca ggg gcc aac tcc agc ttg
96
His Glu Val Asn Pro Ala Ala His Leu Thr Gly Ala Asn Ser Ser Leu
            20                  25                  30

acc ggc agc ggg ggg ccg ctg tta tgg gag act cag ctg ggc ctg gcc
144
Thr Gly Ser Gly Gly Pro Leu Leu Trp Glu Thr Gln Leu Gly Leu Ala
        35                  40                  45

ttc ctg agg ggc ctc agc tac cac gat ggg gcc ctt gtg gtc acc aaa
192
Phe Leu Arg Gly Leu Ser Tyr His Asp Gly Ala Leu Val Val Thr Lys
    50                  55                  60

gct ggc tac tac tac atc tac tcc aag gtg cag ctg ggc ggt gtg ggc
240
Ala Gly Tyr Tyr Tyr Ile Tyr Ser Lys Val Gln Leu Gly Gly Val Gly
65                  70                  75                  80

tgc ccg ctg ggc ctg gcc agc acc atc acc cac ggc ctc tac aag cgc
288
Cys Pro Leu Gly Leu Ala Ser Thr Ile Thr His Gly Leu Tyr Lys Arg
                85                  90                  95

aca ccc cgc tac ccc gag gag ctg gag ctg ttg gtc agc cag cag tca
336
Thr Pro Arg Tyr Pro Glu Glu Leu Glu Leu Leu Val Ser Gln Gln Ser
            100                 105                 110

ccc tgc gga cgg gcc acc agc agc tcc cgg gtc tgg tgg gac agc agc
384
Pro Cys Gly Arg Ala Thr Ser Ser Ser Arg Val Trp Trp Asp Ser Ser
        115                 120                 125

ttc ctg ggt ggt gtg gta cac ctg gag gct ggg gag aag gtg gtc gtc
432
Phe Leu Gly Gly Val Val His Leu Glu Ala Gly Glu Lys Val Val Val
    130                 135                 140

cgt gtg ctg gat gaa cgc ctg gtt cga ctg cgt gat ggt acc cgg tct
480
Arg Val Leu Asp Glu Arg Leu Val Arg Leu Arg Asp Gly Thr Arg Ser
145                 150                 155                 160

tac ttc ggg gct ttc atg gtg
501
Tyr Phe Gly Ala Phe Met Val
                165

<210>  132
<211>  167
<212>  PRT
<213>  Homo sapiens

<220>
<223>  Amino acid sequence of extracellular region of human LIGHT (Asp74-
Val240)

<400>  132

Asp Gly Pro Ala Gly Ser Trp Glu Gln Leu Ile Gln Glu Arg Arg Ser
1               5                   10                  15

His Glu Val Asn Pro Ala Ala His Leu Thr Gly Ala Asn Ser Ser Leu
            20                  25                  30

Thr Gly Ser Gly Gly Pro Leu Leu Trp Glu Thr Gln Leu Gly Leu Ala
        35                  40                  45

Phe Leu Arg Gly Leu Ser Tyr His Asp Gly Ala Leu Val Val Thr Lys
    50                  55                  60

Ala Gly Tyr Tyr Tyr Ile Tyr Ser Lys Val Gln Leu Gly Gly Val Gly
65                  70                  75                  80

Cys Pro Leu Gly Leu Ala Ser Thr Ile Thr His Gly Leu Tyr Lys Arg
                85                  90                  95

Thr Pro Arg Tyr Pro Glu Glu Leu Glu Leu Leu Val Ser Gln Gln Ser
            100                 105                 110

Pro Cys Gly Arg Ala Thr Ser Ser Ser Arg Val Trp Trp Asp Ser Ser
        115                 120                 125

Phe Leu Gly Gly Val Val His Leu Glu Ala Gly Glu Lys Val Val Val
    130                 135                 140

Arg Val Leu Asp Glu Arg Leu Val Arg Leu Arg Asp Gly Thr Arg Ser
145                 150                 155                 160

Tyr Phe Gly Ala Phe Met Val
                165

<210>  133
<211>  501
<212>  DNA
<213>  Macaca fascicularis

<220>
<221>  CDS
<222>  (1)..(501)

<220>
<223>  Nucleotide sequence of extracellular region of Macaca fascicularis
LIGHT (Asp74-Val240)

<400>  133
gac gga cac gga ggc tcc tgg gag cag ctg ata caa gag cga agg tct
48
Asp Gly His Gly Gly Ser Trp Glu Gln Leu Ile Gln Glu Arg Arg Ser
1               5                   10                  15

cac cag aat aac cca gca gca cat ctc aca ggg gcc aac tcc agc ttg
96
His Gln Asn Asn Pro Ala Ala His Leu Thr Gly Ala Asn Ser Ser Leu
            20                  25                  30

acg ggc agc ggg gga ccg ctg cta tgg gag acc cag ctg ggc ctg gcc
144
Thr Gly Ser Gly Gly Pro Leu Leu Trp Glu Thr Gln Leu Gly Leu Ala
        35                  40                  45

ttc ctg agg ggc ctc gac tac cac gat ggg gcc ctt gtg gcc aac aag
192
Phe Leu Arg Gly Leu Asp Tyr His Asp Gly Ala Leu Val Ala Asn Lys
    50                  55                  60

gct ggc tac tac tac atc tac tcc aag gtg cag ctg ggc ggt gtg ggc
240
Ala Gly Tyr Tyr Tyr Ile Tyr Ser Lys Val Gln Leu Gly Gly Val Gly
65                  70                  75                  80

tgc ccg ctg ggc ctg gcc agc acc atc acc cac ggc ctc tac aag cgc
288
Cys Pro Leu Gly Leu Ala Ser Thr Ile Thr His Gly Leu Tyr Lys Arg
                85                  90                  95

acg ccc cgc tac ccc gag gag ctg gag ctg ttg gtc agc cag cag tca
336
Thr Pro Arg Tyr Pro Glu Glu Leu Glu Leu Leu Val Ser Gln Gln Ser
            100                 105                 110

ccc tgc gga cgg gcc acc agc aac tcc cgc gtc tgg tgg gac agc agc
384
Pro Cys Gly Arg Ala Thr Ser Asn Ser Arg Val Trp Trp Asp Ser Ser
        115                 120                 125

ttc ctg ggt ggt gtg gtg cac ctg gag gtt ggg gag gag gtg gtc gtc
432
Phe Leu Gly Gly Val Val His Leu Glu Val Gly Glu Glu Val Val Val
    130                 135                 140

cgt gtg ctg gat gag cgc ctg gtc cga ctg cgt gat ggc acc cgg tct
480
Arg Val Leu Asp Glu Arg Leu Val Arg Leu Arg Asp Gly Thr Arg Ser
145                 150                 155                 160

tac ttc ggg gct ttc atg gtg
501
Tyr Phe Gly Ala Phe Met Val
                165



<210>  134
<211>  167
<212>  PRT
<213>  Macaca fascicularis

<220>
<223>  Amino acid sequence of extracellular region of Macaca fascicularis
LIGHT (Asp74-Val240)

<400>  134

Asp Gly His Gly Gly Ser Trp Glu Gln Leu Ile Gln Glu Arg Arg Ser
1               5                   10                  15


His Gln Asn Asn Pro Ala Ala His Leu Thr Gly Ala Asn Ser Ser Leu
            20                  25                  30


Thr Gly Ser Gly Gly Pro Leu Leu Trp Glu Thr Gln Leu Gly Leu Ala
        35                  40                  45


Phe Leu Arg Gly Leu Asp Tyr His Asp Gly Ala Leu Val Ala Asn Lys
    50                  55                  60


Ala Gly Tyr Tyr Tyr Ile Tyr Ser Lys Val Gln Leu Gly Gly Val Gly
65                  70                  75                  80

Cys Pro Leu Gly Leu Ala Ser Thr Ile Thr His Gly Leu Tyr Lys Arg
                85                  90                  95

Thr Pro Arg Tyr Pro Glu Glu Leu Glu Leu Leu Val Ser Gln Gln Ser
            100                 105                 110

Pro Cys Gly Arg Ala Thr Ser Asn Ser Arg Val Trp Trp Asp Ser Ser
        115                 120                 125

Phe Leu Gly Gly Val Val His Leu Glu Val Gly Glu Glu Val Val Val
    130                 135                 140

Arg Val Leu Asp Glu Arg Leu Val Arg Leu Arg Asp Gly Thr Arg Ser
145                 150                 155                 160

Tyr Phe Gly Ala Phe Met Val
                165

<210>  135
<211>  504
<212>  DNA
<213>  Mus musculus

<220>
<221>  CDS
<222>  (1)..(504)

<220>
<223>  Nucleotide sequence of extracellular region of mouse LIGHT (Asp74-
Val240)

<400>  135
gat gga ggc aaa ggc tcc tgg gag aag ctg ata caa gat caa cga tct
48
Asp Gly Gly Lys Gly Ser Trp Glu Lys Leu Ile Gln Asp Gln Arg Ser
1               5                   10                  15

cac cag gcc aac cca gca gca cat ctt aca gga gcc aac gcc agc ttg
96
His Gln Ala Asn Pro Ala Ala His Leu Thr Gly Ala Asn Ala Ser Leu
            20                  25                  30

ata ggt att ggt gga cct ctg tta tgg gag aca cga ctt ggc ctg gcc
144
Ile Gly Ile Gly Gly Pro Leu Leu Trp Glu Thr Arg Leu Gly Leu Ala
        35                  40                  45

ttc ttg agg ggc ttg acg tat cat gat ggg gcc ctg gtg acc atg gag
192
Phe Leu Arg Gly Leu Thr Tyr His Asp Gly Ala Leu Val Thr Met Glu
    50                  55                  60

ccc ggt tac tac tat gtg tac tcc aaa gtg cag ctg agc ggc gtg ggc
240
Pro Gly Tyr Tyr Tyr Val Tyr Ser Lys Val Gln Leu Ser Gly Val Gly
65                  70                  75                  80

tgc ccc cag ggg ctg gcc aat ggc ctc ccc atc acc cat gga cta tac
288
Cys Pro Gln Gly Leu Ala Asn Gly Leu Pro Ile Thr His Gly Leu Tyr
                85                  90                  95

aag cgc aca tcc cgc tac ccg aag gag tta gaa ctg ctg gtc agt cgg
336
Lys Arg Thr Ser Arg Tyr Pro Lys Glu Leu Glu Leu Leu Val Ser Arg
            100                 105                 110

cgg tca ccc tgt ggc cgg gcc aac agc tcc cga gtc tgg tgg gac agc
384
Arg Ser Pro Cys Gly Arg Ala Asn Ser Ser Arg Val Trp Trp Asp Ser
        115                 120                 125

agc ttc ctg ggc ggc gtg gta cat ctg gag gct ggg gaa gag gtg gtg
432
Ser Phe Leu Gly Gly Val Val His Leu Glu Ala Gly Glu Glu Val Val
    130                 135                 140

gtc cgc gtg cct gga aac cgc ctg gtc aga cca cgt gac ggc acc agg
480
Val Arg Val Pro Gly Asn Arg Leu Val Arg Pro Arg Asp Gly Thr Arg
145                 150                 155                 160

tcc tat ttc gga gct ttc atg gtc
504
Ser Tyr Phe Gly Ala Phe Met Val
                165



<210>  136
<211>  168
<212>  PRT
<213>  Mus musculus

<220>
<223>  Amino acid sequence of extracellular region of mouse LIGHT (Asp74-
Val240)

<400>  136

Asp Gly Gly Lys Gly Ser Trp Glu Lys Leu Ile Gln Asp Gln Arg Ser
1               5                   10                  15


His Gln Ala Asn Pro Ala Ala His Leu Thr Gly Ala Asn Ala Ser Leu
            20                  25                  30


Ile Gly Ile Gly Gly Pro Leu Leu Trp Glu Thr Arg Leu Gly Leu Ala
        35                  40                  45


Phe Leu Arg Gly Leu Thr Tyr His Asp Gly Ala Leu Val Thr Met Glu

50                  55                  60

Pro Gly Tyr Tyr Tyr Val Tyr Ser Lys Val Gln Leu Ser Gly Val Gly
65                  70                  75                  80

Cys Pro Gln Gly Leu Ala Asn Gly Leu Pro Ile Thr His Gly Leu Tyr
                85                  90                  95

Lys Arg Thr Ser Arg Tyr Pro Lys Glu Leu Glu Leu Leu Val Ser Arg
            100                 105                 110

Arg Ser Pro Cys Gly Arg Ala Asn Ser Ser Arg Val Trp Trp Asp Ser
        115                 120                 125

Ser Phe Leu Gly Gly Val Val His Leu Glu Ala Gly Glu Glu Val Val
    130                 135                 140

Val Arg Val Pro Gly Asn Arg Leu Val Arg Pro Arg Asp Gly Thr Arg
145                 150                 155                 160

Ser Tyr Phe Gly Ala Phe Met Val
                165

<210>  137
<211>  540
<212>  DNA
<213>  Homo sapiens

<220>
<221>  CDS
<222>  (1)..(540)

<220>
<223>  Nucleotide sequence of extracellular region of human TL1A (Leu72-
Leu251)

<400>  137
cta aaa gga cag gag ttt gca cct tca cat cag caa gtt tat gca cct
48
Leu Lys Gly Gln Glu Phe Ala Pro Ser His Gln Gln Val Tyr Ala Pro
1               5                   10                  15

ctt aga gca gac gga gat aag cca agg gca cac ctg aca gtt gtg aga
96
Leu Arg Ala Asp Gly Asp Lys Pro Arg Ala His Leu Thr Val Val Arg
            20                  25                  30

caa act ccc aca cag cac ttt aaa aat cag ttc cca gct ctg cac tgg
144
Gln Thr Pro Thr Gln His Phe Lys Asn Gln Phe Pro Ala Leu His Trp
        35                  40                  45

gaa cat gaa cta ggc ctg gcc ttc acc aag aac cga atg aac tat acc
192
Glu His Glu Leu Gly Leu Ala Phe Thr Lys Asn Arg Met Asn Tyr Thr
    50                  55                  60

aac aaa ttc ctg ctg atc cca gag tcg gga gac tac ttc att tac tcc
240
Asn Lys Phe Leu Leu Ile Pro Glu Ser Gly Asp Tyr Phe Ile Tyr Ser
65                  70                  75                  80

cag gtc aca ttc cgt ggg atg acc tct gag tgc agt gaa atc aga caa
288
Gln Val Thr Phe Arg Gly Met Thr Ser Glu Cys Ser Glu Ile Arg Gln
                85                  90                  95

gca ggc cga cca aac aag cca gac tcc atc act gtg gtc atc acc aag
336
Ala Gly Arg Pro Asn Lys Pro Asp Ser Ile Thr Val Val Ile Thr Lys
            100                 105                 110

gta aca gac agc tac cct gag cca acc cag ctc ctc atg ggg acc aag
384
Val Thr Asp Ser Tyr Pro Glu Pro Thr Gln Leu Leu Met Gly Thr Lys
        115                 120                 125

tct gta tgc gaa gta ggt agc aac tgg ttc cag ccc atc tac ctc gga
432
Ser Val Cys Glu Val Gly Ser Asn Trp Phe Gln Pro Ile Tyr Leu Gly
    130                 135                 140

gcc atg ttc tcc ttg caa gaa ggg gac aag cta atg gtg aac gtc agt
480
Ala Met Phe Ser Leu Gln Glu Gly Asp Lys Leu Met Val Asn Val Ser
145                 150                 155                 160

gac atc tct ttg gtg gat tac aca aaa gaa gat aaa acc ttc ttt gga
528
Asp Ile Ser Leu Val Asp Tyr Thr Lys Glu Asp Lys Thr Phe Phe Gly
                165                 170                 175

gcc ttc tta cta
540
Ala Phe Leu Leu
            180



<210>  138
<211>  180
<212>  PRT
<213>  Homo sapiens

<220>
<223>  Amino acid sequence of extracellular region of human TL1A (Leu72-
Leu251)

<400>  138

Leu Lys Gly Gln Glu Phe Ala Pro Ser His Gln Gln Val Tyr Ala Pro
1               5                   10                  15

Leu Arg Ala Asp Gly Asp Lys Pro Arg Ala His Leu Thr Val Val Arg
            20                  25                  30

Gln Thr Pro Thr Gln His Phe Lys Asn Gln Phe Pro Ala Leu His Trp
        35                  40                  45

Glu His Glu Leu Gly Leu Ala Phe Thr Lys Asn Arg Met Asn Tyr Thr
    50                  55                  60

Asn Lys Phe Leu Leu Ile Pro Glu Ser Gly Asp Tyr Phe Ile Tyr Ser
65                  70                  75                  80

Gln Val Thr Phe Arg Gly Met Thr Ser Glu Cys Ser Glu Ile Arg Gln
                85                  90                  95

Ala Gly Arg Pro Asn Lys Pro Asp Ser Ile Thr Val Val Ile Thr Lys
            100                 105                 110

Val Thr Asp Ser Tyr Pro Glu Pro Thr Gln Leu Leu Met Gly Thr Lys
        115                 120                 125

Ser Val Cys Glu Val Gly Ser Asn Trp Phe Gln Pro Ile Tyr Leu Gly
    130                 135                 140

Ala Met Phe Ser Leu Gln Glu Gly Asp Lys Leu Met Val Asn Val Ser
145                 150                 155                 160

Asp Ile Ser Leu Val Asp Tyr Thr Lys Glu Asp Lys Thr Phe Phe Gly
                165                 170                 175

Ala Phe Leu Leu
            180

<210>  139
<211>  540
<212>  DNA
<213>  Macaca fascicularis

<220>
<221>  CDS
<222>  (1)..(540)

<220>
<223>  Nucleotide sequence of extracellular region of Macaca fascicularis
TL1A (Leu72-Leu251)

<400>  139

cta aaa gga cag gag ttt gca cct tca cat cag caa gtt tat gca cct
48
Leu Lys Gly Gln Glu Phe Ala Pro Ser His Gln Gln Val Tyr Ala Pro
1               5                   10                  15

ctt aga gca gat gga gat aag cca agg gca cac ctg aca gtt gtg aga
96
Leu Arg Ala Asp Gly Asp Lys Pro Arg Ala His Leu Thr Val Val Arg
            20                  25                  30

caa act ccc aca cag cac tta aaa aat cag ttc cca gct ctg cac tgg
144
Gln Thr Pro Thr Gln His Leu Lys Asn Gln Phe Pro Ala Leu His Trp
        35                  40                  45

gaa cat gaa cta ggc ctg gcc ttc acc aag aac cga atg aac tat acc
192
Glu His Glu Leu Gly Leu Ala Phe Thr Lys Asn Arg Met Asn Tyr Thr
    50                  55                  60

aac aaa ttc ctg ctg atc cca gag tcg gga gac tac ttc gtt tac tcc
240
Asn Lys Phe Leu Leu Ile Pro Glu Ser Gly Asp Tyr Phe Val Tyr Ser
65                  70                  75                  80

cag gtc aca ttc cgt ggg atg acc tct gag tgc agt gaa atc aga caa
288
Gln Val Thr Phe Arg Gly Met Thr Ser Glu Cys Ser Glu Ile Arg Gln
                85                  90                  95

gca ggc cga cca aac aag cca gac tcc atc act gtg gtc atc acc aag
336
Ala Gly Arg Pro Asn Lys Pro Asp Ser Ile Thr Val Val Ile Thr Lys
            100                 105                 110

gta aca gac agc tac cct gag cca acc cag ctc ctc atg ggg acc aag
384
Val Thr Asp Ser Tyr Pro Glu Pro Thr Gln Leu Leu Met Gly Thr Lys
        115                 120                 125

tct gtg tgt gaa gta ggc agt aac tgg ttc cag ccc atc tac ctc gga
432
Ser Val Cys Glu Val Gly Ser Asn Trp Phe Gln Pro Ile Tyr Leu Gly
    130                 135                 140

gcc atg ttc tcc ttg caa gaa ggg gac aag ctc atg gtg aac gtc agt
480
Ala Met Phe Ser Leu Gln Glu Gly Asp Lys Leu Met Val Asn Val Ser
145                 150                 155                 160

gac atc tct ttg gtg gat tac aca aaa gaa gat aaa acc ttc ttt gga
528
Asp Ile Ser Leu Val Asp Tyr Thr Lys Glu Asp Lys Thr Phe Phe Gly
                165                 170                 175

gcc ttc tta cta
540
Ala Phe Leu Leu
            180

<210>  140
<211>  180
<212>  PRT
<213>  Macaca fascicularis

<220>
<223>  Amino acid sequence of extracellular region of Macaca fascicularis
TL1A (Leu72-Leu251)

<400>  140

Leu Lys Gly Gln Glu Phe Ala Pro Ser His Gln Gln Val Tyr Ala Pro
1               5                   10                  15

Leu Arg Ala Asp Gly Asp Lys Pro Arg Ala His Leu Thr Val Val Arg
            20                  25                  30

Gln Thr Pro Thr Gln His Leu Lys Asn Gln Phe Pro Ala Leu His Trp
        35                  40                  45

Glu His Glu Leu Gly Leu Ala Phe Thr Lys Asn Arg Met Asn Tyr Thr
    50                  55                  60

Asn Lys Phe Leu Leu Ile Pro Glu Ser Gly Asp Tyr Phe Val Tyr Ser
65                  70                  75                  80

Gln Val Thr Phe Arg Gly Met Thr Ser Glu Cys Ser Glu Ile Arg Gln
                85                  90                  95

Ala Gly Arg Pro Asn Lys Pro Asp Ser Ile Thr Val Val Ile Thr Lys
            100                 105                 110

Val Thr Asp Ser Tyr Pro Glu Pro Thr Gln Leu Leu Met Gly Thr Lys
        115                 120                 125

Ser Val Cys Glu Val Gly Ser Asn Trp Phe Gln Pro Ile Tyr Leu Gly
    130                 135                 140

Ala Met Phe Ser Leu Gln Glu Gly Asp Lys Leu Met Val Asn Val Ser
145                 150                 155                 160

Asp Ile Ser Leu Val Asp Tyr Thr Lys Glu Asp Lys Thr Phe Phe Gly
                165                 170                 175

Ala Phe Leu Leu
            180

<210>  141
<211>  531
<212>  DNA
<213>  Mus musculus

<220>
<221>  CDS
<222>  (1)..(531)

<220>
<223>  Nucleotide sequence of extracellular region of mouse TL1A (Ile94-
Leu270)

<400>  141
ata aca gaa gag agg tct gag cct tca cca cag caa gtt tac tca cct
48
Ile Thr Glu Glu Arg Ser Glu Pro Ser Pro Gln Gln Val Tyr Ser Pro
1               5                   10                  15

ccc aga ggc aag ccg aga gca cac ctg aca att aag aaa caa acc cca
96
Pro Arg Gly Lys Pro Arg Ala His Leu Thr Ile Lys Lys Gln Thr Pro
            20                  25                  30

gca cca cat ctg aaa aat cag ctc tct gct cta cac tgg gaa cat gac
144
Ala Pro His Leu Lys Asn Gln Leu Ser Ala Leu His Trp Glu His Asp
        35                  40                  45

cta ggg atg gcc ttc acc aag aac ggg atg aag tac atc aac aaa tcc
192
Leu Gly Met Ala Phe Thr Lys Asn Gly Met Lys Tyr Ile Asn Lys Ser
    50                  55                  60

ctg gtg atc cca gag tca gga gac tat ttc atc tac tcc cag atc aca
240
Leu Val Ile Pro Glu Ser Gly Asp Tyr Phe Ile Tyr Ser Gln Ile Thr
65                  70                  75                  80

ttc cga ggg acc aca tct gtg tgt ggt gac atc agt cgg ggg aga cga
288
Phe Arg Gly Thr Thr Ser Val Cys Gly Asp Ile Ser Arg Gly Arg Arg
                85                  90                  95

cca aac aag cca gac tcc atc acc atg gtt atc acc aag gta gca gac
336
Pro Asn Lys Pro Asp Ser Ile Thr Met Val Ile Thr Lys Val Ala Asp
            100                 105                 110

agc tac cct gag cct gcc cgc cta cta aca ggg tcc aag tct gtg tgt
384
Ser Tyr Pro Glu Pro Ala Arg Leu Leu Thr Gly Ser Lys Ser Val Cys
        115                 120                 125

gaa ata agc aac aac tgg ttc cag tcc ctc tac ctt ggg gcc acg ttc
432
Glu Ile Ser Asn Asn Trp Phe Gln Ser Leu Tyr Leu Gly Ala Thr Phe
    130                 135                 140

tcc ttg gaa gaa gga gac aga cta atg gta aac gtc agt gac atc tcc
480
Ser Leu Glu Glu Gly Asp Arg Leu Met Val Asn Val Ser Asp Ile Ser
145                 150                 155                 160

ttg gtg gat tac aca aaa gaa gat aaa act ttc ttt gga gct ttc ttg
528
Leu Val Asp Tyr Thr Lys Glu Asp Lys Thr Phe Phe Gly Ala Phe Leu
                165                 170                 175

cta
531
Leu

<210>  142
<211>  177
<212>  PRT
<213>  Mus musculus

<220>
<223>  Amino acid sequence of extracellular region of mouse TL1A (Ile94-
Leu270)

<400>  142

Ile Thr Glu Glu Arg Ser Glu Pro Ser Pro Gln Gln Val Tyr Ser Pro
1               5                   10                  15

Pro Arg Gly Lys Pro Arg Ala His Leu Thr Ile Lys Lys Gln Thr Pro
            20                  25                  30

Ala Pro His Leu Lys Asn Gln Leu Ser Ala Leu His Trp Glu His Asp
        35                  40                  45

Leu Gly Met Ala Phe Thr Lys Asn Gly Met Lys Tyr Ile Asn Lys Ser
    50                  55                  60

Leu Val Ile Pro Glu Ser Gly Asp Tyr Phe Ile Tyr Ser Gln Ile Thr
65                  70                  75                  80

Phe Arg Gly Thr Thr Ser Val Cys Gly Asp Ile Ser Arg Gly Arg Arg
                85                  90                  95

Pro Asn Lys Pro Asp Ser Ile Thr Met Val Ile Thr Lys Val Ala Asp
            100                 105                 110

Ser Tyr Pro Glu Pro Ala Arg Leu Leu Thr Gly Ser Lys Ser Val Cys
        115                 120                 125

Glu Ile Ser Asn Asn Trp Phe Gln Ser Leu Tyr Leu Gly Ala Thr Phe

130                 135                 140

Ser Leu Glu Glu Gly Asp Arg Leu Met Val Asn Val Ser Asp Ile Ser
145                 150                 155                 160

Leu Val Asp Tyr Thr Lys Glu Asp Lys Thr Phe Phe Gly Ala Phe Leu
                165                 170                 175

Leu

<210>  143
<211>  444
<212>  DNA
<213>  Homo sapiens

<220>
<221>  CDS
<222>  (1)..(444)

<220>
<223>  Nucleotide sequence of extracellular region of human FasL (Pro134-
Leu281)

<400>  143
ccc agt cca ccc cct gaa aaa aag gag ctg agg aaa gtg gcc cat tta
48
Pro Ser Pro Pro Pro Glu Lys Lys Glu Leu Arg Lys Val Ala His Leu
1               5                   10                  15

aca ggc aag tcc aac tca agg tcc atg cct ctg gaa tgg gaa gac acc
96
Thr Gly Lys Ser Asn Ser Arg Ser Met Pro Leu Glu Trp Glu Asp Thr
            20                  25                  30

tat gga att gtc ctg ctt tct gga gtg aag tat aag aag ggt ggc ctt
144
Tyr Gly Ile Val Leu Leu Ser Gly Val Lys Tyr Lys Lys Gly Gly Leu
        35                  40                  45

gtg atc aat gaa act ggg ctg tac ttt gta tat tcc aaa gta tac ttc
192
Val Ile Asn Glu Thr Gly Leu Tyr Phe Val Tyr Ser Lys Val Tyr Phe
    50                  55                  60

cgg ggt caa tct tgc aac aac ctg ccc ctg agc cac aag gtc tac atg
240
Arg Gly Gln Ser Cys Asn Asn Leu Pro Leu Ser His Lys Val Tyr Met
65                  70                  75                  80

agg aac tct aag tat ccc cag gat ctg gtg atg atg gag ggg aag atg
288
Arg Asn Ser Lys Tyr Pro Gln Asp Leu Val Met Met Glu Gly Lys Met
                85                  90                  95

atg agc tac tgc act act ggg cag atg tgg gcc cgc agc agc tac ctg
336
Met Ser Tyr Cys Thr Thr Gly Gln Met Trp Ala Arg Ser Ser Tyr Leu
            100                 105                 110

ggg gca gtg ttc aat ctt acc agt gct gat cat tta tat gtc aac gta
384
Gly Ala Val Phe Asn Leu Thr Ser Ala Asp His Leu Tyr Val Asn Val
        115                 120                 125

tct gag ctc tct ctg gtc aat ttt gag gaa tct cag acg ttt ttc ggc
432
Ser Glu Leu Ser Leu Val Asn Phe Glu Glu Ser Gln Thr Phe Phe Gly
    130                 135                 140

tta tat aag ctc
444
Leu Tyr Lys Leu
145

<210>  144
<211>  148
<212>  PRT
<213>  Homo sapiens

<220>
<223>  Amino acid sequence of extracellular region of human FasL (Pro134-
Leu281)

<400>  144

Pro Ser Pro Pro Pro Glu Lys Lys Glu Leu Arg Lys Val Ala His Leu
1               5                   10                  15

Thr Gly Lys Ser Asn Ser Arg Ser Met Pro Leu Glu Trp Glu Asp Thr
            20                  25                  30

Tyr Gly Ile Val Leu Leu Ser Gly Val Lys Tyr Lys Lys Gly Gly Leu
        35                  40                  45

Val Ile Asn Glu Thr Gly Leu Tyr Phe Val Tyr Ser Lys Val Tyr Phe
    50                  55                  60

Arg Gly Gln Ser Cys Asn Asn Leu Pro Leu Ser His Lys Val Tyr Met
65                  70                  75                  80

Arg Asn Ser Lys Tyr Pro Gln Asp Leu Val Met Met Glu Gly Lys Met
                85                  90                  95

Met Ser Tyr Cys Thr Thr Gly Gln Met Trp Ala Arg Ser Ser Tyr Leu
            100                 105                 110

Gly Ala Val Phe Asn Leu Thr Ser Ala Asp His Leu Tyr Val Asn Val
        115                 120                 125

Ser Glu Leu Ser Leu Val Asn Phe Glu Glu Ser Gln Thr Phe Phe Gly
    130                 135                 140

Leu Tyr Lys Leu
145

<210>  145
<211>  444
<212>  DNA
<213>  Macaca fascicularis

<220>
<221>  CDS
<222>  (1)..(444)

<220>
<223>  Nucleotide sequence of extracellular region of Macaca fascicularis
FasL (Pro133-Leu280)

<400>  145
ccc agt cca ccc cct gag aaa aag gag cag agg aaa gtg gcc cat tta
48
Pro Ser Pro Pro Pro Glu Lys Lys Glu Gln Arg Lys Val Ala His Leu
1               5                   10                  15

aca ggc aag ccc aac tca agg tcc atg cct ctg gaa tgg gaa gac acc
96
Thr Gly Lys Pro Asn Ser Arg Ser Met Pro Leu Glu Trp Glu Asp Thr
            20                  25                  30

tat gga att gtc ctg ctt tct gga gtg aag tat aag aag ggt ggc ctt
144
Tyr Gly Ile Val Leu Leu Ser Gly Val Lys Tyr Lys Lys Gly Gly Leu
        35                  40                  45

gtg atc aat gaa act ggg ctg tac ttt gta tat tcc aaa gta tac ttc
192
Val Ile Asn Glu Thr Gly Leu Tyr Phe Val Tyr Ser Lys Val Tyr Phe
    50                  55                  60

cgg ggt caa tct tgc acc aac ctg ccc ctg agc cac aag gtc tac atg
240
Arg Gly Gln Ser Cys Thr Asn Leu Pro Leu Ser His Lys Val Tyr Met
65                  70                  75                  80

agg aac tct aag tat ccc cag gat ctg gtg atg atg gag ggg aag atg
288
Arg Asn Ser Lys Tyr Pro Gln Asp Leu Val Met Met Glu Gly Lys Met
                85                  90                  95

atg agc tac tgc act act ggg cag atg tgg gcc cac agc agc tac ctg
336
Met Ser Tyr Cys Thr Thr Gly Gln Met Trp Ala His Ser Ser Tyr Leu
            100                 105                 110

ggg gca gtg ttc aat ctt acc agt gct gat cat tta tat gtc aat gta
384
Gly Ala Val Phe Asn Leu Thr Ser Ala Asp His Leu Tyr Val Asn Val
        115                 120                 125

tct gag ctc tct ctg gtc aat ttt gag gaa tct cag aca ttt ttt ggc
432
Ser Glu Leu Ser Leu Val Asn Phe Glu Glu Ser Gln Thr Phe Phe Gly
    130                 135                 140

cta tat aag ctc
444
Leu Tyr Lys Leu
145

<210>  146
<211>  148
<212>  PRT
<213>  Macaca fascicularis

<220>
<223>  Nucleotide sequence of extracellular region of Macaca fascicularis
FasL (Pro133-Leu280)

<400>  146

Pro Ser Pro Pro Pro Glu Lys Lys Glu Gln Arg Lys Val Ala His Leu
1               5                   10                  15

Thr Gly Lys Pro Asn Ser Arg Ser Met Pro Leu Glu Trp Glu Asp Thr
            20                  25                  30

Tyr Gly Ile Val Leu Leu Ser Gly Val Lys Tyr Lys Lys Gly Gly Leu
        35                  40                  45

Val Ile Asn Glu Thr Gly Leu Tyr Phe Val Tyr Ser Lys Val Tyr Phe
    50                  55                  60

Arg Gly Gln Ser Cys Thr Asn Leu Pro Leu Ser His Lys Val Tyr Met
65                  70                  75                  80

Arg Asn Ser Lys Tyr Pro Gln Asp Leu Val Met Met Glu Gly Lys Met
                85                  90                  95

Met Ser Tyr Cys Thr Thr Gly Gln Met Trp Ala His Ser Ser Tyr Leu
            100                 105                 110

Gly Ala Val Phe Asn Leu Thr Ser Ala Asp His Leu Tyr Val Asn Val
        115                 120                 125

Ser Glu Leu Ser Leu Val Asn Phe Glu Glu Ser Gln Thr Phe Phe Gly
    130                 135                 140

Leu Tyr Lys Leu
145

<210>  147
<211>  444
<212>  DNA
<213>  Mus musculus

<220>
<221>  CDS
<222>  (1)..(444)

<220>
<223>  Nucleotide sequence of extracellular region of mouse FasL (Pro132-
Leu279)

<400>  147
ccc agt aca ccc tct gaa aaa aaa gag ccg agg agt gtg gcc cat tta
48
Pro Ser Thr Pro Ser Glu Lys Lys Glu Pro Arg Ser Val Ala His Leu
1               5                   10                  15

aca ggg aac ccc cac tca agg tcc atc cct ctg gaa tgg gaa gac aca
96
Thr Gly Asn Pro His Ser Arg Ser Ile Pro Leu Glu Trp Glu Asp Thr
            20                  25                  30

tat gga acc gct ctg atc tct gga gtg aag tat aag aaa ggt ggc ctt
144
Tyr Gly Thr Ala Leu Ile Ser Gly Val Lys Tyr Lys Lys Gly Gly Leu
        35                  40                  45

gtg atc aac gaa act ggg ttg tac ttc gtg tat tcc aaa gta tac ttc
192
Val Ile Asn Glu Thr Gly Leu Tyr Phe Val Tyr Ser Lys Val Tyr Phe
    50                  55                  60

cgg ggt cag tct tgc aac aac cag ccc cta aac cac aag gtc tat atg
240
Arg Gly Gln Ser Cys Asn Asn Gln Pro Leu Asn His Lys Val Tyr Met
65                  70                  75                  80

agg aac tct aag tat cct gag gat ctg gtg cta atg gag gag aag agg
288
Arg Asn Ser Lys Tyr Pro Glu Asp Leu Val Leu Met Glu Glu Lys Arg
                85                  90                  95

ttg aac tac tgc act act gga cag ata tgg gcc cac agc agc tac ctg
336
Leu Asn Tyr Cys Thr Thr Gly Gln Ile Trp Ala His Ser Ser Tyr Leu
            100                 105                 110

ggg gca gta ttc aat ctt acc agt gct gac cat tta tat gtc aac ata
384
Gly Ala Val Phe Asn Leu Thr Ser Ala Asp His Leu Tyr Val Asn Ile

115                 120                 125

tct caa ctc tct ctg atc aat ttt gag gaa tct aag acc ttt ttc ggc
432
Ser Gln Leu Ser Leu Ile Asn Phe Glu Glu Ser Lys Thr Phe Phe Gly
    130                 135                 140

tta tat aag ctc
444
Leu Tyr Lys Leu
145

<210>  148
<211>  148
<212>  PRT
<213>  Mus musculus

<220>
<223>  Amino acid sequence of extracellular region of mouse FasL (Pro132-
Leu279)

<400>  148

Pro Ser Thr Pro Ser Glu Lys Lys Glu Pro Arg Ser Val Ala His Leu
1               5                   10                  15

Thr Gly Asn Pro His Ser Arg Ser Ile Pro Leu Glu Trp Glu Asp Thr
            20                  25                  30

Tyr Gly Thr Ala Leu Ile Ser Gly Val Lys Tyr Lys Lys Gly Gly Leu
        35                  40                  45

Val Ile Asn Glu Thr Gly Leu Tyr Phe Val Tyr Ser Lys Val Tyr Phe
    50                  55                  60

Arg Gly Gln Ser Cys Asn Asn Gln Pro Leu Asn His Lys Val Tyr Met
65                  70                  75                  80

Arg Asn Ser Lys Tyr Pro Glu Asp Leu Val Leu Met Glu Glu Lys Arg
                85                  90                  95

Leu Asn Tyr Cys Thr Thr Gly Gln Ile Trp Ala His Ser Ser Tyr Leu
            100                 105                 110

Gly Ala Val Phe Asn Leu Thr Ser Ala Asp His Leu Tyr Val Asn Ile
        115                 120                 125

Ser Gln Leu Ser Leu Ile Asn Phe Glu Glu Ser Lys Thr Phe Phe Gly
    130                 135                 140

Leu Tyr Lys Leu
145

<210> 149
<211> 1212
<212> DNA
<213> Artificial Sequence

<220>
<221> CDS
<222> (1)..(1212)

<220>
<223> Nucleotide sequence of Chimera A-Fc(g1S)

<400> 149
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn

130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag ccc aaa tct
528
Asp Asn Ile Cys Ser Gly Asn Ser Glu Ser Thr Gln Lys Pro Lys Ser
                165                 170                 175

tcc gac aaa act cac aca tgc cca ccg tgc cca gca cct gaa ctc ctg
576
Ser Asp Lys Thr His Thr Cys Pro Pro Cys Pro Ala Pro Glu Leu Leu
            180                 185                 190

ggg gga ccg tca gtc ttc ctc ttc ccc cca aaa ccc aag gac acc ctc
624
Gly Gly Pro Ser Val Phe Leu Phe Pro Pro Lys Pro Lys Asp Thr Leu
        195                 200                 205

atg atc tcc cgg acc cct gag gtc aca tgc gtg gtg gtg gac gtg agc
672
Met Ile Ser Arg Thr Pro Glu Val Thr Cys Val Val Val Asp Val Ser
    210                 215                 220

cac gaa gac cct gag gtc aag ttc aac tgg tac gtg gac ggc gtg gag
720
His Glu Asp Pro Glu Val Lys Phe Asn Trp Tyr Val Asp Gly Val Glu
225                 230                 235                 240

gtg cat aat gcc aag aca aag ccg cgg gag gag cag tac aac agc acg
768
Val His Asn Ala Lys Thr Lys Pro Arg Glu Glu Gln Tyr Asn Ser Thr
                245                 250                 255

tac cgt gtg gtc agc gtc ctc acc gtc ctg cac cag gac tgg ctg aat
816
Tyr Arg Val Val Ser Val Leu Thr Val Leu His Gln Asp Trp Leu Asn
            260                 265                 270

ggc aag gag tac aag tgc aag gtc tcc aac aaa gcc ctc cca gcc ccc
864
Gly Lys Glu Tyr Lys Cys Lys Val Ser Asn Lys Ala Leu Pro Ala Pro
        275                 280                 285

atc gag aaa acc atc tcc aaa gcc aaa ggg cag ccc cga gaa cca cag
912
Ile Glu Lys Thr Ile Ser Lys Ala Lys Gly Gln Pro Arg Glu Pro Gln
    290                 295                 300

gtg tac acc ctg ccc cca tcc cgg gat gag ctg acc aag aac cag gtc
960
Val Tyr Thr Leu Pro Pro Ser Arg Asp Glu Leu Thr Lys Asn Gln Val
305                 310                 315                 320

agc ctg acc tgc ctg gtc aaa ggc ttc tat ccc agc gac atc gcc gtg
1008
Ser Leu Thr Cys Leu Val Lys Gly Phe Tyr Pro Ser Asp Ile Ala Val

                325                 330                 335

gag tgg gag agc aat ggg cag ccg gag aac aac tac aag acc acg cct
1056
Glu Trp Glu Ser Asn Gly Gln Pro Glu Asn Asn Tyr Lys Thr Thr Pro
            340                 345                 350

ccc gtg ctg gac tcc gac ggc tcc ttc ttc ctc tac agc aag ctc acc
1104
Pro Val Leu Asp Ser Asp Gly Ser Phe Phe Leu Tyr Ser Lys Leu Thr
        355                 360                 365

gtg gac aag agc agg tgg cag cag ggg aac gtc ttc tca tgc tcc gtg
1152
Val Asp Lys Ser Arg Trp Gln Gln Gly Asn Val Phe Ser Cys Ser Val
    370                 375                 380

atg cat gag gct ctg cac aac cac tac acg cag aag agc ctc tcc ctg
1200
Met His Glu Ala Leu His Asn His Tyr Thr Gln Lys Ser Leu Ser Leu
385                 390                 395                 400

tct ccg ggt aaa
1212
Ser Pro Gly Lys

<210> 150
<211> 404
<212> PRT
<213> Artificial Sequence

<220>
<223> Amino acid sequence of Chimera A-Fc(g1S)

<400> 150

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210> 151
<211> 1293
<212> DNA
<213> Artificial Sequence

<220>
<221> CDS
<222> (1)..(1293)

<220>
<223> Nucleotide sequence of OPG K194-Fc

<400> 151
atg aac aac ttg ctg tgc tgc gcg ctc gtg ttt ctg gac atc tcc att
48
Met Asn Asn Leu Leu Cys Cys Ala Leu Val Phe Leu Asp Ile Ser Ile
1               5                   10                  15

aag tgg acc acc cag gaa acg ttt cct cca aag tac ctt cat tat gac
96
Lys Trp Thr Thr Gln Glu Thr Phe Pro Pro Lys Tyr Leu His Tyr Asp
            20                  25                  30

gaa gaa acc tct cat cag ctg ttg tgt gac aaa tgt cct cct ggt acc
144
Glu Glu Thr Ser His Gln Leu Leu Cys Asp Lys Cys Pro Pro Gly Thr
        35                  40                  45

tac cta aaa caa cac tgt aca gca aag tgg aag acc gtg tgc gcc cct
192
Tyr Leu Lys Gln His Cys Thr Ala Lys Trp Lys Thr Val Cys Ala Pro
    50                  55                  60

tgc cct gac cac tac tac aca gac agc tgg cac acc agt gac gag tgt
240
Cys Pro Asp His Tyr Tyr Thr Asp Ser Trp His Thr Ser Asp Glu Cys
65                  70                  75                  80

cta tac tgc agc ccc gtg tgc aag gag ctg cag tac gtc aag cag gag
288
Leu Tyr Cys Ser Pro Val Cys Lys Glu Leu Gln Tyr Val Lys Gln Glu
                85                  90                  95

tgc aat cgc acc cac aac cgc gtg tgc gaa tgc aag gaa ggg cgc tac
336
Cys Asn Arg Thr His Asn Arg Val Cys Glu Cys Lys Glu Gly Arg Tyr
            100                 105                 110

ctt gag ata gag ttc tgc ttg aaa cat agg agc tgc cct cct gga ttt
384
Leu Glu Ile Glu Phe Cys Leu Lys His Arg Ser Cys Pro Pro Gly Phe
        115                 120                 125

gga gtg gtg caa gct gga acc cca gag cga aat aca gtt tgc aaa aga
432
Gly Val Val Gln Ala Gly Thr Pro Glu Arg Asn Thr Val Cys Lys Arg
    130                 135                 140

tgt cca gat ggg ttc ttc tca aat gag acg tca tct aaa gca ccc tgt
480
Cys Pro Asp Gly Phe Phe Ser Asn Glu Thr Ser Ser Lys Ala Pro Cys
145                 150                 155                 160

aga aaa cac aca aat tgc agt gtc ttt ggt ctc ctg cta act cag aaa
528
Arg Lys His Thr Asn Cys Ser Val Phe Gly Leu Leu Leu Thr Gln Lys
                165                 170                 175

gga aat gca aca cac gac aac ata tgt tcc gga aac agt gaa tca act
576
Gly Asn Ala Thr His Asp Asn Ile Cys Ser Gly Asn Ser Glu Ser Thr
            180                 185                 190

caa aaa atc gag ggg cgc atg gat ccc aaa tct tcc gac aaa act cac
624
Gln Lys Ile Glu Gly Arg Met Asp Pro Lys Ser Ser Asp Lys Thr His
        195                 200                 205

aca tgc cca ccg tgc cca gca cct gaa ctc ctg ggg gga ccg tca gtc
672
Thr Cys Pro Pro Cys Pro Ala Pro Glu Leu Leu Gly Gly Pro Ser Val
    210                 215                 220

ttc ctc ttc ccc cca aaa ccc aag gac acc ctc atg atc tcc cgg acc
720
Phe Leu Phe Pro Pro Lys Pro Lys Asp Thr Leu Met Ile Ser Arg Thr
225                 230                 235                 240

cct gag gtc aca tgc gtg gtg gtg gac gtg agc cac gaa gac cct gag
768
Pro Glu Val Thr Cys Val Val Val Asp Val Ser His Glu Asp Pro Glu
                245                 250                 255

gtc aag ttc aac tgg tac gtg gac ggc gtg gag gtg cat aat gcc aag
816
Val Lys Phe Asn Trp Tyr Val Asp Gly Val Glu Val His Asn Ala Lys
            260                 265                 270

aca aag ccg cgg gag gag cag tac aac agc acg tac cgt gtg gtc agc
864
Thr Lys Pro Arg Glu Glu Gln Tyr Asn Ser Thr Tyr Arg Val Val Ser
        275                 280                 285

gtc ctc acc gtc ctg cac cag gac tgg ctg aat ggc aag gag tac aag
912
Val Leu Thr Val Leu His Gln Asp Trp Leu Asn Gly Lys Glu Tyr Lys
    290                 295                 300

tgc aag gtc tcc aac aaa gcc ctc cca gcc ccc atc gag aaa acc atc
960
Cys Lys Val Ser Asn Lys Ala Leu Pro Ala Pro Ile Glu Lys Thr Ile
305                 310                 315                 320

tcc aaa gcc aaa ggg cag ccc cga gaa cca cag gtg tac acc ctg ccc
1008
Ser Lys Ala Lys Gly Gln Pro Arg Glu Pro Gln Val Tyr Thr Leu Pro
                325                 330                 335

cca tcc cgg gat gag ctg acc aag aac cag gtc agc ctg acc tgc ctg
1056
Pro Ser Arg Asp Glu Leu Thr Lys Asn Gln Val Ser Leu Thr Cys Leu
            340                 345                 350

gtc aaa ggc ttc tat ccc agc gac atc gcc gtg gag tgg gag agc aat
1104
Val Lys Gly Phe Tyr Pro Ser Asp Ile Ala Val Glu Trp Glu Ser Asn
        355                 360                 365

ggg cag ccg gag aac aac tac aag acc acg cct ccc gtg ctg gac tcc
1152
Gly Gln Pro Glu Asn Asn Tyr Lys Thr Thr Pro Pro Val Leu Asp Ser
    370                 375                 380

gac ggc tcc ttc ttc ctc tac agc aag ctc acc gtg gac aag agc agg
1200
Asp Gly Ser Phe Phe Leu Tyr Ser Lys Leu Thr Val Asp Lys Ser Arg
385                 390                 395                 400

tgg cag cag ggg aac gtc ttc tca tgc tcc gtg atg cat gag gct ctg
1248
Trp Gln Gln Gly Asn Val Phe Ser Cys Ser Val Met His Glu Ala Leu
                405                 410                 415

cac aac cac tac acg cag aag agc ctc tcc ctg tct ccg ggt aaa
1293
His Asn His Tyr Thr Gln Lys Ser Leu Ser Leu Ser Pro Gly Lys
            420                 425                 430


<210>  152
<211>  431
<212>  PRT
<213>  Artificial Sequence

<220>
<223>  Amino acid sequence of OPG K194-Fc

<400>  152

Met Asn Asn Leu Leu Cys Cys Ala Leu Val Phe Leu Asp Ile Ser Ile
1               5                   10                  15

Lys Trp Thr Thr Gln Glu Thr Phe Pro Pro Lys Tyr Leu His Tyr Asp
            20                  25                  30

Glu Glu Thr Ser His Gln Leu Leu Cys Asp Lys Cys Pro Pro Gly Thr
        35                  40                  45

Tyr Leu Lys Gln His Cys Thr Ala Lys Trp Lys Thr Val Cys Ala Pro
    50                  55                  60

Cys Pro Asp His Tyr Tyr Thr Asp Ser Trp His Thr Ser Asp Glu Cys
65                  70                  75                  80

Leu Tyr Cys Ser Pro Val Cys Lys Glu Leu Gln Tyr Val Lys Gln Glu
                85                  90                  95

Cys Asn Arg Thr His Asn Arg Val Cys Glu Cys Lys Glu Gly Arg Tyr
            100                 105                 110

Leu Glu Ile Glu Phe Cys Leu Lys His Arg Ser Cys Pro Pro Gly Phe
        115                 120                 125

Gly Val Val Gln Ala Gly Thr Pro Glu Arg Asn Thr Val Cys Lys Arg
    130                 135                 140

Cys Pro Asp Gly Phe Phe Ser Asn Glu Thr Ser Ser Lys Ala Pro Cys
145                 150                 155                 160

Arg Lys His Thr Asn Cys Ser Val Phe Gly Leu Leu Leu Thr Gln Lys
                165                 170                 175

Gly Asn Ala Thr His Asp Asn Ile Cys Ser Gly Asn Ser Glu Ser Thr
            180                 185                 190

Gln Lys Ile Glu Gly Arg Met Asp Pro Lys Ser Ser Asp Lys Thr His
        195                 200                 205

Thr Cys Pro Pro Cys Pro Ala Pro Glu Leu Leu Gly Gly Pro Ser Val
    210                 215                 220

Phe Leu Phe Pro Pro Lys Pro Lys Asp Thr Leu Met Ile Ser Arg Thr
225                 230                 235                 240

Pro Glu Val Thr Cys Val Val Val Asp Val Ser His Glu Asp Pro Glu
                245                 250                 255

Val Lys Phe Asn Trp Tyr Val Asp Gly Val Glu Val His Asn Ala Lys
            260                 265                 270

Thr Lys Pro Arg Glu Glu Gln Tyr Asn Ser Thr Tyr Arg Val Val Ser
        275                 280                 285

Val Leu Thr Val Leu His Gln Asp Trp Leu Asn Gly Lys Glu Tyr Lys
    290                 295                 300

Cys Lys Val Ser Asn Lys Ala Leu Pro Ala Pro Ile Glu Lys Thr Ile
305                 310                 315                 320

Ser Lys Ala Lys Gly Gln Pro Arg Glu Pro Gln Val Tyr Thr Leu Pro
                325                 330                 335

Pro Ser Arg Asp Glu Leu Thr Lys Asn Gln Val Ser Leu Thr Cys Leu
            340                 345                 350

Val Lys Gly Phe Tyr Pro Ser Asp Ile Ala Val Glu Trp Glu Ser Asn
        355                 360                 365

Gly Gln Pro Glu Asn Asn Tyr Lys Thr Thr Pro Pro Val Leu Asp Ser
    370                 375                 380

Asp Gly Ser Phe Phe Leu Tyr Ser Lys Leu Thr Val Asp Lys Ser Arg
385                 390                 395                 400

Trp Gln Gln Gly Asn Val Phe Ser Cys Ser Val Met His Glu Ala Leu
                405                 410                 415

His Asn His Tyr Thr Gln Lys Ser Leu Ser Leu Ser Pro Gly Lys
            420                 425                 430

<210>  153
<211>  330
<212>  PRT
<213>  Homo sapiens

<220>
<221>  PEPTIDE
<222>  (1)..(330)

<220>
<223>  Amino acid sequence of heavy chain constant region of human IgG1

<400>  153

<210>  154
<211>  327
<212>  PRT
<213>  Homo sapiens

<220>
<221>  PEPTIDE
<222>  (1)..(327)

<220>
<223>  Amino acid sequence of heavy chain constant region of human IgG4

<400>  154

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  155
<211>  696
<212>  DNA
<213>  Artificial Sequence

<220>
<221>  CDS
<222>  (1)..(696)

<220>
<223>  Nucleotide sequence of Fc(Eg1S)

<400>  155
gag ccc aaa tct tcc gac aaa act cac aca tgc cca ccg tgc cca gca
48
Glu Pro Lys Ser Ser Asp Lys Thr His Thr Cys Pro Pro Cys Pro Ala
1               5                   10                  15

cct gaa ctc ctg ggg gga ccg tca gtc ttc ctc ttc ccc cca aaa ccc
96
Pro Glu Leu Leu Gly Gly Pro Ser Val Phe Leu Phe Pro Pro Lys Pro
            20                  25                  30

aag gac acc ctc atg atc tcc cgg acc cct gag gtc aca tgc gtg gtg
144
Lys Asp Thr Leu Met Ile Ser Arg Thr Pro Glu Val Thr Cys Val Val
        35                  40                  45

gtg gac gtg agc cac gaa gac cct gag gtc aag ttc aac tgg tac gtg
192
Val Asp Val Ser His Glu Asp Pro Glu Val Lys Phe Asn Trp Tyr Val
    50                  55                  60

gac ggc gtg gag gtg cat aat gcc aag aca aag ccg cgg gag gag cag
240
Asp Gly Val Glu Val His Asn Ala Lys Thr Lys Pro Arg Glu Glu Gln
65                  70                  75                  80

tac aac agc acg tac cgt gtg gtc agc gtc ctc acc gtc ctg cac cag
288
Tyr Asn Ser Thr Tyr Arg Val Val Ser Val Leu Thr Val Leu His Gln
                85                  90                  95

gac tgg ctg aat ggc aag gag tac aag tgc aag gtc tcc aac aaa gcc
336
Asp Trp Leu Asn Gly Lys Glu Tyr Lys Cys Lys Val Ser Asn Lys Ala
            100                 105                 110

ctc cca gcc ccc atc gag aaa acc atc tcc aaa gcc aaa ggg cag ccc
384
Leu Pro Ala Pro Ile Glu Lys Thr Ile Ser Lys Ala Lys Gly Gln Pro
        115                 120                 125

cga gaa cca cag gtg tac acc ctg ccc cca tcc cgg gat gag ctg acc
432

Arg Glu Pro Gln Val Tyr Thr Leu Pro Pro Ser Arg Asp Glu Leu Thr
    130                 135                 140

aag aac cag gtc agc ctg acc tgc ctg gtc aaa ggc ttc tat ccc agc
480
Lys Asn Gln Val Ser Leu Thr Cys Leu Val Lys Gly Phe Tyr Pro Ser
145                 150                 155                 160

gac atc gcc gtg gag tgg gag agc aat ggg cag ccg gag aac aac tac
528
Asp Ile Ala Val Glu Trp Glu Ser Asn Gly Gln Pro Glu Asn Asn Tyr
                165                 170                 175

aag acc acg cct ccc gtg ctg gac tcc gac ggc tcc ttc ttc ctc tac
576
Lys Thr Thr Pro Pro Val Leu Asp Ser Asp Gly Ser Phe Phe Leu Tyr
            180                 185                 190

agc aag ctc acc gtg gac aag agc agg tgg cag cag ggg aac gtc ttc
624
Ser Lys Leu Thr Val Asp Lys Ser Arg Trp Gln Gln Gly Asn Val Phe
        195                 200                 205

tca tgc tcc gtg atg cat gag gct ctg cac aac cac tac acg cag aag
672
Ser Cys Ser Val Met His Glu Ala Leu His Asn His Tyr Thr Gln Lys
    210                 215                 220

agc ctc tcc ctg tct ccg ggt aaa
696
Ser Leu Ser Leu Ser Pro Gly Lys
225                 230


<210>  156
<211>  232
<212>  PRT
<213>  Artificial Sequence

<220>
<223>  Amino acid sequence of Fc(Eg1S)

<400>  156

Glu Pro Lys Ser Ser Asp Lys Thr His Thr Cys Pro Pro Cys Pro Ala
1               5                   10                  15


Pro Glu Leu Leu Gly Gly Pro Ser Val Phe Leu Phe Pro Pro Lys Pro
            20                  25                  30


Lys Asp Thr Leu Met Ile Ser Arg Thr Pro Glu Val Thr Cys Val Val
        35                  40                  45


Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  157
<211>  696
<212>  DNA
<213>  Artificial Sequence

<220>
<221>  CDS
<222>  (1)..(696)

<220>
<223>  Nucleotide sequence of Fc(Eg1S-YTE)

<400>  157
gag ccc aaa tct tcc gac aaa act cac aca tgc cca ccg tgc cca gca
48

Glu Pro Lys Ser Ser Asp Lys Thr His Thr Cys Pro Pro Cys Pro Ala
1               5                   10                  15

cct gaa ctc ctg ggg gga ccg tca gtc ttc ctc ttc ccc cca aaa ccc
96
Pro Glu Leu Leu Gly Gly Pro Ser Val Phe Leu Phe Pro Pro Lys Pro
            20                  25                  30

aag gac acc ctc tat atc act cgg gag cct gag gtc aca tgc gtg gtg
144
Lys Asp Thr Leu Tyr Ile Thr Arg Glu Pro Glu Val Thr Cys Val Val
        35                  40                  45

gtg gac gtg agc cac gaa gac cct gag gtc aag ttc aac tgg tac gtg
192
Val Asp Val Ser His Glu Asp Pro Glu Val Lys Phe Asn Trp Tyr Val
    50                  55                  60

gac ggc gtg gag gtg cat aat gcc aag aca aag ccg cgg gag gag cag
240
Asp Gly Val Glu Val His Asn Ala Lys Thr Lys Pro Arg Glu Glu Gln
65                  70                  75                  80

tac aac agc acg tac cgt gtg gtc agc gtc ctc acc gtc ctg cac cag
288
Tyr Asn Ser Thr Tyr Arg Val Val Ser Val Leu Thr Val Leu His Gln
                85                  90                  95

gac tgg ctg aat ggc aag gag tac aag tgc aag gtc tcc aac aaa gcc
336
Asp Trp Leu Asn Gly Lys Glu Tyr Lys Cys Lys Val Ser Asn Lys Ala
            100                 105                 110

ctc cca gcc ccc atc gag aaa acc atc tcc aaa gcc aaa ggg cag ccc
384
Leu Pro Ala Pro Ile Glu Lys Thr Ile Ser Lys Ala Lys Gly Gln Pro
        115                 120                 125

cga gaa cca cag gtg tac acc ctg ccc cca tcc cgg gat gag ctg acc
432
Arg Glu Pro Gln Val Tyr Thr Leu Pro Pro Ser Arg Asp Glu Leu Thr
    130                 135                 140

aag aac cag gtc agc ctg acc tgc ctg gtc aaa ggc ttc tat ccc agc
480
Lys Asn Gln Val Ser Leu Thr Cys Leu Val Lys Gly Phe Tyr Pro Ser
145                 150                 155                 160

gac atc gcc gtg gag tgg gag agc aat ggg cag ccg gag aac aac tac
528
Asp Ile Ala Val Glu Trp Glu Ser Asn Gly Gln Pro Glu Asn Asn Tyr
                165                 170                 175

aag acc acg cct ccc gtg ctg gac tcc gac ggc tcc ttc ttc ctc tac
576
Lys Thr Thr Pro Pro Val Leu Asp Ser Asp Gly Ser Phe Phe Leu Tyr
            180                 185                 190

agc aag ctc acc gtg gac aag agc agg tgg cag cag ggg aac gtc ttc
624

Ser Lys Leu Thr Val Asp Lys Ser Arg Trp Gln Gln Gly Asn Val Phe
        195                 200                 205

tca tgc tcc gtg atg cat gag gct ctg cac aac cac tac acg cag aag
672
Ser Cys Ser Val Met His Glu Ala Leu His Asn His Tyr Thr Gln Lys
    210                 215                 220

agc ctc tcc ctg tct ccg ggt aaa
696
Ser Leu Ser Leu Ser Pro Gly Lys
225                 230

<210>  158
<211>  232
<212>  PRT
<213>  Artificial Sequence

<220>
<223>  Amino acid sequence of Fc(Eg1S-YTE)

<400>  158

Glu Pro Lys Ser Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Tyr Ile Thr Arg Glu Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  159
<211>  696
<212>  DNA
<213>  Artificial Sequence

<220>
<221>  CDS
<222>  (1)..(696)

<220>
<223>  Nucleotide sequence of Fc(Eg1S-N434A)

<400>  159
gag ccc aaa tct tcc gac aaa act cac aca tgc cca ccg tgc cca gca
48
Glu Pro Lys Ser Ser Asp Lys Thr His Thr Cys Pro Pro Cys Pro Ala
1               5                   10                  15

cct gaa ctc ctg ggg gga ccg tca gtc ttc ctc ttc ccc cca aaa ccc
96
Pro Glu Leu Leu Gly Gly Pro Ser Val Phe Leu Phe Pro Pro Lys Pro
            20                  25                  30

aag gac acc ctc atg atc tcc cgg acc cct gag gtc aca tgc gtg gtg
144
Lys Asp Thr Leu Met Ile Ser Arg Thr Pro Glu Val Thr Cys Val Val
        35                  40                  45

gtg gac gtg agc cac gaa gac cct gag gtc aag ttc aac tgg tac gtg
192
Val Asp Val Ser His Glu Asp Pro Glu Val Lys Phe Asn Trp Tyr Val
    50                  55                  60

gac ggc gtg gag gtg cat aat gcc aag aca aag ccg cgg gag gag cag
240
Asp Gly Val Glu Val His Asn Ala Lys Thr Lys Pro Arg Glu Glu Gln

65                  70                  75                  80

tac aac agc acg tac cgt gtg gtc agc gtc ctc acc gtc ctg cac cag
288
Tyr Asn Ser Thr Tyr Arg Val Val Ser Val Leu Thr Val Leu His Gln
                85                  90                  95

gac tgg ctg aat ggc aag gag tac aag tgc aag gtc tcc aac aaa gcc
336
Asp Trp Leu Asn Gly Lys Glu Tyr Lys Cys Lys Val Ser Asn Lys Ala
            100                 105                 110

ctc cca gcc ccc atc gag aaa acc atc tcc aaa gcc aaa ggg cag ccc
384
Leu Pro Ala Pro Ile Glu Lys Thr Ile Ser Lys Ala Lys Gly Gln Pro
        115                 120                 125

cga gaa cca cag gtg tac acc ctg ccc cca tcc cgg gat gag ctg acc
432
Arg Glu Pro Gln Val Tyr Thr Leu Pro Pro Ser Arg Asp Glu Leu Thr
    130                 135                 140

aag aac cag gtc agc ctg acc tgc ctg gtc aaa ggc ttc tat ccc agc
480
Lys Asn Gln Val Ser Leu Thr Cys Leu Val Lys Gly Phe Tyr Pro Ser
145                 150                 155                 160

gac atc gcc gtg gag tgg gag agc aat ggg cag ccg gag aac aac tac
528
Asp Ile Ala Val Glu Trp Glu Ser Asn Gly Gln Pro Glu Asn Asn Tyr
                165                 170                 175

aag acc acg cct ccc gtg ctg gac tcc gac ggc tcc ttc ttc ctc tac
576
Lys Thr Thr Pro Pro Val Leu Asp Ser Asp Gly Ser Phe Phe Leu Tyr
            180                 185                 190

agc aag ctc acc gtg gac aag agc agg tgg cag cag ggg aac gtc ttc
624
Ser Lys Leu Thr Val Asp Lys Ser Arg Trp Gln Gln Gly Asn Val Phe
        195                 200                 205

tca tgc tcc gtg atg cat gag gct ctg cac gcc cac tac acg cag aag
672
Ser Cys Ser Val Met His Glu Ala Leu His Ala His Tyr Thr Gln Lys
    210                 215                 220

agc ctc tcc ctg tct ccg ggt aaa
696
Ser Leu Ser Leu Ser Pro Gly Lys
225                 230


<210>  160
<211>  232
<212>  PRT
<213>  Artificial Sequence

<220>
<223>  Amino acid sequence of Fc(Eg1S-N434A)

<400>  160

Glu Pro Lys Ser Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Ala His Tyr Thr Gln Lys
    210                 215                 220

Ser Leu Ser Leu Ser Pro Gly Lys

225                 230

<210>  161
<211>  693
<212>  DNA
<213>  Artificial Sequence

<220>
<221>  CDS
<222>  (1)..(693)

<220>
<223>  Nucleotide sequence of Fc(g1S-LALAGA)

<400>  161
ccc aaa tct tcc gac aaa act cac aca tgc cca ccg tgc cca gca cct
48
Pro Lys Ser Ser Asp Lys Thr His Thr Cys Pro Pro Cys Pro Ala Pro
1               5                   10                  15

gaa gcc gct ggg gcc ccg tca gtc ttc ctc ttc ccc cca aaa ccc aag
96
Glu Ala Ala Gly Ala Pro Ser Val Phe Leu Phe Pro Pro Lys Pro Lys
            20                  25                  30

gac acc ctc atg atc tcc cgg acc cct gag gtc aca tgc gtg gtg gtg
144
Asp Thr Leu Met Ile Ser Arg Thr Pro Glu Val Thr Cys Val Val Val
        35                  40                  45

gac gtg agc cac gaa gac cct gag gtc aag ttc aac tgg tac gtg gac
192
Asp Val Ser His Glu Asp Pro Glu Val Lys Phe Asn Trp Tyr Val Asp
    50                  55                  60

ggc gtg gag gtg cat aat gcc aag aca aag ccg cgg gag gag cag tac
240
Gly Val Glu Val His Asn Ala Lys Thr Lys Pro Arg Glu Glu Gln Tyr
65                  70                  75                  80

aac agc acg tac cgt gtg gtc agc gtc ctc acc gtc ctg cac cag gac
288
Asn Ser Thr Tyr Arg Val Val Ser Val Leu Thr Val Leu His Gln Asp
                85                  90                  95

tgg ctg aat ggc aag gag tac aag tgc aag gtc tcc aac aaa gcc ctc
336
Trp Leu Asn Gly Lys Glu Tyr Lys Cys Lys Val Ser Asn Lys Ala Leu
            100                 105                 110

cca gcc ccc atc gag aaa acc atc tcc aaa gcc aaa ggg cag ccc cga
384
Pro Ala Pro Ile Glu Lys Thr Ile Ser Lys Ala Lys Gly Gln Pro Arg
        115                 120                 125

gaa cca cag gtg tac acc ctg ccc cca tcc cgg gat gag ctg acc aag
432
Glu Pro Gln Val Tyr Thr Leu Pro Pro Ser Arg Asp Glu Leu Thr Lys
    130                 135                 140

aac cag gtc agc ctg acc tgc ctg gtc aaa ggc ttc tat ccc agc gac
480
Asn Gln Val Ser Leu Thr Cys Leu Val Lys Gly Phe Tyr Pro Ser Asp
145                 150                 155                 160

atc gcc gtg gag tgg gag agc aat ggg cag ccg gag aac aac tac aag
528
Ile Ala Val Glu Trp Glu Ser Asn Gly Gln Pro Glu Asn Asn Tyr Lys
                165                 170                 175

acc acg cct ccc gtg ctg gac tcc gac ggc tcc ttc ttc ctc tac agc
576
Thr Thr Pro Pro Val Leu Asp Ser Asp Gly Ser Phe Phe Leu Tyr Ser
            180                 185                 190

aag ctc acc gtg gac aag agc agg tgg cag cag ggg aac gtc ttc tca
624
Lys Leu Thr Val Asp Lys Ser Arg Trp Gln Gln Gly Asn Val Phe Ser
        195                 200                 205

tgc tcc gtg atg cat gag gct ctg cac aac cac tac acg cag aag agc
672
Cys Ser Val Met His Glu Ala Leu His Asn His Tyr Thr Gln Lys Ser
    210                 215                 220

ctc tcc ctg tct ccg ggc aaa
693
Leu Ser Leu Ser Pro Gly Lys
225                 230


<210>  162
<211>  231
<212>  PRT
<213>  Artificial Sequence

<220>
<223>  Amino acid sequence of Fc(g1S-LALAGA)

<400>  162

Pro Lys Ser Ser Asp Lys Thr His Thr Cys Pro Pro Cys Pro Ala Pro
1               5                   10                  15


Glu Ala Ala Gly Ala Pro Ser Val Phe Leu Phe Pro Pro Lys Pro Lys
            20                  25                  30


Asp Thr Leu Met Ile Ser Arg Thr Pro Glu Val Thr Cys Val Val Val
        35                  40                  45


Asp Val Ser His Glu Asp Pro Glu Val Lys Phe Asn Trp Tyr Val Asp
    50                  55                  60


Gly Val Glu Val His Asn Ala Lys Thr Lys Pro Arg Glu Glu Gln Tyr
65                  70                  75                  80

Asn Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Ala Leu
            100                 105                 110

Pro Ala Pro Ile Glu Lys Thr Ile Ser Lys Ala Lys Gly Gln Pro Arg
        115                 120                 125

Glu Pro Gln Val Tyr Thr Leu Pro Pro Ser Arg Asp Glu Leu Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Thr Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Asn His Tyr Thr Gln Lys Ser
    210                 215                 220

Leu Ser Leu Ser Pro Gly Lys
225                 230

<210>  163
<211>  696
<212>  DNA
<213>  Artificial Sequence

<220>
<221>  CDS
<222>  (1)..(696)

<220>
<223>  Nucleotide sequence of Fc(Eg1S-LALAGA)

<400>  163
gag ccc aaa tct tcc gac aaa act cac aca tgc cca ccg tgc cca gca
48
Glu Pro Lys Ser Ser Asp Lys Thr His Thr Cys Pro Pro Cys Pro Ala
1               5                   10                  15

cct gaa gcc gct ggg gcc ccg tca gtc ttc ctc ttc ccc cca aaa ccc
96
Pro Glu Ala Ala Gly Ala Pro Ser Val Phe Leu Phe Pro Pro Lys Pro
            20                  25                  30

aag gac acc ctc atg atc tcc cgg acc cct gag gtc aca tgc gtg gtg
144
Lys Asp Thr Leu Met Ile Ser Arg Thr Pro Glu Val Thr Cys Val Val
        35                  40                  45

gtg gac gtg agc cac gaa gac cct gag gtc aag ttc aac tgg tac gtg
192
Val Asp Val Ser His Glu Asp Pro Glu Val Lys Phe Asn Trp Tyr Val
    50                  55                  60

gac ggc gtg gag gtg cat aat gcc aag aca aag ccg cgg gag gag cag
240
Asp Gly Val Glu Val His Asn Ala Lys Thr Lys Pro Arg Glu Glu Gln
65                  70                  75                  80

tac aac agc acg tac cgt gtg gtc agc gtc ctc acc gtc ctg cac cag
288
Tyr Asn Ser Thr Tyr Arg Val Val Ser Val Leu Thr Val Leu His Gln
                85                  90                  95

gac tgg ctg aat ggc aag gag tac aag tgc aag gtc tcc aac aaa gcc
336
Asp Trp Leu Asn Gly Lys Glu Tyr Lys Cys Lys Val Ser Asn Lys Ala
            100                 105                 110

ctc cca gcc ccc atc gag aaa acc atc tcc aaa gcc aaa ggg cag ccc
384
Leu Pro Ala Pro Ile Glu Lys Thr Ile Ser Lys Ala Lys Gly Gln Pro
        115                 120                 125

cga gaa cca cag gtg tac acc ctg ccc cca tcc cgg gat gag ctg acc
432
Arg Glu Pro Gln Val Tyr Thr Leu Pro Pro Ser Arg Asp Glu Leu Thr
    130                 135                 140

aag aac cag gtc agc ctg acc tgc ctg gtc aaa ggc ttc tat ccc agc
480
Lys Asn Gln Val Ser Leu Thr Cys Leu Val Lys Gly Phe Tyr Pro Ser
145                 150                 155                 160

gac atc gcc gtg gag tgg gag agc aat ggg cag ccg gag aac aac tac
528
Asp Ile Ala Val Glu Trp Glu Ser Asn Gly Gln Pro Glu Asn Asn Tyr
                165                 170                 175

aag acc acg cct ccc gtg ctg gac tcc gac ggc tcc ttc ttc ctc tac
576
Lys Thr Thr Pro Pro Val Leu Asp Ser Asp Gly Ser Phe Phe Leu Tyr
            180                 185                 190

agc aag ctc acc gtg gac aag agc agg tgg cag cag ggg aac gtc ttc
624
Ser Lys Leu Thr Val Asp Lys Ser Arg Trp Gln Gln Gly Asn Val Phe
        195                 200                 205

tca tgc tcc gtg atg cat gag gct ctg cac aac cac tac acg cag aag
672
Ser Cys Ser Val Met His Glu Ala Leu His Asn His Tyr Thr Gln Lys
    210                 215                 220

agc ctc tcc ctg tct ccg ggc aaa
696
Ser Leu Ser Leu Ser Pro Gly Lys
225                 230

<210>  164
<211>  232
<212>  PRT
<213>  Artificial Sequence

<220>
<223>  Amino acid sequence of Fc(Eg1S-LALAGA)

<400>  164

Glu Pro Lys Ser Ser Asp Lys Thr His Thr Cys Pro Pro Cys Pro Ala
1               5                   10                  15

Pro Glu Ala Ala Gly Ala Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  165
<211>  696
<212>  DNA
<213>  Artificial Sequence

<220>
<221>  CDS
<222>  (1)..(696)

<220>
<223>  Nucleotide sequence of Fc(Eg1S-LALAGANA)

<400>  165
gag ccc aaa tct tcc gac aaa act cac aca tgc cca ccg tgc cca gca
48
Glu Pro Lys Ser Ser Asp Lys Thr His Thr Cys Pro Pro Cys Pro Ala
1               5                   10                  15

cct gaa gcc gct ggg gcc ccg tca gtc ttc ctc ttc ccc cca aaa ccc
96
Pro Glu Ala Ala Gly Ala Pro Ser Val Phe Leu Phe Pro Pro Lys Pro
            20                  25                  30

aag gac acc ctc atg atc tcc cgg acc cct gag gtc aca tgc gtg gtg
144
Lys Asp Thr Leu Met Ile Ser Arg Thr Pro Glu Val Thr Cys Val Val
        35                  40                  45

gtg gac gtg agc cac gaa gac cct gag gtc aag ttc aac tgg tac gtg
192
Val Asp Val Ser His Glu Asp Pro Glu Val Lys Phe Asn Trp Tyr Val
    50                  55                  60

gac ggc gtg gag gtg cat aat gcc aag aca aag ccg cgg gag gag cag
240
Asp Gly Val Glu Val His Asn Ala Lys Thr Lys Pro Arg Glu Glu Gln
65                  70                  75                  80

tac aac agc acg tac cgt gtg gtc agc gtc ctc acc gtc ctg cac cag
288
Tyr Asn Ser Thr Tyr Arg Val Val Ser Val Leu Thr Val Leu His Gln
                85                  90                  95

gac tgg ctg aat ggc aag gag tac aag tgc aag gtc tcc aac aaa gcc
336
Asp Trp Leu Asn Gly Lys Glu Tyr Lys Cys Lys Val Ser Asn Lys Ala
            100                 105                 110

ctc cca gcc ccc atc gag aaa acc atc tcc aaa gcc aaa ggg cag ccc
384
Leu Pro Ala Pro Ile Glu Lys Thr Ile Ser Lys Ala Lys Gly Gln Pro
        115                 120                 125

cga gaa cca cag gtg tac acc ctg ccc cca tcc cgg gat gag ctg acc
432
Arg Glu Pro Gln Val Tyr Thr Leu Pro Pro Ser Arg Asp Glu Leu Thr
    130                 135                 140

aag aac cag gtc agc ctg acc tgc ctg gtc aaa ggc ttc tat ccc agc
480
Lys Asn Gln Val Ser Leu Thr Cys Leu Val Lys Gly Phe Tyr Pro Ser
145                 150                 155                 160

gac atc gcc gtg gag tgg gag agc aat ggg cag ccg gag aac aac tac
528
Asp Ile Ala Val Glu Trp Glu Ser Asn Gly Gln Pro Glu Asn Asn Tyr
                165                 170                 175

aag acc acg cct ccc gtg ctg gac tcc gac ggc tcc ttc ttc ctc tac
576
Lys Thr Thr Pro Pro Val Leu Asp Ser Asp Gly Ser Phe Phe Leu Tyr
            180                 185                 190

agc aag ctc acc gtg gac aag agc agg tgg cag cag ggg aac gtc ttc
624
Ser Lys Leu Thr Val Asp Lys Ser Arg Trp Gln Gln Gly Asn Val Phe
        195                 200                 205

tca tgc tcc gtg atg cat gag gct ctg cac gcc cac tac acg cag aag
672
Ser Cys Ser Val Met His Glu Ala Leu His Ala His Tyr Thr Gln Lys
    210                 215                 220

agc ctc tcc ctg tct ccg ggt aaa
696
Ser Leu Ser Leu Ser Pro Gly Lys
225                 230


<210>  166
<211>  232
<212>  PRT
<213>  Artificial Sequence

<220>
<223>  Amino acid sequence of Fc(Eg1S-LALAGANA)

<400>  166

Glu Pro Lys Ser Ser Asp Lys Thr His Thr Cys Pro Pro Cys Pro Ala
1               5                   10                  15

Pro Glu Ala Ala Gly Ala Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Ala His Tyr Thr Gln Lys
    210                 215                 220

Ser Leu Ser Leu Ser Pro Gly Lys
225                 230

<210>  167
<211>  1215
<212>  DNA
<213>  Artificial Sequence

<220>
<221>  CDS
<222>  (1)..(1215)


<220>
<223>  Nucleotide sequence of Chimera A-Fc(Eg1S)


<400>  167
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405

<210>  168
<211>  405
<212>  PRT
<213>  Artificial Sequence

<220>
<223>  Amino acid sequence of Chimera A-Fc(Eg1S)

<400>  168

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
        100                 105                 110

Gly Thr Pro Ser Gln Asn Thr Gln Cys Gln Pro Cys Pro Asp Gly Phe
    115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
            165                 170                 175

Ser Ser Asp Lys Thr His Thr Cys Pro Pro Cys Pro Ala Pro Glu Leu
        180                 185                 190

Leu Gly Gly Pro Ser Val Phe Leu Phe Pro Pro Lys Pro Lys Asp Thr
    195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
            245                 250                 255

Thr Tyr Arg Val Val Ser Val Leu Thr Val Leu His Gln Asp Trp Leu
        260                 265                 270

Asn Gly Lys Glu Tyr Lys Cys Lys Val Ser Asn Lys Ala Leu Pro Ala
    275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
            325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210> 169
<211> 1215
<212> DNA
<213> Artificial Sequence

<220>
<221> CDS
<222> (1)..(1215)

<220>
<223> Nucleotide sequence of Chimera A-Fc(Eg1S-YTE)

<400> 169
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc tat atc act cgg gag cct gag gtc aca tgc gtg gtg gtg gac gtg
672
Leu Tyr Ile Thr Arg Glu Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405

<210>  170
<211>  405
<212>  PRT
<213>  Artificial Sequence

<220>
<223>  Amino acid sequence of Chimera A-Fc(Eg1S-YTE)

<400>  170

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Tyr Ile Thr Arg Glu Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  171
<211>  1215
<212>  DNA
<213>  Artificial Sequence

<220>
<221>  CDS
<222>  (1)..(1215)

<220>
<223>  Nucleotide sequence of Chimera A-Fc(Eg1S-N434A)

<400>  171
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96

Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672

Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215

Leu Ser Pro Gly Lys
                405

<210>  172
<211>  405
<212>  PRT
<213>  Artificial Sequence

<220>
<223>  Amino acid sequence of Chimera A-Fc(Eg1S-N434A)

<400>  172

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  173
<211>  1212

<212>  DNA
<213>  Artificial Sequence

<220>
<221>  CDS
<222>  (1)..(1212)

<220>
<223>  Nucleotide sequence of Chimera A-Fc(g1S-LALAGA)

<400>  173
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag ccc aaa tct
528
Asp Asn Ile Cys Ser Gly Asn Ser Glu Ser Thr Gln Lys Pro Lys Ser
                165                 170                 175

tcc gac aaa act cac aca tgc cca ccg tgc cca gca cct gaa gcc gct
576
Ser Asp Lys Thr His Thr Cys Pro Pro Cys Pro Ala Pro Glu Ala Ala
            180                 185                 190

ggg gcc ccg tca gtc ttc ctc ttc ccc cca aaa ccc aag gac acc ctc
624
Gly Ala Pro Ser Val Phe Leu Phe Pro Pro Lys Pro Lys Asp Thr Leu
        195                 200                 205

atg atc tcc cgg acc cct gag gtc aca tgc gtg gtg gtg gac gtg agc
672
Met Ile Ser Arg Thr Pro Glu Val Thr Cys Val Val Val Asp Val Ser
    210                 215                 220

cac gaa gac cct gag gtc aag ttc aac tgg tac gtg gac ggc gtg gag
720
His Glu Asp Pro Glu Val Lys Phe Asn Trp Tyr Val Asp Gly Val Glu
225                 230                 235                 240

gtg cat aat gcc aag aca aag ccg cgg gag gag cag tac aac agc acg
768
Val His Asn Ala Lys Thr Lys Pro Arg Glu Glu Gln Tyr Asn Ser Thr
                245                 250                 255

tac cgt gtg gtc agc gtc ctc acc gtc ctg cac cag gac tgg ctg aat
816
Tyr Arg Val Val Ser Val Leu Thr Val Leu His Gln Asp Trp Leu Asn
            260                 265                 270

ggc aag gag tac aag tgc aag gtc tcc aac aaa gcc ctc cca gcc ccc
864
Gly Lys Glu Tyr Lys Cys Lys Val Ser Asn Lys Ala Leu Pro Ala Pro
        275                 280                 285

atc gag aaa acc atc tcc aaa gcc aaa ggg cag ccc cga gaa cca cag
912
Ile Glu Lys Thr Ile Ser Lys Ala Lys Gly Gln Pro Arg Glu Pro Gln
    290                 295                 300

gtg tac acc ctg ccc cca tcc cgg gat gag ctg acc aag aac cag gtc
960
Val Tyr Thr Leu Pro Pro Ser Arg Asp Glu Leu Thr Lys Asn Gln Val
305                 310                 315                 320

agc ctg acc tgc ctg gtc aaa ggc ttc tat ccc agc gac atc gcc gtg
1008
Ser Leu Thr Cys Leu Val Lys Gly Phe Tyr Pro Ser Asp Ile Ala Val
                325                 330                 335

gag tgg gag agc aat ggg cag ccg gag aac aac tac aag acc acg cct
1056
Glu Trp Glu Ser Asn Gly Gln Pro Glu Asn Asn Tyr Lys Thr Thr Pro
            340                 345                 350

ccc gtg ctg gac tcc gac ggc tcc ttc ttc ctc tac agc aag ctc acc
1104
Pro Val Leu Asp Ser Asp Gly Ser Phe Phe Leu Tyr Ser Lys Leu Thr
        355                 360                 365

gtg gac aag agc agg tgg cag cag ggg aac gtc ttc tca tgc tcc gtg
1152
Val Asp Lys Ser Arg Trp Gln Gln Gly Asn Val Phe Ser Cys Ser Val
    370                 375                 380

atg cat gag gct ctg cac aac cac tac acg cag aag agc ctc tcc ctg
1200
Met His Glu Ala Leu His Asn His Tyr Thr Gln Lys Ser Leu Ser Leu
385                 390                 395                 400

tct ccg ggc aaa
1212
Ser Pro Gly Lys

<210>  174
<211>  404
<212>  PRT
<213>  Artificial Sequence

<220>
<223>  Amino acid sequence of Chimera A-Fc(g1S-LALAGA)

<400>  174

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Ala Ala
            180                 185                 190

Gly Ala Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210> 175
<211> 1215
<212> DNA
<213> Artificial Sequence

<220>
<221> CDS
<222> (1)..(1215)

<220>
<223> Nucleotide sequence of Chimera A-Fc(Eg1S-LALAGA)

<400> 175
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggc aaa
1215
Leu Ser Pro Gly Lys
                405


<210>  176
<211>  405
<212>  PRT
<213>  Artificial Sequence

<220>
<223>  Amino acid sequence of Chimera A-Fc(Eg1S-LALAGA)

<400>  176

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His

35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala

275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  177
<211>  1215
<212>  DNA
<213>  Artificial Sequence

<220>
<221>  CDS
<222>  (1)..(1215)

<220>
<223>  Nucleotide sequence of Chimera A-Fc(Eg1S-LALAGANA)

<400>  177
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144

Cys Thr Ala Lys Trp Lys Thr Val Cys Ala Pro Cys Pro Pro Arg His
        35                  40                  45

tac act cag ttt tgg aat tac ctg gag aga tgc cgc tat tgt aac gtt
192
Tyr Thr Gln Phe Trp Asn Tyr Leu Glu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720

Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405

<210>  178

<211>  405
<212>  PRT
<213>  Artificial Sequence

<220>
<223>  Amino acid sequence of Chimera A-Fc(Eg1S-LALAGANA)

<400>  178

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  179
<211>  519
<212>  DNA
<213>  Artificial Sequence

<220>
<221>  CDS

<222>  (1)..(519)

<220>
<223>  Nucleotide sequence of Chimera A-E57R

<400>  179
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg aga tgc cgc tat tgt aac gtt
192
Tyr Thr Gln Phe Trp Asn Tyr Leu Arg Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210>  180
<211>  173
<212>  PRT
<213>  Artificial Sequence

<220>
<223>  Amino acid sequence of Chimera A-E57R

<400>  180

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  181
<211>  519
<212>  DNA
<213>  Artificial Sequence

<220>
<221>  CDS
<222>  (1)..(519)

<220>
<223>  Nucleotide sequence of Chimera A-E57V

<400>  181
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg aga tgc cgc tat tgt aac gtt
192
Tyr Thr Gln Phe Trp Asn Tyr Leu Val Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210>  182
<211>  173
<212>  PRT
<213>  Artificial Sequence

<220>
<223>  Amino acid sequence of Chimera A-E57V

<400>  182

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210> 183
<211> 519
<212> DNA
<213> Artificial Sequence

<220>
<221> CDS
<222> (1)..(519)

<220>
<223> Nucleotide sequence of Chimera A-E57K_R58D

<400> 183
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gac tgc cgc tat tgt aac gtt
192
Tyr Thr Gln Phe Trp Asn Tyr Leu Lys Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480

Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210>  184
<211>  173
<212>  PRT
<213>  Artificial Sequence

<220>
<223>  Amino acid sequence of Chimera A-E57K_R58D

<400>  184

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys

165                 170

<210>  185
<211>  519
<212>  DNA
<213>  Artificial Sequence

<220>
<221>  CDS
<222>  (1)..(519)

<220>
<223>  Nucleotide sequence of Chimera A-E57K_R58E

<400>  185
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gag tgc cgc tat tgt aac gtt
192
Tyr Thr Gln Phe Trp Asn Tyr Leu Lys Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210>  186
<211>  173
<212>  PRT
<213>  Artificial Sequence

<220>
<223>  Amino acid sequence of Chimera A-E57K_R58E

<400>  186

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  187
<211>  519
<212>  DNA
<213>  Artificial Sequence

<220>
<221>  CDS
<222>  (1)..(519)

<220>
<223>  Nucleotide sequence of Chimera A-E57R_R58D

<400>  187
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg gac tgc cgc tat tgt aac gtt
192
Tyr Thr Gln Phe Trp Asn Tyr Leu Arg Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  188
<211>  173
<212>  PRT
<213>  Artificial Sequence

<220>
<223>  Amino acid sequence of Chimera A-E57R_R58D

<400>  188

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Arg Asp Cys Arg Tyr Cys Asn Val
    50                  55                  60


Leu Cys Gly Glu Arg Glu Glu Glu Ala Arg Ala Cys His Ala Thr His
65                  70                  75                  80


Asn Arg Ala Cys Arg Cys Arg Thr Gly Phe Phe Ala His Ala Gly Phe
                85                  90                  95


Cys Leu Glu His Ala Ser Cys Pro Pro Gly Ala Gly Val Ile Ala Pro
            100                 105                 110


Gly Thr Pro Ser Gln Asn Thr Gln Cys Gln Pro Cys Pro Asp Gly Phe
        115                 120                 125


Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  189
<211>  1206
<212>  DNA
<213>  Artificial Sequence

<220>
<221>  CDS
<222>  (1)..(1206)

<220>
<223>  Nucleotide sequence of Chimera A-E57R-Fc(g4PEK)

<400>  189
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg aga tgc cgc tat tgt aac gtt
192
Tyr Thr Gln Phe Trp Asn Tyr Leu Arg Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384

Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960

Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  190
<211>  402
<212>  PRT
<213>  Artificial Sequence

<220>
<223>  Amino acid sequence of Chimera A-E57R-Fc(g4PEK)

<400>  190

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  191
<211>  1206
<212>  DNA
<213>  Artificial Sequence

<220>
<221>  CDS
<222>  (1)..(1206)

<220>
<223>  Nucleotide sequence of Chimera A-E57V-Fc(g4PEK)

<400>  191
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg aga tgc cgc tat tgt aac gtt
192
Tyr Thr Gln Phe Trp Asn Tyr Leu Val Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  192
<211>  402
<212>  PRT
<213>  Artificial Sequence

<220>
<223>  Amino acid sequence of Chimera A-E57V-Fc(g4PEK)

<400>  192

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  193
<211>  1206
<212>  DNA
<213>  Artificial Sequence

<220>
<221>  CDS
<222>  (1)..(1206)

<220>
<223>  Nucleotide sequence of Chimera A-E57K_R58D-Fc(g4PEK)

<400>  193

gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gac tgc cgc tat tgt aac gtt
192
Tyr Thr Gln Phe Trp Asn Tyr Leu Lys Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  194
<211>  402
<212>  PRT
<213>  Artificial Sequence

<220>
<223>  Amino acid sequence of Chimera A-E57K_R58D-Fc(g4PEK)

<400>  194

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210> 195
<211> 1206
<212> DNA
<213> Artificial Sequence

<220>
<221> CDS
<222> (1)..(1206)

<220>
<223> Nucleotide sequence of Chimera A-E57K_R58E-Fc(g4PEK)

<400> 195
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gag tgc cgc tat tgt aac gtt
192
Tyr Thr Gln Phe Trp Asn Tyr Leu Lys Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432

Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008

Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  196
<211>  402
<212>  PRT
<213>  Artificial Sequence

<220>
<223>  Amino acid sequence of Chimera A-E57K_R58E-Fc(g4PEK)

<400>  196

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe

85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp

325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  197
<211>  1206
<212>  DNA
<213>  Artificial Sequence

<220>
<221>  CDS
<222>  (1)..(1206)

<220>
<223>  Nucleotide sequence of Chimera A-E57R_R58D-Fc(g4PEK)

<400>  197
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg gac tgc cgc tat tgt aac gtt
192
Tyr Thr Gln Phe Trp Asn Tyr Leu Arg Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  198
<211>  402
<212>  PRT
<213>  Artificial Sequence

<220>
<223>  Amino acid sequence of Chimera A-E57R_R58D-Fc(g4PEK)

<400>  198

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210> 199
<211> 1215
<212> DNA
<213> Artificial Sequence

<220>
<221> CDS
<222> (1)..(1215)

<220>
<223> Nucleotide sequence of Chimera A-E57K-Fc(Eg1S)

<400> 199
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aga tgc cgc tat tgt aac gtt
192
Tyr Thr Gln Phe Trp Asn Tyr Leu Lys Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405

<210>  200
<211>  405
<212>  PRT
<213>  Artificial Sequence

<220>
<223>  Amino acid sequence of Chimera A-E57K-Fc(Eg1S)

<400>  200

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
        180                 185                 190

Leu Gly Gly Pro Ser Val Phe Leu Phe Pro Pro Lys Pro Lys Asp Thr
    195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
        260                 265                 270

Asn Gly Lys Glu Tyr Lys Cys Lys Val Ser Asn Lys Ala Leu Pro Ala
    275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
        340                 345                 350

Pro Pro Val Leu Asp Ser Asp Gly Ser Phe Phe Leu Tyr Ser Lys Leu
    355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  201
<211>  1215
<212>  DNA
<213>  Artificial Sequence

<220>
<221>  CDS
<222>  (1)..(1215)

<220>
<223>  Nucleotide sequence of Chimera A-E57L-Fc(Eg1S)

<400>  201
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg aga tgc cgc tat tgt aac gtt
192
Tyr Thr Gln Phe Trp Asn Tyr Leu Leu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480

Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056

Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405


<210>  202
<211>  405
<212>  PRT
<213>  Artificial Sequence

<220>
<223>  Amino acid sequence of Chimera A-E57L-Fc(Eg1S)

<400>  202

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Leu Arg Cys Arg Tyr Cys Asn Val
    50                  55                  60


Leu Cys Gly Glu Arg Glu Glu Glu Ala Arg Ala Cys His Ala Thr His
65                  70                  75                  80


Asn Arg Ala Cys Arg Cys Arg Thr Gly Phe Phe Ala His Ala Gly Phe
                85                  90                  95


Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  203
<211>  1215
<212>  DNA
<213>  Artificial Sequence

<220>
<221>  CDS
<222>  (1)..(1215)

<220>
<223>  Nucleotide sequence of Chimera A-E57R-Fc(Eg1S)

<400>  203
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg aga tgc cgc tat tgt aac gtt
192
Tyr Thr Gln Phe Trp Asn Tyr Leu Arg Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405


<210>  204
<211>  405
<212>  PRT
<213>  Artificial Sequence

<220>
<223>  Amino acid sequence of Chimera A-E57R-Fc(Eg1S)

<400>  204

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  205
<211>  1215
<212>  DNA
<213>  Artificial Sequence

<220>
<221>  CDS
<222>  (1)..(1215)

<220>
<223>  Nucleotide sequence of Chimera A-E57V-Fc(Eg1S)

<400>  205
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg aga tgc cgc tat tgt aac gtt
192
Tyr Thr Gln Phe Trp Asn Tyr Leu Val Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405

<210>  206
<211>  405
<212>  PRT
<213>  Artificial Sequence

<220>
<223>  Amino acid sequence of Chimera A-E57V-Fc(Eg1S)

<400>  206

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr

195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  207
<211>  1215
<212>  DNA
<213>  Artificial Sequence

<220>
<221>  CDS
<222>  (1)..(1215)

<220>
<223>  Nucleotide sequence of Chimera A-E57K_R58D-Fc(Eg1S)

<400>  207
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gac tgc cgc tat tgt aac gtt
192
Tyr Thr Gln Phe Trp Asn Tyr Leu Lys Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528

Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104

Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405

<210>  208
<211>  405
<212>  PRT
<213>  Artificial Sequence

<220>
<223>  Amino acid sequence of Chimera A-E57K_R58D-Fc(Eg1S)

<400>  208

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  209
<211>  1215
<212>  DNA
<213>  Artificial Sequence

<220>
<221>  CDS
<222>  (1)..(1215)

<220>
<223>  Nucleotide sequence of Chimera A-E57K_R58E-Fc(Eg1S)

<400>  209
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gag tgc cgc tat tgt aac gtt
192
Tyr Thr Gln Phe Trp Asn Tyr Leu Lys Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405



<210>  210
<211>  405
<212>  PRT
<213>  Artificial Sequence

<220>
<223>  Amino acid sequence of Chimera A-E57K_R58E-Fc(Eg1S)

<400>  210

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Lys Glu Cys Arg Tyr Cys Asn Val

50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro

290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  211
<211>  1215
<212>  DNA
<213>  Artificial Sequence

<220>
<221>  CDS
<222>  (1)..(1215)

<220>
<223>  Nucleotide sequence of Chimera A-E57R_R58D-Fc(Eg1S)

<400>  211
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg gac tgc cgc tat tgt aac gtt
192
Tyr Thr Gln Phe Trp Asn Tyr Leu Arg Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405


<210>  212
<211>  405
<212>  PRT
<213>  Artificial Sequence

<220>
<223>  Amino acid sequence of Chimera A-E57R_R58D-Fc(Eg1S)

<400>  212

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  213
<211>  1215
<212>  DNA
<213>  Artificial Sequence

<220>
<221>  CDS
<222>  (1)..(1215)

<220>

<223>  Nucleotide sequence of Chimera A-E57K-Fc(Eg1S YTE)

<400>  213
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aga tgc cgc tat tgt aac gtt
192
Tyr Thr Gln Phe Trp Asn Tyr Leu Lys Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576

Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc tat atc act cgg gag cct gag gtc aca tgc gtg gtg gtg gac gtg
672
Leu Tyr Ile Thr Arg Glu Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152

Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405

<210>  214
<211>  405
<212>  PRT
<213>  Artificial Sequence

<220>
<223>  Amino acid sequence of Chimera A-E57K-Fc(Eg1S YTE)

<400>  214

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Tyr Ile Thr Arg Glu Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  215
<211>  1215
<212>  DNA
<213>  Artificial Sequence

<220>
<221>  CDS
<222>  (1)..(1215)

<220>
<223>  Nucleotide sequence of Chimera A-E57L-Fc(Eg1S YTE)

<400>  215
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg aga tgc cgc tat tgt aac gtt
192
Tyr Thr Gln Phe Trp Asn Tyr Leu Leu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc tat atc act cgg gag cct gag gtc aca tgc gtg gtg gtg gac gtg
672
Leu Tyr Ile Thr Arg Glu Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405

<210>  216
<211>  405
<212>  PRT
<213>  Artificial Sequence

<220>
<223>  Amino acid sequence of Chimera A-E57L-Fc(Eg1S YTE)

<400>  216

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Tyr Ile Thr Arg Glu Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  217
<211>  1215
<212>  DNA
<213>  Artificial Sequence

<220>
<221>  CDS
<222>  (1)..(1215)

<220>
<223>  Nucleotide sequence of Chimera A-E57R-Fc(Eg1S YTE)

<400>  217
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg aga tgc cgc tat tgt aac gtt
192
Tyr Thr Gln Phe Trp Asn Tyr Leu Arg Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc tat atc act cgg gag cct gag gtc aca tgc gtg gtg gtg gac gtg
672
Leu Tyr Ile Thr Arg Glu Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405


<210>  218
<211>  405
<212>  PRT
<213>  Artificial Sequence

<220>
<223>  Amino acid sequence of Chimera A-E57R-Fc(Eg1S YTE)

<400>  218

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Tyr Ile Thr Arg Glu Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  219
<211>  1215
<212>  DNA
<213>  Artificial Sequence

<220>
<221>  CDS
<222>  (1)..(1215)

<220>
<223>  Nucleotide sequence of Chimera A-E57V-Fc(Eg1S YTE)

<400>  219
gag acc ttt ccc cct aag tac ctc cat tac gac gaa gaa aca agc cac
48

Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg aga tgc cgc tat tgt aac gtt
192
Tyr Thr Gln Phe Trp Asn Tyr Leu Val Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624

Leu Gly Gly Pro Ser Val Phe Leu Phe Pro Pro Lys Pro Lys Asp Thr
        195                 200                 205

ctc tat atc act cgg gag cct gag gtc aca tgc gtg gtg gtg gac gtg
672
Leu Tyr Ile Thr Arg Glu Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200

Val Met His Glu Ala Leu His Asn His Tyr Thr Gln Lys Ser Leu Ser
385                 390                 395                 400

ctg tct ccg ggt aaa
1215
Leu Ser Pro Gly Lys
                405

<210>  220
<211>  405
<212>  PRT
<213>  Artificial Sequence

<220>
<223>  Amino acid sequence of Chimera A-E57V-Fc(Eg1S YTE)

<400>  220

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys

                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Tyr Ile Thr Arg Glu Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys

                405

<210>  221
<211>  1215
<212>  DNA
<213>  Artificial Sequence

<220>
<221>  CDS
<222>  (1)..(1215)

<220>
<223>  Nucleotide sequence of Chimera A-E57K_R58D-Fc(Eg1S YTE)

<400>  221
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gac tgc cgc tat tgt aac gtt
192
Tyr Thr Gln Phe Trp Asn Tyr Leu Lys Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc tat atc act cgg gag cct gag gtc aca tgc gtg gtg gtg gac gtg
672
Leu Tyr Ile Thr Arg Glu Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405

<210> 222
<211> 405
<212> PRT
<213> Artificial Sequence

<220>
<223> Amino acid sequence of Chimera A-E57K_R58D-Fc(Eg1S YTE)

<400> 222

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Tyr Ile Thr Arg Glu Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210> 223
<211> 1215
<212> DNA
<213> Artificial Sequence

<220>
<221> CDS
<222> (1)..(1215)

<220>
<223> Nucleotide sequence of Chimera A-E57K_R58E-Fc(Eg1S YTE)

<400> 223
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gag tgc cgc tat tgt aac gtt
192
Tyr Thr Gln Phe Trp Asn Tyr Leu Lys Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc tat atc act cgg gag cct gag gtc aca tgc gtg gtg gtg gac gtg
672
Leu Tyr Ile Thr Arg Glu Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215
Leu Ser Pro Gly Lys
                405


<210>  224
<211>  405
<212>  PRT
<213>  Artificial Sequence

<220>
<223>  Amino acid sequence of Chimera A-E57K_R58E-Fc(Eg1S YTE)

<400>  224

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Tyr Ile Thr Arg Glu Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  225
<211>  1215
<212>  DNA
<213>  Artificial Sequence

<220>
<221>  CDS
<222>  (1)..(1215)

<220>
<223>  Nucleotide sequence of Chimera A-E57R_R58D-Fc(Eg1S YTE)

<400>  225
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96

Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg gac tgc cgc tat tgt aac gtt
192
Tyr Thr Gln Phe Trp Asn Tyr Leu Arg Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc tat atc act cgg gag cct gag gtc aca tgc gtg gtg gtg gac gtg
672

Leu Tyr Ile Thr Arg Glu Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggt aaa
1215

Leu Ser Pro Gly Lys
                405

<210>  226
<211>  405
<212>  PRT
<213>  Artificial Sequence

<220>
<223>  Amino acid sequence of Chimera A-E57R_R58D-Fc(Eg1S YTE)

<400>  226

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Tyr Ile Thr Arg Glu Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  227
<211>  1215

<212>  DNA
<213>  Artificial Sequence

<220>
<221>  CDS
<222>  (1)..(1215)

<220>
<223>  Nucleotide sequence of Chimera A-E57K-Fc(Eg1S N434A)

<400>  227
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aga tgc cgc tat tgt aac gtt
192
Tyr Thr Gln Phe Trp Asn Tyr Leu Lys Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405

<210>  228
<211>  405
<212>  PRT
<213>  Artificial Sequence

<220>
<223>  Amino acid sequence of Chimera A-E57K-Fc(Eg1S N434A)

<400>  228

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  229
<211>  1215
<212>  DNA
<213>  Artificial Sequence

<220>
<221>  CDS
<222>  (1)..(1215)

<220>
<223>  Nucleotide sequence of Chimera A-E57L-Fc(Eg1S N434A)

<400>  229
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg aga tgc cgc tat tgt aac gtt
192
Tyr Thr Gln Phe Trp Asn Tyr Leu Leu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405


<210>  230
<211>  405
<212>  PRT
<213>  Artificial Sequence

<220>
<223>  Amino acid sequence of Chimera A-E57L-Fc(Eg1S N434A)

<400>  230

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His

35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala

275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  231
<211>  1215
<212>  DNA
<213>  Artificial Sequence

<220>
<221>  CDS
<222>  (1)..(1215)

<220>
<223>  Nucleotide sequence of Chimera A-E57R-Fc(Eg1S N434A)

<400>  231
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144

Cys Thr Ala Lys Trp Lys Thr Val Cys Ala Pro Cys Pro Pro Arg His
        35                  40                  45

tac act cag ttt tgg aat tac ctg cgg aga tgc cgc tat tgt aac gtt
192
Tyr Thr Gln Phe Trp Asn Tyr Leu Arg Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720

Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405

<210>  232

<211>  405
<212>  PRT
<213>  Artificial Sequence

<220>
<223>  Amino acid sequence of Chimera A-E57R-Fc(Eg1S N434A)

<400>  232

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  233
<211>  1215
<212>  DNA
<213>  Artificial Sequence

<220>
<221>  CDS

<222>  (1)..(1215)

<220>
<223>  Nucleotide sequence of Chimera A-E57V-Fc(Eg1S N434A)

<400>  233
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg aga tgc cgc tat tgt aac gtt
192
Tyr Thr Gln Phe Trp Asn Tyr Leu Val Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405

<210>  234
<211>  405
<212>  PRT
<213>  Artificial Sequence

<220>
<223>  Amino acid sequence of Chimera A-E57V-Fc(Eg1S N434A)

<400>  234

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn

130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser

370                 375                 380

Val Met His Glu Ala Leu His Ala His Tyr Thr Gln Lys Ser Leu Ser
385                 390                 395                 400

Leu Ser Pro Gly Lys
                405

<210>  235
<211>  1215
<212>  DNA
<213>  Artificial Sequence

<220>
<221>  CDS
<222>  (1)..(1215)

<220>
<223>  Nucleotide sequence of Chimera A-E57K_R58D-Fc(Eg1S N434A)

<400>  235
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gac tgc cgc tat tgt aac gtt
192
Tyr Thr Gln Phe Trp Asn Tyr Leu Lys Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405


<210>  236
<211>  405
<212>  PRT
<213>  Artificial Sequence

<220>
<223>  Amino acid sequence of Chimera A-E57K_R58D-Fc(Eg1S N434A)

<400>  236

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  237
<211>  1215
<212>  DNA
<213>  Artificial Sequence

<220>
<221>  CDS
<222>  (1)..(1215)

<220>
<223>  Nucleotide sequence of Chimera A-E57K_R58E-Fc(Eg1S N434A)

<400>  237
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gag tgc cgc tat tgt aac gtt
192

Tyr Thr Gln Phe Trp Asn Tyr Leu Lys Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768

Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405


<210>  238
<211>  405
<212>  PRT
<213>  Artificial Sequence

<220>

<223>  Amino acid sequence of Chimera A-E57K_R58E-Fc(Eg1S N434A)

<400>  238

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  239
<211>  1215
<212>  DNA
<213>  Artificial Sequence

<220>
<221>  CDS
<222>  (1)..(1215)

<220>
<223>  Nucleotide sequence of Chimera A-E57R_R58D-Fc(Eg1S N434A)

<400>  239
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg gac tgc cgc tat tgt aac gtt
192
Tyr Thr Gln Phe Trp Asn Tyr Leu Arg Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa ctc
576
Ser Ser Asp Lys Thr His Thr Cys Pro Pro Cys Pro Ala Pro Glu Leu
            180                 185                 190

ctg ggg gga ccg tca gtc ttc ctc ttc ccc cca aaa ccc aag gac acc
624
Leu Gly Gly Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405

<210>  240
<211>  405
<212>  PRT
<213>  Artificial Sequence

<220>
<223>  Amino acid sequence of Chimera A-E57R_R58D-Fc(Eg1S N434A)

<400>  240

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Leu
            180                 185                 190

Leu Gly Gly Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  241
<211>  1215
<212>  DNA
<213>  Artificial Sequence

<220>
<221>  CDS
<222>  (1)..(1215)

<220>
<223>  Nucleotide sequence of Chimera A-E57K-Fc(Eg1S LALAGA)

<400>  241
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aga tgc cgc tat tgt aac gtt
192
Tyr Thr Gln Phe Trp Asn Tyr Leu Lys Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggc aaa
1215
Leu Ser Pro Gly Lys
                405

<210>  242
<211>  405
<212>  PRT
<213>  Artificial Sequence

<220>
<223>  Amino acid sequence of Chimera A-E57K-Fc(Eg1S LALAGA)

<400>  242

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  243
<211>  1215
<212>  DNA
<213>  Artificial Sequence

<220>
<221>  CDS
<222>  (1)..(1215)

<220>
<223>  Nucleotide sequence of Chimera A-E57L-Fc(Eg1S LALAGA)

<400>  243
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg aga tgc cgc tat tgt aac gtt
192
Tyr Thr Gln Phe Trp Asn Tyr Leu Leu Arg Cys Arg Tyr Cys Asn Val
    50                  55                  60

ctg tgt ggt gag cgt gag gaa gaa gct cga gca tgc cat gct act cac
240

Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816

Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggc aaa
1215
Leu Ser Pro Gly Lys
                405


<210>  244
<211>  405
<212>  PRT
<213>  Artificial Sequence

<220>
<223>  Amino acid sequence of Chimera A-E57L-Fc(Eg1S LALAGA)

<400>  244

Glu Thr Phe Pro Pro Lys Tyr Leu His Tyr Asp Glu Glu Thr Ser His

1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser

245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  245
<211>  1215
<212>  DNA
<213>  Artificial Sequence

<220>
<221>  CDS
<222>  (1)..(1215)

<220>
<223>  Nucleotide sequence of Chimera A-E57R-Fc(Eg1S LALAGA)

<400>  245
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg aga tgc cgc tat tgt aac gtt
192
Tyr Thr Gln Phe Trp Asn Tyr Leu Arg Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggc aaa
1215
Leu Ser Pro Gly Lys
                405

<210>  246
<211>  405
<212>  PRT
<213>  Artificial Sequence

<220>
<223>  Amino acid sequence of Chimera A-E57R-Fc(Eg1S LALAGA)

<400>  246

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
        180                 185                 190

Ala Gly Ala Pro Ser Val Phe Leu Phe Pro Pro Lys Pro Lys Asp Thr
    195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
        260                 265                 270

Asn Gly Lys Glu Tyr Lys Cys Lys Val Ser Asn Lys Ala Leu Pro Ala
    275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
        340                 345                 350

Pro Pro Val Leu Asp Ser Asp Gly Ser Phe Phe Leu Tyr Ser Lys Leu
    355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  247
<211>  1215
<212>  DNA
<213>  Artificial Sequence

<220>
<221>  CDS
<222>  (1)..(1215)

<220>
<223>  Nucleotide sequence of Chimera A-E57V-Fc(Eg1S LALAGA)

<400>  247
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg aga tgc cgc tat tgt aac gtt
192
Tyr Thr Gln Phe Trp Asn Tyr Leu Val Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggc aaa
1215
Leu Ser Pro Gly Lys
                405


<210>  248
<211>  405
<212>  PRT
<213>  Artificial Sequence

<220>
<223>  Amino acid sequence of Chimera A-E57V-Fc(Eg1S LALAGA)

<400>  248

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Val Arg Cys Arg Tyr Cys Asn Val
    50                  55                  60


Leu Cys Gly Glu Arg Glu Glu Glu Ala Arg Ala Cys His Ala Thr His
65                  70                  75                  80


Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210> 249
<211> 1215
<212> DNA
<213> Artificial Sequence

<220>
<221> CDS
<222> (1)..(1215)

<220>
<223> Nucleotide sequence of Chimera A-E57K_R58D-Fc(Eg1S LALAGA)

<400> 249
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gac tgc cgc tat tgt aac gtt
192
Tyr Thr Gln Phe Trp Asn Tyr Leu Lys Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288

Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864

Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggc aaa
1215
Leu Ser Pro Gly Lys
                405


<210>  250
<211>  405
<212>  PRT
<213>  Artificial Sequence

<220>
<223>  Amino acid sequence of Chimera A-E57K_R58D-Fc(Eg1S LALAGA)

<400>  250

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  251
<211>  1215
<212>  DNA
<213>  Artificial Sequence

<220>
<221>  CDS
<222>  (1)..(1215)

<220>
<223>  Nucleotide sequence of Chimera A-E57K_R58E-Fc(Eg1S LALAGA)

<400>  251
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gag tgc cgc tat tgt aac gtt
192
Tyr Thr Gln Phe Trp Asn Tyr Leu Lys Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggc aaa
1215
Leu Ser Pro Gly Lys
                405

<210>  252
<211>  405
<212>  PRT
<213>  Artificial Sequence

<220>
<223>  Amino acid sequence of Chimera A-E57K_R58E-Fc(Eg1S LALAGA)

<400>  252

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  253
<211>  1215
<212>  DNA
<213>  Artificial Sequence

<220>
<221>  CDS
<222>  (1)..(1215)

<220>
<223>  Nucleotide sequence of Chimera A-E57R_R58D-Fc(Eg1S LALAGA)

<400>  253
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg gac tgc cgc tat tgt aac gtt
192
Tyr Thr Gln Phe Trp Asn Tyr Leu Arg Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac aac cac tac acg cag aag agc ctc tcc
1200
Val Met His Glu Ala Leu His Asn His Tyr Thr Gln Lys Ser Leu Ser
385                 390                 395                 400

ctg tct ccg ggc aaa
1215
Leu Ser Pro Gly Lys
                405

<210>  254
<211>  405
<212>  PRT
<213>  Artificial Sequence

<220>
<223>  Amino acid sequence of Chimera A-E57R_R58D-Fc(Eg1S LALAGA)

<400>  254

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe

115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu

355                 360                 365

Thr Val Asp Lys Ser Arg Trp Gln Gln Gly Asn Val Phe Ser Cys Ser
    370                 375                 380

Val Met His Glu Ala Leu His Asn His Tyr Thr Gln Lys Ser Leu Ser
385                 390                 395                 400

Leu Ser Pro Gly Lys
                405

<210>  255
<211>  1215
<212>  DNA
<213>  Artificial Sequence

<220>
<221>  CDS
<222>  (1)..(1215)

<220>
<223>  Nucleotide sequence of Chimera A-E57K-Fc(Eg1S LALAGANA)

<400>  255
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aga tgc cgc tat tgt aac gtt
192
Tyr Thr Gln Phe Trp Asn Tyr Leu Lys Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336

Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912

Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405


<210>  256
<211>  405
<212>  PRT
<213>  Artificial Sequence

<220>
<223>  Amino acid sequence of Chimera A-E57K-Fc(Eg1S LALAGANA)

<400>  256

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  257
<211>  1215
<212>  DNA
<213>  Artificial Sequence

<220>
<221>  CDS
<222>  (1)..(1215)

<220>
<223>  Nucleotide sequence of Chimera A-E57L-Fc(Eg1S LALAGANA)

<400>  257
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg aga tgc cgc tat tgt aac gtt
192
Tyr Thr Gln Phe Trp Asn Tyr Leu Leu Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405


<210>  258
<211>  405
<212>  PRT

<213>  Artificial Sequence

<220>
<223>  Amino acid sequence of Chimera A-E57L-Fc(Eg1S LALAGANA)

<400>  258

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val

210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  259
<211>  1215
<212>  DNA
<213>  Artificial Sequence

<220>
<221>  CDS
<222>  (1)..(1215)

<220>
<223>  Nucleotide sequence of Chimera A-E57R-Fc(Eg1S LALAGANA)

<400>  259
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg aga tgc cgc tat tgt aac gtt
192
Tyr Thr Gln Phe Trp Asn Tyr Leu Arg Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405

<210>  260
<211>  405
<212>  PRT
<213>  Artificial Sequence

<220>
<223>  Amino acid sequence of Chimera A-E57R-Fc(Eg1S LALAGANA)

<400>  260

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  261
<211>  1215
<212>  DNA
<213>  Artificial Sequence

<220>
<221>  CDS
<222>  (1)..(1215)

<220>
<223>  Nucleotide sequence of Chimera A-E57V-Fc(Eg1S LALAGANA)

<400>  261
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg aga tgc cgc tat tgt aac gtt
192
Tyr Thr Gln Phe Trp Asn Tyr Leu Val Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384

Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960

Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405

<210>  262
<211>  405
<212>  PRT
<213>  Artificial Sequence

<220>
<223>  Amino acid sequence of Chimera A-E57V-Fc(Eg1S LALAGANA)

<400>  262

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  263
<211>  1215
<212>  DNA
<213>  Artificial Sequence

<220>
<221>  CDS
<222>  (1)..(1215)

<220>
<223>  Nucleotide sequence of Chimera A-E57K_R58D-Fc(Eg1S LALAGANA)

<400>  263
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gac tgc cgc tat tgt aac gtt
192
Tyr Thr Gln Phe Trp Asn Tyr Leu Lys Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405


<210>  264
<211>  405
<212>  PRT
<213>  Artificial Sequence

<220>
<223>  Amino acid sequence of Chimera A-E57K_R58D-Fc(Eg1S LALAGANA)

<400>  264

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  265
<211>  1215
<212>  DNA
<213>  Artificial Sequence

<220>
<221>  CDS
<222>  (1)..(1215)

<220>
<223>  Nucleotide sequence of Chimera A-E57K_R58E-Fc(Eg1S LALAGANA)

<400>  265

gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag gag tgc cgc tat tgt aac gtt
192
Tyr Thr Gln Phe Trp Asn Tyr Leu Lys Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008
Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405

<210>  266
<211>  405
<212>  PRT
<213>  Artificial Sequence

<220>
<223>  Amino acid sequence of Chimera A-E57K_R58E-Fc(Eg1S LALAGANA)

<400>  266

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala
                325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  267
<211>  1215
<212>  DNA
<213>  Artificial Sequence

<220>
<221>  CDS
<222>  (1)..(1215)

<220>
<223>  Nucleotide sequence of Chimera A-E57R_R58D-Fc(Eg1S LALAGANA)

<400>  267
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cgg gac tgc cgc tat tgt aac gtt
192
Tyr Thr Gln Phe Trp Asn Tyr Leu Arg Asp Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432

Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag ccc aaa
528
Asp Asn Ile Cys Ser Gly Asn Ser Glu Ser Thr Gln Lys Glu Pro Lys
                165                 170                 175

tct tcc gac aaa act cac aca tgc cca ccg tgc cca gca cct gaa gcc
576
Ser Ser Asp Lys Thr His Thr Cys Pro Pro Cys Pro Ala Pro Glu Ala
            180                 185                 190

gct ggg gcc ccg tca gtc ttc ctc ttc ccc cca aaa ccc aag gac acc
624
Ala Gly Ala Pro Ser Val Phe Leu Phe Pro Pro Lys Pro Lys Asp Thr
        195                 200                 205

ctc atg atc tcc cgg acc cct gag gtc aca tgc gtg gtg gtg gac gtg
672
Leu Met Ile Ser Arg Thr Pro Glu Val Thr Cys Val Val Val Asp Val
    210                 215                 220

agc cac gaa gac cct gag gtc aag ttc aac tgg tac gtg gac ggc gtg
720
Ser His Glu Asp Pro Glu Val Lys Phe Asn Trp Tyr Val Asp Gly Val
225                 230                 235                 240

gag gtg cat aat gcc aag aca aag ccg cgg gag gag cag tac aac agc
768
Glu Val His Asn Ala Lys Thr Lys Pro Arg Glu Glu Gln Tyr Asn Ser
                245                 250                 255

acg tac cgt gtg gtc agc gtc ctc acc gtc ctg cac cag gac tgg ctg
816
Thr Tyr Arg Val Val Ser Val Leu Thr Val Leu His Gln Asp Trp Leu
            260                 265                 270

aat ggc aag gag tac aag tgc aag gtc tcc aac aaa gcc ctc cca gcc
864
Asn Gly Lys Glu Tyr Lys Cys Lys Val Ser Asn Lys Ala Leu Pro Ala
        275                 280                 285

ccc atc gag aaa acc atc tcc aaa gcc aaa ggg cag ccc cga gaa cca
912
Pro Ile Glu Lys Thr Ile Ser Lys Ala Lys Gly Gln Pro Arg Glu Pro
    290                 295                 300

cag gtg tac acc ctg ccc cca tcc cgg gat gag ctg acc aag aac cag
960
Gln Val Tyr Thr Leu Pro Pro Ser Arg Asp Glu Leu Thr Lys Asn Gln
305                 310                 315                 320

gtc agc ctg acc tgc ctg gtc aaa ggc ttc tat ccc agc gac atc gcc
1008

Val Ser Leu Thr Cys Leu Val Lys Gly Phe Tyr Pro Ser Asp Ile Ala
                325                 330                 335

gtg gag tgg gag agc aat ggg cag ccg gag aac aac tac aag acc acg
1056
Val Glu Trp Glu Ser Asn Gly Gln Pro Glu Asn Asn Tyr Lys Thr Thr
            340                 345                 350

cct ccc gtg ctg gac tcc gac ggc tcc ttc ttc ctc tac agc aag ctc
1104
Pro Pro Val Leu Asp Ser Asp Gly Ser Phe Phe Leu Tyr Ser Lys Leu
        355                 360                 365

acc gtg gac aag agc agg tgg cag cag ggg aac gtc ttc tca tgc tcc
1152
Thr Val Asp Lys Ser Arg Trp Gln Gln Gly Asn Val Phe Ser Cys Ser
    370                 375                 380

gtg atg cat gag gct ctg cac gcc cac tac acg cag aag agc ctc tcc
1200
Val Met His Glu Ala Leu His Ala His Tyr Thr Gln Lys Ser Leu Ser
385                 390                 395                 400

ctg tct ccg ggt aaa
1215
Leu Ser Pro Gly Lys
                405


<210>  268
<211>  405
<212>  PRT
<213>  Artificial Sequence

<220>
<223>  Amino acid sequence of Chimera A-E57R_R58D-Fc(Eg1S LALAGANA)

<400>  268

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Arg Asp Cys Arg Tyr Cys Asn Val
    50                  55                  60


Leu Cys Gly Glu Arg Glu Glu Glu Ala Arg Ala Cys His Ala Thr His
65                  70                  75                  80


Asn Arg Ala Cys Arg Cys Arg Thr Gly Phe Phe Ala His Ala Gly Phe

                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Pro Lys
                165                 170                 175

Ser Ser Asp Lys Thr His Thr Cys Pro Pro Cys Pro Ala Pro Glu Ala
            180                 185                 190

Ala Gly Ala Pro Ser Val Phe Leu Phe Pro Pro Lys Pro Lys Asp Thr
        195                 200                 205

Leu Met Ile Ser Arg Thr Pro Glu Val Thr Cys Val Val Val Asp Val
    210                 215                 220

Ser His Glu Asp Pro Glu Val Lys Phe Asn Trp Tyr Val Asp Gly Val
225                 230                 235                 240

Glu Val His Asn Ala Lys Thr Lys Pro Arg Glu Glu Gln Tyr Asn Ser
                245                 250                 255

Thr Tyr Arg Val Val Ser Val Leu Thr Val Leu His Gln Asp Trp Leu
            260                 265                 270

Asn Gly Lys Glu Tyr Lys Cys Lys Val Ser Asn Lys Ala Leu Pro Ala
        275                 280                 285

Pro Ile Glu Lys Thr Ile Ser Lys Ala Lys Gly Gln Pro Arg Glu Pro
    290                 295                 300

Gln Val Tyr Thr Leu Pro Pro Ser Arg Asp Glu Leu Thr Lys Asn Gln
305                 310                 315                 320

Val Ser Leu Thr Cys Leu Val Lys Gly Phe Tyr Pro Ser Asp Ile Ala

325                 330                 335

Val Glu Trp Glu Ser Asn Gly Gln Pro Glu Asn Asn Tyr Lys Thr Thr
            340                 345                 350

Pro Pro Val Leu Asp Ser Asp Gly Ser Phe Phe Leu Tyr Ser Lys Leu
        355                 360                 365

Thr Val Asp Lys Ser Arg Trp Gln Gln Gly Asn Val Phe Ser Cys Ser
    370                 375                 380

Val Met His Glu Ala Leu His Ala His Tyr Thr Gln Lys Ser Leu Ser
385                 390                 395                 400

Leu Ser Pro Gly Lys
                405

<210>  269
<211>  519
<212>  DNA
<213>  Artificial Sequence

<220>
<221>  CDS
<222>  (1)..(519)

<220>
<223>  Nucleotide sequence of Chimera A-E57A

<400>  269
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gcc aga tgc cgc tat tgt aac gtt
192
Tyr Thr Gln Phe Trp Asn Tyr Leu Ala Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  270
<211>  173
<212>  PRT
<213>  Artificial Sequence

<220>
<223>  Amino acid sequence of Chimera A-E57A

<400>  270

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Ala Arg Cys Arg Tyr Cys Asn Val
    50                  55                  60


Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  271
<211>  519
<212>  DNA
<213>  Artificial Sequence

<220>
<221>  CDS
<222>  (1)..(519)

<220>
<223>  Nucleotide sequence of Chimera A-E57F

<400>  271
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ttc aga tgc cgc tat tgt aac gtt
192
Tyr Thr Gln Phe Trp Asn Tyr Leu Phe Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  272
<211>  173
<212>  PRT
<213>  Artificial Sequence

<220>
<223>  Amino acid sequence of Chimera A-E57F

<400>  272

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Phe Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  273
<211>  519
<212>  DNA
<213>  Artificial Sequence

<220>
<221>  CDS
<222>  (1)..(519)

<220>
<223>  Nucleotide sequence of Chimera A-E57H

<400>  273
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cac aga tgc cgc tat tgt aac gtt
192

Tyr Thr Gln Phe Trp Asn Tyr Leu His Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  274
<211>  173
<212>  PRT
<213>  Artificial Sequence

<220>
<223>  Amino acid sequence of Chimera A-E57H

<400>  274

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45

Tyr Thr Gln Phe Trp Asn Tyr Leu His Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  275
<211>  519
<212>  DNA
<213>  Artificial Sequence

<220>
<221>  CDS
<222>  (1)..(519)

<220>
<223>  Nucleotide sequence of Chimera A-E57I

<400>  275
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg atc aga tgc cgc tat tgt aac gtt
192
Tyr Thr Gln Phe Trp Asn Tyr Leu Ile Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210>  276
<211>  173
<212>  PRT
<213>  Artificial Sequence

<220>
<223>  Amino acid sequence of Chimera A-E57I

<400>  276

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Ile Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  277
<211>  519
<212>  DNA
<213>  Artificial Sequence

<220>
<221>  CDS
<222>  (1)..(519)

<220>
<223>  Nucleotide sequence of Chimera A-E57M

<400>  277
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg atg aga tgc cgc tat tgt aac gtt
192
Tyr Thr Gln Phe Trp Asn Tyr Leu Met Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  278
<211>  173
<212>  PRT
<213>  Artificial Sequence

<220>
<223>  Amino acid sequence of Chimera A-E57M

<400>  278

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Met Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  279
<211>  519
<212>  DNA
<213>  Artificial Sequence

<220>
<221>  CDS
<222>  (1)..(519)

<220>
<223>  Nucleotide sequence of Chimera A-E57K_R58T

<400>  279
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96

Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aca tgc cgc tat tgt aac gtt
192
Tyr Thr Gln Phe Trp Asn Tyr Leu Lys Thr Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  280
<211>  173
<212>  PRT
<213>  Artificial Sequence

<220>
<223>  Amino acid sequence of Chimera A-E57K_R58T

<400>  280

Glu Thr Phe Pro Pro Lys Tyr Leu His Tyr Asp Glu Glu Thr Ser His

1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Thr Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170

<210>  281
<211>  519
<212>  DNA
<213>  Artificial Sequence

<220>
<221>  CDS
<222>  (1)..(519)

<220>
<223>  Nucleotide sequence of Chimera A-E57L_R58E

<400>  281
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg gag tgc cgc tat tgt aac gtt
192
Tyr Thr Gln Phe Trp Asn Tyr Leu Leu Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  282
<211>  173
<212>  PRT
<213>  Artificial Sequence

<220>
<223>  Amino acid sequence of Chimera A-E57L_R58E

<400>  282

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170


<210>  283
<211>  519
<212>  DNA
<213>  Artificial Sequence

<220>
<221>  CDS
<222>  (1)..(519)

<220>
<223>  Nucleotide sequence of Chimera A-E57V_R58T

<400>  283

gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg aca tgc cgc tat tgt aac gtt
192
Tyr Thr Gln Phe Trp Asn Tyr Leu Val Thr Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170


<210>  284
<211>  173
<212>  PRT
<213>  Artificial Sequence

<220>
<223>  Amino acid sequence of Chimera A-E57V_R58T

<400>  284

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Thr Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170


<210>  285
<211>  519
<212>  DNA
<213>  Artificial Sequence

<220>
<221>  CDS
<222>  (1)..(519)

<220>

<223>  Nucleotide sequence of Chimera A-E57V_R58E

<400>  285
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg gag tgc cgc tat tgt aac gtt
192
Tyr Thr Gln Phe Trp Asn Tyr Leu Val Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag
519
Asp Asn Ile Cys Ser Gly Asn Ser Glu Ser Thr Gln Lys
                165                 170

<210>  286

<211>  173
<212>  PRT
<213>  Artificial Sequence

<220>
<223>  Amino acid sequence of Chimera A-E57V_R58E

<400>  286

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys
                165                 170


<210>  287
<211>  1206
<212>  DNA
<213>  Artificial Sequence

<220>
<221>  CDS

<222>  (1)..(1206)

<220>
<223>  Nucleotide sequence of Chimera A-E57A-Fc(g4PEK)

<400>  287
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gcc aga tgc cgc tat tgt aac gtt
192
Tyr Thr Gln Phe Trp Asn Tyr Leu Ala Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  288
<211>  402
<212>  PRT
<213>  Artificial Sequence

<220>
<223>  Amino acid sequence of Chimera A-E57A-Fc(g4PEK)

<400>  288

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Ala Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn

130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His

370                 375                 380

Glu Ala Leu His Asn His Tyr Thr Gln Lys Ser Leu Ser Leu Ser Leu
385                 390                 395                 400

Gly Lys

<210>  289
<211>  1206
<212>  DNA
<213>  Artificial Sequence

<220>
<221>  CDS
<222>  (1)..(1206)

<220>
<223>  Nucleotide sequence of Chimera A-E57F-Fc(g4PEK)

<400>  289
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ttc aga tgc cgc tat tgt aac gtt
192
Tyr Thr Gln Phe Trp Asn Tyr Leu Phe Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys



<210>  290
<211>  402
<212>  PRT
<213>  Artificial Sequence

<220>
<223>  Amino acid sequence of Chimera A-E57F-Fc(g4PEK)

<400>  290

Glu Thr Phe Pro Pro Lys Tyr Leu His Tyr Asp Glu Glu Thr Ser His
1               5                   10                  15


Gln Leu Leu Cys Asp Lys Cys Pro Pro Gly Thr Tyr Leu Lys Gln His
            20                  25                  30


Cys Thr Ala Lys Trp Lys Thr Val Cys Ala Pro Cys Pro Pro Arg His
        35                  40                  45


Tyr Thr Gln Phe Trp Asn Tyr Leu Phe Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  291
<211>  1206
<212>  DNA
<213>  Artificial Sequence

<220>
<221>  CDS
<222>  (1)..(1206)

<220>
<223>  Nucleotide sequence of Chimera A-E57H-Fc(g4PEK)

<400>  291
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg cac aga tgc cgc tat tgt aac gtt
192

Tyr Thr Gln Phe Trp Asn Tyr Leu His Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768

Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  292
<211>  402
<212>  PRT
<213>  Artificial Sequence

<220>

<223>  Amino acid sequence of Chimera A-E57H-Fc(g4PEK)

<400>  292

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu His Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  293
<211>  1206
<212>  DNA
<213>  Artificial Sequence

<220>
<221>  CDS
<222>  (1)..(1206)

<220>
<223>  Nucleotide sequence of Chimera A-E57I-Fc(g4PEK)

<400>  293
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg atc aga tgc cgc tat tgt aac gtt
192
Tyr Thr Gln Phe Trp Asn Tyr Leu Ile Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  294
<211>  402
<212>  PRT
<213>  Artificial Sequence

<220>
<223>  Amino acid sequence of Chimera A-E57I-Fc(g4PEK)

<400>  294

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Ile Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210> 295
<211> 1206
<212> DNA
<213> Artificial Sequence

<220>
<221> CDS
<222> (1)..(1206)

<220>
<223> Nucleotide sequence of Chimera A-E57M-Fc(g4PEK)

<400> 295
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg atg aga tgc cgc tat tgt aac gtt
192
Tyr Thr Gln Phe Trp Asn Tyr Leu Met Arg Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210> 296
<211> 402
<212> PRT
<213> Artificial Sequence

<220>
<223> Amino acid sequence of Chimera A-E57M-Fc(g4PEK)

<400> 296

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Met Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210> 297
<211> 1206
<212> DNA
<213> Artificial Sequence

<220>
<221> CDS
<222> (1)..(1206)

<220>
<223> Nucleotide sequence of Chimera A-E57K_R58T-Fc(g4PEK)

<400> 297
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg aag aca tgc cgc tat tgt aac gtt
192
Tyr Thr Gln Phe Trp Asn Tyr Leu Lys Thr Cys Arg Tyr Cys Asn Val
    50                  55                  60

ctg tgt ggt gag cgt gag gaa gaa gct cga gca tgc cat gct act cac
240

Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816

Val Val Ser Val Leu Thr Val Leu His Gln Asp Trp Leu Asn Gly Lys
                260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
                340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys



<210>  298
<211>  402
<212>  PRT
<213>  Artificial Sequence

<220>
<223>  Amino acid sequence of Chimera A-E57K_R58T-Fc(g4PEK)

<400>  298

Glu Thr Phe Pro Pro Lys Tyr Leu His Tyr Asp Glu Glu Thr Ser His

1                   5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Thr Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg

                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210> 299
<211> 1206
<212> DNA
<213> Artificial Sequence

<220>
<221> CDS
<222> (1)..(1206)

<220>
<223> Nucleotide sequence of Chimera A-E57L_R58E-Fc(g4PEK)

<400> 299
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg ctg gag tgc cgc tat tgt aac gtt
192
Tyr Thr Gln Phe Trp Asn Tyr Leu Leu Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210> 300
<211> 402
<212> PRT
<213> Artificial Sequence

<220>
<223> Amino acid sequence of Chimera A-E57L_R58E-Fc(g4PEK)

<400> 300

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210> 301
<211> 1206
<212> DNA
<213> Artificial Sequence

<220>
<221> CDS
<222> (1)..(1206)

<220>
<223> Nucleotide sequence of Chimera A-E57V_R58T-Fc(g4PEK)

<400> 301
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg aca tgc cgc tat tgt aac gtt
192
Tyr Thr Gln Phe Trp Asn Tyr Leu Val Thr Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288
Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864
Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
                340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
            355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  302
<211>  402
<212>  PRT
<213>  Artificial Sequence

<220>
<223>  Amino acid sequence of Chimera A-E57V_R58T-Fc(g4PEK)

<400>  302

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Thr Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210> 303
<211> 1206
<212> DNA
<213> Artificial Sequence

<220>
<221> CDS
<222> (1)..(1206)

<220>
<223> Nucleotide sequence of Chimera A-E57V_R58E-Fc(g4PEK)

<400> 303
gag acc ttt ccc cct aag tac ctc cat tac gac gaa gaa aca agc cac
48
Glu Thr Phe Pro Pro Lys Tyr Leu His Tyr Asp Glu Glu Thr Ser His
1               5                   10                  15

caa ctc ctc tgt gac aag tgc ccc cca ggg aca tat ttg aaa cag cac
96
Gln Leu Leu Cys Asp Lys Cys Pro Pro Gly Thr Tyr Leu Lys Gln His
            20                  25                  30

tgt acc gca aag tgg aaa aca gta tgc gcc cct tgc cca cca aga cac
144
Cys Thr Ala Lys Trp Lys Thr Val Cys Ala Pro Cys Pro Pro Arg His
        35                  40                  45

tac act cag ttt tgg aat tac ctg gtg gag tgc cgc tat tgt aac gtt
192
Tyr Thr Gln Phe Trp Asn Tyr Leu Val Glu Cys Arg Tyr Cys Asn Val
    50                  55                  60

ctg tgt ggt gag cgt gag gaa gaa gct cga gca tgc cat gct act cac
240
Leu Cys Gly Glu Arg Glu Glu Glu Ala Arg Ala Cys His Ala Thr His
65                  70                  75                  80

aat cgt gct tgc cgg tgt aga acc ggt ttt ttt gcc cat gca ggc ttc
288

Asn Arg Ala Cys Arg Cys Arg Thr Gly Phe Phe Ala His Ala Gly Phe
                85                  90                  95

tgc ctg gaa cat gca agc tgt ccc cct ggc gct ggc gtt atc gca ccc
336
Cys Leu Glu His Ala Ser Cys Pro Pro Gly Ala Gly Val Ile Ala Pro
            100                 105                 110

gga act cct agc cag aat aca caa tgt cag cct tgc cct gac ggc ttt
384
Gly Thr Pro Ser Gln Asn Thr Gln Cys Gln Pro Cys Pro Asp Gly Phe
        115                 120                 125

ttc tct aat gag act tca agt aaa gct ccc tgc cgc aag cac acc aac
432
Phe Ser Asn Glu Thr Ser Ser Lys Ala Pro Cys Arg Lys His Thr Asn
    130                 135                 140

tgt agt gtg ttt ggg ttg ttg ctg acc cag aag ggg aac gca act cac
480
Cys Ser Val Phe Gly Leu Leu Leu Thr Gln Lys Gly Asn Ala Thr His
145                 150                 155                 160

gac aac att tgt agc ggg aat tca gaa agc acc caa aag gag tcc aaa
528
Asp Asn Ile Cys Ser Gly Asn Ser Glu Ser Thr Gln Lys Glu Ser Lys
                165                 170                 175

tat ggt ccc cca tgc cca cca tgc cca gca cct gag ttc gag ggg gga
576
Tyr Gly Pro Pro Cys Pro Pro Cys Pro Ala Pro Glu Phe Glu Gly Gly
            180                 185                 190

cca tca gtc ttc ctg ttc ccc cca aaa ccc aag gac act ctc atg atc
624
Pro Ser Val Phe Leu Phe Pro Pro Lys Pro Lys Asp Thr Leu Met Ile
        195                 200                 205

tcc cgg acc cct gag gtc acg tgc gtg gtg gtg gac gtg agc cag gaa
672
Ser Arg Thr Pro Glu Val Thr Cys Val Val Val Asp Val Ser Gln Glu
    210                 215                 220

gac ccc gag gtc cag ttc aac tgg tac gtg gat ggc gtg gag gtg cat
720
Asp Pro Glu Val Gln Phe Asn Trp Tyr Val Asp Gly Val Glu Val His
225                 230                 235                 240

aat gcc aag aca aag ccg cgg gag gag cag ttc aac agc acg tac cgt
768
Asn Ala Lys Thr Lys Pro Arg Glu Glu Gln Phe Asn Ser Thr Tyr Arg
                245                 250                 255

gtg gtc agc gtc ctc acc gtc ctg cac cag gac tgg ctg aac ggc aag
816
Val Val Ser Val Leu Thr Val Leu His Gln Asp Trp Leu Asn Gly Lys
            260                 265                 270

gag tac aag tgc aag gtc tcc aac aaa ggc ctc ccg tcc tcc atc gag
864

Glu Tyr Lys Cys Lys Val Ser Asn Lys Gly Leu Pro Ser Ser Ile Glu
        275                 280                 285

aaa acc atc tcc aaa gcc aaa ggg cag ccc cga gag cca cag gtg tac
912
Lys Thr Ile Ser Lys Ala Lys Gly Gln Pro Arg Glu Pro Gln Val Tyr
    290                 295                 300

acc ctg ccc cca tcc cag gag gag atg acc aag aac cag gtc agc ctg
960
Thr Leu Pro Pro Ser Gln Glu Glu Met Thr Lys Asn Gln Val Ser Leu
305                 310                 315                 320

acc tgc ctg gtc aaa ggc ttc tac ccc agc gac atc gcc gtg gag tgg
1008
Thr Cys Leu Val Lys Gly Phe Tyr Pro Ser Asp Ile Ala Val Glu Trp
                325                 330                 335

gag agc aat ggg cag ccg gag aac aac tac aag acc acg cct ccc gtg
1056
Glu Ser Asn Gly Gln Pro Glu Asn Asn Tyr Lys Thr Thr Pro Pro Val
            340                 345                 350

ctg gac tcc gac ggc tcc ttc ttc ctc tac agc aag cta acc gtg gac
1104
Leu Asp Ser Asp Gly Ser Phe Phe Leu Tyr Ser Lys Leu Thr Val Asp
        355                 360                 365

aag agc agg tgg cag gag ggg aat gtc ttc tca tgc tcc gtg atg cat
1152
Lys Ser Arg Trp Gln Glu Gly Asn Val Phe Ser Cys Ser Val Met His
    370                 375                 380

gag gct ctg cac aac cac tac aca cag aag agc ctc tcc ctg tct ctg
1200
Glu Ala Leu His Asn His Tyr Thr Gln Lys Ser Leu Ser Leu Ser Leu
385                 390                 395                 400

ggt aaa
1206
Gly Lys

<210>  304
<211>  402
<212>  PRT
<213>  Artificial Sequence

<220>
<223>  Amino acid sequence of Chimera A-E57V_R58E-Fc(g4PEK)

<400>  304

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Glu Ser Lys
                165                 170                 175

Tyr Gly Pro Pro Cys Pro Pro Cys Pro Ala Pro Glu Phe Glu Gly Gly
            180                 185                 190

Pro Ser Val Phe Leu Phe Pro Pro Lys Pro Lys Asp Thr Leu Met Ile
        195                 200                 205

Ser Arg Thr Pro Glu Val Thr Cys Val Val Val Asp Val Ser Gln Glu
    210                 215                 220

Asp Pro Glu Val Gln Phe Asn Trp Tyr Val Asp Gly Val Glu Val His
225                 230                 235                 240

Asn Ala Lys Thr Lys Pro Arg Glu Glu Gln Phe Asn Ser Thr Tyr Arg
                245                 250                 255

Val Val Ser Val Leu Thr Val Leu His Gln Asp Trp Leu Asn Gly Lys
            260                 265                 270

Glu Tyr Lys Cys Lys Val Ser Asn Lys Gly Leu Pro Ser Ser Ile Glu
        275                 280                 285

Lys Thr Ile Ser Lys Ala Lys Gly Gln Pro Arg Glu Pro Gln Val Tyr
    290                 295                 300

Thr Leu Pro Pro Ser Gln Glu Glu Met Thr Lys Asn Gln Val Ser Leu
305                 310                 315                 320

Thr Cys Leu Val Lys Gly Phe Tyr Pro Ser Asp Ile Ala Val Glu Trp
                325                 330                 335

Glu Ser Asn Gly Gln Pro Glu Asn Asn Tyr Lys Thr Thr Pro Pro Val
            340                 345                 350

Leu Asp Ser Asp Gly Ser Phe Phe Leu Tyr Ser Lys Leu Thr Val Asp
        355                 360                 365

Lys Ser Arg Trp Gln Glu Gly Asn Val Phe Ser Cys Ser Val Met His
    370                 375                 380

Glu Ala Leu His Asn His Tyr Thr Gln Lys Ser Leu Ser Leu Ser Leu
385                 390                 395                 400

Gly Lys

<210>  305
<211>  528
<212>  DNA
<213>  Homo sapiens

<220>
<221>  CDS
<222>  (1)..(528)

<220>
<223>  Nucleotide sequence of soluble recombinant of LIGHT with FLAG tag

<400>  305
gac tac aaa gac gat gac gac aag ctt gac gga cct gca ggc tcc tgg
48
Asp Tyr Lys Asp Asp Asp Asp Lys Leu Asp Gly Pro Ala Gly Ser Trp
1               5                   10                  15

gag cag ctg ata caa gag cga agg tct cac gag gtc aac cca gca gcg
96
Glu Gln Leu Ile Gln Glu Arg Arg Ser His Glu Val Asn Pro Ala Ala
            20                  25                  30

cat ctc aca ggg gcc aac tcc agc ttg acc ggc agc ggg ggg ccg ctg
144
His Leu Thr Gly Ala Asn Ser Ser Leu Thr Gly Ser Gly Gly Pro Leu
        35                  40                  45

tta tgg gag act cag ctg ggc ctg gcc ttc ctg agg ggc ctc agc tac
192
Leu Trp Glu Thr Gln Leu Gly Leu Ala Phe Leu Arg Gly Leu Ser Tyr
    50                  55                  60

cac gat ggg gcc ctt gtg gtc acc aaa gct ggc tac tac tac atc tac
240
His Asp Gly Ala Leu Val Val Thr Lys Ala Gly Tyr Tyr Tyr Ile Tyr
65                  70                  75                  80

tcc aag gtg cag ctg ggc ggt gtg ggc tgc ccg ctg ggc ctg gcc agc
288
Ser Lys Val Gln Leu Gly Gly Val Gly Cys Pro Leu Gly Leu Ala Ser
                85                  90                  95

acc atc acc cac ggc ctc tac aag cgc aca ccc cgc tac ccc gag gag
336
Thr Ile Thr His Gly Leu Tyr Lys Arg Thr Pro Arg Tyr Pro Glu Glu
            100                 105                 110

ctg gag ctg ttg gtc agc cag cag tca ccc tgc gga cgg gcc acc agc
384
Leu Glu Leu Leu Val Ser Gln Gln Ser Pro Cys Gly Arg Ala Thr Ser
        115                 120                 125

agc tcc cgg gtc tgg tgg gac agc agc ttc ctg ggt ggt gtg gta cac
432
Ser Ser Arg Val Trp Trp Asp Ser Ser Phe Leu Gly Gly Val Val His
    130                 135                 140

ctg gag gct ggg gag aag gtg gtc gtc cgt gtg ctg gat gaa cgc ctg
480
Leu Glu Ala Gly Glu Lys Val Val Val Arg Val Leu Asp Glu Arg Leu
145                 150                 155                 160

gtt cga ctg cgt gat ggt acc cgg tct tac ttc ggg gct ttc atg gtg
528
Val Arg Leu Arg Asp Gly Thr Arg Ser Tyr Phe Gly Ala Phe Met Val
                165                 170                 175


<210>  306
<211>  176
<212>  PRT
<213>  Homo sapiens

<220>
<223>  Amino acid sequence of soluble recombinant of LIGHT with FLAG tag

<400>  306

Asp Tyr Lys Asp Asp Asp Asp Lys Leu Asp Gly Pro Ala Gly Ser Trp
1               5                   10                  15

Glu Gln Leu Ile Gln Glu Arg Arg Ser His Glu Val Asn Pro Ala Ala
            20                  25                  30

His Leu Thr Gly Ala Asn Ser Ser Leu Thr Gly Ser Gly Gly Pro Leu
        35                  40                  45

Leu Trp Glu Thr Gln Leu Gly Leu Ala Phe Leu Arg Gly Leu Ser Tyr
    50                  55                  60

His Asp Gly Ala Leu Val Val Thr Lys Ala Gly Tyr Tyr Tyr Ile Tyr
65                  70                  75                  80

Ser Lys Val Gln Leu Gly Gly Val Gly Cys Pro Leu Gly Leu Ala Ser
                85                  90                  95

Thr Ile Thr His Gly Leu Tyr Lys Arg Thr Pro Arg Tyr Pro Glu Glu
            100                 105                 110

Leu Glu Leu Leu Val Ser Gln Gln Ser Pro Cys Gly Arg Ala Thr Ser
        115                 120                 125

Ser Ser Arg Val Trp Trp Asp Ser Ser Phe Leu Gly Gly Val Val His
    130                 135                 140

Leu Glu Ala Gly Glu Lys Val Val Val Arg Val Leu Asp Glu Arg Leu
145                 150                 155                 160

Val Arg Leu Arg Asp Gly Thr Arg Ser Tyr Phe Gly Ala Phe Met Val
                165                 170                 175

<210>  307
<211>  777
<212>  DNA
<213>  Homo sapiens

<220>
<221>  CDS
<222>  (1)..(777)

<220>
<223>  Nucleotide sequence of TL1A wherein FLAG tag has been inserted
into N-terminus thereof

<400>  307
atg gac tac aag gac gac gat gac aag gcc gag gat ctg gga ctg agc
48
Met Asp Tyr Lys Asp Asp Asp Asp Lys Ala Glu Asp Leu Gly Leu Ser
1               5                   10                  15

ttt ggg gaa aca gcc agt gtg gaa atg ctg cca gag cac ggc agc tgc
96
Phe Gly Glu Thr Ala Ser Val Glu Met Leu Pro Glu His Gly Ser Cys
            20                  25                  30

agg ccc aag gcc agg agc agc agc gca cgc tgg gct ctc acc tgc tgc
144
Arg Pro Lys Ala Arg Ser Ser Ser Ala Arg Trp Ala Leu Thr Cys Cys
        35                  40                  45

ctg gtg ttg ctc ccc ttc ctt gca gga ctc acc aca tac ctg ctt gtc
192
Leu Val Leu Leu Pro Phe Leu Ala Gly Leu Thr Thr Tyr Leu Leu Val
    50                  55                  60

agc cag ctc cgg gcc cag gga gag gcc tgt gtg cag ttc cag gct cta
240
Ser Gln Leu Arg Ala Gln Gly Glu Ala Cys Val Gln Phe Gln Ala Leu
65                  70                  75                  80

aaa gga cag gag ttt gca cct tca cat cag caa gtt tat gca cct ctt
288
Lys Gly Gln Glu Phe Ala Pro Ser His Gln Gln Val Tyr Ala Pro Leu
                85                  90                  95

aga gca gac gga gat aag cca agg gca cac ctg aca gtt gtg aga caa
336
Arg Ala Asp Gly Asp Lys Pro Arg Ala His Leu Thr Val Val Arg Gln
            100                 105                 110

act ccc aca cag cac ttt aaa aat cag ttc cca gct ctg cac tgg gaa
384
Thr Pro Thr Gln His Phe Lys Asn Gln Phe Pro Ala Leu His Trp Glu
        115                 120                 125

cat gaa cta ggc ctg gcc ttc acc aag aac cga atg aac tat acc aac
432
His Glu Leu Gly Leu Ala Phe Thr Lys Asn Arg Met Asn Tyr Thr Asn
    130                 135                 140

aaa ttc ctg ctg atc cca gag tcg gga gac tac ttc att tac tcc cag
480
Lys Phe Leu Leu Ile Pro Glu Ser Gly Asp Tyr Phe Ile Tyr Ser Gln
145                 150                 155                 160

gtc aca ttc cgt ggg atg acc tct gag tgc agt gaa atc aga caa gca
528
Val Thr Phe Arg Gly Met Thr Ser Glu Cys Ser Glu Ile Arg Gln Ala
                165                 170                 175

ggc cga cca aac aag cca gac tcc atc act gtg gtc atc acc aag gta
576
Gly Arg Pro Asn Lys Pro Asp Ser Ile Thr Val Val Ile Thr Lys Val
            180                 185                 190

aca gac agc tac cct gag cca acc cag ctc ctc atg ggg acc aag tct
624
Thr Asp Ser Tyr Pro Glu Pro Thr Gln Leu Leu Met Gly Thr Lys Ser
        195                 200                 205

gta tgc gaa gta ggt agc aac tgg ttc cag ccc atc tac ctc gga gcc
672
Val Cys Glu Val Gly Ser Asn Trp Phe Gln Pro Ile Tyr Leu Gly Ala
    210                 215                 220

atg ttc tcc ttg caa gaa ggg gac aag cta atg gtg aac gtc agt gac
720
Met Phe Ser Leu Gln Glu Gly Asp Lys Leu Met Val Asn Val Ser Asp
225                 230                 235                 240

atc tct ttg gtg gat tac aca aaa gaa gat aaa acc ttc ttt gga gcc
768
Ile Ser Leu Val Asp Tyr Thr Lys Glu Asp Lys Thr Phe Phe Gly Ala
                245                 250                 255

ttc tta cta
777
Phe Leu Leu

<210>  308
<211>  259
<212>  PRT
<213>  Homo sapiens

<220>
<223>  Amino acid sequence of TL1A wherein FLAG tag has been inserted
into N-terminus thereof

<400>  308

Met Asp Tyr Lys Asp Asp Asp Asp Lys Ala Glu Asp Leu Gly Leu Ser
1               5                   10                  15

Phe Gly Glu Thr Ala Ser Val Glu Met Leu Pro Glu His Gly Ser Cys
            20                  25                  30

Arg Pro Lys Ala Arg Ser Ser Ser Ala Arg Trp Ala Leu Thr Cys Cys
        35                  40                  45

Leu Val Leu Leu Pro Phe Leu Ala Gly Leu Thr Thr Tyr Leu Leu Val
    50                  55                  60

Ser Gln Leu Arg Ala Gln Gly Glu Ala Cys Val Gln Phe Gln Ala Leu
65                  70                  75                  80

Lys Gly Gln Glu Phe Ala Pro Ser His Gln Gln Val Tyr Ala Pro Leu
                85                  90                  95

Arg Ala Asp Gly Asp Lys Pro Arg Ala His Leu Thr Val Val Arg Gln
            100                 105                 110

Thr Pro Thr Gln His Phe Lys Asn Gln Phe Pro Ala Leu His Trp Glu
        115                 120                 125

His Glu Leu Gly Leu Ala Phe Thr Lys Asn Arg Met Asn Tyr Thr Asn
    130                 135                 140

Lys Phe Leu Leu Ile Pro Glu Ser Gly Asp Tyr Phe Ile Tyr Ser Gln
145                 150                 155                 160

Val Thr Phe Arg Gly Met Thr Ser Glu Cys Ser Glu Ile Arg Gln Ala
                165                 170                 175

Gly Arg Pro Asn Lys Pro Asp Ser Ile Thr Val Val Ile Thr Lys Val
            180                 185                 190

Thr Asp Ser Tyr Pro Glu Pro Thr Gln Leu Leu Met Gly Thr Lys Ser
        195                 200                 205

Val Cys Glu Val Gly Ser Asn Trp Phe Gln Pro Ile Tyr Leu Gly Ala
    210                 215                 220

Met Phe Ser Leu Gln Glu Gly Asp Lys Leu Met Val Asn Val Ser Asp
225                 230                 235                 240

Ile Ser Leu Val Asp Tyr Thr Lys Glu Asp Lys Thr Phe Phe Gly Ala
                245                 250                 255

Phe Leu Leu

<210>  309
<211>  867
<212>  DNA
<213>  Homo sapiens

<220>
<221>  CDS
<222>  (1)..(867)

<220>
<223>  Nucleotide sequence of FasL wherein FLAG tag has been inserted
into N-terminus thereof

<400>  309
atg gac tac aag gac gac gat gac aag cag cag ccc ttc aat tac cca
48
Met Asp Tyr Lys Asp Asp Asp Asp Lys Gln Gln Pro Phe Asn Tyr Pro
1               5                   10                  15

tat ccc cag atc tac tgg gtg gac agc agt gcc agc tct ccc tgg gcc
96
Tyr Pro Gln Ile Tyr Trp Val Asp Ser Ser Ala Ser Ser Pro Trp Ala
            20                  25                  30

cct cca ggc aca gtt ctt ccc tgt cca acc tct gtg ccc aga agg cct
144
Pro Pro Gly Thr Val Leu Pro Cys Pro Thr Ser Val Pro Arg Arg Pro
        35                  40                  45

ggt caa agg agg cca cca cca cca ccg cca ccg cca cca cta cca cct
192
Gly Gln Arg Arg Pro Pro Pro Pro Pro Pro Pro Pro Pro Leu Pro Pro
    50                  55                  60

ccg ccg ccg ccg cca cca ctg cct cca cta ccg ctg cca ccc ctg aag
240
Pro Pro Pro Pro Pro Pro Leu Pro Pro Leu Pro Leu Pro Pro Leu Lys
65                  70                  75                  80

aag aga ggg aac cac agc aca ggc ctg tgt ctc ctt gtg atg ttt ttc
288
Lys Arg Gly Asn His Ser Thr Gly Leu Cys Leu Leu Val Met Phe Phe
                85                  90                  95

atg gtt ctg gtt gcc ttg gta gga ttg ggc ctg ggg atg ttt cag ctc
336
Met Val Leu Val Ala Leu Val Gly Leu Gly Leu Gly Met Phe Gln Leu
            100                 105                 110

ttc cac cta cag aag gag ctg gca gaa ctc cga gag tct acc agc cag
384
Phe His Leu Gln Lys Glu Leu Ala Glu Leu Arg Glu Ser Thr Ser Gln
        115                 120                 125

atg cac aca gca tca tct ttg gag aag caa ata ggc cac ccc agt cca
432
Met His Thr Ala Ser Ser Leu Glu Lys Gln Ile Gly His Pro Ser Pro
    130                 135                 140

ccc cct gaa aaa aag gag ctg agg aaa gtg gcc cat tta aca ggc aag
480
Pro Pro Glu Lys Lys Glu Leu Arg Lys Val Ala His Leu Thr Gly Lys
145                 150                 155                 160

tcc aac tca agg tcc atg cct ctg gaa tgg gaa gac acc tat gga att
528
Ser Asn Ser Arg Ser Met Pro Leu Glu Trp Glu Asp Thr Tyr Gly Ile
                165                 170                 175

gtc ctg ctt tct gga gtg aag tat aag aag ggt ggc ctt gtg atc aat
576
Val Leu Leu Ser Gly Val Lys Tyr Lys Lys Gly Gly Leu Val Ile Asn
            180                 185                 190

gaa act ggg ctg tac ttt gta tat tcc aaa gta tac ttc cgg ggt caa
624
Glu Thr Gly Leu Tyr Phe Val Tyr Ser Lys Val Tyr Phe Arg Gly Gln
        195                 200                 205

tct tgc aac aac ctg ccc ctg agc cac aag gtc tac atg agg aac tct
672
Ser Cys Asn Asn Leu Pro Leu Ser His Lys Val Tyr Met Arg Asn Ser
    210                 215                 220

aag tat ccc cag gat ctg gtg atg atg gag ggg aag atg atg agc tac
720
Lys Tyr Pro Gln Asp Leu Val Met Met Glu Gly Lys Met Met Ser Tyr
225                 230                 235                 240

tgc act act ggg cag atg tgg gcc cgc agc agc tac ctg ggg gca gtg
768
Cys Thr Thr Gly Gln Met Trp Ala Arg Ser Ser Tyr Leu Gly Ala Val
                245                 250                 255

ttc aat ctt acc agt gct gat cat tta tat gtc aac gta tct gag ctc
816
Phe Asn Leu Thr Ser Ala Asp His Leu Tyr Val Asn Val Ser Glu Leu
            260                 265                 270

tct ctg gtc aat ttt gag gaa tct cag acg ttt ttc ggc tta tat aag
864
Ser Leu Val Asn Phe Glu Glu Ser Gln Thr Phe Phe Gly Leu Tyr Lys
        275                 280                 285

ctc
867
Leu

<210>  310
<211>  289
<212>  PRT
<213>  Homo sapiens

<220>
<223>  Amino acid sequence of FasL wherein FLAG tag has been inserted
into N-terminus thereof

<400>  310

Met Asp Tyr Lys Asp Asp Asp Asp Lys Gln Gln Pro Phe Asn Tyr Pro
1               5                   10                  15

Tyr Pro Gln Ile Tyr Trp Val Asp Ser Ser Ala Ser Ser Pro Trp Ala
            20                  25                  30

Pro Pro Gly Thr Val Leu Pro Cys Pro Thr Ser Val Pro Arg Arg Pro
        35                  40                  45

Gly Gln Arg Arg Pro Pro Pro Pro Pro Pro Pro Pro Pro Leu Pro Pro
    50                  55                  60

Pro Pro Pro Pro Pro Pro Leu Pro Pro Leu Pro Leu Pro Pro Leu Lys
65                  70                  75                  80

Lys Arg Gly Asn His Ser Thr Gly Leu Cys Leu Leu Val Met Phe Phe
                85                  90                  95

Met Val Leu Val Ala Leu Val Gly Leu Gly Leu Gly Met Phe Gln Leu
            100                 105                 110

Phe His Leu Gln Lys Glu Leu Ala Glu Leu Arg Glu Ser Thr Ser Gln
        115                 120                 125

Met His Thr Ala Ser Ser Leu Glu Lys Gln Ile Gly His Pro Ser Pro
    130                 135                 140

Pro Pro Glu Lys Lys Glu Leu Arg Lys Val Ala His Leu Thr Gly Lys
145                 150                 155                 160

Ser Asn Ser Arg Ser Met Pro Leu Glu Trp Glu Asp Thr Tyr Gly Ile
                165                 170                 175

Val Leu Leu Ser Gly Val Lys Tyr Lys Lys Gly Gly Leu Val Ile Asn
            180                 185                 190

Glu Thr Gly Leu Tyr Phe Val Tyr Ser Lys Val Tyr Phe Arg Gly Gln
        195                 200                 205

Ser Cys Asn Asn Leu Pro Leu Ser His Lys Val Tyr Met Arg Asn Ser
    210                 215                 220

Lys Tyr Pro Gln Asp Leu Val Met Met Glu Gly Lys Met Met Ser Tyr
225                 230                 235                 240

Cys Thr Thr Gly Gln Met Trp Ala Arg Ser Ser Tyr Leu Gly Ala Val
                245                 250                 255

Phe Asn Leu Thr Ser Ala Asp His Leu Tyr Val Asn Val Ser Glu Leu
            260                 265                 270

Ser Leu Val Asn Phe Glu Glu Ser Gln Thr Phe Phe Gly Leu Tyr Lys
        275                 280                 285

Leu

<210>  311
<211>  231

<212>  PRT
<213>  Artificial Sequence

<220>
<223>  Amino acid sequence of Fc(g1S YTE)

<400>  311

Pro Lys Ser Ser Asp Lys Thr His Thr Cys Pro Pro Cys Pro Ala Pro
1               5                   10                  15

Glu Leu Leu Gly Gly Pro Ser Val Phe Leu Phe Pro Pro Lys Pro Lys
            20                  25                  30

Asp Thr Leu Tyr Ile Thr Arg Glu Pro Glu Val Thr Cys Val Val Val
        35                  40                  45

Asp Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Asn Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Ala Leu
            100                 105                 110

Pro Ala Pro Ile Glu Lys Thr Ile Ser Lys Ala Lys Gly Gln Pro Arg
        115                 120                 125

Glu Pro Gln Val Tyr Thr Leu Pro Pro Ser Arg Asp Glu Leu Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Thr Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Asn His Tyr Thr Gln Lys Ser
    210                 215                 220

Leu Ser Leu Ser Pro Gly Lys
225                 230

<210>  312
<211>  231
<212>  PRT
<213>  Artificial Sequence

<220>
<223>  Amino acid sequence of Fc(g1S N434A)

<400>  312

Pro Lys Ser Ser Asp Lys Thr His Thr Cys Pro Pro Cys Pro Ala Pro
1               5                   10                  15

Glu Leu Leu Gly Gly Pro Ser Val Phe Leu Phe Pro Pro Lys Pro Lys
            20                  25                  30

Asp Thr Leu Met Ile Ser Arg Thr Pro Glu Val Thr Cys Val Val Val
        35                  40                  45

Asp Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Asn Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Ala Leu
            100                 105                 110

Pro Ala Pro Ile Glu Lys Thr Ile Ser Lys Ala Lys Gly Gln Pro Arg
        115                 120                 125

Glu Pro Gln Val Tyr Thr Leu Pro Pro Ser Arg Asp Glu Leu Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Thr Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Ala His Tyr Thr Gln Lys Ser
    210                 215                 220

Leu Ser Leu Ser Pro Gly Lys
225                 230

<210>  313
<211>  231
<212>  PRT
<213>  Artificial Sequence

<220>
<223>  Amino acid sequence of Fc(g1S LALAGANA)

<400>  313

Pro Lys Ser Ser Asp Lys Thr His Thr Cys Pro Pro Cys Pro Ala Pro
1               5                   10                  15

Glu Ala Ala Gly Ala Pro Ser Val Phe Leu Phe Pro Pro Lys Pro Lys
            20                  25                  30

Asp Thr Leu Met Ile Ser Arg Thr Pro Glu Val Thr Cys Val Val Val
        35                  40                  45

Asp Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Asn Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Ala Leu
            100                 105                 110

Pro Ala Pro Ile Glu Lys Thr Ile Ser Lys Ala Lys Gly Gln Pro Arg
        115                 120                 125

Glu Pro Gln Val Tyr Thr Leu Pro Pro Ser Arg Asp Glu Leu Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Thr Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Ala His Tyr Thr Gln Lys Ser
    210                 215                 220

Leu Ser Leu Ser Pro Gly Lys
225                 230

<210>  314
<211>  404
<212>  PRT
<213>  Artificial Sequence

<220>
<223>  Amino acid sequence of Chimera A-Fc(g1S YTE)

<400>  314

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Tyr Ile Thr Arg Glu Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210> 315
<211> 404
<212> PRT
<213> Artificial Sequence

<220>
<223> Amino acid sequence of Chimera A-Fc(g1S N434A)

<400> 315

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  316
<211>  404
<212>  PRT
<213>  Artificial Sequence

<220>
<223>  Amino acid sequence of Chimera A-Fc(g1S LALAGANA)

<400>  316

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Ala Ala
            180                 185                 190

Gly Ala Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  317
<211>  404
<212>  PRT
<213>  Artificial Sequence

<220>
<223>  Amino acid sequence of Chimera A-E57K-Fc(g1S YTE)

<400>  317

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Tyr Ile Thr Arg Glu Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210> 318
<211> 404
<212> PRT
<213> Artificial Sequence

<220>
<223> Amino acid sequence of Chimera A-E57L-Fc(g1S YTE)

<400> 318

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Tyr Ile Thr Arg Glu Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210>  319
<211>  404

<212>  PRT
<213>  Artificial Sequence

<220>
<223>  Amino acid sequence of Chimera A-E57R-Fc(g1S YTE)

<400>  319

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Tyr Ile Thr Arg Glu Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210>  320
<211>  404
<212>  PRT
<213>  Artificial Sequence

<220>
<223>  Amino acid sequence of Chimera A-E57V-Fc(g1S YTE)

<400>  320

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Tyr Ile Thr Arg Glu Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210>  321
<211>  404
<212>  PRT
<213>  Artificial Sequence

<220>
<223>  Amino acid sequence of Chimera A-E57K_R58D-Fc(g1S YTE)

<400>  321

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Tyr Ile Thr Arg Glu Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210>  322
<211>  404
<212>  PRT
<213>  Artificial Sequence

<220>
<223>  Amino acid sequence of Chimera A-E57K_R58E-Fc(g1S YTE)

<400>  322

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Tyr Ile Thr Arg Glu Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210>  323
<211>  404
<212>  PRT
<213>  Artificial Sequence

<220>
<223>  Amino acid sequence of Chimera A-E57R_R58D-Fc(g1S YTE)

<400>  323

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Tyr Ile Thr Arg Glu Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Asn His Tyr Thr Gln Lys Ser Leu Ser Leu
385                 390                 395                 400

Ser Pro Gly Lys

<210>  324
<211>  404
<212>  PRT
<213>  Artificial Sequence

<220>
<223>  Amino acid sequence of Chimera A-E57K-Fc(g1S N434A)

<400>  324

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
        340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  325
<211>  404
<212>  PRT
<213>  Artificial Sequence

<220>
<223>  Amino acid sequence of Chimera A-E57L-Fc(g1S N434A)

<400>  325

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  326
<211>  404
<212>  PRT
<213>  Artificial Sequence

<220>
<223>  Amino acid sequence of Chimera A-E57R-Fc(g1S N434A)

<400>  326

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  327
<211>  404
<212>  PRT
<213>  Artificial Sequence

<220>
<223>  Amino acid sequence of Chimera A-E57V-Fc(g1S N434A)

<400>  327

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210> 328
<211> 404
<212> PRT
<213> Artificial Sequence

<220>
<223> Amino acid sequence of Chimera A-E57K_R58D-Fc(g1S N434A)

<400> 328

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  329
<211>  404

<212>  PRT
<213>  Artificial Sequence

<220>
<223>  Amino acid sequence of Chimera A-E57K_R58E-Fc(g1S N434A)

<400>  329

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  330
<211>  404
<212>  PRT
<213>  Artificial Sequence

<220>
<223>  Amino acid sequence of Chimera A-E57R_R58D-Fc(g1S N434A)

<400>  330

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Leu Leu
            180                 185                 190

Gly Gly Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  331
<211>  404
<212>  PRT
<213>  Artificial Sequence

<220>
<223>  Amino acid sequence of Chimera A-E57K-Fc(g1S LALAGANA)

<400>  331

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Ala Ala
            180                 185                 190

Gly Ala Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  332
<211>  404
<212>  PRT
<213>  Artificial Sequence

<220>
<223>  Amino acid sequence of Chimera A-E57L-Fc(g1S LALAGANA)

<400>  332

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Leu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Ala Ala
            180                 185                 190

Gly Ala Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  333
<211>  404
<212>  PRT
<213>  Artificial Sequence

<220>
<223>  Amino acid sequence of Chimera A-E57R-Fc(g1S LALAGANA)

<400>  333

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Ala Ala
            180                 185                 190

Gly Ala Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  334
<211>  404
<212>  PRT
<213>  Artificial Sequence

<220>
<223>  Amino acid sequence of Chimera A-E57V-Fc(g1S LALAGANA)

<400>  334

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Val Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Ala Ala
            180                 185                 190

Gly Ala Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  335
<211>  404
<212>  PRT
<213>  Artificial Sequence

<220>
<223>  Amino acid sequence of Chimera A-E57K_R58D-Fc(g1S LALAGANA)

<400>  335

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Ala Ala
            180                 185                 190

Gly Ala Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  336
<211>  404
<212>  PRT
<213>  Artificial Sequence

<220>
<223>  Amino acid sequence of Chimera A-E57K_R58E-Fc(g1S LALAGANA)

<400>  336

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Ala Ala
            180                 185                 190

Gly Ala Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210>  337
<211>  404
<212>  PRT
<213>  Artificial Sequence

<220>
<223>  Amino acid sequence of Chimera A-E57R_R58D-Fc(g1S LALAGANA)

<400>  337

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Arg Asp Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys Pro Lys Ser
                165                 170                 175

Ser Asp Lys Thr His Thr Cys Pro Pro Cys Pro Ala Pro Glu Ala Ala
            180                 185                 190

Gly Ala Pro Ser Val Phe Leu Phe Pro Pro Lys Pro Lys Asp Thr Leu
        195                 200                 205

Met Ile Ser Arg Thr Pro Glu Val Thr Cys Val Val Val Asp Val Ser
    210                 215                 220

His Glu Asp Pro Glu Val Lys Phe Asn Trp Tyr Val Asp Gly Val Glu
225                 230                 235                 240

Val His Asn Ala Lys Thr Lys Pro Arg Glu Glu Gln Tyr Asn Ser Thr
                245                 250                 255

Tyr Arg Val Val Ser Val Leu Thr Val Leu His Gln Asp Trp Leu Asn
            260                 265                 270

Gly Lys Glu Tyr Lys Cys Lys Val Ser Asn Lys Ala Leu Pro Ala Pro
        275                 280                 285

Ile Glu Lys Thr Ile Ser Lys Ala Lys Gly Gln Pro Arg Glu Pro Gln
    290                 295                 300

Val Tyr Thr Leu Pro Pro Ser Arg Asp Glu Leu Thr Lys Asn Gln Val
305                 310                 315                 320

Ser Leu Thr Cys Leu Val Lys Gly Phe Tyr Pro Ser Asp Ile Ala Val
                325                 330                 335

Glu Trp Glu Ser Asn Gly Gln Pro Glu Asn Asn Tyr Lys Thr Thr Pro
            340                 345                 350

Pro Val Leu Asp Ser Asp Gly Ser Phe Phe Leu Tyr Ser Lys Leu Thr
        355                 360                 365

Val Asp Lys Ser Arg Trp Gln Gln Gly Asn Val Phe Ser Cys Ser Val
    370                 375                 380

Met His Glu Ala Leu His Ala His Tyr Thr Gln Lys Ser Leu Ser Leu
385                 390                 395                 400

Ser Pro Gly Lys

<210> 338
<211> 711
<212> DNA
<213> Artificial Sequence

<220>
<221> CDS
<222> (1)..(711)

<220>
<223> Nucleotide sequence of Fc(IEGRMD g1S)

<400> 338
atc gag ggg cgc atg gat ccc aaa tct tcc gac aaa act cac aca tgc
48
Ile Glu Gly Arg Met Asp Pro Lys Ser Ser Asp Lys Thr His Thr Cys
1               5                   10                  15

cca ccg tgc cca gca cct gaa ctc ctg ggg gga ccg tca gtc ttc ctc
96
Pro Pro Cys Pro Ala Pro Glu Leu Leu Gly Gly Pro Ser Val Phe Leu
            20                  25                  30

ttc ccc cca aaa ccc aag gac acc ctc atg atc tcc cgg acc cct gag
144
Phe Pro Pro Lys Pro Lys Asp Thr Leu Met Ile Ser Arg Thr Pro Glu
        35                  40                  45

gtc aca tgc gtg gtg gtg gac gtg agc cac gaa gac cct gag gtc aag
192
Val Thr Cys Val Val Val Asp Val Ser His Glu Asp Pro Glu Val Lys
    50                  55                  60

ttc aac tgg tac gtg gac ggc gtg gag gtg cat aat gcc aag aca aag
240
Phe Asn Trp Tyr Val Asp Gly Val Glu Val His Asn Ala Lys Thr Lys
65                  70                  75                  80

ccg cgg gag gag cag tac aac agc acg tac cgt gtg gtc agc gtc ctc
288
Pro Arg Glu Glu Gln Tyr Asn Ser Thr Tyr Arg Val Val Ser Val Leu
                85                  90                  95

acc gtc ctg cac cag gac tgg ctg aat ggc aag gag tac aag tgc aag
336
Thr Val Leu His Gln Asp Trp Leu Asn Gly Lys Glu Tyr Lys Cys Lys
            100                 105                 110

gtc tcc aac aaa gcc ctc cca gcc ccc atc gag aaa acc atc tcc aaa
384
Val Ser Asn Lys Ala Leu Pro Ala Pro Ile Glu Lys Thr Ile Ser Lys
        115                 120                 125

gcc aaa ggg cag ccc cga gaa cca cag gtg tac acc ctg ccc cca tcc
432
Ala Lys Gly Gln Pro Arg Glu Pro Gln Val Tyr Thr Leu Pro Pro Ser

130                 135                 140

cgg gat gag ctg acc aag aac cag gtc agc ctg acc tgc ctg gtc aaa
480
Arg Asp Glu Leu Thr Lys Asn Gln Val Ser Leu Thr Cys Leu Val Lys
145                 150                 155                 160

ggc ttc tat ccc agc gac atc gcc gtg gag tgg gag agc aat ggg cag
528
Gly Phe Tyr Pro Ser Asp Ile Ala Val Glu Trp Glu Ser Asn Gly Gln
                165                 170                 175

ccg gag aac aac tac aag acc acg cct ccc gtg ctg gac tcc gac ggc
576
Pro Glu Asn Asn Tyr Lys Thr Thr Pro Pro Val Leu Asp Ser Asp Gly
            180                 185                 190

tcc ttc ttc ctc tac agc aag ctc acc gtg gac aag agc agg tgg cag
624
Ser Phe Phe Leu Tyr Ser Lys Leu Thr Val Asp Lys Ser Arg Trp Gln
        195                 200                 205

cag ggg aac gtc ttc tca tgc tcc gtg atg cat gag gct ctg cac aac
672
Gln Gly Asn Val Phe Ser Cys Ser Val Met His Glu Ala Leu His Asn
    210                 215                 220

cac tac acg cag aag agc ctc tcc ctg tct ccg ggt aaa
711
His Tyr Thr Gln Lys Ser Leu Ser Leu Ser Pro Gly Lys
225                 230                 235


<210>  339
<211>  237
<212>  PRT
<213>  Artificial Sequence

<220>
<223>  Amino acid sequence of Fc(IEGRMD g1S)

<400>  339

Ile Glu Gly Arg Met Asp Pro Lys Ser Ser Asp Lys Thr His Thr Cys
1               5                   10                  15


Pro Pro Cys Pro Ala Pro Glu Leu Leu Gly Gly Pro Ser Val Phe Leu
            20                  25                  30


Phe Pro Pro Lys Pro Lys Asp Thr Leu Met Ile Ser Arg Thr Pro Glu
        35                  40                  45


Val Thr Cys Val Val Val Asp Val Ser His Glu Asp Pro Glu Val Lys
    50                  55                  60


Phe Asn Trp Tyr Val Asp Gly Val Glu Val His Asn Ala Lys Thr Lys

65                  70                  75                  80

Pro Arg Glu Glu Gln Tyr Asn Ser Thr Tyr Arg Val Val Ser Val Leu
                85                  90                  95

Thr Val Leu His Gln Asp Trp Leu Asn Gly Lys Glu Tyr Lys Cys Lys
            100                 105                 110

Val Ser Asn Lys Ala Leu Pro Ala Pro Ile Glu Lys Thr Ile Ser Lys
        115                 120                 125

Ala Lys Gly Gln Pro Arg Glu Pro Gln Val Tyr Thr Leu Pro Pro Ser
    130                 135                 140

Arg Asp Glu Leu Thr Lys Asn Gln Val Ser Leu Thr Cys Leu Val Lys
145                 150                 155                 160

Gly Phe Tyr Pro Ser Asp Ile Ala Val Glu Trp Glu Ser Asn Gly Gln
                165                 170                 175

Pro Glu Asn Asn Tyr Lys Thr Thr Pro Pro Val Leu Asp Ser Asp Gly
            180                 185                 190

Ser Phe Phe Leu Tyr Ser Lys Leu Thr Val Asp Lys Ser Arg Trp Gln
        195                 200                 205

Gln Gly Asn Val Phe Ser Cys Ser Val Met His Glu Ala Leu His Asn
    210                 215                 220

His Tyr Thr Gln Lys Ser Leu Ser Leu Ser Pro Gly Lys
225                 230                 235

<210>  340
<211>  508
<212>  PRT
<213>  Artifical Sequence

<220>
<223>  Amino acid sequence of R218Q-Fc

<400>  340

Val Ala Glu Thr Pro Thr Tyr Pro Trp Arg Asp Ala Glu Thr Gly Glu
1               5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser Cys Thr Gly Phe Pro Leu Ser Thr Arg Val
                165                 170                 175

Pro Gly Ala Glu Glu Cys Glu Arg Ala Val Ile Asp Phe Val Ala Phe
            180                 185                 190

Gln Asp Ile Ser Ile Lys Arg Leu Gln Arg Leu Leu Gln Ala Leu Glu
        195                 200                 205

Ala Pro Glu Gly Trp Gly Pro Thr Pro Gln Ala Gly Arg Ala Ala Leu
    210                 215                 220

Gln Leu Lys Leu Arg Arg Arg Leu Thr Glu Leu Leu Gly Ala Gln Asp
225                 230                 235                 240

Gly Ala Leu Leu Val Arg Leu Leu Gln Ala Leu Arg Val Ala Arg Met
                245                 250                 255

Pro Gly Leu Glu Arg Ser Val Arg Glu Arg Phe Leu Pro Val His Ile
            260                 265                 270

Glu Gly Arg Met Asp Pro Lys Ser Ser Asp Lys Thr His Thr Cys Pro
        275                 280                 285

Pro Cys Pro Ala Pro Glu Leu Leu Gly Gly Pro Ser Val Phe Leu Phe
    290                 295                 300

Pro Pro Lys Pro Lys Asp Thr Leu Met Ile Ser Arg Thr Pro Glu Val
305                 310                 315                 320

Thr Cys Val Val Val Asp Val Ser His Glu Asp Pro Glu Val Lys Phe
                325                 330                 335

Asn Trp Tyr Val Asp Gly Val Glu Val His Asn Ala Lys Thr Lys Pro
            340                 345                 350

Arg Glu Glu Gln Tyr Asn Ser Thr Tyr Arg Val Val Ser Val Leu Thr
        355                 360                 365

Val Leu His Gln Asp Trp Leu Asn Gly Lys Glu Tyr Lys Cys Lys Val
    370                 375                 380

Ser Asn Lys Ala Leu Pro Ala Pro Ile Glu Lys Thr Ile Ser Lys Ala
385                 390                 395                 400

Lys Gly Gln Pro Arg Glu Pro Gln Val Tyr Thr Leu Pro Pro Ser Arg
                405                 410                 415

Asp Glu Leu Thr Lys Asn Gln Val Ser Leu Thr Cys Leu Val Lys Gly
            420                 425                 430

Phe Tyr Pro Ser Asp Ile Ala Val Glu Trp Glu Ser Asn Gly Gln Pro
        435                 440                 445

Glu Asn Asn Tyr Lys Thr Thr Pro Pro Val Leu Asp Ser Asp Gly Ser
    450                 455                 460

Phe Phe Leu Tyr Ser Lys Leu Thr Val Asp Lys Ser Arg Trp Gln Gln
465                 470                 475                 480

Gly Asn Val Phe Ser Cys Ser Val Met His Glu Ala Leu His Asn His
                485                 490                 495

Tyr Thr Gln Lys Ser Leu Ser Leu Ser Pro Gly Lys
            500                 505

<210>  341
<211>  528
<212>  DNA
<213>  Artificial

<220>
<221>  CDS
<222>  (1)..(528)

<220>
<223>  Nucleotide sequence of FLAG-cynoLIGHT

<400>  341
gac tac aaa gac gat gac gac aag gac gga cac gga ggc tcc tgg gag
48
Asp Tyr Lys Asp Asp Asp Asp Lys Asp Gly His Gly Gly Ser Trp Glu
1               5                   10                  15

cag ctg ata caa gag cga agg tct cac cag aat aac cca gca gca cat
96
Gln Leu Ile Gln Glu Arg Arg Ser His Gln Asn Asn Pro Ala Ala His
            20                  25                  30

ctc aca ggg gcc aac tcc agc ttg acg ggc agc ggg gga ccg ctg cta
144
Leu Thr Gly Ala Asn Ser Ser Leu Thr Gly Ser Gly Gly Pro Leu Leu
        35                  40                  45

tgg gag acc cag ctg ggc ctg gcc ttc ctg agg ggc ctc gac tac cac
192
Trp Glu Thr Gln Leu Gly Leu Ala Phe Leu Arg Gly Leu Asp Tyr His
    50                  55                  60

gat ggg gcc ctt gtg gcc aac aag gct ggc tac tac tac atc tac tcc
240
Asp Gly Ala Leu Val Ala Asn Lys Ala Gly Tyr Tyr Tyr Ile Tyr Ser
65                  70                  75                  80

aag gtg cag ctg ggc ggt gtg ggc tgc ccg ctg ggc ctg gcc agc acc
288
Lys Val Gln Leu Gly Gly Val Gly Cys Pro Leu Gly Leu Ala Ser Thr
                85                  90                  95

atc acc cac ggc ctc tac aag cgc acg ccc cgc tac ccc gag gag ctg
336
Ile Thr His Gly Leu Tyr Lys Arg Thr Pro Arg Tyr Pro Glu Glu Leu
            100                 105                 110

gag ctg ttg gtc agc cag cag tca ccc tgc gga cgg gcc acc agc aac
384
Glu Leu Leu Val Ser Gln Gln Ser Pro Cys Gly Arg Ala Thr Ser Asn
        115                 120                 125

tcc cgc gtc tgg tgg gac agc agc ttc ctg ggt ggt gtg gtg cac ctg
432
Ser Arg Val Trp Trp Asp Ser Ser Phe Leu Gly Gly Val Val His Leu
    130                 135                 140

gag gtt ggg gag gag gtg gtc gtc cgt gtg ctg gat gag cgc ctg gtc
480
Glu Val Gly Glu Glu Val Val Val Arg Val Leu Asp Glu Arg Leu Val
145                 150                 155                 160

cga ctg cgt gat ggc acc cgg tct tac ttc ggg gct ttc atg gtg tga
528
Arg Leu Arg Asp Gly Thr Arg Ser Tyr Phe Gly Ala Phe Met Val
                165                 170                 175

<210>  342
<211>  175
<212>  PRT
<213>  Artificial

<220>
<223>  Amino acid sequence of FLAG-cynoLIGHT

<400>  342

Asp Tyr Lys Asp Asp Asp Asp Lys Asp Gly His Gly Gly Ser Trp Glu
1               5                   10                  15

Gln Leu Ile Gln Glu Arg Arg Ser His Gln Asn Asn Pro Ala Ala His
            20                  25                  30

Leu Thr Gly Ala Asn Ser Ser Leu Thr Gly Ser Gly Gly Pro Leu Leu
        35                  40                  45

Trp Glu Thr Gln Leu Gly Leu Ala Phe Leu Arg Gly Leu Asp Tyr His
    50                  55                  60

Asp Gly Ala Leu Val Ala Asn Lys Ala Gly Tyr Tyr Tyr Ile Tyr Ser
65                  70                  75                  80

Lys Val Gln Leu Gly Gly Val Gly Cys Pro Leu Gly Leu Ala Ser Thr
                85                  90                  95

Ile Thr His Gly Leu Tyr Lys Arg Thr Pro Arg Tyr Pro Glu Glu Leu
            100                 105                 110

Glu Leu Leu Val Ser Gln Gln Ser Pro Cys Gly Arg Ala Thr Ser Asn
        115                 120                 125

Ser Arg Val Trp Trp Asp Ser Ser Phe Leu Gly Gly Val Val His Leu
    130                 135                 140

Glu Val Gly Glu Glu Val Val Val Arg Val Leu Asp Glu Arg Leu Val
145                 150                 155                 160

Arg Leu Arg Asp Gly Thr Arg Ser Tyr Phe Gly Ala Phe Met Val
                165                 170                 175

<210>  343
<211>  666
<212>  DNA
<213>  Artificial

<220>
<221>  CDS
<222>  (1)..(666)

<220>
<223>  Nucleotide sequence of a synthetic construct obtained by deleting
a cleavage site and an intracellular proline-rich domain from FasL and
insert a FLAG tag into N-terminus of FasL

<400>  343
gac tac aag gac gac gat gac aag cag cag ccc ttc aat tac ccc ctg
48
Asp Tyr Lys Asp Asp Asp Asp Lys Gln Gln Pro Phe Asn Tyr Pro Leu
1               5                   10                  15

aag aag aga ggg aac cac agc aca ggc ctg tgt ctc ctt gtg atg ttt
96
Lys Lys Arg Gly Asn His Ser Thr Gly Leu Cys Leu Leu Val Met Phe
            20                  25                  30

ttc atg gtt ctg gtt gcc ttg gta gga ttg ggc ctg ggg atg ttt cag
144
Phe Met Val Leu Val Ala Leu Val Gly Leu Gly Leu Gly Met Phe Gln
        35                  40                  45

ctc ttc cac cta cag aag gag ctg gca gaa ctc cga gag tct acc agc
192
Leu Phe His Leu Gln Lys Glu Leu Ala Glu Leu Arg Glu Ser Thr Ser
    50                  55                  60

cag atg cac aca gca tca tct ttg ggc cac ccc agt cca ccc cct gaa
240
Gln Met His Thr Ala Ser Ser Leu Gly His Pro Ser Pro Pro Pro Glu
65                  70                  75                  80

aaa aag gag ctg agg aaa gtg gcc cat tta aca ggc aag tcc aac tca
288
Lys Lys Glu Leu Arg Lys Val Ala His Leu Thr Gly Lys Ser Asn Ser
                85                  90                  95

agg tcc atg cct ctg gaa tgg gaa gac acc tat gga att gtc ctg ctt
336
Arg Ser Met Pro Leu Glu Trp Glu Asp Thr Tyr Gly Ile Val Leu Leu
            100                 105                 110

tct gga gtg aag tat aag aag ggt ggc ctt gtg atc aat gaa act ggg
384
Ser Gly Val Lys Tyr Lys Lys Gly Gly Leu Val Ile Asn Glu Thr Gly
        115                 120                 125

ctg tac ttt gta tat tcc aaa gta tac ttc cgg ggt caa tct tgc aac
432
Leu Tyr Phe Val Tyr Ser Lys Val Tyr Phe Arg Gly Gln Ser Cys Asn
    130                 135                 140

aac ctg ccc ctg agc cac aag gtc tac atg agg aac tct aag tat ccc
480
Asn Leu Pro Leu Ser His Lys Val Tyr Met Arg Asn Ser Lys Tyr Pro
145                 150                 155                 160

cag gat ctg gtg atg atg gag ggg aag atg atg agc tac tgc act act
528
Gln Asp Leu Val Met Met Glu Gly Lys Met Met Ser Tyr Cys Thr Thr
                165                 170                 175

ggg cag atg tgg gcc cgc agc agc tac ctg ggg gca gtg ttc aat ctt
576
Gly Gln Met Trp Ala Arg Ser Ser Tyr Leu Gly Ala Val Phe Asn Leu
            180                 185                 190

acc agt gct gat cat tta tat gtc aac gta tct gag ctc tct ctg gtc
624
Thr Ser Ala Asp His Leu Tyr Val Asn Val Ser Glu Leu Ser Leu Val
        195                 200                 205

aat ttt gag gaa tct cag acg ttt ttc ggc tta tat aag ctc
666
Asn Phe Glu Glu Ser Gln Thr Phe Phe Gly Leu Tyr Lys Leu
    210                 215                 220


<210>  344
<211>  222
<212>  PRT
<213>  Artificial

<220>
<223>  Amino acid sequence of a synthetic construct obtained by deleting
a cleavage site and an intracellular proline-rich domain from FasL and
insert a FLAG tag into N-terminus of FasL

<400>  344

Asp Tyr Lys Asp Asp Asp Asp Lys Gln Gln Pro Phe Asn Tyr Pro Leu
1               5                   10                  15


Lys Lys Arg Gly Asn His Ser Thr Gly Leu Cys Leu Leu Val Met Phe
            20                  25                  30


Phe Met Val Leu Val Ala Leu Val Gly Leu Gly Leu Gly Met Phe Gln
        35                  40                  45


Leu Phe His Leu Gln Lys Glu Leu Ala Glu Leu Arg Glu Ser Thr Ser
    50                  55                  60


Gln Met His Thr Ala Ser Ser Leu Gly His Pro Ser Pro Pro Pro Glu

65                  70                  75                  80

Lys Lys Glu Leu Arg Lys Val Ala His Leu Thr Gly Lys Ser Asn Ser
                85                  90                  95

Arg Ser Met Pro Leu Glu Trp Glu Asp Thr Tyr Gly Ile Val Leu Leu
            100                 105                 110

Ser Gly Val Lys Tyr Lys Lys Gly Gly Leu Val Ile Asn Glu Thr Gly
        115                 120                 125

Leu Tyr Phe Val Tyr Ser Lys Val Tyr Phe Arg Gly Gln Ser Cys Asn
    130                 135                 140

Asn Leu Pro Leu Ser His Lys Val Tyr Met Arg Asn Ser Lys Tyr Pro
145                 150                 155                 160

Gln Asp Leu Val Met Met Glu Gly Lys Met Met Ser Tyr Cys Thr Thr
                165                 170                 175

Gly Gln Met Trp Ala Arg Ser Ser Tyr Leu Gly Ala Val Phe Asn Leu
            180                 185                 190

Thr Ser Ala Asp His Leu Tyr Val Asn Val Ser Glu Leu Ser Leu Val
        195                 200                 205

Asn Phe Glu Glu Ser Gln Thr Phe Phe Gly Leu Tyr Lys Leu
    210                 215                 220

<210>  345
<211>  516
<212>  DNA
<213>  Artificial

<220>
<221>  CDS
<222>  (1)..(516)

<220>
<223>  Nucleotide sequence of S195-His6

<400>  345
gta gca gag aca ccc acc tat cca tgg agg gac gct gag acc ggt gag
48
Val Ala Glu Thr Pro Thr Tyr Pro Trp Arg Asp Ala Glu Thr Gly Glu
1               5                   10                  15

cgg ctc gtt tgc gcc caa tgt cca cca ggg acc ttc gtt cag cgg ccc
96
Arg Leu Val Cys Ala Gln Cys Pro Pro Gly Thr Phe Val Gln Arg Pro

                20                          25                          30

tgc cga cgg gac agt cca aca aca tgt ggc cct tgt cct ccc cgc cac
144
Cys Arg Arg Asp Ser Pro Thr Thr Cys Gly Pro Cys Pro Pro Arg His
            35                          40                          45

tac act cag ttc tgg aac tac ctg gag cgc tgt cgc tac tgc aat gtg
192
Tyr Thr Gln Phe Trp Asn Tyr Leu Glu Arg Cys Arg Tyr Cys Asn Val
        50                          55                          60

ctg tgt ggg gaa cgc gag gag gag gct cgc gct tgt cat gcc act cac
240
Leu Cys Gly Glu Arg Glu Glu Glu Ala Arg Ala Cys His Ala Thr His
65                          70                          75                          80

aat cgc gcc tgt cgc tgt cgc act ggc ttc ttc gcc cat gcc ggc ttc
288
Asn Arg Ala Cys Arg Cys Arg Thr Gly Phe Phe Ala His Ala Gly Phe
                        85                          90                          95

tgt ctg gag cat gcc tcc tgt cct cct ggg gct ggc gtg atc gct cct
336
Cys Leu Glu His Ala Ser Cys Pro Pro Gly Ala Gly Val Ile Ala Pro
                100                         105                         110

ggg act ccc tcc cag aac act cag tgt cag ccc tgc cct ccc ggc act
384
Gly Thr Pro Ser Gln Asn Thr Gln Cys Gln Pro Cys Pro Pro Gly Thr
            115                         120                         125

ttt tct gcc tct tcc agt tca tca gag caa tgc cag cct cac aga aac
432
Phe Ser Ala Ser Ser Ser Ser Ser Glu Gln Cys Gln Pro His Arg Asn
        130                         135                         140

tgt acc gct ctg ggg ctc gcc ctg aat gtc ccc ggc tct tca agt cac
480
Cys Thr Ala Leu Gly Leu Ala Leu Asn Val Pro Gly Ser Ser Ser His
145                         150                         155                         160

gat acc ctg tgc acc agc cat cat cat cat cat cat
516
Asp Thr Leu Cys Thr Ser His His His His His His
                        165                         170

<210>  346
<211>  172
<212>  PRT
<213>  Artificial

<220>
<223>  Amino acid sequence of S195-His6

<400>  346

Val Ala Glu Thr Pro Thr Tyr Pro Trp Arg Asp Ala Glu Thr Gly Glu
1                   5                   10                  15

Arg Leu Val Cys Ala Gln Cys Pro Pro Gly Thr Phe Val Gln Arg Pro
            20                  25                  30

Cys Arg Arg Asp Ser Pro Thr Thr Cys Gly Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Pro Gly Thr
        115                 120                 125

Phe Ser Ala Ser Ser Ser Ser Ser Glu Gln Cys Gln Pro His Arg Asn
    130                 135                 140

Cys Thr Ala Leu Gly Leu Ala Leu Asn Val Pro Gly Ser Ser Ser His
145                 150                 155                 160

Asp Thr Leu Cys Thr Ser His His His His His His
                165                 170

<210>  347
<211>  537
<212>  DNA
<213>  Artificial

<220>
<221>  CDS
<222>  (1)..(537)

<220>
<223>  Nucleotide sequence of Chimera A-His6

<400>  347
gag act ttc ccc ccc aag tat ctg cac tat gat gag gag act tcc cat
48
Glu Thr Phe Pro Pro Lys Tyr Leu His Tyr Asp Glu Glu Thr Ser His
1               5                   10                  15

cag ctg ctg tgt gac aag tgt ccc ccc ggg acc tat ctg aag cag cat
96
Gln Leu Leu Cys Asp Lys Cys Pro Pro Gly Thr Tyr Leu Lys Gln His
            20                  25                  30

tgt act gcc aag tgg aag act gtg tgt gct cct tgt cct ccc cgc cac
144
Cys Thr Ala Lys Trp Lys Thr Val Cys Ala Pro Cys Pro Pro Arg His
        35                  40                  45

tac act cag ttc tgg aac tac ctg gag cgc tgt cgc tac tgc aat gtg
192
Tyr Thr Gln Phe Trp Asn Tyr Leu Glu Arg Cys Arg Tyr Cys Asn Val
    50                  55                  60

ctg tgt ggg gaa cgc gag gag gag gct cgc gct tgt cat gcc act cac
240
Leu Cys Gly Glu Arg Glu Glu Glu Ala Arg Ala Cys His Ala Thr His
65                  70                  75                  80

aat cgc gcc tgt cgc tgt cgc act ggc ttc ttc gcc cat gcc ggc ttc
288
Asn Arg Ala Cys Arg Cys Arg Thr Gly Phe Phe Ala His Ala Gly Phe
                85                  90                  95

tgt ctg gag cat gcc tcc tgt cct cct ggg gct ggc gtg atc gct cct
336
Cys Leu Glu His Ala Ser Cys Pro Pro Gly Ala Gly Val Ile Ala Pro
            100                 105                 110

ggg act ccc tcc cag aac act cag tgt cag ccc tgt ccc gat ggc ttc
384
Gly Thr Pro Ser Gln Asn Thr Gln Cys Gln Pro Cys Pro Asp Gly Phe
        115                 120                 125

ttc tcc aat gag act tcc tcc aag gcc ccc tgt cgc aag cac acc aac
432
Phe Ser Asn Glu Thr Ser Ser Lys Ala Pro Cys Arg Lys His Thr Asn
    130                 135                 140

tgt tcc gtg ttc ggg ctg ctg ctg act cag aag ggg aat gcc act cac
480
Cys Ser Val Phe Gly Leu Leu Leu Thr Gln Lys Gly Asn Ala Thr His
145                 150                 155                 160

gac aac atc tgt tcc ggg aac tcc gag tcc act cag aag cat cat cat
528
Asp Asn Ile Cys Ser Gly Asn Ser Glu Ser Thr Gln Lys His His His
                165                 170                 175

cat cat cat
537
His His His

<210>  348
<211>  179
<212>  PRT
<213>  Artificial

<220>
<223>  Amino acid sequence of Chimera A-His6

<400>  348

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Glu Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys His His His
                165                 170                 175

His His His

<210>  349
<211>  537
<212>  DNA
<213>  Artificial

<220>

<221>  CDS
<222>  (1)..(537)

<220>
<223>  Nucleotide sequence of Chimera A-E57K-His6

<400>  349
gag act ttc ccc ccc aag tat ctg cac tat gat gag gag act tcc cat
48
Glu Thr Phe Pro Pro Lys Tyr Leu His Tyr Asp Glu Glu Thr Ser His
1               5                   10                  15

cag ctg ctg tgt gac aag tgt ccc ccc ggg acc tat ctg aag cag cat
96
Gln Leu Leu Cys Asp Lys Cys Pro Pro Gly Thr Tyr Leu Lys Gln His
            20                  25                  30

tgt act gcc aag tgg aag act gtg tgt gct cct tgt cct ccc cgc cac
144
Cys Thr Ala Lys Trp Lys Thr Val Cys Ala Pro Cys Pro Pro Arg His
        35                  40                  45

tac act cag ttc tgg aac tac ctg aag cgc tgt cgc tac tgc aat gtg
192
Tyr Thr Gln Phe Trp Asn Tyr Leu Lys Arg Cys Arg Tyr Cys Asn Val
    50                  55                  60

ctg tgt ggg gaa cgc gag gag gag gct cgc gct tgt cat gcc act cac
240
Leu Cys Gly Glu Arg Glu Glu Glu Ala Arg Ala Cys His Ala Thr His
65                  70                  75                  80

aat cgc gcc tgt cgc tgt cgc act ggc ttc ttc gcc cat gcc ggc ttc
288
Asn Arg Ala Cys Arg Cys Arg Thr Gly Phe Phe Ala His Ala Gly Phe
                85                  90                  95

tgt ctg gag cat gcc tcc tgt cct cct ggg gct ggc gtg atc gct cct
336
Cys Leu Glu His Ala Ser Cys Pro Pro Gly Ala Gly Val Ile Ala Pro
            100                 105                 110

ggg act ccc tcc cag aac act cag tgt cag ccc tgt ccc gat ggc ttc
384
Gly Thr Pro Ser Gln Asn Thr Gln Cys Gln Pro Cys Pro Asp Gly Phe
        115                 120                 125

ttc tcc aat gag act tcc tcc aag gcc ccc tgt cgc aag cac acc aac
432
Phe Ser Asn Glu Thr Ser Ser Lys Ala Pro Cys Arg Lys His Thr Asn
    130                 135                 140

tgt tcc gtg ttc ggg ctg ctg ctg act cag aag ggg aat gcc act cac
480
Cys Ser Val Phe Gly Leu Leu Leu Thr Gln Lys Gly Asn Ala Thr His
145                 150                 155                 160

gac aac atc tgt tcc ggg aac tcc gag tcc act cag aag cat cat cat
528
Asp Asn Ile Cys Ser Gly Asn Ser Glu Ser Thr Gln Lys His His His

                165                 170                 175

cat cat cat
537
His His His

<210> 350
<211> 179
<212> PRT
<213> Artificial

<220>
<223> Amino acid sequence of Chimera A-E57K-His6

<400> 350

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Arg Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe
        115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys His His His
                165                 170                 175

His His His

<210> 351
<211> 537
<212> DNA
<213> Artificial

<220>
<221> CDS
<222> (1)..(537)

<220>
<223> Nucleotide sequence of Chimera A-E57K_R58E-His6

<400> 351
gag act ttc ccc ccc aag tat ctg cac tat gat gag gag act tcc cat
48
Glu Thr Phe Pro Pro Lys Tyr Leu His Tyr Asp Glu Glu Thr Ser His
1               5                   10                  15

cag ctg ctg tgt gac aag tgt ccc ccc ggg acc tat ctg aag cag cat
96
Gln Leu Leu Cys Asp Lys Cys Pro Pro Gly Thr Tyr Leu Lys Gln His
            20                  25                  30

tgt act gcc aag tgg aag act gtg tgt gct cct tgt cct ccc cgc cac
144
Cys Thr Ala Lys Trp Lys Thr Val Cys Ala Pro Cys Pro Pro Arg His
        35                  40                  45

tac act cag ttc tgg aac tac ctg aag gag tgt cgc tac tgc aat gtg
192
Tyr Thr Gln Phe Trp Asn Tyr Leu Lys Glu Cys Arg Tyr Cys Asn Val
    50                  55                  60

ctg tgt ggg gaa cgc gag gag gag gct cgc gct tgt cat gcc act cac
240
Leu Cys Gly Glu Arg Glu Glu Glu Ala Arg Ala Cys His Ala Thr His
65                  70                  75                  80

aat cgc gcc tgt cgc tgt cgc act ggc ttc ttc gcc cat gcc ggc ttc
288
Asn Arg Ala Cys Arg Cys Arg Thr Gly Phe Phe Ala His Ala Gly Phe
                85                  90                  95

tgt ctg gag cat gcc tcc tgt cct cct ggg gct ggc gtg atc gct cct
336
Cys Leu Glu His Ala Ser Cys Pro Pro Gly Ala Gly Val Ile Ala Pro
            100                 105                 110

ggg act ccc tcc cag aac act cag tgt cag ccc tgt ccc gat ggc ttc
384
Gly Thr Pro Ser Gln Asn Thr Gln Cys Gln Pro Cys Pro Asp Gly Phe
        115                 120                 125

ttc tcc aat gag act tcc tcc aag gcc ccc tgt cgc aag cac acc aac
432
Phe Ser Asn Glu Thr Ser Ser Lys Ala Pro Cys Arg Lys His Thr Asn
    130                 135                 140

tgt tcc gtg ttc ggg ctg ctg ctg act cag aag ggg aat gcc act cac
480
Cys Ser Val Phe Gly Leu Leu Leu Thr Gln Lys Gly Asn Ala Thr His
145                 150                 155                 160

gac aac atc tgt tcc ggg aac tcc gag tcc act cag aag cat cat cat
528
Asp Asn Ile Cys Ser Gly Asn Ser Glu Ser Thr Gln Lys His His His
                165                 170                 175

cat cat cat
537
His His His

<210>  352
<211>  179
<212>  PRT
<213>  Artificial

<220>
<223>  Amino acid sequence of Chimera A-E57K_R58E-His6

<400>  352

Glu Thr Phe Pro Pro Lys Tyr Leu His Tyr Asp Glu Glu Thr Ser His
1               5                   10                  15

Gln Leu Leu Cys Asp Lys Cys Pro Pro Gly Thr Tyr Leu Lys Gln His
            20                  25                  30

Cys Thr Ala Lys Trp Lys Thr Val Cys Ala Pro Cys Pro Pro Arg His
        35                  40                  45

Tyr Thr Gln Phe Trp Asn Tyr Leu Lys Glu Cys Arg Tyr Cys Asn Val
    50                  55                  60

Leu Cys Gly Glu Arg Glu Glu Glu Ala Arg Ala Cys His Ala Thr His
65                  70                  75                  80

Asn Arg Ala Cys Arg Cys Arg Thr Gly Phe Phe Ala His Ala Gly Phe
                85                  90                  95

Cys Leu Glu His Ala Ser Cys Pro Pro Gly Ala Gly Val Ile Ala Pro
            100                 105                 110

Gly Thr Pro Ser Gln Asn Thr Gln Cys Gln Pro Cys Pro Asp Gly Phe

115                 120                 125

Phe Ser Asn Glu Thr Ser Ser Lys Ala Pro Cys Arg Lys His Thr Asn
    130                 135                 140

Cys Ser Val Phe Gly Leu Leu Leu Thr Gln Lys Gly Asn Ala Thr His
145                 150                 155                 160

Asp Asn Ile Cys Ser Gly Asn Ser Glu Ser Thr Gln Lys His His His
                165                 170                 175

His His His

<210>  353
<211>  519
<212>  DNA
<213>  Artificial

<220>
<221>  CDS
<222>  (1)..(519)

<220>
<223>  Optimized nucleotide sequence of Chimera A

<400>  353
gag act ttc ccc ccc aag tat ctg cac tat gat gag gag act tcc cat
48
Glu Thr Phe Pro Pro Lys Tyr Leu His Tyr Asp Glu Glu Thr Ser His
1               5                   10                  15

cag ctg ctg tgt gac aag tgt ccc ccc ggg acc tat ctg aag cag cat
96
Gln Leu Leu Cys Asp Lys Cys Pro Pro Gly Thr Tyr Leu Lys Gln His
            20                  25                  30

tgt act gcc aag tgg aag act gtg tgt gct cct tgt cct ccc cgc cac
144
Cys Thr Ala Lys Trp Lys Thr Val Cys Ala Pro Cys Pro Pro Arg His
        35                  40                  45

tac act cag ttc tgg aac tac ctg gag cgc tgt cgc tac tgc aat gtg
192
Tyr Thr Gln Phe Trp Asn Tyr Leu Glu Arg Cys Arg Tyr Cys Asn Val
    50                  55                  60

ctg tgt ggg gaa cgc gag gag gag gct cgc gct tgt cat gcc act cac
240
Leu Cys Gly Glu Arg Glu Glu Glu Ala Arg Ala Cys His Ala Thr His
65                  70                  75                  80

aat cgc gcc tgt cgc tgt cgc act ggc ttc ttc gcc cat gcc ggc ttc
288
Asn Arg Ala Cys Arg Cys Arg Thr Gly Phe Phe Ala His Ala Gly Phe

85                  90                  95

tgt ctg gag cat gcc tcc tgt cct cct ggg gct ggc gtg atc gct cct
336
Cys Leu Glu His Ala Ser Cys Pro Pro Gly Ala Gly Val Ile Ala Pro
            100                 105                 110

ggg act ccc tcc cag aac act cag tgt cag ccc tgt ccc gat ggc ttc
384
Gly Thr Pro Ser Gln Asn Thr Gln Cys Gln Pro Cys Pro Asp Gly Phe
        115                 120                 125

ttc tcc aat gag act tcc tcc aag gcc ccc tgt cgc aag cac acc aac
432
Phe Ser Asn Glu Thr Ser Ser Lys Ala Pro Cys Arg Lys His Thr Asn
    130                 135                 140

tgt tcc gtg ttc ggg ctg ctg ctg act cag aag ggg aat gcc act cac
480
Cys Ser Val Phe Gly Leu Leu Leu Thr Gln Lys Gly Asn Ala Thr His
145                 150                 155                 160

gac aac atc tgt tcc ggg aac tcc gag tcc act cag aag
519
Asp Asn Ile Cys Ser Gly Asn Ser Glu Ser Thr Gln Lys
                165                 170


<210>  354
<211>  519
<212>  DNA
<213>  Artificial

<220>
<221>  CDS
<222>  (1)..(519)

<220>
<223>  Optimized nucleotide sequence of Chimera A-E57K

<400>  354
gag act ttc ccc ccc aag tat ctg cac tat gat gag gag act tcc cat
48
Glu Thr Phe Pro Pro Lys Tyr Leu His Tyr Asp Glu Glu Thr Ser His
1               5                   10                  15

cag ctg ctg tgt gac aag tgt ccc ccc ggg acc tat ctg aag cag cat
96
Gln Leu Leu Cys Asp Lys Cys Pro Pro Gly Thr Tyr Leu Lys Gln His
            20                  25                  30

tgt act gcc aag tgg aag act gtg tgt gct cct tgt cct ccc cgc cac
144
Cys Thr Ala Lys Trp Lys Thr Val Cys Ala Pro Cys Pro Pro Arg His
        35                  40                  45

tac act cag ttc tgg aac tac ctg aag cgc tgt cgc tac tgc aat gtg
192
Tyr Thr Gln Phe Trp Asn Tyr Leu Lys Arg Cys Arg Tyr Cys Asn Val
    50                  55                  60

ctg tgt ggg gaa cgc gag gag gag gct cgc gct tgt cat gcc act cac
240
Leu Cys Gly Glu Arg Glu Glu Glu Ala Arg Ala Cys His Ala Thr His
65                  70                  75                  80

aat cgc gcc tgt cgc tgt cgc act ggc ttc ttc gcc cat gcc ggc ttc
288
Asn Arg Ala Cys Arg Cys Arg Thr Gly Phe Phe Ala His Ala Gly Phe
                85                  90                  95

tgt ctg gag cat gcc tcc tgt cct cct ggg gct ggc gtg atc gct cct
336
Cys Leu Glu His Ala Ser Cys Pro Pro Gly Ala Gly Val Ile Ala Pro
            100                 105                 110

ggg act ccc tcc cag aac act cag tgt cag ccc tgt ccc gat ggc ttc
384
Gly Thr Pro Ser Gln Asn Thr Gln Cys Gln Pro Cys Pro Asp Gly Phe
        115                 120                 125

ttc tcc aat gag act tcc tcc aag gcc ccc tgt cgc aag cac acc aac
432
Phe Ser Asn Glu Thr Ser Ser Lys Ala Pro Cys Arg Lys His Thr Asn
    130                 135                 140

tgt tcc gtg ttc ggg ctg ctg ctg act cag aag ggg aat gcc act cac
480
Cys Ser Val Phe Gly Leu Leu Leu Thr Gln Lys Gly Asn Ala Thr His
145                 150                 155                 160

gac aac atc tgt tcc ggg aac tcc gag tcc act cag aag
519
Asp Asn Ile Cys Ser Gly Asn Ser Glu Ser Thr Gln Lys
                165                 170


<210>  355
<211>  519
<212>  DNA
<213>  Artificial

<220>
<221>  CDS
<222>  (1)..(519)

<220>
<223>  Optimized nucleotide sequence of Chimera A-E57K_R58E

<400>  355
gag act ttc ccc ccc aag tat ctg cac tat gat gag gag act tcc cat
48
Glu Thr Phe Pro Pro Lys Tyr Leu His Tyr Asp Glu Glu Thr Ser His
1               5                   10                  15

cag ctg ctg tgt gac aag tgt ccc ccc ggg acc tat ctg aag cag cat
96
Gln Leu Leu Cys Asp Lys Cys Pro Pro Gly Thr Tyr Leu Lys Gln His
            20                  25                  30

tgt act gcc aag tgg aag act gtg tgt gct cct tgt cct ccc cgc cac
144
Cys Thr Ala Lys Trp Lys Thr Val Cys Ala Pro Cys Pro Pro Arg His
        35                  40                  45

tac act cag ttc tgg aac tac ctg aag gag tgt cgc tac tgc aat gtg
192
Tyr Thr Gln Phe Trp Asn Tyr Leu Lys Glu Cys Arg Tyr Cys Asn Val
    50                  55                  60

ctg tgt ggg gaa cgc gag gag gag gct cgc gct tgt cat gcc act cac
240
Leu Cys Gly Glu Arg Glu Glu Glu Ala Arg Ala Cys His Ala Thr His
65                  70                  75                  80

aat cgc gcc tgt cgc tgt cgc act ggc ttc ttc gcc cat gcc ggc ttc
288
Asn Arg Ala Cys Arg Cys Arg Thr Gly Phe Phe Ala His Ala Gly Phe
                85                  90                  95

tgt ctg gag cat gcc tcc tgt cct cct ggg gct ggc gtg atc gct cct
336
Cys Leu Glu His Ala Ser Cys Pro Pro Gly Ala Gly Val Ile Ala Pro
            100                 105                 110

ggg act ccc tcc cag aac act cag tgt cag ccc tgt ccc gat ggc ttc
384
Gly Thr Pro Ser Gln Asn Thr Gln Cys Gln Pro Cys Pro Asp Gly Phe
        115                 120                 125

ttc tcc aat gag act tcc tcc aag gcc ccc tgt cgc aag cac acc aac
432
Phe Ser Asn Glu Thr Ser Ser Lys Ala Pro Cys Arg Lys His Thr Asn
    130                 135                 140

tgt tcc gtg ttc ggg ctg ctg ctg act cag aag ggg aat gcc act cac
480
Cys Ser Val Phe Gly Leu Leu Leu Thr Gln Lys Gly Asn Ala Thr His
145                 150                 155                 160

gac aac atc tgt tcc ggg aac tcc gag tcc act cag aag
519
Asp Asn Ile Cys Ser Gly Asn Ser Glu Ser Thr Gln Lys
                165                 170

## Description

### BACKGROUND OF THE INVENTION

### Field of the Invention

The present invention relates to a DcR3 variant, which is a variant of wild-type DcR3. More particularly, the present invention relates to a DcR3 variant that has binding activity (preferably neutralizing activity) to a ligand of DcR3, and that results in a decreased amount of aggregates as compared to wild-type DcR3 when produced using a cell derived from a mammal as a host, and/or that exhibits improved *in vivo* kinetics.

### Background Art

A tumor necrosis factor (TNF) superfamily (TNFSF) and a TNF receptor superfamily (TNFRSF) each form 18 ligands and 29 receptor families having similar structures. Antibodies and Fc fusion proteins against many molecules included in these families have been developed and launched, and have exhibited therapeutic effects in treatment of various autoimmune diseases (Non-Patent Document 1).

Although many TNFRSFs are expressed on a cell membrane and transmit a signal downstream by ligand binding, some molecules are decoy receptors (DcRs) not involved in signaling. As the decoy receptors, four types of DcR1, DcR2, DcR3 and osteoprotegerin (OPG) have been identified.

OPG is a soluble decoy receptor for RANKL and TRAIL, and inhibits signaling by competing with the binding to ligands of a RANKL receptor and a TRAIL receptor. Meanwhile, DcR1 and DcR2 are decoy receptors for TRAIL, and DcR3 is a decoy receptor for three molecules of LIGHT, TL1A and FasL, and all of the decoy receptors neutralize ligands by competitively inhibiting the binding of the ligands to a signaling receptor (Non-Patent Document 2).

DcR3 is a soluble molecule consisting of 300 amino acid residues. The N-terminal side has a signal peptide, followed by four cysteine-rich domains (CRDs) (CRD1, CRD2, CRD3 and CRD4), which are characteristic of TNFRSF, and the C-terminal side has a heparan sulfate-binding domain (HBD), which includes a heparan sulfate-binding motif and is rich in basic amino acids. All of LIGHT, TL1A and FasL bind via CRD2 and CRD3 of DcR3 (Non-Patent Documents 3, 4 and 5).

DcR3 has, in addition to a function as a decoy receptor by ligand neutralization, a function as an immunomodulatory molecule based on the activity of HBD. For example, it has been reported that DcR3 directly binds via HBD to glycosaminoglycan (GAG) including heparan sulfate on the cell membrane of monocytes, macrophages or dendritic cells, thus initiating various immunosuppressive and immunostimulatory effects such as Th2 induction by differentiation of dendritic cells, induction of M2 macrophages, enhancement of adhesion of monocytes, osteoclast differentiation, or decreased expression of MHC class II molecules (Non-Patent Documents 6 and 12).

DcR3 ligands have been reported to be involved in autoimmune diseases, inflammatory diseases, allergy, cancer, infection or other various inflammation reactions. For example, all of LIGHT, TL1A and FasL are included in the susceptibility locus in inflammatory bowel disease (IBD), and particularly, regarding TL1A, existence of a plurality of gene polymorphisms associated with pathological conditions has been known. There have also been reports of increased expression of DcR3 ligands in the blood or tissue of IBD patients, and improvement in pathological conditions by inhibition of DcR3 ligands in a mouse enteritis model (Non-Patent Documents 6 to 9).

Although expression of DcR3 in human normal tissues is at an extremely low level, the expression is induced by infection or tissue damage. Furthermore, it has been known that the level of DcR3 in blood is increased in various autoimmune diseases or inflammatory diseases such as IBD, systemic lupus erythematosus (SLE), atopic dermatitis (AD) or rheumatoid arthritis (RA). Although no DcR3 homolog has been identified in mice, improvement in pathological conditions in human DcR3 transgenic mice and drug efficacy by administration of plasmids or recombinant DcR3 have been confirmed in mouse disease models such as a type I diabetes mellitus model, a multiple sclerosis model or a nephritis model (Non-Patent Documents 6 and 10).

Genentech Inc. cloned the human DcR3 gene, and showed that a fusion of DcR3 and the Fc region of human IgG1 binds to soluble human FasL and inhibits human FasL-dependent apoptosis in vitro (Patent Document 1).

Eli Lilly and Company obtained FLINT corresponding to a protease-resistant DcR3 mutant obtained by introducing one-amino-acid mutation (R218Q) into wild-type DcR3, and reported that the *in vivo* kinetics thereof is more improved in mice and monkeys than that of wild-type DcR3. However, the half-lives in blood when wild-type DcR3 and FLINT were administered at 0.5 mg/kg by intravenous injection to cynomolgus monkeys were extremely short, with values of 9 hours or 12.3 hours, respectively (Patent Documents 2 and 3, Non-Patent Document 11).

### Prior Art Document

### [Patent Document]

Patent Document 1: JP 4303883 B2
Patent Document 2: US 6,835,814 B1
Patent Document 3: US 6,965,012 B1

### [Non-Patent Document]

Non-Patent Document 1: Nature Reviews Drug Discovery, 2013, 12: p.147-168
Non-Patent Document 2: Nature Reviews Cancer, 2002.2: p.420-430
Non-Patent Document 3: Structure, 2011, 19: p.162-171
Non-Patent Document 4: Structure, 2014, 22: p.1252-1262
Non-Patent Document 5: Structure, 2016, 24: p.2016-2023
Non-Patent Document 6: Biochemical Pharmacology, 2011, 81: p.838-847
Non-Patent Document 7: Immunology, 2009, 128: p.451-458
Non-Patent Document 8: P.N.A.S., 2006, 103: p.8441-8446
Non-Patent Document 9: Am. J. Physiol. Gastrointest. Liver Physiol., 2003, 285: p.G754-G760
Non-Patent Document 10: Journal of Biomedical Science, 2017, 24:39
Non-Patent Document 11: Drug Metabolism and Disposition, 2003, 31: p.502-507
Non-Patent Document 12: J. Immunol., 2006, 176: p.173-180

### SUMMARY OF THE INVENTION

FLINT, which is an amino acid variant of wild-type DcR3, has markedly poor *in vivo* kinetics, and requires frequent administration as a recombinant formulation whose mechanism of action is ligand neutralization, and this is undesirable as a pharmaceutical product. Therefore, a DcR3 variant in which a certain administration interval can be ensured by improvement in the *in vivo* kinetics is expected to be useful as a pharmaceutical product.

As far as is known to date, there has not been developed a functional DcR3 variant that results in a decreased amount of aggregates when expressed, isolated and purified using a cell derived from a mammal as a host, and that has neutralizing activity to a DcR3 ligand.

An object of the present invention is to provide a DcR3 variant that has binding activity (preferably neutralizing activity) to a ligand of DcR3, and that results in a decreased amount of aggregates as compared to wild-type DcR3 when a DcR3 protein is produced using a cell derived from a mammal as a host, and/or that exhibits improved *in vivo* kinetics; a DNA encoding the DcR3 variant; a vector including the DNA; a transformant obtained by introducing the vector; a method for producing the variant using the transformant; and a pharmaceutical composition and a prophylactic or therapeutic agent for an autoimmune disease, an inflammatory disease or allergy including the variant as an active ingredient.

In order to achieve the abovementioned object, the present invention provides the following inventions.
[1] A DcR3 variant, the DcR3 variant being a variant of wild-type decoy receptor 3 (hereinafter abbreviated as DcR3) and exhibiting improved *in vivo* kinetics as compared to the wild-type DcR3.
[2] The DcR3 variant according to [1], wherein the DcR3 variant comprises one or more complex N-glycoside-linked glycans.
[3] The DcR3 variant according to [1] or [2], wherein the DcR3 variant has neutralizing activity to at least one or more of LIGHT, TL1A and FasL.
[4] The DcR3 variant according to any one of [1] to [3], wherein the DcR3 variant has neutralizing activity to all of LIGHT, TL1A and FasL.
[5] The DcR3 variant according to any one of [1] to [3], wherein the DcR3 variant has no neutralizing activity to FasL and has neutralizing activity to one or more of LIGHT and TL1A.
[6] The DcR3 variant according to any one of [1] to [3] and [5], wherein the DcR3 variant has no neutralizing activity to FasL and has neutralizing activity to both of LIGHT and TL1A.
[7] A DcR3 variant comprising a first chimeric cysteine-rich region or a second chimeric cysteine-rich region, wherein:
   the first chimeric cysteine-rich region consists of an amino acid sequence obtained by introducing into a cysteine-rich domain (hereinafter abbreviated as CRD) of wild-type DcR3, substitution of at least a part of the cysteine-rich domain of the wild-type DcR3 with at least a part of a cysteine-rich domain of a TNF receptor superfamily molecule other than DcR3; and
   the second chimeric cysteine-rich region consists of an amino acid sequence obtained by introducing into the amino acid sequence of the first chimeric cysteine-rich region, deletion, substitution, insertion or addition of 1 to 30 amino acids.
[8] The DcR3 variant according to [7], wherein the DcR3 variant comprises one or more complex N-glycoside-linked glycans.
[9] The DcR3 variant according to [7] or [8], wherein the DcR3 variant has neutralizing activity to at least one or more of LIGHT, TL1A and FasL.
[10] The DcR3 variant according to any one of [7] to [9], wherein the DcR3 variant has neutralizing activity to all of LIGHT, TL1A and FasL.
[11] The DcR3 variant according to any one of [7] to [9], wherein the DcR3 variant has no neutralizing activity to FasL and has neutralizing activity to one or more of LIGHT and TL1A.
[12] The DcR3 variant according to any one of [7] to [9] and [11], wherein the DcR3 variant has no neutralizing activity to FasL and has neutralizing activity to both of LIGHT and TL1A.
[13] The DcR3 variant according to any one of [7] to [12], wherein the TNF receptor superfamily molecule is OPG.
[14] The DcR3 variant according to any one of [7] to [13], wherein:
   the at least a part of the cysteine-rich domain of the wild-type DcR3 is selected from a part or a whole of CRD1, a part or a whole of CRD2, a part or a whole of CRD3 and a part or a whole of CRD4; and
   the at least a part of the cysteine-rich domain of the TNF receptor superfamily molecule is selected from a part or a whole of CRD1, a part or a whole of CRD2, a part or a whole of CRD3 and a part or a whole of CRD4.
[15] The DcR3 variant according to [14], wherein the first chimeric cysteine-rich region comprises one or more substitution selected from:
   substitution of the part of the CRD1 of the wild-type DcR3 with a part of the CRD1 of the TNF receptor superfamily molecule, wherein the part of the CRD1 of the TNF receptor superfamily molecule corresponds to the part of the CRD1 of the wild-type DcR3;
   substitution of the whole of the CRD1 of the wild-type DcR3 with the whole of the CRD1 of the TNF receptor superfamily molecule;
   substitution of the part of the CRD2 of the wild-type DcR3 with a part of the CRD2 of the TNF receptor superfamily molecule, wherein the part of the CRD2 of the TNF receptor superfamily molecule corresponds to the part of the CRD2 of the wild-type DcR3;
   substitution of the whole of the CRD2 of the wild-type DcR3 with the whole of the CRD2 of the TNF receptor superfamily molecule;
   substitution of the part of the CRD3 of the wild-type DcR3 with a part of the CRD3 of the TNF receptor superfamily molecule, wherein the part of the CRD3 of the TNF receptor superfamily molecule corresponds to the part of the CRD3 of the wild-type DcR3;
   substitution of the whole of the CRD3 of the wild-type DcR3 with the whole of the CRD3 of the TNF receptor superfamily molecule;
   substitution of the part of the CRD4 of the wild-type DcR3 with a part of the CRD4 of the TNF receptor superfamily molecule, wherein the part of the CRD4 of the TNF receptor superfamily molecule corresponds to the part of the CRD4 of the wild-type DcR3; and
   substitution of the whole of the CRD4 of the wild-type DcR3 with the whole of the CRD4 of the TNF receptor superfamily molecule.
[16] The DcR3 variant according to [15], wherein the part or the whole of the CRD2 of the wild-type DcR3 is maintained in the first chimeric cysteine-rich region.
[17] The DcR3 variant according to [15] or [16], wherein the part or the whole of the CRD3 of the wild-type DcR3 is maintained in the first chimeric cysteine-rich region.
[18] The DcR3 variant according to any one of [14] to [17], wherein the first chimeric cysteine-rich region comprises the following amino acid sequence (a), (b), (c) or (d), and the second chimeric cysteine-rich region comprises the following amino acid sequence (e):
   (a) an amino acid sequence obtained by introducing into the amino acid sequence of the cysteine-rich region of the wild-type DcR3, substitution of the CRD1 of the wild-type DcR3 with CRD1 of OPG;
   (b) an amino acid sequence obtained by introducing into the amino acid sequence of the cysteine-rich region of the wild-type DcR3, substitution of the CRD4 of the wild-type DcR3 with CRD4 of OPG;
   (c) an amino acid sequence obtained by introducing into the amino acid sequence of the cysteine-rich region of the wild-type DcR3, substitution of the CRD1 of the wild-type DcR3 with CRD1 of OPG, and substitution of the CRD4 of the wild-type DcR3 with CRD4 of OPG;
   (d) an amino acid sequence obtained by introducing into the amino acid sequence (a), (b) or (c), substitution of a part at positions 103 to 123 from the N-terminus with a corresponding part of an amino acid sequence of a cysteine-rich domain of OPG; and
   (e) an amino acid sequence obtained by introducing into the amino acid sequence (a), (b), (c) or (d), deletion, substitution, insertion or addition of 1 to 30 amino acids.
[19] The DcR3 variant according to [18], wherein:
   the amino acid sequence (a) is an amino acid sequence consisting of amino acids at positions 1 to 164 from the N-terminus of the amino acid sequence set forth in SEQ ID NO: 26 or 50;
   the amino acid sequence (b) is an amino acid sequence consisting of amino acids at positions 1 to 164 from the N-terminus of the amino acid sequence set forth in SEQ ID NO: 28 or 52;
   the amino acid sequence (c) is an amino acid sequence consisting of amino acids at positions 1 to 164 from the N-terminus of the amino acid sequence set forth in SEQ ID NO: 30 or 54; and
   the amino acid sequence (d) is an amino acid sequence consisting of amino acids at positions 1 to 164 from the N-terminus of the amino acid sequence set forth in SEQ ID NO: 32 or 56.
[20] The DcR3 variant according to [18] or [19], wherein the amino acid sequence (e) comprises one or two or more substitution selected from the group consisting of:
   substitution of Glu at position 57 from the N-terminus of the amino acid sequence (a), (b), (c) or (d) with another amino acid;
   substitution of Arg at position 58 from the N-terminus of the amino acid sequence (a), (b), (c) or (d) with another amino acid; and
   substitution of Arg at position 60 from the N-terminus of the amino acid sequence (a), (b), (c) or (d) with another amino acid.
[21] The DcR3 variant according to any one of [18] to [20], wherein the amino acid sequence (e) comprises substitution of Glu at position 57 and Arg at position 58 from the N-terminus of the amino acid sequence (a), (b), (c) or (d) with other amino acids.
[22] The DcR3 variant according to any one of [18] to [21], wherein the amino acid sequence (e) comprises one or two or more substitution selected from the group consisting of:
   substitution of Glu at position 57 from the N-terminus of the amino acid sequence (a), (b), (c) or (d) with Lys, Leu, Arg, Val, Ala, Phe, His, Ile or Met;
   substitution of Arg at position 58 from the N-terminus of the amino acid sequence (a), (b), (c) or (d) with Asp, Glu or Thr; and
   substitution of Arg at position 60 from the N-terminus of the amino acid sequence (a), (b), (c) or (d) with Lys.
[23] The DcR3 variant according to any one of [18] to [22], wherein the amino acid sequence (e) comprises substitution of Glu at position 57 from the N-terminus of the amino acid sequence (a), (b), (c) or (d) with Lys, Leu, Arg, Val, Ala, Phe, His, Ile or Met, and substitution of Arg at position 58 from the N-terminus of the amino acid sequence (a), (b), (c) or (d) with Asp, Glu or Thr.
[24] The DcR3 variant according to any one of [18] to [23], wherein the amino acid sequence (e) comprises substitution selected from the following (f) to (i):
   (f) substitution of Asn at positions 131 and 144 from the N-terminus of the amino acid sequence (b), (c) or (d) with other amino acids;
   (g) substitution of Asn at positions 131, 144 and 157 from the N-terminus of the amino acid sequence (b), (c) or (d) with other amino acids;
   (h) substitution of Thr at position 133 and Ser at position 146 from the N-terminus of the amino acid sequence (b), (c) or (d) with other amino acids; and
   (i) substitution of Thr at position 133, Ser at position 146 and Thr at position 159 from the N-terminus of the amino acid sequence (b), (c) or (d) with other amino acids.
[25] The DcR3 variant according to any one of [18] to [24], wherein the amino acid sequence (e) comprises substitution selected from the following (f') to (i'):
   (f') substitution of Asn at positions 131 and 144 from the N-terminus of the amino acid sequence (b), (c) or (d) with Ser;
   (g') substitution of Asn at positions 131, 144 and 157 from the N-terminus of the amino acid sequence (b), (c) or (d) with Ser;
   (h') substitution of Thr at position 133 and Ser at position 146 from the N-terminus of the amino acid sequence (b), (c) or (d) with Ala; and
   (i') substitution of Thr at position 133, Ser at position 146 and Thr at position 159 from the N-terminus of the amino acid sequence (b), (c) or (d) with Ala.
[26] The DcR3 variant according to any one of [18] to [25], wherein the amino acid sequence (e) is an amino acid sequence consisting of amino acids at positions 1 to 164 from the N-terminus of an amino acid sequence set forth in SEQ ID NO: 58, 60, 62, 64, 66, 68, 70, 180, 182, 184, 186, 188, 270, 272, 274, 276, 278, 280, 282, 284 or 286.
[27] The DcR3 variant according to any one of [7] to [26], wherein the DcR3 variant is a DcR3 variant comprising the first or second chimeric cysteine-rich region, and a part or a whole of a heparan sulfate-binding domain of the wild-type DcR3 bound to the C-terminal side of the first or second chimeric cysteine-rich region, or a DcR3 variant comprising the first or second chimeric cysteine-rich region and not comprising a heparan sulfate-binding domain of the wild-type DcR3.
[28] The DcR3 variant according to [27], wherein the DcR3 variant comprises one of amino acid sequences selected from:
   (I) an amino acid sequence set forth in SEQ ID NO: 26, 28, 30, 32, 34, 36, 38, 40, 42, 44 or 46, or an amino acid sequence obtained by introducing into the abovementioned amino acid sequence, deletion, substitution, insertion or addition of 1 to 30 amino acids; and
   (II) an amino acid sequence set forth in SEQ ID NO: 50, 52, 54, 56, 58, 60, 62, 64, 66, 68, 70, 180, 182, 184, 186, 188, 270, 272, 274, 276, 278, 280, 282, 284 or 286, or an amino acid sequence obtained by introducing into the abovementioned amino acid sequence, deletion, substitution, insertion or addition of 1 to 30 amino acids.
[29] The DcR3 variant according to any one of [7] to [28], wherein the DcR3 variant comprises an Fc region derived from a human IgG1, IgG2 or IgG4 antibody, or a mutated Fc region consisting of an amino acid sequence obtained by introducing into an amino acid sequence of the abovementioned Fc region, deletion, substitution, insertion or addition of one or several amino acids.
[30] The DcR3 variant according to [29], wherein the Fc region or the mutated Fc region is bound to the C-terminal side of the first or second chimeric cysteine-rich region via another region or a linker.
[31] The DcR3 variant according to [29] or [30], wherein the mutated Fc region comprises substitution of Cys with Ser at EU-index position 220 of an amino acid sequence of a heavy chain of human IgG1.
[32] The DcR3 variant according to [31], wherein the mutated Fc region comprises substitution of Leu with Ala at EU-index position 234, substitution of Leu with Ala at EU-index position 235, and substitution of Gly with Ala at EU-index position 237 of an amino acid sequence of a heavy chain of human IgG1.
[33] The DcR3 variant according to [31] or [32], wherein the mutated Fc region comprises substitution of Asn with Ala at EU-index position 434 of an amino acid sequence of a heavy chain of human IgG1.
[34] The DcR3 variant according to [31], wherein the mutated Fc region comprises substitution of Met with Tyr at EU-index position 252, substitution of Ser with Thr at EU-index position 254, and substitution of Thr with Glu at EU-index position 256 of an amino acid sequence of a heavy chain of human IgG1.
[35] The DcR3 variant according to [29] or [30], wherein the mutated Fc region comprises substitution of Ser with Pro at EU-index position 228, substitution of Leu with Glu at EU-index position 235, and substitution of Arg with Lys at EU-index position 409 of an amino acid sequence of a heavy chain of human IgG4.
[36] The DcR3 variant according to [29] or [30], wherein the DcR3 variant comprises a mutated Fc region consisting of an amino acid sequence set forth in SEQ ID NO: 72, 74, 156, 158, 160, 162, 164, 166, 311, 312 or 313.
[37] The DcR3 variant according to any one of [29] to [36], wherein the DcR3 variant comprises an amino acid sequence set forth in SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 or 337, or an amino acid sequence obtained by introducing into the abovementioned amino acid sequence, deletion, substitution, insertion or addition of 1 to 30 amino acids.
[38] A DcR3 variant composition comprising the DcR3 variant according to any one of [1] to [37].
[39] The composition according to [38], wherein the composition comprises a DcR3 variant having one or more complex N-glycoside-linked glycans, and a DcR3 variant not having a complex N-glycoside-linked glycan.
[40] A DNA encoding the DcR3 variant according to any one of [1] to [37].
[41] A gene recombinant vector comprising the DNA according to [40].
[42] A transformant obtained by introducing the gene recombinant vector according to [41] into a host cell.
[43] The transformant according to [42], wherein the host cell is a cell derived from a mammal.
[44] The transformant according to [43], wherein the cell derived from a mammal is a CHO cell.
[45] A method of producing a DcR3 variant or a DcR3 variant composition, wherein the method comprises culturing the transformant according to any one of [42] to [44] in a medium to generate and accumulate the DcR3 variant according to any one of [1] to [37], and purifying the DcR3 variant from the obtained culture solution.
[46] A DcR3 variant or a DcR3 variant composition produced using the production method according to [45].
[47] A pharmaceutical composition comprising the DcR3 variant or the DcR3 variant composition according to any one of [1] to [39] and [46] as an active ingredient.
[48] The pharmaceutical composition according to [47], wherein the pharmaceutical composition is a prophylactic or therapeutic agent for an autoimmune disease, an inflammatory disease or an allergic disease.
[49] A method of preventing or treating an autoimmune disease, an inflammatory disease or an allergic disease, the method comprising administering the pharmaceutical composition according to [47] or [48] to a patient in need thereof.

According to the present invention, there is provided a DcR3 variant that has binding activity (preferably neutralizing activity) to a ligand of DcR3, and that results in a decreased amount of aggregates as compared to wild-type DcR3 when a DcR3 protein is produced using a cell derived from a mammal as a host, and/or that exhibits improved *in vivo* kinetics; a DNA encoding the DcR3 variant; a vector including the DNA; a transformant obtained by introducing the vector; a method for producing the variant using the transformant; and a pharmaceutical composition and a prophylactic or therapeutic agent for an autoimmune disease, an inflammatory disease or allergy including the variant as an active ingredient.

### BRIEF DESCRIPTION OF THE DRAWINGS

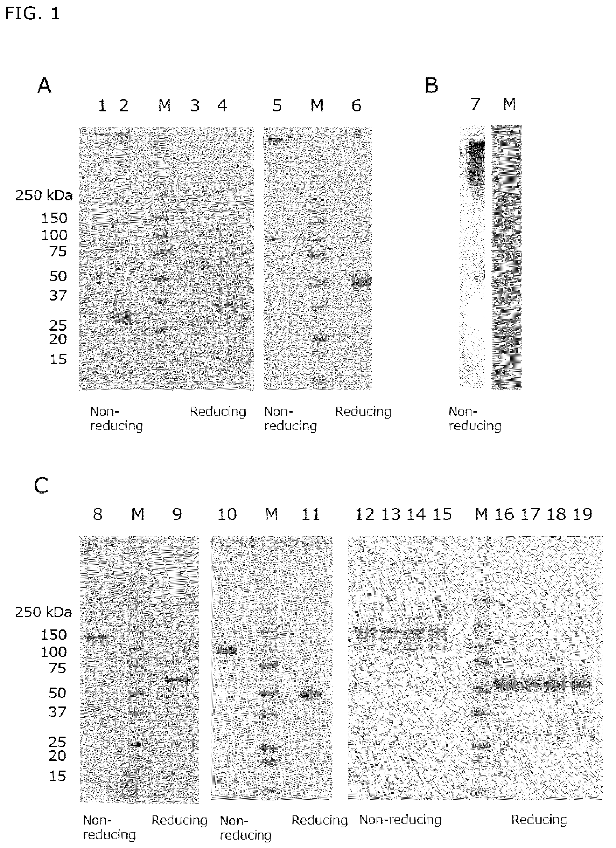

FIG. 1A shows results of SDS-PAGE of various wild-type DcR3 controls produced using mammalian cells. DcR3 FL-Fc in lanes 1 and 3, DcR3 FL-FLAG in lanes 2 and 4, and S195-Fc (g1S) in lanes 5 and 6 each were electrophoresed under non-reducing or reducing conditions. FIG. 1B shows that commercially available human DcR3-Fc produced using an HEK293 cell (lane 7) was electrophoresed under non-reducing conditions and detected by immunoblotting using an anti-human IgG antibody. FIG. 1C shows that commercially available DcR3 FL-Fc (lanes 8 and 9), S195-Fc (g1S) (lanes 10 and 11), DcR3 FL-Fc (g1S) (lanes 14, 15, 18 and 19) and R218Q-Fc(g1S) (lanes 12, 13, 16 and 17) produced using insect cells each were electrophoresed under non-reducing or reducing conditions. M represents a molecular weight marker (Bio-Rad Laboratories, Inc.).

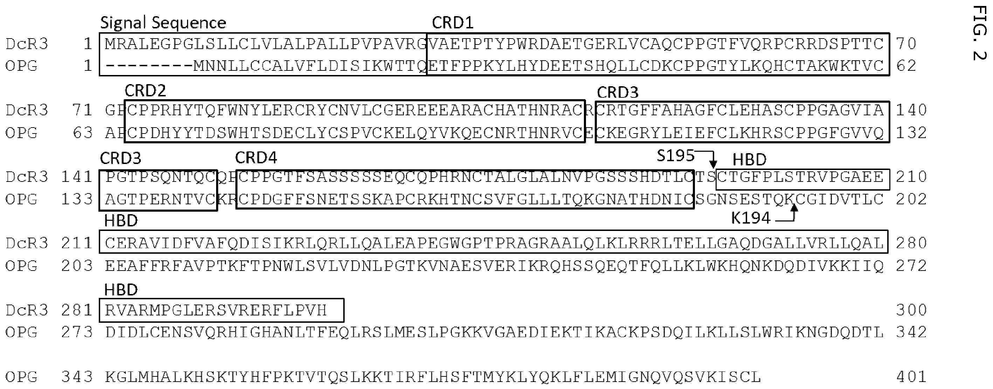

FIG. 2 shows cysteine-rich domains each represented by CRD1, CRD2, CRD3 and CRD4 in the alignment of premature amino acid sequences of human DcR3 and human OPG.

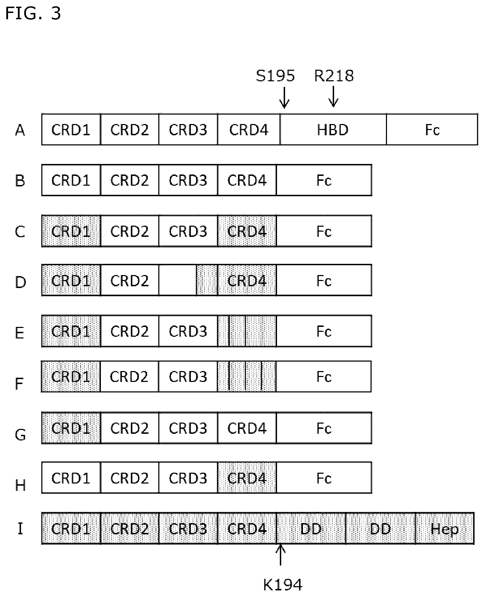

FIG. 3 schematically shows respective domain structures of various wild-type DcR3 controls and various DcR3 variants produced and human OPG. A: DcR3 FL-Fc, B: S195-Fc, C: chimera A-Fc, D: 103-123OPG-Fc, E: N-linked glycan disubstitute-Fc, F: N-linked glycan trisubstitute-Fc, G: chimera B-Fc, H: chimera C-Fc, I: OPG. The vertical lines of CRD4 in E and F represent existence of two or three substitutions of N-linked glycosylated residues, respectively, and DD in I represents a death domain.

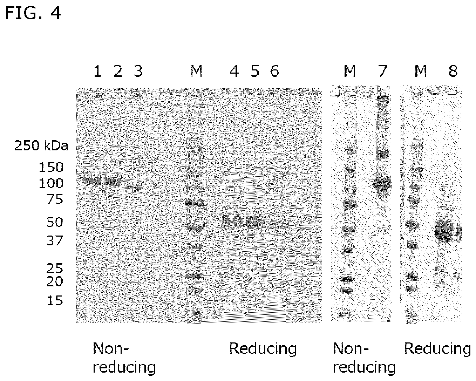

FIG. 4 shows results of SDS-PAGE of various DcR3 variants produced using mammalian cells. Chimera A-Fc (IEGRMD g1S) produced using an Expi293 cell in lanes 1 and 4, chimera A-Fc (IEGRMD g1S) produced using a CHO-S cell in lanes 2 and 5, chimera B-Fc (g1S) produced using an Expi293 cell in lanes 3 and 6, and chimera C-Fc (IEGRMD g1S) produced using an Expi293 cell in lanes 7 and 8 were electrophoresed under non-reducing or reducing conditions. M represents a molecular weight marker (Bio-Rad Laboratories, Inc.).

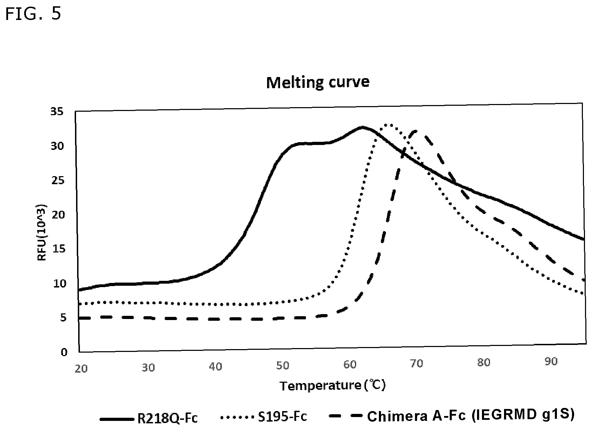

FIG. 5 shows melting curves by the DSF method for R218Q-Fc, S195-Fc and chimera A-Fc (IEGRMD g1S). The vertical axis represents fluorescence intensity RFU (10³), and the horizontal axis represents temperature (°C).

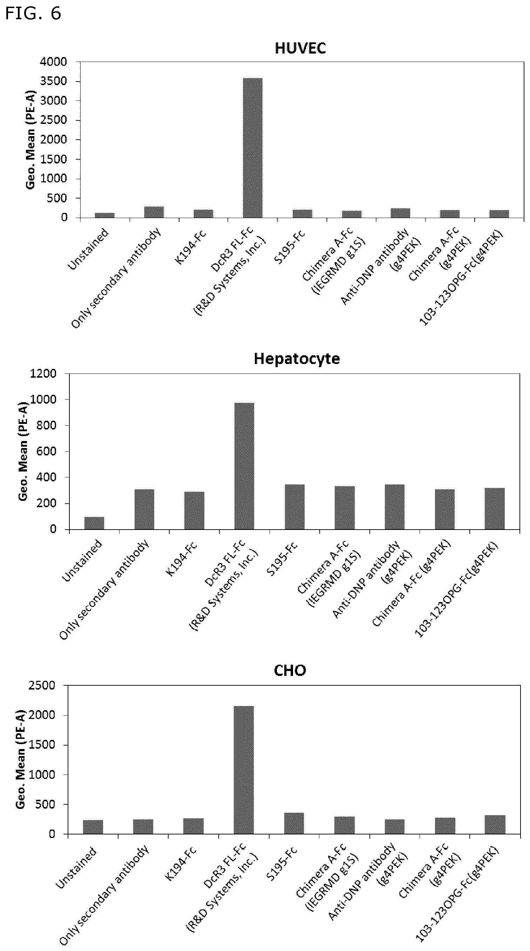

FIG. 6 shows the bindings of various wild-type DcR3 controls and various DcR3 variants to human primary cells and CHO cells. To each cell, 10 µg/mL of each DcR3 variant was reacted, followed by staining with 0.1 µg/mL of a PE-labeled anti-human antibody, and the fluorescence intensity of PE was measured by flow cytometry. The vertical axis of the graph represents the geometric mean (Geo. Mean) of PE. The upper graph represents results of staining of HUVECs, the middle graph represents results of staining of hepatocytes, and the lower graph represents results of staining of CHO cells.

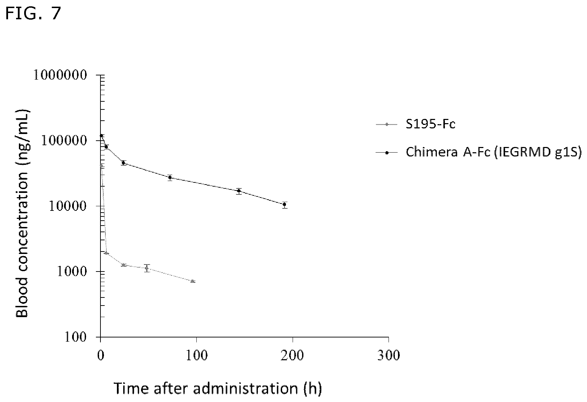

FIG. 7 shows the time course of blood concentration after i.v. administration of 10 mg/kg of S195-Fc and chimera A-Fc (IEGRMD g1S) in BALB/c mice. The vertical axis represents blood concentration (ng/mL), and the horizontal axis represents time after administration (h).

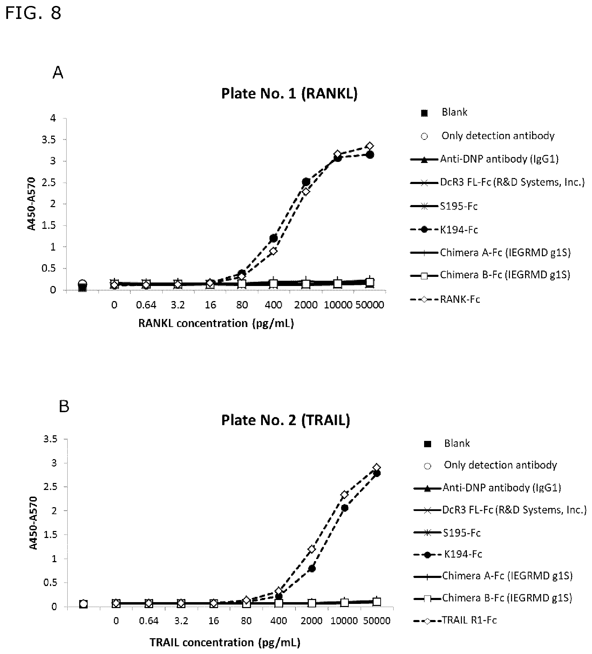

FIG. 8A and FIG. 8B show the bindings of various wild-type DcR3 controls and various DcR3 variants to RANKL (OPG ligand) and TRAIL (OPG ligand), respectively. After various wild-type DcR3 controls and various DcR3 variants were captured on plates to which anti-human antibodies were immobilized, RANKL or TRAIL diluted to each concentration was added, and the bindings were evaluated. For detection, a biotinylated anti-RANKL antibody or a biotinylated anti-TRAIL antibody and streptavidin-HRP were used. The horizontal axis represents the concentration (pg/mL) of RANKL or TRAIL, and the vertical axis represents absorbance (value obtained by subtracting the absorbance at 570 nm from the absorbance at 450 nm).

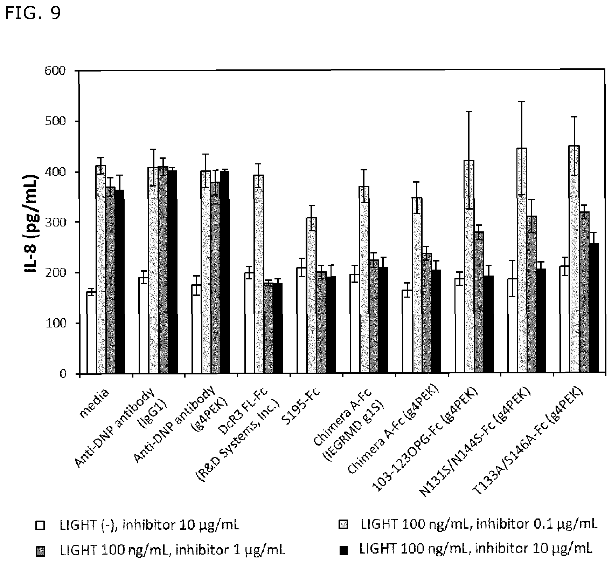

FIG. 9 shows the neutralizing activities of various wild-type DcR3 controls and various DcR3 variants to LIGHT. Production of IL-8 from HT-29 cells when 100 ng/mL of LIGHT and 0.1, 1 or 10 µg/mL of various wild-type DcR3 controls and various DcR3 variants were added is shown. The vertical axis represents IL-8 concentration (pg/mL), and the horizontal axis represents various DcR3 variants added as inhibitors.

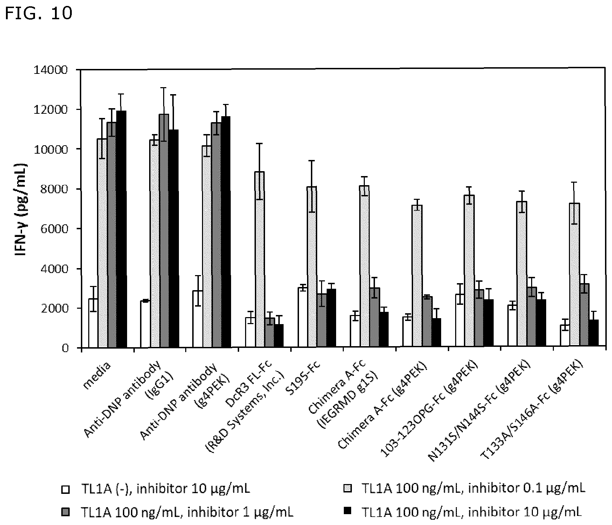

FIG. 10 shows the neutralizing activities of various wild-type DcR3 controls and various DcR3 variants to TL1A. Production of IFN-γ from human T cells when 100 ng/mL of TL1A and 0.1, 1 or 10 µg/mL of various wild-type DcR3 controls and various DcR3 variants were added is shown. The vertical axis represents IFN-γ concentration (pg/mL), and the horizontal axis represents various DcR3 variants added as inhibitors.

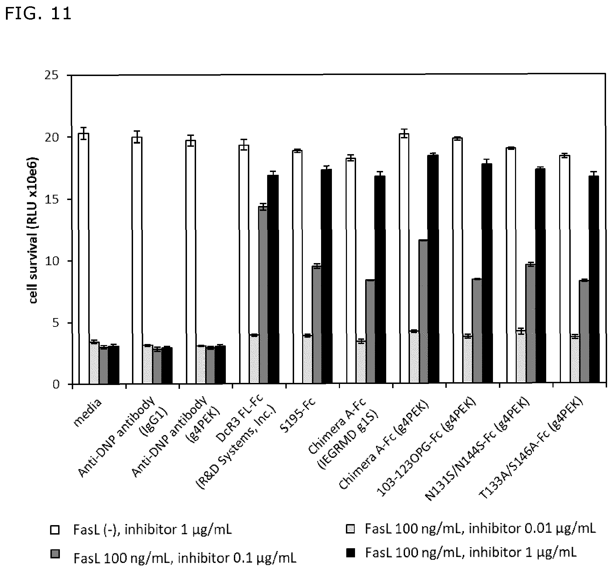

FIG. 11 shows the neutralizing activities of various wild-type DcR3 controls and various DcR3 variants to FasL. The production amount of ATP derived from Jurkat viable cells when 100 ng/mL of FasL and 0.01, 0.1 or 1 µg/mL of various wild-type DcR3 controls and various DcR3 variants were added is represented by RLU. The vertical axis represents cell survival (RLU × 10⁶) using ATP-dependent chemiluminescence as an index, and the horizontal axis represents various DcR3 variants added as inhibitors.

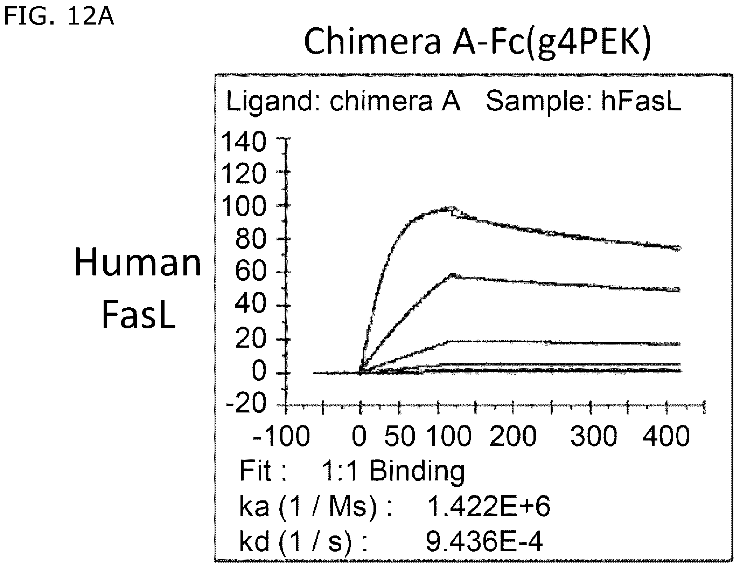

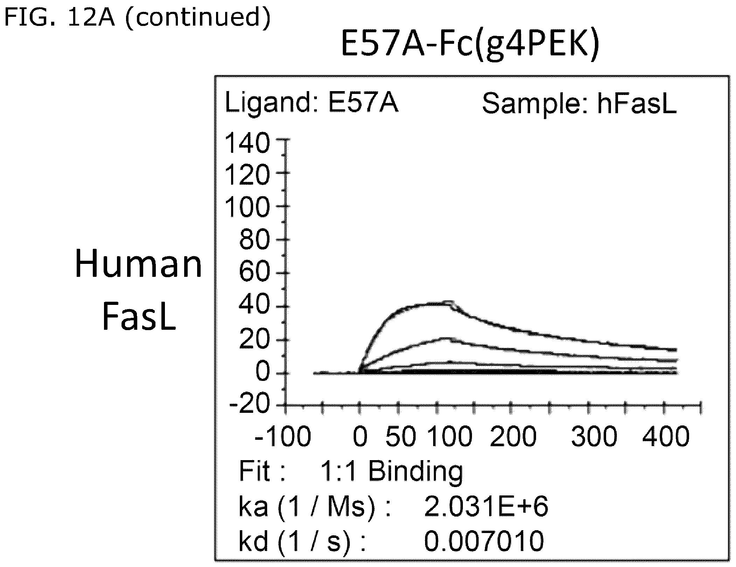

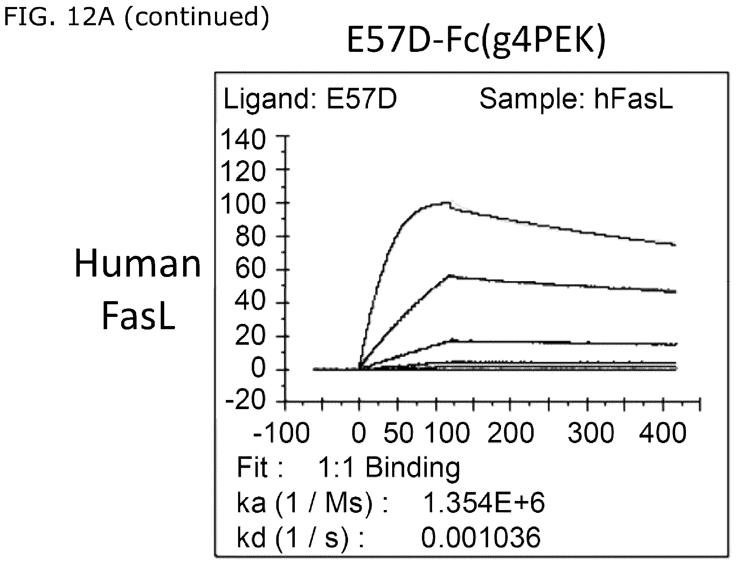

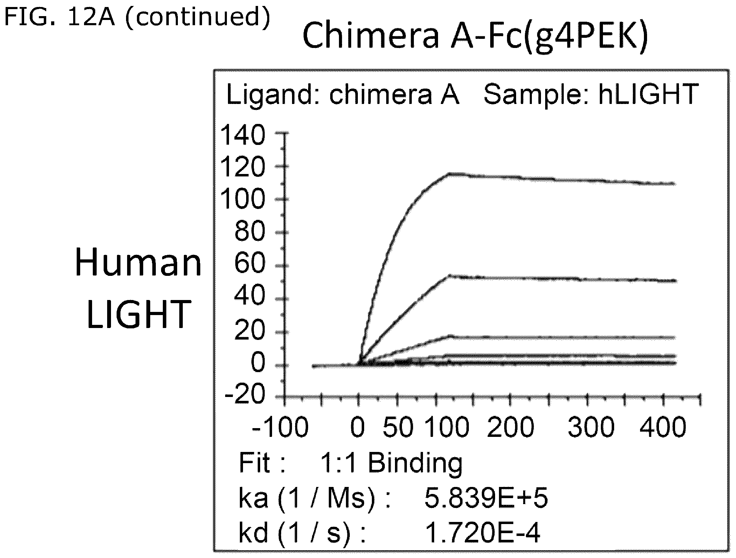

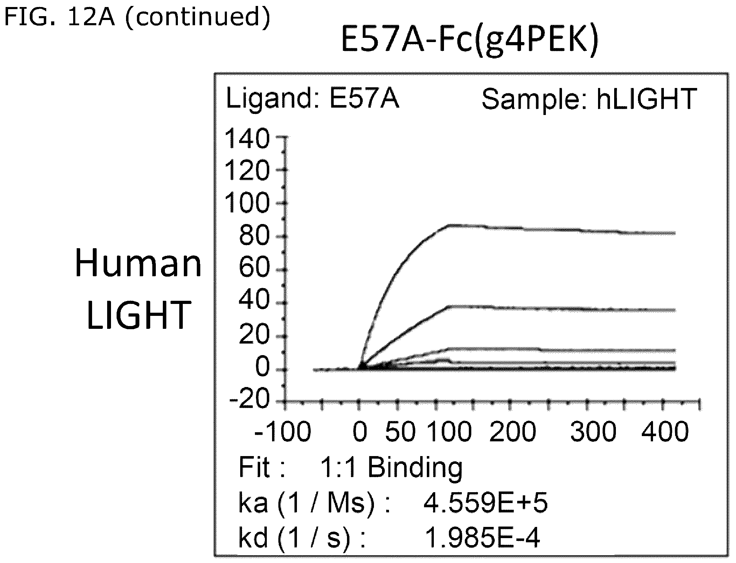

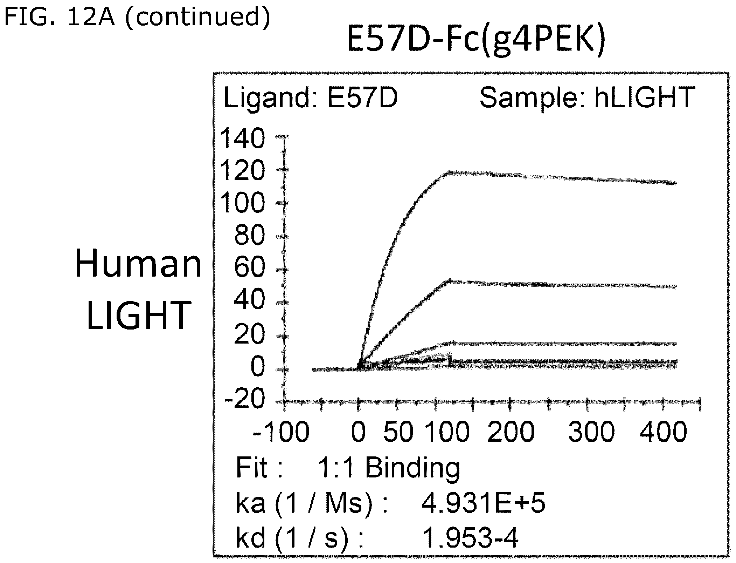

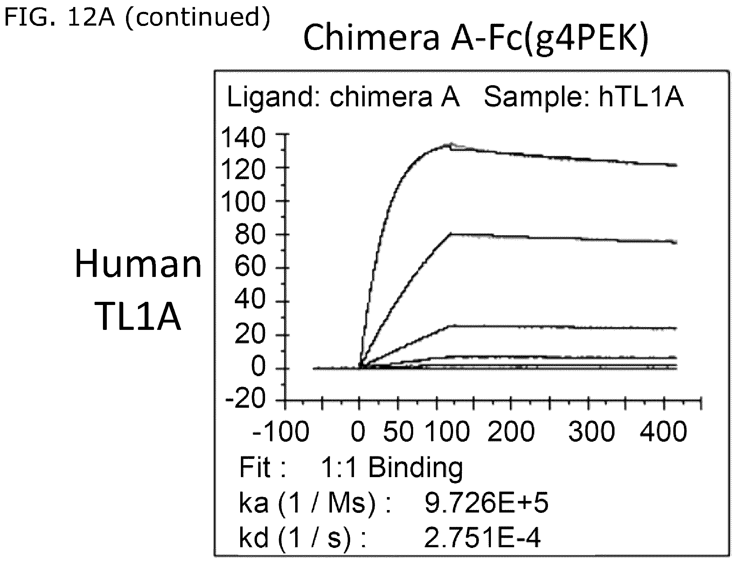

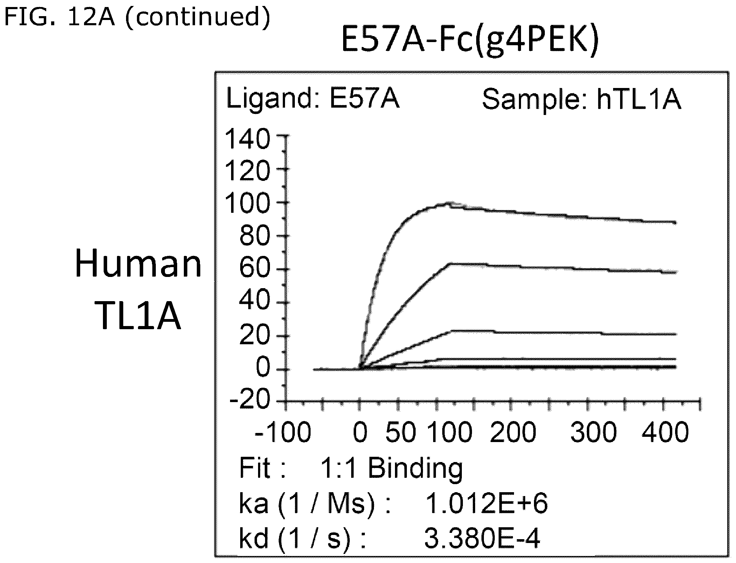

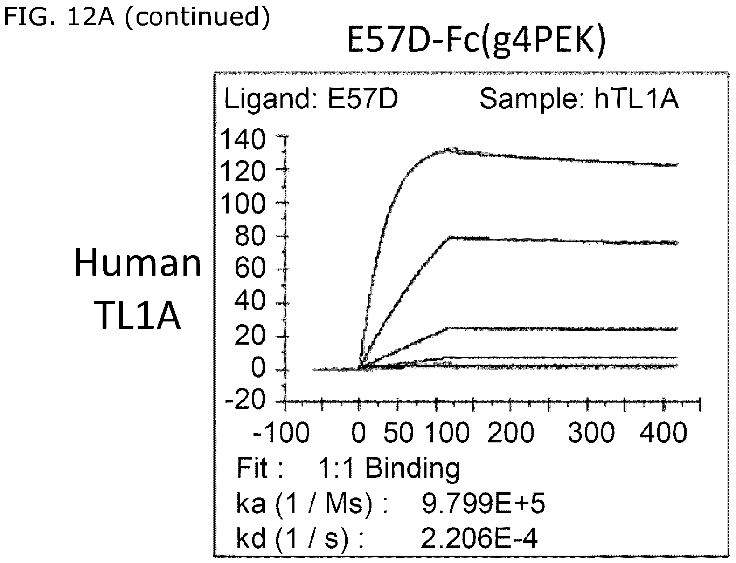

FIG. 12A shows the bindings of chimera A-Fc (g4PEK) and the variants (g4PEK) with low FasL binding activities to each DcR3 ligand. Sensorgrams when chimera A-Fc (g4PEK) or each of the variants with low FasL binding activities was captured on a sensor chip to which anti-human antibodies were immobilized, and a DcR3 ligand (human FasL, human LIGHT or human TL1A) as an analyte was injected are shown. The vertical axis represents resonance unit (RU), and the horizontal axis represents time (sec).

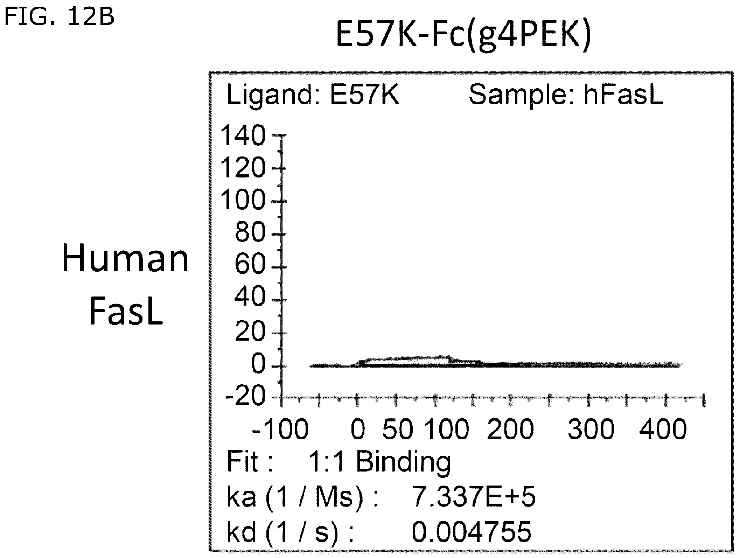

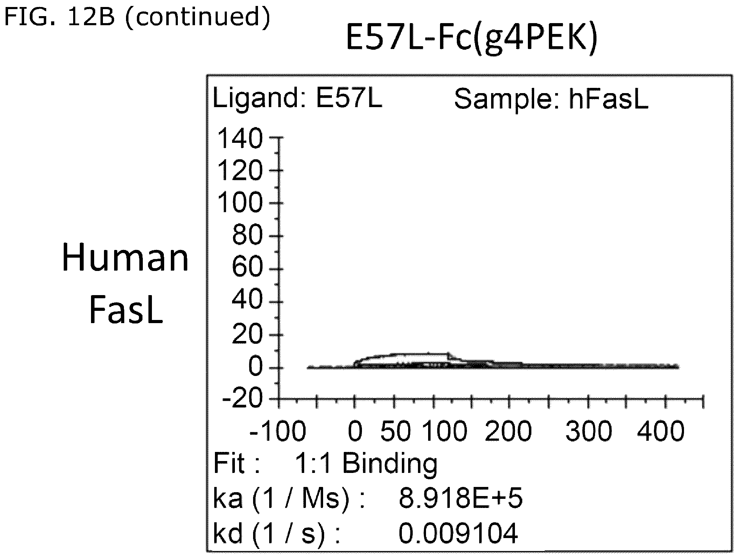

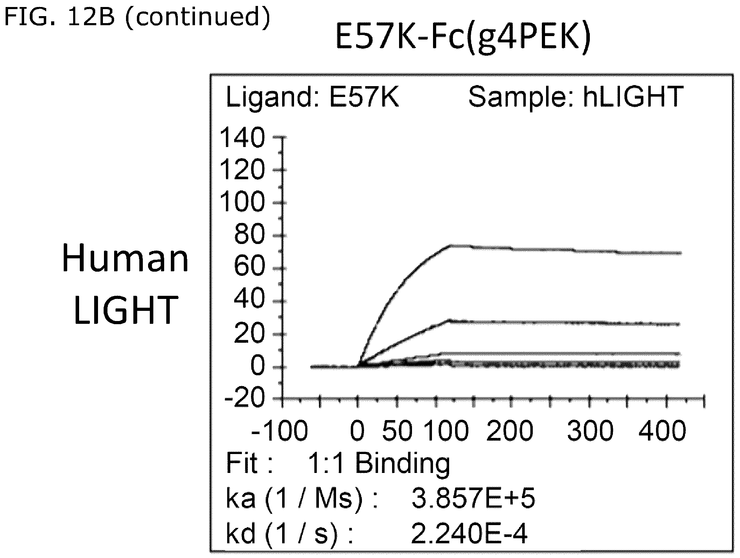

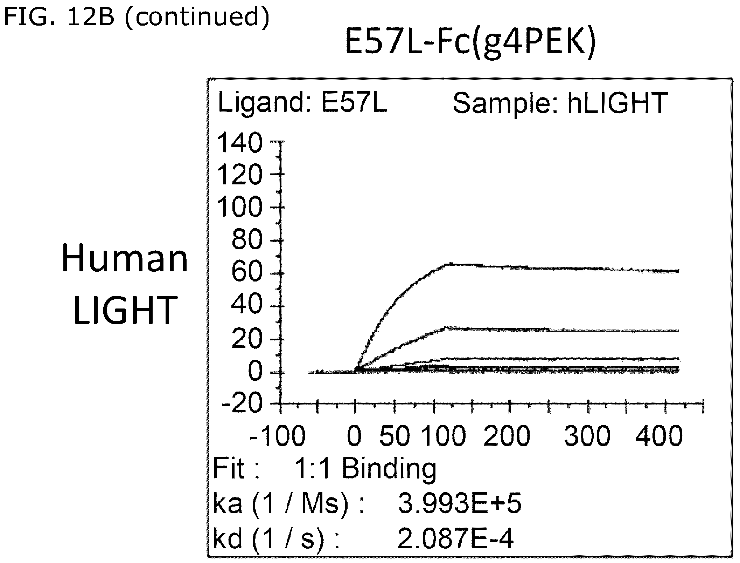

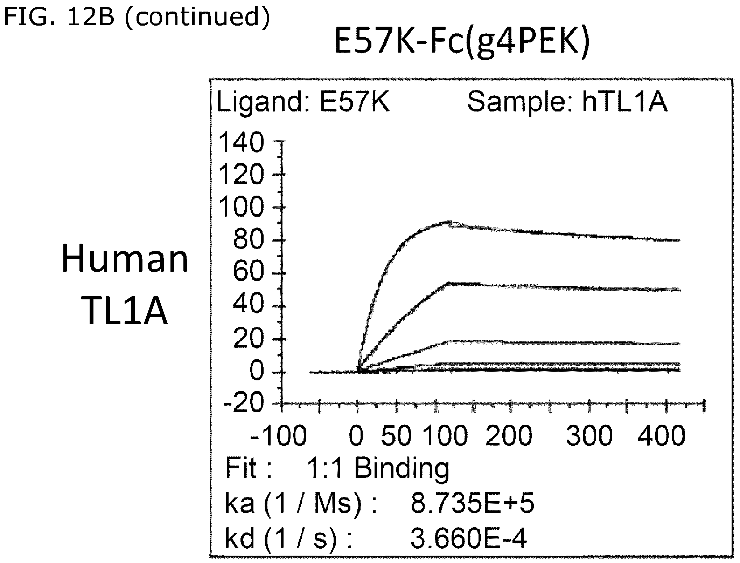

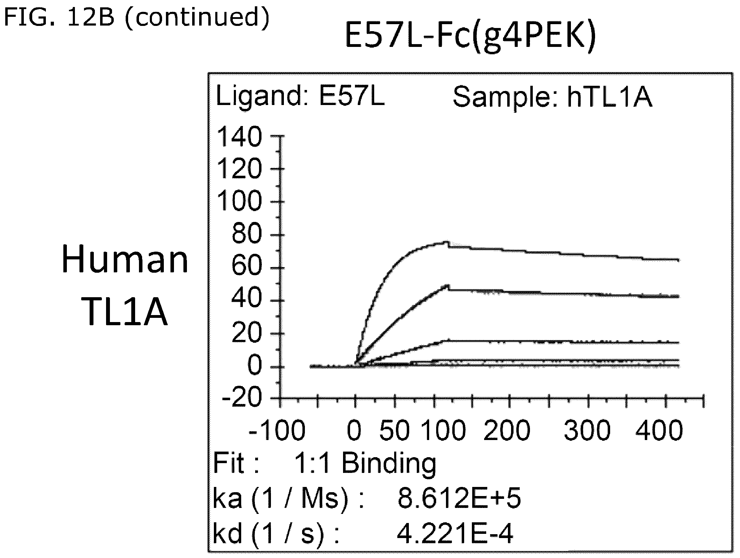

FIG. 12B shows the bindings of the variants (g4PEK) with low FasL binding activities to each DcR3 ligand. Sensorgrams when each of the variants with low FasL binding activities was captured on a sensor chip to which anti-human antibodies were immobilized, and a DcR3 ligand (human FasL, human LIGHT or human TL1A) as an analyte was injected are shown. The vertical axis represents resonance unit (RU), and the horizontal axis represents time (sec).

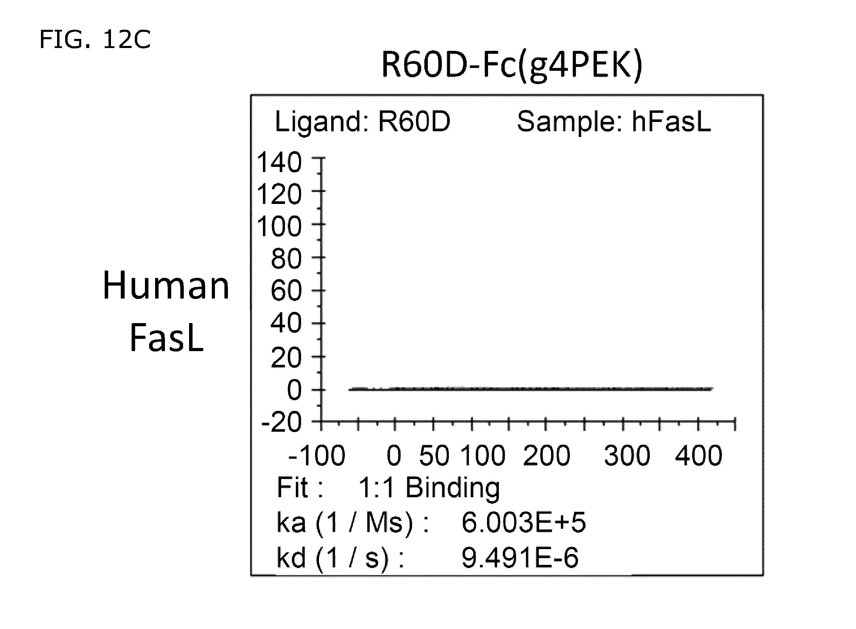

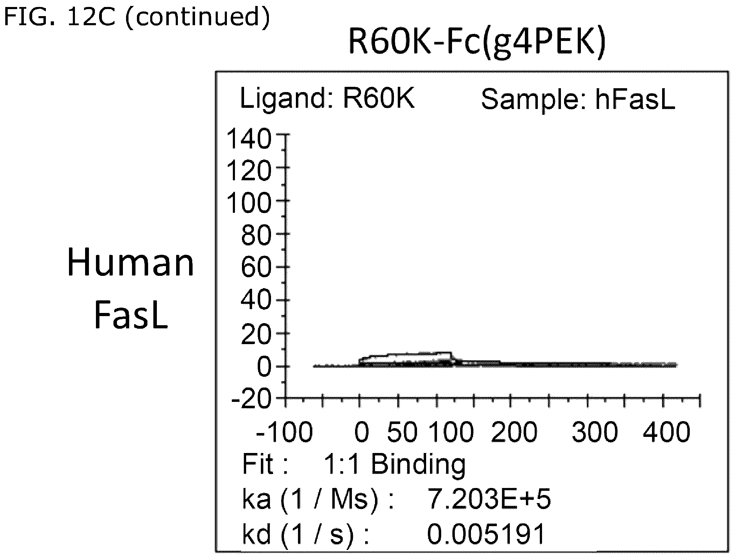

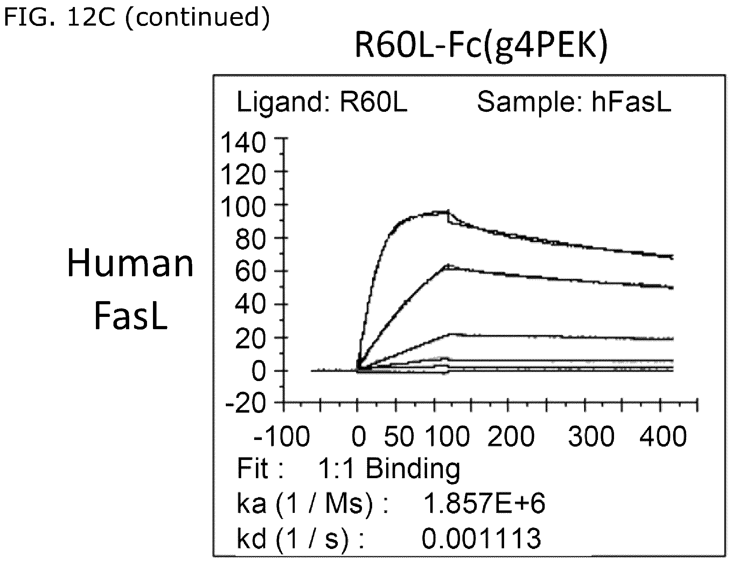

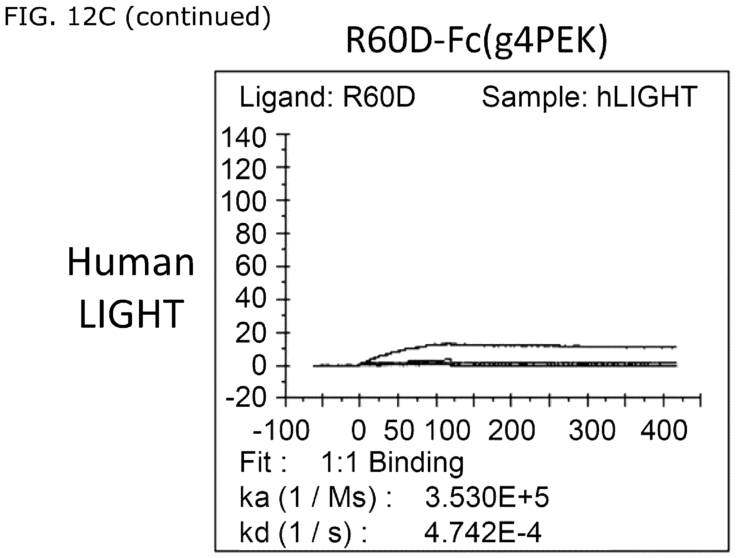

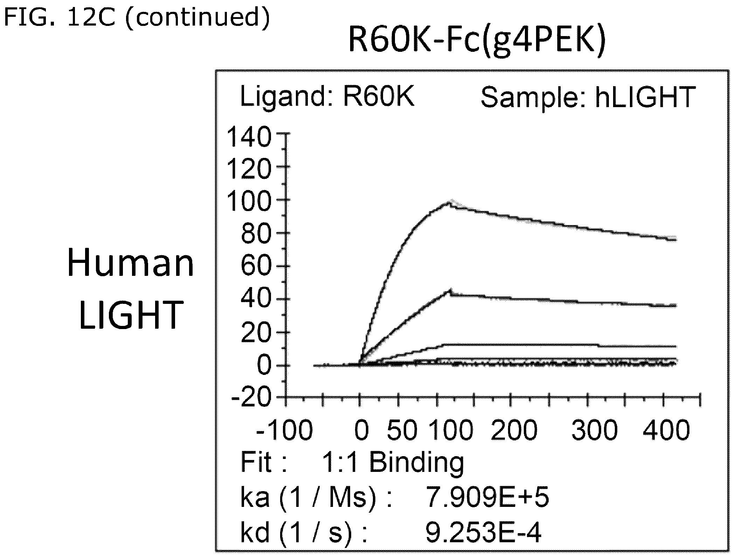

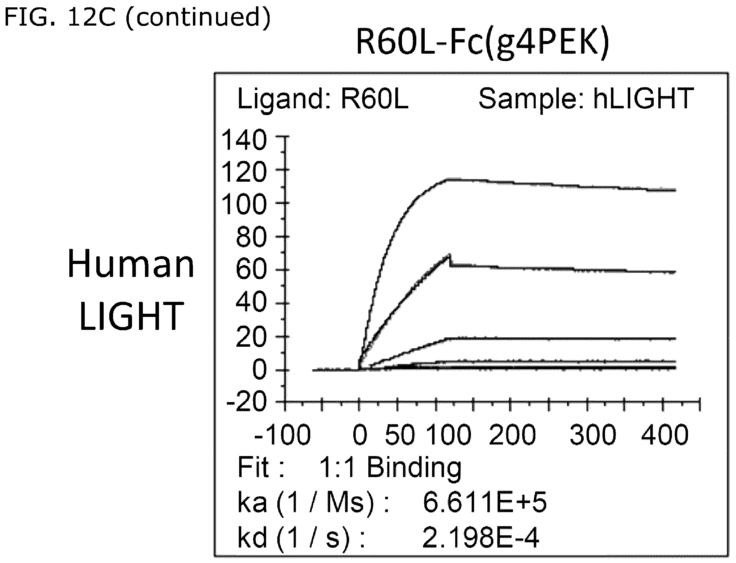

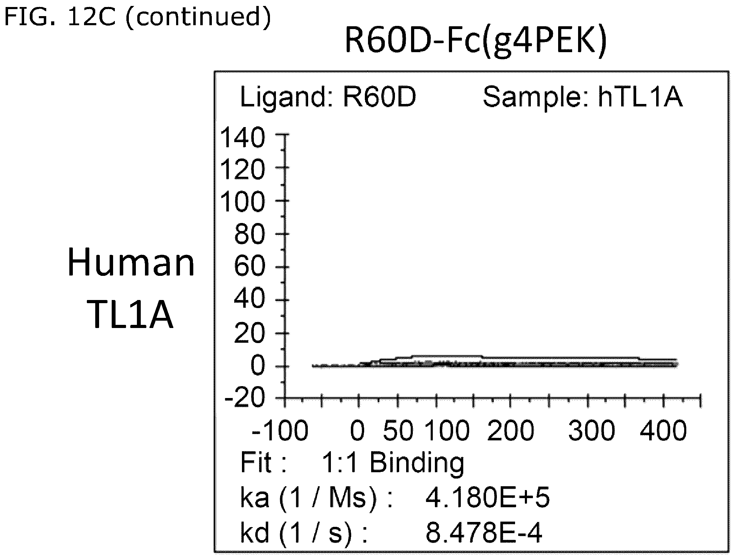

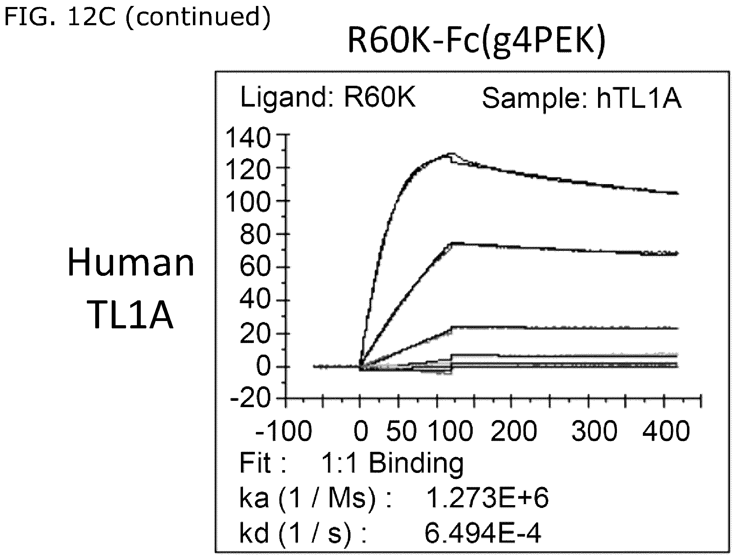

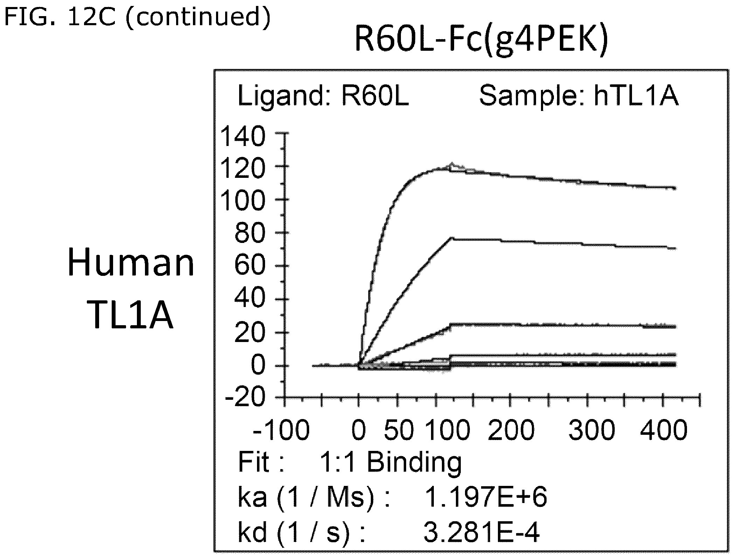

FIG. 12C shows the bindings of the variants (g4PEK) with low FasL binding activities to each DcR3 ligand. Sensorgrams when each of the variants with low FasL binding activities was captured on a sensor chip to which anti-human antibodies were immobilized, and a DcR3 ligand (human FasL, human LIGHT or human TL1A) as an analyte was injected are shown. The vertical axis represents resonance unit (RU), and the horizontal axis represents time (sec).

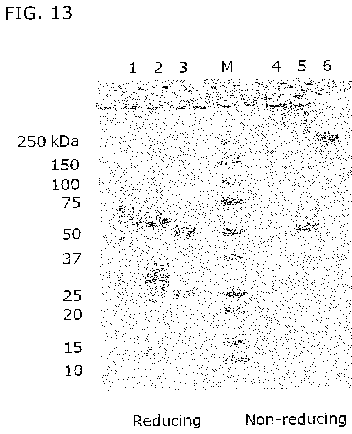

FIG. 13 shows results of SDS-PAGE of wild-type DcR3 controls produced using various mammalian cells. SDS-PAGE was performed by electrophoresing commercially available human DcR3-Fc produced using various mammalian cells under reducing or non-reducing conditions. As the mammalian cells, HEK293 cells (Abcam plc.) were used in lanes 1 and 4, CHO cells (AdipoGen Life Sciences, Inc.) were used in lanes 2 and 5, and HEK293 cells (Enzo Life Sciences, Inc.) were used in lanes 3 and 6. M represents a molecular weight marker (Bio-Rad Laboratories, Inc.).

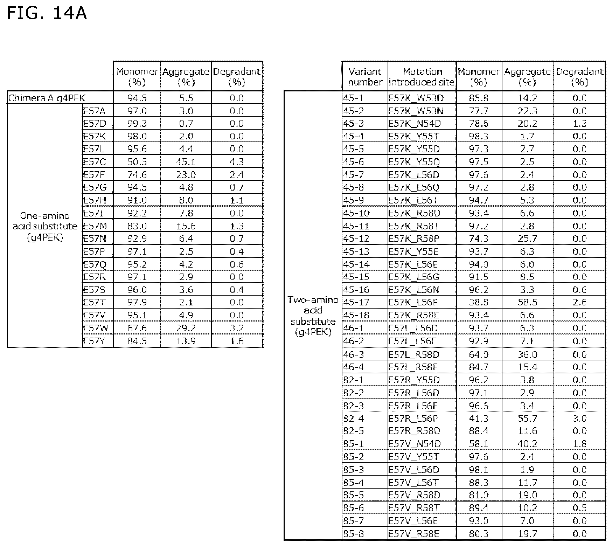

FIG. 14A shows the proportions (%) of the contents of monomers, aggregates and degradants calculated from the peak areas in SEC-HPLC or SEC-UPLC of the Protein A-purified variants with low FasL binding activities. FIG. 14A shows results of all the produced variants (g4PEK) with low FasL binding activities.

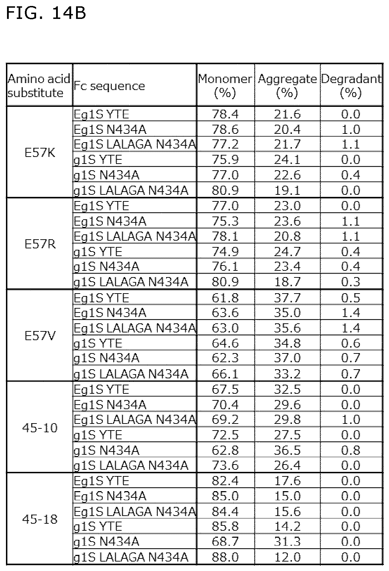

FIG. 14B shows the proportions (%) of the contents of monomers, aggregates and degradants calculated from the peak areas in SEC-HPLC or SEC-UPLC of the Protein A-purified variants with low FasL binding activities. FIG. 14B shows results of various mutated Fc fusions of the selected variants with low FasL binding activities.

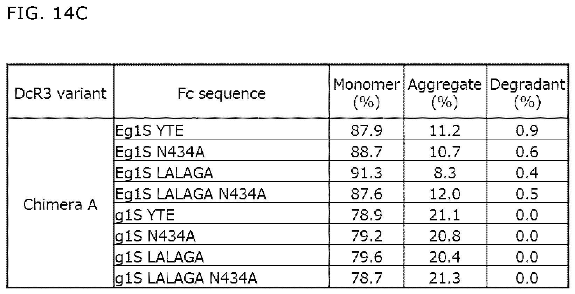

FIG. 14C shows the proportions (%) of the contents of monomers, aggregates and degradants calculated from the peak areas in SEC-HPLC or SEC-UPLC of various Protein A-purified chimera A-mutated Fc fusions.

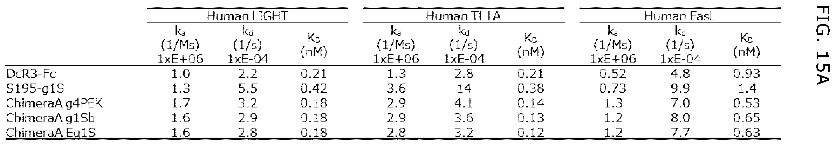

FIG. 15A shows measurement results of the binding activities of various DcR3 variants to each DcR3 ligand by BIAcore. Various kinetic constants (ka, kd and K_{D}) when various DcR3 variants were captured on a sensor chip to which anti-human antibodies were immobilized, and a trimeric DcR3 ligand (human FasL, human LIGHT or human TL1A) as an analyte was injected are shown. FIG. 15A shows results of various chimera A-Fc variants including different Fc sequences.

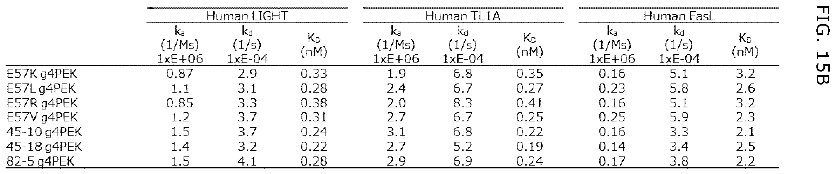

FIG. 15B shows measurement results of the binding activities of various DcR3 variants to each DcR3 ligand by BIAcore. Various kinetic constants (ka, kd and KD) when various DcR3 variants were captured on a sensor chip to which anti-human antibodies were immobilized, and a trimeric DcR3 ligand (human FasL, human LIGHT or human TL1A) as an analyte was injected are shown. FIG. 15B shows results of the variants with low FasL binding activities.

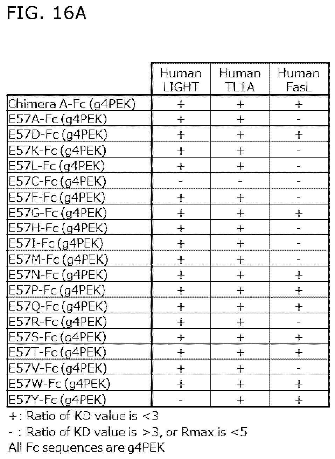

FIG. 16A shows results of the kinetic constant (KD value) to each DcR3 ligand calculated by BIAcore at selection of the variants with low FasL binding activities, compared to that of chimera A-Fc (g4PEK). FIG. 16A shows results of one-amino-acid substitutes.

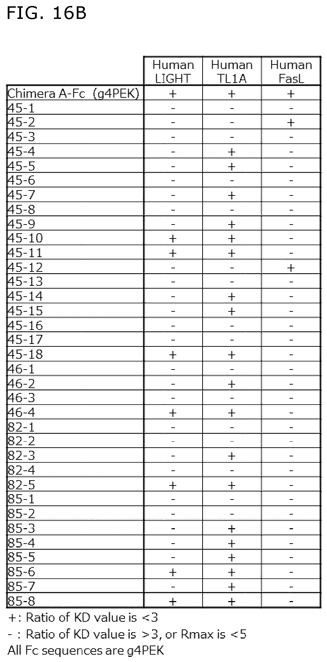

FIG. 16B shows results of the kinetic constant (KD value) to each DcR3 ligand calculated by BIAcore at selection of the variants with low FasL binding activities, compared to that of chimera A-Fc (g4PEK). FIG. 16B shows results of two-amino-acid substitutes.

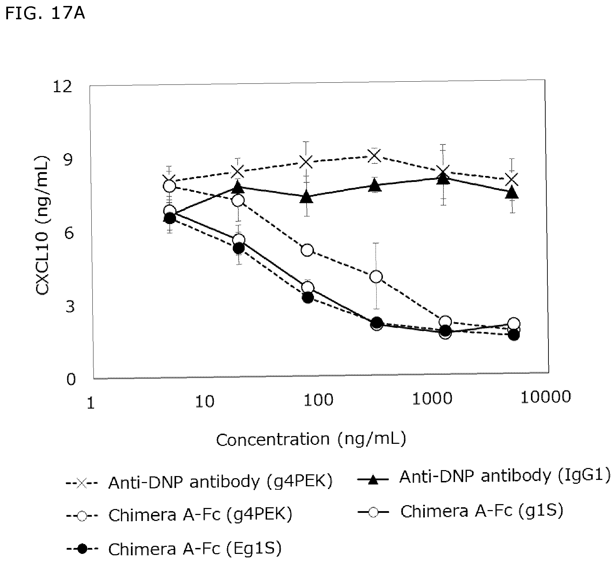

FIG. 17A shows evaluation results of the neutralizing activities of various DcR3 variants to soluble LIGHT. FIG. 17A shows the inhibitory activities of various chimera A-Fc variants including different Fc sequences to LIGHT-dependent-CXCL10 production from IFN-γ-stimulated intestinal myofibroblasts. The vertical axis represents CXCL10 concentration (ng/mL), and the horizontal axis represents the concentration (ng/mL) of DcR3 variants added.

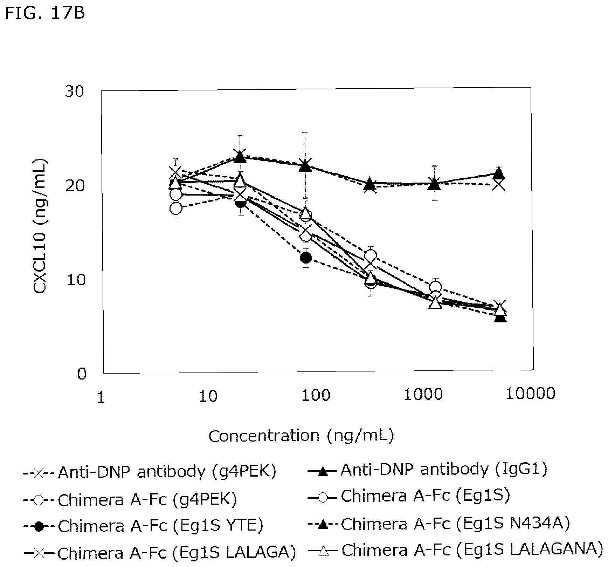

FIG. 17B shows evaluation results of the neutralizing activities of various DcR3 variants to soluble LIGHT. FIG. 17B shows the inhibitory activities of chimera A-Fc variants including different mutation-introduced Fc sequences to LIGHT-dependent CXCL10 production from IFN-γ-stimulated intestinal myofibroblasts. The vertical axis represents CXCL10 concentration (ng/mL), and the horizontal axis represents the concentration (ng/mL) of DcR3 variants added.

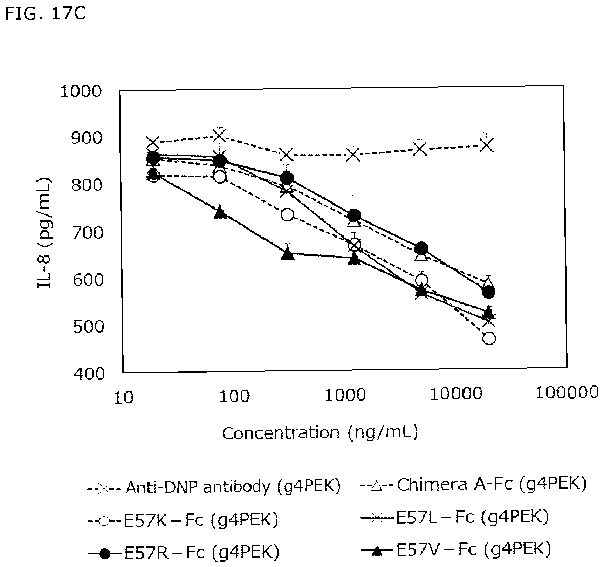

FIG. 17C shows evaluation results of the neutralizing activities of various DcR3 variants to soluble LIGHT. FIG. 17C shows the inhibitory activities of one-amino-acid substituted variants with low FasL binding activites to LIGHT-dependent IL-8 production from HT-29 cells. The vertical axis represents IL-8 concentration (ng/mL), and the horizontal axis represents the concentration (ng/mL) of DcR3 variants added.

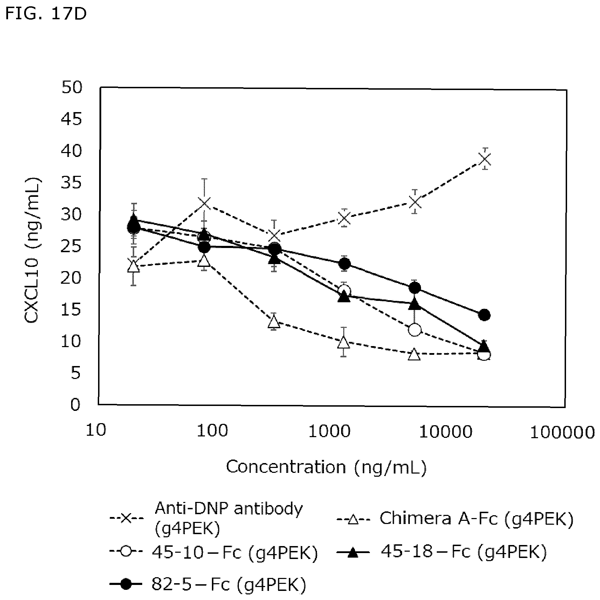

FIG. 17D shows evaluation results of the neutralizing activities of various DcR3 variants to soluble LIGHT. FIG. 17D shows the inhibitory activities of two-amino-acid substituted variants with low FasL binding activities to LIGHT-dependent CXCL10 production from IFN-γ-stimulated intestinal myofibroblasts. The vertical axis represents CXCL10 concentration (ng/mL), and the horizontal axis represents the concentration (ng/mL) of DcR3 variants added.

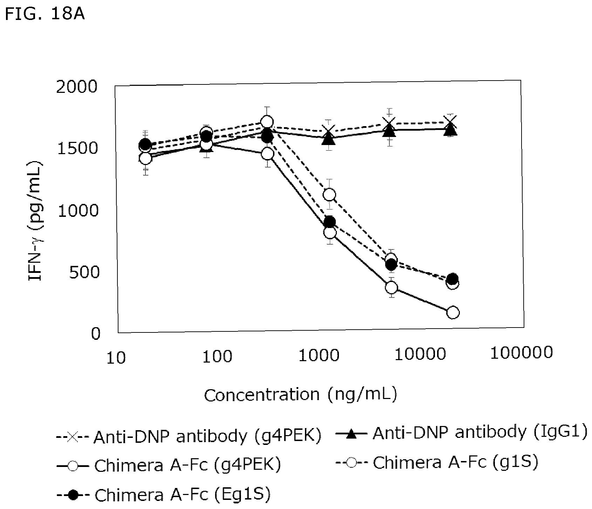

FIG. 18A shows evaluation results of the neutralizing activities of various DcR3 variants to soluble TL1A. FIG. 18A shows the inhibitory activities of various chimera A-Fc variants including different Fc sequences to TL1A-dependent IFN-γ production from IL-12- and IL-18-stimulated human T cells. The vertical axis represents IFN-γ concentration (pg/mL), and the horizontal axis represents the concentration (ng/mL) of DcR3 variants added.

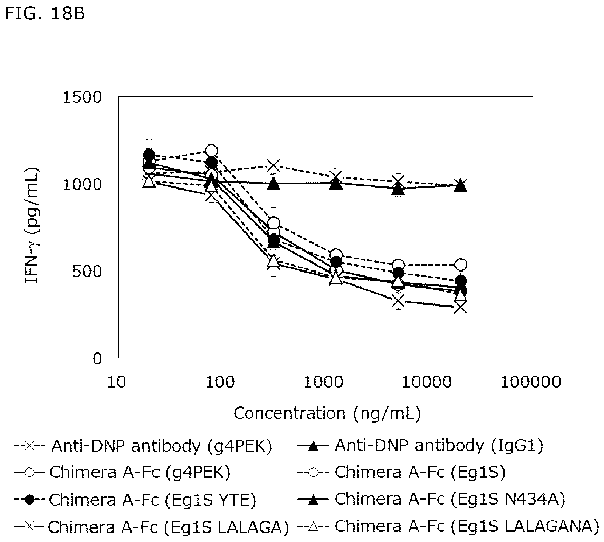

FIG. 18B shows evaluation results of the neutralizing activities of various DcR3 variants to soluble TL1A. FIG. 18B shows the inhibitory activities of chimera A-Fc variants including different mutation-introduced Fc sequences to TL1A-dependent IFN-γ production from IL-12- and IL-18-stimulated human T cells. The vertical axis represents IFN-γ concentration (pg/mL), and the horizontal axis represents the concentration (ng/mL) of DcR3 variants added.

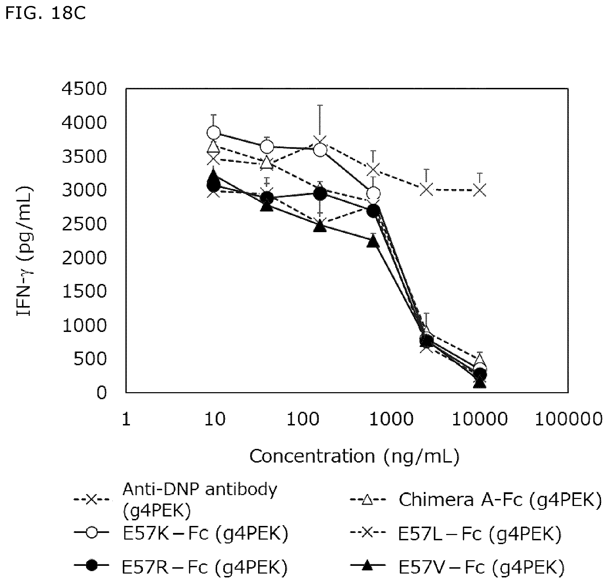

FIG. 18C shows evaluation results of the neutralizing activities of various DcR3 variants to soluble TL1A. FIG. 18C shows the inhibitory activities of one-amino-acid substituted variants with low FasL binding activities to TL1A-dependent IFN-γ production from IL-12- and IL-18-stimulated human T cells. The vertical axis represents IFN-γ concentration (pg/mL), and the horizontal axis represents the concentration (ng/mL) of DcR3 variants added.

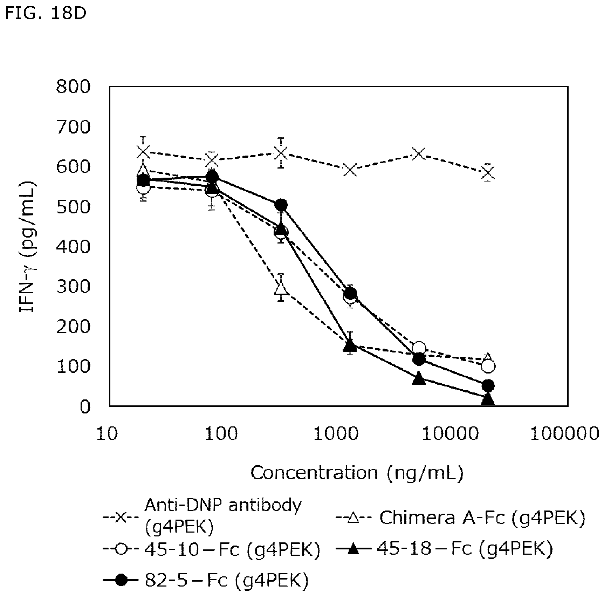

FIG. 18D shows evaluation results of the neutralizing activities of various DcR3 variants to soluble TL1A. FIG. 18D shows the inhibitory activities of two-amino-acid substituted variants with low FasL binding activities to TL1A-dependent IFN-γ production from IL-12- and IL-18-stimulated human T cells. The vertical axis represents IFN-γ concentration (pg/mL), and the horizontal axis represents the concentration (ng/mL) of DcR3 variants added.

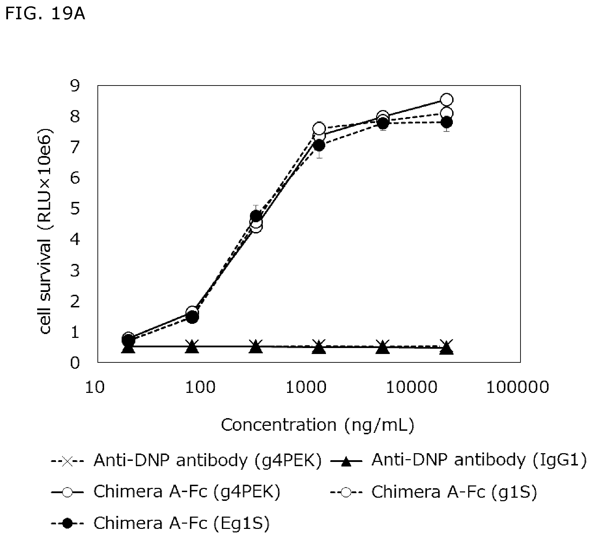

FIG. 19A shows evaluation results of the neutralizing activities of various DcR3 variants to soluble FasL. FIG. 19A shows the inhibitory activities of various chimera A-Fc variants including different Fc sequences to cell death of A3 cells. The vertical axis represents cell survival (RLU × 10⁶) using ATP-dependent chemiluminescence as an index, and the horizontal axis represents the concentration (ng/mL) of various DcR3 variants added.

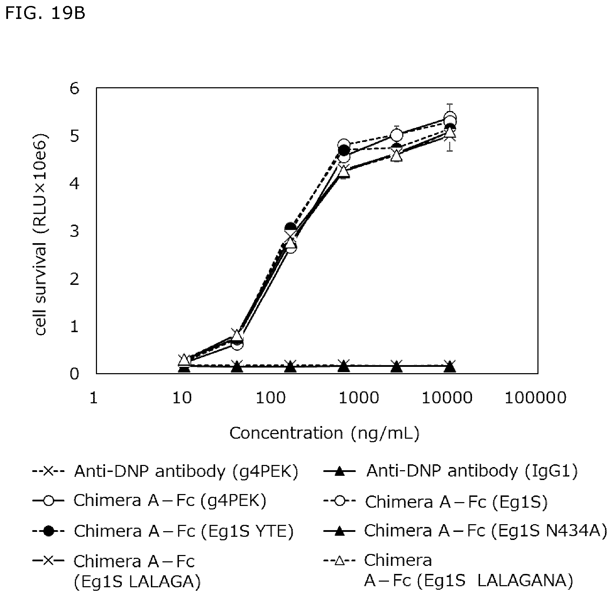

FIG. 19B shows evaluation results of the neutralizing activities of various DcR3 variants to soluble FasL. FIG. 19B shows the inhibitory activities of chimera A-Fc variants including different mutation-introduced Fc sequences to cell death of Jurkat cells. The vertical axis represents cell survival (RLU × 10⁶) using the production amount of ATP as an index, and the horizontal axis represents the concentration (ng/mL) of various DcR3 variants added.

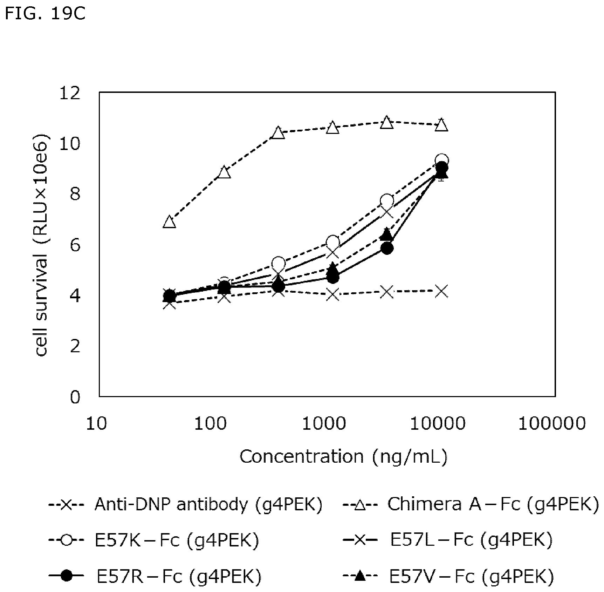

FIG. 19C shows evaluation results of the neutralizing activities of various DcR3 variants to soluble FasL. FIG. 19C shows the inhibitory activities of one-amino-acid substituted variants with low FasL binding activities to cell death of Jurkat cells. The vertical axis represents cell survival (RLU × 10⁶) using the production amount of ATP as an index, and the horizontal axis represents the concentration (ng/mL) of various DcR3 variants added.

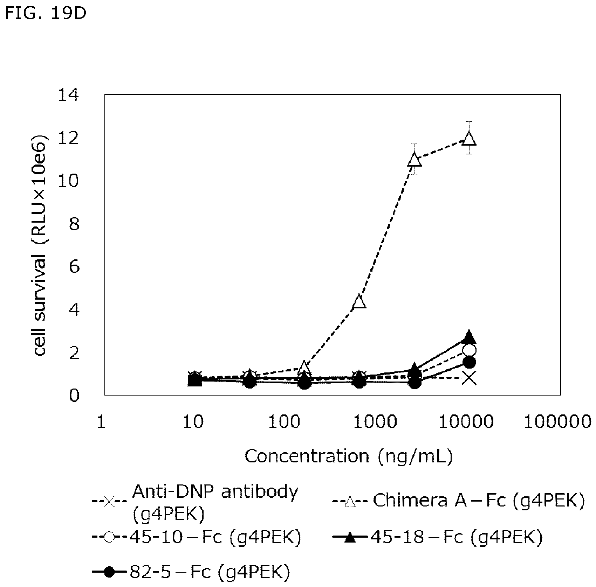

FIG. 19D shows evaluation results of the neutralizing activities of various DcR3 variants to soluble FasL. FIG. 19D shows the inhibitory activities of two-amino-acid substituted variants with low FasL binding activities to cell death of Jurkat cells. The vertical axis represents cell survival (RLU × 10⁶) using the production amount of ATP as an index, and the horizontal axis represents the concentration (ng/mL) of various DcR3 variants added.

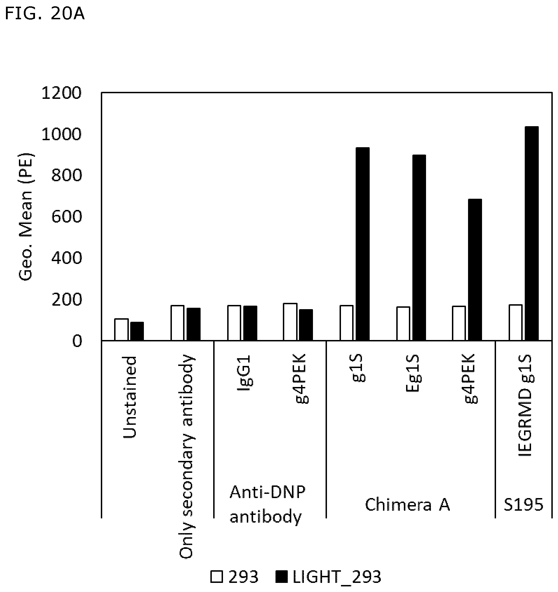

FIG. 20A shows evaluation results of the binding activities of S195-Fc and various chimera A-Fc variants including different Fc sequences to cells of a membrane-bound LIGHT forced expression cell line. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

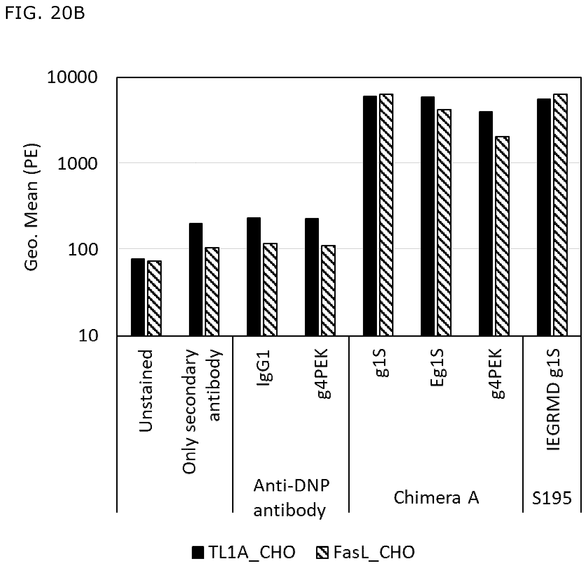

FIG. 20B shows evaluation results of the binding activities of S195-Fc and various chimera A-Fc variants including different Fc sequences to cells of membrane-bound TL1A and membrane-bound FasL forced expression cell lines. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

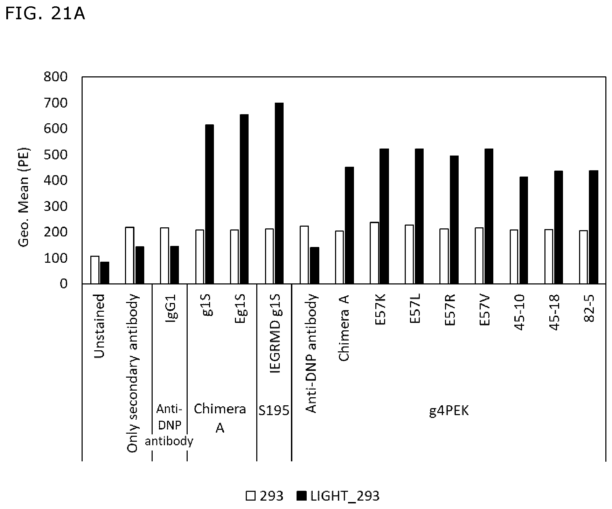

FIG. 21A shows evaluation results of the binding activities of S195-Fc, various chimera A-Fc variants and the variants with low FasL binding activities to cells of a membrane-bound LIGHT forced expression cell line. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

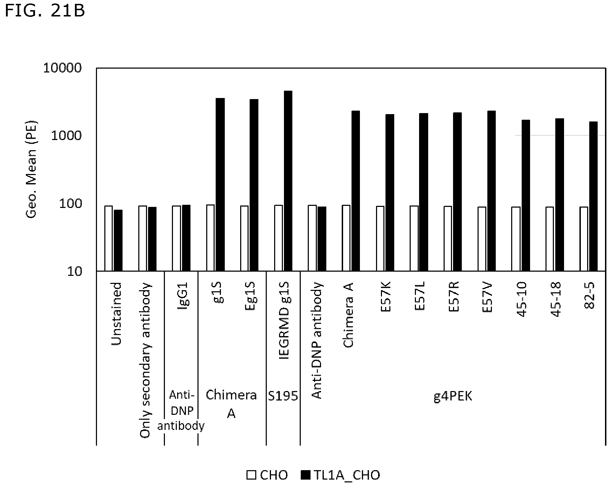

FIG. 21B shows evaluation results of the binding activities of S195-Fc, various chimera A-Fc variants and the variants with low FasL binding activities to cells of a membrane-bound TL1A forced expression cell line. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

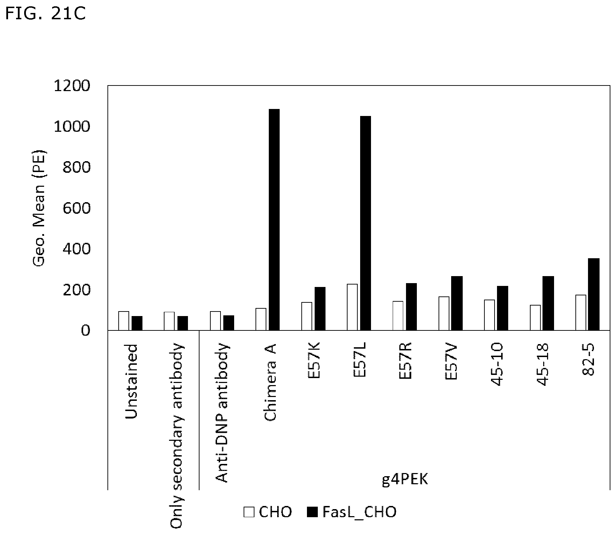

FIG. 21C shows evaluation results of the binding activities of chimera A-Fc and the variants (g4PEK) with low FasL binding activities to cells of a membrane-bound FasL forced expression cell line. To each cell, various DcR3 variants were reacted, followed by staining with a PE-labeled anti-human antibody, and the fluorescence intensity of PE was measured by flow cytometry. The vertical axis of the graph represents the geometric mean (Geo. Mean) of PE.

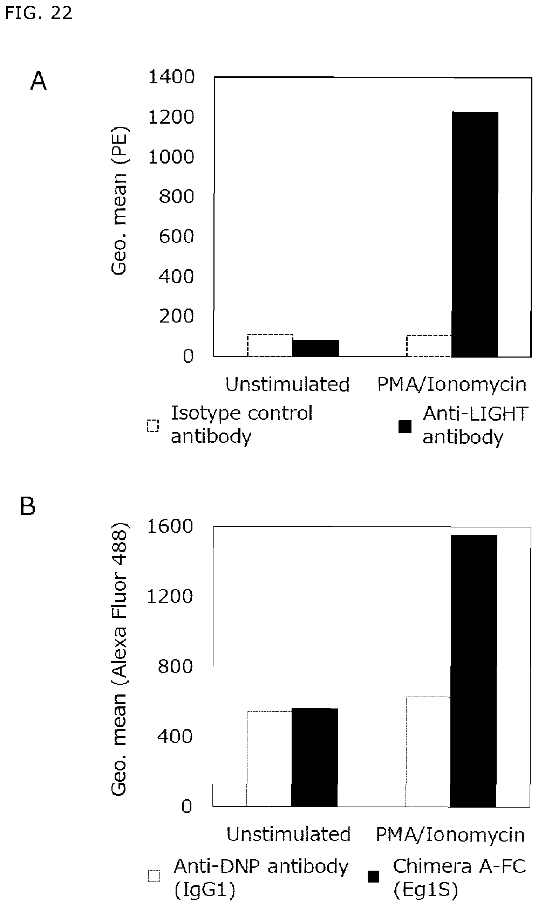

FIG. 22 shows evaluation results of the binding activity of chimera A-Fc to membrane-bound LIGHT of a primary cell. FIG. 22A shows measurement results of the expression level of membrane-bound LIGHT on activated human T cells by a PE-labeled anti-LIGHT antibody. FIG. 22B shows the geometric mean (Geo. Mean) calculated by measuring the binding of Alexa Fluor 488-labeled chimera A-Fc to membrane-bound LIGHT on activated human T cells by flow cytometry. In both figures, the vertical axis represents the geometric mean (Geo. Mean) of fluorescence intensity.

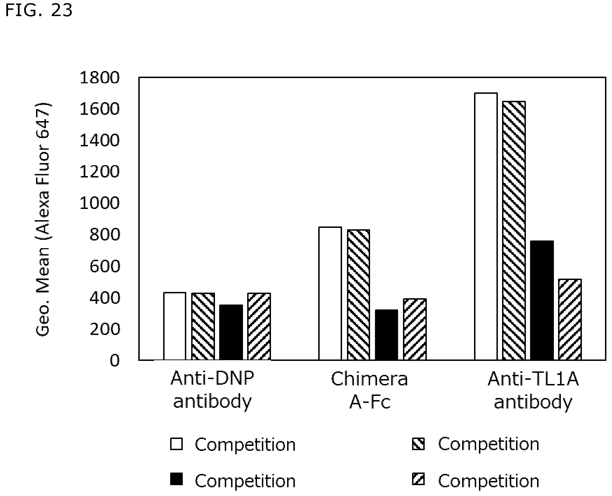

FIG. 23 shows evaluation results of the binding activity of chimera A-Fc to membrane-bound TL1A of primary cells. FIG. 23 shows the geometric mean (Geo. Mean) calculated by measuring the binding of Alexa Fluor 647-labeled chimera A-Fc in the presence or absence of competitive proteins to membrane-bound TL1A on HUVECs by flow cytometry.

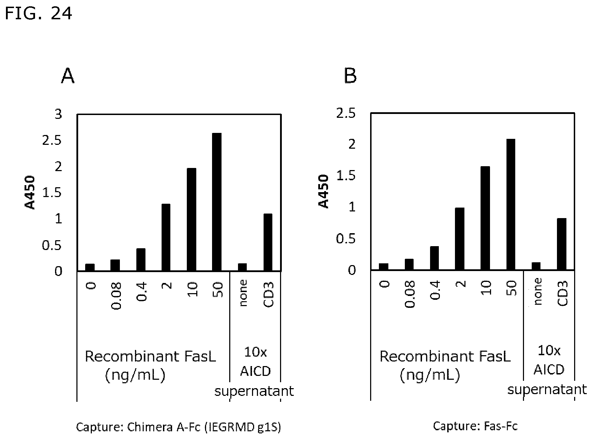

FIG. 24 shows measurement results of the binding activity of chimera A-Fc to FasL derived from primary cells by sandwich ELISA to soluble FasL in culture supernatants of human T cells stimulated for AICD. The figure shows the value of absorbance at 450 nm when a culture supernatant was added to a plate on which chimera A-Fc (FIG. 24A) or Fas-Fc (FIG. 24B) was captured, followed by detection with an anti-FasL antibody.

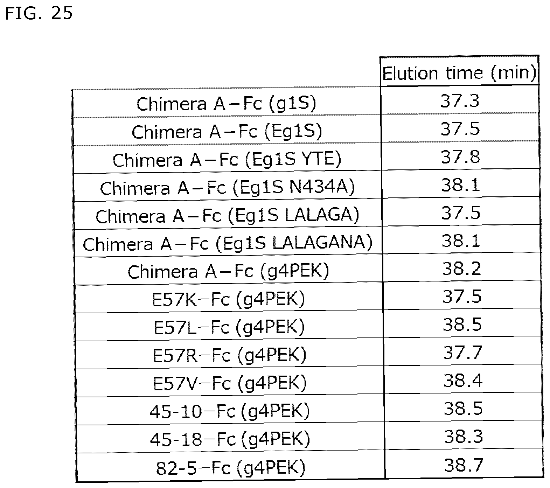

FIG. 25 shows results of the elution times (minute) of various chimera A-Fc variants including different Fc sequences and the variants with low FasL binding activities in hydrophobic interaction chromatography (HIC).

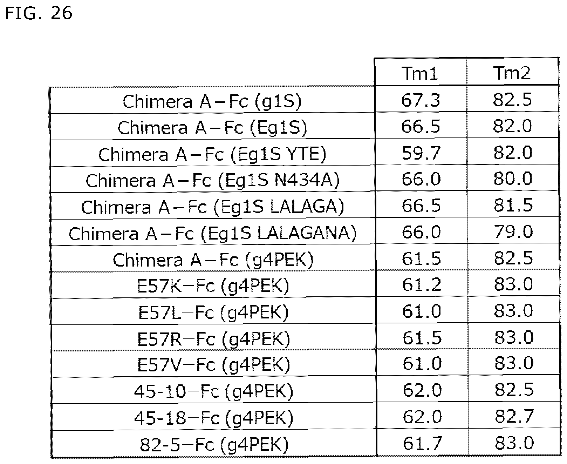

FIG. 26 shows results of the Tm values (°C) calculated by the differential scanning fluorimetry (DSF) method of various chimera A-Fc variants including different Fc sequences and the variants with low FasL binding activities.

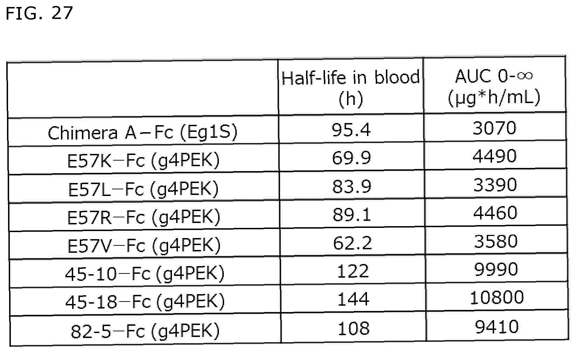

FIG. 27 shows the values of the half-life in blood (h) during the elimination phase and the area under the concentration-time curve to infinity AUC0-∞ (µg*h/mL) after i.v. administration of 10 mg/kg of chimera A-Fc (Eg1S) including a different Fc sequence and the variants with low FasL binding activities in BALB/c mice.

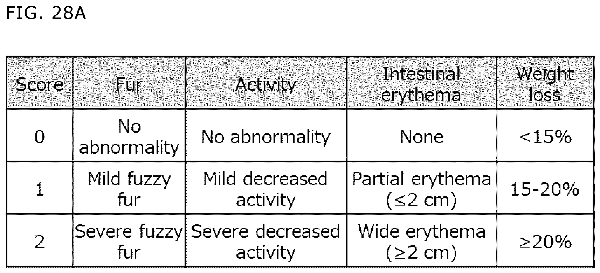

FIG. 28A shows indices of gross pathological scores in a drug efficacy study of chimera A-Fc using a mouse acute xenogeneic GVHD model.

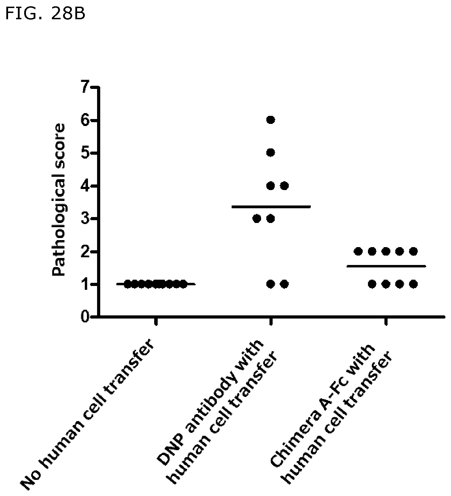

FIG. 28B shows pathological scores for each individual of each group and as a mean of each group. The vertical axis represents the pathological score, and the horizontal axis represents each treatment group.

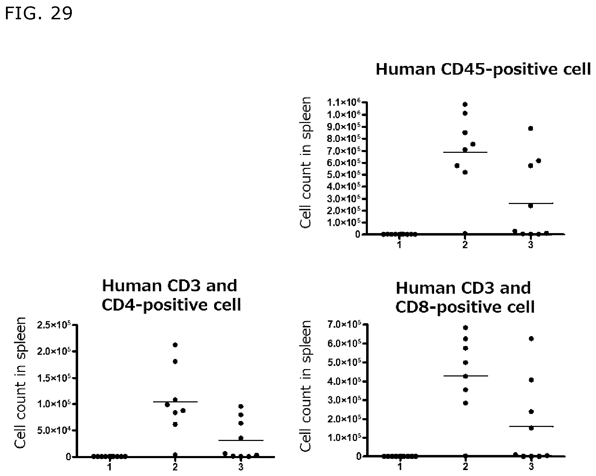

FIG. 29 shows the cell count per spleen for each individual of each group and as a mean value of each group regarding human CD45-positive cells, human CD3 and CD4-positive cells, and human CD3 and CD8-positive cells in a drug efficacy study of chimera A-Fc using a mouse acute xenogeneic GVHD model. Group 1 represents no human cell transfer, group 2 represents a group of administration of anti-DNP antibody with human cell transfer, and group 3 represents a group of administration of chimera A-Fc with human cell transfer. The vertical axis represents each surface marker-positive cell count, and the horizontal axis represents each treatment group.

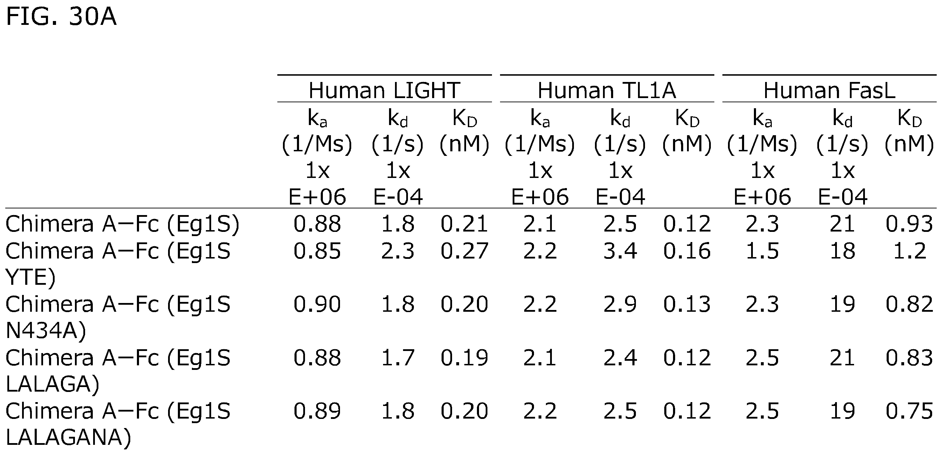

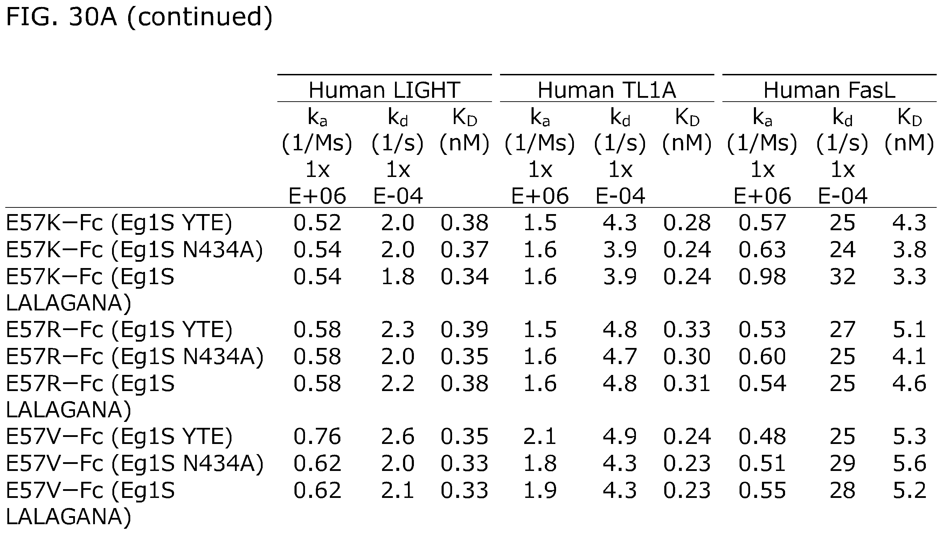

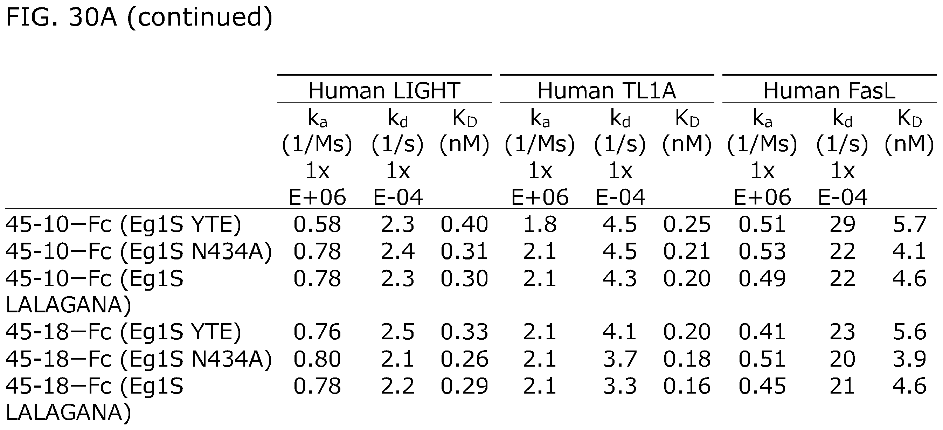

FIG. 30A shows measurement results of the binding activities of various DcR3 variants to human DcR3 ligands by BIAcore. Various kinetic constants (ka, kd and K_{D}) when a trimer of human FasL, human LIGHT or human TL1A was used as a human DcR3 ligand are shown.

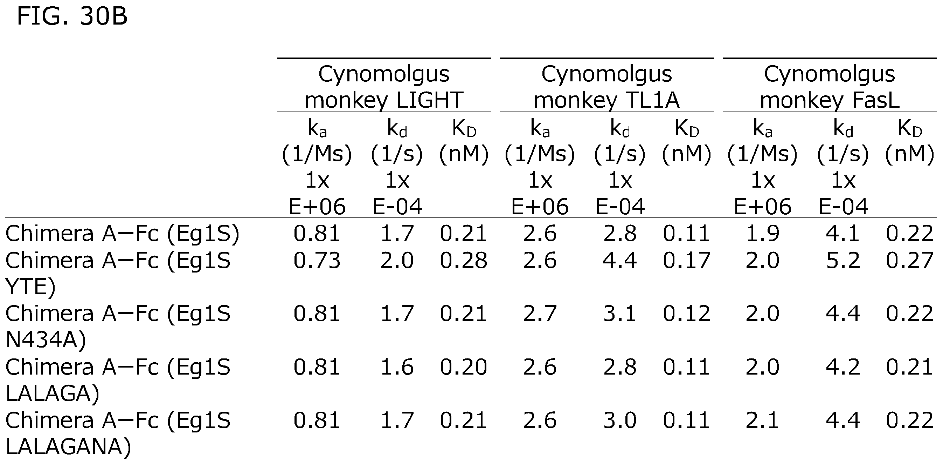

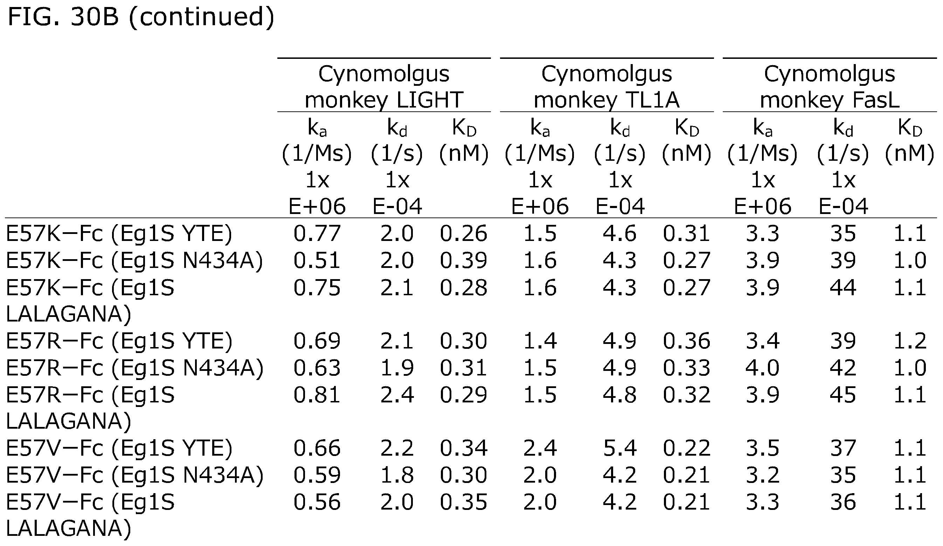

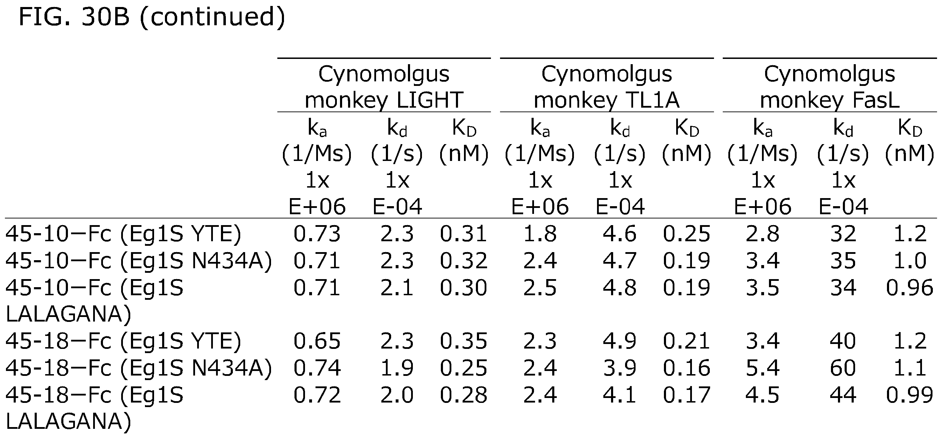

FIG. 30B shows measurement results of the binding activities of various DcR3 variants to cynomolgus monkey DcR3 ligands by BIAcore. Various kinetic constants (ka, kd and K_{D}) when a trimer of cynomolgus monkey FasL, cynomolgus monkey LIGHT or cynomolgus monkey TL1A was used as a cynomolgus monkey DcR3 ligand are shown.

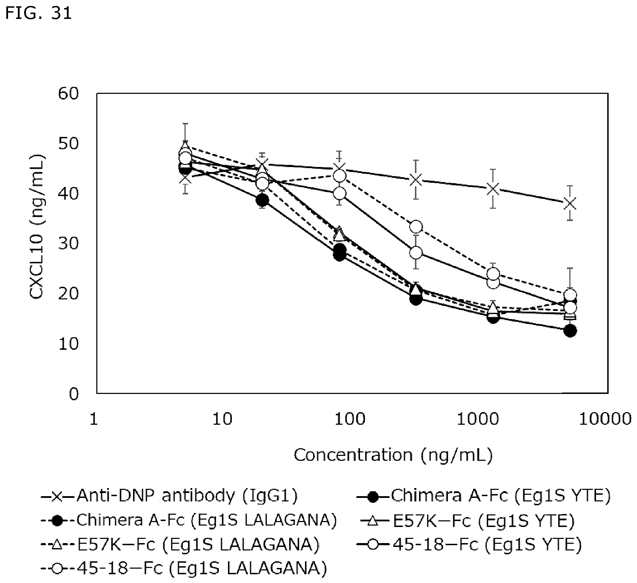

FIG. 31 shows evaluation results of the neutralizing activities of various DcR3 variants to soluble LIGHT. FIG. 31 shows the inhibitory activities of chimera A-Fc variants and the variants with low FasL binding activities including various mutated Fc sequences to LIGHT-dependent CXCL10 production from intestinal myofibroblasts. The vertical axis represents CXCL10 concentration (ng/mL), and the horizontal axis represents the concentration (ng/mL) of DcR3 variants added.

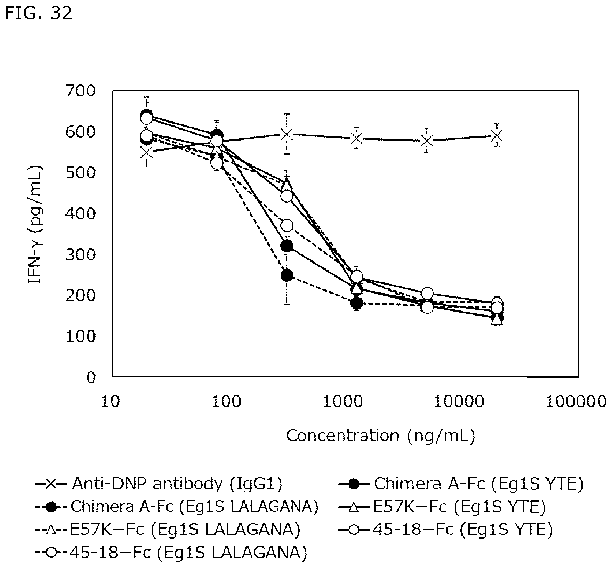

FIG. 32 shows evaluation results of the neutralizing activities of various DcR3 variants to soluble TL1A. FIG. 32 shows the inhibitory activities of chimera A-Fc variants and the variants with low FasL binding activities including various mutated Fc sequences to TL1A-dependent IFN-γ production from human T cells. The vertical axis represents IFN-γ concentration (pg/mL), and the horizontal axis represents the concentration (ng/mL) of DcR3 variants added.

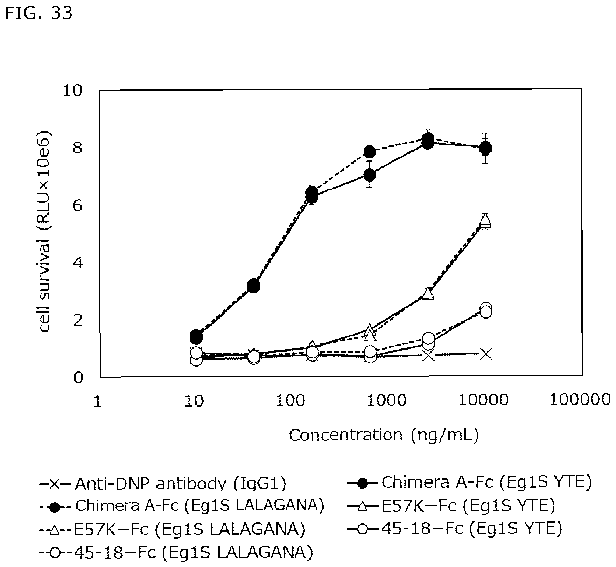

FIG. 33 shows evaluation results of the neutralizing activities of various DcR3 variants to soluble FasL. FIG. 33 shows the inhibitory activities of chimera A-Fc variants and the variants with low FasL binding activities including various mutated Fc sequences to cell death of Jurkat cells. The vertical axis represents cell survival (RLU × 10⁶) using ATP-dependent chemiluminescence as an index, and the horizontal axis represents the concentration (ng/mL) of various DcR3 variants added.

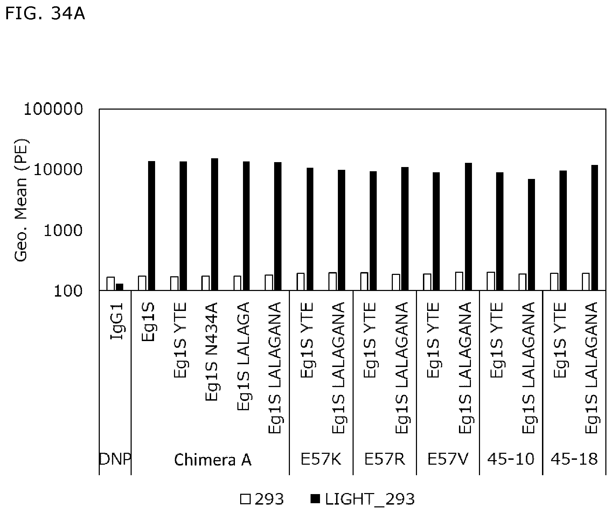

FIG. 34A shows evaluation results of the binding activities of various DcR3 variants to cells of a membrane-bound LIGHT forced expression cell line by flow cytometry. The vertical axis of the graph represents the geometric mean (Geo. Mean) of PE.

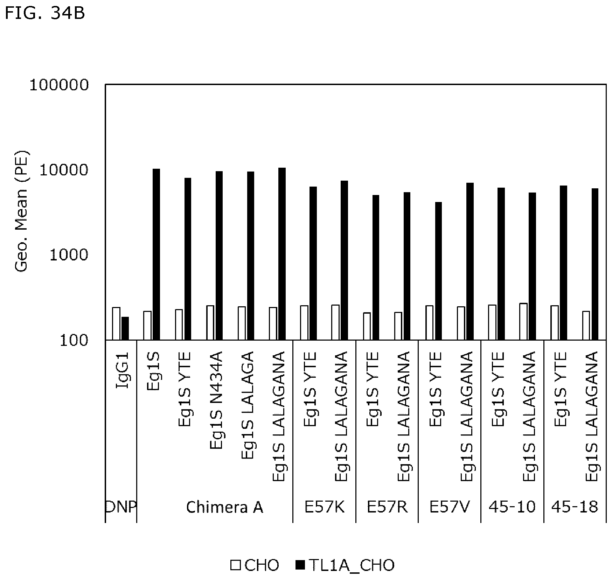

FIG. 34B shows evaluation results of the binding activities of various DcR3 variants to cells of a membrane-bound TL1A forced expression cell line by flow cytometry. The vertical axis of the graph represents the geometric mean (Geo. Mean) of PE.

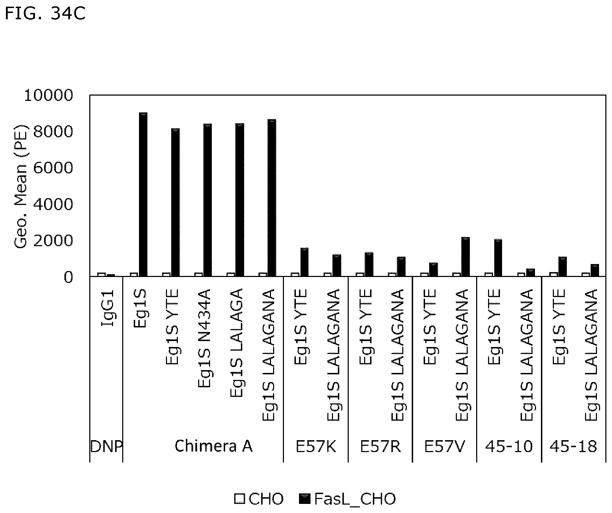

FIG. 34C shows evaluation results of the binding activities of various DcR3 variants to cells of a membrane-bound FasL forced expression cell line by flow cytometry. The vertical axis of the graph represents the geometric mean (Geo. Mean) of PE.

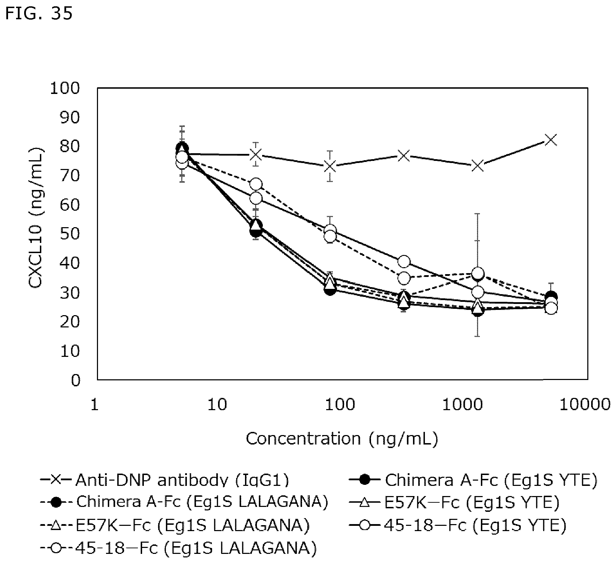

FIG. 35 shows evaluation results of the neutralizing activities of various DcR3 variants to membrane-bound LIGHT. FIG. 35 shows the inhibitory activities of chimera A-Fc variants and the variants with low FasL binding activities including various mutated Fc sequences to membrane-bound LIGHT-dependent CXCL10 production from intestinal myofibroblasts. The vertical axis represents CXCL10 concentration (ng/mL), and the horizontal axis represents the concentration (ng/mL) of DcR3 variants added.

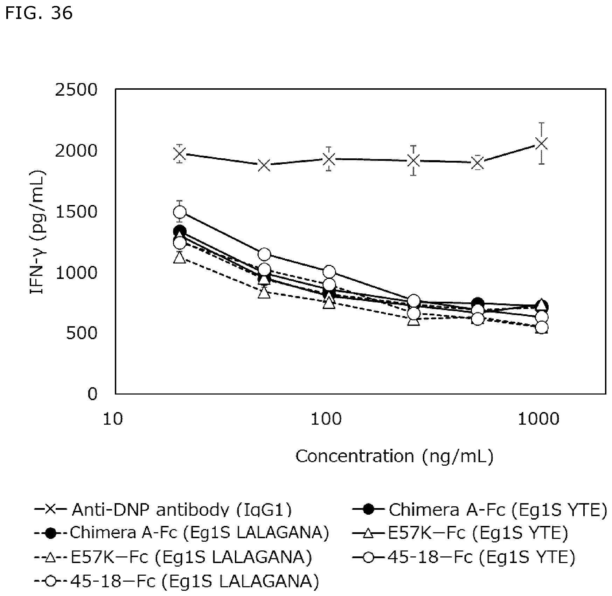

FIG. 36 shows evaluation results of the neutralizing activities of various DcR3 variants to membrane-bound TL1A. FIG. 36 shows the inhibitory activities of chimera A-Fc variants and the variants with low FasL binding activities including various mutated Fc sequences to membrane-bound TL1A-dependent IFN-γ production from human CD4-positive T cells. The vertical axis represents IFN-γ concentration (pg/mL), and the horizontal axis represents the concentration (ng/mL) of DcR3 variants added.

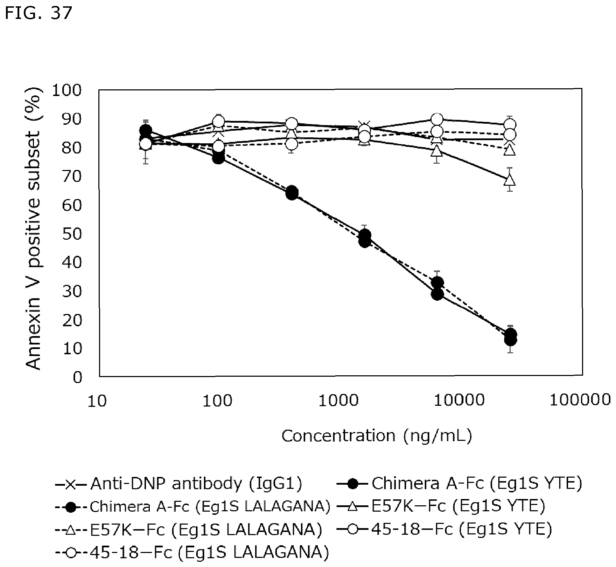

FIG. 37 shows evaluation results of the neutralizing activities of various DcR3 variants to membrane-bound FasL. FIG. 37 shows the inhibitory activities of chimera A-Fc variants and the variants with low FasL binding activities including various mutated Fc sequences to membrane-bound FasL-dependent cell death of Jurkat cells. The vertical axis of the graph represents the proportion (%) of an Annexin V-positive dead cell subset, and the horizontal axis represents the concentration (ng/mL) of various DcR3 variants added.

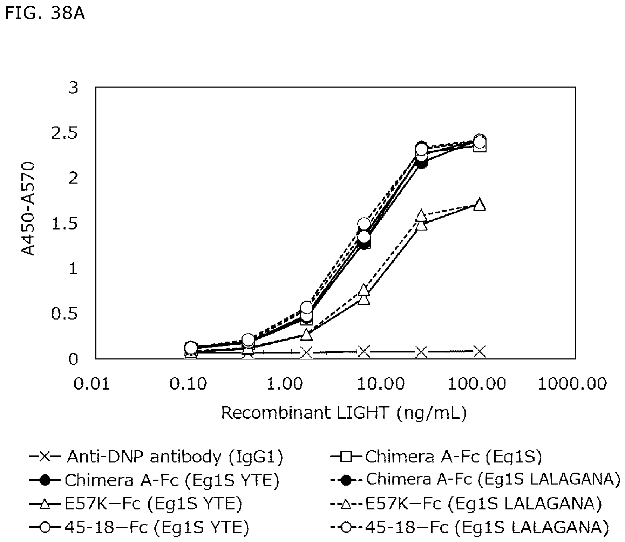

FIG. 38A shows measurement results of the binding activities of various DcR3 variants to recombinant human LIGHT by sandwich ELISA. The vertical axis represents the value obtained by subtracting the absorbance at 570 nm from the absorbance at 450 nm, and the horizontal axis represents the concentration (ng/mL) of recombinant human LIGHT added.

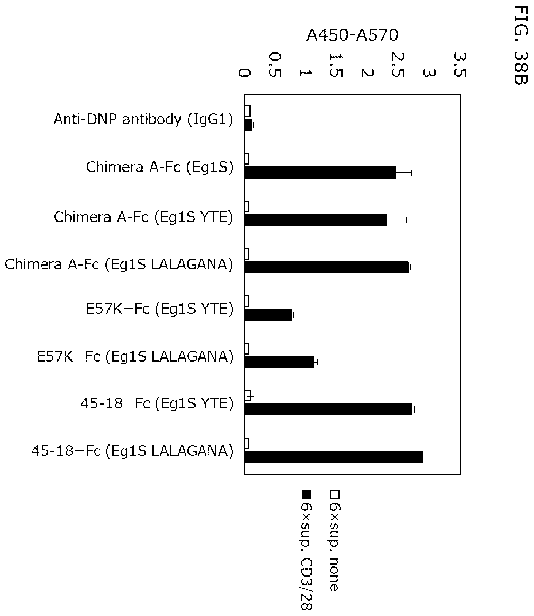

FIG. 38B shows results of the binding activities of various DcR3 variants to LIGHT derived from human primary cells, measured by sandwich ELISA to soluble LIGHT in culture supernatants of human T cells. The open bar represents results using culture supernatants when human T cells were cultured without stimulation, and the solid bar represents results using culture supernatants of human T cells stimulated with an anti-CD3 antibody and an anti-CD28 antibody. The vertical axis represents the value obtained by subtracting the absorbance at 570 nm from the absorbance at 450 nm.

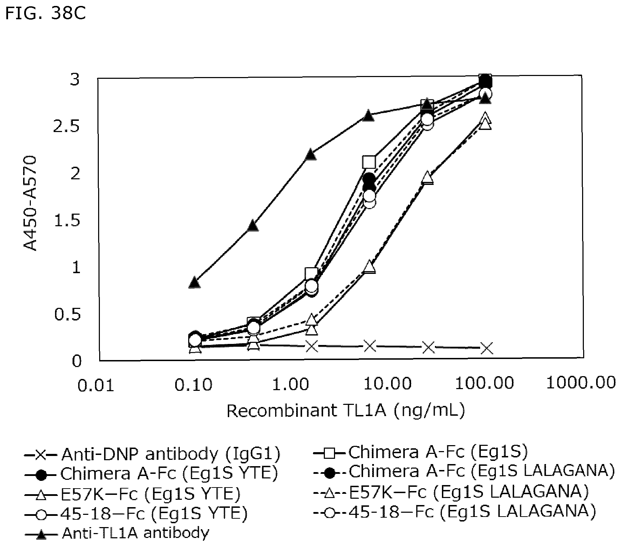

FIG. 38C shows results of the binding activities of various DcR3 variants to recombinant human TL1A, measured by sandwich ELISA. The vertical axis represents the value obtained by subtracting the absorbance at 570 nm from the absorbance at 450 nm, and the horizontal axis represents the concentration (ng/mL) of recombinant TL1A.

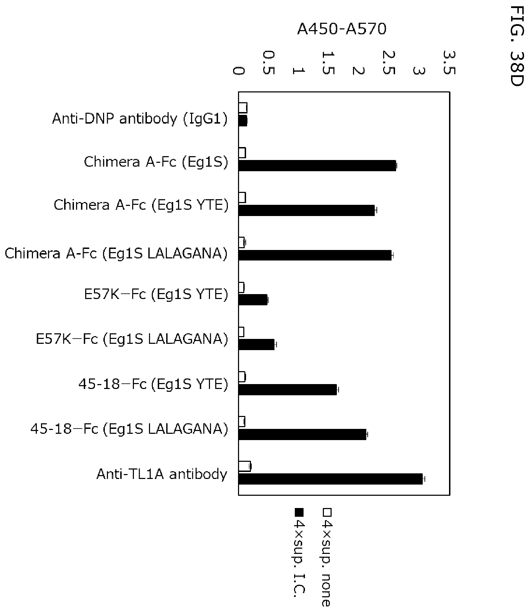

FIG. 38D shows results of the binding activities of various DcR3 variants to TL1A derived from human primary cells, measured by sandwich ELISA to soluble TL1A in culture supernatants of human PBMCs. The open bar represents results using culture supernatants when human PBMCs were cultured without stimulation, and the solid bar represents results using culture supernatants of human PBMCs stimulated with an immune complex. The vertical axis represents the value obtained by subtracting the absorbance at 570 nm from the absorbance at 450 nm.

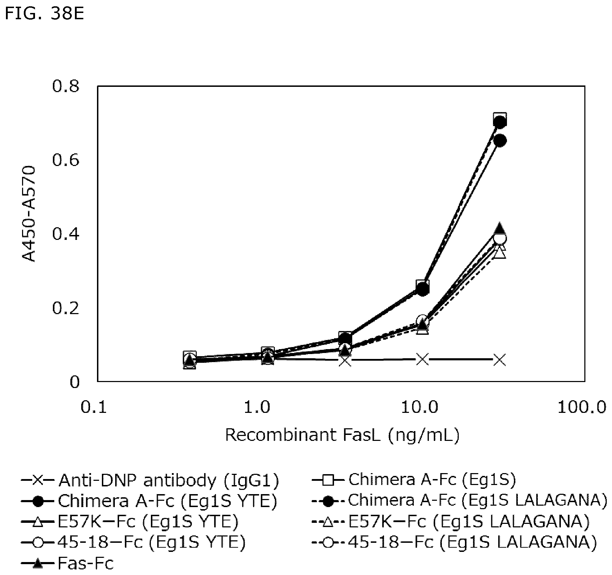

FIG. 38E shows results of the binding activities of various DcR3 variants to recombinant human FasL, measured by sandwich ELISA. The vertical axis represents the value obtained by subtracting the absorbance at 570 nm from the absorbance at 450 nm, and the horizontal axis represents the concentration (ng/mL) of human FasL.

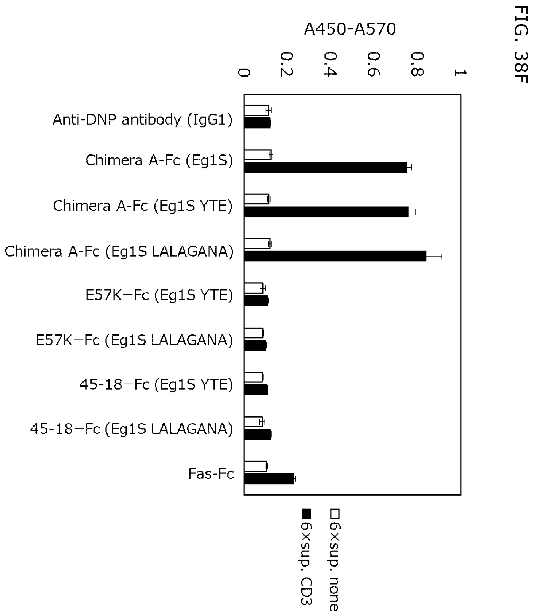

FIG. 38F shows results of the binding activities of various DcR3 variants to FasL derived from human primary cells, measured by sandwich ELISA to soluble FasL in culture supernatants of human T cells stimulated for AICD. The open bar represents results using culture supernatants of human T cells not stimulated for AICD, and the solid bar represents results using culture supernatants of human T cells stimulated for AICD. The vertical axis represents the value obtained by subtracting the absorbance at 570 nm from the absorbance at 450 nm.

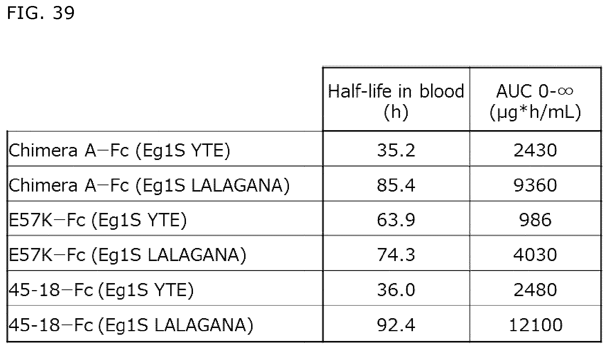

FIG. 39 shows the values of the half-life in blood (h) during the elimination phase and the area under the concentration-time curve to infinity AUC0-∞ (µg*h/mL) after i.v. administration of 10 mg/kg of various DcR3 variants to BALB/c mice.

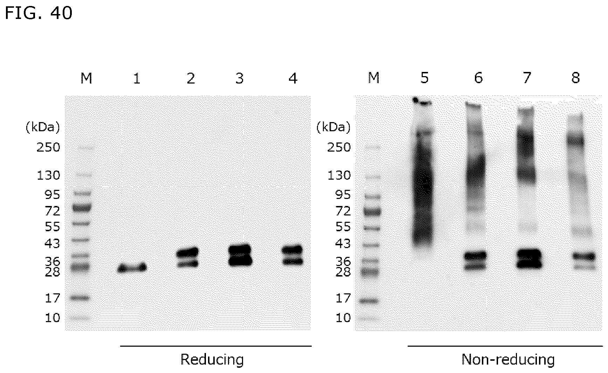

FIG. 40 shows results of immunoblotting detection of various DcR3 variants without Fc fusion that were transiently expressed on mammalian cells. A 30-fold concentrated solution of a culture supernatant of the cells expressing S195-His6 in lanes 1 and 5, a 6-fold diluted solution of a culture supernatant of the cells expressing chimera A-His6 in lanes 2 and 6, a 6-fold diluted solution of a culture supernatant of the cells expressing E57K-His6 in lanes 3 and 7, and a 6-fold diluted solution of a culture supernatant of the cells expessing 45-18-His6 in lanes 4 and 8 each were electrophoresed under non-reducing or reducing conditions, followed by detection by immunoblotting using an anti-6-His tag antibody.

### DETAILED DESCRIPTION OF THE INVENTION

Preferred embodiments for carrying out the present invention will be described below. The embodiments described below illustrate examples of representative embodiments of the present invention, and the scope of the present invention is not narrowly interpreted thereby.

It is possible to combine two or more embodiments of the embodiments described below, and such combinations are also included in the present invention.

The present invention relates to a DcR3 variant, which is a variant of wild-type DcR3. Specifically, the present invention relates to a DcR3 variant that has binding activity (or neutralizing activity) to a ligand of DcR3, and that exhibits improved *in vivo* kinetics as compared to wild-type DcR3 and/or lower aggregability as compared to wild-type DcR3 when produced using a mammalian cell, and to a DcR3 variant including a cysteine-rich domain obtained by introducing a mutation(s) into one or two or more amino acids in a cysteine-rich domain of wild-type DcR3.

### 1. Wild-Type DcR3

DcR3 is generally also referred to as decoy receptor 3, DCR3, tumor necrosis factor receptor superfamily member 6B (TNFRSF6B), TR6 or M68. DcR3 is a soluble decoy receptor that belongs to a TNF receptor superfamily and that has no transmembrane domain, and by binding to each of three ligands, i.e., LIGHT, TL1A or FasL, DcR3 competitively inhibits the binding of each ligand to a receptor, thus neutralizing the ligand. As a different function from neutralization of a ligand, it has been reported that DcR3 directly binds via a heparan sulfate-binding domain (HBD) to glycosaminoglycan (GAG) including heparan sulfate on the cell membrane of monocytes, macrophages or dendritic cells, thus initiating various immunosuppressive and immunostimulatory effects [Biochemical. Pharmacology, 2011, 81: p.838-847, J. Immunol., 2006, 176: p.173-180].

Naturally occurring DcR3 includes CRD1, CRD2, CRD3, CRD4 and HBD in this order from the N-terminal side. Naturally occurring DcR3 further includes a region existing between CRD1 and CRD2, a region existing between CRD2 and CRD3, a region existing between CRD3 and CRD4, and a region existing between CRD4 and HBD. A cysteine-rich region of naturally occurring DcR3 is a region from the N-terminus of CRD1 to the C-terminus of CRD4, and includes CRD1, CRD2, CRD3 and CRD4, and further includes a region existing between CRD1 and CRD2, a region existing between CRD2 and CRD3, and a region existing between CRD3 and CRD4. "Wild-type DcR3" means a molecule including a cysteine-rich region and HBD, wherein the cysteine-rich region and the HBD of the molecule are wild-type (in other word, the cysteine-rich region and the HBD of the molecule are the same as those of naturally occurring DcR3). Therefore, in addition to naturally occurring DcR3, mutants of naturally occurring DcR3, such as gene polymorphisms and isoforms, are also included in "wild-type DcR3" as long as they include the cysteine-rich region and the HBD of naturally occurring DcR3. Premature DcR3 and mature DcR3 are also included in "wild-type DcR3". "Premature DcR3" means DcR3 including a signal peptide, and "mature DcR3" means DcR3 from which a signal peptide has been cleaved. The signal peptide may be any of a sequence derived from naturally occurring DcR3, an artificial sequence, a sequence derived from an expression vector, and a sequence derived from another protein suitable for a host cell in which naturally occurring DcR3 is expressed. When a cleavage site is different according to a signal peptide to be used, a mature sequence having a different amino acid(s) at the N-terminus is also included in "wild-type DcR3". The CRD of naturally occurring DcR3 and the CRD of wild-type DcR3 are also referred to as "wild-type CRD", and the cysteine-rich region of naturally occurring DcR3 and the cysteine-rich region of wild-type DcR3 are also referred to as "wild-type cysteine-rich region".

In the present invention, the origin of wild-type DcR3 is not limited, and examples of wild-type DcR3 include DcR3 derived from various eukaryotes. Examples thereof include DcR3 derived from amphibians such as frogs, birds such as chickens, or mammals, for example, primates including humans, or artiodactyls such as pigs and cattle. When the DcR3 variant of the present invention is used for a human, it is preferable that DcR3 derived from a human is used as wild-type DcR3.

The cDNA sequence of DcR3 derived from a human is represented by SEQ ID NO: 1, and the corresponding mRNA sequence has been registered as Accession Number: NM_003823.3 in GenBank (US NCBI). The amino acid sequence of DcR3 derived from a human is represented by SEQ ID NO: 2, and has been registered as Accession Number: NP_003814.1 in GenBank (US NCBI). Premature human DcR3 includes a signal peptide at the N-terminus, followed by four CRDs (CRD1, CRD2, CRD3, CRD4), which are characteristic of a TNF receptor superfamily, and includes HBD, which is rich in basic amino acids, at the C-terminus. Mature human DcR3 is obtained by cleaving a signal peptide from premature one, and the amino acid sequence of the mature human DcR3 is represented by, for example, SEQ ID NO: 4, and the nucleotide sequence of a DNA encoding the amino acid sequence of the mature human DcR3 is represented by, for example, SEQ ID NO: 3. In the present invention, of the amino acid sequence (SEQ ID NO: 2) of human DcR3, a region at positions 30 to 70 from the N-terminus is defined as CRD1 (SEQ ID NO: 6), a region at positions 73 to 113 is defined as CRD2 (SEQ ID NO: 8), a region at positions 115 to 150 is defined as CRD3 (SEQ ID NO: 10), a region at positions 153 to 193 is defined as CRD4 (SEQ ID NO: 12), and a region at positions 196 to 300 is defined as HBD (SEQ ID NO: 48) (FIG. 2). The nucleotide sequences of DNAs encoding the amino acid sequences of CRD1, CRD2, CRD3, CRD4 and HBD of human DcR3 are represented by, for example, SEQ ID NOs: 5, 7, 9, 11 and 47, respectively. With respect to an amino acid sequence of CRD, although there are a plurality of definitions other than those mentioned above, any definition of an amino acid sequence of CRD can be used for the DcR3 variant of the present invention by using known information [UniProt O95407, GenBank NP_003814.1, Structure, 2011,19: p.162-171].

As mentioned above, examples of the ligand of DcR3 include LIGHT, TL1A and FasL, all of which belong to TNFSF.

LIGHT (lymphotoxin-like, exhibits inducible expression, and competes with herpes simplex virus (HSV) glycoprotein D (gD) for HVEM, a receptor expressed by T lymphocytes) is generally also referred to as tumor necrosis factor superfamily member 14 (TNFSF14), LTg, HVEM-L or CD258. The mRNA sequence of human LIGHT and the corresponding cDNA sequence have been registered as Accession Number: NM_003807.4, and the amino acid sequence thereof has been registered as Accession Number: NP_003798.2 in GenBank (US NCBI). Soluble LIGHT is generated by being expressed on the cell membrane as membrane-bound LIGHT, followed by shedding of the extracellular region by protease. A cleavage site in membrane-bound LIGHT is between amino acids at positions 82 and 83 of NP_003798.2. Both soluble and membrane-bound LIGHTs are functional.

Tumor necrosis factor (TNF)-like cytokine 1A (TL1A) is generally also referred to as TNF superfamily member 15 (TNFSF15), TL1, VEGI or VEGI-251. The mRNA sequence of human TL1A and the corresponding cDNA sequence have been registered as Accession Number: NM_005118.3, and the amino acid sequence thereof has been registered as Accession Number: NP_005109.2 in GenBank (US NCBI). Soluble TL1A is generated by being expressed on the cell membrane as membrane-bound TL1A, followed by shedding of the extracellular region by protease. A cleavage site in membrane-bound TL1A is between amino acids at positions 71 and 72 of NP_005109.2, and both soluble and membrane-bound TL1As are functional.

Fas ligand (FasL) is generally also referred to as FASLG, tumor necrosis factor superfamily member 6 (TNFSF6), CD178 or APT1LG1. The mRNA sequence of human FasL and the corresponding cDNA sequence have been registered as Accession Number: NM_000639.2, and the amino acid sequence thereof has been registered as Accession Number: NP_000630.1 in GenBank (US NCBI). Soluble FasL is generated by being expressed on the cell membrane as membrane-bound FasL, followed by shedding of the extracellular region by protease. A cleavage site in membrane-bound FasL is between amino acids at positions 81 and 82 or between amino acids at positions 129 and 130 of NP_000630.1. It has been reported that membrane-bound FasL is mainly a functional ligand *in vivo.*

In a gene encoding a protein of a eukaryote, polymorphisms or isoforms of the gene are often observed. With respect to a gene used in the present invention, a gene including a mutation introduced into a nucleotide sequence or amino acid sequence by such polymorphisms is also included in a gene encoding LIGHT, TL1A or FasL used in the present invention.

### 2. DcR3 Variant

The DcR3 variant of the present invention includes a chimeric cysteine-rich region.

### 2-1. Chimeric Cysteine-Rich Region

The chimeric cysteine-rich region of the present invention consists of an amino acid sequence obtained by introducing a mutation(s) into one or two or more amino acids in the amino acid sequence of the cysteine-rich region of wild-type DcR3. "A mutation(s) introduced into an amino acid sequence/nucleotide sequence" means substitution, deletion, insertion or addition of one or two or more amino acids/bases in the sequence. "Substitution, deletion, insertion or addition" also includes a combination of two or more mutations selected from substitution, deletion, insertion and addition. A mutation(s) is/are introduced into at least one or two or more CRDs selected from CRD1, CRD2, CRD3 and CRD4 of the cysteine-rich region of wild-type DcR3. A mutation(s) may be or may not be introduced into a region existing between CRD1 and CRD2, a region existing between CRD2 and CRD3, and a region existing between CRD3 and CRD4 of the cysteine-rich region of wild-type DcR3. When a mutation(s) is/are introduced into one or two or more regions selected from a region existing between CRD1 and CRD2, a region existing between CRD2 and CRD3, and a region existing between CRD3 and CRD4, the number of amino acids constituting each region after introduction of the mutation(s) is usually 1 to 10, preferably 1 to 7, more preferably 1 to 5, still more preferably 1 to 3, and yet more preferably 1 to 2. The amino acid sequence of each region after introduction of the mutation(s) is not particularly limited. The mutation(s) to be introduced into the cysteine-rich region of wild-type DcR3 may be a naturally occurring mutation or an artificial mutation. Examples of the chimeric cysteine-rich region in the present invention include a chimeric cysteine-rich region consisting of an amino acid sequence obtained by introducing into the amino acid sequence of the cysteine-rich region of wild-type DcR3, substitution, deletion, insertion or addition of one or two or more amino acids, and examples of such a chimeric cysteine-rich region can include first and second chimeric cysteine-rich regions mentioned below.

### 2-1-1. First Chimeric Cysteine-Rich Region

The first chimeric cysteine-rich region consists of an amino acid sequence obtained by introducing into the amino acid sequence of the cysteine-rich region of wild-type DcR3, substitution of at least a part(s) of CRDs of the wild-type DcR3 with (an)other peptide(s) or protein(s). In other words, the first chimeric cysteine-rich region includes both of an amino acid sequence derived from the cysteine-rich region of wild-type DcR3 and an amino acid sequence(s) derived from (an)other peptide(s) or protein(s).

The part(s) to be substituted with (an)other peptide(s) or protein(s), of the cysteine-rich region of wild-type DcR3, is/are preferably at least a part(s) of at least one CRD selected from CRD1, CRD2, CRD3 and CRD4. Therefore, the part(s) to be substituted with (an)other peptide(s) or protein(s), of the cysteine-rich region of wild-type DcR3, can be selected from a part(s) or a whole of CRD1, a part(s) or a whole of CRD2, a part(s) or a whole of CRD3, and a part(s) or a whole of CRD4.

The amino acid sequence of the first chimeric cysteine-rich region also includes an amino acid sequence obtained by introducing substitution of a part(s) other than the wild-type CRDs in addition to at least a part(s) of CRDs of wild-type DcR3 with (an)other peptide(s) or protein(s) into the amino acid sequence of the cysteine-rich region of wild-type DcR3. The part(s) other than CRDs to be substituted with (an)other peptide(s) or protein(s) can be selected from a region existing between CRD1 and CRD2, a region existing between CRD2 and CRD3, and a region existing between CRD3 and CRD4. The part(s) other than CRDs to be substituted with (an)other peptide(s) or protein(s) may be one part or two or more parts. For example, an amino acid sequence obtained by introducing into the region existing between CRD1 and CRD2, the region existing between CRD2 and CRD3, or the region existing between CRD3 and CRD4, substitution of the amino acid sequence with another peptide or protein is also included in the amino acid sequence of the first chimeric cysteine-rich region.

The part(s) to be substituted with (an)other peptide(s) or protein(s), of the cysteine-rich region of wild-type DcR3, may be one part or two or more parts. When at least a part(s) of CRDs of the cysteine-rich region of wild-type DcR3 is substituted, the part(s) to be substituted may be a part(s) or a whole of one CRD or a part(s) or a whole of a plurality of CRDs, but it is more preferable that a part(s) or a whole of CRD2 of wild-type DcR3, which is associated with the binding to LIGHT, TL1A and FasL, and/or a part(s) or a whole of CRD3 is/are maintained, and a part(s) or a whole of the other CRDs is substituted with (an)other peptide(s) or protein(s).

(An)other peptide(s) or protein(s) used for substitution may be a natural or artificial peptide(s) or protein(s), but examples thereof include at least a part of CRDs derived from a protein other than DcR3. In a preferred embodiment, (an)other peptide(s) or protein(s) used for substitution is/are at least a part(s) of CRDs of a TNF receptor superfamily (TNFRSF) molecule other than DcR3. In other words, in a preferred embodiment, the first chimeric cysteine-rich region consists of an amino acid sequence obtained by introducing into the amino acid sequence of the cysteine-rich region of wild-type DcR3, substitution of at least a part(s) of the cysteine-rich domain of the wild-type DcR3 with at least a part(s) of a cysteine-rich domain of a TNFRSF molecule other than DcR3. At least a part(s) of CRDs of a TNFRSF molecule can be selected from a part(s) or a whole of CRD1, a part(s) or a whole of CRD2, a part(s) or a whole of CRD3, and a part(s) or a whole of CRD4. Therefore, the first chimeric cysteine-rich region includes one or more substitution selected from:
substitution of a part of CRD1 of wild-type DcR3 with a part of CRD1 of a TNFRSF molecule, wherein the part of the CRD1 of the TNFRSF molecule corresponds to the part of the CRD1 of the wild-type DcR3;
substitution of the whole of CRD1 of wild-type DcR3 with the whole of CRD1 of a TNFRSF molecule;
substitution of a part of CRD2 of wild-type DcR3 with a part of CRD2 of a TNFRSF molecule, wherein the part of the CRD2 of the TNFRSF molecule corresponds to the part of the CRD2 of the wild-type DcR3;
substitution of the whole of CRD2 of wild-type DcR3 with the whole of CRD2 of a TNFRSF molecule;
substitution of a part of CRD3 of wild-type DcR3 with a part of CRD3 of a TNFRSF molecule, wherein the part of the CRD3 of the TNFRSF molecule corresponds to the part of the CRD3 of the wild-type DcR3;
substitution of the whole of CRD3 of wild-type DcR3 with the whole of CRD3 of a TNFRSF molecule;
substitution of a part of CRD4 of wild-type DcR3 with a part of CRD4 of a TNFRSF molecule, wherein the part of the CRD4 of the TNFRSF molecule corresponds to the part of the CRD4 of the wild-type DcR3; and
substitution of the whole of CRD4 of wild-type DcR3 with the whole of CRD4 of a TNFRSF molecule.

Examples of the first chimeric cysteine-rich region include a chimeric cysteine-rich region including substitution of CRD1 of wild-type DcR3 with CRD1 of a TNFRSF molecule (in the chimeric cysteine-rich region, preferably the other CRDs of the wild-type DcR3 are maintained), a chimeric cysteine-rich region including substitution of CRD4 of wild-type DcR3 with CRD4 of a TNFRSF molecule (in the chimeric cysteine-rich region, preferably the other CRDs of the wild-type DcR3 are maintained), or a chimeric cysteine-rich region including both of substitution of CRD1 of wild-type DcR3 with CRD1 of a TNFRSF molecule, and substitution of CRD4 of wild-type DcR3 with CRD4 of a TNFRSF molecule (in the chimeric cysteine-rich region, preferably the other CRDs of the wild-type DcR3 are maintained) or the like. Examples of the first chimeric cysteine-rich region also include a chimeric cysteine-rich region further including, in addition to one or more of the abovementioned substitutions, substitution of a part of CRD2 of wild-type DcR3 with a part of CRD2 of a TNFRSF molecule, wherein the part of the CRD2 of the TNFRSF molecule corresponds to the part of the CRD2 of the wild-type DcR3, and/or substitution of a part of CRD3 of wild-type DcR3 with a part of CRD3 of a TNFRSF molecule , wherein the part of the CRD3 of the TNFRSF molecule corresponds to the part of the CRD3 of the wild-type DcR3.

Twenty-nine (29) receptors belong to human TNFRSF, and all receptors have CRDs in the extracellular domain of the N-terminus, and it is typical that six Cys residues form three disulfide bonds per CRD, and each receptor has one to four CRDs [Trends Biochem Sci, 2002. 27: p-19-26.].

Examples of the human TNFRSF include DcR1 (TNFRSF10C, TRAIL-R3, LIT, TRID, CD263), DcR2 (TNFRSF10D, TRAIL-R4, TRUNDD, CD264), TNFR type I (TNFRSF1A, TNF-R, CD120a, TNFAR, TNF-R55, TNFR60), TNFR type II (TNFRSF1B, TNFBR, CD120b, TNFR80, p75, TNF-R75), LTBR (lymphotoxin beta receptor, TNFRSF3, TNFR III, TNFCR, TNFR-RP, TNFR2-RP), OX-40 (TNFRSF4, ACT35, TXGPLL, CD134), CD40 (TNFRSF5, Bp50, p50), Fas (Fas cell surface death receptor, TNFRSF6, CD95, APO-1, APT1, FAS1), CD27 (TNFRSF7, S152, Tp55), CD30 (TNFRSF8, Ki-1), 4-1BB (TNFRSF9, CD137, ILA), DR4 (TNFRSF10A, Apo2, TRAILR-1, CD261), DR5 (TNFRSF10B, TRAIL-R2, KILLER, TRICK2A, TRICKB, CD262), RANK (TNFRSF11A, CD265, FEO), FN14 (TNFRSF12A, TweakR, CD266), TACI (TNFRSF13B, CD267, IGAD2), BAFFR (TNFRSF13C, CD268), HVEM (TNFRSF14, ATAR, TR2, LIGHTR, HVEA, CD270), NGFR (nerve growth factor receptor, TNFRSF16, p75NTR, CD271), BCMA (TNFRSF17, BCM, CD269, TNFRSF13A), GITR (TNFRSF18, AITR, CD357), TROY (TNFRSF19, TAJ-alpha, TAJ, TRADE), RELT (TNFRSF19L), DR6 (death receptor 6, TNFRSF21, CD358), DR3 (death receptor 3, TNFRSF25, TRAMP, WSL-1, LARD, WSL-LR, DDR3, TR3, APO-3), EDAR (ectodysplasin A receptor, ED3, DL, ED5, EDA3, Edar, ED1R, EDA1R), EDAR2R (ectodysplasin A2 receptor, XEDAR, EDAA2R, EDA-A2R, TNFRSF27) or osteoprotegerin (OPG, TNFRSF11B, TR1, OCIF) or the like.

In the first chimeric cysteine-rich region, (an)other peptide(s) or protein(s) used for substitution of at least a part(s) of wild-type DcR3 is/are not limited, but, of TNFRSF, OPG is particularly preferable.

The origin of OPG is not limited, and examples of OPG include OPG derived from various eukaryotes. Examples thereof include OPG derived from amphibians such as frogs, birds such as chickens, or mammals, for example, primates including humans, artiodactyls such as pigs and cattle, or rodents including mice, or the like.

The cDNA sequence of OPG derived from a human is represented by SEQ ID NO: 13, and the corresponding mRNA sequence has been registered as Accession Number: NM_002546.3 in GenBank (US NCBI). The amino acid sequence of OPG derived from a human is represented by SEQ ID NO: 14, and has been registered as Accession Number: NP_002537.3 in GenBank (US NCBI).

Premature human OPG (SEQ ID NO: 14) has a signal peptide at the N-terminus. Mature human OPG is obtained by cleaving a signal peptide from premature one. The amino acid sequence of the mature human OPG is represented by, for example, SEQ ID NO: 16, and the nucleotide sequence of a DNA encoding the amino acid sequence of the mature human OPG is represented by, for example, SEQ ID NO: 15. In the present invention, of the amino acid sequence (SEQ ID NO: 14) of premature human OPG, a region at positions 22 to 62 from the N-terminus is defined as CRD1 (SEQ ID NO: 18), a region at positions 65 to 105 is defined as CRD2 (SEQ ID NO: 20), a region at positions 107 to 142 is defined as CRD3 (SEQ ID NO: 22), and a region at positions 145 to 185 is defined as CRD4 (SEQ ID NO: 24). The nucleotide sequences of DNAs encoding the amino acid sequences of CRD1, CRD2, CRD3 and CRD4 of human OPG are represented by, for example, SEQ ID NOs: 17, 19, 21 and 23, respectively. With respect to an amino acid sequence of CRD, although there are a plurality of definitions other than those mentioned above, any definition of an amino acid sequence of CRD can be used for the DcR3 variant of the present invention by using known information [UniProt O00300, GenBank NP_002537.3].

In a gene encoding a protein of a eukaryote, polymorphisms or isoforms of the gene are often observed. With respect to a gene used in the present invention, a gene including a mutation introduced into a nucleotide sequence or amino acid sequence by such polymorphisms is also included in a gene encoding OPG used in the present invention.

OPG binds to RANKL, and neutralizes the activity thereof, thus suppressing bone destruction by osteoclasts [J. Immunol., 2012, 189: p.245-252]. OPG also binds to TRAIL, and neutralizes the activity thereof, thus inhibiting apoptosis mediated by TRAIL [Am. J. Cancer. Res., 2012, 2: p.45-64]. Since neutralization of any ligand may cause undesired activity or the like, it is desirable that the DcR3 variant of the present invention has no neutralizing activity to any of RANKL and TRAIL.

In a preferred embodiment, the first chimeric cysteine-rich region includes or consists of the following amino acid sequence (a), (b), (c) or (d):
(a) an amino acid sequence obtained by introducing into the amino acid sequence of the cysteine-rich region of wild-type DcR3, substitution of CRD1 of the wild-type DcR3 with CRD1 of OPG (in the amino acid sequence, preferably the other CRDs of the wild-type DcR3 are maintained),
(b) an amino acid sequence obtained by introducing into the amino acid sequence of the cysteine-rich region of wild-type DcR3, substitution of CRD4 of the wild-type DcR3 with CRD4 of OPG (in the amino acid sequence, preferably the other CRDs of the wild-type DcR3 are maintained),
(c) an amino acid sequence obtained by introducing into the amino acid sequence of the cysteine-rich region of wild-type DcR3, substitution of CRD1 of the wild-type DcR3 with CRD1 of OPG and substitution of CRD4 of the wild-type DcR3 with CRD4 of OPG (in the amino acid sequence, preferably the other CRDs of the wild-type DcR3 are maintained), and
(d) an amino acid sequence obtained by introducing into the amino acid sequence (a), (b) or (c), substitution of a part of CRD2 of the wild-type DcR3 with a corresponding part of CRD2 of OPG and/or substitution of a part of CRD3 of the wild-type DcR3 with a corresponding part of CRD3 of OPG (in the amino acid sequence, preferably the other CRDs of the wild-type DcR3 are maintained).

Examples of the amino acid sequence (d) include an amino acid sequence obtained by introducing into the amino acid sequence (a), (b) or (c), substitution of a part at positions 103 to 123 from the N-terminus with a corresponding part of an amino acid sequence of CRD of OPG. This amino acid sequence includes substitution of an amino acid sequence including a part at positions 18 to 36 of CRD3 of wild-type DcR3 and two amino acid residues at the C-terminal side thereof with a part of OPG, wherein the part of OPG corresponds to the amino acid sequence to be substituted.

Specific examples of the amino acid sequence (a) include an amino acid sequence consisting of amino acids at positions 1 to 164 from the N-terminus of the amino acid sequence set forth in SEQ ID NO: 26 or 50; specific examples of the amino acid sequence (b) include an amino acid sequence consisting of amino acids at positions 1 to 164 from the N-terminus of the amino acid sequence set forth in SEQ ID NO: 28 or 52; specific examples of the amino acid sequence (c) include an amino acid sequence consisting of amino acids at positions 1 to 164 from the N-terminus of the amino acid sequence set forth in SEQ ID NO: 30 or 54; and specific examples of the amino acid sequence (d) include an amino acid sequence consisting of amino acids at positions 1 to 164 from the N-terminus of the amino acid sequence set forth in SEQ ID NO: 32 or 56.

SEQ ID NO: 26 represents an amino acid sequence of chimera B-HBD (a DcR3 variant obtained by introducing into wild-type DcR3 (SEQ ID NO: 4), substitution of CRD1 with CRD1 of OPG); SEQ ID NO: 28 represents an amino acid sequence of chimera C-HBD (a DcR3 variant obtained by introducing into wild-type DcR3 (SEQ ID NO: 4), substitution of CRD4 with CRD4 of OPG); SEQ ID NO: 30 represents an amino acid sequence of chimera A-HBD (a DcR3 variant obtained by introducing into wild-type DcR3 (SEQ ID NO: 4), substitution of CDR1 and CDR4 with CDR1 and CDR4 of OPG, respectively); SEQ ID NO: 32 represents an amino acid sequence of 103-123OPG-HBD (a DcR3 variant obtained by introducing into chimera A-HBD, substitution of an amino acid sequence including a part at positions 18 to 36 of CRD3 and two amino acids at the C-terminal side thereof with human OPG); SEQ ID NO: 50 represents an amino acid sequence of chimera B (a DcR3 variant obtained by introducing into wild-type DcR3 (SEQ ID NO: 4), substitution of CRD1 with CRD1 of OPG and deletion of a heparan sulfate-binding domain); SEQ ID NO: 52 represents an amino acid sequence of chimera C (a DcR3 variant obtained by introducing into wild-type DcR3 (SEQ ID NO: 4), substitution of CRD4 with CRD4 of OPG and deletion of a heparan sulfate-binding domain); SEQ ID NO: 54 represents an amino acid sequence of chimera A (a DcR3 variant obtained by introducing into wild-type DcR3 (SEQ ID NO: 4), substitution of CDR1 and CDR4 with CDR1 and CDR4 of OPG, respectively, and deletion of a heparan sulfate-binding domain); and SEQ ID NO: 56 represents an amino acid sequence of 103-123OPG (a DcR3 variant obtained by introducing into chimera A, substitution of an amino acid sequence including a part at positions 18 to 36 of CRD3 and two amino acids at the C-terminal side thereof with human OPG).

In a preferred embodiment, examples of the DcR3 variant including the first chimeric cysteine-rich region include a DcR3 variant having binding activity to at least one or more of LIGHT, TL1A and FasL, a DcR3 variant having binding activity to all of LIGHT, TL1A and FasL, a DcR3 variant having no binding activity to FasL and having binding activity to one of LIGHT and TL1A, or a DcR3 variant having no binding activity to FasL and having binding activity to both of LIGHT and TL1A.

In the present invention, "having binding activity to a ligand" is used as a meaning including the case where the binding activity of the DcR3 variant including the first chimeric cysteine-rich region to the ligand is comparable and is not significantly lowered compared to the binding activity of wild-type DcR3 to the ligand, and the case where the binding activity of the DcR3 variant to the ligand is significantly enhanced compared to the binding activity of wild-type DcR3 to the ligand. For example, in the case of measurement by the surface plasmon resonance method (SPR method), when the dissociation constant (K_{D}) value of a DcR3 variant is less than 3-fold compared to that of wild-type DcR3, it can be judged that the DcR3 variant has binding activity to the ligand.

In the present invention, the expression of the DcR3 variant "having no binding activity to a ligand" is used as a meaning including the case where the binding activity of the DcR3 variant including the first chimeric cysteine-rich region to the ligand is not detected, and the case where the binding activity of the DcR3 variant to the ligand is significantly lowered compared to the binding activity of wild-type DcR3 to the ligand. For example, in the case of measurement by the SPR method, when the KD value of a DcR3 variant is more than 3-fold compared to that of wild-type DcR3, or the Rmax of a DcR3 variant is less than 5, it is judged that the binding activity to the ligand is significantly lowered, and it can be defined that the DcR3 variant has no binding activity to the ligand.

In a particularly preferred embodiment, examples of the DcR3 variant including the first chimeric cysteine-rich region include a DcR3 variant with lowered FasL binding activity. "Variant with lowered FasL binding activity" means a DcR variant including a chimeric cysteine-rich region, having no binding activity to FasL and having binding activity to one or more of LIGHT and TL1A, or a DcR3 variant including a chimeric cysteine-rich region, having no binding activity to FasL and having binding activity to both of LIGHT and TL1A.

In a preferred embodiment, the DcR3 variant of the present invention is a DcR3 variant having neutralizing activity to at least one or more of LIGHT, TL1A and FasL, a DcR3 variant having neutralizing activity to all of LIGHT, TL1A and FasL, a DcR3 variant having no neutralizing activity to FasL and having neutralizing activity to one of LIGHT and TL1A, or a DcR3 variant having no neutralizing activity to FasL and having neutralizing activity to both of LIGHT and TL1A.

In the present invention, the expression of "neutralizing activity to a ligand" includes the case where the binding of the ligand to the DcR3 variant inhibits the binding of the ligand to a receptor on the cell membrane surface, and the case where inhibition of the binding of the ligand to a receptor on the cell membrane surface inhibits a cell function initiated by the binding of the ligand to the receptor on the cell membrane surface, namely, biological activity of the ligand (e.g., biological activity such as cytokine production, proliferation enhancement and apoptosis induction to a cell).

In the present invention, the expression of the DcR3 variant "having neutralizing activity" is used as a meaning including the case where the neutralizing activity of the DcR3 variant to the ligand has no significant difference compared to the neutralizing activity of wild-type DcR3 to the ligand, and the case where the neutralizing activity of the DcR3 variant to the ligand is significantly enhanced compared to the neutralizing activity of wild-type DcR3 to the ligand.

In the present invention, the expression of the DcR3 variant "having no neutralizing activity" is used as a meaning including the case where the neutralizing activity of the DcR3 variant to the ligand is significantly lowered compared to the neutralizing activity of wild-type DcR3 to the ligand.

In a particularly preferred embodiment, the DcR3 variant in the present invention is a DcR3 variant in which the binding activity to FasL is lowered. "Variant with lowered FasL binding activity" means a DcR3 variant having no neutralizing activity to FasL and having neutralizing activity to one or more of LIGHT and TL1A, or a DcR3 variant having no neutralizing activity to FasL and having neutralizing activity to both of LIGHT and TL1A.

### 2-1-2. Second Chimeric Cysteine-Rich Region

The second chimeric cysteine-rich region is a region obtained by introducing into the amino acid sequence of the first chimeric cysteine-rich region, deletion, substitution, insertion or addition of 1 to 30 amino acids.

Examples of a method for obtaining a polypeptide including an amino acid sequence obtained by introducing into the amino acid sequence of the first chimeric cysteine-rich region, deletion, substitution, insertion or addition of one or more amino acids include a site-specific mutation introduce method [Molecular Cloning, A Laboratory Manual, Second Edition, Cold Spring Harbor Laboratory Press (1989), Current Protocols in Molecular Biology, John Wiley & Sons (1987-1997), Nucleic Acids Research, 10, 6487 (1982), Proc. Natl. Acad. Sci. USA., 79, 6409, (1982), Gene, 34, 315 (1985), Proc. Natl. Acad. Sci. USA., 82, 488 (1985)].

A mutation (modification) to be added to the first chimeric cysteine-rich region may be a natural mutation or an artificial substitution, deletion, insertion or addition of an amino acid. Examples of the amino acid sequence of the second chimeric cysteine-rich region include an amino acid sequence obtained by introducing into the amino acid sequence of the first chimeric cysteine-rich region, substitution, deletion, insertion or addition of 1 or 2 or more, preferably 1 to 30, more preferably 1 to 10, still more preferably 1 to 5, and yet more preferably 1 to 3 amino acids, or an amino acid sequence having 80% or more, preferably 85% or more, more preferably 90% or more, for example, 93% or more, 95% or more, 97% or more, 98% or more or 99% or more identity to the amino acid sequence of the first chimeric cysteine-rich region. With respect to a method for describing an amino acid substitute, for example, when an amino acid substitution is obtained by introducing substitution of Asn with Ser at position 131 from the N-terminus into an amino acid sequence, the substitute can be expressed as N131S.

In a preferred embodiment, the second chimeric cysteine-rich region includes or consists of the following amino acid sequence (e): (e) an amino acid sequence obtained by introducing into the amino acid sequence (a), (b), (c) or (d), deletion, substitution, insertion or addition of 1 to 30 amino acids.

Examples of a mutation (modification) to be added to the first chimeric cysteine-rich region include addition or deletion of a glycosylation site. By adding or deleting a glycosylation site to the first chimeric cysteine-rich region, it is possible to control the biological activity of the DcR3 variant of the present invention or properties thereof, *in vivo* kinetics such as half-life in blood, or physical or chemical properties such as stability of a protein.

Glycosylation generally means that a glycan is N-glycoside-linked to an asparagine residue of a peptide or a protein, and/or that a glycan is O-glycoside-linked to a serine or threonine residue. Examples of the O-linked glycan added to a DcR3 variant include core 1, core 2 or the like, or examples of the N-linked glycan include high-mannose-type, hybrid-type or complex glycan, but a complex glycan is preferable.

Examples of a mutation (modification) to be added to the first chimeric cysteine-rich region include, for example, substitution of at least one or more amino acids of the amino acid sequence of the first chimeric cysteine-rich region with an amino acid(s) to which a glycan(s) can be added via an N-glycoside linkage or O-glycoside linkage, and followed by addition of a glycan(s). Particularly, addition of an N-glycoside-linked glycan is preferable.

The present invention also includes, for example, substitution of at least one or more, preferably two or more amino acids associated with an N-glycoside linkage, of the amino acid sequence of the chimeric cysteine-rich region, with (an)other amino acid(s), followed by removal of a glycan(s). Generally, when a peptide or a protein is expressed using a yeast, an insect cell or a mammalian cell, an N-glycoside linkage of a glycan occurs by recognizing the sequence of Asn-X-Thr/Ser (wherein X represents any amino acid residue other than Pro). For example, by substituting Asn, Ser or Thr of the Asn-X-Thr/Ser sequence existing in the DcR3 variant with another amino acid, it is possible to remove an N-glycoside-linked glycan.

As the second chimeric cysteine-rich region, in order to decrease aggregates of the DcR3 variant of the present invention, particularly preferred is a chimeric cysteine-rich region having an N-glycoside-linked glycan to Asn at position 157 from the N-terminus of the amino acid sequence of the first chimeric cysteine-rich region (e.g., an amino acid sequence consisting of amino acids at positions 1 to 164 from the N-terminus of SEQ ID NO: 30).

In a preferred embodiment, the amino acid sequence of the second chimeric cysteine-rich region lacking a glycosylation site (one embodiment of the amino acid sequence (e)) includes substitution selected from:
(f) substitution of Asn at positions 131 and 144 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with other amino acids;
(g) substitution of Asn at positions 131, 144 and 157 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with other amino acids;
(h) substitution of Thr at position 133 and Ser at position 146 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with other amino acids; and
(i) substitution of Thr at position 133, Ser at position 146 and Thr at position 159 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with other amino acids.

In a further preferred embodiment, the amino acid sequence lacking a glycosylation site (one embodiment of the amino acid sequence (e)) includes substitution selected from: (f') substitution of Asn at positions 131 and 144 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with Ser;
(g') substitution of Asn at positions 131, 144 and 157 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with Ser;
(h') substitution of Thr at position 133 and Ser at position 146 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with Ala; and
(i') substitution of Thr at position 133, Ser at position 146 and Thr at position 159 from the N-terminus of the amino acid sequence (b), (c) or (d) (e.g., an amino acid sequence consisting of amino acids at positions 1 to 164 from the N-terminus of the amino acid sequence set forth in SEQ ID NO: 28, 30, 32, 52, 54 or 56) with Ala.

Examples of an amino acid sequence including the substitution (f') include an amino acid sequence obtained by introducing into the amino acid sequence consisting of amino acids at positions 1 to 164 from the N-terminus of the amino acid sequence set forth in SEQ ID NO: 30 or SEQ ID NO: 54, substitution of Asn at positions 131 and 144 from the N-terminus with Ser (N131S/N144S) (an amino acid sequence consisting of amino acids at positions 1 to 164 from the N-terminus of the amino acid sequence set forth in SEQ ID NO: 34 or SEQ ID NO: 58) and the like.

SEQ ID NO: 34 represents an amino acid sequence of N131S/N144S-HBD (a DcR3 variant obtained by introducing into chimera A-HBD, substitution of Asn at positions 131 and 144 from the N-terminus with Ser), and SEQ ID NO: 58 represents an amino acid sequence of N131S/N144S (a DcR3 variant obtained by introducing into chimera A, substitution of Asn at positions 131 and 144 from the N-terminus with Ser).

Examples of an amino acid sequence including the substitution (h') include an amino acid sequence obtained by introducing into the amino acid sequence consisting of amino acids at positions 1 to 164 from the N-terminus of the amino acid sequence set forth in SEQ ID NO: 30 or SEQ ID NO: 54, substitution of Thr at position 133 and Ser at position 146 from the N-terminus with Ala (T133A/S146A) (an amino acid sequence consisting of amino acids at positions 1 to 164 from the N-terminus of the amino acid sequence set forth in SEQ ID NO: 36 or SEQ ID NO: 60) and the like.

SEQ ID NO: 36 represents an amino acid sequence of T133A/S146A-HBD (a DcR3 variant obtained by introducing into chimera A-HBD, substitution of Thr at position 133 and Ser at position 146 from the N-terminus with Ala), and SEQ ID NO: 60 represents an amino acid sequence of T133A/S146A (a DcR3 variant obtained by introducing into chimera A, substitution of Thr at position 133 and Ser at position 146 from the N-terminus with Ala).

Examples of an amino acid sequence including the substitution (g') include an amino acid sequence obtained by introducing into the amino acid sequence consisting of amino acids at positions 1 to 164 from the N-terminus of the amino acid sequence set forth in SEQ ID NO: 30 or SEQ ID NO: 54, substitution of Asn at positions 131, 144 and 157 from the N-terminus with Ser (N131S/N144S/N157S) (an amino acid sequence consisting of amino acids at positions 1 to 164 from the N-terminus of the amino acid sequence set forth in SEQ ID NO: 38 or SEQ ID NO: 62) and the like.

SEQ ID NO: 38 represents an amino acid sequence of N131S/N144S/N157S-HBD (a DcR3 variant obtained by introducing into chimera A-HBD, substitution of Asn at positions 131, 144 and 157 from the N-terminus with Ser), and SEQ ID NO: 62 represents an amino acid sequence of N131S/N144S/N157S (a DcR3 variant obtained by introducing into chimera A, substitution of Asn at positions 131, 144 and 157 from the N-terminus with Ser).

Examples of an amino acid sequence including the substitution (i') include an amino acid sequence obtained by introducing into the amino acid sequence consisting of amino acids at positions 1 to 164 from the N-terminus of the amino acid sequence set forth in SEQ ID NO: 30 or SEQ ID NO: 54, substitution of Thr at position 133, Ser at position 146 and Thr at position 159 from the N-terminus with Ala (T133A/S146A/T159A) (an amino acid sequence consisting of amino acids at positions 1 to 164 from the N-terminus of the amino acid sequence set forth in SEQ ID NO: 40 or SEQ ID NO: 64) and the like.

SEQ ID NO: 40 represents an amino acid sequence of T133A/S146A/T159A-HBD (a DcR3 variant obtained by introducing into chimera A-HBD, substitution of Thr at position 133, Ser at position 146 and Thr at position 159 from the N-terminus with Ala), and SEQ ID NO: 64 represents an amino acid sequence of T133A/S146A/T159A (a DcR3 variant obtained by introducing into chimera A, substitution of Thr at position 133, Ser at position 146 and Thr at position 159 from the N-terminus with Ala).

With respect to a mutation (modification) to be added to the first chimeric cysteine-rich region, for example, by not introducing a mutation into CRD2 and CRD3 of the first chimeric cysteine-rich region, which are associated with the binding to LIGHT, TL1A and FasL, and introducing a mutation into CRD1 and/or CRD4 of the first chimeric cysteine-rich region, it is possible to obtain a DcR3 variant including a chimeric cysteine-rich region in which the binding activities of LIGHT, TL1A and FasL to the chimeric cysteine-rich region are not lowered. Meanwhile, by introducing a mutation into CRD2 or/and CRD3 of the first chimeric cysteine-rich region, which are associated with the binding to LIGHT, TL1A or FasL, it is possible to obtain a DcR3 variant including a chimeric cysteine-rich region in which the binding activity of LIGHT, TL1A or FasL to the chimeric cysteine-rich region is changed. In other words, by introducing the abovementioned mutation, it is possible to obtain a DcR3 variant including a chimeric cysteine-rich region and having a desirable binding property to LIGHT, TL1A or FasL.

In a preferred embodiment, examples of the DcR3 variant including the second chimeric cysteine-rich region include a DcR3 variant including a chimeric cysteine-rich region and having binding activity to at least one or more of LIGHT, TL1A and FasL, a DcR3 variant including a chimeric cysteine-rich region and having binding activity to all of LIGHT, TL1A and FasL, a DcR3 variant including a chimeric cysteine-rich region, having no binding activity to FasL and having binding activity to one of LIGHT and TL1A, or a DcR3 variant including a chimeric cysteine-rich region, having no binding activity to FasL and having binding activity to both of LIGHT and TL1A, or the like. With respect to the DcR3 variant of the present invention, the meanings of the expressions of "having binding activity to a ligand" and "having no binding activity to a ligand" are as mentioned above.

In a particularly preferred embodiment, examples of the DcR3 variant including the second chimeric cysteine-rich region include a DcR3 variant with lowered FasL binding activity. "Variant with lowered FasL binding activity" means a DcR3 variant including a chimeric cysteine-rich region, having no binding activity to FasL and having binding activity to one or more of LIGHT and TL1A, or a DcR3 variant including a chimeric cysteine-rich region, having no binding activity to FasL and having binding activity to both of LIGHT and TL1A.

In a preferred embodiment, examples of the DcR3 variant including the second chimeric cysteine-rich region include a DcR3 variant including a chimeric cysteine-rich region and having neutralizing activity to at least one or more of LIGHT, TL1A and FasL, a DcR3 variant including a chimeric cysteine-rich region and having neutralizing activity to all of LIGHT, TL1A and FasL, a DcR3 variant including a chimeric cysteine-rich region, having no neutralizing activity to FasL and having neutralizing activity to one of LIGHT and TL1A, or a DcR3 variant including a chimeric cysteine-rich region, having no neutralizing activity to FasL and having neutralizing activity to both of LIGHT and TL1A, or the like. With respect to the DcR3 variant of the present invention, the meanings of the expressions of "having neutralizing activity to a ligand" and "having no neutralizing activity to a ligand" are as mentioned above.

In a particularly preferred embodiment, examples of the DcR3 variant including the second chimeric cysteine-rich region include a DcR3 variant with lowered FasL neutralizing activity. "Variant with lowered FasL neutralizing activity" means a DcR3 variant including a chimeric cysteine-rich region, having no neutralizing activity to FasL and having neutralizing activity to one or more of LIGHT and TL1A, or a DcR3 variant including a chimeric cysteine-rich region, having no neutralizing activity to FasL and having neutralizing activity to both of LIGHT and TL1A.

For example, a DcR3 variant that is a variant with lowered FasL binding activity and includes a chimeric cysteine-rich region can be obtained by producing a modified product in which a binding site to each ligand of DcR3 or a DcR3 variant predicted by a crystal structure analysis or the like is substituted with Ala or another amino acid, and measuring the binding activity and the neutralizing activity to a LIGHT, TL1A or FasL ligand. The DcR3 variant can also be obtained by producing a gene library in which regions around the ligand-binding site of DcR3 or a DcR3 variant are randomly converted to other amino acids, displaying it on a phage, a yeast, a mammalian cell or the like, and performing screening using the binding activity and the neutralizing activity to a LIGHT, TL1A or FasL ligand as indices.

Examples of an amino acid sequence of a chimeric cysteine-rich region included in a variant with lowered FasL binding activity (one embodiment of the amino acid sequence (e)) include an amino acid sequence obtained by introducing into the amino acid sequence (a), (b), (c) or (d), substitution of one or two or more amino acids selected from the group consisting of Glu at position 57, Arg at position 58 and Arg at position 60 from the N-terminus with (an)other amino acid(s).

Another amino acid used for substitution of Glu at position 57 is not particularly limited, and can be appropriately selected from 19 types of amino acids excluding Glu, of 20 types of amino acids (Glu, Ala, Asp, Lys, Leu, Cys, Phe, Gly, His, Ile, Met, Asn, Pro, Gln, Arg, Ser, Thr, Val, Trp and Tyr), but it is preferably selected from Lys, Leu, Arg, Val, Ala, Phe, His, Ile and Met, more preferably selected from Lys, Leu, Arg and Val, still more preferably selected from Lys, Arg and Val, and yet more preferably selected from Lys and Arg.

Another amino acid used for substitution of Arg at position 58 is not particularly limited, and can be appropriately selected from 19 types of amino acids excluding Arg, of 20 types of amino acids, but it is preferably selected from Asp, Glu and Thr, and more preferably selected from Asp and Glu.

Another amino acid used for substitution of Arg at position 60 is not particularly limited, and can be appropriately selected from 19 types of amino acids excluding Arg, of 20 types of amino acids, but it is preferably Lys.

In a preferred embodiment, one amino acid consisting of Glu at position 57 is selected as the one or two or more amino acids selected from the group consisting of Glu at position 57, Arg at position 58 and Arg at position 60.

In further another preferred embodiment, two amino acids consisting of Glu at position 57 and Arg at position 58 are selected as the one or two or more amino acids selected from the group consisting of Glu at position 57, Arg at position 58 and Arg at position 60. In this embodiment, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

The second chimeric cysteine-rich region may include substitution of one or two or more amino acids other than Glu at position 57, Arg at position 58 and Arg at position 60 with (an)other amino acid(s). Examples of an amino acid other than Glu at position 57, Arg at position 58 and Arg at position 60 include Trp at position 53, Asn at position 54, Tyr at position 55, Leu at position 56 or the like from the N-terminus of the amino acid sequence (a), (b), (c) or (d).

Another amino acid used for substitution of Trp at position 53 is not particularly limited, and can be appropriately selected from 19 types of amino acids excluding Trp, of 20 types of amino acids, but it is preferably selected from Asp and Asn. Substitution of Trp at position 53 with another amino acid can be combined with, for example, substitution of Glu at position 57 with another amino acid.

Another amino acid used for substitution of Asn at position 54 is not particularly limited, and can be appropriately selected from 19 types of amino acids excluding Asn, of 20 types of amino acids, but it is preferably Asp. Substitution of Asn at position 54 with another amino acid can be combined with, for example, substitution of Glu at position 57 with another amino acid.

Another amino acid used for substitution of Tyr at position 55 is not particularly limited, and can be appropriately selected from 19 types of amino acids excluding Tyr, of 20 types of amino acids, but it is preferably selected from Thr, Asp, Gln and Glu. Substitution of Tyr at position 55 with another amino acid can be combined with, for example, substitution of Glu at position 57 with another amino acid.

Another amino acid used for substitution of Leu at position 56 is not particularly limited, and can be appropriately selected from 19 types of amino acids excluding Leu, of 20 types of amino acids, but it is preferably selected from Asp, Gln, Thr, Glu, Gly, Asn and Pro. Substitution of Leu at position 56 with another amino acid can be combined with, for example, substitution of Glu at position 57 with another amino acid.

Specific examples of an amino acid sequence including substitution of one or two or more amino acids selected from the group consisting of Glu at position 57, Arg at position 58 and Arg at position 60 from the N-terminus of the amino acid sequence (a), (b), (c) or (d) with (an)other amino acid(s) include:
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Lys (an amino acid sequence consisting of amino acids at positions 1 to 164 from the N-terminus of the amino acid sequence set forth in SEQ ID NO: 42);
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Lys (an amino acid sequence consisting of amino acids at positions 1 to 164 from the N-terminus of the amino acid sequence set forth in SEQ ID NO: 66);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Leu (an amino acid sequence consisting of amino acids at positions 1 to 164 from the N-terminus of the amino acid sequence set forth in SEQ ID NO: 44);
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Leu (an amino acid sequence consisting of amino acids at positions 1 to 164 from the N-terminus of the amino acid sequence set forth in SEQ ID NO: 68);
an amino acid sequence obtained by introducing into the amino acid sequence consisting of amino acids at positions 1 to 164 from the N-terminus of the amino acid sequence set forth in SEQ ID NO: 30, substitution of Arg at position 60 from the N-terminus with Lys (an amino acid sequence consisting of amino acids at positions 1 to 164 from the N-terminus of the amino acid sequence set forth in SEQ ID NO: 46);
an amino acid sequence obtained by introducing into the amino acid sequence consisting of amino acids at positions 1 to 164 from the N-terminus of the amino acid sequence set forth in SEQ ID NO: 54, substitution of Arg at position 60 from the N-terminus with Lys (an amino acid sequence consisting of amino acids at positions 1 to 164 from the N-terminus of the amino acid sequence set forth in SEQ ID NO: 70);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Arg;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Arg (an amino acid sequence consisting of amino acids at positions 1 to 164 from the N-terminus of the amino acid sequence set forth in SEQ ID NO: 180);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Val;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Val (an amino acid sequence consisting of amino acids at positions 1 to 164 from the N-terminus of the amino acid sequence set forth in SEQ ID NO: 182);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Ala;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Ala (an amino acid sequence consisting of amino acids at positions 1 to 164 from the N-terminus of the amino acid sequence set forth in SEQ ID NO: 270);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Phe;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Phe (an amino acid sequence consisting of amino acids at positions 1 to 164 from the N-terminus of the amino acid sequence set forth in SEQ ID NO: 272);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with His;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with His (an amino acid sequence consisting of amino acids at positions 1 to 164 from the N-terminus of the amino acid sequence set forth in SEQ ID NO: 274);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Ile;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Ile (an amino acid sequence consisting of amino acids at positions 1 to 164 from the N-terminus of the amino acid sequence set forth in SEQ ID NO: 276);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Met;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Met (an amino acid sequence consisting of amino acids at positions 1 to 164 from the N-terminus of the amino acid sequence set forth in SEQ ID NO: 278);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Lys, and substitution of Arg at position 58 from the N-terminus with Asp;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Lys, and substitution of Arg at position 58 from the N-terminus with Asp (an amino acid sequence consisting of amino acids at positions 1 to 164 from the N-terminus of the amino acid sequence set forth in SEQ ID NO: 184);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Lys, and substitution of Arg at position 58 from the N-terminus with Glu;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Lys, and substitution of Arg at position 58 from the N-terminus with Glu (an amino acid sequence consisting of amino acids at positions 1 to 164 from the N-terminus of the amino acid sequence set forth in SEQ ID NO: 186);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Arg, and substitution of Arg at position 58 from the N-terminus with Asp;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Arg, and substitution of Arg at position 58 from the N-terminus with Asp (an amino acid sequence consisting of amino acids at positions 1 to 164 from the N-terminus of the amino acid sequence set forth in SEQ ID NO: 188);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Lys, and substitution of Arg at position 58 from the N-terminus with Thr;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Lys, and substitution of Arg at position 58 from the N-terminus with Thr (an amino acid sequence consisting of amino acids at positions 1 to 164 from the N-terminus of the amino acid sequence set forth in SEQ ID NO: 280);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Leu, and substitution of Arg at position 58 from the N-terminus with Glu;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Leu, and substitution of Arg at position 58 from the N-terminus with Glu (an amino acid sequence consisting of amino acids at positions 1 to 164 from the N-terminus of the amino acid sequence set forth in SEQ ID NO: 282);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Val, and substitution of Arg at position 58 from the N-terminus with Thr;
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Val, and substitution of Arg at position 58 from the N-terminus with Thr (an amino acid sequence consisting of amino acids at positions 1 to 164 from the N-terminus of the amino acid sequence set forth in SEQ ID NO: 284);
an amino acid sequence obtained by introducing into the amino acid sequence consisting of amino acids at positions 1 to 164 from the N-terminus of the amino acid sequence set forth in SEQ ID NO: 30, substitution of Glu at position 57 from the N-terminus with Val, and substitution of Arg at position 58 from the N-terminus with TGlu; and
an amino acid sequence obtained by introducing into the amino acid sequence consisting of amino acids at positions 1 to 164 from the N-terminus of the amino acid sequence set forth in SEQ ID NO: 54, substitution of Glu at position 57 from the N-terminus with Val, and substitution of Arg at position 58 from the N-terminus with Glu (an amino acid sequence consisting of amino acids at positions 1 to 164 from the N-terminus of the amino acid sequence set forth in SEQ ID NO: 286).

SEQ ID NO: 42 represents an amino acid sequence of chimera A-E57K-HBD (a DcR3 variant obtained by introducing into chimera A-HBD, substitution of Glu at position 57 from the N-terminus with Lys); SEQ ID NO: 44 represents an amino acid sequence of chimera A-E57L-HBD (a DcR3 variant obtained by introducing into chimera A-HBD, substitution of Glu at position 57 from the N-terminus with Leu); SEQ ID NO: 46 represents an amino acid sequence of chimera A-R60K-HBD (a DcR3 variant obtained by introducing into chimera A-HBD, substitution of Arg at position 60 from the N-terminus with Lys); SEQ ID NO: 66 represents an amino acid sequence of chimera A-E57K (a DcR3 variant obtained by introducing into chimera A, substitution of Glu at position 57 from the N-terminus with Lys); SEQ ID NO: 68 represents an amino acid sequence of chimera A-E57L (a DcR3 variant obtained by introducing into chimera A, Glu at position 57 from the N-terminus with Leu); SEQ ID NO: 70 represents an amino acid sequence of chimera A-R60K (a DcR3 variant obtained by introducing into chimera A, substitution of Arg at position 60 from the N-terminus with Lys); SEQ ID NO: 180 represents an amino acid sequence of chimera A-E57R (a DcR3 variant obtained by introducing into chimera A, substitution of Glu at position 57 from the N-terminus with Arg); SEQ ID NO: 182 represents an amino acid sequence of chimera A-E57V (a DcR3 variant obtained by introducing into chimera A, substitution of Glu at position 57 from the N-terminus with Val); SEQ ID NO: 184 represents an amino acid sequence of chimera A-E57K_R58D (a DcR3 variant obtained by introducing into chimera A, substitution of Glu at position 57 from the N-terminus with Lys, and substitution of Arg at position 58 from the N-terminus with Asp); SEQ ID NO: 186 represents an amino acid sequence of chimera A-E57K_R58E (a DcR3 variant obtained by introducing into chimera A, substitution of Glu at position 57 from the N-terminus with Lys, and substitution of Arg at position 58 from the N-terminus with Glu); SEQ ID NO: 188 represents an amino acid sequence of chimera A-E57R_R58D (a DcR3 variant obtained by introducing into chimera A, substitution of Glu at position 57 from the N-terminus with Arg, and substitution of Arg at position 58 from the N-terminus with Asp); SEQ ID NO: 270 represents an amino acid sequence of chimera A-E57A (a DcR3 variant obtained by introducing into chimera A, substitution of Glu at position 57 from the N-terminus with Ala); SEQ ID NO: 272 represents an amino acid sequence of chimera A-E57F (a DcR3 variant obtained by introducing into chimera A, substitution of Glu at position 57 from the N-terminus with Phe); SEQ ID NO: 274 represents an amino acid sequence of chimera A-E57H (a DcR3 variant obtained by introducing into chimera A, substitution of Glu at position 57 from the N-terminus with His); SEQ ID NO: 276 represents an amino acid sequence of chimera A-E57I (a DcR3 variant obtained by introducing into chimera A, substitution of Glu at position 57 from the N-terminus with Ile); SEQ ID NO: 278 represents an amino acid sequence of chimera A-E57M (a DcR3 variant obtained by introducing into chimera A, substitution of Glu at position 57 from the N-terminus with Met); SEQ ID NO: 280 represents an amino acid sequence of chimera A-E57K_R58T (a DcR3 variant obtained by introducing into chimera A, substitution of Glu at position 57 from the N-terminus with Lys, and substitution of Arg at position 58 from the N-terminus with Thr); SEQ ID NO: 282 represents an amino acid sequence of chimera A-E57K_R58T (a DcR3 variant obtained by introducing into chimera A, substitution of Glu at position 57 from the N-terminus with Lys, and substitution of Arg at position 58 from the N-terminus with Thr); SEQ ID NO: 284 represents an amino acid sequence of chimera A-E57V_R58T (a DcR3 variant obtained by introducing into chimera A, substitution of Glu at position 57 from the N-terminus with Val, and substitution of Arg at position 58 from the N-terminus with Thr); and SEQ ID NO: 286 represents an amino acid sequence of chimera A-E57V_R58E (a DcR3 variant obtained by introducing into chimera A, substitution of Glu at position 57 from the N-terminus with Val, and substitution of Arg at position 58 from the N-terminus with Glu).

### 2-2. Other Region

The DcR3 variant of the present invention may or may not include one or two or more other regions bound to the C-terminal side of the first or second chimeric cysteine-rich region. Examples of the other regions include a part or a whole of a region existing between CRD4 and HBD in wild-type DcR3, a part or a whole of a region following the C-terminus of the amino acid sequence of CRD4 in a TNF receptor superfamily (TNFRSF) molecule other than DcR3, a part or a whole of HBD of wild-type DcR3 and the like. The expression of "other region bound to the C-terminal side of the first or second chimeric cysteine-rich region" is used as a meaning including the case where the other region is directly bound to the C-terminus of the first or second chimeric cysteine-rich region, and the case where the other region is bound to the C-terminus of the first or second chimeric cysteine-rich region via another further region.

In one embodiment, the DcR3 variant of the present invention includes a part or a whole of a region existing between CRD4 and HBD in wild-type DcR3 as the other region bound to the C-terminal side of the first or second chimeric cysteine-rich region. In this embodiment, the other region is preferably directly bound to the C-terminus of the first or second chimeric cysteine-rich region.

In another embodiment, the DcR3 variant of the present invention includes a part or a whole of a region extending to the C-terminus of the amino acid sequence of CRD4 in a TNF receptor superfamily (TNFRSF) molecule other than DcR3 as the other region bound to the C-terminal side of the first or second chimeric cysteine-rich region. In this embodiment, the other region is preferably directly bound to the C-terminus of the first or second chimeric cysteine-rich region. The description on TNFRSF is the same as mentioned above. TNFRSF is preferably OPG.

When the first or second chimeric cysteine-rich region (e.g., the first chimeric cysteine-rich region consisting of the amino acid sequence (b), (c) or (d)) includes substitution of CRD4 of wild-type DcR3 with CRD4 of OPG, the DcR3 variant of the present invention preferably includes a plurality of amino acid residues following the C-terminus of the amino acid sequence of the CRD4 of the OPG as the other region bound to the C-terminus of the chimeric cysteine-rich region. In other words, when CR4 of wild-type DcR3 is substituted with CRD4 of OPG, a plurality of amino acid residues following the C-terminus of the amino acid sequence of the CRD4 of the wild-type DcR3 is also preferably substituted with a plurality of amino acid residues following the C-terminus of the amino acid sequence of the CRD4 of the OPG. As a plurality of amino acid residues following the C-terminus of the amino acid sequence of CRD4 of OPG, for example, amino acid residues at positions 186 to 194 of the amino acid sequence (SEQ ID NO: 14) of OPG are preferable, but it is possible to appropriately adjust the number of amino acid residues used for substitution of a plurality of amino acid residues following the C-terminus of CRD4 of wild-type DcR3. The number of a plurality of amino acid residues extending to the C-terminus of the amino acid sequence of CRD4 of OPG is usually 1 to 12, preferably 1 to 10, more preferably 1 to 9, 1 to 6, or 1 to 3. When the first or second chimeric cysteine-rich region (e.g., the first chimeric cysteine-rich region consisting of the amino acid sequence (a) or (d)) includes CRD4 derived from wild-type DcR3, it is preferable that an amino acid sequence following the C-terminus of the amino acid sequence of the CRD4 of the wild-type DcR3 is bound to the C-terminus of the amino acid sequence of the CRD4 of the first chimeric cysteine-rich region. Examples of such amino acid sequence include amino acids at positions 194 to 195 of SEQ ID NO: 2, but it is possible to appropriately adjust the number of amino acid residues constituting the amino acid sequence following the C-terminus of the amino acid sequence of CRD4 of wild-type DcR3. The number of amino acid residues constituting the amino acid sequence extending to the C-terminus of CRD4 of wild-type DcR3 is usually 1 to 10, preferably 1 to 5, more preferably 1 to 3, and still more preferably 1 to 2.

In further another embodiment, the DcR3 variant of the present invention includes a part or a whole of HBD of wild-type DcR3 as the other region bound to the C-terminal side of the first or second chimeric cysteine-rich region. In this embodiment, the other region may be directly bound to the C-terminus of the first or second chimeric cysteine-rich region, or may be bound to the C-terminus of the first or second chimeric cysteine-rich region via a part or a whole of a region existing between CRD4 and HBD in wild-type DcR3 or via a part or a whole of a region extending to the C-terminus of the amino acid sequence of CRD4 in a TNFRSF molecule other than DcR3.

It is preferable that the DcR3 variant of the present invention does not include HBD and includes amino acid residues at positions 186 to 194 of the amino acid sequence (SEQ ID NO: 14) of OPG as the other regions bound to the C-terminal side of the first or second chimeric cysteine-rich region.

### 3. DcR3 Variant Including Glycan

The DcR3 variant of the present invention also includes a DcR3 variant including at least one glycan. If at least one glycan is bound to the cysteine-rich region included in the DcR3 variant mentioned above or an amino acid residue other than it, any DcR3 variant including a glycan is also included in the present invention.

Glycoprotein has one or two or more glycans. When glycoprotein has two or more glycans, one type of glycan may be included or two or more types of glycans may be included in the glycoprotein. Examples of the glycan included in the glycoprotein include a glycan that is N-glycoside-linked to an amino acid residue (e.g., an asparagine residue, etc.) of a peptide or a protein, a glycan that is O-glycoside-linked to an amino acid residue (e.g., a serine residue, a threonine residue, etc.) of a peptide or a protein, or the like. Examples of the O-linked glycan include core 1, core 2 or the like, or examples of the N-linked glycan include high-mannose-type, hybrid-type or complex glycan, but a complex glycan is preferable.

### 4. DcR3 Variant Including Fc Region

The DcR3 variant of the present invention also includes a protein in which a homogeneous or heterogeneous peptide, polypeptide or protein is bound or fused directly or, if necessary, via an appropriate peptide linker to the N-terminal side or the C-terminal side of the chimeric cysteine-rich region (or a chimeric cysteine-rich region including (an)other region(s)). The number of amino acids constituting the peptide linker is not particularly limited, but examples thereof include 4, 5, 6 or 15 or the like.

Examples of the polypeptide or protein to be bound or fused to the chimeric cysteine-rich region include a polypeptide or protein such as a constant region or Fc region of an immunoglobulin, a peptide that binds to a neonatal Fc receptor (FcRn), albumin, Protein A, Protein G, β-galactosidase, glutathione-S-transferase (GST), a maltose-binding protein, polyhistidine and an FLAG peptide, but it is preferably an Fc region of an immunoglobulin or a mutant thereof (mutated Fc region), and more preferably an Fc region of an immunoglobulin derived from a mammal or a mutant thereof (mutated Fc region).

As an Fc region of an immunoglobulin (also referred to as antibody), an Fc region of a human immunoglobulin is preferable when used for a human. Examples of a class and a subclass of the immunoglobulin include, but are not limited to, IgG, IgD, IgE, IgM, IgA, IgG1, IgG2, IgG2a, IgG2b, IgG2c, IgG3, IgG4 or IgA1 or the like, and, if used for a human, it is preferable to use a class and a subclass of a human immunoglobulin. When an Fc region of an immunoglobulin is used as a polypeptide or protein to be bound or fused to the chimeric cysteine-rich region, preferably the Fc region of the immunoglobulin is bound or fused to the C-terminal side of the chimeric cysteine-rich region.

The immunoglobulin is composed of polypeptides of a heavy chain and a light chain, and the constant region of a heavy chain of human IgG is composed of a CH1 domain, a hinge domain, a CH2 domain and a CH3 domain in this order from the N-terminus. The Fc region of IgG in the present invention also includes a region combining the CH2 domain and the CH3 domain, and a region combining a part or a whole of the hinge domain, the CH2 domain and the CH3 domain. Each domain included in the Fc region of IgG in the present invention can be identified by the number of the EU index. Specifically, the hinge domain is identified by EU-index positions 216 to 230, the CH2 domain is identified by EU-index positions 231 to 340, and CH3 is identified by EU-index positions 341 to 447.

The polypeptide or protein to be bound or fused to the chimeric cysteine-rich region also includes a modified polypeptide or protein further including substitution, deletion, insertion or addition of one or more amino acids in order to change the biological activity of the DcR3 variant or properties thereof, *in vivo* kinetics such as half-life in blood, or physical or chemical properties such as stability of a protein. The modification of the polypeptide or protein to be bound or fused to the chimeric cysteine-rich region may be a natural mutation or an artificial substitution, deletion, insertion or addition of an amino acid. Examples of the modified polypeptide or protein include a mutated Fc region consisting of an amino acid sequence obtained by introducing into the amino acid sequence of the Fc region of the immunoglobulin, substitution, deletion, insertion or addition of one or several amino acids. Examples of the amino acid sequence of the mutated Fc region include an amino acid sequence obtained by introducing into the amino acid sequence of the Fc region of the immunoglobulin, substitution, deletion, insertion or addition of one or several, preferably 2 to 30, more preferably 2 to 10, and particularly preferably 2 to 5 amino acids, or an amino acid sequence having 80% or more, preferably 85% or more, and more preferably 90% or more identity to the amino acid sequence of the Fc region of the immunoglobulin.

An addition or deletion of the abovementioned glycosylation site is also included in the modification of a polypeptide or protein to be bound or fused to the chimeric cysteine-rich region. For example, by adding or inserting a polypeptide including an N-linked glycosylation site, it is possible to add the glycosylation site. Specific examples of the sequence of the polypeptide include GGNGT or YGNGT consisting of five amino acids [WO 2014/153111 A].

Examples of substitution introduced into human IgG1 in order to lower or eliminate complement-dependent cytotoxicity (CDC) activity include substitution of one or two or more amino acids selected from Leu at EU-index position 234 (L234), Leu at EU-index position 235 (L235), Asp at EU-index position 265 (D265), Asp at EU-index position 270 (D265), Lys at EU-index position 322 (K322), Pro at EU-index position 329 (P329), Pro at EU-index position 331 (P331) and the like with (an)other amino acid(s) or the like, and specific examples thereof include substitution of one or two or more amino acids selected from L234, L235, D270, K322, P329, P331 and the like indicated by the EU index with Ala, or substitution of P331 with Ser or Gly [J. Immunol., 2000, 164: p.4178-4184, Cell. Immunol., 2000, 200: p.16-26].

Examples of substitution introduced into human IgG1 in order to lower or eliminate effector activity such as antibody-dependent cellular cytotoxicity (ADCC) activity and antibody-dependent cellular phagocytosis (ADCP) include substitution of one or two or more amino acids selected from Asn at EU-index position 297 (N297), Leu at EU-index position 234 (L234), Leu at EU-index position 235 (L235), Gly at EU-index position 237 (G237), Cys at EU-index position 226 (C226), Cys at EU-index position 229 (C229), Pro at EU-index position 238 (P238), Glu at EU-index position 233 (E233), Ser at EU-index position 267 (S267), Leu at EU-index position 328 (L328), Pro at EU-index position 331 (P331) and the like with (an)other amino acid(s), and specific examples thereof include an amino acid substitution such as substitution of N297 indicated by the EU index with Ala (N297A), substitution of N297 indicated by the EU index with Gln (N297Q), substitution of N297 indicated by the EU index with Gly (N297G), or substitution of L234 indicated by the EU index with Ala (L234A)/substitution of L235 indicated by the EU index with Ala (L235A)/substitution of G237 indicated by the EU index with Ala (G237A) or the like. "/" means "and" (the same hereinafter).

In addition, examples of modification that increases the binding activity to FcγRIIb, which is an inhibitory receptor, include substitution of Gly at EU-index position 236 with Asp (G236D), substitution of Leu at EU-index position 328 with Phe (L328F), substitution of Ser at EU-index position 239 with Asp (S239D), substitution of Ser at EU-index position 267 with Glu (S267E) or the like [Curr. Opin. Cell. Biol., 2009, 20: p.685-691]. Examples of modification that enhances the binding to a neonatal Fc receptor (FcRn) in an environment with low pH in an endosome, thus avoiding the elimination of an antibody and prolonging the half-life in blood include substitution of Thr at EU-index position 250 with Gln (T250Q), substitution of Met at EU-index position 428 with Leu (M428L), substitution of Met at EU-index position 252 with Tyr (M252Y), substitution of Ser at EU-index position 254 with Thr (S254T), substitution of Thr at EU-index position 256 with Glu (T256E), substitution of Met at EU-index position 252 with Tyr (M252Y)/substitution of Ser at EU-index position 254 with Thr (S254T)/substitution of Thr at EU-index position 256 with Glu (T256E), substitution of Met at EU-index position 428 with Leu (M428L)/substitution of Asn at EU-index position 434 with Ser (N434S), substitution of Asn at EU-index position 434 with Ala (N434A), substitution of Asn at EU-index position 434 with His (N434H) or the like [J. Immunol., 2009, 182: p.7663-7671, MAbs, 2017, 9: p.844-853].

In a preferred embodiment, the mutated Fc region includes substitution of Cys at EU-index position 220 with Ser, of an amino acid sequence of a heavy chain of an antibody belonging to a human IgG1 subclass. Examples of the mutated Fc region according to this embodiment include an Fc region of an immunoglobulin including an amino acid sequence obtained by introducing into an Fc region (EU-index positions 217 to 447) in which a CH1 domain and Glu at EU-index position 216 of a hinge domain have been removed from a constant region (SEQ ID NO: 153) of a heavy chain of human IgG1, substitution of Cys at EU-index position 220 , which is associated with the binding to a light chain, with Ser (C220S) (hereinafter referred to as g1S, SEQ ID NO: 72), an Fc region of an immunoglobulin including an amino acid sequence obtained by introducing into an Fc region (EU-index positions 216 to 447) in which a CH1 domain has been removed from a constant region (SEQ ID NO: 153) of a heavy chain of human IgG1, substitution of Cys at position 220 with Ser (C220S) (hereinafter referred to as Eg1S, SEQ ID NO: 156) or the like. When the DcR3 variant of the present invention includes the mutated Fc region according to this embodiment, as the first chimeric cysteine-rich region included in the DcR3 variant of the present invention, preferred is the first chimeric cysteine-rich region including substitution of CRD1 of wild-type DcR3 with CRD1 of OPG and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

In another preferred embodiment, the mutated Fc region includes substitution of Ser at EU-index position 228 with Pro, substitution of Leu at EU-index position 235 with Glu, and substitution of Arg at EU-index position 409 with Lys, of an amino acid sequence of a heavy chain of an antibody belonging to a human IgG4 subclass. Examples of the mutated Fc region according to this embodiment include Fc of an immunoglobulin including an amino acid sequence including, in addition to removal of a CH1 domain from a constant region (SEQ ID NO: 154) of a heavy chain of human IgG4, substitution of Ser at EU-index position 228 with Pro, substitution of Leu at EU-index position 235 with Glu, and substitution of Arg at EU-index position 409 with Lys as described in WO 2006/33386 A (hereinafter referred to as g4PEK, SEQ ID NO: 74). When the DcR3 variant of the present invention includes the mutated Fc region according to this embodiment, as the first chimeric cysteine-rich region included in the DcR3 variant of the present invention, preferred is the first chimeric cysteine-rich region including substitution of CRD1 of wild-type DcR3 with CRD1 of OPG, and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

In further another preferred embodiment, the mutated Fc region includes substitution of Leu at EU-index position 234 with Ala, substitution of Leu at EU-index position 235 with Ala, and substitution of Gly at EU-index position 237 with Ala, of an amino acid sequence of a heavy chain of an antibody belonging to a human IgG1 subclass. Examples of the mutated Fc region according to this embodiment include an Fc region of an immunoglobulin including an amino acid sequence obtained by introducing into an Fc region of g1S set forth in SEQ ID NO: 72 or Eg1S set forth in SEQ ID NO: 156, substitution of Leu at position 234 with Ala, substitution of Leu at position 235 with Ala, and substitution of Gly at position 237 with Ala (hereinafter referred to as g1S LALAGA or Eg1S LALAGA, SEQ ID NO: 162 or 164) or the like. When the DcR3 variant of the present invention includes the mutated Fc region according to this embodiment, as the first chimeric cysteine-rich region included in the DcR3 variant of the present invention, preferred is the first chimeric cysteine-rich region including substitution of CRD1 of wild-type DcR3 with CRD1 of OPG, and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

In further another preferred embodiment, the mutated Fc region includes substitution of Asn at EU-index position 434 with Ala, of an amino acid sequence of a heavy chain of an antibody belonging to a human IgG1 subclass. Examples of the mutated Fc region according to this embodiment include an Fc region of an immunoglobulin including an amino acid sequence obtained by introducing into an Fc region of g1S set forth in SEQ ID NO: 72 or Eg1S set forth in SEQ ID NO: 156, substitution of Asn at position 434 with Ala (hereinafter referred to as g1S N434A or Eg1S N434A, SEQ ID NO: 312 or 160), an Fc region of an immunoglobulin including an amino acid sequence obtained by introducing into an Fc region of g1S LALAGA set forth in SEQ ID NO: 162 or Eg1S LALAGA set forth in SEQ ID NO: 164, substitution of Asn at position 434 with Ala (hereinafter referred to as g1S LALAGANA or Eg1S LALAGANA, SEQ ID NO: 313 or 166) or the like. When the DcR3 variant of the present invention includes the mutated Fc region according to this embodiment, as the first chimeric cysteine-rich region included in the DcR3 variant of the present invention, preferred is the first chimeric cysteine-rich region including substitution of CRD1 of wild-type DcR3 with CRD1 of OPG, and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

In further another preferred embodiment, the mutated Fc region includes substitution of Met at EU-index position 252 with Tyr, substitution of Ser at EU-index position 254 with Thr, and substitution of Thr at EU-index position 256 with Glu, of an amino acid sequence of a heavy chain of an antibody belonging to a human IgG1 subclass. Examples of the mutated Fc region according to this embodiment include an Fc region of an immunoglobulin including an amino acid sequence obtained by introducing into an Fc region of g1S set forth in SEQ ID NO: 72 or Eg1S set forth in SEQ ID NO: 156, substitution of Met at position 252 with Tyr, substitution of Ser at position 254 with Thr, and substitution of Thr at position 256 with Glu (hereinafter referred to as g1S YTE or Eg1S YTE, SEQ ID NO: 311 or 158) or the like. When the DcR3 variant of the present invention includes the mutated Fc region according to this embodiment, as the first chimeric cysteine-rich region included in the DcR3 variant of the present invention, preferred is the first chimeric cysteine-rich region including substitution of CRD1 of wild-type DcR3 with CRD1 of OPG, and substitution of CRD4 of wild-type DcR3 with CRD4 of OPG. When the DcR3 variant of the present invention includes the mutated Fc region according to this embodiment, the second chimeric cysteine-rich region included in the DcR3 variant of the present invention preferably includes substitution of Glu at position 57 with another amino acid, or substitution of Glu at position 57 with another amino acid and substitution of Arg at position 58 with another amino acid. Another amino acid used for substitution of Glu at position 57 is preferably selected from Lys, Leu, Arg, Val, Ala, Phe, His, Ile and Met, and more preferably selected from Lys, Leu, Arg and Val. Another amino acid used for substitution of Arg at position 58 is preferably selected from Asp, Glu and Thr, and more preferably selected from Asp and Glu. When combining substitution of Glu at position 57 with another amino acid and substitution of Arg at position 58 with another amino acid, it is preferable to combine substitution of Glu at position 57 with Lys, Leu, Arg, Val, Ala, Phe, His, Ile or Met and substitution of Arg at position 58 with Asp, Glu or Thr, it is more preferable to combine substitution of Glu at position 57 with Lys, Leu, Arg or Val and substitution of Arg at position 58 with Asp or Glu, and it is still more preferable to combine substitution of Glu at position 57 with Lys or Arg and substitution of Arg at position 58 with Asp or Glu.

Examples of the mutated Fc region include, but are not limited to, a mutated Fc region including or consisting of an amino acid sequence set forth in SEQ ID NO: 72, 74, 156, 158, 160, 162, 164, 166, 311, 312 or 313 the amino acid sequence.

Examples of the peptide linker to be added in order to link further a homogeneous or heterogeneous peptide, polypeptide or protein to the N-terminal or C-terminal side of the chimeric cysteine-rich region include, but are not limited to, a peptide linker such as an IEGRMD linker or a GS linker, chemical linker or the like.

As the DcR3 variant of the present invention, most preferably a DcR3 variant including the first or second chimeric cysteine-rich region and the Fc region or the mutated Fc region is exemplified.

In one embodiment, examples of an amino acid sequence included in the DcR3 variant of the present invention include an amino acid sequence set forth in SEQ ID NO: 26, 28, 30, 32, 34, 36, 38, 40, 42, 44 or 46, or an amino acid sequence obtained by introducing into the amino acid sequence, deletion, substitution, insertion or addition of 1 to 30 amino acids. In this embodiment, the DcR3 variant of the present invention may consist of the abovementioned amino acid sequence or may include the abovementioned amino acid sequence, but preferably consists of the abovementioned amino acid sequence. The abovementioned amino acid sequence includes HBD.

In another embodiment, examples of an amino acid sequence included in the DcR3 variant of the present invention include an amino acid sequence set forth in SEQ ID NO: 50, 52, 54, 56, 58, 60, 62, 64, 66, 68, 70, 180, 182, 184, 186, 188, 270, 272, 274, 276, 278, 280, 282, 284 or 286, or an amino acid sequence obtained by introducing into the amino acid sequence, deletion, substitution, insertion or addition of 1 to 30 amino acids. In this embodiment, the DcR3 variant of the present invention may consist of the abovementioned amino acid sequence or may include the abovementioned amino acid sequence, but preferably consists of the abovementioned amino acid sequence. The abovementioned amino acid sequence does not include HBD.

In another embodiment, examples of an amino acid sequence included in the DcR3 variant of the present invention include an amino acid sequence including both of an amino acid sequence set forth in SEQ ID NO: 50, 52, 54, 56, 58, 60, 62, 64, 66, 68, 70, 180, 182, 184, 186, 188, 270, 272, 274, 276, 278, 280, 282, 284 or 286, or an amino acid sequence obtained by introducing into the amino acid sequence, deletion, substitution, insertion or addition of 1 to 30 amino acids, and, as a mutated Fc region, an amino acid sequence set forth in SEQ ID NO: 72, 74, 156, 158, 160, 162, 164, 166, 311, 312 or 313.

In further another embodiment, examples of an amino acid sequence included in the DcR3 variant of the present invention include an amino acid sequence set forth in SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 or 337, or an amino acid sequence obtained by introducing into the amino acid sequence, deletion, substitution, insertion or addition of 1 to 30 amino acids. In this embodiment, the DcR3 variant of the present invention may consist of the abovementioned amino acid sequence or may include the abovementioned amino acid sequence, but preferably consists of the abovementioned amino acid sequence. The abovementioned amino acid sequence includes an Fc region or a mutant thereof (mutated Fc region).

SEQ ID NO: 76 represents an amino acid sequence of chimera B-Fc (IEGRMD g1S) (a DcR3 variant obtained by fusion of chimera B, an IEGRMD linker and Fc (g1S)); SEQ ID NO: 78 represents an amino acid sequence of chimera C-Fc (IEGRMD g1S) (a DcR3 variant obtained by fusion of chimera C, an IEGRMD linker and Fc (g1S)); SEQ ID NO: 80 represents an amino acid sequence of chimera A-Fc (IEGRMD g1S) (a DcR3 variant obtained by fusion of chimera A, an IEGRMD linker and Fc (g1S)); SEQ ID NO: 82 represents an amino acid sequence of chimera A-Fc (g4PEK) (a DcR3 variant obtained by fusion of chimera A and Fc (g4PEK)); SEQ ID NO: 84 represents an amino acid sequence of 103-123OPG-Fc (g4PEK) (a DcR3 variant obtained by fusion of 103-123OPG and Fc (g4PEK)); SEQ ID NO: 86 represents an amino acid sequence of N131S/N144S-Fc (g4PEK) (a DcR3 variant obtained by fusion of N131S/N144S and Fc (g4PEK)); SEQ ID NO: 88 represents an amino acid sequence of T133A/S146A-Fc (g4PEK) (a DcR3 variant obtained by fusion of T133A/S146A and Fc (g4PEK)); SEQ ID NO: 90 represents an amino acid sequence of N131S/N144S/N157S-Fc (g4PEK) (a DcR3 variant obtained by fusion of N131S/N144S/N157S and Fc (g4PEK)); SEQ ID NO: 92 represents an amino acid sequence of T133A/S146A/T159A-Fc (g4PEK) (a DcR3 variant obtained by fusion of T133A/S146A/T159A and Fc (g4PEK)); SEQ ID NO: 94 represents an amino acid sequence of chimera A-E57K-Fc (g4PEK) (a DcR3 variant obtained by fusion of chimera A-E57K and Fc (g4PEK)); SEQ ID NO: 96 represents an amino acid sequence of chimera A-E57L-Fc (g4PEK) (a DcR3 variant obtained by fusion of chimera A-E57L and Fc (g4PEK)); SEQ ID NO: 98 represents an amino acid sequence of chimera A-R60K-Fc (g4PEK) (a DcR3 variant obtained by fusion of chimera A-R60K and Fc (g4PEK)); SEQ ID NO: 150 represents an amino acid sequence of chimera A-Fc (g1S) (a DcR3 variant obtained by fusion of chimera A and Fc (g1S)); SEQ ID NO: 168 represents an amino acid sequence of chimera A-Fc (Eg1S) (a DcR3 variant obtained by fusion of chimera A and Fc (Eg1S)); SEQ ID NO: 170 represents an amino acid sequence of chimera A-Fc (Eg1S-YTE) (a DcR3 variant obtained by fusion of chimera A and Fc (Eg1S YTE)); SEQ ID NO: 172 represents an amino acid sequence of chimera A-Fc (Eg1S-N434A) (a DcR3 variant obtained by fusion of chimera A and Fc (Eg1S-N434A)); SEQ ID NO: 174 represents an amino acid sequence of chimera A-Fc (g1S-LALAGA) (a DcR3 variant obtained by fusion of chimera A and Fc (g1S-LALAGA)); SEQ ID NO: 176 represents an amino acid sequence of chimera A-Fc (Eg1S-LALAGA) (a DcR3 variant obtained by fusion of chimera A and Fc (Eg1S-LALAGA)); SEQ ID NO: 178 represents an amino acid sequence of chimera A-Fc (Eg1S-LALAGANA) (a DcR3 variant obtained by fusion of chimera A and Fc (Eg1S-LALAGANA)); SEQ ID NO: 190 represents an amino acid sequence of chimera A-E57R-Fc (g4PEK) (a DcR3 variant obtained by fusion of chimera A-E57R and Fc (g4PEK)); SEQ ID NO: 192 represents an amino acid sequence of chimera A-E57V-Fc (g4PEK) (a DcR3 variant obtained by fusion of chimera A-E57V and Fc (g4PEK)); SEQ ID NO: 194 represents an amino acid sequence of chimera A-E57K_R58D-Fc (g4PEK) (a DcR3 variant obtained by fusion of chimera A-E57K_R58D and Fc (g4PEK)); SEQ ID NO: 196 represents an amino acid sequence of chimera A-E57K_R58E-Fc (g4PEK) (a DcR3 variant obtained by fusion of chimera A-E57K_R58E and Fc (g4PEK)); SEQ ID NO: 198 represents an amino acid sequence of chimera A-E57R_R58D-Fc (g4PEK) (a DcR3 variant obtained by fusion of chimera A-E57R_R58D and Fc (g4PEK)); SEQ ID NO: 200 represents an amino acid sequence of chimera A-E57K-Fc (Eg1S) (a DcR3 variant obtained by fusion of chimera A-E57K and Fc (Eg1S)); SEQ ID NO: 202 represents an amino acid sequence of chimera A-E57L-Fc (Eg1S) (a DcR3 variant obtained by fusion of chimera A-E57L and Fc (Eg1S)); SEQ ID NO: 204 represents an amino acid sequence of chimera A-E57R-Fc (Eg1S) (a DcR3 variant obtained by fusion of chimera A-E57R and Fc (Eg1S)); SEQ ID NO: 206 represents an amino acid sequence of chimera A-E57V-Fc (Eg1S) (a DcR3 variant obtained by fusion of chimera A-E57V and Fc (Eg1S)); SEQ ID NO: 208 represents an amino acid sequence of chimera A-E57K_R58D-Fc (Eg1S) (a DcR3 variant obtained by fusion of chimera A-E57K_R58D and Fc (Eg1S)); SEQ ID NO: 210 represents an amino acid sequence of chimera A-E57K_R58E-Fc (Eg1S) (a DcR3 variant obtained by fusion of chimera A-E57K_R58E and Fc (Eg1S)); SEQ ID NO: 212 represents an amino acid sequence of chimera A-E57R_R58D-Fc (Eg1S) (a DcR3 variant obtained by fusion of chimera A-E57R_R58D and Fc (Eg1S)); SEQ ID NO: 214 represents an amino acid sequence of chimera A-E57K-Fc (Eg1S YTE) (a DcR3 variant obtained by fusion of chimera A-E57K and Fc (Eg1S YTE)); SEQ ID NO: 216 represents an amino acid sequence of chimera A-E57L-Fc (Eg1S YTE) (a DcR3 variant obtained by fusion of chimera A-E57L and Fc (Eg1S YTE)); SEQ ID NO: 218 represents an amino acid sequence of chimera A-E57R-Fc (Eg1S YTE) (a DcR3 variant obtained by fusion of chimera A-E57R and Fc (Eg1S YTE)); SEQ ID NO: 220 represents an amino acid sequence of chimera A-E57V-Fc (Eg1S YTE) (a DcR3 variant obtained by fusion of chimera A-E57V and Fc (Eg1S YTE)); SEQ ID NO: 222 represents an amino acid sequence of chimera A-E57K_R58D-Fc (Eg1S YTE) (a DcR3 variant obtained by fusion of chimera A-E57K_R58D and Fc (Eg1S YTE)); SEQ ID NO: 224 represents an amino acid sequence of chimera A-E57K_R58E-Fc (Eg1S YTE) (a DcR3 variant obtained by fusion of chimera A-E57K_R58E and Fc (Eg1S YTE)); SEQ ID NO: 226 represents an amino acid sequence of chimera A-E57R_R58D-Fc (Eg1S YTE) (a DcR3 variant obtained by fusion of chimera A-E57R_R58D and Fc (Eg1S YTE)); SEQ ID NO: 228 represents an amino acid sequence of chimera A-E57K-Fc (Eg1S N434A) (a DcR3 variant obtained by fusion of chimera A-E57K and Fc (Eg1S N434A)); SEQ ID NO: 230 represents an amino acid sequence of chimera A-E57L-Fc (Eg1S N434A) (a DcR3 variant obtained by fusion of chimera A-E57L and Fc (Eg1S N434A)); SEQ ID NO: 232 represents an amino acid sequence of chimera A-E57R-Fc (Eg1S N434A) (a DcR3 variant obtained by fusion of chimera A-E57R and Fc (Eg1S N434A)); SEQ ID NO: 234 represents an amino acid sequence of chimera A-E57V-Fc (Eg1S N434A) (a DcR3 variant obtained by fusion of chimera A-E57V and Fc (Eg1S N434A)); SEQ ID NO: 236 represents an amino acid sequence of chimera A-E57K_R58D-Fc (Eg1S N434A) (a DcR3 variant obtained by fusion of chimera A-E57K_R58D and Fc (Eg1S N434A)); SEQ ID NO: 238 represents an amino acid sequence of chimera A-E57K_R58E-Fc (Eg1S N434A) (a DcR3 variant obtained by fusion of chimera A-E57K_R58E and Fc (Eg1S N434A)); SEQ ID NO: 240 represents an amino acid sequence of chimera A-E57R_R58D-Fc (Eg1S N434A) (a DcR3 variant obtained by fusion of chimera A-E57R_R58D and Fc (Eg1S N434A)); SEQ ID NO: 242 represents an amino acid sequence of chimera A-E57K-Fc (Eg1S LALAGA) (a DcR3 variant obtained by fusion of chimera A-E57K and Fc (Eg1S LALAGA)); SEQ ID NO: 244 represents an amino acid sequence of chimera A-E57L-Fc (Eg1S LALAGA) (a DcR3 variant obtained by fusion of chimera A-E57L and Fc (Eg1S LALAGA)); SEQ ID NO: 246 represents an amino acid sequence of chimera A-E57R-Fc (Eg1S LALAGA) (a DcR3 variant obtained by fusion of chimera A-E57R and Fc (Eg1S LALAGA)); SEQ ID NO: 248 represents an amino acid sequence of chimera A-E57V-Fc (Eg1S LALAGA) (a DcR3 variant obtained by fusion of chimera A-E57V and Fc (Eg1S LALAGA)); SEQ ID NO: 250 represents an amino acid sequence of chimera A-E57K_R58D-Fc (Eg1S LALAGA) (a DcR3 variant obtained by fusion of chimera A-E57K_R58D and Fc (Eg1S LALAGA)); SEQ ID NO: 252 represents an amino acid sequence of chimera A-E57K_R58E-Fc (Eg1S LALAGA) (a DcR3 variant obtained by fusion of chimera A-E57K_R58E and Fc (Eg1S LALAGA)); SEQ ID NO: 254 represents an amino acid sequence of chimera A-E57R_R58D-Fc (Eg1S LALAGA) (a DcR3 variant obtained by fusion of chimera A-E57R_R58D and Fc (Eg1S LALAGA)); SEQ ID NO: 256 represents an amino acid sequence of chimera A-E57K-Fc (Eg1S LALAGANA) (a DcR3 variant obtained by fusion of chimera A-E57K and Fc (Eg1S LALAGANA)); SEQ ID NO: 258 represents an amino acid sequence of chimera A-E57L-Fc (Eg1S LALAGANA) (a DcR3 variant obtained by fusion of chimera A-E57L and Fc (Eg1S LALAGANA)); SEQ ID NO: 260 represents an amino acid sequence of chimera A-E57R-Fc (Eg1S LALAGANA) (a DcR3 variant obtained by fusion of chimera A-E57R and Fc (Eg1S LALAGANA)); SEQ ID NO: 262 represents an amino acid sequence of chimera A-E57V-Fc (Eg1S LALAGANA) (a DcR3 variant obtained by fusion of chimera A-E57V and Fc (Eg1S LALAGANA)); SEQ ID NO: 264 represents an amino acid sequence of chimera A-E57K_R58D-Fc (Eg1S LALAGANA) (a DcR3 variant obtained by fusion of chimera A-E57K_R58D and Fc (Eg1S LALAGANA)); SEQ ID NO: 266 represents an amino acid sequence of chimera A-E57K_R58E-Fc (Eg1S LALAGANA) (a DcR3 variant obtained by fusion of chimera A-E57K_R58E and Fc (Eg1S LALAGANA)); SEQ ID NO: 268 represents an amino acid sequence of chimera A-E57R_R58D-Fc (Eg1S LALAGANA) (a DcR3 variant obtained by fusion of chimera A-E57R_R58D and Fc (Eg1S LALAGANA)); SEQ ID NO: 288 represents an amino acid sequence of chimera A-E57A-Fc (g4PEK) (a DcR3 variant obtained by fusion of chimera A-E57A and Fc (g4PEK)); SEQ ID NO: 290 represents an amino acid sequence of chimera A-E57F-Fc (g4PEK) (a DcR3 variant obtained by fusion of chimera A-E57F and Fc (g4PEK)); SEQ ID NO: 292 represents an amino acid sequence of chimera A-E57H-Fc (g4PEK) (a DcR3 variant obtained by fusion of chimera A-E57H and Fc (g4PEK)); SEQ ID NO: 294 represents an amino acid sequence of chimera A-E57I-Fc (g4PEK) (a DcR3 variant obtained by fusion of chimera A-E57I and Fc (g4PEK)); SEQ ID NO: 296 represents an amino acid sequence of chimera A-E57M-Fc (g4PEK) (a DcR3 variant obtained by fusion of chimera A-E57M and Fc (g4PEK)); SEQ ID NO: 298 represents an amino acid sequence of chimera A-E57K_R58T-Fc (g4PEK) (a DcR3 variant obtained by fusion of chimera A-E57K_R58T and Fc (g4PEK)); SEQ ID NO: 300 represents an amino acid sequence of chimera A-E57L_R58E-Fc (g4PEK) (a DcR3 variant obtained by fusion of chimera A-E57L_R58E and Fc (g4PEK)); SEQ ID NO: 302 represents an amino acid sequence of chimera A-E57V_R58T-Fc (g4PEK) (a DcR3 variant obtained by fusion of chimera A-E57V_R58T and Fc (g4PEK)); SEQ ID NO: 304 represents an amino acid sequence of chimera A-E57V_R58E-Fc (g4PEK) (a DcR3 variant obtained by fusion of chimera A-E57V_R58E and Fc (g4PEK)); SEQ ID NO: 314 represents an amino acid sequence of chimera A-Fc (g1S YTE) (a DcR3 variant obtained by fusion of chimera A and Fc (g1S YTE)); SEQ ID NO: 315 represents an amino acid sequence of chimera A-Fc (g1S N434A) (a DcR3 variant obtained by fusion of chimera A and Fc (g1S N434A)); SEQ ID NO: 316 represents an amino acid sequence of chimera A-Fc (g1S LALAGANA) (a DcR3 variant obtained by fusion of chimera A and Fc (g1S LALAGANA)); SEQ ID NO: 317 represents an amino acid sequence of chimera A-E57K-Fc (g1S YTE) (a DcR3 variant obtained by fusion of chimera A-E57K and Fc (g1S YTE)); SEQ ID NO: 318 represents an amino acid sequence of chimera A-E57L-Fc (g1S YTE) (a DcR3 variant obtained by fusion of chimera A-E57L and Fc (g1S YTE)); SEQ ID NO: 319 represents an amino acid sequence of chimera A-E57R-Fc (g1S YTE) (a DcR3 variant obtained by fusion of chimera A-E57R and Fc (g1S YTE)); SEQ ID NO: 320 represents an amino acid sequence of chimera A-E57V-Fc (g1S YTE) (a DcR3 variant obtained by fusion of chimera A-E57V and Fc (g1S YTE)); SEQ ID NO: 321 represents an amino acid sequence of chimera A-E57K_R58D-Fc (g1S YTE) (a DcR3 variant obtained by fusion of chimera A-E57K_R58D and Fc (g1S YTE)); SEQ ID NO: 322 represents an amino acid sequence of chimera A-E57K_R58E-Fc (g1S YTE) (a DcR3 variant obtained by fusion of chimera A-E57K_R58E and Fc (g1S YTE)); SEQ ID NO: 323 represents an amino acid sequence of chimera A-E57R_R58D-Fc (g1S YTE) (a DcR3 variant obtained by fusion of chimera A-E57R_R58D and Fc (g1S YTE)); SEQ ID NO: 324 represents an amino acid sequence of chimera A-E57K-Fc (g1S N434A) (a DcR3 variant obtained by fusion of chimera A-E57K and Fc (g1S N434A)); SEQ ID NO: 325 represents an amino acid sequence of chimera A-E57L-Fc (g1S N434A) (a DcR3 variant obtained by fusion of chimera A-E57L and Fc(g1S N434A)); SEQ ID NO: 326 represents an amino acid sequence of chimera A-E57R-Fc (g1S N434A) (a DcR3 variant obtained by fusion of chimera A-E57R and Fc (g1S N434A)); SEQ ID NO: 327 represents an amino acid sequence of chimera A-E57V-Fc (g1S N434A) (a DcR3 variant obtained by fusion of chimera A-E57V and Fc (g1S N434A)); SEQ ID NO: 328 represents an amino acid sequence of chimera A-E57K_R58D-Fc (g1S N434A) (a DcR3 variant obtained by fusion of chimera A-E57K_R58D and Fc (g1S N434A)); SEQ ID NO: 329 represents an amino acid sequence of chimera A-E57K_R58E-Fc (g1S N434A) (a DcR3 variant obtained by fusion of chimera A-E57K_R58E and Fc (g1S N434A)); SEQ ID NO: 330 represents an amino acid sequence of chimera A-E57R_R58D-Fc (g1S N434A) (a DcR3 variant obtained by fusion of chimera A-E57R_R58D and Fc (g1S N434A)); SEQ ID NO: 331 represents an amino acid sequence of chimera A-E57K-Fc (g1S LALAGANA) (a DcR3 variant obtained by fusion of chimera A-E57K and Fc (g1S LALAGANA)); SEQ ID NO: 332 represents an amino acid sequence of chimera A-E57L-Fc (g1S LALAGANA) (a DcR3 variant obtained by fusion of chimera A-E57L and Fc (g1S LALAGANA)); SEQ ID NO: 333 represents an amino acid sequence of chimera A-E57R-Fc (g1S LALAGANA) (a DcR3 variant obtained by fusion of chimera A-E57R and Fc (g1S LALAGANA)); SEQ ID NO: 334 represents an amino acid sequence of chimera A-E57V-Fc (g1S LALAGANA) (a DcR3 variant obtained by fusion of chimera A-E57V and Fc (g1S LALAGANA)); SEQ ID NO: 335 represents an amino acid sequence of chimera A-E57K_R58D-Fc (g1S LALAGANA) (a DcR3 variant obtained by fusion of chimera A-E57K_R58D and Fc (g1S LALAGANA)); SEQ ID NO: 336 represents an amino acid sequence of chimera A-E57K_R58E-Fc (g1S LALAGANA) (a DcR3 variant obtained by fusion of chimera A-E57K_R58E and Fc (g1S LALAGANA)); and SEQ ID NO: 337 represents an amino acid sequence of chimera A-E57R_R58D-Fc (g1S LALAGANA) (a DcR3 variant obtained by fusion of chimera A-E57R_R58D and Fc (g1S LALAGANA)).

In any of the abovementioned embodiments, the mutation (modification) to be added to the abovementioned amino acid sequence may be a natural mutation or an artificial substitution, deletion, insertion or addition of an amino acid. In any of the abovementioned embodiments, examples of an amino acid sequence obtained by introducing into the abovementioned amino acid sequence, deletion, substitution, insertion or addition of 1 to 30 amino acids include an amino acid sequence obtained by introducing into the abovementioned amino acid sequence, deletion, substitution, insertion or addition of 1 or 2 or more, preferably 2 to 30, more preferably 2 to 10, and particularly preferably 2 to 5 amino acids, or an amino acid sequence having 80% or more, preferably 85% or more, more preferably 90% or more, for example, 93% or more, 95% or more, 97% or more, 98% or more or 99% or more identity to the abovementioned amino acid sequence.

The DcR3 variant of the present invention may be further chemically modified in order to change the biological activity or properties thereof, *in vivo* kinetics such as half-life in blood, or physical or chemical properties such as stability of a protein.

Examples of the chemical modification include polyethylene glycolylation (PEGylation), acetylation, amidation or phosphorylation or the like, and PEGylation is particularly preferable. PEGylation means, for example, binding of one or a plurality of PEG molecules to an amino group of the N-terminus of a protein, or an amino acid residue having a functional group, such as an ε-amino group of Lys, a carboxyl group, a thiol group or a hydroxyl group, at the side chain.

The average molecular weight of the PEG molecule can be used in the range of about 3,000 to about 50,000, but it is not limited thereto.

Examples of the method for binding the PEG molecule to the DcR3 variant include a method introducing, for example, an active group such as a carboxyl group, a formyl (aldehyde) group, an N-hydroxysuccinimide ester group, an amino group, a thiol group or a maleimide group into the PEG terminal part, thus reacting with a functional group such as an amino group, a carboxyl group, a thiol group or a hydroxyl group included at the side chain of the DcR3 variant.

Examples of the signal peptide include, but are not limited to, a signal peptide of human DcR3 consisting of an amino acid sequence at positions 1 to 29 of the amino acid sequence set forth in SEQ ID NO: 2 when the CRD domain at the N-terminal side included in the DcR3 variant is derived from human DcR3. When the CRD domain at the N-terminal side included in the DcR3 variant is derived from human OPG, a signal peptide derived from human OPG consisting of an amino acid sequence at positions 1 to 21 of the amino acid sequence set forth in SEQ ID NO: 14 is exemplified. All of an artificial sequence, a sequence derived from an expression vector, and a sequence derived from another protein suitable for a host cell in which a DcR3 variant is expressed are also included in the signal peptide of the present invention. A DcR3 variant including the signal peptide is a premature polypeptide, and in one embodiment, the signal peptide is cleaved in a maturation process. The DcR3 variant of the present invention also includes a DcR3 variant having a different N-terminus resulting from cleavage of the abovementioned signal peptide at a position different from a predicted site.

### 5. Method for Producing DcR3 Variant

The DcR3 variant of the present invention can be produced by using the method mentioned in Molecular Cloning: A Laboratory Manual, 3rd edition, Cold Spring Harbor Laboratory Press (2001) or the like and expressing a DNA encoding the DcR3 variant in a host cell by, for example, the following method.

Examples of the signal peptide include, but are not limited to, a signal peptide of human DcR3 consisting of an amino acid sequence at positions 1 to 29 set forth in SEQ ID NO: 2 when the CRD domain at the N-terminal side included in the DcR3 variant is derived from human DcR3. When the CRD domain at the N-terminal side included in the DcR3 variant is derived from human OPG, a signal peptide derived from human OPG consisting of an amino acid sequence at positions 1 to 21 set forth in SEQ ID NO: 14 is exemplified. All of an artificial sequence, a sequence derived from an expression vector, and a sequence derived from another protein suitable for a host cell in which a DcR3 variant is expressed can also be used for production of the DcR3 variant of the present invention. The DcR3 variant of the present invention also includes a DcR3 variant having a different N-terminus obtained by cleaving the abovementioned signal peptide at a position different from a predicted site.

The DcR3 variant of the present invention can be obtained by artificially designing based on an amino acid sequence of a cysteine-rich region of wild-type DcR3 before amino acid substitution, an amino acid sequence of a cysteine-rich region of a DcR3 variant, or an amino acid sequence of a DcR3 variant, or can be obtained by analyzing a mutant. As the mutation introduction method, a site-specific mutagenesis method utilizing the PCR method using a primer is preferable (Kunkel et al., Proc. Natl. Acad. Sci. USA, 1985, 82: 488-492). Other examples thereof include a method for totally synthesizing a gene including a mutation introduced thereinto, or a method in which, using a primer including a mutation, PCR is performed for a part before a mutation site and a part after the mutation site separately, and the two fragments are linked by an overlap of a region including the mutation, followed by insertion into a vector by the in-fusion cloning method (Clontech Laboratories, Inc.) or the like.

Examples of the method for identifying a mutation having a target binding mode include a method for obtaining a mutant having a target binding mode by producing a library including mutations randomly introduced thereinto, displaying it on a phage, a yeast or the like, and performing screening using the binding activity. Alternatively, exemplified is a method for obtaining a mutant having a target binding mode by expressing in a host cell a vector including a DNA mutation thereinto for substituting a certain amino acid with a different amino acid and producing the mutant. The DNA encoding the DcR3 variant of the present invention can be synthesized with a DNA synthesizer by designing, from the amino acid sequence of the DcR3 variant of the present invention, a nucleotide sequence encoding it. The DNA can also be isolated by PCR using a cDNA of a human or the like as a template.

By inserting the DNA encoding the DcR3 variant of the present invention obtained above into downstream of a promoter of an appropriate expression vector, a recombinant vector is produced, and the recombinant vector is introduced into a host cell compatible with the expression vector.

As the nucleotide sequence of the DNA encoding the DcR3 variant, a nuceleotide can be substituted so that the codon is optimal for expression in a host, thereby it is possible to improve the production rate of a target DcR3 variant. When the DNA encoding the DcR3 variant mentioned above is produced, it is possible to produce the DNA by adding a DNA encoding a signal peptide of a secretory protein at the 5' end thereof, using the DNA to produce a recombinant vector in the same manner as mentioned above, and introducing the recombinant vector into a host cell to allow secretion of the peptide into a medium. Examples of the signal peptide include a sequence derived from DcR3 or OPG, an artificial sequence, a sequence derived from an expression vector, or a sequence derived from another protein suitable for a host cell.

In one embodiment, examples of the nucleotide sequence of the DNA encoding the amino acid sequence of the DcR3 variant include nucleotide sequences set forth in SEQ ID NO: 25, 27, 29, 31, 33, 35, 37, 39, 41, 43 and 45.

In another embodiment, examples of the nucleotide sequence of the DNA encoding the amino acid sequence of the DcR3 variant include nucleotide sequences set forth in SEQ ID NO: 49, 51, 53, 55, 57, 59, 61, 63, 65, 67, 69, 179, 181, 183, 185, 187, 269, 271, 273, 275, 277, 279, 281, 283 and 285.

In further another embodiment, examples of the nucleotide sequence of the DNA encoding the amino acid sequence of the DcR3 variant include nucleotide sequences set forth in SEQ ID NO: 75, 77, 79, 81, 83, 85, 87, 89, 91, 93, 95, 97, 149, 167, 169, 171, 173, 175, 177, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 287, 289, 291, 293, 295, 297, 299, 301 and 303.

As the expression vector, any expression vector can be used as long as autonomous replication in a host cell used or integration into a chromosome is possible, and an appropriate promoter is contained at a position such that a DNA encoding a polypeptide can be transcribed.

As the host cell, any cell can be used as long as it can express a gene encoding a DcR3 variant, such as a yeast, an insect cell or an animal cell. Preferably a yeast, animal cell or insect cell that is capable of glycosylating a protein is exemplified.

Examples of the yeast include a microorganism belonging to the genus *Saccharomyces,* the genus *Schizosaccharomyces,* the genus *Kluyveromyces,* the genus *Trichosporon,* the genus *Schwanniomyces,* the genus *Pichia,* the genus *Candida* or the like, for example, *Saccharomyces cerevisiae, Schizosaccharomyces pombe, Kluyveromyces lactis, Trichosporon pullulans, Schwanniomyces alluvius,* or *Candida utilis* or the like.

Examples of the insect cell include Sf9 or Sf21, which is an ovary cell of *Spodoptera frugiperda* [Baculovirus Expression Vectors, A Laboratory Manual, W. H. Freeman and Company, New York (1992)], High 5 (manufactured by Invitrogen), which is an ovary cell of *Trichoplusia ni,* or an S2 (Schneider 2) cell (Thermo Scientific) derived from a terminal embryo of *Drosophila melanogaster.*

Examples of the animal cell include a Namalwa cell, which is a human cell, a COS cell, which is a monkey cell, a CHO cell, which is a Chinese hamster cell [Journal of Experimental Medicine, 108, 945 (1958); Proc. Natl. Acad. Sci. USA, 60, 1275 (1968); Genetics, 55, 513 (1968); Chromosoma, 41, 129 (1973); Methods in Cell Science, 18, 115 (1996); Radiation Research, 148, 260 (1997); Proc. Natl. Acad. Sci. USA, 77, 4216 (1980); Proc. Natl. Acad. Sci. USA, 60, 1275 (1968); Cell, 6, 121 (1975); Molecular Cellgenetics, Appendix I, II (pp.883-900)], CHO/DG44, CHO-K1 (ATCC Number :CCL-61), an Freestyle CHO-S cell, an CHO cell deficient in a dihydrofolate reductase gene [Proc. Natl. Acad. Sci. USA, 77, 4216 (1980)], a CHO cell deficient in a 6-fucose transferase gene (WO 2005/035586 A, WO 02/31140 A), a 293 cell, which is a human cell (ATCC Number: CRL-1573), a Freestyle 293F cell, an Expi293 cell (Thermo Scientific), DUkXB11 (ATCC Number: CCL-9096), Pro-5 (ATCC Number: CCL-1781), CHO-S (Life Technologies, Cat#11619), Pro-3, a rat myeloma cell YB2/3HL.P2.G11.16Ag.20 (or also referred to as YB2/0), a mouse myeloma cell NSO, a mouse myeloma cell SP2/0-Ag14, or a Syrian hamster cell BHK, HBT5637 (JP 63-299 A) or the like.

When a yeast is used as a host cell, examples of the expression vector can include YEP13 (ATCC Number: 37115), YEp24 (ATCC37051), YCp50 (ATCC37419), pHS19 or pHS15 or the like.

As the promoter, any promoter may be used as long as it can function in a yeast strain, and examples thereof can include a promoter of a glycolytic gene such as hexosekinase, a PHO5 promoter, a PGK promoter, a GAP promoter, an ADH promoter, a gal 1 promoter, a gal 10 promoter, a heat shock polypeptide promoter, an MFα1 promoter or a CUP 1 promoter or the like.

As the method for introducing a recombinant vector, any method can be used as long as it introduces a DNA into a yeast, and examples thereof can include the electroporation method [Methods Enzymol., 194, 182 (1990)], the spheroplast method [Proc. Natl. Acad. Sci. USA, 75, 1929 (1978)], the lithium acetate method [J. Bacteriology, 153, 163 (1983)], or the method mentioned in Proc. Natl. Acad. Sci. USA, 75, 1929 (1978) or the like.

When an insect cell is used as a host, it is possible to express a peptide by the method mentioned in, for example, Current Protocols in Molecular Biology, Baculovirus Expression Vectors, A Laboratory Manual, W.H. Freeman and Company, New York (1992), Bio/Technology, 6, 47 (1988) or the like.

In other words, a recombinant gene transfer vector and a defective baculovirus genome are co-introduced into an insect cell to obtain a recombinant virus in the insect cell culture supernatant, followed by further infection of an insect cell with the recombinant virus, thereby it is possible to express a peptide.

Examples of the gene transfer vector used in the method can include pVL1392, pVL1393 (manufactured by Becton, Dickinson and Company), or pBlueBac4.5 (manufactured by Invitrogen) or the like.

As the baculovirus, it is possible to use, for example, an *Autographa californica* nuclear polyhedrosis virus, which is a virus which can infect a *Noctuidae* insect or the like.

Examples of the method for co-introducing the abovementioned recombinant gene transfer vector and the abovementioned baculovirus into an insect cell in order to prepare a recombinant virus can include the calcium phosphate method (JP 2-227075 A) or the lipofection method [Proc. Natl. Acad. Sci. USA, 84, 7413 (1987)] or the like.

When an S2 (Schneider 2) cell (Thermo Scientific) corresponding to an insect cell derived from a terminal embryo of *Drosophila melanogaster* is used as a host, it is possible to express a peptide by introducing a gene transfer vector such as pMTBiPV5-HisA (Thermo Scientific) into a host cell by the calcium phosphate method, for example, using the method mentioned in Mol. Biotechnol., 2015, 10: p.914-922 or the like.

When an animal cell is used as a host, examples of the expression vector can include a pCI mammalian expression vector (Promega Corporation), pcDNA3.1(+) (manufactured by Invitrogen), pcDNA I/Amp, pcDNA I, pcDM8 (manufactured by Funakoshi Co., Ltd.), pAGE107 [JP 3-22979 A, Cytotechnology, 3, 133 (1990)], pAS3-3 (JP 2-227075 A), pCDM8 [Nature, 329, 840 (1987)], pREP4 (manufactured by Invitrogen), pAGE103 [J. Biochem., 101, 1307 (1987)], pAGE210, pME18SFL3, or pKANTEX93 (WO 97/10354 A) or the like.

As the promoter, any promoter can be used as long as it functions in an animal cell, and examples thereof can include a promoter of an immediate early (IE) gene of a cytomegalovirus (CMV), an early promoter of SV40, a promoter of a retrovirus, a metallothionein promoter, a heat shock promoter, or an SRa promoter or the like. An enhancer of an IE gene of human CMV may be used together with a promoter.

As the method for introducing a recombinant vector into an animal cell, any method can be used as long as it introduces a DNA into an animal cell, and examples thereof can include the electroporation method [Cytotechnology, 3, 133 (1990)], the calcium phosphate method (JP 2-227075 A), the lipofection method [Proc. Natl. Acad. Sci. USA, 84, 7413 (1987)], or the method mentioned in Virology, 52, 456 (1973) or the like.

By using expression vectors of the DcR3 variant of the present invention obtained by the abovementioned method, or expression vectors obtained by modifying them, it is possible to transiently express the DcR3 variant.

As the host cell into which an expression vector is introduced, any cell can be used as long as it is a host cell that can express the DcR3 variant, and, for example, a COS-7 cell (ATCC Number: CRL1651) is used [Methods in Nucleic Acids Res., CRC Press, 283 (1991)]. For introduction of an expression vector into a COS-7 cell, the DEAE-dextran method [Methods in Nucleic Acids Res., CRC Press, (1991)], or the lipofection method [Proc. Natl. Acad. Sci. USA, 84, 7413 (1987)] or the like is used.

When a CHO-S cell or an Expi293 cell (Thermo Scientific) is used, the lipofection method [Proc. Natl. Acad. Sci. USA, 84, 7413 (1987)] or the like is used for introduction of an expression vector.

By using expression vectors of the DcR3 variant of the present invention obtained by the abovementioned method, or expression vectors obtained by modifying them, it is possible to obtain a transformant stably expressing the DcR3 variant.

After introduction of an expression vector, the transformant stably expressing a gene recombinant antibody is selected by culturing in a medium for culture of animal cells containing a drug such as G418 sulfate (JP 2-257891 A).

When the transformant is a transformant obtained using a eukaryote such as a yeast as a host, as the culture medium of the transformant, any of a natural medium or a synthetic medium can be used as long as the medium contains a carbon source, a nitrogen source and/or inorganic salts that can be assimilated by the transformant, and the medium can efficiently culture the transformant.

As the carbon source, any carbon source may be used as long as the transformant can assimilate it, and it is possible to use, for example, carbohydrates such as glucose, fructose, sucrose, molasses containing these, starch or starch hydrolysates; organic acids such as acetic acid or propionic acid; alcohols such as ethanol or propanol; or the like.

As the nitrogen source, it is possible to use, for example, ammonium salts of inorganic acids or organic acids such as ammonia, ammonium chloride, ammonium sulfate, ammonium acetate and/or ammonium phosphate; other nitrogen-containing compounds, as well as peptone, meat extracts, yeast extracts, corn steep liquor, casein hydrolysates, soybean meal and soybean meal hydrolysates, various fermentative bacterial cells and digests thereof or the like.

As the inorganic salt, it is possible to use, for example, monopotassium phosphate, dipotassium phosphate, magnesium phosphate, magnesium sulfate, sodium chloride, ferrous sulfate, manganese sulfate, copper sulfate and/or calcium carbonate or the like.

Culture is preferably performed under aerobic conditions such as shaking culture or deep aeration stirring culture. The culture temperature is preferably 15 to 40°C, and the culture time is preferably usually 16 hours to 7 days. The pH during culture is preferably kept at 3.0 to 9.0. The pH can be adjusted using an inorganic or organic acid, an alkali solution, urea, calcium carbonate or ammonia or the like.

During culture, if necessary, an antibiotic such as ampicillin or tetracycline may be added to a medium.

As the culture medium of the transformant obtained using an insect cell as a host, it is possible to use a TNM-FH medium (manufactured by Becton, Dickinson and Company), an Sf-900 II SFM medium (manufactured by Invitrogen), ExCell400, ExCell405(both are manufactured by JRH Biosciences, Inc.), a Grace's insect medium [Nature, 195, 788 (1962)], or Schneider's Medium (Thermo Fisher Scientific, Inc.), which is generally used, or the like.

Regarding the culture of the transformant obtained using an insect cell as a host, usually it is preferable that the pH is 6 to 7 and the temperature is 25 to 30°C, and the culture is preferably performed for 1 to 5 days. During culture, if necessary, an antibiotic such as gentamicin may be added to a medium.

As the medium for animal cell culture, used is an RPMI 1640 medium (manufactured by Invitrogen), a GIT medium (manufactured by NIHON PHARMACEUTICAL CO., LTD.), an EX-CELL301 medium (manufactured by JRH Biosciences, Inc.), an EX-CELL325 PF CHO serum-Free medium (Sigma-Aldrich Co., LLC), an IMDM medium (manufactured by Invitrogen), a Hybridoma-SFM medium (manufactured by Invitrogen), an Eagle's minimal essential medium (MEM) [Science, 122, 501 (1952)], a Dulbecco's modified Eagle's medium [Virology, 8, 396 (1959)], a 199 medium [Proceeding of the Society for the Biological Medicine, 73, 1 (1950)] or a medium obtained by adding various additives such as FBS to these media or the like.

Culture of an animal cell is usually preferably performed in the presence of 5% CO₂, and it is preferable that the pH is 6 to 8 and the temperature is 30 to 40°C, and the culture is preferably performed for 1 to 7 days. During culture, if necessary, an antibiotic such as kanamycin or penicillin may be added to a medium.

By culturing the transformant thus obtained in a medium, the DcR3 variant is expressed and accumulated in a culture supernatant. The transformant can increase the expression amount of the DcR3 variant using a DHFR amplification system (JP 2-257891 A) or the like.

In the manner as mentioned above, by generating and accumulating the DcR3 variant according to the present invention in a culture, and collecting from the culture, it is possible to produce the DcR3 variant according to the present invention.

In order to isolate and purify the peptide produced by the transformant, it is possible to use a usual protein isolation and purification method.

For example, when the DcR3 variant of the present invention is extracellularly secreted, the DcR3 variant can be collected from the culture supernatant. In other words, the culture is subjected to a method such as centrifugation to obtain a culture supernatant, and from the culture supernatant, a usual protein isolation and purification method, namely, a method such as a solvent extraction method, a salting-out method with ammonium sulfate or the like, a desalting method, a precipitation method with an organic solvent, an anion exchange chromatography method using a resin such as diethylaminoethyl (DEAE)-Sepharose or DIAION HPA-75 (Mitsubishi Chemical Corporation), a cation exchange chromatography method using a resin such as S-Sepharose FF (GE Healthcare), a hydrophobic chromatography method using a resin such as Butyl Sepharose or Phenyl Sepharose, a gel filtration method using a molecular sieve, an affinity chromatography method, a chromatofocusing method, or an electrophoresis method such as an isoelectric focusing is used alone or in combination, thereby it is possible to obtain a purified product.

For example, when the DcR3 variant of the present invention has Fc of an immunoglobulin that can bind to Protein G or Protein A, a Protein G chromatography method or a Protein A chromatography method in which Protein G or Protein A is bound to a carrier as an affinity ligand can be used as an affinity chromatography method [Monoclonal Antibodies-Principles and practice, Third edition, Academic Press (1996), Antibodies-A Laboratory Manual, Cold Spring Harbor Laboratory (1988)]. It is also possible to combine methods used for protein purification such as gel filtration, ion exchange chromatography and ultrafiltration.

From the DcR3 variant that could be obtained by the abovementioned method, by using a conventional chemical modification method, it is also possible to obtain a DcR3 variant in which a peptide, a glycan or PEG or the like is further chemically modified.

### 6. Method for Evaluating Biological Activity, Physical Property and Kinetics of DcR3 Variant

Examples of the DcR3 variant of the present invention preferably include a DcR3 variant having neutralizing activity to at least one or more of LIGHT, TL1A and FasL, a DcR3 variant having neutralizing activity to all of LIGHT, TL1A and FasL, a DcR3 variant having no neutralizing activity to FasL and having neutralizing activity to one of LIGHT and TL1A, a DcR3 variant having no neutralizing activity to FasL and having neutralizing activity to both of LIGHT and TL1A, or the like.

It is possible to measure the biological activity such as neutralizing activity, the physical property and the *in vivo* kinetics of the DcR3 variant of the present invention by using the following methods.

### (1) Preparation of Ligand

The origin of LIGHT, TL1A and FasL used in the present invention is not limited, but LIGHT, TL1A and FasL derived from eukaryotes are exemplified. Examples of LIGHT, TL1A and FasL derived from eukaryotes include LIGHT, TL1A and FasL derived from yeasts, insects or mammals. Preferably examples thereof include LIGHT, TL1A and FasL derived from primates including humans, or rodents including mice or the like.

LIGHT, TL1A or FasL or a cell expressing the ligand can be obtained by introducing an expression vector including a cDNA encoding the full length or partial length of LIGHT, TL1A or FasL into an appropriate host cell such as *Escherichia coli*, a yeast, an insect cell or an animal cell or the like. It can also be obtained by purifying the ligand from various human cultured cells or human tissues or the like expressing a large amount of LIGHT, TL1A or FasL. The cultured cell or the tissue can also be used as a ligand as it is. Furthermore, it is also possible to prepare a synthetic peptide having a partial sequence of LIGHT, TL1A or FasL by a chemical synthesis method such as the Fmoc method or the tBoc method.

LIGHT, TL1A and FasL can be produced by using the method mentioned in Molecular Cloning, A Laboratory Manual, Second Edition, Cold Spring Harbor Laboratory Press (1989) or the like and introducing a DNA encoding LIGHT, TL1A or FasL into a host cell to express by, for example, the following method.

Soluble LIGHT, TL1A and FasL are generated by being expressed on the cell membrane as membrane-bound LIGHT, membrane-bound TL1A and membrane-bound FasL, respectively, followed by shedding of the extracellular region by protease. Although a cleavage site has been identified for each ligand, when a recombinant of a soluble ligand is produced, any sequence in the vicinity of the cleavage site can be included. For example, a soluble LIGHT recombinant can be produced from a region at positions 66 to 240, a region at positions 74 to 240, a region at positions 83 to 240 or the like from the N-terminus in the amino acid sequence of membrane-bound LIGHT. A soluble TL1A recombinant can be produced from a region at positions 72 to 251 or the like from the N-terminus in the amino acid sequence of membrane-bound TL1A. A soluble FasL recombinant can be produced from a region at positions 130 to 281, a region at positions 134 to 281 or the like from the N-terminus in the amino acid sequence of membrane-bound FasL.

A recombinant of a soluble ligand can be produced by adding a His tag or a FLAG tag or the like to the N-terminus of the abovementioned region, and obtained by affinity purification. Examples of the amino acid sequences of the soluble recombinants of LIGHT, TL1A and FasL include amino acid sequences set forth in SEQ ID NO: 114, 116, 118, 120, 122, 124, 126, 128, 130 and 306. Examples of the nucleotide sequences of the DNAs encoding the amino acid sequences of the soluble recombinants of LIGHT, TL1A and FasL include nucleotide sequences set forth in SEQ ID NO: 113, 115, 117, 119, 121, 123, 125, 127, 129 and 305.

Functional LIGHT, TL1A or FasL can form a homotrimer. Soluble LIGHT, TL1A or FasL prepared by any of the abovementioned methods is analyzed by SEC-MALS, and the molecular weight can be calculated. SEC-MALS is an analysis method for calculating a molecular weight for each peak detected at a wavelength of 215 nm separated by size exclusion chromatography (SEC)-HPLC, using a maximum value of scattering intensity detected with multi angle light scattering (MALS). Examples of the HPLC system include Prominence manufactured by Shimadzu Corporation; examples of the SEC column include TSKgel manufactured by Tosoh Corporation; and examples of the MALS detector include miniDAWN TREOS manufactured by Wyatt Technology Corporation or the like. Examples of the method for isolating a homotrimer from a crude purified product that forms those other than trimers include SEC purification or the like. Examples of the SEC purification method include a method in which a crude purified product is fractionated according to the molecular weight size using AKTApurifier manufactured by GE Healthcare as an HPLC system and Superdex 200 Increase 10/300 GL manufactured by GE Healthcare as a SEC column, and only a molecular weight fraction of a homotrimer is collected, or the like.

### (2) Evaluation of Binding Activity of DcR3 Variant Including Human Fc Region

Examples of the method for measuring the binding activity of the DcR3 variant to soluble LIGHT, soluble TL1A or soluble FasL include a binding assay by enzyme-linked immunosorbent assay (ELISA), a kinetics analysis by Biacore or the like. As the ligand, a transgenic cell or a recombinant protein obtained by introducing an expression vector including a DNA sequence encoding an extracellular domain of each ligand of LIGHT, TL1A or FasL into *Escherichia coli,* a yeast, an insect cell or an animal cell or the like, and serum, plasma or a culture supernatant or the like including a ligand obtained from a human tissue or a human cell are used.

In the case of ELISA, for example, an anti-human IgG antibody is immobilized to a 96 well plate or the like, and the DcR3 variant is reacted, and then each ligand is dispensed to bind. After washing, an unlabeled anti-ligand antibody or a receptor-Fc fusion of each ligand or the like is reacted, and then an anti-Fc antibody or the like labeled with biotin, an enzyme or a chemiluminescence substance or the like is reacted, or a labeled anti-ligand antibody or a receptor-Fc fusion of each ligand or the like is reacted, followed by detection according to the labeled substance, thereby it is possible to measure the binding of the DcR3 variant to a ligand. In the case of Biacore, for example, using Biacore T100 or Biacore T200, kinetics in the binding between each ligand and the DcR3 variant is measured, and the results are analyzed with analysis software included with the apparatus. Specifically, after an anti-human IgG antibody is immobilized on a sensor chip CM5 by an amine coupling method, the DcR3 variant is injected and an appropriate amount is bound on the sensor chip, and further a plurality of ligands having known concentrations are injected, thereby binding and dissociation are measured. For the data thus obtained, a kinetics analysis is performed with a 1:1 binding model using software included with the apparatus, and various parameters are obtained. Alternatively, after each ligand is immobilized on a sensor chip by, for example, an amine coupling method, a plurality of DcR3 variants having known concentrations are injected, thereby binding and dissociation are measured. For the data thus obtained, a kinetics analysis is performed with a bivalent binding model using software included with the apparatus, and various parameters are obtained. Alternatively, to sensor chip Protein A obtained by immobilizing a MabSelect Sure ligand, which is an IgG binding domain variant protein of Protein A, on a sensor chip, the DcR3 variant is injected and an appropriate amount is bound on the sensor chip, and further a plurality of ligands having known concentrations are injected, thereby binding and dissociation are measured. For the data thus obtained, a kinetics analysis is performed with a 1:1 binding model using software included with the apparatus, and various parameters are obtained.

The binding activity of the DcR3 variant to membrane-bound LIGHT, membrane-bound TL1A or membrane-bound FasL can be measured by flow cytometry, for example, using a transgenic cell obtained by introducing an expression vector including a DNA sequence encoding the full length of each ligand of LIGHT, TL1A and FasL into an animal cell or the like, or a cell such as PBMC or HUVEC stimulated for induction of expression of the membrane-bound ligand by adding appropriate stimulation such as stimulation with a mitogen such as PHA-L, PMA or ionomycin or anti-CD3 antibody and anti-CD28 antibody stimulation, cytokine, IgG or immune complex stimulation. Specifically, measurement can be performed by reacting the DcR3 variant with a cell expressing a membrane-bound ligand, followed by reaction with a fluorescently labeled anti-Fc antibody or the like, or reacting the DcR3 variant labeled with biotin or a fluorescent dye or the like, and after washing, detecting the fluorescence intensity according to the labeled substance using a flow cytometer.

Examples of the DcR3 variant of the present invention measured by the abovementioned method include a DcR3 variant having binding activity to at least one or more of LIGHT, TL1A and FasL, a DcR3 variant having binding activity to all of LIGHT, TL1A and FasL, a DcR3 variant having no binding activity to FasL and having binding activity to one of LIGHT and TL1A, or a DcR3 variant having no binding activity to FasL and having binding activity to both of LIGHT and TL1A.

### (3) Evaluation of Neutralizing Activity of DcR3 Variant

Examples of the method for measuring the neutralizing activity of the DcR3 variant include a method for measuring the binding of LIGHT, TL1A or FasL with a corresponding receptor in a solution supplemented with the DcR3 variant, or a method for measuring cell functions such as cytokine production or cell proliferation by adding a corresponding ligand to a cell expressing a receptor of LIGHT, TL1A or FasL in a medium supplemented with the DcR3 variant.

The inhibitory activity to the binding of human LIGHT with a corresponding receptor can be measured by the following method using, for example, the method mentioned in US 8,974,787 B2 or the like. Measurement is performed by, in a reaction solution supplemented with the DcR3 variant, reacting HVEM or LTβR labeled with biotin, a fluorescent dye or the like with a cell expressing membrane-bound LIGHT, and after washing, detecting the fluorescence intensity according to the labeled substance using a flow cytometer. Alternatively, measurement is performed by, in a reaction solution supplemented with the DcR3 variant, reacting LIGHT labeled with biotin, a fluorescent dye or the like with a cell expressing HVEM or LTβR, and after washing, detecting the fluorescence intensity according to the labeled substance using a flow cytometer. The inhibitory activity of the DcR3 variant to the binding of LIGHT with a corresponding receptor is confirmed by a decrease in the fluorescence intensity in comparison with the case where no DcR3 variant is added.

Using the same method as mentioned above, it is also possible to measure the inhibitory activity to the binding of TL1A or FasL with a corresponding receptor.

As the DcR3 variant of the present invention, preferred is a DcR3 variant having inhibitory activity to at least one or more of the bindings of LIGHT, TL1A and FasL with corresponding receptors, a DcR3 variant having inhibitory activity to all of the bindings of LIGHT, TL1A and FasL with corresponding receptors, a DcR3 variant having no inhibitory activity to the binding of FasL with a corresponding receptor and having inhibitory activity to one of the bindings of LIGHT and TL1A with corresponding receptors, or a DcR3 variant having no inhibitory activity to the binding of FasL with a corresponding receptor and having inhibitory activity to the bindings of LIGHT and TL1A with corresponding receptors. In the present invention, the expression of "having no inhibitory activity to the binding of a ligand with a corresponding receptor" is used as a meaning including the case where the inhibitory activity is significantly lowered than that of wild-type DcR3.

The inhibitory activity to a cell function initiated by addition of LIGHT can be measured as follows using, for example, the method mentioned in US 8,974,787 B2 or the like. In a medium supplemented with the DcR3 variant, using a cell such as HT-29 (ATCC Number: HTB-38) which was confirmed to express HVEM or LTβR corresponding to a LIGHT receptor, LIGHT-dependent production of chemokine such as IL-8, CCL5 or CCL20 is measured by the ELISA method, alphaLISA (PerkinElmer, Inc.) or CBA assay (BD Biosciences) or the like using a culture supernatant. Alternatively, using a cell such as an intestinal myofibroblast (Lonza K.K.) stimulated with IFN-γ, soluble or membrane-bound LIGHT-dependent production of a chemokine such as CXCL-10 is also measured using the same method. Alternatively, using, instead of soluble or membrane-bound LIGHT, LIGHT whose expression was induced from PBMC or a T cell stimulated with an anti-CD3 antibody and an anti-CD28 antibody or PMA and ionomycin or the like, measurement can be performed using the same method. The inhibitory activity of the DcR3 variant to a cell function initiated by addition of soluble or membrane-bound LIGHT is confirmed by a decrease in the production amount of the chemokine in comparison with the case where no DcR3 variant is added. Using, for example, the method mentioned in US 8,974,787 B2 or the like, it is possible to evaluate *in vivo* the improvement in survival, body weight, a pathological condition, pathology, engraftment of a human cell or the like by administration of the DcR3 variant in an acute graft versus host disease (GVHD) model in which allogeneic transplantation of human PBMC was performed to an immunodeficient mouse.

The inhibitory activity to a cell function initiated by addition of TL1A can be measured as follows using, for example, the method mentioned in Mucosal Immunology, 2015, 8: p.545-558 or the like. In a medium supplemented with the DcR3 variant, using blood, PBMC, a pan T cell, a CD4-positive T cell or a memory CD4-positive T cell or the like derived from primates such as humans or rodents such as mice, the cell is stimulated with each cytokine cocktail of IL-12, IL-18 and TL1A, or IL-12, IL-18, IL-15 and TL1A, and TL1A-dependent production of a cytokine such as IFN-γ, IL-6, GM-CSF, TNF-α, IL-5, IL-13, IL-17 or IL-22 or the like is measured by the ELISA method, alphaLISA or CBA assay or the like using a culture supernatant. Alternatively, using, for example, the method mentioned in Immunity, 2002, 16: p.479-492, in a medium supplemented with the DcR3 variant, a pan T cell, a CD4-positive T cell or a memory CD4-positive T cell or the like derived from primates such as humans or rodents such as mice is stimulated with an anti-CD3 antibody and an anti-CD28 antibody, and TL1A-dependent production of IFN-γ and IL-2 is measured by the same method as mentioned above. Alternatively, using a cell line forcedly expressing membrane-bound TL1A instead of soluble TL1A, measurement can also be performed by the same method. Alternatively, using TL1A whose expression was induced from PBMC or a monocyte stimulated with IgG or an immune complex or the like, measurement can also be performed by the same method. The inhibitory activity of the DcR3 variant to a cell function initiated by addition of TL1A is confirmed by a decrease in the cytokine or inhibition of cell proliferation in comparison with the case where no DcR3 variant is added. Furthermore, it is possible to evaluate *in vivo* the improvement in survival, body weight, a pathological condition and pathology and the like by administration of the DcR3 variant in, for example, the 2,4,6-trinitrobenzenesulfonic acid (TNBS)-induced colitis model mentioned in Mucosal Immunology, 2011, 4: p.172-185 or the dextran sodium sulfate (DSS)-induced colitis model mentioned in Mucosal Immunology, 2014, 7: p.1492-1503. In addition, it is possible to evaluate the effect on improving a pathological condition in a TL1A-dependent inflammation, allergic disease or autoimmune disease model in rodents such as mice.

The inhibitory activity to a cell function initiated by addition of FasL can be measured as follows using, for example, the method mentioned in J. Rheumatol., 2013, 40: p.1316-1326 or the like. In a medium supplemented with the DcR3 variant, using Jurkat cells (DSMZ Number: ACC 282), apoptosis dependent on soluble FasL or soluble FasL cross-linked with an antibody is measured by Annexin V/Propium Iodide staining or BrdU uptake or ATP amount of a viable cell or the like. Alternatively, using a cell line forcedly expressing membrane-bound FasL instead of soluble FasL, measurement can also be performed by the same method. Alternatively, using FasL induced from PBMC or a T cell stimulated, measurement can also be performed by the same method. The inhibitory activity of the DcR3 variant to a cell function initiated by addition of FasL is confirmed by a decrease in the apoptosis in comparison with the case in which no DcR3 variant is added.

Examples of the DcR3 variant of the present invention measured by the abovementioned method include a DcR3 variant having inhibitory activity to at least one or more of cell functions initiated by addition of LIGHT, TL1A and FasL, a DcR3 variant having inhibitory activity to all of cell functions initiated by addition of LIGHT, TL1A and FasL, a DcR3 variant having no inhibitory activity to a cell function initiated by addition of FasL and having inhibitory activity to one or more of cell functions initiated by addition of LIGHT and TL1A, or a DcR3 variant having no inhibitory activity to a cell function initiated by addition of FasL and having inhibitory activity to cell functions initiated by addition of LIGHT and TL1A. In the present invention, the expression of "having no inhibitory activity to a cell function initiated by addition of a ligand" is used as a meaning including the case where the inhibitory activity is significantly lowered than that of wild-type DcR3.

### (4) Evaluation of OPG Ligand Reactivity of DcR3 Variant

One characteristic of the DcR3 variant of the present invention is that any of parts of or all of CRD1, CRD4 and CRD3 not involved in ligand binding are sequences derived from OPG.

The fact that the DcR3 variant having no binding activity to RANKL and TRAIL, which are OPG ligands, can be evaluated by, for example, the same method as the method for measuring the binding activity to LIGHT, TL1A and FasL mentioned above or the like.

One characteristic of the DcR3 variant of the present invention is having no neutralizing activity also to RANKL and TRAIL.

The neutralizing activity of the DcR3 variant to RANKL can be evaluated using, for example, tartrate-resistant acid phosphatase (TRAP) activity measurement, which is a differentiation assay of an osteoclast precursor by RANKL stimulation, mentioned in J. Immunol., 2012, 189: p.245-252 or the like.

Regarding the neutralizing activity of the DcR3 variant to TRAIL, for example, apoptosis induction by soluble TRAIL or cross-linked soluble TRAIL in a human cancer cell line expressing DR4 or DR5 can be evaluated by the same method as the method for measuring the neutralizing activity to FasL mentioned above or the like.

### (5) Evaluation of Kinetics of DcR3 Variant

As the DcR3 variant of the present invention, a DcR3 variant in which the binding to heparan sulfate included in heparan sulfate proteoglycan (HSPG) on the cell membrane has been lowered or eliminated is preferable, and a DcR3 variant having no heparan sulfate-binding domain (HBD) is more preferable.

The presence or absence of the binding to heparan sulfate on the cell membrane via HBD can be measured as follows using, for example, the method mentioned in J. Immunol., 2006, 176: p.173-180 or the like. Measurement can be performed by reacting wild-type DcR3 or the DcR3 variant with any cell, for example, a CHO cell or a cell of a human cell line, a vascular endothelial cell, a hepatocyte or a blood cell or the like, followed by reaction with a fluorescently labeled detection antibody or the like, or reacting wild-type DcR3 or the DcR3 variant labeled with biotin or a fluorescent dye or the like, and after washing, detecting the fluorescence intensity according to the labeled substance using a flow cytometer (FCM). Lowering or elimination of the binding to the cell membrane due to deficiency of HBD of DcR3 can be examined by a decrease in the fluorescence intensity by FCM.

Examples of the method for evaluating whether the abovementioned binding on the cell membrane is a binding via HBD of DcR3 or not include a method in which GAG such as heparin, heparan sulfate or the like as an inhibitor is added during a reaction of wild-type DcR3 or the DcR3 variant, or a cell is treated with an enzyme such as heparinase or trypsin or the like in advance, and then the wild-type DcR3 or the DcR3 variant is reacted, using, for example, the method mentioned in J. Immunol., 2006, 176: p.173-180 or the like.

One characteristic of the DcR3 variant of the present invention is that *in vivo* kinetics is improved compared to that of wild-type DcR3 since elimination via heparan sulfate is relieved *in vivo.* In the present invention, "*in vivo* kinetics is improved" means that the half-life in blood is longer compared to that of wild-type DcR3 or the value of the area under the concentration-time curve (AUC) to infinity is higher compared to that of wild-type DcR3.

As a target for comparison, it is also possible to use a molecule including CRD of wild-type DcR3 instead of wild-type DcR3. Specific examples thereof include DcR3 FL-Fc (amino acid sequence: SEQ ID NO: 100, nucleotide sequence of DNA: SEQ ID NO: 99), DcR3 FL-FLAG (amino acid sequence: SEQ ID NO: 104, nucleotide sequence of DNA: SEQ ID NO: 103), S195-Fc obtained by fusing CRD of wild-type DcR3 and Fc to each other (amino acid sequence: SEQ ID NO: 102, nucleotide sequence of DNA: SEQ ID NO: 101), and R128Q-Fc obtained by fusing Fc to the C-terminus of full-length DcR3 having one amino acid mutation in HBD (amino acid sequence: SEQ ID NO: 112, nucleotide sequence of DNA: SEQ ID NO: 111) [US 6,835,814 B1, US 6,965,01 B1] and the like. When a molecule including CRD of these wild-type DcR3 as a target for comparison, it is also referred to as wild-type DcR3 control.

The time course of blood concentration of the DcR3 variant in rodents such as mice or non-human primates such as cynomolgus monkeys can be measured by intravenously or subcutaneously administering an appropriately-selected dose, collecting blood at an appropriately-selected time, and using a detection system of DcR3 or human Fc. Kinetic parameters such as half-life in blood and AUC can be calculated by using a method such as a moment analysis from the time course of blood concentration using, for example, the method mentioned in Xenobiotic Metabolism and Disposition, 1999, 14: p.286-293.

Since it has been known that *in vivo* kinetics is improved if many sialic acids are added to the terminus of an N-linked glycan (J. Pharm. Sci., 2015, 104: p.1866-1884), it is preferable that the DcR3 variant of the present invention having an N-linked glycan also has many sialic acids added. The number of additions of sialic acids per protein molecule can be calculated by, for example, separating sialic acids labeled using a reagent kit for fluorescent labeling of sialic acid (Takara Bio Inc.) or the like by reversed-phase HPLC, and comparing with a standard curve of sialic acid.

### (6) Evaluation of Physical Property of DcR3 Variant

One characteristic of the DcR3 variant of the present invention is that, when expressed in mammalian cells, isolated or purified, the content of aggregates is lower than that of wild-type DcR3.

Whether aggregates are generated during protein expression and/or secretion can be determined by, for example, collecting host cells into which a recombinant vector carrying the DcR3 variant have been introduced and/or a culture supernatant, and examining an approximate molecular weight under non-reducing conditions by immunoblotting using an anti-DcR3 antibody, or, in the case of an Fc fusion or a tag adduct such as His or FLAG, an antibody against it. When proteins are aggregated during protein expression and/or secretion, one or a plurality of band(s) of a larger size than the expected molecular weight is/are detected.

A method for calculating the molecular weight of a protein from the amino acid sequence is exemplified. When a more accurate molecular weight is calculated, an analysis method by SEC-MALS is exemplified. Regarding each peak detected at a wavelength of 215 nm separated by size exclusion chromatography (SEC)-HPLC, the molecular weight can be calculated using a maximum value of scattering intensity detected with multi angle light scattering (MALS). Examples of the HPLC system include Prominence manufactured by Shimadzu Corporation; examples of the SEC column include TSKgel manufactured by Tosoh Corporation; and examples of the MALS detector include miniDAWN TREOS manufactured by Wyatt Technology Corporation or the like.

Whether isolated or purified proteins are aggregated or not can be examined by subjecting the proteins to SDS-PAGE under non-reducing conditions, followed by detection by CBB staining or the like, or by immunoblotting using the same method as mentioned above. Regarding the content of aggregates, the proportion of each peak can be calculated from the area of each peak detected at a wavelength of 215 nm separated by gel filtration chromatography (SEC) using HPLC. Examples of the SEC column include TSKgel G3000SW manufactured by Tosoh Corporation or ACQUITY UPLC Protein BEH SEC manufactured by Waters K.K. or the like. When analysis is performed using the method mentioned above, a preferable content of aggregates is 0 to 60%, a more preferable content of aggregates is 0 to 40%, a still more preferable content of aggregates is 0 to 30%, a yet more preferable content of aggregates is 0 to 20%, and most preferable content of aggregates is 0 to 10%.

The DcR3 variant of the present invention is characterized by having lower hydrophobicity than that of wild-type DcR3. The hydrophobicity of a protein can be measured using a hydrophobic interaction chromatography (HIC) column that interacts with a hydrophobic region existing on the surface of the protein. Examples of the HIC column include TSKgel Butyl-NPR manufactured by Tosoh Corporation or the like.

The DcR3 variant of the present invention is characterized by having more improved thermostability than that of wild-type DcR3. Examples of the method for measuring the thermostability of a protein include calorimetry such as differential scanning calorimetry (DSC); spectroscopy that obtains an intrinsic fluorescence spectrum or a circular dichroism (CD) spectrum during thermal denaturation or chemical denaturation; differential scanning fluorimetry (DSF) that detects exposure of a hydrophobic region existing in the inside of a protein associated with a rise in temperature using a fluorescent dye (Sypro Orange or the like); or the like [J Am Chem Soc, 2009, 131: p.3794-3795].

Regarding DSF that evaluates the thermostability of a protein, it is possible to measure the fluorescence amount at each temperature using, for example, the method mentioned in J. Pharm. Sci., 2013, 102: p.2471-2483 or the like. It is possible to calculate the thermal unfolding transition midpoints (Tm) value by drawing a melting curve using software such as CFX manager manufactured by Bio-Rad Laboratories, Inc. Similarly, regarding DSC that evaluates the thermostability of a protein, it is possible to calculate the heat capacity and the Tm value at each temperature using, for example, the method mentioned in J. Pharm. Sci., 2012, 101: p.955-964 or the like.

### 7. DcR3 Variant Composition

Examples of the DcR3 variant composition of the present invention include a composition including a DcR3 variant molecule(s). Examples of the DcR3 variant composition of the present invention include a composition including a plurality of DcR3 variant molecules that consist of equivalent primary amino acid sequences and can occur by posttranslational modification such as oxidation/reduction reaction, glycosylation reaction or sulfation addition reaction in the amino acid sequence. The DcR3 variant of the present invention may include, for example, a DcR3 variant having one or more complex N-glycoside-linked glycans, and a DcR3 variant not having a complex N-glycoside-linked glycan. The proportion of the DcR3 variants having one or more complex N-glycoside-linked glycans is, for example, preferably 70 to 100%, more preferably 90 to 99%, and particularly preferably 95 to 98%, relative to the total number of the DcR3 variants of the present invention.

### 8. Pharmaceutical Composition Including DcR3 Variant

One embodiment of the present invention is a composition including an effective dose of the DcR3 variant of the present invention. The composition including the DcR3 variant of the present invention can be used as an active ingredient of a prophylactic or therapeutic agent for an autoimmune disease, an inflammatory disease or an allergic disease, including a mucosal disease. In other words, it is possible to prevent or treat an autoimmune disease, an inflammatory disease or an allergic disease by administering the pharmaceutical composition including the DcR3 variant of the present invention to a patient in need of prevention or treatment of an autoimmune disease, an inflammatory disease or an allergic disease.

Examples of the pathological condition or the disease for which the composition of the present invention is used include, but are not limited to, an inflammatory disease, an autoimmune disease or an allergic disease or the like, such as inflammatory bowel disease (IBD), systemic lupus erythematosus, psoriasis, chronic graft versus host disease, acute graft versus host disease, Crohn's disease, ulcerative colitis, inflammatory bowel disease, multiple sclerosis, celiac disease, idiopathic thrombotic thrombocytopenic purpura, myasthenia gravis, Sjogren's syndrome, scleroderma, asthma, uveitis, epidermal hyperplasia, alopecia areata, Behcet's disease, Takayasu's arteritis, cartilaginous inflammation, bone degradation, arthritis, juvenile arthritis, juvenile rheumatoid arthritis, pauciarticular juvenile rheumatoid arthritis, polyarticular juvenile rheumatoid arthritis, systemic onset juvenile rheumatoid arthritis, juvenile ankylosing spondylitis, juvenile enteropathic arthritis, juvenile reactive arthritis, juvenile Reiter's syndrome, seronegative enthesopathy and arthropathy syndrome (SEA syndrome), juvenile dermatomyositis, juvenile psoriatic arthritis, juvenile scleroderma, juvenile systemic lupus erythematosus, juvenile vasculitis, pauciarticular rheumatoid arthritis, polyarticular rheumatoid arthritis, systemic onset rheumatoid arthritis, ankylosing spondylitis, enteropathic arthritis, reactive arthritis, Reiter's syndrome, dermatomyositis, psoriatic arthritis, vasculitis, myositis, polymyositis, dermatomyositis, osteoarthritis, polyarteritis nodosa, Wegener's granulomatosis, arteritis, polymyalgia rheumatica, sarcoidosis, sclerosis, primary biliary cirrhosis, sclerosing cholangitis, dermatitis, atopic dermatitis, atherosclerosis, Still's disease, chronic obstructive pulmonary disease, Guillain-Barre syndrome, type 1 diabetes mellitus, Graves' disease, Addison's disease, Raynaud's phenomenon, autoimmune hepatitis or Wiskott-Aldrich syndrome or the like.

The pharmaceutical composition containing the DcR3 variant of the present invention can contain, as an active ingredient, the DcR3 variant or a mixture of the DcR3 variant with any other active ingredients for treatment. These pharmaceutical formulations are produced by any method well known in the technical field of pharmaceutics by mixing an active ingredient with one or more pharmacologically acceptable carriers.

The content of the DcR3 variant of the present invention in the pharmaceutical composition varies depending on the dosage form, the pharmacologically acceptable dose of the DcR3 variant of the present invention or the like, and the content is, for example, about 0.01 to 100% by weight. The content of the pharmacologically acceptable carrier in the pharmaceutical formulation varies depending on the dosage form, the pharmacologically acceptable dose of the DcR3 variant of the present invention or the like, and the content is, for example, 0 to 99.9% by weight.

Regarding the route of administration, it is desirable to use the most effective one for treatment, and examples thereof include oral administration, or parenteral administration such as intravenous, subcutaneous, intraoral, intratracheal, intrarectal, intramuscular or intraperitoneal administration.

The administration form includes tablets, powders, granules, syrups or injections or the like.

As an appropriate formulation for oral administration, for example, a liquid preparation such as a syrup can be produced using water; saccharides such as sucrose, sorbit or fructose; glycols such as polyethylene glycol or propylene glycol; oils such as sesame oil, olive oil or soybean oil; preservatives such as p-hydroxybenzoic acid esters; flavors such as strawberry flavor or peppermint; or the like. A tablet, a powder and a granule and the like can be produced using excipients such as lactose, glucose, sucrose or mannit; disintegrators such as starch or sodium alginate; lubricants such as magnesium stearate or talc; binders such as polyvinyl alcohol, hydroxypropyl cellulose or gelatin; surfactants such as fatty acid ester; plasticizers such as glycerin; or the like.

An appropriate formulation for parenteral administration preferably consists of a sterilized aqueous agent containing an active compound isotonic with blood of a recipient. For example, in the case of an injection, a solution for injection is prepared using a carrier consisting of a mixture of a salt solution, a glucose solution or a salt water with a glucose solution, or the like.

In these parenteral agents, it is also possible to add one or more auxiliary ingredients selected from the diluents, preservatives, flavors, excipients, disintegrators, lubricants, binders, surfactants and plasticizers and the like exemplified in the oral agents.

A medicament containing the DcR3 variant of the present invention can be safely administered to mammals (e.g., humans, mice, rats, rabbits, dogs, cats, cattle, horses, pigs or monkeys or the like).

The dose and the frequency of administration of the DcR3 variant of the present invention vary depending on administration form, age of a patient, body weight, disease, or nature or seriousness of symptoms to be treated, but usually in the case of oral administration, 0.01 mg to 1 g, and preferably 0.05 to 50 mg per adult are administered once or several times daily. In the case of parenteral administration such as intravenous administration, 0.001 to 100 mg, and preferably 0.01 to 10 mg per adult are administered once or several times daily. However, these dose and frequency of administration vary depending on the various conditions mentioned above.

### EXAMPLES

Examples of the present invention will be described below. However, the present invention is not limited to these Examples.

### [Example 1] Evaluation of Aggregability of Wild-Type DcR3 in Mammalian Cell

A fusion protein (DcR3 FL-Fc) (FIG. 3A, SEQ ID NO: 100) of wild-type DcR3 (also referred to as full-length DcR3) (SEQ ID NO: 4), a linker sequence IEGRMD (SEQ ID NO: 106) and a human IgG1 Fc region (g1S) (SEQ ID NO: 72), a fusion protein (S195-Fc) (FIG. 3B, SEQ ID NO: 102) of human DcR3 lacking an HBD region (SEQ ID NO: 108), a linker sequence IEGRMD (SEQ ID NO: 106) and a human IgG1 Fc region (SEQ ID NO: 72), and a protein (DcR3 FL-FLAG) (SEQ ID NO: 104) obtained by adding a FLAG tag (SEQ ID NO: 110) to full-length DcR3 (SEQ ID NO: 4) were produced as follows.

Regarding DcR3 FL-Fc, a DNA fragment of a signal peptide sequence, a DNA fragment (SEQ ID NO: 3) of human DcR3, a DNA fragment (SEQ ID NO: 105) of a linker sequence IEGRMD, and a DNA fragment (SEQ ID NO: 71) of Fc (g1S) were artificially synthesized (GENEWIZ, Inc. or Sigma-Aldrich Co. LLC), followed by insertion using In-Fusion HD Cloning Kit (Clontech Laboratories, Inc.) into a pCIpuro vector (pCI manufactured by Promega Corporation was partially modified) digested with restriction enzymes NheI and SalI (New England Biolabs, Inc.), and *Escherichia coli* DH5a competent cells (TOYOBO CO., LTD.) were transformed, thus obtaining transformants each including a DcR3 FL-Fc DNA fragment (SEQ ID NO: 99) introduced thereinto. Similarly, regarding S195-Fc, a DNA fragment of a signal peptide sequence, a DNA fragment (SEQ ID NO: 107) of human DcR3 lacking HBD, a DNA fragment (SEQ ID NO: 105) of a linker sequence IEGRMD, and a DNA fragment (SEQ ID NO: 71) of Fc (g1S) were artificially synthesized, thus obtaining transformants each including an S195-Fc DNA fragment (SEQ ID NO: 101) introduced thereinto. Regarding DcR3 FL-FLAG, a DNA fragment of a signal peptide sequence, a DNA fragment (SEQ ID NO: 3) of human DcR3, and a DNA fragment (SEQ ID NO: 109) of a FLAG tag were artificially synthesized, thus obtaining transformants each including a DcR3 FL-FLAG DNA fragment (SEQ ID NO: 103) introduced thereinto.

Each plasmid obtained from each transformant was introduced into host cells selected from Freestyle CHO-S cells, Freestyle 293F cells and Expi293 cells (all of which are manufactured by Thermo Scientific) to transiently express proteins. For plasmid introduction, Freestyle MAX Reagent, 293Fectin Transfection Reagent or ExpiFectamine 293 (all of which are manufactured by Thermo Scientific) was used.

After the transfected cells were cultured for several days, the culture supernatants were collected, and DcR3 FL-Fc and S195-Fc were subjected to affinity purification using MabSelect SuRe (GE Healthcare), and DcR3 FL-FLAG was subjected to affinity purification using Anti-FLAG M2 affinity gel (Sigma-Aldrich Co. LLC). The culture supernatants for DcR3 FL-Fc and S195-Fc were passed through a column filled with a resin, washed with PBS (Nacalai Tesque, Inc.), and eluted with an elution buffer (20 mM citric acid, 50 mM NaCl, pH 3.4), followed by rapid neutralization with a neutralization buffer (1M sodium phosphate, pH 7.0). The culture supernatant for DcR3 FL-FLAG was similarly charged into a resin, washed with PBS, and eluted with an elution buffer (100 mM glycine-HCl, pH 3.5), followed by rapid neutralization with a neutralization buffer (1M Tris-HCl, pH 8.0). The absorbance at 280 nm (A₂₈₀) of each elution fraction was measured, and consecutive fractions having high measurement values were collected. The buffer of the collected fractions was substituted with PBS using a NAP column (GE Healthcare) and passed through a 0.22 µm filter, and the resultant product was regarded as a purified protein. The concentrations of the purified proteins were calculated by using the extinction coefficient of DcR3 FL-Fc, S195-Fc and DcR3 FL-FLAG at 280 nm as 1.03, 1.17 and 0.77, respectively, and after SDS-PAGE under non-reducing conditions and 100 mM DTT reducing conditions, the gel was subjected to Coomassie staining (Nacalai Tesque, Inc.), and the molecular weights were confirmed. The estimated molecular weights of monomers of DcR3 FL-Fc, S195-Fc and DcR3 FL-FLAG, predicted from the amino acid sequences thereof, are about 56.4 kDa, 44.7 kDa and 31.0 kDa, respectively. Under non-reducing conditions, DcR3 FL-Fc and S195-Fc, which are Fc fusions, exist as dimers, and thus the estimated molecular weights are about 112.8 kDa and 89.4 kDa, respectively.

As a result of SDS-PAGE of DcR3 FL-Fc, S195-Fc and DcR3 FL-FLAG that were transiently expressed in mammalian cells under non-reducing conditions, the majorities of the samples of DcR3 FL-Fc, S195-Fc and DcR3 FL-FLAG remained in sample wells, and the electrophoresed parts of the samples resulted in smear-like or ladder-like patterns, revealing that all recombinants were highly aggregated (FIG. 1A).

Furthermore, regarding commercially available full-length DcR3-Fc (Abcam plc.) produced using HEK293 cells as host cells, immunoblotting under non-reducing conditions was performed using a rabbit anti-human IgG Fc polyclonal antibody as a primary antibody and a goat anti-rabbit IgG antibody (Dako) as a secondary antibody, almost all thereof existed as aggregates, like the abovementioned purified products (FIG. 1B).

Meanwhile, commercially available DcR3 FL-Fc (R&D Systems, Inc.) expressed using insect cells Sf21 showed almost no aggregation, and there was a difference in aggregability depending on the types of host cells producing wild-type DcR3 (FIG. 1C). By the following method using insect cells S2 as host cells, DcR3 FL-Fc (SEQ ID NO: 100), R218Q-Fc (US 6835814 B1, US 6965012 B1, SEQ ID NO: 340) obtained by introducing into human DcR3 (SEQ ID NO: 2) an R218Q mutation (i.e., substitution of Arg at position 218 with Gln), and S195-Fc lacking HBD (SEQ ID NO: 102) were produced, and aggregabilities were evaluated. One of a DNA fragment (SEQ ID NO: 3) of human DcR3, a DNA fragment (SEQ ID NO: 111) obtained by introducing into human DcR3 an R218Q mutation, and a DNA fragment (SEQ ID NO: 107) of human DcR3 lacking HBD was artificially synthesized connected to a DNA fragment (SEQ ID NO: 105) of a linker sequence IEGRMD and a DNA fragment (SEQ ID NO: 71) of Fc (g1S), and using pMTBiPV5-HisA (Thermo Scientific) and Drosophila Expression System (Thermo Scientific), an S2 cell line that stably expresses each of DcR3 FL-Fc, R218Q-Fc and S195-Fc was obtained. From the culture supernatants of the cells of the stable expression cell lines, affinity purification by MabSelect SuRe was performed using the same method as mentioned above.

As a result, DcR3 FL-Fc, R218Q-Fc and S195-Fc that were produced using S2 cells showed almost no aggregation (FIG. 1C). Quantitative evaluation of the aggregate content was performed by gel filtration chromatography (SEC) (TSKgel G3000 SWXL 7.8 mm × 300 mm) (Tosoh Corporation) using HPLC (Shimadzu Corporation). S195-Fc derived from each of Expi293 and S2 was analyzed, and the proportions (%) of monomers, aggregates and degradants calculated from the peak areas are shown in Table 1.

**[Table 1]**

| Host cell | Monomer | Aggregate | Degradant |
|---|---|---|---|
| Expi293 | 15.1% | 81.9% | 3.0% |
| S2 | 84.9% | 15.1% | 0.0% |

The above results revealed that, when wild-type DcR3 is expressed using a mammalian cell, the generation amount of aggregates is increased.

### [Example 2] Production of DcR3 Variant That Is Not Aggregated in Mammalian Cell Expression System

There has not been known a human DcR3 variant that is not aggregated in a mammalian cell expression system. Thus, it was attempted to produce a DcR3 variant that results in a decreased amount of aggregates while maintaining the activity of DcR3. DcR3 is a soluble molecule consisting of 300 residues, and has a signal peptide at the N-terminal side, followed by four cysteine rich domains (CRD1, CRD2, CRD3, CRD4), which are characteristic of a TNF receptor superfamily (TNFRSF), and the C-terminal side of DcR3 consists of a heparan sulfate binding domain (HBD), which includes a heparan sulfate-binding motif and is rich in basic amino acids (FIG. 2, 3A). All of LIGHT, TL1A and FasL, which are DcR3 ligands, bind to CRD2 and CRD3 of DcR3. Thus, while maintaining a region including CRD2 and CRD3 of DcR3, CRD1 and/or CRD4 of DcR3 was/were substituted with CRD1 and/or CRD4 of a soluble decoy receptor osteoprotegerin (OPG), which is a related TNFRSF molecule, thus producing a DcR3 variant.

Various DcR3 variants having various structures, i.e.,
chimera A-Fc (FIG. 3C, SEQ ID NO: 80 or 82) obtained by fusion of chimera A (SEQ ID NO: 54) and an Fc sequence, wherein the chimera A was obtained by introducing into human DcR3, substitution of amino acid sequences of CRD1 and CRD4 with amino acid sequences of CRD1 and CRD4 of human OPG, respectively, and removal of an amino acid sequence of HBD, while maintaining amino acid sequences of CRD2 and CRD3 of human DcR3, and wherein the Fc sequence was selected from IEGRMD g1S (SEQ ID NO: 339) and g4PEK (SEQ ID NO: 74) obtained by introducing into an Fc region of heavy chain of human IgG4, substitution of Ser at EU-index position 228 with Pro, substitution of Leu at EU-index position 235 with Glu, and substitution of Arg at EU-index position 409 with Lys;
chimera B-Fc (FIG. 3G, SEQ ID NO: 76) obtained by fusion of chimera B (SEQ ID NO: 50) and an Fc sequence (IEGRMD g1S), wherein the chimera B was obtained by introducing into human DcR3, substitution of an amino acid sequence of CRD1 with an amino acid sequence of CRD1 of human OPG, and removal of an amino acid sequence of HBD, while maintaining amino acid sequences of CRD2, CRD3 and CRD4 of human DcR3;
chimera C-Fc (FIG. 3H, SEQ ID NO: 78) obtained by fusion of chimera C (SEQ ID NO: 52) and an Fc sequence (IEGRMD g1S), wherein the chimera C was obtained by introducing into human DcR3, substitution of an amino acid sequence of CRD4 with an amino acid sequence of CDR4 of human OPG, and removal of an amino acid sequence of HBD, while maintaining amino acid sequences of CRD1, CRD2 and CRD3 of human DcR3; and
103-123OPG-Fc (g4PEK) (FIG. 3D, SEQ ID NO: 84) obtained by fusion of 103-123OPG (SEQ ID NO: 56) and an Fc sequence (g4PEK), wherein the 103-123OPG was obtained by introducing into CRD3 of chimera A, substitution of an amino acid sequence including a part at positions 18 to 36 and two amino acids at the C-terminal side thereof with an amino acid sequence of human OPGwere produced by the following method. In the DcR3 variant including CRD4 derived from DcR3, a TS sequence corresponding to an amino acid sequence at positions 194 to 195 of DcR3 (SEQ ID NO: 2) was added to the C-terminus of the CRD4. In the DcR3 variant including CRD4 derived from OPG, an SGNSESTQK sequence corresponding to an amino acid sequence at positions 186 to 194 of OPG (SEQ ID NO: 14) was added to the C-terminus of the CRD4.

A DNA fragment of a signal peptide sequence and a DNA fragment encoding chimera A, chimera B, chimera C or 103-123OPG lacking an HBD sequence (chimera A: SEQ ID NO: 53, chimera B: SEQ ID NO: 49, chimera C: SEQ ID NO: 51, 103-123OPG: SEQ ID NO: 55) were artificially synthesized, followed by insertion into a pCIpuro vector by the same method as in Example 1 in such a manner that the DNA fragments were linked to a DNA fragment (SEQ ID NO: 338, 73) encoding an Fc sequence (IEGRMD g1S or g4PEK), thus obtaining a plasmid into which DNA fragments (chimera A-Fc: SEQ ID NO: 79 or 81, chimera B-Fc: SEQ ID NO: 75, chimera C-Fc: SEQ ID NO: 77, 103-123OPG-Fc: SEQ ID NO: 83) encoding various DcR3 variants were inserted. In the same manner as in Example 1, the plasmid thus obtained was introduced into host cells selected from Freestyle CHO-S cells, Freestyle 293F cells and Expi293 cells to transiently express a protein, and from the culture supernatant, affinity purification by MabSelect SuRe was performed.

The proportions (%) of monomers, aggregates and degradants of the various DcR3 variants produced were calculated by SEC-HPLC using the same method as in Example 1, or by SEC (Waters K.K.) using ACQUITY UPLC Protein BEH SEC 4.6 mm × 150 mm (Table 2).

**[Table 2]**

| DcR3 variant | Fc | Host cell | Monomer | Aggregate | Degradant |
|---|---|---|---|---|---|
| Chimera A-Fc | IEGRMD g1S | CHO-S | 93.8% | 4.6% | 1.6% |
| Chimera A-Fc | g4PEK | Expi293 | 96.6% | 3.4% | 0.0% |
| Chimera B-Fc | IEGRMD g1S | Expi293 | 83.8% | 15.4% | 0.8% |
| Chimera C-Fc | IEGRMD g1S | Expi293 | 70.0% | 29.5% | 0.5% |
| 103-123OPG-Fc | g4PEK | Expi293 | 98.5% | 1.5% | 0.0% |

As a result, in each of the DcR3 variants, i.e., chimera A-Fc, chimera B-Fc, chimera C-Fc and 103-123OPG-Fc, the proportion of aggregates was decreased compared to that of wild-type DcR3. The effect on decreasing aggregates was highest in chimera A-Fc, followed by chimera B-Fc and chimera C-Fc. Therefore, it was shown that each of substitution with CRD1 derived from OPG and substitution with CRD4 derived from OPG has an effect on decreasing aggregation, and that a combination of substitution with CRD1 and substitution with CRD4 has an effect on further decreasing aggregation.

As a result of subjecting chimera A-Fc, chimera B-Fc and chimera C-Fc (each including IEGRMD g1S) to SDS-PAGE under non-reducing conditions and 100 mM DTT reducing conditions, almost no smear or ladder was observed in chimera A-Fc and chimera B-Fc even when the variants were produced using mammalian cells as host cells (FIG. 4).

In order to calculate the absolute molecular weight, an analysis by SEC (TSKgel G3000 SWXL 7.8 mm × 300 mm) (Tosoh Corporation) using HPLC (Prominence, Shimadzu Corporation) was performed for chimera A-Fc (IEGRMD g1S or g4PEK) and 103-123OPG-Fc (g4PEK). After separation at a flow rate of 0.75 mL/min using a 50 mmol/L phosphate buffer (pH 7.0, 500 mmol/L NaCl) as a mobile phase, the scattered light intensity of each peak detected at a wavelength of 215 nm was detected by multi angle light scattering (MALS) (miniDAWN TREOS, Wyatt Technology Corporation), and the absolute molecular weight (% represents uncertainty) was calculated using the scattered light intensity. The results are shown in Table 3.

**[Table 3]**

| DcR3 variant | Fc | Molecular weight (%) | kDa |
|---|---|---|---|
| Chimera A-Fc | IEGRMD g1S | 8.858 x 10⁴ (1%) | 89 |
| Chimera A-Fc | g4PEK | 9.744 x 10⁴ (0.8%) | 97 |
| 103-123OPG-Fc | g4PEK | 1.052 x 10⁵ (0.7%) | 105 |

The molecular weights of dimers predicted from the amino acid sequences of chimera A-Fc (IEGRMD g1S or g4PEK) and 103-123OPG-Fc are 92.0 kDa, 90.1 kDa and 90.5 kDa, respectively, and thus it was confirmed that each of the DcR3 variants exists as dimers.

### [Example 3] Analysis of Proportion of N-Linked Glycosylation of Chimera A-Fc (g4PEK) and Evaluation of Influence on Aggregability

From the amino acid sequence of chimera A-Fc (g4PEK) (SEQ ID NO: 82), it is predicted that the CRD4 derived from OPG includes three potential N-linked glycosylation sites each consisting of Asn (N131, N144, N157), and that the Fc region includes one potential N-linked glycosylation site consisting of Asn (N260). Thus, the presence or absence of N-linked glycosylation of chimera A-Fc (g4PEK) was evaluated by the following method. As the sample preparation, chimera A-Fc (g4PEK) was subjected to reduction and alkylation treatments and the N-linked glycans of chimera A-Fc (g4PEK) were cleaved by PNGaseF treatment, and then the proteins were digested by each protease of trypsin, endoproteinase Asp-N and chymotrypsin. The peptide mixture thus obtained was dissolved in 5%(v/v) acetonitrile/0.1% formic acid, followed by an analysis with a liquid chromatography-electrospray ionization-mass spectrometer (LC-ESI-MS). Using MAGIC2000 HPLC (Michrom Bioresources, Inc.) equipped with a C18 reversed-phase column (0.2 mm × 150 mm) (GL Science Inc.) and LTQ Orbitrap XL ion trap-Orbitrap hybrid mass spectrometer (Thermo Scientific), an analysis was performed by gradient elution of 5-65%(v/v) acetonitrile/0.1% formic acid. The peptide fragments thus obtained were identified by a MASCOT analysis (Matrix Science Inc.) using the amino acid sequence of chimera A-Fc (g4PEK). Using as an index the mass increase by 0.984 Da caused by PNGaseF treatment for converting N-linked glycosylated Asn to Asp, a peptide exhibiting a shift in a peak of an MS spectrum and a glycosylation site included in the peptide were identified. As a result, it was shown that N-linked glycans were added for each of the Asn residues N131, N144, N157 and N260. Of these, regarding N157 and N260, glycans were added in almost all peptide fragments, while regarding N131 and N144, both of glycosylated fragments and non-glycosylated fragments were detected.

Next, in order to evaluate the influence on the aggregability of N-linked glycans added to three sites each consisting of Asn (N131, N144, N157) of CRD4 derived from OPG, an amino acid substitute lacking glycans at two sites of N131 and N144 or at three sites of N131, N144 and N157 was produced. As a variant lacking the two glycans, N131S/N144S-Fc (g4PEK) (SEQ ID NO: 86) obtained by substitution of each of N131 and N144 with Ser or T133A/S146A-Fc (g4PEK) (SEQ ID NO: 88) obtained by substitution of each of T133 and S146 with Ala was produced. As a variant lacking the three glycans, N131S/N144S/N157S-Fc (g4PEK) (SEQ ID NO: 90) obtained by substitution of each of N131, N144 and N157 with Ser or T133A/S146A/T159A-Fc (g4PEK) (SEQ ID NO: 92) obtained by substitution of each of T133, S146 and T159 with Ala was produced. A DNA fragment of a signal peptide sequence and DNA fragments (SEQ ID NO: 85, 87, 89, 91) encoding the respective variants lacking the glycans were artificially synthesized, followed by insertion into a pCIpuro vector by the same method as in Example 1, and each DNA fragment was introduced into Expi293 cells. Each of the variants lacking the glycans was transiently expressed, and then affinity purification from using the culture supernatant by MabSelect SuRe was performed. Each of the produced variants lacking the glycans was analyzed by SEC-UPLC (apparatus: ACQUITY UPLC, column; ACQUITY UPLC Protein BEH SEC 200 Å, 1.7 µm, 4.6 × 150 mm) (Waters K.K.). The proportions (%) of monomers, aggregates and degradants calculated from the peak areas are shown in Table 4.

**[Table 4]**

| Variant lacking glycans | Fc | Monomer | Aggregate | Degradant |
|---|---|---|---|---|
| N131S/N144S-Fc | g4PEK | 92.5% | 7.5% | 0.0% |
| T133A/S146A-Fc | g4PEK | 91.2% | 8.8% | 0.0% |
| N131S/N144S/ N157S-Fc | g4PEK | 64.0% | 36.0% | 0.0% |
| T133A/S146A/ T159A-Fc | g4PEK | 62.3% | 37.7% | 0.0% |

Regarding the aggregate contents of the variants lacking the two glycans, the aggregates were slightly increased in all of the amino acid substitutes compared to those of chimera A-Fc (Table 2), and regarding the aggregate contents of the variants lacking the three glycans, further increases in the aggregates were observed in all of the amino acid substitutes. From these results, it was shown that, of the N-linked glycans at three sites (N131, N144, N157) added to CRD4 derived from OPG, particularly a glycan added to N157, contributes to a decrease in the aggregates of chimera A. Meanwhile, even when all of the N-linked glycans at three sites were removed, the aggregates were decreased compared to those of DcR3 FL-Fc and S195-Fc (Table 1). Therefore, it was shown that each of an N-linked glycan and a sequence of OPG has an effect on decreasing the aggregates of wild-type DcR3, and that a combination of both has an effect on further decreasing the aggregates.

### [Example 4] Evaluation of Physical Property of Various DcR3 Variants

In order to analyze the cause of the effect on decreasing aggregation of various DcR3 variants produced in Examples 2 and 3, the following physical and chemical analyses were performed. Hydrophobic interaction chromatography (HIC) is an analysis method in which the higher the hydrophobicity of a protein surface is, the longer the elution time from a column is. Using a hydrophobic interaction chromatography column (TSKgel Butyl-NPR 4.6 mm × 35 mm) (Tosoh Corporation), and using a gradient of buffer A (2 mmol/L ammonium sulfate, 20 mmol/L Tris buffer, pH 7.0) and buffer B (20 mmol/L Tris buffer, pH 7.0) as a mobile phase, 8 µg of a sample was separated at a flow rate of 0.5 mL/min, and the elution time (min) was detected at a wavelength of 215 nm. The elution time (min) is shown in Table 5.

**[Table 5]**

| | | Fc | Host cell | Elution time (min) |
|---|---|---|---|---|
| Wild-type DcR3 control | R218Q-Fc | IEGRMD g1S | S2 | 39.8 |
| | S195-Fc | IEGRMD g1S | S2 | 36.7 |
| DcR3 variant | Chimera A-Fc | IEGRMD g1S | CHO-S | 37.8 |
| | Chimera A-Fc | g4PEK | Expi293 | 38.7 |
| | Chimera C-Fc | IEGRMD g1S | Expi293 | 39.7 |
| | 103-123OPG-Fc | g4PEK | Expi293 | 37.8 |
| | N131S/ N144S-Fc | g4PEK | Expi293 | 39.2 |
| | T133A/ S146A-Fc | g4PEK | Expi293 | 39.3 |
| | N131S/N144S/ N157S-Fc | g4PEK | Expi293 | 40.6 |
| | T133A/S146A/ T159A-Fc | g4PEK | Expi293 | 40.9 |

Each of S195-Fc derived from S2 and the variants derived from mammalian cells, i.e., chimera A-Fc (IEGRMD g1S, g4PEK), 103-123OPG-Fc (g4PEK) and the variants lacking the two glycans, showed a shorter elution time as compared to R218Q-Fc corresponding to a one-amino-acid mutant of full-length DcR3 produced using insect cells S2. It is considered that a decrease in hydrophobicity resulted in improvement in the physical property and a decrease in the amount of aggregates. Regarding chimera C and the variants lacking the three glycans, each of which exhibited the slightly-attenuated effect of decreasing the amount of aggregates, the hydrophobicities were equivalent or superior to that of R218Q-Fc, and it was shown that there is a correlation between the proportion of aggregates and the hydrophobicity of the protein.

Subsequently, the thermostability of the protein was evaluated by the differential scanning fluorimetry (DSF) method. In a 96-well white microplate (Bio-Rad Laboratories, Inc.), 9.5 µg of various DcR3 variants and 1 µL of SYPRO Orange Protein Gel Stain (Invitrogen) diluted with water 50-fold were mixed in 20 µL of a system, and the temperature was raised from 20°C to 95°C by 0.5°C every 10 seconds using a C1000 thermal cycler (Bio-Rad Laboratories, Inc.). Fluorescence at each temperature was detected with a FRET channel, and the melting curve (FIG. 5) and the melting temperature (Tm value) (°C) (Table 6) were calculated using CFX Manager software (Bio-Rad Laboratories, Inc.).

**[Table 6]**

| | | Fc | Host cell | Tm1 | Tm2 |
|---|---|---|---|---|---|
| Wild-type DcR3 control | R218Q-Fc | IEGRMD g1S | S2 | 47.5 | 60.5 |
| | S195-Fc | IEGRMD g1S | S2 | 62.0 | 81.0 |
| DcR3 variant | Chimera A-Fc | IEGRMD g1S | CHO-S | 66.5 | 82.5 |
| | Chimera A-Fc | g4PEK | Expi293 | 61.0 | 82.5 |
| | Chimera C-Fc | IEGRMD g1S | Expi293 | 67.5 | 82.0 |
| | 103-123OPG-Fc | g4PEK | Expi293 | 61.0 | 82.5 |
| | N131S/ N144S-Fc | g4PEK | Expi293 | 60.0 | 82.5 |
| | T133A/ S146A-Fc | g4PEK | Expi293 | 60.0 | 82.5 |
| | N131S/N144S/ N157S-Fc | g4PEK | Expi293 | 61.0 | 83.0 |
| | T133A/S146A/ T159A-Fc | g4PEK | Expi293 | 61.0 | 83.5 |

S195-Fc derived from S2 showed a greatly increased Tm value and improved thermostability as compared to R218Q-Fc corresponding to a one-amino-acid mutant of full-length DcR3 produced using insect cells S2. This suggests that an HBD region contributes to thermolability. Chimera A-Fc (IEGRMD g1S) derived from a mammalian cell showed a further increased Tm value as compared to S195-Fc. This reveals that a DcR3 variant in which a part of CRDs of DcR3 has been substituted with a part of CRDs of OPG shows not only decreased aggregability but also improved thermostability of the protein (Table 6, FIG. 5).

### [Example 5] Evaluation of Reactivity of DcR3 Variant Lacking Heparan Sulfate-Binding Domain (HBD) with Human Normal Cell and CHO Cell

It has been reported that *in vivo* kinetics of wild-type DcR3 and a mutant thereof, FLINT (R218Q mutation), in mice and cynomolgus monkeys are extremely poor (Drug Metabolism and Disposition, 2003, 31: p.502-507). One of the factors thereof is considered as the fact that DcR3 directly binds to heparan sulfate proteoglycan on the cell membrane by a heparan sulfate-binding domain (HBD) existing in wild-type DcR3 (J. Immunol., 2006, 176: 173-180).

Thus, the reactivities of DcR3 variants lacking HBD, i.e., S195-Fc, chimera A-Fc (IEGRMD g1S, g4PEK) and 103-123OPG-Fc (g4PEK) with human primary cells and CHO cells (producing cells) were analyzed by flow cytometry (FCM). As a positive control, DcR3 FL-Fc including an HBD region was used, and as negative controls, K194-Fc and an anti-DNP antibody (g4PEK) were used. K194-Fc (amino acid sequence: SEQ ID NO: 152, nucleotide sequence of DNA: SEQ ID NO: 151) is a protein obtained by fusing an IEGRMD linker (SEQ ID NO: 106) and an amino acid sequence (SEQ ID NO: 72) of Fc (g1S) to an amino acid sequence of a part extending from Met at position 1 to Lys at position 194 of OPG (SEQ ID NO: 14), and using the method mentioned in Example 1, K194-Fc was transiently expressed using Expi293 cells, and purified from the culture supernatant using Mabselect SuRe (GE Healthcare). As the anti-DNP antibody (g4PEK), an antibody obtained by inserting the nucleotide sequence of the variable region of the anti-2,4-dinitrophenol (DNP) antibody mentioned in Clin. Cancer Res., 2005, 11(8), p.3126-3135 into a vector encoding an Fc sequence (g4PEK), followed by introduction into CHO cells to express, and performing Protein A purification from the culture supernatant, was used. As the human primary cells, HUVECs (Lonza K.K.) and male human hepatocytes (BioreclamationIVT, LLC) were used. The human primary cells were cultured with a medium designated for each cell in accordance with the package insert using a collagen-coated adherent plate (IWAKI). The CHO cells were suspension-cultured with EX-CELL 325 PF CHO Serum-Free Medium (Sigma Aldrich Co. LLC).

HUVECs and hepatocytes were detached using a 0.02% EDTA solution and a cell scraper, and passed through a cell strainer (40 µm). The detached HUVECs and hepatocytes and CHO cells collected from the suspension culture solution were washed with an FCM buffer (PBS containing 1% BSA, 1 mmol/L EDTA and 0.05% NaN₃), and then suspended with an FCM buffer.

Next, the cells were seeded in a 96-well U-bottom plate (Falcon) so that the concentrations were 1 × 10⁵ cells/well, and each Fc fusion protein produced was added so that the concentration was 10 µg/mL, followed by reaction on ice for 1 hour. The cells were washed with an FCM buffer, and then suspended with LIVE/DEAD Fixable Aqua Dead Cell Stain Kit (Molecular Probes) and 0.1 µg/mL of Goat F(ab')₂ Anti-Human IgG R-phycoerythrin Conjugate (Southern Biotechnology Associates, Inc.), followed by staining on ice for 1 hour. During staining of HUVECs and hepatocytes, Human FcR Blocking Reagent (Miltenyi Biotec K.K.) was added. The cells were washed with an FCM buffer, and then the fluorescence intensity was analyzed by flow cytometer FACS Fortessa (BD Biosciences).

As a result of analysis of the PE staining intensities for viable cell fractions that were negative for LIVE/DEAD Fixable Aqua Dead Cell Stain Kit, DcR3 FL-Fc (IgG1) (R&D Systems, Inc.) showed remarkable binding to all of HUVECs, hepatocytes and CHO cells. Meanwhile, all of the DcR3 variants lacking HBD showed no binding (FIG. 6).

### [Example 6] Evaluation of Mouse In Vivo Kinetics of DcR3 Variant

**[Table 7]**

| | | Fc | Host cell |
|---|---|---|---|
| Wild-type DcR3 control | S195-Fc | IEGRMD g1S | S2 |
| DcR3 variant | Chimera A-Fc | IEGRMD g1S | CHO-S |
| | Chimera A-Fc | g4PEK | CHO-S |
| | Chimera A-Fc | g4PEK | CHO |
| | 103-123OPG-Fc | g4PEK | CHO |
| | T133A/S146A-Fc | g4PEK | CHO-S |

A kinetic study in mouse on S195-Fc and each of the DcR3 variants shown in Table 7 was conducted. Regarding chimera A-Fc (g4PEK) and 103-123OPG-Fc (g4PEK), each of the DcR3 variants was produced by stable expression in CHO cells using the following method. A DNA fragment of a signal peptide sequence and DNA fragments (SEQ ID NO: 81, 83) encoding the respective DcR3 variants were artificially synthesized, followed by insertion into a recombinant vector produced by the method mentioned in WO 2012/081628 using the same method as in Example 1, and the vectors were introduced into CHO cells by electroporation. Culture and drug selection were performed by the general method, and cell lines in which the proportions of viable cells collected to about 98% were regarded as stable expression cell lines. The cells of these stable expression cell lines were cultured in a medium such as EX-CELL 325 PF CHO Serum-Free Medium (Sigma Aldrich Co. LLC) for a certain period of time, followed by collection of the culture supernatants, and each of the DcR3 variants was purified by the method mentioned in Example 1.

Single i.v. administration of 10 mg/kg of S195-Fc or each of the DcR3 variants was performed to 5- to 6-week-old BALB/c mice (female) (n = 2 or 3), followed by blood collection from the tail vein at an appropriate time after administration, and concentrations of S195-Fc and each of the DcR3 variants in blood were measured by the following method. A biotinylated monkey anti-human IgG polyclonal antibody was reacted with a streptavidin-immobilized bead, and S195-Fc and each of the DcR3 variants in serum bound to the biotinylated monkey anti-human IgG polyclonal antibody were detected with an Alexa Fluor 647-labeled monkey anti-human IgG polyclonal antibody. Measurement was performed using Gyrolab xP workstation (Gyros AB), and each kinetic parameter was calculated by the moment analysis method. As the standard substance for preparation of a calibration curve, the same substance as the test substance administered to the animals was used. The time courses of blood concentrations of S195-Fc produced using S2 cells and chimera A-Fc (IEGRMD g1S) produced using CHO-S cells are shown in FIG. 7. Regarding each of S195-Fc and the DcR3 variants shown in Table 7, the half-life in blood (h) during the elimination phase and the area under the concentration-time curve to infinity AUC0-∞ (µg*h/mL) after single administration are shown in Table 8.

**[Table 8]**

| | | Fc | Host cell | Half-life in blood (h) | AUC0-∞ (µg*h/mL) |
|---|---|---|---|---|---|
| Wild-type DcR3 control | S195-Fc | IEGRMD g1S | S2 | 84.1 | 354 |
| DcR3 variant | Chimera A-Fc | IEGRMD g1S | CHO-S | 87.9 | 7140 |
| | Chimera A-Fc | g4PEK | CHO-S | 114 | 5680 |
| | Chimera A-Fc | g4PEK | CHO | 89.5 | 4530 |
| | 103-123OPG-Fc | g4PEK | CHO | 79.3 | 2400 |
| | T133A/S146A-Fc | g4PEK | CHO-S | 114 | 5810 |

The time course of blood concentration of each of the DcR3 variants was greatly improved (FIG. 7), the half-life of each of the DcR3 variants was almost equivalent, and the AUC0-∞ of each of the DcR3 variants was improved 10-fold or more (Table 8), compared to those of S195-Fc corresponding to a wild-type DcR3 control.

It has been reported that, after single i.v. administration of 0.5 mg/kg of wild-type DcR3 or FLINT (R218Q mutation) to CD-1 mice, the half-life in blood was 1.2 hours and 3.1 hours, respectively, and the AUC0-∞ (µg*h/mL) was 0.48 and 0.36, respectively (Drug Metabolism and Disposition, 2003. 31: p.502-507.). When the time courses of concentrations multiplied by a dose ratio assuming linearity were compared, the DcR3 variant showed higher exposure than that of wild-type DcR3 and FLINT.

### [Example 7] Evaluation of Binding Activity to DcR3 Ligand (1) Production of DcR3 Ligand

Soluble recombinants of DcR3 ligands (LIGHT, TL1A, FasL) of a human, a cynomolgus monkey or a mouse were produced (amino acid sequence: SEQ ID NO: 114, 116, 118, 120, 122, 124, 126, 128, 130, nucleotide sequence of DNA: SEQ ID NO: 113, 115, 117, 119, 121, 123, 125, 127, 129).

As a soluble recombinant LIGHT of a human, a cynomolgus monkey or a mouse, a sequence obtained by adding a His tag (His10) and a GS linker (GGGSGGGSGGGSIEGR) to the N-terminus and linking an extracellular region of LIGHT (human LIGHT: Asp74-Val240 (SEQ ID NO: 132), cynomolgus monkey LIGHT: Asp74-Val240 (SEQ ID NO: 134), mouse LIGHT: Asp72-Val239 (SEQ ID NO: 136)) to downstream thereof was used.

As a soluble recombinant TL1A of a human, a cynomolgus monkey or a mouse, a sequence obtained by adding a His tag (His6) and a GS linker (GGGSGGGSGGGS) to the N-terminus and linking an extracellular region of TL1A (human TL1A: Leu72-Leu251 (SEQ ID NO: 138), cynomolgus monkey TL1A: Leu72-Leu251 (SEQ ID NO: 140), mouse TL1A: Ile94-Leu270 (SEQ ID NO: 142)) to downstream thereof was used.

As a soluble recombinant FasL of a human, a cynomolgus monkey or a mouse, a sequence obtained by adding a His tag (His6) to the N-terminus and linking an extracellular region of FasL (human FasL: Pro134-Leu281 (SEQ ID NO: 144), cynomolgus monkey FasL: Pro133-Leu280 (SEQ ID NO: 146), mouse FasL: Pro132-Leu279 (SEQ ID NO: 148)) to downstream thereof was used.

A DNA fragment of a signal peptide sequence and a DNA sequence of a soluble DcR3 ligand including an attached tag were artificially synthesized (GENEWIZ, Inc.), and using In-Fusion HD Cloning Kit (Clontech Laboratories, Inc.), they were inserted into downstream of a CMV promoter of a pCI-Hygro2.01 vector (pCI manufactured by Promega Corporation was partially modified), and *Escherichia coli* DH5a competent cells (TOYOBO CO., LTD.) were transformed.

The plasmids thus obtained were introduced into Expi293 cells (Thermo Scientific) to transiently express the proteins. Using ExpiFectamine 293 (Thermo Scientific), the plasmids were introduced, and after culture for 3 days, the culture supernatants were collected.

Human FasL and cynomolgus monkey FasL were introduced into host cells of Freestyle CHO-S cells (Thermo Scientific) to transiently express proteins. Using Freestyle MAX Reagent (Thermo Scientific), the plasmids were introduced, and after culture for 3 days, the culture supernatants were collected.

Protein purification from the culture supernatants of the Expi293 cells was performed using Ni Sepharose Fast Flow resin and His Buffer Kit (all of which are manufactured by GE Healthcare). The culture supernatants were passed through a column filled with a resin, washed with a washing buffer (60 mmol/L imidazole, 20 mmol/L sodium phosphate, 0.5 mol/L NaCl, pH 7.4), and eluted with an elution buffer (250 mmol/L imidazole, 20 mmol/L sodium phosphate, 0.5 mol/L NaCl, pH 7.4).

Protein purification from the culture supernatants of the Freestyle CHO-S cells was performed using Complete His-Tag Purification Resin (Roche) and His Buffer Kit (GE Healthcare). The culture supernatants were passed through a column filled with a resin, washed with a washing buffer (2 mmol/L imidazole, 20 mmol/L sodium phosphate, 0.5 mol/L NaCl, pH 7.4), and eluted with an elution buffer (250 mmol/L imidazole, 20 mmol/L sodium phosphate, 0.5 mol/L NaCl, pH 7.4).

Each elution buffer of elution fractions was substituted with PBS using a NAP column (GE Healthcare) and passed through a 0.22 µm filter to perform sterilization. Regarding the purified proteins thus obtained, the purities were confirmed by SDS-PAGE. When formations of multimers were analyzed by SEC-UPLC (apparatus: ACQUITY UPLC, column; ACQUITY UPLC Protein BEH SEC 200Å, 1.7 µm, 4.6 × 150 mm) (Waters K.K.), almost all recombinant soluble DcR3 ligands showed a peak of a molecular weight corresponding to a trimer, but in only mouse LIGHT, no peak of a trimer was detected, and a peak of a monomer was detected.

### (2) Measurement of Binding Activity Using BIAcore

Regarding each of the various wild-type DcR3 controls and the DcR3 variants shown in Table 9, the binding activities to DcR3 ligands (LIGHT, TL1A, FasL) were analyzed by the SPR method using BIAcore T-100 (GE Healthcare). As the buffer, HBS-EP+ Buffer was used.

After 10,000 RU of anti-human antibodies were immobilized to Series S Sensor Chip CM5 using Human Antibody Capture Kit (GE Healthcare), various wild-type DcR3 controls and DcR3 variants were injected at the flow rate of 10 µL/min for 30 seconds and captured. Meanwhile, the buffer containing no protein was injected into reference flow cells. Thereafter, as an analyte, DcR3 ligands of a human, a cynomolgus monkey or a mouse diluted to 0.08 to 80 nmol/L were injected at 10 µL/min for 2 minutes to monitor the binding, and subsequently the buffer was injected for 3 minutes to monitor dissociation. Then, 3 mol/L magnesium chloride was injected at 20 µL/min for 1 minute to perform a regeneration reaction. By using BIAcore T-100 evaluation software and 1:1 Binding model, and by regarding each DcR3 ligand as a monomer (human LIGHT monomer: 20.8 kDa, human TL1A monomer: 22.1 kDa, human FasL monomer: 17.7 kDa, cynomolgus monkey LIGHT monomer: 20.8 kDa, cynomolgus monkey TL1A monomer: 22.0 kDa, cynomolgus monkey FasL monomer: 17.7 kDa, mouse LIGHT monomer: 20.9 kDa, mouse TL1A monomer: 21.5 kDa, mouse FasL monomer: 17.7 kDa), each kinetic constant (kₐ, k_{d}, K_{D}) was calculated (Tables 10 to 12). As a result of measurement, except for mouse LIGHT for which a trimer could not be produced, it was confirmed that various wild-type DcR3 controls and DcR3 variants bind to each DcR3 ligand of a human, a cynomolgus monkey or a mouse.

**[Table 9]**

| | | Linker | Fc | Host cell |
|---|---|---|---|---|
| 1 | DcR3 FL-Fc (R&D Systems, Inc.) | IEGRMD | IgG1 | Sf9 |
| 2 | S195-Fc | IEGRMD | g1S | S2 |
| 3 | Chimera A-Fc | IEGRMD | g1S | CHO-S |
| 4 | Chimera A-Fc | None | g4PEK | Expi293 |
| 5 | 103-123OPG-Fc | None | g4PEK | Expi293 |
| 6 | T133A/S146A-Fc | None | g4PEK | Expi293 |
| 7 | N131S/N144S-Fc | None | g4PEK | Expi293 |

**[Table 10]**

| Binding affinity to LIGHT | | | | | | | | | |
|---|---|---|---|---|---|---|---|---|---|
| | Human LIGHT | | | Cynomolgus monkey LIGHT | | | Mouse LIGHT | | |
| | Kₐ (1/Ms) 1xE+05 | K_{d} (1/s) 1xE-04 | K_{D} (nM) | Kₐ (1/Ms) 1xE+05 | K_{d} (1/s) 1xE-04 | K_{D} (nM) | Kₐ (1/Ms) 1xE+05 | K_{d} (1/s) 1xE-04 | K_{D} (nM) |
| 1 | 3.8 | 4.0 | 1.1 | 3.1 | 3.8 | 1.2 | N.D. | N.D. | N.D. |
| 2 | 2.8 | 6.1 | 2.2 | 3.4 | 5.9 | 1.7 | N.D. | N.D. | N.D. |
| 3 | 3.9 | 2.5 | 0.63 | 5.4 | 2.4 | 0.44 | N.D. | N.D. | N.D. |
| 4 | 5.4 | 3.3 | 0.62 | N.T. | N.T. | N.T. | N.T. | N.T. | N.T. |
| 5 | 4.5 | 3.0 | 0.66 | N.T. | N.T. | N.T. | N.T. | N.T. | N.T. |
| 6 | 3.8 | 3.1 | 0.83 | N.T. | N.T. | N.T. | N.T. | N.T. | N.T. |
| 7 | 3.5 | 3.3 | 0.87 | N.T. | N.T. | N.T. | N.T. | N.T. | N.T. |

| | | | | | | | | | |
|---|---|---|---|---|---|---|---|---|---|
| N.D.: Not detected N.T.: Not tested | | | | | | | | | |

**[Table 11]**

| Binding affinity to TL1A | | | | | | | | | |
|---|---|---|---|---|---|---|---|---|---|
| | Human TL1A | | | Cynomolgus monkey TL1A | | | Mouse TL1A | | |
| | Kₐ (1/Ms) 1xE+05 | K_{d} (1/s) 1xE-04 | K_{D} (nM) | Kₐ (1/Ms) 1xE+05 | K_{d} (1/s) 1xE-04 | K_{D} (nM) | Kₐ (1/Ms) 1xE+05 | K_{d} (1/s) 1xE-04 | K_{D} (nM) |
| 1 | 3.9 | 3.5 | 0.89 | 3.8 | 3.5 | 0.92 | 8.4 | 4.9 | 0.58 |
| 2 | 8.4 | 9.1 | 1.1 | 7.9 | 8.8 | 1.1 | 17 | 20 | 1.2 |
| 3 | 8.3 | 3.0 | 0.36 | 7.6 | 2.9 | 0.38 | 31 | 23 | 0.73 |
| 4 | 8.7 | 3.6 | 0.42 | N.T. | N.T. | N.T. | N.T. | N.T. | N.T. |
| 5 | 7.1 | 3.3 | 0.47 | N.T. | N.T. | N.T. | N.T. | N.T. | N.T. |
| 6 | 6.3 | 3.3 | 0.53 | N.T. | N.T. | N.T. | N.T. | N.T. | N.T. |
| 7 | 6.1 | 3.1 | 0.51 | N.T. | N.T. | N.T. | N.T. | N.T. | N.T. |

| | | | | | | | | | |
|---|---|---|---|---|---|---|---|---|---|
| N.T.: Not tested | | | | | | | | | |

**[Table 12]**

| Cynomolgus monkey FasL | | | | | | | | | |
|---|---|---|---|---|---|---|---|---|---|
| | Human FasL | | | Cynomolgus monkey FasL | | | Mouse FasL | | |
| | Kₐ (1/Ms) 1xE+05 | K_{d} (1/s) 1xE-04 | K_{D} (nM) | Kₐ (1/Ms) 1xE+05 | K_{d} (1/s) 1xE-04 | K_{D} (nM) | Kₐ (1/Ms) 1xE+05 | K_{d} (1/s) 1xE-04 | K_{D} (nM) |
| 1 | 3.1 | 4.0 | 1.3 | 3.0 | 3.3 | 1.1 | 1.5 | 97 | 65 |
| 2 | 8.6 | 15 | 1.7 | 7.5 | 8.4 | 1.1 | 5.8 | 56 | 96 |
| 3 | 10 | 7.8 | 0.76 | 8.4 | 4.0 | 0.47 | 12 | 587 | 47 |
| 4 | 10 | 10 | 1.0 | N.T. | N.T. | N.T. | N.T. | N.T. | N.T. |
| 5 | 8.5 | 12 | 1.4 | N.T. | N.T. | N.T. | N.T. | N.T. | N.T. |
| 6 | 5.6 | 13 | 2.3 | N.T. | N.T. | N.T. | N.T. | N.T. | N.T. |
| 7 | 5.7 | 15 | 2.5 | N.T. | N.T. | N.T. | N.T. | N.T. | N.T. |

| | | | | | | | | | |
|---|---|---|---|---|---|---|---|---|---|
| N.T.: Not tested | | | | | | | | | |

### [Example 8] Evaluation of Binding Activity to OPG Ligands (RANKL, TRAIL)

It has been known that RANKL binds to RANK and is involved in bone resorption, and that TRAIL binds to a TRAIL receptor and is involved in cell death. Using various human DcR3 recombinants produced, the binding activities to RANKL and TRAIL were analyzed by ELISA. As positive controls that bind to the OPG ligands, K194-Fc corresponding to a variant lacking a C-terminus region of OPG, produced in Example 5, RANK-Fc (Enzo Life Sciences, Inc.) and TRAIL R1-Fc (R&D systems, Inc.) were used. As a negative control, an anti-DNP antibody (IgG1) corresponding to an antibody obtained by inserting the nucleotide sequence of the variable region of the anti-2,4-dinitrophenol (DNP) antibody mentioned in Clin. Cancer Res., 2005, 11(8), p.3126-3135 into a vector encoding Fc of IgG1, followed by introduction into CHO cells to express, and performing Protein A purification, was used.

Into 96-well plates (MAXISORP NUNC-IMMUNO PLATE, Thermo Scientific), anti-human IgG1 (American Qualex International, Inc.) prepared to 10 µg/mL with PBS was dispensed at 50 µL/well, followed by allowing to stand at 4°C overnight for adsorption. After removal of the immobilizing solution, 1% Block Ace produced by dissolving 1 g of Block Ace powder (DS Pharma Biomedical Co., Ltd.) in 100 mL of water was dispensed at 100 µL/well, followed by allowing to stand at room temperature for 1 hour to perform blocking, and the plates were washed 3 times with PBS containing 0.1% Tween (hereinafter referred to as PBST). Then, into plate No. 1, various human DcR3 recombinants or various DcR3 variants and RANK-Fc diluted to the concentration of 1 µg/mL with 1% BSA-PBS were dispensed at 50 µL/well, and into plate No. 2, various wild-type DcR3 controls or various DcR3 variants and TRAIL R1-Fc diluted to the concentration of 1 µg/mL with 1% BSA-PBS were dispensed at 50 µL/well, followed by allowing to stand at room temperature for 1 hour.

After each plate was washed 3 times with PBST, into plate No. 1, RANKL (Peprotech, Inc.) diluted to 0.64 to 50,000 pg/mL with 1% BSA-PBS was dispensed at 50 µL/well, and into plate No. 2, TRAIL (Peprotech, Inc.) to 0.64 to 50,000 pg/mL diluted with 1% BSA-PBS was dispensed at 50 µL/well, followed by allowing to stand at room temperature for 1 hour.

After each plate was washed 3 times with PBST, into plate No. 1, a biotinylated anti-RANKL antibody (Peprotech, Inc.) diluted to 0.4 µg/mL with 1% BSA-PBS was dispensed at 50 µL/well, and into plate No. 2, a biotinylated anti-TRAIL antibody (R&D Systems, Inc.) diluted to 0.4 µg/mL with 1% BSA-PBS was dispensed at 50 µL/well, followed by allowing to stand at room temperature for 1 hour.

After each plate was washed 3 times with PBST, streptavidin-HRP (PIERCE) diluted 10,000-fold with 0.1% Block Ace was dispensed thereinto at 50 µL/well, followed by allowing to stand at room temperature for 1 hour.

After each plate was washed 3 times with PBST, TMB+ Substrate Chromogen (Dako) was dispensed at 50 µL/well, followed by allowing to stand at room temperature for 1 minute. 0.5 mol/L sulfuric acid solution was dispensed at 50 µL/well to stop the chromogenic reaction, and the absorbances at a sample wavelength of 450 nm and a reference wavelength of 570 nm were measured using a plate reader.

As a result, K194-Fc showed binding to RANKL and TRAIL, which are OPG ligands, and RANK-Fc and TRAIL R1-Fc showed binding to RANKL and TRAIL, respectively, but DcR3 FL-Fc (R&D Systems, Inc.), S195-Fc, chimera A-Fc (IEGRMD g1S) and chimera B-Fc (IEGRMD g1S) did not show binding to any of the ligands (FIG. 8). In conjunction with the results of Example 7, it was confirmed that DcR3 variants produced by substituting a part of CRDs of DcR3 with a part of CRDs of OPG show binding activities to DcR3 ligands equivalent to that of wild-type DcR3, but do not bind to OPG ligands.

### [Example 9] Measurement of Neutralizing Activity to DcR3 Ligand

By the following method, the neutralizing activities of various wild-type DcR3 controls and DcR3 variants to LIGHT, TL1A and FasL were measured. A DNP antibody was produced by the methods mentioned in Examples 5 and 8.

### (1) Measurement of Neutralizing Activity to LIGHT

Using a human colon cancer cell HT-29 cell line (ATCC Number: HTB-38), the neutralizing activities of various wild-type DcR3 controls and various DcR3 variants were measured using IL-8 production by addition of LIGHT as an index. For cell culture and evaluation of the neutralizing activity, a McCoy's 5A medium (Gibco) supplemented with 10% FBS (Gibco) and penicillin/streptomycin (Nacalai Tesque, Inc.) was used.

The cells of HT-29 cell line were seeded at 2 x 10⁴ cells/well in a 96-well adherent culture plate (Sumitomo Bakelite Co., Ltd.), and then various wild-type DcR3 controls or various DcR3 variants were added at the final concentration of 0.1, 1 or 10 µg/mL. Thereafter, human LIGHT (Gly66-Val240) including a FLAG tag (DYKDDDDK) added to the N-terminus (mentioned in JP 2013153749 A) was added at the final concentration of 0.1 µg/mL so that the total amount of the culture solution was adjusted at 200 µL/well. After culture in a 5% CO₂ incubator at 37°C for 3 days, the culture supernatant was collected, and the IL-8 concentration in the culture supernatant was measured using AlphaLISA IL-8 Immunoassay Research Kit (PerkinElmer, Inc.).

As a result of the measurement, it was confirmed that, in each of the various human DcR3 variants, the production amount of IL-8 is concentration-dependently decreased, and the neutralizing activity to LIGHT is exhibited (FIG. 9).

### (2) Measurement of Neutralizing Activity to TL1A

Using a human T cell, the neutralizing activities of various wild-type DcR3 controls and various DcR3 variants were measured using IFN-γ production by addition of TL1A as an index. For cell culture and evaluation of the neutralizing activity, an X-VIVO15 medium (Lonza K.K.) was used.

Frozen healthy individual PBMCs (AllCells, LLC) were thawed in a water bath at 37°C, and suspended in a medium containing DNaseI (STEMCELL Technologies Inc.) warmed to 37°C. After shaking at a low speed at 37°C for 2 hours, T cells were isolated using EasySep Human T cell Enrichment Kit (STEMCELL Technologies Inc.). The isolated T cells were seeded at 1 x 10⁵ cells/well in a 96-well suspension culture plate (Sumitomo Bakelite Co., Ltd.), and then various wild-type DcR3 controls or various DcR3 variants were added at the final concentration of 0.1, 1 or 10 µg/mL. Thereafter, the recombinant His10 human TL1A produced in Example 6 was added at the final concentration of 0.1 µg/mL. Human IL-12 (Miltenyi Biotec K.K.) at a final concentration of 2 ng/mL and Recombinant Human IL-18 (MEDICAL & BIOLOGICAL LABORATORIES CO., LTD.) at a final concentration of 50 ng/mL were added so that the total amount of the culture solution was adjusted to 200 µL/well. After culture in a 5% CO₂ incubator at 37°C for 3 days, the culture supernatant was collected, and the IFN-γ concentration in the culture supernatant was measured using AlphaLISA IFN-γ Immunoassay Research Kit (PerkinElmer, Inc.).

As a result of the measurement, it was confirmed that, in each of the various DcR3 variants, the production amount of IFN-γ is concentration-dependently decreased, and the neutralizing activity to TL1A is exhibited (FIG. 10).

### (3) Measurement of Neutralizing Activity to FasL

Using a T-cell leukemia Jurkat cell line (DSMZ Number: ACC 282), the neutralizing activities of various wild-type DcR3 controls and various DcR3 variants were measured using apoptosis by addition of FasL as an index. For cell culture and evaluation of the neutralizing activity, an RPMI1640 medium (Nacalai Tesque, Inc.) supplemented with 10% FBS and penicillin/streptomycin was used.

The cells of Jurkat cell line were seeded at 5 x 10⁴ cells/well in a 96-well suspension culture plate, and then various wild-type DcR3 controls or DcR3 variants were added at the final concentration of 0.01, 0.1 or 1 µg/mL. Thereafter, recombinant human His6 Fas Ligand (Cell Signaling Technology Japan, K.K.) was added at the final concentration of 0.1 µg/mL so that the total amount of the culture solution was adjusted to 100 µL/well. After culture in a 5% CO₂ incubator at 37°C overnight, CellTiter-Glo (Promega Corporation) was added at 100 µL/well in a Jurkat culture plate, and using a luminometer (Veritas, Promega Corporation), the viable cell count using the production amount of ATP as an index was measured.

As a result of the measurement, it was confirmed that, in each of the various DcR3 variants, the production amount of ATP derived from viable cells is concentration-dependently increased, and the neutralizing activity to FasL is exhibited (FIG. 11).

### [Example 10] Neutralizing Activity and Binding Activity of Variant with lowered FasL binding activity to Ligand

Based on chimera A-Fc (g4PEK), one-amino-acid substitutes that have binding and neutralizing activities to TL1A and LIGHT of DcR3 ligands, but have low binding and neutralizing activities to FasL of DcR3 ligands were produced and evaluated. Variants were produced by substituting an appropriately-selected amino acid of CRD2 or CRD3 with Ala or an amino acid other than Ala as shown in Table 13, and the neutralizing activities to DcR3 ligands were measured by the same method as in Example 9. As a result, the neutralizing activities to FasL were selectively lowered in one-amino-acid substitutes, i.e., chimera A-E57K-Fc (g4PEK) (amino acid sequence: SEQ ID NO: 94, nucleotide sequence of DNA: SEQ ID NO: 93) and chimera A-E57L-Fc (g4PEK) (amino acid sequence: SEQ ID NO: 96, nucleotide sequence of DNA: SEQ ID NO: 95), which were obtained by substituting Glu at position 57 from the N-terminus of chimera A-Fc (g4PEK) with Lys and Leu, respectively, and chimera A-R60K-Fc (g4PEK) (amino acid sequence: SEQ ID NO: 98, nucleotide sequence of DNA: SEQ ID NO: 97), which was obtained by substituting Arg at position 60 with Lys (Table 13).

Regarding each of the variants exhibiting the selectively-lowered neutralizing activities to FasL, i.e., chimera A-E57K-Fc (g4PEK) (SEQ ID NO: 94), chimera A-E57L-Fc (g4PEK) (SEQ ID NO: 96) and chimera A-R60K-Fc (g4PEK) (SEQ ID NO: 98), the binding activities to DcR3 ligands were measured by the same method as in Example 7. As a result, in each of the variants of chimera A-E57K-Fc (g4PEK), chimera A-E57L-Fc (g4PEK) and chimera A-R60K-Fc (g4PEK), only the binding activity to FasL was remarkably lowered, and the binding activities to TL1A and LIGHT were maintained (FIG. 12A, B, C).

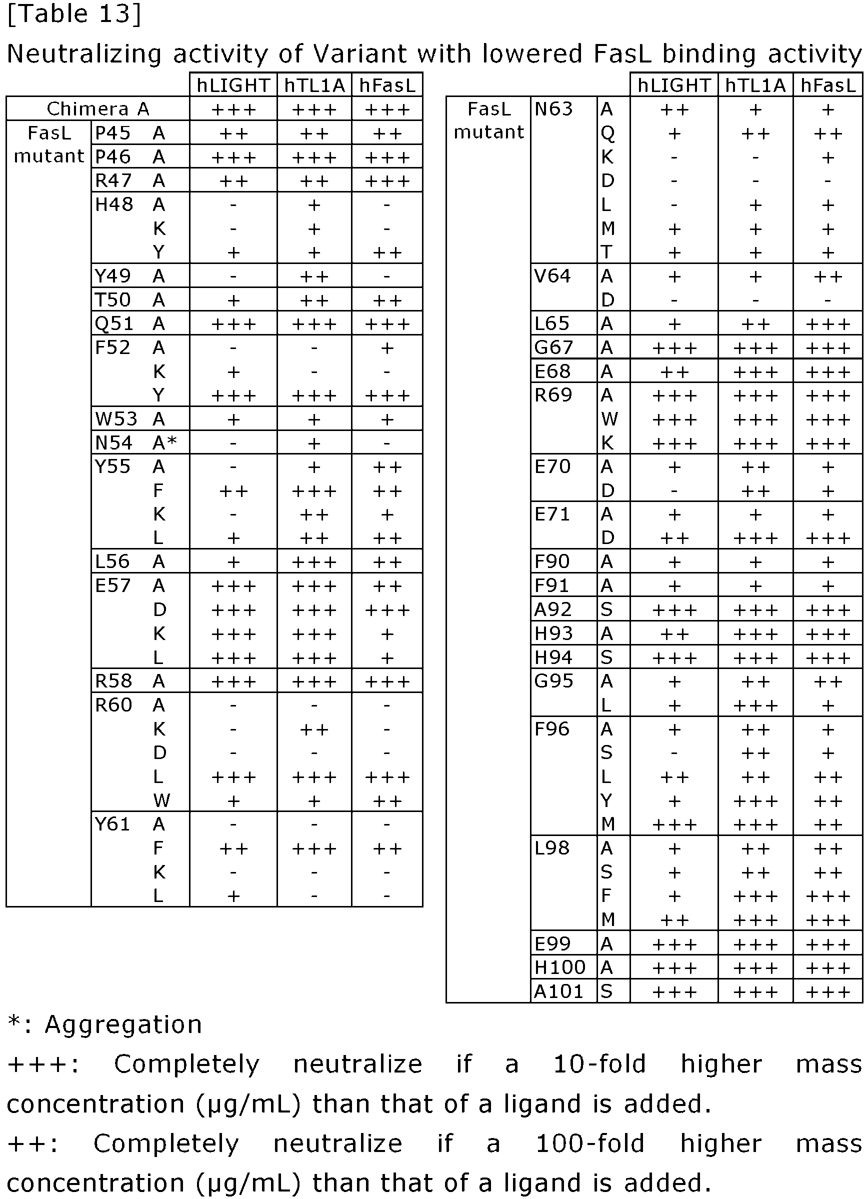

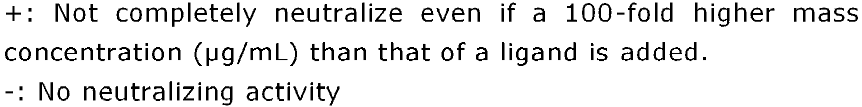

### [Example 11] Evaluation of Aggregability of Commercially Available Wild-Type DcR3 in Mammalian Cell

By the same method as in Example 1, commercially available full-length DcR3-Fc (Abcam plc.) produced using HEK293 cells as host cells, commercially available full-length DcR3-Fc (AdipoGen Life Sciences, Inc.) produced using CHO cells as host cells, and a commercially available Fc fusion (Enzo Life Sciences, Inc.) of a DcR3 molecule lacking about half of the C-terminal side of HBD produced using HEK293 cells as host cells were electrophoresed under reducing conditions and non-reducing conditions. The molecular weights of monomers expected from the mobilities under reducing conditions were about 50 kDa to 60 kDa, while the mobilities under non-reducing conditions were greatly higher than the molecular weights of dimers expected, and all of the commercially available products mostly existed as aggregates (FIG. 13).

### [Example 12] Production of Variant with lowered FasL binding activity

In order to select variants that bind to TL1A and LIGHT among DcR3 ligands and have the selectively-lowered binding activity to FasL, one-amino-acid substitutes (E57X; X is any amino acid other than Glu) were produced by introducing substitution of Glu at position 57 (E57) from the N-terminus with an amino acid other than Glu into chimera A-Fc (g4PEK) (SEQ ID NO: 82),. Regarding E57K, E57L, E57R and E57V (which correspond to substitution of E57 with Lys, Leu, Arg and Val, respectively), Fc (g4PEK) fusions each including a two-amino-acid substitute (E57X_W53Z, E57X_N54Z, E57X_Y55Z, E57X_L56Z, E57X_R58Z; a variant number shown in FIG. 14A is imparted to each two-amino-acid substitute) were produced by introducing in addition to one of E57K, E57L, E57R and E57V, one of further substitution of Trp at position 53 (W53) from the N-terminus, Asn at position 54 (N54) from the N-terminus, Tyr at position 55 (Y55) from the N-terminus, Leu at position 56 (L56) from the N-terminus, and Arg at position 58 (R58) from the N-terminus, which are amino acids in the vicinity of E57K, E57L, E57R and E57V, with a certain amino acid Z (Z is selected from Asp, Glu, Asn, Gln, Pro, Thr and Gly) into chimera A-Fc (g4PEK) (SEQ ID NO: 82).

Of these, regarding chimera A-E57K (amino acid sequence: SEQ ID NO: 66, nucleotide sequence of DNA: SEQ ID NO: 65), chimera A-E57R (amino acid sequence: SEQ ID NO: 180, nucleotide sequence of DNA: SEQ ID NO: 179), chimera A-E57V (amino acid sequence: SEQ ID NO: 182, nucleotide sequence of DNA: SEQ ID NO: 181), chimera A-E57K_R58D (variant number: 45-10, amino acid sequence: SEQ ID NO: 184, nucleotide sequence of DNA: SEQ ID NO: 183), chimera A-E57K_R58E (variant number: 45-18, amino acid sequence: SEQ ID NO: 186, nucleotide sequence of DNA: SEQ ID NO: 185) and chimera A, fusions with some of various mutation-introduced Fc sequences shown in Table 14 were produced (nucleotide sequence: SEQ ID NO: 213, 217, 219, 221, 223, 227, 231, 233, 235, 237, 255, 259, 261, 263, 265, 149, 167, 169, 171, 173, 175, 177, amino acid sequence: SEQ ID NO: 214, 218, 220, 222, 224, 228, 232, 234, 236, 238, 256, 260, 262, 264, 266, 317, 319, 320, 321, 322, 324, 326, 327, 328, 329, 331, 333, 334, 335, 336, 150, 168, 170, 172, 174, 176, 178). In Table 14, E216 represents Glu at EU-index position 216 of a human IgG1 heavy chain. As mutation-introduced sites, C220S, M252Y, S254T, T256E, N434A, L234A, L235A and G237A represent substitution of Cys at EU-index position 220 of a human IgG1 heavy chain with Ser, substitution of Met at EU-index position 252 of the human IgG1 heavy chain with Tyr, substitution of Ser at EU-index position 254 of the human IgG1 heavy chain with Thr, substitution of Thr at EU-index position 256 of the human IgG1 heavy chain with Glu, substitution of Asn at EU-index position 434 of the human IgG1 heavy chain with Ala, substitution of Leu at EU-index position 234 of the human IgG1 heavy chain with Ala, substitution of Leu at EU-index position 235 of the human IgG1 heavy chain with Ala, and substitution of Gly at EU-index position 237 of the human IgG1 heavy chain with Ala, respectively.

**[Table 14]**

| Fc name | E216 | Mutation introduced site | SEQ ID NO |
|---|---|---|---|
| Eg1S | Include | C220S | 156 |
| Eg1S YTE | Include | C220S, M252Y, S254T, T256E | 158 |
| Eg1S N434A | Include | C220S, N434A | 160 |
| Eg1S LALAGANA | Include | C220S, L234A, L235A, G237A, N434A | 166 |
| Eg1S LALAGA | Include | C220S, L234A, L235A, G237A | 164 |
| g1S | Not include | C220S | 72 |
| g1S YTE | Not include | C220S, M252Y, S254T, T256E | 311 |
| g1S N434A | Not include | C220S, N434A | 312 |
| g1S LALAGANA | Not include | C220S, L234A, L235A, G237A, N434A | 313 |
| g1S LALAGA | Not include | C220S, L234A, L235A, G237A | 162 |

Regarding chimera A-Fc (g1S) (nucleotide sequence: SEQ ID NO: 149, amino acid sequence: SEQ ID NO: 150), a variant obtained by deleting the IEGRMD sequence from chimera A-Fc (IEGRMD g1S) (SEQ ID NO: 80) including a linker sequence IEGRMD (SEQ ID NO: 106), produced in Example 2, was produced by stable expression in CHO cells using the method mentioned in Example 6. Regarding chimera A-Fc (Eg1S) (nucleotide sequence: SEQ ID NO: 167, amino acid sequence: SEQ ID NO: 168), a sequence obtained by linking chimera A (SEQ ID NO: 54) and Eg1S to each other was produced by stable expression in CHO cells by the method mentioned in Example 6.

Regarding each of the chimera A-Fc variants (Eg1S YTE, Eg1S N434A, Eg1S LALAGA, Eg1S LALAGANA) (nucleotide sequence: SEQ ID NO: 169, 171, 175, 177, amino acid sequence: SEQ ID NO: 170, 172, 176, 178), a sequence obtained by fusing each Fc to the C-terminus of chimera A (SEQ ID NO: 54) was obtained by transient expression in CHO-S cells.

Regarding each of the chimera A-Fc variants (g1S YTE, g1S N434A, g1S LALAGA, g1S LALAGANA) (amino acid sequence: SEQ ID NO: 314, 315, 174, 316), a sequence obtained by fusing each Fc to the C-terminus of chimera A (SEQ ID NO: 54) was obtained by transient expression in Expi293 cells.

A plasmid expressing a fusion of each amino acid substitute and Fc (g4PEK) was produced as follows: using a DNA sequence of either chimera A-Fc (g4PEK) or E57X-Fc (g4PEK; X is K, R or V) as a template, and using a PCR primer designed to include a mutation site, two regions of a region from an NheI site to a site into which an amino acid substitution was introduced and a region from the site into which an amino acid substitution was introduced to an SalI site each were subjected to PCR amplification, and the resultant products were inserted under a CMV promoter of a pCIpuro vector by the same method as in Example 1.

Regarding a plasmid expressing a fusion of each amino acid substitute and each Fc of Eg1S YTE, Eg1S N434A or Eg1S LALAGANA, using a DNA sequence encoding one of chimera A-Fc variants including the respective mutated Fc sequences as a template, and using a PCR primer designed to include a mutation site, each of two regions, i.e., a region extending from an EcoRI site to a site into which an amino acid substitution was introduced and a region extending from the site into which an amino acid substitution was introduced to a Bsu36I site was subjected to PCR amplification, and each of the resultant products was inserted into the EcoRI and Bsu36I sites of the chimera A-Fc vector including the mutated Fc sequence used as a template to produce the plasmid.

Regarding a plasmid expressing a fusion of each amino acid substitute and each Fc of g1S YTE, g1S N434A or g1S LALAGANA, using a DNA sequence encoding one of the abovementioned amino acid substitute-Fc variants as a template, and using a PCR primer designed to exclude E216, each of two regions, i.e., a region extending from an EcoRI site to a site of Glu to be deleted and a region extending from the site of Glu to be deleted to a Bsu36I site was subjected to PCR amplification, and each of the resultant products was inserted into the EcoRI and Bsu36I sites of the amino acid substitute-Fc vector used as a template to produce the plasmid.

Each plasmid was introduced into Expi293 cells to transiently express, and affinity purification from the culture supernatant was performed by MabSelect SuRe. Each content of monomers, aggregates and degradants of the chimera A-Fc variants, the one-amino-acid substitute-Fc variants and the two-amino-acid substitute-Fc variants produced was calculated from the peak areas analyzed by SEC-UPLC (ACQUITY UPLC Protein BEH SEC 4.6 mm × 150 mm) (Waters K.K.) or SEC-HPLC (TSKgel SuperSW3000 4.0 µm, 4.6 mm x 300 mm) (Tosoh Corporation) using the same method as in Example 2.

As a result, many of the chimera A-Fc variants, the one-amino-acid substitute-Fc variants and the two-amino-acid substitute-Fc variants produced, regardless of mutated Fc sequences, maintained lower aggregate contents than that of S195-Fc (FIG. 14A, B, C).

### [Example 13] Evaluation of Binding Activity to DcR3 Soluble Trimer Ligand

Regarding each of DcR3-Fc, S195-Fc, the various chimera A-Fc variants including different Fc sequences and the various variants with lowered FasL binding activities produced in Example 12, the binding activities to a soluble human LIGHT trimer, a soluble human TL1A trimer and a soluble human FasL trimer were evaluated by partially modifying the method mentioned in Example 7.

### (1) Production of DcR3 Soluble Trimer Ligand

As a human soluble recombinant LIGHT, a sequence (FLAG-LIGHT) obtained by adding a FLAG tag (DYKDDDDK) to the N-terminus and linking an extracellular region (Asp74-Val240) (SEQ ID NO: 132) of LIGHT to downstream thereof was used (nucleotide sequence: SEQ ID NO: 305, amino acid sequence: SEQ ID NO: 306). As a human soluble recombinant TL1A, a sequence (His6-TL1A) obtained by adding a His tag (His6) and a GS linker (GGGSGGGSGGGS) to the N-terminus and linking an extracellular region (Leu72-Leu251) (SEQ ID NO: 138) of TL1A to downstream thereof was used (nucleotide sequence: SEQ ID NO: 115, amino acid sequence: SEQ ID NO: 116). Each plasmid was produced by the same method as in Example 7, and transient expression by Expi293 cells was performed.

FLAG-LIGHT was purified using ANTI-FLAG M2 Affinity Gel (Sigma-Aldrich Co. LLC). The culture supernatant was passed through a column filled with a resin, washed with a washing buffer (50 mM Tris HCl, 150 mM NaCl, pH 7.4), and eluted with an eluent (0.1M glycine hydrochloride, pH 3.5).

His6-TL1A was purified by the following method. The culture supernatant was passed through a column filled with Complete His-Tag Purification Resin (Roche), washed with a washing buffer (50 mM NaH₂PO₄ pH 8.0, 300 mM NaCl), and eluted with an eluent (50 mM NaH₂PO₄ pH 8.0, 300 mM NaCl, 250 mM imidazole). The eluent substituted with PBS using a NAP column (GE Healthcare), passed through a column filled with Ni Sepharose Fast Flow resin (GE Healthcare), washed with a washing buffer (60 mM imidazole, 20 mM sodium phosphate, 0.5 M NaCl, pH 7.4) prepared using His Buffer Kit (GE Healthcare), and eluted with an elution buffer (250 mM imidazole, 20 mM sodium phosphate, 0.5 M NaCl, pH 7.4).

Each elution buffer of elution fractions of FLAG-LIGHT and His6-TL1A was substituted with PBS using a NAP column (GE Healthcare) and passed through a 0.22 µm filter to perform sterilization. The purified proteins thus obtained were subjected to gel filtration chromatography (SEC) (TSKgel G3000 SWXL 7.8 mm x 300 mm) (Tosoh Corporation) using HPLC (Shimadzu Corporation) to collect trimer fractions.

As a human soluble recombinant FasL, Human His6 Fas Ligand/TNFSF6 (Cell Signaling Technology, Inc.) obtained by adding a His tag (His6) to the N-terminus and linking an extracellular region (Pro134-Leu281) (SEQ ID NO: 144) of FasL to downstream thereof was used. SEC-MALS was performed by the same method as in Example 2 to confirm it was a trimer.

### (2) Measurement of Binding Activity Using BIAcore

Regarding both of DcR3-Fc (R&D Systems, Inc.) and S195-Fc, those mentioned in Example 7 were evaluated. Chimera A-g4PEK (SEQ ID NO: 82) was produced in Example 6. Chimera A-Fc (g1S, Eg1S) was produced in Example 12. As various variants with lowered FasL binding activities, the Fc (g4PEK) fusions produced in Example 9 or Example 12 were evaluated.

The binding activities to human DcR3 trimer ligands were analyzed by the SPR method. BIAcore T-100 (GE Healthcare) was used for measurement of the binding activities to human LIGHT and human TL1A, and BIAcore T-100 (GE Healthcare) or BIAcore T-200 (GE Healthcare) was used for measurement of the binding activity to human FasL. As the buffer, HBS-EP+ Buffer was used.

After 10,000 RU of anti-human antibodies were immobilized to Series S Sensor Chip CM5 using Human Antibody Capture Kit (both of which are manufactured by GE Healthcare), various wild-type DcR3 controls and DcR3 variants were injected at the flow rate of 10 µL/min for 30 seconds and captured. Meanwhile, the buffer containing no protein was injected into a reference flow cell. Thereafter, as an analyte, each human DcR3 trimer ligand diluted to 0.02 to 80 nmol/L was injected at 30 µL/min for 2 minutes to monitor the binding, and subsequently the buffer was injected for 3 minutes to monitor dissociation. Then, 3 mol/L magnesium chloride was injected at 30 µL/min for 1 minute to perform a regeneration reaction. For measurement of the binding activities to human LIGHT and human TL1A, BIAcore T-100 evaluation software and 1:1 Binding model were used, and by regarding each ligand as a trimer (human LIGHT trimer: 62.4 kDa, human TL1A trimer: 66.2 kDa), each kinetic constant (kₐ, k_{d}, K_{D}) was calculated. For measurement of the binding activity to human FasL, BIAcore T-100 evaluation software and 1:1 Binding model or BIAcore T-200 evaluation software and 1:1 Binding model were used, and by regarding the ligand as a monomer (human FasL monomer: 19.8 kDa), each kinetic constant (kₐ, k_{d}, K_{D}) was calculated.

As a result, it was confirmed that each of chimera A-Fc (g1S), chimera A-Fc (Eg1S) and chimera A-Fc (g4PEK) binds to each DcR3 trimer ligand (FIG. 15A).

Of the variants (g4PEK) with lowered FasL binding activities produced in Example 12, in each of the one-amino-acid substitutes, i.e., chimera A-E57K-Fc (g4PEK) (amino acid sequence: SEQ ID NO: 94, nucleotide sequence of DNA: SEQ ID NO: 93) (hereinafter sometimes referred to as "E57K-Fc"), chimera A-E57L-Fc (g4PEK) (amino acid sequence: SEQ ID NO: 96, nucleotide sequence of DNA: SEQ ID NO: 95) (hereinafter sometimes referred to as "E57L-Fc"), chimera A-E57R-Fc (g4PEK) (amino acid sequence: SEQ ID NO: 190, nucleotide sequence of DNA: SEQ ID NO: 189) (hereinafter sometimes referred to as "E57R-Fc"), chimera A-E57V-Fc (g4PEK) (amino acid sequence: SEQ ID NO: 192, nucleotide sequence of DNA : SEQ ID NO: 191) (hereinafter sometimes referred to as "E57V-Fc"), chimera A-E57A-Fc (g4PEK) (amino acid sequence: SEQ ID NO: 288, nucleotide sequence of DNA: SEQ ID NO: 287) (hereinafter sometimes referred to as "E57A-Fc"), chimera A-E57F-Fc (g4PEK) (amino acid sequence: SEQ ID NO: 290, nucleotide sequence of DNA: SEQ ID NO: 289) (hereinafter sometimes referred to as "E57F-Fc"), chimera A-E57H-Fc (g4PEK) (amino acid sequence: SEQ ID NO: 292, nucleotide sequence of DNA: SEQ ID NO: 291) (hereinafter sometimes referred to as "E57H-Fc"), chimera A-E57I-Fc (g4PEK) (amino acid sequence: SEQ ID NO: 294, nucleotide sequence of DNA: SEQ ID NO: 293) (hereinafter sometimes referred to as "E57I-Fc"), and chimera A-E57M-Fc (g4PEK) (amino acid sequence: SEQ ID NO: 296, nucleotide sequence of DNA: SEQ ID NO: 295) (hereinafter sometimes referred to as "E57M-Fc"), and in each of the two-amino-acid substitutes, i.e., chimera A-E57K_R58D-Fc (g4PEK) (variant number: 45-10, amino acid sequence: SEQ ID NO: 194, nucleotide sequence of DNA: SEQ ID NO: 193) (hereinafter sometimes referred to as "45-10-Fc"), chimera A-E57K_R58T-Fc (g4PEK) (variant number: 45-11, amino acid sequence: SEQ ID NO: 298, nucleotide sequence of DNA: SEQ ID NO: 297) (hereinafter sometimes referred to as "45-11-Fc"), chimera A-E57K_R58E-Fc (g4PEK) (variant number: 45-18, amino acid sequence: SEQ ID NO: 196, nucleotide sequence of DNA: SEQ ID NO: 195) (hereinafter sometimes referred to as "45-18-Fc"), chimera A-E57L_R58E-Fc (g4PEK) (variant number: 46-4, amino acid sequence: SEQ ID NO: 300, nucleotide sequence of DNA: SEQ ID NO: 299) (hereinafter sometimes referred to as "46-4-Fc"), chimera A-E57R_R58D-Fc (g4PEK) (variant number: 82-5, amino acid sequence: SEQ ID NO: 198, nucleotide sequence of DNA: SEQ ID NO: 197) (hereinafter sometimes referred to as "82-5-Fc"), chimera A-E57V_R58T-Fc (g4PEK) (variant number: 85-6, amino acid sequence: SEQ ID NO: 302, nucleotide sequence of DNA: SEQ ID NO: 301) (hereinafter sometimes referred to as "85-6-Fc"), and chimera A-E57V_R58E-Fc (g4PEK) (variant number: 85-8, amino acid sequence: SEQ ID NO: 304, nucleotide sequence of DNA: SEQ ID NO: 303) (hereinafter sometimes referred to as "85-8-Fc"), it was confirmed that the K_{D} values to LIGHT trimer and TL1A trimer were less than 3-fold and the K_{D} value to FasL trimer was 3-fold or more or the Rmax value was decreased to less than 5, compared to those of chimera A-Fc (g4PEK), and that the binding activity to FasL was selectively lowered (FIG. 16A, B).

Of these, E57K-Fc, E57L-Fc, E57R-Fc, E57V-Fc, 45-10-Fc, 45-18-Fc and 82-5-Fc (each having g4PEK as an Fc) were purified to 95% or more of monomers by SEC-HPLC (column; TSKgel G3000 SWXL 7.8 mm x 300 mm, Tosoh Corporation, HPLC; Shimadzu Corporation) and subjected to BIAcore measurement, and each kinetic constant is shown in FIG. 15B.

### [Example 14] Evaluation of Neutralizing Activity to DcR3 Soluble Ligand

The neutralizing activities of various DcR3 variants to soluble human LIGHT, soluble human TL1A and soluble human FasL were evaluated by partially modifying the method mentioned in Example 9.

### (1) Production of Various Chimera A-Fc variants including Different Fc Sequences

The chimera A-Fc variants (g1S, Eg1S) produced in Example 12 were used. The chimera A-Fc variants produced in Example 12, each including chimera A and an Fc fused to the C-terminal side of chimera A selected from Eg1S YTE, Eg1S N434A, Eg1S LALAGA and Eg1S LALAGANA shown in Table 14, were produced by transient expression in CHO-S cells, and purified to 95% or more of monomers by SEC (Superdex 200 Increase 10/300 GL) (GE Healthcare) using AKTApurifier (GE Healthcare) for use.

### (2) Neutralizing Activity to Soluble Human LIGHT

Regarding each of the chimera A-Fc variants (g1S, Eg1S, Eg1S YTE, Eg1S N434A, Eg1S LALAGA, Eg1S LALAGANA, g4PEK), the inhibitory activity to LIGHT-dependent CXCL10 production from an IFN-γ-stimulated intestinal myofibroblast (Lonza K.K.) was evaluated. The intestinal myofibroblast was cultured in a collagen I-coated flask (Becton, Dickinson and Company) using an SmGM-2 Bullet Kit (Lonza K.K.) medium. After the cells were seeded at 1 x 10⁴ cells/well in a collagen I-coated 96-well plate (Becton, Dickinson and Company), IFN-γ at the final concentration of 10 ng/mL, the trimer FLAG-LIGHT produced in Example 13 at the final concentration of 20 ng/mL, and various DcR3 variants at each final concentration of 19.5, 78.1, 313, 1,250, 5,000, 20,000 ng/mL, or 4.88, 19.5, 78.1, 313, 1,250, 5,000 ng/mL were added, followed by culture for 3 days. The culture supernatants were collected, and the CXCL10 concentrations in the culture supernatants were measured using CXCL10/IP-10 (human) AlphaLisa Detection Kit (PerkinElmer, Inc.). As a result, it was confirmed that each of the chimera A-Fc variants, regardless of an Fc sequence, concentration-dependently inhibits the CXCL10 production and has neutralizing activity to soluble LIGHT (FIG. 17A, B).

Regarding each of the variants with lowered FasL binding activities, i.e., E57K-Fc, E57L-Fc, E57R-Fc and E57V-Fc (each including g4PEK as an Fc), the inhibitory activity to LIGHT-dependent IL-8 production from HT-29 cells was evaluated by the same method as in Example 9. As a result, it was confirmed that each of the variants concentration-dependently inhibits the IL-8 production and has neutralizing activity to soluble LIGHT (FIG. 17C).

Regarding each of the variants with lowered FasL binding activities, i.e., 45-10-Fc, 45-18-Fc and 82-5-Fc (each including g4PEK as an Fc), similarly to chimera A-Fc, the inhibitory activity to LIGHT-dependent CXCL10 production from an IFN-γ-stimulated intestinal myofibroblast was evaluated under the conditions of 2 x 10⁴ of intestinal myofibroblast per well, and final concentration of 19.5, 78.1, 313, 1,250, 5,000 or 20,000 ng/mL of each test substance. As a result, it was confirmed that each of the variants concentration-dependently inhibits the CXCL10 production and has neutralizing activity to soluble LIGHT (FIG. 17D).

### (3) Neutralizing Activity to Soluble Human TL1A

Regarding each of the DcR3 variants, the inhibitory activity to TL1A-dependent IFN-γ production from IL-12- and IL-18-stimulated human T cells was evaluated by the same method as in Example 9. As a result, it was confirmed that, each of the chimera A-Fc variants, regardless of an Fc sequence, concentration-dependently inhibits the production amount of IFN-γ and has neutralizing activity to soluble TL1A (FIG. 18A, B). Furthermore, it was also confirmed that each of the variants with lowered FasL binding activities, i.e., E57K-Fc, E57L-Fc, E57R-Fc, E57V-Fc, 45-10-Fc, 45-18-Fc and 82-5-Fc (each including g4PEK as an Fc) similarly has neutralizing activity to soluble TL1A (FIG. 18C, D).

### (4) Neutralizing Activity to Soluble Human FasL

Regarding each of the DcR3 variants, the inhibitory activity to FasL-dependent cell death of Jurkat cells or A3 cells corresponding to a Jurkat subclone was evaluated by the same method as in Example 9. As a result, it was confirmed that each of the chimera A-Fc variants, regardless of an Fc sequence, concentration-dependently inhibits cell death of A3 cells and has neutralizing activity to soluble FasL (FIG. 19A, B). Meanwhile, regarding each of the variants with lowered FasL binding activities, i.e., E57K-Fc, E57L-Fc, E57R-Fc, E57V-Fc, 45-10-Fc, 45-18-Fc and 82-5-Fc (each including g4PEK as an Fc), it was confirmed that, in each of the variants, the inhibitory activity to Jurkat cell death was remarkably decreased and the neutralizing activity to soluble FasL was selectively lowered (FIG. 19C, D).

### [Example 15] Evaluation of Binding Activity to Membrane-bound DcR3 Ligand

The binding activities of various DcR3 variants to membrane-bound human LIGHT, membrane-bound human TL1A and membrane-bound human FasL were evaluated by flow cytometry using membrane-bound ligand forced expression cell lines. As the membrane-bound human LIGHT forced expression HEK293 cells, those mentioned in US 8974787 were used. Regarding each of membrane-bound TL1A and membrane-bound FasL, using a commercially available ORF clone (Origene Technologies, Inc.) thereof as a template, a sequence obtained by adding a Met residue and a FLAG tag (DYKDDDDK) to the N-terminus was subjected to PCR amplification (nucleotide sequence: SEQ ID NO: 307, 309, amino acid sequence: SEQ ID NO: 308, 310), and the resultant product was inserted into downstream of a CMV promoter of a pCIpuro vector using InFusion HD Cloning Kit (Clontech Laboratories, Inc.), and *Escherichia coli* OH5α competent cells (TOYOBO CO., LTD.) were transformed.

The plasmids thus obtained were introduced into CHO-K1 cells (ECACC) using Nucleofector and Cell Line Nucleofector Kit T (both of which are manufactured by Lonza K.K.), and subjected to drug selection by 10 µg/mL of Puromycin (Thermo Fisher Scientific, Inc.). The drug-resistant cells thus obtained were stained with a DyLight488-labeled anti-human TL1A antibody (Novus Biologicals, LLC) or an APC-labeled anti-human FasL antibody (BD Pharmingen), and high expression fractions were sorted using a cell sorter (Sony Corporation). After expansion culture, the cells were stained with a PE-labeled anti-human TL1A antibody (Novus Biologicals, LLC) or a PE-labeled anti-human FasL antibody (BioLegend, Inc.), followed by sorting again, thus obtaining a membrane-bound human TL1A or membrane-bound human FasL high expression cell line.

The binding activities of various DcR3 variants to membrane-bound ligand forced expression cell lines and host cells were evaluated using the same method as in Example 5 by changing the following conditions. Regarding HEK293 and a membrane-bound LIGHT forced expression cell line, 1 µg/mL of each protein and 10 ng/mL of a secondary antibody Goat F(ab')₂ Anti-Human IgG R-phycoerythrin Conjugate (Southern Biotechnology Associates, Inc.) were reacted. Regarding membrane-bound TL1A and a membrane-bound FasL forced expression cell line, 1 or 10 µg/mL of each protein and 0.1 or 1 µg/mL of the secondary antibody each were reacted.

As a result, each of the chimera A-Fc variants (g1S, Eg1S, g4PEK) did not react with 293 or CHO-K1 cells used as host cells, but reacted specifically with each cell of membrane-bound ligand forced expression cell line. Therefore, it was confirmed that each of the various chimera A variants including different Fc sequences has binding activities to membrane-bound DcR3 ligands (FIG. 20A, B). Furthermore, regarding each of the variants with lowered FasL binding activities, i.e., E57K-Fc, E57L-Fc, E57R-Fc, E57V-Fc, 45-10-Fc, 45-18-Fc and 82-5-Fc (each including g4PEK as an Fc), the binding activities to ligands were similarly evaluated, and as a result, it was confirmed that the binding activities to membrane-bound human LIGHT and membrane-bound human TL1A were maintained, while the binding activity to membrane-bound human FasL was remarkably decreased except for E57L (FIG. 21A, B, C).

### [Example 16] Evaluation of Binding Activity to DcR3 Primary Ligand

The binding activities of various DcR3 variants to each DcR3 ligand derived from a primary cell were evaluated by the following method.

### (1) Binding Activity to Primary LIGHT

It has been known that expression of membrane-bound LIGHT is induced in activated human T cells (The Journal of Immunology, 2004, 173: p.502-507.). Frozen healthy individual PBMCs (AllCells, LLC) were thawed, and stimulated with PMA (Sigma-Aldrich Co. LLC) at a final concentration of 50 ng/mL and ionomycin (Sigma-Aldrich Co. LLC) at 1 µg/mL overnight, and then the binding of chimera A-Fc (Eg1S) to membrane-bound LIGHT, expression of which was induced in CD3-positive T cells, was evaluated by the following method. As a negative control, the anti-DNP antibody (IgG1) mentioned in Example 8 was used. The stimulated PBMCs were collected and reacted using Human FcR Blocking Reagent (Miltenyi Biotec B.V. & Co. KG), and then a BV421-labeled CD3 antibody (BD Pharmingen), 7-AAD Staining Solution (BD Pharmingen) and chimera A-Fc or anti-DNP antibody at a final concentration of 0.4 µg/mL labeled with Alexa Fluor488 Antibody Labeling Kit (Thermo Scientific) each were added. After reaction, washing was performed, and the fluorescence intensities of Alexa Fluor488 in CD3-positive T cells of viable cell fractions were analyzed with a flow cytometer. For confirmation of expression of membrane-bound LIGHT in the PBMCs stimulated for induction, the PBMCs stimulated for induction were reacted using Human FcR Blocking Reagent (Miltenyi Biotec B.V. & Co. KG), and then a BV510-labeled CD3 antibody (BioLegend, Inc.), 7-AAD Staining Solution (BD Pharmingen) and a PE-labeled LIGHT antibody (LifeSpan BioSciences, Inc.) or a PE-labeled mouse IgG1κ isotype control antibody (BioLegend, Inc.) each were added. After reaction, washing was performed, and the fluorescence intensities of PE in CD3-positive T cells of viable cell fractions were analyzed with a flow cytometer.

As a result, it was confirmed that expression of LIGHT is induced on CD3-positive human T cells in PBMCs stimulated for induction, and that Alexa Fluor488-labeled chimera A-Fc binds to membrane-bound LIGHT on activated CD3-positive human T cells (FIG. 22A, B).

### (2) Binding Activity to Primary TL1A

HUVECs (Lonza K.K.) cultured by the same method as in Example 5 were treated with Recombinant Human IL-1 alpha (R&D Systems, Inc.) at a final concentration of 10 ng/mL and a TACE inhibitor TAPI-1 (Calbiochem) at a final concentration of 20 µM for 24 hours, and the binding of chimera A-Fc (IEGRMD g1S) to membrane-bound TL1A, expression of which was induced, was evaluated by a competition experiment with a TL1A antibody 1D1 1.31 (US2015/0132311). As the TL1A antibody 1D1 1.31, an antibody obtained by linking the amino acid sequences of VL and VH mentioned in US 2015/0132311 to a constant region of human IgG1, followed by transient expression using Expi293 cells and purification using Mabselect SuRe (GE Healthcare) from the culture supernatant by the method mentioned in Example 1, was used. As a negative control, the anti-DNP antibody (IgG1) mentioned in Example 8 was used. The collected cells were reacted with Human FcR Blocking Reagent (Miltenyi Biotec B.V. & Co. KG), and then either anti-DNP antibody, chimera A-Fc or TL1A antibody labeled with Zenon Alexa Fluor 647 Human IgG Labeling Kit (Molecular Probes) was added at the final concentration of 8.3 µg/mL. After reaction, washing was performed, and the fluorescence intensities of Alexa Fluor 647 were analyzed with a flow cytometer. Under the competition conditions, either unlabeled DNP antibody, TL1A antibody or chimera A-Fc at a final concentration of 50 µg/mL was reacted in advance.

As a result, the binding of the labeled chimera A-Fc competed with the unlabeled TL1A antibody and the binding of the labeled TL1A antibody competed with the unlabeled chimera A-Fc, and thus it was confirmed that chimera A binds to membrane-bound TL1A on the stimulated HUVEC (FIG. 23).

### (3) Binding Activity to Primary FasL

The binding of chimera A-Fc (IEGRMD g1S) to primary soluble FasL produced from human T cells stimulated for activation-induced cell death (AICD) was evaluated by the following method. The primary soluble FasL was prepared by the following method. In other words, human T cells isolated from frozen healthy individual PBMCs by the same method as in Example 9 were seeded at 2 x 10⁴ cells/well in a 96-well U-bottom plate (Becton, Dickinson and Company), and cultured with PHA-L (eBioscience, Inc.) at a final concentration of 1 µg/mL for 24 hours, and then IL-2 (Peprotech, Inc.) at a final concentration of 1 µg/mL was added to culture for 5 days. After 5 days, the cells were collected and seeded in a 96-well U-bottom plate to which 5 µg/mL of anti-CD3 antibody OKT3 (BioLegend, Inc.) was immobilized. AICD was induced and culture was performed overnight, and then the culture supernatant was collected, and using Amicon Ultra-15 having a molecular weight cutoff of 10 kDa (Merck Millipore) prerinsed with sterile water, the solution volume was concentrated to one-tenth.

A 96-well immunoplate (Thermo Scientific), to which 10 µg/mL of anti-human IgG antibodies (American Qualex International, Inc.) were immobilized, was blocked with 1% Block Ace (DS Pharma Biomedical Co., Ltd.), and then 20 µg/mL of chimera A-Fc (IEGRMD g1S) or Fas-Fc (R&D Systems, Inc.) was captured. After washing, recombinant FasL (Abcam plc) used as a standard or a 10-fold concentrated AICD culture supernatant was reacted. After washing, a biotinylated anti-FasL antibody (Abcam plc) was reacted. After washing, streptavidin-HRP (PIERCE) was reacted. After washing again, a TMB solution (Abcam plc) was added to develop a color. Then, a 2 N sulfuric acid solution was used to stop the chromogenic reaction, and the absorbance at 450 nm was measured.

The results for the plate on which chimera A-Fc (IEGRMD g1S) was captured are shown in FIG. 24A, and the results for the plate on which Fas-Fc was captured are shown in FIG. 24B. "CD3" for 10 × AICD supernatant refers to use of the culture supernatant of the T cells stimulated for AICD under the anti-CD3 antibody OKT3 stimulation conditions, and "none" for 10 × AICD supernatant refers to use of the culture supernatant of the T cells not stimulated for AICD. Also regarding the plate on which any of chimera A-Fc or Fas-Fc was captured, FasL was detected only in the culture supernatant of the T cells stimulated for AICD. The results confirmed that chimera A-Fc binds to soluble FasL produced from the T cells stimulated for AICD (FIG. 24A, B).

### [Example 17] Evaluation of Physical Property of DcR3 Variant

Regarding various chimera A-Fc variants (g1S, Eg1S, Eg1S YTE, Eg1S N434A, Eg1S LALAGANA, Eg1S LALAGA, g4PEK) and variants with lowered FasL binding activities, i.e., E57K-Fc, E57L-Fc, E57R-Fc, E57V-Fc, 45-10-Fc, 45-18-Fc and 82-5-Fc (each having g4PEK as an Fc), using the same method as in Example 4, the elution times (min) were calculated by hydrophobic interaction chromatography (HIC) and the Tm values (°C) were calculated by the differential scanning fluorimetry (DSF) method.

As a result, it was confirmed that introduction of multiple amino acid mutations into an IgG1 Fc sequence does not remarkably affect the hydrophobicity. Since the elution time of any of variants with lowered FasL binding activities did not remarkably differ from that of chimera A-g4PEK, it was confirmed that one-amino-acid or two-amino-acid substitution does not affect the hydrophobicity (FIG. 25).

Regarding the Tm value by DSF, of amino acid substitution or insertion in an IgG1 Fc sequence, introduction of a YTE mutation affected the thermostability. Since the Tm value for any of variants with lowered FasL binding activities did not remarkably differ from that of chimera A-g4PEK, it was confirmed that introduction of one-amino-acid or two-amino-acid substitution into a part of CRDs of DcR3 does not affect the thermostability (FIG. 26).

### [Example 18] Evaluation of Mouse In Vivo Kinetics of DcR3 Variant

Regarding chimera A-Fc (Eg1S) and the variants with lowered FasL binding activities, i.e., E57K-Fc, E57L-Fc, E57R-Fc, E57V-Fc, 45-10-Fc, 45-18-Fc and 82-5-Fc (each having g4PEK as an Fc), *in vivo* kinetics in mice were evaluated by the same method as the method mentioned in Example 6. Chimera A-Fc (Eg1S) was produced by stable expression in CHO-K1 cells and each of the variants with lowered FasL binding activities was produced by transient expression in CHO-S cells, and purified to 95% or more of monomers by SEC (Superdex 200 Increase 10/300 GL) (GE Healthcare) using AKTApurifier (GE Healthcare) for use.

When single i.v. administration of 10 mg/kg of each DcR3 variant was performed to 5- to 6-week-old BALB/c mice (female) (n = 2 or 3), the half-life in blood (h) during the elimination phase and the area under the concentration-time curve to infinity AUC0-∞ (µg*h·mL) after single administration were calculated (FIG. 27). As a standard substance in measurement of the concentration of each DcR3 variant in serum of a mouse to which E57K-Fc, E57R-Fc or E57V-Fc was administered, each DcR3 variant produced by transient expression in Expi293 cells was used.

As a result, each of the DcR3 variants showed greater improvement in AUC as compared to S195-Fc (IEGRMD g1S) corresponding wild-type DcR3 mentioned in Example 6.

### [Example 19] Evaluation of In Vivo Drug Efficacy of DcR3 Variant

*In vivo* drug efficacy of chimera A-Fc (g4PEK) was evaluated in a mouse acute xenogeneic graft versus host disease (GVHD) model.

### (1) Production of Mouse Acute Xenogeneic GVHD Model

A mouse acute xenogeneic GVHD model was produced by the same method as mentioned in JP 5209625 B2. Using 6-week-old severe combined immunodeficient (SCID) female mice, on days -2 and -5, 100 µg of rat anti-mouse IL2 receptor-β (IL2Rβ) chain antibody TMβI (Bio X Cell, Inc.) was intraperitoneally injected to deplete endogenous mouse natural killer cells. On day -1, using a CellRad X-ray irradiator (Faxitron), irradiation treatment with a sublethal dose of 1.7 Gy was performed to the mice. On day 0, 3 x 10⁶ human PBMCs (AllCells, LLC) were intraperitoneally transferred, and subsequently 300 µg of chimera A-Fc or a DNP antibody (both are g4PEK) prepared in 100 µL of PBS was intraperitoneally injected. Regarding the chimera A-Fc administration group, 300 µg of chimera A-Fc was additionally administered on days 4 and 8. After 12 days, gross pathological scores by GVHD reaction were determined, and in addition, spleen was collected from sacrificed mice, and the human cell count in the spleen was evaluated.

### (2) Evaluation of GVHD Pathological Score

The gross pathological conditions observed on day 12 were scored based on four indices of fur, erythema in the intestinal tract, activity and weight loss (FIG. 28A). Regarding indices, none, mild and severe were scored as 0, 1 and 2, respectively, and by summing the scores of all indices, the GVHD pathological scores were determined.

As a result, an increase in the GVHD pathological scores by transfer of human PBMCs and a decrease in the pathological scores by administration of chimera A-Fc as compared to the DNP antibody administration group were confirmed, and drug efficacy by chimera A-Fc was observed (FIG. 28B).

### (3) Measurement of Human Cell Count in Spleen

Mouse spleen was collected, and using gentleMACS Dissociators (Miltenyi Biotec B.V. & Co. KG), the spleen was homogenized to prepare a splenic cell suspension. After a hemolysis treatment with Lysing buffer (BD Biosciences), staining was performed with a PE/Cy7-labeled anti-human CD45 antibody, an FITC-labeled anti-human CD3 antibody, an APC-labeled anti-human CD4 antibody and a PE-labeled anti-human CD8 antibody (all of which are manufactured by BioLegend, Inc.), and each human cell subset was analyzed by a flow cytometry analysis. For measurement of the cell count, CountBright Absolute Counting Beads, for flow cytometry (Thermo Fisher Scientific, Inc.) was used, and the abundance ratio of the fluorescent bead count and each cell subset count detected by flow cytometry was corrected by a known amount of beads added, thus calculating the total cell count in the spleen.

As a result, by transfer of human PBMCs, human cells were detected in mouse spleen, and it was confirmed that the human cell count in mouse spleen is decreased by administration of chimera A-Fc as compared to the DNP antibody administration group (FIG. 29).

### [Example 20] Evaluation of Binding Activity to Soluble DcR3 Ligand Trimer

Regarding each of the chimera A-Fc variants and the variants with lowered FasL binding activities including various mutated Fc sequences produced in Example 12, the binding activities to a soluble human LIGHT trimer, a soluble cynomolgus monkey LIGHT trimer, a soluble human TL1A trimer, a soluble cynomolgus monkey TL1A trimer, a soluble human FasL trimer or a soluble cynomolgus monkey FasL trimer were evaluated as follows by partially modifying the methods mentioned in Examples 7 and 13.

### (1) Production of Soluble DcR3 Ligand Trimer

As human soluble DcR3 ligand trimers, those produced in Example 13 were used. As a cynomolgus monkey soluble recombinant LIGHT, a sequence (FLAG-cynoLIGHT) obtained by adding a FLAG tag (DYKDDDDK) to the N-terminus and linking an extracellular region (Asp74-Val240) (SEQ ID NO: 134) of cynomolgus monkey LIGHT to downstream thereof was used (nucleotide sequence: SEQ ID NO: 341, amino acid sequence: SEQ ID NO: 342). As a cynomolgus monkey soluble recombinant TL1A (His6-cynoTL1A) and a cynomolgus monkey soluble FasL (His6-cynoFasL) (amino acid sequence is SEQ ID NO: 122 and 124, respectively), the same sequences as in Example 7 were used. For LIGHT and TL1A, transient expression by Expi293 was performed, and for FasL, transient expression by CHO-S was performed.

FLAG-cynoLIGHT and His6-cynoTL1A were purified by the method mentioned in Example 13. Regarding His6-cynoFasL, the culture supernatant was passed through a column filled with Ni Sepharose Fast Flow resin (GE Healthcare), washed with a washing buffer (60 mM imidazole, 20 mM sodium phosphate, 0.5 M NaCl, pH 7.4) prepared using His Buffer Kit (GE Healthcare), and eluted with an elution buffer (250 mM imidazole, 20 mM sodium phosphate, 0.5 M NaCl, pH 7.4), and the buffer was substituted with PBS.

Purified FLAG-cynoLIGHT and His6-cynoTL1A were subjected to gel filtration chromatography (SEC) (Superdex 200 Increase 10/300 GL) (GE Healthcare) using AKTApurifier (GE Healthcare) to collect trimer fractions. Purified His6-cynoFasL was analyzed by SEC-UHPLC (apparatus: Nexera X2 (Shimadzu Corporation), column: ACQUITY UPLC Protein BEH SEC 200Å, 1.7 µm, 4.6 × 150 mm (Waters K.K.)) to confirm that 85% or more of purified His6-cynoFasL was trimers.

### (2) Measurement of Binding Activity Using BIAcore

As chimera A-Fc variants and the variants with lowered FasL binding activities including various mutated Fc sequences, the variants mentioned in Example 12 were purified to 95% or more of monomers by SEC (Superdex 200 Increase 10/300 GL) (GE Healthcare) using AKTApurifier (GE Healthcare) or SEC (HiLoad 26/600 Superdex 200 pg) (GE Healthcare) using AKTA pure 25 (GE Healthcare) for use.

The binding activity to human and cynomolgus monkey DcR3 ligand trimers were analyzed by the SPR method. BIAcore T-100 (GE Healthcare) was used for measurement of the binding activities to human LIGHT, cynomolgus monkey LIGHT, human TL1A and cynomolgus monkey TL1A, and BIAcore T-200 (GE Healthcare) was used for measurement of the binding activities to human FasL and cynomolgus monkey FasL. As the buffer, HBS-EP+ Buffer was used.

Various DcR3 variants were injected into Series S Sensor Chip Protein A (GE Healthcare) at 10 µL/min for 30 seconds and captured. Meanwhile, the buffer containing no protein was injected into a reference flow cell. Thereafter, as an analyte, each human DcR3 ligand trimer or each cynomolgus monkey DcR3 ligand trimer diluted to 0.08 to 20 nmol/L was injected. A human LIGHT trimer, a human TL1A trimer or each cynomolgus monkey DcR3 ligand trimer was injected at the flow rate of 30 µL/min for 2 minutes to monitor the binding, and subsequently the buffer was injected for 5 minutes to monitor dissociation. A human FasL trimer was injected at 30 µL/min for 1 minute to monitor the binding, and subsequently the buffer was injected for 3 minutes to monitor dissociation. Then, 3 mol/L magnesium chloride was injected at 30 µL/min for 1 minute to perform a regeneration reaction. For measurement of the binding activities to human LIGHT, cynomolgus monkey LIGHT, human TL1A and cynomolgus monkey TL1A, BIAcore T-100 evaluation software and 1:1 Binding model were used, and for measurement of the binding activities to human FasL and cynomolgus monkey FasL, BIAcore T-200 evaluation software and 1:1 Binding model were used, and by regarding each DcR3 ligand as a trimer (human LIGHT trimer: 62.4 kDa, cynomolgus monkey LIGHT trimer: 57.9 kDa, human TL1A trimer: 66.2 kDa, cynomolgus monkey TL1A trimer: 66.1 kDa, human FasL trimer: 53.1 kDa, cynomolgus monkey FasL trimer: 53.1 kDa), each kinetic constant (kₐ, k_{d}, K_{D}) was calculated.

As a result, it was confirmed that each of chimera A-Fc variants including various mutated Fc sequences (Eg1S, Eg1S YTE, Eg1S N434A, Eg1S LALAGA, Eg1S LALAGANA) (each amino acid sequence: SEQ ID NO: 168, 170, 172, 176, 178) binds to each DcR3 trimer ligand of a human and a cynomolgus monkey (FIG. 30A, B).

Each of the variants with lowered FasL binding activities including various mutated Fc sequences, i.e., E57K-Fc, E57R-Fc, E57V-Fc, 45-10-Fc and 45-18-Fc (each including Eg1S YTE, Eg1S N434A or Eg1S LALAGANA as an Fc) showed no remarkable difference in the K_{D} values to a human LIGHT trimer and a human TL1A trimer compared to those of chimera A-Eg1S. However, in each of these variants with lowered FasL binding activities, it was confirmed that the K_{D} value to a human FasL trimer is greatly increased compared to that of chimera A-Eg1S, and that the binding activity to human FasL is selectively lowered (FIG. 30A).

In each of the variants with lowered FasL binding activities including various mutated Fc sequences, the K_{D} value to a cynomolgus monkey LIGHT trimer or a cynomolgus monkey TL1A trimer was similar to that of chimera A-Eg1S, but in E57R-Fc (Eg1S YTE, Eg1S N434A), the binding activity to a cynomolgus monkey TL1A trimer tended to be attenuated. Meanwhile, in each of the variants with lowered FasL binding activities, it was confirmed that the K_{D} value to a cynomolgus monkey FasL trimer is greatly increased compared to that of chimera A-Eg1S, and that the binding activity to FasL is selectively lowered (FIG. 30B).

### [Example 21] Evaluation of Neutralizing Activity to DcR3 Soluble Ligand

Regarding each of the variants with lowered FasL binding activities including various mutated Fc sequences, evaluation of the neutralizing activities to soluble human LIGHT, soluble human TL1A and soluble human FasL was performed by the same method as in Example 9 or 14. As various DcR3 variants, chimera A-Fc variants produced in Example 12 or the variants with lowered FasL binding activities produced by transient expression in CHO-S cells, i.e., E57K-Fc variants and 45-18-Fc variants (each including Eg1S YTE or Eg1S LALAGANA as an Fc) were purified to 95% or more of monomers by the method mentioned in Example 14(1) or SEC (HiLoad 16/600 Superdex 200 pg) (GE Healthcare) using AKTApurifier (GE Healthcare) for use.

### (1) Neutralizing Activity to Soluble Human LIGHT

Regarding each of various DcR3 variants, the inhibitory activity to LIGHT-dependent CXCL10 production from an IFN-γ-stimulated intestinal myofibroblast was evaluated by the method mentioned in Example 14. As a result, it was confirmed that each of chimera A-Fc variants, i.e., E57K-Fc variants and 45-18-Fc variants (each including Eg1S YTE or Eg1S LALAGANA as an Fc) concentration-dependently inhibits the CXCL10 production and has neutralizing activity to soluble LIGHT. The results are shown in FIG. 31.

### (2) Neutralizing Activity to Soluble Human TL1A

Regarding each of various DcR3 variants, the inhibitory activity to TL1A-dependent IFN-γ production from IL-12- and IL-18-stimulated human T cells was evaluated by the method mentioned in Example 9. As a result, it was confirmed that each of chimera A-Fc variants, E57K-Fc variants and 45-18-Fc variants (each including Eg1S YTE or Eg1S LALAGANA as an Fc) concentration-dependently inhibits the IFN-γ production and has neutralizing activity to soluble TL1A. The results are shown in FIG. 32.

### (3) Neutralizing Activity to Soluble Human FasL

Regarding each of various DcR3 variants, the inhibitory activity to FasL-dependent cell death of Jurkat cells was evaluated by the method mentioned in Example 9. As a result, it was confirmed that each of chimera A-Fc variants (Eg1S YTE, Eg1S LALAGANA) concentration-dependently inhibits cell death of Jurkat cells and has neutralizing activity to soluble FasL. Meanwhile, regarding E57K-Fc variants and 45-18-Fc variants (each including Eg1S YTE or Eg1S LALAGANA as an Fc), it was confirmed that, in each of the variants, the inhibitory activity to Jurkat cell death is remarkably decreased and the neutralizing activity to soluble FasL is selectively lowered. The results are shown in FIG. 33.

In FIGS. 31 to 33, the solid line and X (-×-) represents an anti-DNP antibody as a negative control; the solid line and solid circle (-●-) represents chimera A-Fc (Eg1S YTE); the solid line and open triangle (-△-) represents E57K-Fc (Eg1S YTE): the solid line and open circle (-∘-) represents 45-18-Fc (Eg1S YTE); the dotted line and solid circle (--●--) represents chimera A-Fc (Eg1S LALAGANA); the dotted line and open triangle (--Δ--) represents E57K-Fc (Eg1S LALAGANA); and the dotted line and open circle (--∘--) represents 45-18-Fc (Eg1S LALAGANA).

### [Example 22] Evaluation of Binding Activity to Membrane-bound DcR3 Ligand

Regarding each of the chimera A variants and the variants with lowered FasL binding activities including various mutated Fc sequences, the binding activities to membrane-bound human LIGHT, membrane-bound human TL1A and membrane-bound human FasL were evaluated by the same method as in Example 15.

Chimera A-Fc (Eg1S) was produced by cells of a stable expression cell line from CHO cells, and purified to 95% or more of monomers by SEC for use. All of chimera A-Fc variants (Eg1S YTE, Eg1S N434A, Eg1S LALAGA, Eg1S LALAGANA) were produced by transient expression in CHO-S cells, and purified to 95% or more of monomers by SEC for use. All of the variants with lowered FasL binding activities, i.e., E57K-Fc, E57R-Fc, E57V-Fc, 45-10-Fc and 45-18-Fc (each including Eg1S YTE or Eg1S LALAGANA as an Fc) were produced by transient expression in Expi293F cells, and purified to 95% or more of monomers by SEC for use. In all cases, SEC was performed by the same method as in Example 21.

With the cell lines each forcedly expressing a membrane molecule of human LIGHT, human TL1A and human FasL (also referred to as LIGHT/HEK293, TL1A/CHO and FasL/CHO, respectively) mentioned in Example 15, and HEK293 and CHO-K1 cells, which were used host cells thereof, the abovementioned samples were reacted at a final concentration of 10 µg/mL, and a secondary antibody Goat F(ab')2 Anti-Human IgG R-phycoerythrin Conjugate (Southern Biotechnology Associates, Inc.) was reacted at a final concentration of 100 ng/mL, and the fluorescence intensity of PE was analyzed with a flow cytometer. The results are shown in FIGS. 34A, 34B and 34C. In FIG. 34A, each of the solid bars represents reactivity to LIGHT/HEK293, and each of the open bars represents reactivity to an HEK293 cell. In FIG. 34B, each of the solid bars represents reactivity to TL1A/CHO, and each of the open bars represents reactivity to a CHO-K1 cell. In FIG. 34C, each of the solid bars represents reactivity to FasL/CHO, and each of the open bars represents reactivity to a CHO-K1 cell.

As a result, as shown in FIGs. 34A and 34B, it was confirmed that each of chimera A-Fc variants (Eg1S, Eg1S YTE, Eg1S N434A, Eg1S LALAGA, Eg1S LALAGANA), E57K-Fc, E57R-Fc, E57V-Fc, 45-10-Fc and 45-18-Fc (each including Eg1S YTE or Eg1S LALAGANA as an Fc) binds to membrane-bound human LIGHT and membrane-bound TL1A. Meanwhile, as shown in FIG. 34C, it was confirmed that each of the variants with lowered FasL binding activities shows remarkably decreased binding activity to membrane-bound FasL as compared to chimera A variants including various mutated Fc sequences.

### [Example 23] Evaluation of Neutralizing Activity to Membrane-bound DcR3 Ligand

Regarding each of chimera A variants and the variants with lowered FasL binding activities including various mutated Fc sequences, the neutralizing activities to membrane-bound human LIGHT, membrane-bound human TL1A and membrane-bound human FasL were evaluated by the method shown below. All of chimera A-Fc, E57K-Fc and 45-18-Fc (each including Eg1S YTE or Eg1S LALAGANA as an Fc) were produced by transient expression in CHO-S, and, similarly to Example 21, purified to 95% or more of monomers by SEC for use.

### (1) Neutralizing Activity to Membrane-bound Human LIGHT

Regarding each of chimera A-Fc, E57K-Fc and 45-18-Fc (each including Eg1S YTE or Eg1S LALAGANA as an Fc), the neutralizing activity to membrane-bound human LIGHT was evaluated using a human intestinal myofibroblast (Lonza K.K.) and using CXCL10 production by co-culture with the cells of the membrane-bound LIGHT forced expression HEK293 cell line (LIGHT/HEK293) used in Example 22 as an index.

Human intestinal myofibroblasts were seeded at 1 × 10⁴ cells/well in a Collagen I coat 96 well plate (Corning Incorporated), and cultured at 37°C overnight. After fixation with Fixation Buffer (BD Biosciences), 1 × 10⁴ cells/well of LIGHT/HEK293s washed with PBS and a medium, recombinant human IFN-γ (FUJIFILM Wako Pure Chemical Corporation) at the final concentration of 10 ng/mL, chimera A-Fc, E57K-Fc and 45-18-Fc (in all of which Fc is Eg1S YTE or Eg1S LALAGANA) at the final concentration of 4.88, 19.5, 78.1, 313, 1,250 or 5,000 ng/mL were added so that the total amount of the culture solution was adjusted to 200 µL/well. The culture supernatants after culture for 4 days were collected, and the CXCL10 concentrations in the culture supernatants were measured using CXCL10/IP-10 (human) AlphaLISA Detection Kit (PerkinElmer, Inc.). The results are shown in FIG. 35. In FIG. 35, the solid line and X (-×-) represents an anti-DNP antibody as a negative control; the solid line and solid circle (-●-) represents chimera A-Fc (Eg1S YTE); the solid line and open triangle (-Δ-) represents E57K-Fc (Eg1S YTE); the solid line and open circle (-∘-) represents 45-18-Fc (Eg1S YTE); the dotted line and solid circle (--●--) represents chimera A-Fc (Eg1S LALAGANA); the dotted line and open triangle (--△--) represents E57K-Fc (Eg1S LALAGANA); and the dotted line and open circle (--∘--) represents 45-18-Fc (Eg1S LALAGANA).

As shown in FIG. 35, it was confirmed that each of chimera A-Fc, E57K-Fc and 45-18-Fc (each including Eg1S YTE or Eg1S LALAGANA as an Fc) concentration-dependently inhibits the CXCL10 production by membrane-bound LIGHT, and that each of chimera A and the variants with lowered FasL binding activities, i.e., E57K-Fc and 45-18-Fc, has neutralizing activity to membrane-bound LIGHT.

### (2) Neutralizing Activity to Membrane-bound Human TL1A

Regarding each of chimera A-Fc, E57K-Fc and 45-18-Fc (each including Eg1S YTE or Eg1S LALAGANA as an Fc), the neutralizing activity to membrane-bound human TL1A was evaluated using human CD4-positive T cells and using IFN-γ production by co-culture with the cells of the membrane-bound TL1A forced expression CHO-K1 cell line (TL1A/CHO) used in Example 22 as an index.

From frozen healthy individual PBMCs (AllCells, LLC), CD4-positive T cells were isolated using EasySep Human CD4+ T cell Isolation Kit (STEMCELL Technologies Inc.). The isolated CD4-positive T cells were seeded at 2 × 10⁵ cells/well in a 96-well suspension culture plate, and then Human IL-12 (Miltenyi Biotec K.K.) at the final concentration of 2 ng/mL, Recombinant Human IL-18 (MEDICAL & BIOLOGICAL LABORATORIES CO., LTD.) at the final concentration of 50 ng/mL, and Human IL-15 (Miltenyi Biotec K.K.) at the final concentration of 25 ng/mL were added, followed by fixation with Fixation Buffer (BD Biosciences). Thereafter, membrane-bound TL1A expression CHO-K1 cells washed with PBS and a medium at the concentration of 3 × 10⁴ cells/well, and chimera A-Fc, E57K-Fc and 45-18-Fc (each including Eg1S YTE or Eg1S LALAGANA as an Fc) at the final concentration of 20, 50, 100, 250, 500 or 1,000 ng/mL were added so that the total amounts of the culture solution were adjusted to 200 µL/well. The culture supernatants after culture for 2 days were collected, and the IFN-γ concentrations in the culture supernatants were measured using AlphaLISA IFN-γ Immunoassay Research Kit (PerkinElmer, Inc.). The results are shown in FIG. 36. In FIG. 36, the solid line and X (-×-) represents an anti-DNP antibody as a negative control; the solid line and solid circle (-●-) represents chimera A-Fc (Eg1S YTE); the solid line and open triangle (-△-) represents E57K-Fc (Eg1S YTE); the solid line and open circle (-∘-) represents 45-18-Fc (Eg1S YTE); the dotted line and solid circle (--●--) represents chimera A-Fc (Eg1S LALAGANA); the dotted line and open triangle (--Δ--) represents E57K-Fc (Eg1S LALAGANA); and the dotted line and open circle (--∘--) represents 45-18-Fc (Eg1S LALAGANA).

As shown in FIG. 36, it was confirmed that each of chimera A-Fc, E57K-Fc and 45-18-Fc (each including Eg1S YTE or Eg1S LALAGANA as an Fc) concentration-dependently inhibits the IFN-γ production by membrane-bound TL1A, and that each of the variants with lowered FasL binding activities, i.e., E57K-Fc and 45-18-Fc, maintains neutralizing activity to membrane-bound TL1A similar to that of chimera A-Fc.

### (3) Neutralizing Activity to Membrane-bound Human FasL

Regarding each of chimera A-Fc, E57K-Fc and 45-18-Fc (each including Eg1S YTE or Eg1S LALAGANA as an Fc), the neutralizing activity to membrane-bound human FasL was measured using as an index apoptosis of Jurkat cells caused by co-culture with a cell line forcedly expressing membrane-bound FasL modified so as not to be cleaved to a soluble form.

A plasmid expressing a membrane-bound human FasL variant sequence (nucleotide sequence: SEQ ID NO: 343, amino acid sequence: SEQ ID NO: 344) obtained by adding a FLAG tag (DYKDDDDK) to the N-terminus and removing a FasL cleavage site (Glu128-Ile131) (Nat. Med., 1998, 4(1), p.31-36) and a proline-rich region of an intracellular region (Pro8-Pro69) (Nat. Med., 1996, 2(3), p.317-322) was produced by the following method. Using the membrane-bound FasL expression plasmid mentioned in Example 15 as a template and a primer designed to delete a target sequence, each of three regions, i.e., a region extending from a PstI site to Tyr7, a region extending from Pro70 to Leu127 and a region extending from Gly132 to a NotI site each was subjected to PCR amplification, and each of the resultant products was inserted under a CMV promoter of a pCIpuro vector by the same method as in Example 1. The plasmid thus obtained was introduced into CHO-K1 cells by the same method as in Example 15, and a membrane-bound human FasL variant high expression CHO-K1 cell line (FasLdel/CHO) was obtained by cell sorting of PE-labeled anti-human FasL antibody (BioLegend, Inc.)-positive fractions.

The FasLdel/CHO was seeded at 5 × 10³ cells/well in a 96-well adherent culture plate (Sumitomo Bakelite Co., Ltd.), and cultured in a 5% CO₂ incubator at 37°C overnight, followed by washing the cells once. Jurkat cells were seeded at 5 x 10⁴ cells/well therein, and chimera A-Fc, E57K-Fc and 45-18-Fc (in all of which Fc is Eg1S YTE or Eg1S LALAGANA) were added at the final concentration of 24.4, 97.7, 391, 1,563, 6,250 or 25,000 ng/mL so that the total amount of the culture solution was adjusted to 100 µL/well. After culture in a 5% CO₂ incubator at 37°C for 4 hours, only Jurkat cells were collected. The collected Jurkat cells were seeded in a 96-well U-bottom plate (Falcon), and stained using PE annexin V apoptosis detection kit (BD Biosciences) at room temperature for 15 minutes. After the cells were washed with an FCM buffer, the fluorescence intensity was analyzed by flow cytometer FACS Fortessa (BD Biosciences), and Annexin V-positive cells were detected as a dead cell subset. The results are shown in FIG. 37. In FIG. 37, the solid line and X (-×-) represents an anti-DNP antibody as a negative control; the solid line and solid circle (-●-) represents chimera A-Fc (Eg1S YTE); the solid line and open triangle (-△-) represents E57K-Fc (Eg1S YTE); the solid line and open circle (-∘-) represents 45-18-Fc (Eg1S YTE); the dotted line and solid circle (--●--) represents chimera A-Fc (Eg1S LALAGANA); the dotted line and open triangle (--Δ- -) represents E57K-Fc (Eg1S LALAGANA); and the dotted line and open circle (--∘--) represents 45-18-Fc (Eg1S LALAGANA).

As shown in FIG. 37, it was confirmed that chimera A-Fc (Eg1S YTE, Eg1S LALAGANA) concentration-dependently inhibits cell death of Jurkat cells and has neutralizing activity to membrane-bound FasL. Meanwhile, regarding each of E57K-Fc and 45-18-Fc (each including Eg1S YTE or Eg1S LALAGANA as an Fc), it was confirmed that, in each of the variants, the inhibitory activity to Jurkat cell death is hardly exhibited even in the 25,000 ng/mL high concentration group and the neutralizing activity to membrane-bound FasL is remarkably decreased.

### [Example 24] Evaluation of Binding Activity to Soluble DcR3 Ligand Derived from Human Primary Cell

Regarding each of chimera A-Fc, E57K-Fc and 45-18-Fc (each including Eg1S YTE or Eg1S LALAGANA as an Fc), the binding activities to various soluble DcR ligands were evaluated using soluble DcR3 ligands derived from human primary cells. All of the test substances were produced by transient expression in CHO-S, and, similarly to Example 21, purified to 95% or more of monomers by SEC for use.

### (1) Binding Activity to Primary LIGHT

Regarding each of chimera A-Fc, E57K-Fc and 45-18-Fc (each including Eg1S YTE or Eg1S LALAGANA as an Fc), the binding activity to primary soluble LIGHT produced from activated human T cells was evaluated by the following method. The primary soluble LIGHT was prepared by the following method. In a 6-well adherent culture plate (Sumitomo Bakelite Co., Ltd.) to which 10 µg/mL of anti-CD3 antibody OKT3 (BioLegend, Inc.) was immobilized, human T cells isolated from thawed frozen healthy individual PBMCs (AllCells, LLC) were seeded at 7 × 10⁶ cells/well, and anti-CD28 antibodies (BioLegend, Inc.) at a final concentration of 1 µg/mL were added. After culture in a 5% CO₂ incubator for 3 days, the culture supernatant was collected. The concentration of primary soluble LIGHT in the collected culture supernatant was measured using Human LIGHT/TNFSF14 Quantikine ELISA Kit (R&D Systems, Inc.), and it was confirmed that soluble LIGHT existed at 14 pg/mL in the culture supernatant of unstimulated human T cells and at 5.6 ng/mL in the culture supernatant of human T cells stimulated with an anti-CD3 antibody and an anti-CD28 antibody.

Then, 10 µg/mL of anti-human IgG antibodies (American Qualex International, Inc.) were immobilized to a 96-well immunoplate (Thermo Scientific), and blocking was performed with 2% Block Ace (DS Pharma Biomedical Co., Ltd.), and then 2 µg/mL of chimera A-Fc, E57K-Fc and 45-18-Fc (in all of which Fc is Eg1S YTE or Eg1S LALAGANA) were captured. The culture supernatant of human T cells stimulated for LIGHT production was concentrated 6-fold using Amicon Ultra-15 having a molecular weight cutoff of 3 kDa (Merck Millipore) prerinsed with sterile water. After the plate was washed, the 6-fold concentrated culture supernatant was reacted. As a standard of LIGHT, the soluble recombinant FLAG-LIGHT trimer produced in Example 13 was used. After the plate was washed, a biotinylated anti-LIGHT antibody (R&D Systems, Inc.) was reacted. After the plate was washed, streptavidin-HRP (PIERCE) was reacted. After washing again, a TMB solution (Dako) was added to develop a color. Then, a 2 N sulfuric acid solution was used to stop the chromogenic reaction, and the absorbance at 450 nm (reference wavelength of 570 nm) was measured.

The results for the soluble recombinant FLAG-LIGHT trimer are shown in FIG. 38A, and the results for the culture supernatant of the human T cells stimulated for LIGHT expression are shown in FIG. 38B. In FIG. 38A, the solid line with X (-×-) represents an anti-DNP antibody as a negative control; the solid line with solid circle (-●-) represents chimera A-Fc (Eg1S YTE); the solid line with open square (-o-) represents chimera A-Fc(Eg1S); the solid line with open triangle (-Δ-) represents E57K-Fc (Eg1S YTE); the solid line with open circle (-∘-) represents 45-18-Fc (Eg1S YTE); the dotted line with solid circle (--●--) represents chimera A-Fc (Eg1S LALAGANA); the dotted line with open triangle (--Δ--) represents E57K-Fc (Eg1S LALAGANA); and the dotted line with open circle (--∘--) represents 45-18-Fc (Eg1S LALAGANA). In FIG. 38B, "6×sup. CD3/28" represented by the solid bar represents use of the 6-fold concentrated culture supernatant of human T cells stimulated for LIGHT expression under anti-CD3 antibody OKT3 and anti-CD28 antibody stimulation conditions, and "6×sup. none" represented by the open bar represents use of the 6-fold concentrated culture supernatant of unstimulated human T cells.

As shown in FIG. 38B, in a well in which each of chimera A-Fc, E57K-Fc and 45-18-Fc (each including Eg1S, Eg1S YTE or Eg1S LALAGANA as an Fc) was captured, LIGHT was detected only in the culture supernatant of human T cells stimulated for LIGHT expression. The results confirmed that each of chimera A-Fc, E57K-Fc and 45-18-Fc (each including Eg1S, Eg1S YTE or Eg1S LALAGANA as an Fc) binds to soluble LIGHT produced from human T cells stimulated with an anti-CD3 antibody and an anti-CD28 antibody.

### (2) Binding Activity to Primary TL1A

Regarding each of chimera A-Fc, E57K-Fc and 45-18-Fc (each including Eg1S YTE or Eg1S LALAGANA as an Fc), the binding activity to primary soluble TL1A produced from an activated human PBMC was evaluated. The primary soluble TL1A was prepared by the following method. To a 6-well adherent culture plate (Sumitomo Bakelite Co., Ltd.), 250 µg/mL of human IgG antibodies (Jackson ImmunoResearch Laboratories, Inc.) were immobilized, and further anti-human κ light chain antibodies (Bethyl Laboratories, Inc.) and anti-human λ light chain antibodies (Bethyl Laboratories, Inc.) were added at each final concentration of 100 µg/mL to treat for 1 hour, thus producing the immune complex. In the plate, thawed frozen healthy individual PBMCs (AllCells, LLC) were seeded at 6 × 10⁶ cells/well, followed by culture in a 5% CO₂ incubator at 37°C. After 3 days, the culture supernatant was collected, and concentrated 4-fold using Amicon Ultra-15 having a molecular weight cutoff of 3 kDa (Merck Millipore) prerinsed with sterile water.

Sandwich ELISA was performed by the same method as in (1). On a 96-well immunoplate, chimera A-Fc, E57K-Fc and 45-18-Fc (in all of which Fc is Eg1S YTE or Eg1S LALAGANA) or the anti-human TL1A antibody produced in Example 16 was captured. Then, with the plate, a 4-fold concentrated culture supernatant of PBMCs stimulated for TL1A production or as a standard, the soluble recombinant His6-TL1A trimer produced in Example 13 was reacted, followed by detection with a biotinylated anti-TL1A antibody (R&D Systems, Inc.).

The results for the soluble recombinant His6-TL1A trimer are shown in FIG. 38C, and the results for the culture supernatant of PBMCs stimulated for TL1A production are shown in FIG. 38D. In FIG. 38C, the solid line with X (-×-) represents an anti-DNP antibody as a negative control; the solid line with solid circle (-●-) represents chimera A-Fc (Eg1S YTE); the solid line with open square (-o-) represents chimera A-Fc (Eg1S); the solid line with open triangle (-△-) represents E57K-Fc (Eg1S YTE); the solid line with open circle (-∘-) represents 45-18-Fc (Eg1S YTE); the dotted line with solid circle (--●--) represents chimera A-Fc (Eg1S LALAGANA); the dotted line with open triangle (-△--) represents E57K-Fc (Eg1S LALAGANA); the dotted line with open circle (--∘--) represents 45-18-Fc (Eg1S LALAGANA); and the solid line with solid triangle (-A-) represents an anti-TL1A antibody. In FIG. 38D, "4×sup. I.C." represented by the solid bar represents use of the culture supernatant of PBMCs stimulated for TL1A expression under immune complex stimulation conditions, and "4×sup. none" represented by the open bar represents use of the culture supernatant of unstimulated PBMCs. As shown in FIG. 38D, in a well in which each of chimera A-Fc, E57K-Fc and 45-18-Fc (each including Eg1S, Eg1S YTE or Eg1S LALAGANA as an Fc) was captured, TL1A was detected only in the culture supernatant of PBMCs stimulated for TL1A expression. The results confirmed that chimera A-Fc, E57K-Fc and 45-18-Fc (each including Eg1S, Eg1S YTE or Eg1S LALAGANA as an Fc) binds to soluble TL1A produced from PBMCs stimulated with the immune complex.

### (3) Binding Activity to Primary FasL

Regarding each of chimera A-Fc, E57K-Fc and 45-18-Fc (each including Eg1S YTE or Eg1S LALAGANA as an Fc), the binding activity to primary soluble human FasL was evaluated. As the primary soluble FasL, soluble FasL produced from human T cells stimulated for activation-induced cell death (AICD) was prepared by the same method as in Example 16 for use.

Sandwich ELISA was performed by the same method as in (1). On a 96-well immunoplate, chimera A-Fc, E57K-Fc and 45-18-Fc (each including Eg1S YTE or Eg1S LALAGANA as an Fc) or Fas-Fc (R&D Systems, Inc.) was captured, and then a 6-fold concentrated culture supernatant of human T cells stimulated for AICD or as a standard, Human His6 Fas Ligand/TNFSF6 (Cell Signaling Technology, Inc.) was reacted, followed by detection with a biotinylated anti-FasL antibody included with Human Fas Ligand ELISA Kit (Abcam plc).

The results for the soluble recombinant His6-FasL are shown in FIG. 38E, and the results for the culture supernatant of human T cells stimulated for AICD are shown in FIG. 38F. In FIG. 38E, the solid line with X (-×-) represents an anti-DNP antibody as a negative control; the solid line with solid circle (-●-) represents chimera A-Fc (Eg1S YTE); the solid line with open square (-o-) represents chimera A-Fc(Eg1S); the solid line with open triangle (-Δ-) represents E57K-Fc (Eg1S YTE); the solid line with open circle (-∘-) represents 45-18-Fc (Eg1S YTE); the dotted line with solid circle (--●--) represents chimera A-Fc (Eg1S LALAGANA); the dotted line with open triangle (--Δ- -) represents E57K-Fc (Eg1S LALAGANA); the dotted line with open circle (--∘--) represents 45-18-Fc (Eg1S LALAGANA); and the solid line with solid triangle (-A-) represents Fas-Fc. In FIG. 38F, "6×sup. CD3" represented by the solid bar represents use of the culture supernatant of human T cells stimulated for AICD under anti-CD3 antibody OKT3 stimulation conditions, and "6×sup. none" represented by the open bar represents use of the culture supernatant of human T cells not stimulated for AICD. As shown in FIG. 38F, in a well in which chimera A-Fc (Eg1S, Eg1S YTE, Eg1S LALAGANA) was captured, FasL was detected only in the culture supernatant of human T cells stimulated for AICD. Meanwhile, in a well in which E57K-Fc or 45-18-Fc (each including Eg1S YTE or Eg1S LALAGANA as an Fc) was captured, it was confirmed that FasL detected in the culture supernatant of human T cells stimulated for AICD is remarkably decreased, and that, in each of these variants, the binding activity to soluble FasL derived from human T cells is attenuated.

### [Example 25] Evaluation of Physical Property of DcR3 Variant

Regarding E57K-Fc and 45-18-Fc (each including Eg1S YTE or Eg1S LALAGANA as an Fc) that were produced by transient expression in CHO-S and purified to 95% or more of monomers by SEC similarly to Example 21, using the same method as in Example 4, the elution times (min) were calculated by hydrophobic interaction chromatography (HIC) and the Tm values (°C) were calculated by the differential scanning fluorimetry (DSF) method.

As a result, since all values showed values equivalent to those in Example 17, it was confirmed that a combination of the abovementioned amino acid mutation introduction into an IgG1 Fc sequence and the abovementioned amino acid substitution into the chimera A sequence does not affect the hydrophobicity and the thermostability.

### [Example 26] Evaluation of Mouse In Vivo Kinetics of DcR3 Variant

Regarding chimera A-Fc, E57K-Fc and 45-18-Fc (each including Eg1S YTE or Eg1S LALAGANA as an Fc) that were produced by transient expression in CHO-S and purified to 95% or more of monomers by SEC similarly to Example 21, *in vivo* kinetics in mice were evaluated by the same method as the method mentioned in Example 6. The results are shown in FIG. 39.

As shown in FIG. 39, compared to the AUC value of 354 for S195-Fc (IEGRMD g1S) corresponding to wild-type DcR3 mentioned in Example 6, each of the DcR3 variants showed greater improvement in AUC.

### [Example 27] Evaluation of Aggregability of DcR3 Variant without Fc Fusion

A protein obtained by adding a His6 tag to the C-terminus of human wild-type DcR3 (S195) lacking an HBD region (S195-His6) (nucleotide sequence: SEQ ID NO: 345, amino acid sequence: SEQ ID NO: 346), a protein obtained by adding a His6 tag to the C-terminus of chimera A (chimera A-His6) (nucleotide sequence: SEQ ID NO: 347, amino acid sequence: SEQ ID NO: 348), a protein obtained by adding a His6 tag to the C-terminus of chimera A-E57K (chimera A-E57K-His6) (nucleotide sequence: SEQ ID NO: 349, amino acid sequence: SEQ ID NO: 350), and a protein obtained by adding a His6 tag to the C-terminus of 45-18 (45-18-His6) (nucleotide sequence: SEQ ID NO: 351, amino acid sequence: SEQ ID NO: 352) were produced as follows.

Regarding S195-His6, a sequence obtained by adding a signal peptide sequence to the N-terminus of human DcR3 lacking HBD (DNA fragment: SEQ ID NO: 107) and adding a His6 tag sequence to the C-terminus thereof was artificially synthesized (Fasmac Co., Ltd.). Regarding chimera A-His6, chimera A-E57K-His6 and 45-18-His6, sequences obtained by adding a signal peptide sequence to the N-terminus of chimera A (DNA fragment: SEQ ID NO: 353), chimera A-E57K (DNA fragment: SEQ ID NO: 354) or 45-18 (DNA fragment: SEQ ID NO: 355), and adding a His6 tag sequence to the C-terminus thereof were artificially synthesized. Each artificial synthesis fragment was introduced into a pCIpuro vector by the method mentioned in Example 1, and transient expression by Expi293 cells was performed. Using Amicon Ultra-0.5 having a molecular weight cutoff of 3 kDa (Merck Millipore) prerinsed with sterile water, the culture supernatant of the cells expressing S195-His6 was concentrated 30-fold. Each culture supernatant of the cells expressing chimera A-His6, chimera A-E57K-His6 or 45-18-His6 was diluted 6-fold.

Using these culture supernatants, SDS-PAGE was performed under non-reducing conditions or 100 mM DTT reducing conditions, and then immunoblotting was performed using a mouse anti-6-His tag monoclonal antibody (Thermo Scientific) as a primary antibody and a goat anti-mouse IgG antibody (Southern Biotechnology Associates, Inc.) as a secondary antibody.

The results are shown in FIG. 40. In the culture supernatant of the cells transiently expressing S195-His6, under reducing conditions in lane 1, a band was detected at near 20.9 kDa corresponding to an expected molecular weight, while under non-reducing conditions in lane 5, no bands of monomers were detected, and the whole was smear. The results confirmed that all of S195-His6 existed as aggregates in the culture supernatant. Meanwhile, in all of the culture supernatants of the cells transiently expressing chimera A-His6, chimera A-E57K-His6 and 45-18-His6, under non-reducing conditions in lanes 2 to 4, bands of monomers were detected at near 22.2 kDa corresponding to an expected molecular weight, like reducing conditions in lanes 6 to 8. The results showed that, in the DcR3 variant of the present invention, the aggregate content is greatly decreased compared to that of wild-type DcR3. In other words, it was shown that substitution of CRD1 and CRD4 of DcR3 with CRD1 and CRD4 derived from OPG, respectively, has an effect to decrease aggregation that is observed in wild-type DcR3, and that this effect is exerted regardless of whether the binding activity to FasL is lowered.

### Sequence Listing Free Text

SEQ ID NO: 1: nucleotide sequence of human DcR3 cDNA (Accession Number: NM_003823.3)
SEQ ID NO: 2: amino acid sequence of human DcR3 (Accession Number: NP_003814.1)
SEQ ID NO: 3: nucleotide sequence of human DcR3 (without signal peptide)
SEQ ID NO: 4: amino acid sequence of human DcR3 (without signal peptide)
SEQ ID NO: 5: nucleotide sequence of CRD1 of human DcR3
SEQ ID NO: 6: amino acid sequence of CRD1 of human DcR3
SEQ ID NO: 7: nucleotide sequence of CRD2 of human DcR3
SEQ ID NO: 8: amino acid sequence of CRD2 of human DcR3
SEQ ID NO: 9: nucleotide sequence of CRD3 of human DcR3
SEQ ID NO: 10: amino acid sequence of CRD3 of human DcR3
SEQ ID NO: 11: nucleotide sequence of CRD4 of human DcR3
SEQ ID NO: 12: amino acid sequence of CRD4 of human DcR3
SEQ ID NO: 13: nucleotide sequence of human OPG cDNA (Accession Number: NM_002546.3)
SEQ ID NO: 14: amino acid sequence of human OPG (Accession Number: NP_002537.3)
SEQ ID NO: 15: nucleotide sequence of human OPG (without signal peptide)
SEQ ID NO: 16: amino acid sequence of human OPG (without signal peptide)
SEQ ID NO: 17: nucleotide sequence of CRD1 of human OPG
SEQ ID NO: 18: amino acid sequence of CRD1 of human OPG
SEQ ID NO: 19: nucleotide sequence of CRD2 of human OPG
SEQ ID NO: 20: amino acid sequence of CRD2 of human OPG
SEQ ID NO: 21: nucleotide sequence of CRD3 of human OPG
SEQ ID NO: 22: amino acid sequence of CRD3 of human OPG
SEQ ID NO: 23: nucleotide sequence of CRD4 of human OPG
SEQ ID NO: 24: amino acid sequence of CRD4 of human OPG
SEQ ID NO: 25: nucleotide sequence of chimera B-HBD
SEQ ID NO: 26: amino acid sequence of chimera B-HBD
SEQ ID NO: 27: nucleotide sequence of chimera C-HBD
SEQ ID NO: 28: amino acid sequence of chimera C-HBD
SEQ ID NO: 29: nucleotide sequence of chimera A-HBD
SEQ ID NO: 30: amino acid sequence of chimera A-HBD
SEQ ID NO: 31: nucleotide sequence of 103-123OPG-HBD
SEQ ID NO: 32: amino acid sequence of 103-123OPG-HBD
SEQ ID NO: 33: nucleotide sequence of N131S/N144S-HBD
SEQ ID NO: 34: amino acid sequence of N131S/N144S-HBD
SEQ ID NO: 35: nucleotide sequence of T133A/S146A-HBD
SEQ ID NO: 36: amino acid sequence of T133A/S146A-HBD
SEQ ID NO: 37: nucleotide sequence of N131S/N144S/N157S-HBD
SEQ ID NO: 38: amino acid sequence of N131S/N144S/N157S-HBD
SEQ ID NO: 39: nucleotide sequence of T133A/S146A/T159A-HBD
SEQ ID NO: 40: amino acid sequence of T133A/S146A/T159A-HBD
SEQ ID NO: 41: nucleotide sequence of chimera A-E57K-HBD
SEQ ID NO: 42: amino acid sequence of chimera A-E57K-HBD
SEQ ID NO: 43: nucleotide sequence of chimera A-E57L-HBD
SEQ ID NO: 44: amino acid sequence of chimera A-E57L-HBD
SEQ ID NO: 45: nucleotide sequence of chimera A-R60K-HBD
SEQ ID NO: 46: amino acid sequence of chimera A-R60K-HBD
SEQ ID NO: 47: nucleotide sequence of HBD of wild-type DcR3
SEQ ID NO: 48: amino acid sequence of HBD of wild-type DcR3
SEQ ID NO: 49: nucleotide sequence of chimera B
SEQ ID NO: 50: amino acid sequence of chimera B
SEQ ID NO: 51: nucleotide sequence of chimera C
SEQ ID NO: 52: amino acid sequence of chimera C
SEQ ID NO: 53: nucleotide sequence of chimera A
SEQ ID NO: 54: amino acid sequence of chimera A
SEQ ID NO: 55: nucleotide sequence of 103-123OPG
SEQ ID NO: 56: amino acid sequence of 103-123OPG
SEQ ID NO: 57: nucleotide sequence of N131S/N144S
SEQ ID NO: 58: amino acid sequence of N131S/N144S
SEQ ID NO: 59: nucleotide sequence of T133A/S146A
SEQ ID NO: 60: amino acid sequence of T133A/S146A
SEQ ID NO: 61: nucleotide sequence of N131S/N144S/N157S
SEQ ID NO: 62: amino acid sequence of N131S/N144S/N157S
SEQ ID NO: 63: nucleotide sequence of T133A/S146A/T159A
SEQ ID NO: 64: amino acid sequence of T133A/S146A/T159A
SEQ ID NO: 65: nucleotide sequence of chimera A-E57K
SEQ ID NO: 66: amino acid sequence of chimera A-E57K
SEQ ID NO: 67: nucleotide sequence of chimera A-E57L
SEQ ID NO: 68: amino acid sequence of chimera A-E57L
SEQ ID NO: 69: nucleotide sequence of chimera A-R60K
SEQ ID NO: 70: amino acid sequence of chimera A-R60K
SEQ ID NO: 71: nucleotide sequence of Fc (g1S)
SEQ ID NO: 72: amino acid sequence of Fc (g1S)
SEQ ID NO: 73: nucleotide sequence of Fc (g4PEK)
SEQ ID NO: 74: amino acid sequence of Fc (g4PEK)
SEQ ID NO: 75: nucleotide sequence of chimera B-Fc (IEGRMD g1S)
SEQ ID NO: 76: amino acid sequence of chimera B-Fc (IEGRMD g1S)
SEQ ID NO: 77: nucleotide sequence of chimera C-Fc (IEGRMD g1S)
SEQ ID NO: 78: amino acid sequence of chimera C-Fc (IEGRMD g1S)
SEQ ID NO: 79: nucleotide sequence of chimera A-Fc (IEGRMD g1S)
SEQ ID NO: 80: amino acid sequence of chimera A-Fc (IEGRMD g1S)
SEQ ID NO: 81: nucleotide sequence of chimera A-Fc (g4PEK)
SEQ ID NO: 82: amino acid sequence of chimera A-Fc (g4PEK)
SEQ ID NO: 83: nucleotide sequence of 103-123OPG-Fc (g4PEK)
SEQ ID NO: 84: amino acid sequence of 103-123OPG-Fc (g4PEK)
SEQ ID NO: 85: nucleotide sequence of N131S/N144S-Fc (g4PEK)
SEQ ID NO: 86: amino acid sequence of N131S/N144S-Fc (g4PEK)
SEQ ID NO: 87: nucleotide sequence of T133A/S146A-Fc (g4PEK)
SEQ ID NO: 88: amino acid sequence of T133A/S146A-Fc (g4PEK)
SEQ ID NO: 89: nucleotide sequence of N131S/N144S/N157S-Fc (g4PEK)
SEQ ID NO: 90: amino acid sequence of N131S/N144S/N157S-Fc (g4PEK)
SEQ ID NO: 91: nucleotide sequence of T133A/S146A/T159A-Fc (g4PEK)
SEQ ID NO: 92: amino acid sequence of T133A/S146A/T159A-Fc (g4PEK)
SEQ ID NO: 93: nucleotide sequence of chimera A-E57K-Fc (g4PEK)
SEQ ID NO: 94: amino acid sequence of chimera A-E57K-Fc (g4PEK)
SEQ ID NO: 95: nucleotide sequence of chimera A-E57L-Fc (g4PEK)
SEQ ID NO: 96: amino acid sequence of chimera A-E57L-Fc (g4PEK)
SEQ ID NO: 97: nucleotide sequence of chimera A-R60K-Fc (g4PEK)
SEQ ID NO: 98: amino acid sequence of chimera A-R60K-Fc (g4PEK)
SEQ ID NO: 99: nucleotide sequence of DcR3 FL-Fc
SEQ ID NO: 100: amino acid sequence of DcR3 FL-Fc
SEQ ID NO: 101: nucleotide sequence of S195-Fc
SEQ ID NO: 102: amino acid sequence of S195-Fc
SEQ ID NO: 103: nucleotide sequence of DcR3 FL-FLAG
SEQ ID NO: 104: amino acid sequence of DcR3 FL-FLAG
SEQ ID NO: 105: nucleotide sequence of linker IEGRMD
SEQ ID NO: 106: amino acid sequence of linker IEGRMD
SEQ ID NO: 107: nucleotide sequence of DcR3 (without signal peptide) with HBD deleted
SEQ ID NO: 108: amino acid sequence of DcR3 (without signal peptide) with HBD deleted
SEQ ID NO: 109: nucleotide sequence of FLAG tag
SEQ ID NO: 110: amino acid sequence of FLAG tag
SEQ ID NO: 111: nucleotide sequence of R218Q DcR3
SEQ ID NO: 112: amino acid sequence of R218Q DcR3
SEQ ID NO: 113: nucleotide sequence of soluble recombinant of human LIGHT
SEQ ID NO: 114: amino acid sequence of soluble recombinant of human LIGHT
SEQ ID NO: 115: nucleotide sequence of soluble recombinant of human TL1A
SEQ ID NO: 116: amino acid sequence of soluble recombinant of human TL1A
SEQ ID NO: 117: nucleotide sequence of soluble recombinant of human FasL
SEQ ID NO: 118: amino acid sequence of soluble recombinant of human FasL
SEQ ID NO: 119: nucleotide sequence of soluble recombinant of cynomolgus monkey LIGHT
SEQ ID NO: 120: amino acid sequence of soluble recombinant of cynomolgus monkey LIGHT
SEQ ID NO: 121: nucleotide sequence of soluble recombinant of cynomolgus monkey TL1A
SEQ ID NO: 122: amino acid sequence of soluble recombinant of cynomolgus monkey TL1A
SEQ ID NO: 123: nucleotide sequence of soluble recombinant of cynomolgus monkey FasL
SEQ ID NO: 124: amino acid sequence of soluble recombinant of cynomolgus monkey FasL
SEQ ID NO: 125: nucleotide sequence of soluble recombinant of mouse LIGHT
SEQ ID NO: 126: amino acid sequence of soluble recombinant of mouse LIGHT
SEQ ID NO: 127: nucleotide sequence of soluble recombinant of mouse TL1A
SEQ ID NO: 128: amino acid sequence of soluble recombinant of mouse TL1A
SEQ ID NO: 129: nucleotide sequence of soluble recombinant of mouse FasL
SEQ ID NO: 130: amino acid sequence of soluble recombinant of mouse FasL
SEQ ID NO: 131: nucleotide sequence of extracellular region (Asp74-Val240) of human LIGHT
SEQ ID NO: 132: amino acid sequence of extracellular region (Asp74-Val240) of human LIGHT
SEQ ID NO: 133: nucleotide sequence of extracellular region (Asp74-Val240) of cynomolgus monkey LIGHT
SEQ ID NO: 134: amino acid sequence of extracellular region (Asp74-Val240) of cynomolgus monkey LIGHT
SEQ ID NO: 135: nucleotide sequence of extracellular region (Asp72-Val239) of mouse LIGHT
SEQ ID NO: 136: amino acid sequence of extracellular region (Asp72-Val239) of mouse LIGHT
SEQ ID NO: 137: nucleotide sequence of extracellular region (Leu72-Leu251) of human TL1A
SEQ ID NO: 138: amino acid sequence of extracellular region (Leu72-Leu251) of human TL1A
SEQ ID NO: 139: nucleotide sequence of extracellular region (Leu72-Leu251) of cynomolgus monkey TL1A
SEQ ID NO: 140: amino acid sequence of extracellular region (Leu72-Leu251) of cynomolgus monkey TL1A
SEQ ID NO: 141: nucleotide sequence of extracellular region (Ile94-Leu270) of mouse TL1A
SEQ ID NO: 142: amino acid sequence of extracellular region (Ile94-Leu270) of mouse TL1A
SEQ ID NO: 143: nucleotide sequence of extracellular region (Pro134-Leu281) of human FasL
SEQ ID NO: 144: amino acid sequence of extracellular region (Pro134-Leu281) of human FasL
SEQ ID NO: 145: nucleotide sequence of extracellular region (Pro133-Leu280) of cynomolgus monkey FasL
SEQ ID NO: 146: amino acid sequence of extracellular region (Pro133-Leu280) of cynomolgus monkey FasL
SEQ ID NO: 147: nucleotide sequence of extracellular region (Pro132-Leu279) of mouse FasL
SEQ ID NO: 148: amino acid sequence of extracellular region (Pro132-Leu279) of mouse FasL
SEQ ID NO: 149: nucleotide sequence of chimera A-Fc (g1S)
SEQ ID NO: 150: amino acid sequence of chimera A-Fc (g1S)
SEQ ID NO: 151: nucleotide sequence of OPG K194-Fc
SEQ ID NO: 152: amino acid sequence of OPG K194-Fc
SEQ ID NO: 153: amino acid sequence of constant region of human IgG1 heavy chain
SEQ ID NO: 154: amino acid sequence of constant region of human IgG4 heavy chain
SEQ ID NO: 155: nucleotide sequence of Fc (Eg1S)
SEQ ID NO: 156: amino acid sequence of Fc (Eg1S)
SEQ ID NO: 157: nucleotide sequence of Fc (Eg1S-YTE)
SEQ ID NO: 158: amino acid sequence of Fc (Eg1S-YTE)
SEQ ID NO: 159: nucleotide sequence of Fc (Eg1S-N434A)
SEQ ID NO: 160: amino acid sequence of Fc (Eg1S-N434A)
SEQ ID NO: 161: nucleotide sequence of Fc (g1S-LALAGA)
SEQ ID NO: 162: amino acid sequence of Fc (g1S-LALAGA)
SEQ ID NO: 163: nucleotide sequence of Fc (Eg1S-LALAGA)
SEQ ID NO: 164: amino acid sequence of Fc (Eg1S-LALAGA)
SEQ ID NO: 165: nucleotide sequence of Fc (Eg1S-LALAGANA)
SEQ ID NO: 166: amino acid sequence of Fc (Eg1S-LALAGANA)
SEQ ID NO: 167: nucleotide sequence of chimera A-Fc (Eg1S)
SEQ ID NO: 168: amino acid sequence of chimera A-Fc (Eg1S)
SEQ ID NO: 169: nucleotide sequence of chimera A-Fc (Eg1S-YTE)
SEQ ID NO: 170: amino acid sequence of chimera A-Fc (Eg1S-YTE)
SEQ ID NO: 171: nucleotide sequence of chimera A-Fc (Eg1S-N434A)
SEQ ID NO: 172: amino acid sequence of chimera A-Fc (Eg1S-N434A)
SEQ ID NO: 173: nucleotide sequence of chimera A-Fc (g1S-LALAGA)
SEQ ID NO: 174: amino acid sequence of chimera A-Fc (g1S-LALAGA)
SEQ ID NO: 175: nucleotide sequence of chimera A-Fc (Eg1S-LALAGA)
SEQ ID NO: 176: amino acid sequence of chimera A-Fc (Eg1S-LALAGA)
SEQ ID NO: 177: nucleotide sequence of chimera A-Fc (Eg1S-LALAGANA)
SEQ ID NO: 178: amino acid sequence of chimera A-Fc (Eg1S-LALAGANA)
SEQ ID NO: 179: nucleotide sequence of chimera A-E57R
SEQ ID NO: 180: amino acid sequence of chimera A-E57R
SEQ ID NO: 181: nucleotide sequence of chimera A-E57V
SEQ ID NO: 182: amino acid sequence of chimera A-E57V
SEQ ID NO: 183: nucleotide sequence of chimera A-E57K_R58D
SEQ ID NO: 184: amino acid sequence of chimera A-E57K_R58D
SEQ ID NO: 185: nucleotide sequence of chimera A-E57K_R58E
SEQ ID NO: 186: amino acid sequence of chimera A-E57K_R58E
SEQ ID NO: 187: nucleotide sequence of chimera A-E57R_R58D
SEQ ID NO: 188: amino acid sequence of chimera A-E57R_R58D
SEQ ID NO: 189: nucleotide sequence of chimera A-E57R-Fc (g4PEK)
SEQ ID NO: 190: amino acid sequence of chimera A-E57R-Fc (g4PEK)
SEQ ID NO: 191: nucleotide sequence of chimera A-E57V-Fc (g4PEK)
SEQ ID NO: 192: amino acid sequence of chimera A-E57V-Fc (g4PEK)
SEQ ID NO: 193: nucleotide sequence of chimera A-E57K_R58D-Fc (g4PEK)
SEQ ID NO: 194: amino acid sequence of chimera A-E57K_R58D-Fc (g4PEK)
SEQ ID NO: 195: nucleotide sequence of chimera A-E57K_R58E-Fc (g4PEK)
SEQ ID NO: 196: amino acid sequence of chimera A-E57K_R58E-Fc (g4PEK)
SEQ ID NO: 197: nucleotide sequence of chimera A-E57R_R58D-Fc (g4PEK)
SEQ ID NO: 198: amino acid sequence of chimera A-E57R_R58D-Fc (g4PEK)
SEQ ID NO: 199: nucleotide sequence of chimera A-E57K-Fc (Eg1S)
SEQ ID NO: 200: amino acid sequence of chimera A-E57K-Fc (Eg1S)
SEQ ID NO: 201: nucleotide sequence of chimera A-E57L-Fc (Eg1S)
SEQ ID NO: 202: amino acid sequence of chimera A-E57L-Fc (Eg1S)
SEQ ID NO: 203: nucleotide sequence of chimera A-E57R-Fc (Eg1S)
SEQ ID NO: 204: amino acid sequence of chimera A-E57R-Fc (Eg1S)
SEQ ID NO: 205: nucleotide sequence of chimera A-E57V-Fc (Eg1S)
SEQ ID NO: 206: amino acid sequence of chimera A-E57V-Fc (Eg1S)
SEQ ID NO: 207: nucleotide sequence of chimera A-
E57K_R58D-Fc (Eg1S)
SEQ ID NO: 208: amino acid sequence of chimera A-E57K_R58D-Fc (Eg1S)
SEQ ID NO: 209: nucleotide sequence of chimera A-E57K_R58E-Fc (Eg1S)
SEQ ID NO: 210: amino acid sequence of chimera A-E57K_R58E-Fc (Eg1S)
SEQ ID NO: 211: nucleotide sequence of chimera A-E57R_R58D-Fc (Eg1S)
SEQ ID NO: 212: amino acid sequence of chimera A-E57R_R58D-Fc (Eg1S)
SEQ ID NO: 213: nucleotide sequence of chimera A-E57K-Fc (Eg1S YTE)
SEQ ID NO: 214: amino acid sequence of chimera A-E57K-Fc (Eg1S YTE)
SEQ ID NO: 215: nucleotide sequence of chimera A-E57L-Fc (Eg1S YTE)
SEQ ID NO: 216: amino acid sequence of chimera A-E57L-Fc (Eg1S YTE)
SEQ ID NO: 217: nucleotide sequence of chimera A-E57R-Fc (Eg1S YTE)
SEQ ID NO: 218: amino acid sequence of chimera A-E57R-Fc (Eg1S YTE)
SEQ ID NO: 219: nucleotide sequence of chimera A-E57V-Fc (Eg1S YTE)
SEQ ID NO: 220: amino acid sequence of chimera A-E57V-Fc (Eg1S YTE)
SEQ ID NO: 221: nucleotide sequence of chimera A-E57K_R58D-Fc (Eg1S YTE)
SEQ ID NO: 222: amino acid sequence of chimera A-E57K_R58D-Fc (Eg1S YTE)
SEQ ID NO: 223: nucleotide sequence of chimera A-E57K_R58E-Fc (Eg1S YTE)
SEQ ID NO: 224: amino acid sequence of chimera A-E57K_R58E-Fc (Eg1S YTE)
SEQ ID NO: 225: nucleotide sequence of chimera A-E57R_R58D-Fc (Eg1S YTE)
SEQ ID NO: 226: amino acid sequence of chimera A-E57R_R58D-Fc (Eg1S YTE)
SEQ ID NO: 227: nucleotide sequence of chimera A-E57K-Fc (Eg1S N434A)
SEQ ID NO: 228: amino acid sequence of chimera A-E57K-Fc (Eg1S N434A)
SEQ ID NO: 229: nucleotide sequence of chimera A-E57L-Fc (Eg1S N434A)
SEQ ID NO: 230: amino acid sequence of chimera A-E57L-Fc (Eg1S N434A)
SEQ ID NO: 231: nucleotide sequence of chimera A-E57R-Fc (Eg1S N434A)
SEQ ID NO: 232: amino acid sequence of chimera A-E57R-Fc (Eg1S N434A)
SEQ ID NO: 233: nucleotide sequence of chimera A-E57V-Fc (Eg1S N434A)
SEQ ID NO: 234: amino acid sequence of chimera A-E57V-Fc (Eg1S N434A)
SEQ ID NO: 235: nucleotide sequence of chimera A-E57K_R58D-Fc (Eg1S N434A)
SEQ ID NO: 236: amino acid sequence of chimera A-E57K_R58D-Fc (Eg1S N434A)
SEQ ID NO: 237: nucleotide sequence of chimera A-E57K_R58E-Fc (Eg1S N434A)
SEQ ID NO: 238: amino acid sequence of chimera A-E57K_R58E-Fc (Eg1S N434A)
SEQ ID NO: 239: nucleotide sequence of chimera A-E57R_R58D-Fc (Eg1S N434A)
SEQ ID NO: 240: amino acid sequence of chimera A-E57R_R58D-Fc (Eg1S N434A)
SEQ ID NO: 241: nucleotide sequence of chimera A-E57K-Fc (Eg1S LALAGA)
SEQ ID NO: 242: amino acid sequence of chimera A-E57K-Fc (Eg1S LALAGA)
SEQ ID NO: 243: nucleotide sequence of chimera A-E57L-Fc (Eg1S LALAGA)
SEQ ID NO: 244: amino acid sequence of chimera A-E57L-Fc (Eg1S LALAGA)
SEQ ID NO: 245: nucleotide sequence of chimera A-E57R-Fc (Eg1S LALAGA)
SEQ ID NO: 246: amino acid sequence of chimera A-E57R-Fc (Eg1S LALAGA)
SEQ ID NO: 247: nucleotide sequence of chimera A-E57V-Fc (Eg1S LALAGA)
SEQ ID NO: 248: amino acid sequence of chimera A-E57V-Fc (Eg1S LALAGA)
SEQ ID NO: 249: nucleotide sequence of chimera A-E57K_R58D-Fc (Eg1S LALAGA)
SEQ ID NO: 250: amino acid sequence of chimera A-E57K_R58D-Fc (Eg1S LALAGA)
SEQ ID NO: 251: nucleotide sequence of chimera A-E57K_R58E-Fc (Eg1S LALAGA)
SEQ ID NO: 252: amino acid sequence of chimera A-E57K_R58E-Fc (Eg1S LALAGA)
SEQ ID NO: 253: nucleotide sequence of chimera A-E57R_R58D-Fc (Eg1S LALAGA)
SEQ ID NO: 254: amino acid sequence of chimera A-E57R_R58D-Fc (Eg1S LALAGA)
SEQ ID NO: 255: nucleotide sequence of chimera A-E57K-Fc (Eg1S LALAGANA)
SEQ ID NO: 256: amino acid sequence of chimera A-E57K-Fc (Eg1S LALAGANA)
SEQ ID NO: 257: nucleotide sequence of chimera A-E57L-Fc (Eg1S LALAGANA)
SEQ ID NO: 258: amino acid sequence of chimera A-E57L-Fc (Eg1S LALAGANA)
SEQ ID NO: 259: nucleotide sequence of chimera A-E57R-Fc (Eg1S LALAGANA)
SEQ ID NO: 260: amino acid sequence of chimera A-E57R-Fc (Eg1S LALAGANA)
SEQ ID NO: 261: nucleotide sequence of chimera A-E57V-Fc (Eg1S LALAGANA)
SEQ ID NO: 262: amino acid sequence of chimera A-E57V-Fc (Eg1S LALAGANA)
SEQ ID NO: 263: nucleotide sequence of chimera A-E57K_R58D-Fc (Eg1S LALAGANA)
SEQ ID NO: 264: amino acid sequence of chimera A-E57K_R58D-Fc (Eg1S LALAGANA)
SEQ ID NO: 265: nucleotide sequence of chimera A-E57K_R58E-Fc (Eg1S LALAGANA)
SEQ ID NO: 266: amino acid sequence of chimera A-E57K_R58E-Fc (Eg1S LALAGANA)
SEQ ID NO: 267: nucleotide sequence of chimera A-E57R_R58D-Fc (Eg1S LALAGANA)
SEQ ID NO: 268: amino acid sequence of chimera A-E57R_R58D-Fc (Eg1S LALAGANA)
SEQ ID NO: 269: nucleotide sequence of chimera A-E57A
SEQ ID NO: 270: amino acid sequence of chimera A-E57A
SEQ ID NO: 271: nucleotide sequence of chimera A-E57F
SEQ ID NO: 272: amino acid sequence of chimera A-E57F
SEQ ID NO: 273: nucleotide sequence of chimera A-E57H
SEQ ID NO: 274: amino acid sequence of chimera A-E57H
SEQ ID NO: 275: nucleotide sequence of chimera A-E57I
SEQ ID NO: 276: amino acid sequence of chimera A-E57I
SEQ ID NO: 277: nucleotide sequence of chimera A-E57M
SEQ ID NO: 278: amino acid sequence of chimera A-E57M
SEQ ID NO: 279: nucleotide sequence of chimera A-E57K_R58T
SEQ ID NO: 280: amino acid sequence of chimera A-E57K_R58T
SEQ ID NO: 281: nucleotide sequence of chimera A-E57L_R58E
SEQ ID NO: 282: amino acid sequence of chimera A-E57L_R58E
SEQ ID NO: 283: nucleotide sequence of chimera A-E57V_R58T
SEQ ID NO: 284: amino acid sequence of chimera A-E57V_R58T
SEQ ID NO: 285: nucleotide sequence of chimera A-E57V_R58E
SEQ ID NO: 286: amino acid sequence of chimera A-E57V_R58E
SEQ ID NO: 287: nucleotide sequence of chimera A-E57A-Fc (g4PEK)
SEQ ID NO: 288: amino acid sequence of chimera A-E57A-Fc (g4PEK)
SEQ ID NO: 289: nucleotide sequence of chimera A-E57F-Fc (g4PEK)
SEQ ID NO: 290: amino acid sequence of chimera A-E57F-Fc (g4PEK)
SEQ ID NO: 291: nucleotide sequence of chimera A-E57H-Fc (g4PEK)
SEQ ID NO: 292: amino acid sequence of chimera A-E57H-Fc (g4PEK)
SEQ ID NO: 293: nucleotide sequence of chimera A-E57I-Fc (g4PEK)
SEQ ID NO: 294: amino acid sequence of chimera A-E57I-Fc (g4PEK)
SEQ ID NO: 295: nucleotide sequence of chimera A-E57M-Fc (g4PEK)
SEQ ID NO: 296: amino acid sequence of chimera A-E57M-Fc (g4PEK)
SEQ ID NO: 297: nucleotide sequence of chimera A-E57K_R58T-Fc (g4PEK)
SEQ ID NO: 298: amino acid sequence of chimera A-E57K_R58T-Fc (g4PEK)
SEQ ID NO: 299: nucleotide sequence of chimera A-E57L_R58E-Fc (g4PEK)
SEQ ID NO: 300: amino acid sequence of chimera A-E57L_R58E-Fc (g4PEK)
SEQ ID NO: 301: nucleotide sequence of chimera A-E57V_R58T-Fc (g4PEK)
SEQ ID NO: 302: amino acid sequence of chimera A-E57V_R58T-Fc (g4PEK)
SEQ ID NO: 303: nucleotide sequence of chimera A-E57V_R58E-Fc (g4PEK)
SEQ ID NO: 304: amino acid sequence of chimera A-E57V_R58E-Fc (g4PEK)
SEQ ID NO: 305: nucleotide sequence of soluble recombinant FLAG tagged LIGHT
SEQ ID NO: 306: amino acid sequence of soluble recombinant FLAG tagged LIGHT
SEQ ID NO: 307: nucleotide sequence of TL1A variant in which FLAG tag has been inserted at N-terminus of TL1A
SEQ ID NO: 308: amino acid sequence of TL1A variant in which FLAG tag is inserted at N-terminus of TL1A
SEQ ID NO: 309: nucleotide sequence of FasL variant in which FLAG tag is inserted at N-terminus of FasL
SEQ ID NO: 310: amino acid sequence of FasL variant in which FLAG tag is inserted at N-terminus of FasL
SEQ ID NO: 311: amino acid sequence of Fc (g1S YTE)
SEQ ID NO: 312: amino acid sequence of Fc (g1S N434A)
SEQ ID NO: 313: amino acid sequence of Fc (g1S LALAGANA)
SEQ ID NO: 314: amino acid sequence of chimera A-Fc (g1S YTE)
SEQ ID NO: 315: amino acid sequence of chimera A-Fc (g1S N434A)
SEQ ID NO: 316: amino acid sequence of chimera A-Fc (g1S LALAGANA)
SEQ ID NO: 317: amino acid sequence of chimera A-E57K-Fc (g1S YTE)
SEQ ID NO: 318: amino acid sequence of chimera A-E57L-Fc (g1S YTE)
SEQ ID NO: 319: amino acid sequence of chimera A-E57R-Fc (g1S YTE)
SEQ ID NO: 320: amino acid sequence of chimera A-E57V-Fc (g1S YTE)
SEQ ID NO: 321: amino acid sequence of chimera A-E57K_R58D-Fc (g1S YTE)
SEQ ID NO: 322: amino acid sequence of chimera A-E57K_R58E-Fc (g1S YTE)
SEQ ID NO: 323: amino acid sequence of chimera A-E57R_R58D-Fc (g1S YTE)
SEQ ID NO: 324: amino acid sequence of chimera A-E57K-Fc (g1S N434A)
SEQ ID NO: 325: amino acid sequence of chimera A-E57L-Fc (g1S N434A)
SEQ ID NO: 326: amino acid sequence of chimera A-E57R-Fc (g1S N434A)
SEQ ID NO: 327: amino acid sequence of chimera A-E57V-Fc (g1S N434A)
SEQ ID NO: 328: amino acid sequence of chimera A-E57K_R58D-Fc (g1S N434A)
SEQ ID NO: 329: amino acid sequence of chimera A-E57K_R58E-Fc (g1S N434A)
SEQ ID NO: 330: amino acid sequence of chimera A-E57R_R58D-Fc (g1S N434A)
SEQ ID NO: 331: amino acid sequence of chimera A-E57K-Fc (g1S LALAGANA)
SEQ ID NO: 332: amino acid sequence of chimera A-E57L-Fc (g1S LALAGANA)
SEQ ID NO: 333: amino acid sequence of chimera A-E57R-Fc (g1S LALAGANA)
SEQ ID NO: 334: amino acid sequence of chimera A-E57V-Fc (g1S LALAGANA)
SEQ ID NO: 335: amino acid sequence of chimera A-E57K_R58D-Fc (g1S LALAGANA)
SEQ ID NO: 336: amino acid sequence of chimera A-E57K_R58E-Fc (g1S LALAGANA)
SEQ ID NO: 337: amino acid sequence of chimera A-E57R_R58D-Fc (g1S LALAGANA)
SEQ ID NO: 338: nucleotide sequence of Fc (IEGRMD g1S)
SEQ ID NO: 339: amino acid sequence of Fc (IEGRMD g1S)
SEQ ID NO: 340: amino acid sequence of R218Q-Fc
SEQ ID NO: 341: nucleotide sequence of FLAG-cynoLIGHT
SEQ ID NO: 342: amino acid sequence of FLAG-cynoLIGHT
SEQ ID NO: 343: nucleotide sequence of FasL variant in which cleavage site and intracellular proline-rich region are deleted from FasL as human DcR3 ligand and a FLAG tag is inserted at N-terminus
SEQ ID NO: 344: amino acid sequence of FasL variant in which cleavage site and intracellular proline-rich region are deleted from FasL as human DcR3 ligand and a FLAG tag is inserted at N-terminus
SEQ ID NO: 345: nucleotide sequence of S195-His6
SEQ ID NO: 346: amino acid sequence of S195-His6
SEQ ID NO: 347: nucleotide sequence of chimera A-His6
SEQ ID NO: 348: amino acid sequence of chimera A-His6
SEQ ID NO: 349: nucleotide sequence of chimera A-E57K-His6
SEQ ID NO: 350: amino acid sequence of chimera A-E57K-His6
SEQ ID NO: 351: nucleotide sequence of chimera A-E57K_R58E-His6
SEQ ID NO: 352: amino acid sequence of chimera A-E57K_R58E-His6
SEQ ID NO: 353: optimized nucleotide sequence of chimera A
SEQ ID NO: 354: optimized nucleotide sequence of chimera A-E57K
SEQ ID NO: 355: optimized nucleotide sequence of chimera A-E57K_R58E

The application contains the following items:
1. A DcR3 variant, the DcR3 variant being a variant of wild-type decoy receptor 3 (hereinafter abbreviated as DcR3) and exhibiting improved *in vivo* kinetics as compared to the wild-type DcR3.
2. The DcR3 variant according to item 1, wherein the DcR3 variant comprises one or more complex N-glycoside-linked glycans.
3. The DcR3 variant according to item 1, wherein the DcR3 variant has neutralizing activity to at least one or more of LIGHT, TL1A and FasL.
4. The DcR3 variant according to item 1, wherein the DcR3 variant has neutralizing activity to all of LIGHT, TL1A and FasL.
5. The DcR3 variant according to item 1, wherein the DcR3 variant has no neutralizing activity to FasL and has neutralizing activity to one or more of LIGHT and TL1A.
6. The DcR3 variant according to item 1, wherein the DcR3 variant has no neutralizing activity to FasL and has neutralizing activity to both of LIGHT and TL1A.
7. A DcR3 variant comprising a first chimeric cysteine-rich region or a second chimeric cysteine-rich region, wherein:
   the first chimeric cysteine-rich region consists of an amino acid sequence obtained by introducing into a cysteine-rich domain (hereinafter abbreviated as CRD) of wild-type DcR3, substitution of at least a part of the cysteine-rich domain of the wild-type DcR3 with at least a part of a cysteine-rich domain of a TNF receptor superfamily molecule other than DcR3; and
   the second chimeric cysteine-rich region consists of an amino acid sequence obtained by introducing into the amino acid sequence of the first chimeric cysteine-rich region, deletion, substitution, insertion or addition of 1 to 30 amino acids.
8. The DcR3 variant according to item 7, wherein the DcR3 variant comprises one or more complex N-glycoside-linked glycans.
9. The DcR3 variant according to item 7, wherein the DcR3 variant has neutralizing activity to at least one or more of LIGHT, TL1A and FasL.
10. The DcR3 variant according to item 7, wherein the DcR3 variant has neutralizing activity to all of LIGHT, TL1A and FasL.
11. The DcR3 variant according to item 7, wherein the DcR3 variant has no neutralizing activity to FasL and has neutralizing activity to one or more of LIGHT and TL1A.
12. The DcR3 variant according to item 7, wherein the DcR3 variant has no neutralizing activity to FasL and has neutralizing activity to both of LIGHT and TL1A.
13. The DcR3 variant according to item 7, wherein the TNF receptor superfamily molecule is OPG.
14. The DcR3 variant according to item 7, wherein:
   the at least a part of the cysteine-rich domain of the wild-type DcR3 is selected from a part or a whole of CRD1, a part or a whole of CRD2, a part or a whole of CRD3 and a part or a whole of CRD4; and
   the at least a part of the cysteine-rich domain of the TNF receptor superfamily molecule is selected from a part or a whole of CRD1, a part or a whole of CRD2, a part or a whole of CRD3 and a part or a whole of CRD4.
15. The DcR3 variant according to item 14, wherein the first chimeric cysteine-rich region comprises one or more substitution selected from:
   substitution of the part of the CRD1 of the wild-type DcR3 with a part of the CRD1 of the TNF receptor superfamily molecule, wherein the part of the CRD1 of the TNF receptor superfamily molecule corresponds to the part of the CRD1 of the wild-type DcR3;
   substitution of the whole of the CRD1 of the wild-type DcR3 with the whole of the CRD1 of the TNF receptor superfamily molecule;
   substitution of the part of the CRD2 of the wild-type DcR3 with a part of the CRD2 of the TNF receptor superfamily molecule, wherein the part of the CRD2 of the TNF receptor superfamily molecule corresponds to the part of the CRD2 of the wild-type DcR3;
   substitution of the whole of the CRD2 of the wild-type DcR3 with the whole of the CRD2 of the TNF receptor superfamily molecule;
   substitution of the part of the CRD3 of the wild-type DcR3 with a part of the CRD3 of the TNF receptor superfamily molecule, wherein the part of the CRD3 of the TNF receptor superfamily molecule corresponds to the part of the CRD3 of the wild-type DcR3;
   substitution of the whole of the CRD3 of the wild-type DcR3 with the whole of the CRD3 of the TNF receptor superfamily molecule;
   substitution of the part of the CRD4 of the wild-type DcR3 with a part of the CRD4 of the TNF receptor superfamily molecule, wherein the part of the CRD4 of the TNF receptor superfamily molecule corresponds to the part of the CRD4 of the wild-type DcR3; and
   substitution of the whole of the CRD4 of the wild-type DcR3 with the whole of the CRD4 of the TNF receptor superfamily molecule.
16. The DcR3 variant according to item 15, wherein the part or the whole of the CRD2 of the wild-type DcR3 is maintained in the first chimeric cysteine-rich region.
17. The DcR3 variant according to item 15, wherein the part or the whole of the CRD3 of the wild-type DcR3 is maintained in the first chimeric cysteine-rich region.
18. The DcR3 variant according to item 14, wherein the first chimeric cysteine-rich region comprises the following amino acid sequence (a), (b), (c) or (d), and the second chimeric cysteine-rich region comprises the following amino acid sequence (e):
   (a) an amino acid sequence obtained by introducing into the amino acid sequence of the cysteine-rich region of the wild-type DcR3, substitution of the CRD1 of the wild-type DcR3 with CRD1 of OPG;
   (b) an amino acid sequence obtained by introducing into the amino acid sequence of the cysteine-rich region of the wild-type DcR3, substitution of the CRD4 of the wild-type DcR3 with CRD4 of OPG;
   (c) an amino acid sequence obtained by introducing into the amino acid sequence of the cysteine-rich region of the wild-type DcR3, substitution of the CRD1 of the wild-type DcR3 with CRD1 of OPG, and substitution of the CRD4 of the wild-type DcR3 with CRD4 of OPG;
   (d) an amino acid sequence obtained by introducing into the amino acid sequence (a), (b) or (c), substitution of a part at positions 103 to 123 from the N-terminus with a corresponding part of an amino acid sequence of a cysteine-rich domain of OPG; and
   (e) an amino acid sequence obtained by introducing into the amino acid sequence (a), (b), (c) or (d), deletion, substitution, insertion or addition of 1 to 30 amino acids.
19. The DcR3 variant according to item 18, wherein:
   the amino acid sequence (a) is an amino acid sequence consisting of amino acids at positions 1 to 164 from the N-terminus of the amino acid sequence set forth in SEQ ID NO: 26 or 50;
   the amino acid sequence (b) is an amino acid sequence consisting of amino acids at positions 1 to 164 from the N-terminus of the amino acid sequence set forth in SEQ ID NO: 28 or 52;
   the amino acid sequence (c) is an amino acid sequence consisting of amino acids at positions 1 to 164 from the N-terminus of the amino acid sequence set forth in SEQ ID NO: 30 or 54; and
   the amino acid sequence (d) is an amino acid sequence consisting of amino acids at positions 1 to 164 from the N-terminus of the amino acid sequence set forth in SEQ ID NO: 32 or 56.
20. The DcR3 variant according to item 18, wherein the amino acid sequence (e) comprises one or two or more substitution selected from the group consisting of:
   substitution of Glu at position 57 from the N-terminus of the amino acid sequence (a), (b), (c) or (d) with another amino acid;
   substitution of Arg at position 58 from the N-terminus of the amino acid sequence (a), (b), (c) or (d) with another amino acid; and
   substitution of Arg at position 60 from the N-terminus of the amino acid sequence (a), (b), (c) or (d) with another amino acid.
21. The DcR3 variant according to item 18, wherein the amino acid sequence (e) comprises substitution of Glu at position 57 and Arg at position 58 from the N-terminus of the amino acid sequence (a), (b), (c) or (d) with other amino acids.
22. The DcR3 variant according to item 18, wherein the amino acid sequence (e) comprises one or two or more substitution selected from the group consisting of:
   substitution of Glu at position 57 from the N-terminus of the amino acid sequence (a), (b), (c) or (d) with Lys, Leu, Arg, Val, Ala, Phe, His, Ile or Met;
   substitution of Arg at position 58 from the N-terminus of the amino acid sequence (a), (b), (c) or (d) with Asp, Glu or Thr; and
   substitution of Arg at position 60 from the N-terminus of the amino acid sequence (a), (b), (c) or (d) with Lys.
23. The DcR3 variant according to item 18, wherein the amino acid sequence (e) comprises substitution of Glu at position 57 from the N-terminus of the amino acid sequence (a), (b), (c) or (d) with Lys, Leu, Arg, Val, Ala, Phe, His, Ile or Met, and substitution of Arg at position 58 from the N-terminus of the amino acid sequence (a), (b), (c) or (d) with Asp, Glu or Thr.
24. The DcR3 variant according to item 18, wherein the amino acid sequence (e) comprises substitution selected from the following (f) to (i):
   (f) substitution of Asn at positions 131 and 144 from the N-terminus of the amino acid sequence (b), (c) or (d) with other amino acids;
   (g) substitution of Asn at positions 131, 144 and 157 from the N-terminus of the amino acid sequence (b), (c) or (d) with other amino acids;
   (h) substitution of Thr at position 133 and Ser at position 146 from the N-terminus of the amino acid sequence (b), (c) or (d) with other amino acids; and
   (i) substitution of Thr at position 133, Ser at position 146 and Thr at position 159 from the N-terminus of the amino acid sequence (b), (c) or (d) with other amino acids.
25. The DcR3 variant according to item 18, wherein the amino acid sequence (e) comprises substitution selected from the following (f') to (i'):
   (f') substitution of Asn at positions 131 and 144 from the N-terminus of the amino acid sequence (b), (c) or (d) with Ser;
   (g') substitution of Asn at positions 131, 144 and 157 from the N-terminus of the amino acid sequence (b), (c) or (d) with Ser;
   (h') substitution of Thr at position 133 and Ser at position 146 from the N-terminus of the amino acid sequence (b), (c) or (d) with Ala; and
   (i') substitution of Thr at position 133, Ser at position 146 and Thr at position 159 from the N-terminus of the amino acid sequence (b), (c) or (d) with Ala.
26. The DcR3 variant according to item 18, wherein the amino acid sequence (e) is an amino acid sequence consisting of amino acids at positions 1 to 164 from the N-terminus of an amino acid sequence set forth in SEQ ID NO: 58, 60, 62, 64, 66, 68, 70, 180, 182, 184, 186, 188, 270, 272, 274, 276, 278, 280, 282, 284 or 286.
27. The DcR3 variant according to item 7, wherein the DcR3 variant is a DcR3 variant comprising the first or second chimeric cysteine-rich region, and a part or a whole of a heparan sulfate-binding domain of the wild-type DcR3 bound to the C-terminal side of the first or second chimeric cysteine-rich region, or a DcR3 variant comprising the first or second chimeric cysteine-rich region and not comprising a heparan sulfate-binding domain of the wild-type DcR3.
28. The DcR3 variant according to item 27, wherein the DcR3 variant comprises one of amino acid sequences selected from:
   (I) an amino acid sequence set forth in SEQ ID NO: 26, 28, 30, 32, 34, 36, 38, 40, 42, 44 or 46, or an amino acid sequence obtained by introducing into the abovementioned amino acid sequence, deletion, substitution, insertion or addition of 1 to 30 amino acids; and
   (II) an amino acid sequence set forth in SEQ ID NO: 50, 52, 54, 56, 58, 60, 62, 64, 66, 68, 70, 180, 182, 184, 186, 188, 270, 272, 274, 276, 278, 280, 282, 284 or 286, or an amino acid sequence obtained by introducing into the abovementioned amino acid sequence, deletion, substitution, insertion or addition of 1 to 30 amino acids.
29. The DcR3 variant according to item 7, wherein the DcR3 variant comprises an Fc region derived from a human IgG1, IgG2 or IgG4 antibody, or a mutated Fc region consisting of an amino acid sequence obtained by introducing into an amino acid sequence of the abovementioned Fc region, deletion, substitution, insertion or addition of one or several amino acids.
30. The DcR3 variant according to item 29, wherein the Fc region or the mutated Fc region is bound to the C-terminal side of the first or second chimeric cysteine-rich region via another region or a linker.
31. The DcR3 variant according to item 29, wherein the mutated Fc region comprises substitution of Cys with Ser at EU-index position 220 of an amino acid sequence of a heavy chain of human IgG1.
32. The DcR3 variant according to item 31, wherein the mutated Fc region comprises substitution of Leu with Ala at EU-index position 234, substitution of Leu with Ala at EU-index position 235, and substitution of Gly with Ala at EU-index position 237 of an amino acid sequence of a heavy chain of human IgG1.
33. The DcR3 variant according to item 31, wherein the mutated Fc region comprises substitution of Asn with Ala at EU-index position 434 of an amino acid sequence of a heavy chain of human IgG1.
34. The DcR3 variant according to item 31, wherein the mutated Fc region comprises substitution of Met with Tyr at EU-index position 252, substitution of Ser with Thr at EU-index position 254, and substitution of Thr with Glu at EU-index position 256 of an amino acid sequence of a heavy chain of human IgG1.
35. The DcR3 variant according to item 29, wherein the mutated Fc region comprises substitution of Ser with Pro at EU-index position 228, substitution of Leu with Glu at EU-index position 235, and substitution of Arg with Lys at EU-index position 409 of an amino acid sequence of a heavy chain of human IgG4.
36. The DcR3 variant according to item 29, wherein the DcR3 variant comprises a mutated Fc region consisting of an amino acid sequence set forth in SEQ ID NO: 72, 74, 156, 158, 160, 162, 164, 166, 311, 312 or 313.
37. The DcR3 variant according to item 29, wherein the DcR3 variant comprises an amino acid sequence set forth in SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 or 337, or an amino acid sequence obtained by introducing into the abovementioned amino acid sequence, deletion, substitution, insertion or addition of 1 to 30 amino acids.
38. A DcR3 variant composition comprising the DcR3 variant according to item 1 or 7.
39. The composition according to item 38, wherein the composition comprises a DcR3 variant having one or more complex N-glycoside-linked glycans, and a DcR3 variant not having a complex N-glycoside-linked glycan.
40. A DNA encoding the DcR3 variant according to item 1 or 7.
41. A gene recombinant vector comprising the DNA according to item 40.
42. A transformant obtained by introducing the gene recombinant vector according to item 41 into a host cell.
43. The transformant according to item 42, wherein the host cell is a cell derived from a mammal.
44. The transformant according to item 43, wherein the cell derived from a mammal is a CHO cell.
45. A method of producing a DcR3 variant or a DcR3 variant composition, wherein the method comprises culturing the transformant according to item 42 in a medium to generate and accumulate the DcR3 variant according to item 1 or 7, and purifying the DcR3 variant from the obtained culture solution.
46. A DcR3 variant or a DcR3 variant composition produced using the production method according to item 45.
47. A pharmaceutical composition comprising the DcR3 variant or the DcR3 variant composition according to item 1, 7 or 46 as an active ingredient.
48. The pharmaceutical composition according to item 47, wherein the pharmaceutical composition is a prophylactic or therapeutic agent for an autoimmune disease, an inflammatory disease or an allergic disease.
49. A method of preventing or treating an autoimmune disease, an inflammatory disease or an allergic disease, the method comprising administering the pharmaceutical composition according to item 47 to a patient in need thereof.

## Claims

1. A method of producing a DcR3 variant or a DcR3 variant composition, wherein the method comprises culturing a transformant obtained by introducing a vector comprising DNA encoding said DcR3 variant into a host cell in a medium to generate and accumulate the DcR3 variant, and purifying the DcR3 variant from the obtained culture solution, the DcR3 variant comprising a first chimeric cysteine-rich region or a second chimeric cysteine-rich region, wherein:
the first chimeric cysteine-rich region consists of any one of the following amino acid sequences:
(a) an amino acid sequence consisting of amino acids at positions 1 to 164 from the N-terminus of the amino acid sequence set forth in SEQ ID NO: 26 or 50;
(b) an amino acid sequence consisting of amino acids at positions 1 to 164 from the N-terminus of the amino acid sequence set forth in SEQ ID NO: 28 or 52;
(c) an amino acid sequence consisting of amino acids at positions 1 to 164 from the N-terminus of the amino acid sequence set forth in SEQ ID NO: 30 or 54; and
(d) an amino acid sequence consisting of amino acids at positions 1 to 164 from the N-terminus of the amino acid sequence set forth in SEQ ID NO: 32 or 56; and
the second chimeric cysteine-rich region consists of an amino acid sequence obtained by introducing into the amino acid sequence of the first chimeric cysteine-rich region, deletion, substitution, insertion or addition of 1 to 5 amino acids,
wherein the transformant is stably expressing the DcR3 variant.

2. A method of producing a DcR3 variant or a DcR3 variant composition, wherein the method comprises culturing a transformant obtained by introducing a vector comprising DNA encoding said DcR3 variant into a host cell in a medium to generate and accumulate the DcR3 variant, and purifying the DcR3 variant from the obtained culture solution, the DcR3 variant comprising a first chimeric cysteine-rich region or a second chimeric cysteine-rich region, wherein:
the first chimeric cysteine-rich region consists of any one of the following amino acid sequences:
(a) an amino acid sequence consisting of amino acids at positions 1 to 164 from the N-terminus of the amino acid sequence set forth in SEQ ID NO: 26 or 50;
(b) an amino acid sequence consisting of amino acids at positions 1 to 164 from the N-terminus of the amino acid sequence set forth in SEQ ID NO: 28 or 52;
(c) an amino acid sequence consisting of amino acids at positions 1 to 164 from the N-terminus of the amino acid sequence set forth in SEQ ID NO: 30 or 54; and
(d) an amino acid sequence consisting of amino acids at positions 1 to 164 from the N-terminus of the amino acid sequence set forth in SEQ ID NO: 32 or 56; and
the second chimeric cysteine-rich region consists of an amino acid sequence obtained by introducing into the amino acid sequence of the first chimeric cysteine-rich region, deletion, substitution, insertion or addition of 1 to 5 amino acids,
wherein said culture is performed under aerobic conditions.

3. A method of producing a DcR3 variant or a DcR3 variant composition, wherein the method comprises culturing a transformant obtained by introducing a vector comprising DNA encoding said DcR3 variant into a host cell in a medium to generate and accumulate the DcR3 variant, and purifying the DcR3 variant from the obtained culture solution, the DcR3 variant comprising a first chimeric cysteine-rich region or a second chimeric cysteine-rich region, wherein:
the first chimeric cysteine-rich region consists of any one of the following amino acid sequences:
(a) an amino acid sequence consisting of amino acids at positions 1 to 164 from the N-terminus of the amino acid sequence set forth in SEQ ID NO: 26 or 50;
(b) an amino acid sequence consisting of amino acids at positions 1 to 164 from the N-terminus of the amino acid sequence set forth in SEQ ID NO: 28 or 52;
(c) an amino acid sequence consisting of amino acids at positions 1 to 164 from the N-terminus of the amino acid sequence set forth in SEQ ID NO: 30 or 54; and
(d) an amino acid sequence consisting of amino acids at positions 1 to 164 from the N-terminus of the amino acid sequence set forth in SEQ ID NO: 32 or 56; and
the second chimeric cysteine-rich region consists of an amino acid sequence obtained by introducing into the amino acid sequence of the first chimeric cysteine-rich region, deletion, substitution, insertion or addition of 1 to 5 amino acids,
wherein the pH during culture is kept at 3.0 to 9.0.

4. The method according to any of claims 1-3, wherein the host cell is a cell derived from a mammal.

5. The method according to claim 4, wherein the host cell is a CHO cell.

6. The method according to any one of claims 1-5, wherein the DcR3 variant is extracellularly secreted, and the DcR3 variant is collected from the culture supernatant.

7. The method according to claim 6, wherein the culture is subjected to a method to obtain a culture supernatant, and from the culture supernatant, a protein isolation and purification method to obtain a purified product.

8. The method according to claim 7, wherein the protein isolation and purification method is selected from a solvent extraction method, a salting-out method with ammonium sulfate or the like, a desalting method, a precipitation method with an organic solvent, an anion exchange chromatography method using a resin, a cation exchange chromatography method using a resin, a hydrophobic chromatography method using a resin, a gel filtration method using a molecular sieve, an affinity chromatography method, a chromatofocusing method, or an electrophoresis method , alone or in combination.

9. The method according to claim 7 or 8, wherein the DcR3 variant has a Fc of an immunoglobulin that can bind to Protein G or Protein A, and wherein a Protein G chromatography method or a Protein A chromatography method in which Protein G or Protein A is bound to a carrier as an affinity ligand is used as an affinity chromatography.

10. The method according to any one of the previous claims, wherein the DcR3 variant comprises an amino acid sequence consisting of amino acids at positions 1 to 164 from the N-terminus of an amino acid sequence set forth in SEQ ID NO: 58, 60, 62, 64, 66, 68, 70, 180, 182, 184, 186, 188, 270, 272, 274, 276, 278, 280, 282, 284 or 286.

11. The method according to any of the previous claims, wherein the DcR3 variant comprises an Fc region derived from a human IgG1, IgG2 or IgG4 antibody, or a mutated Fc region consisting of an amino acid sequence obtained by introducing into an amino acid sequence of the abovementioned Fc region, deletion, substitution, insertion or addition of one or several amino acids.

12. The method according to claim 11, wherein the DcR3 variant comprises an amino acid sequence set forth in SEQ ID NO: 76, 78, 80, 82, 84, 86, 88, 90, 92, 94, 96, 98, 150, 168, 170, 172, 174, 176, 178, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 288, 290, 292, 294, 296, 298, 300, 302, 304, 314, 315, 316, 317, 318, 319, 320, 321, 322, 323, 324, 325, 326, 327, 328, 329, 330, 331, 332, 333, 334, 335, 336 or 337, or an amino acid sequence obtained by introducing into the abovementioned amino acid sequence, deletion, substitution, insertion or addition of 1 amino acid.

13. The method according to any one of the previous claims, wherein the DcR3 variant comprises an amino acid sequence set forth in SEQ ID NO: 214, or an amino acid sequence having 99% or more identity to SEQ ID NO: 214.

14. The method according to any one of claims 1-12, wherein the DcR3 variant comprises an amino acid sequence set forth in SEQ ID NO: 224, or an amino acid sequence having 99% or more identity to SEQ ID NO: 224.

15. The method according to any one of claims 1-12, wherein the DcR3 variant comprises an amino acid sequence set forth in SEQ ID NO: 256, or an amino acid sequence having 99% or more identity to SEQ ID NO: 256.

16. The method according to any one of claims 1-12, wherein the DcR3 variant comprises an amino acid sequence set forth in SEQ ID NO: 266, or an amino acid sequence having 99% or more identity to SEQ ID NO: 266.
